(19) Europäisches Patentamt
European Patent Office
Office européen des brevets

(11) **EP 3 418 276 A1**

(12) **EUROPEAN PATENT APPLICATION**
published in accordance with Art. 153(4) EPC

(43) Date of publication:
**26.12.2018 Bulletin 2018/52**

(21) Application number: **17753183.7**

(22) Date of filing: **14.02.2017**

(51) Int Cl.:
***C07D 401/04*** *(2006.01)* ***A61K 31/4439*** *(2006.01)*
***A61K 31/444*** *(2006.01)* ***A61K 31/4545*** *(2006.01)*
***A61K 31/46*** *(2006.01)* ***A61K 31/4709*** *(2006.01)*
***A61K 31/498*** *(2006.01)* ***A61K 31/5355*** *(2006.01)*
***A61K 31/5377*** *(2006.01)* ***A61P 13/12*** *(2006.01)*
***A61P 43/00*** *(2006.01)* ***C07D 401/14*** *(2006.01)*
***C07D 405/14*** *(2006.01)* ***C07D 409/14*** *(2006.01)*
***C07D 413/14*** *(2006.01)* ***C07D 417/14*** *(2006.01)*
***C07D 451/02*** *(2006.01)*

(86) International application number:
**PCT/JP2017/005388**

(87) International publication number:
**WO 2017/141927 (24.08.2017 Gazette 2017/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **15.02.2016 JP 2016025878**

(71) Applicant: **Taisho Pharmaceutical Co., Ltd.**
**Tokyo 170-8633 (JP)**

(72) Inventors:
- **TANAKA, Hiroaki**
  **Tokyo 170-8633 (JP)**
- **BOHNO, Ayako**
  **Tokyo 170-8633 (JP)**
- **HAMADA, Makoto**
  **Tokyo 170-8633 (JP)**
- **ITO, Yuji**
  **Tokyo 170-8633 (JP)**
- **KOBASHI, Yohei**
  **Tokyo 170-8633 (JP)**
- **KAWAMURA, Madoka**
  **Tokyo 170-8633 (JP)**

(74) Representative: **Hoffmann Eitle**
**Patent- und Rechtsanwälte PartmbB**
**Arabellastraße 30**
**81925 München (DE)**

(54) **AZOLE-SUBSTITUTED PYRIDINE COMPOUND**

(57) The present invention provides a compound represented by formula [I'| shown below or a pharmaceutically acceptable salt thereof that has an inhibitory effect on 20-HETE producing enzyme, wherein the structure represented by formula [III] shown below represents any of the structures represented by formula group [IV] shown below, wherein $R^1$ represents a hydrogen atom, a fluorine atom, methyl, etc.; $R^2$, $R^3$, and $R^4$ each independently represent a hydrogen atom, a fluorine atom, or methyl; W represents a single bond, $C_{1-3}$alkanediyl, or the formula $-O-CH_2CH_2-$; and ring A represents (a) substituted $C_{4-6}$cycloalkyl, (b) substituted 4- to 6-membered saturated nitrogen-containing heterocyclyl, (c) substituted phenyl, (d) substituted pyridyl, (e) substituted 2,3-dihydrobenzofuran, (f) 4- to 6-membered saturated oxygen-containing heterocyclyl, etc.

EP 3 418 276 A1

[Formula 1]

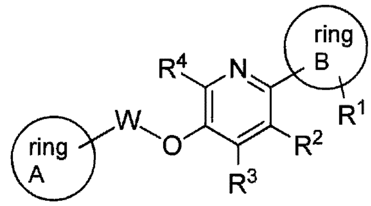

[I']

[Formula 2]

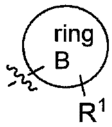

[III]

[Formula 3]

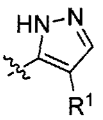 , 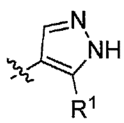 , 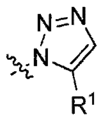 , 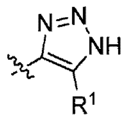 , 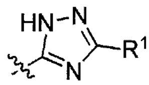 ,

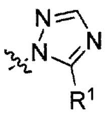 , 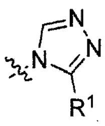 , 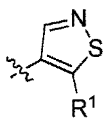 , 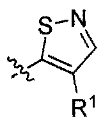

[IV]

## Description

### TECHNICAL FIELD

The present invention relates to an inhibitor of enzymes that produce 20-hydroxyeicosatetraenoic acid (hereinafter also referred to as "20-HETE"). More specifically, the present invention relates to an azole-substituted pyridine compound which is an inhibitor of 20-HETE producing enzymes.

### BACKGROUND ART

Physiologically active substances produced from arachidonic acid conventionally include prostaglandins produced by cyclooxygenase and leukotrienes produced by lipoxygenase; in addition to these, 20-HETE produced from arachidonic acid by enzymes belonging to cytochrome P450 have recently been shown to display diverse functions in a living body. So far, 20-HETE has been demonstrated to control vascular tone or evoke cell growth in cerebral blood vessels and key organs such as kidney, suggesting that 20-HETE plays an important physiological role in a living body while being deeply involved in the pathology of various cerebro-vascular diseases, kidney diseases, cardiovascular diseases, and others (Non Patent Literatures 1 to 3). Furthermore, it has been proven in recent years that 20-HETE is involved in the onset of polycystic kidney disease. Polycystic kidney disease is a hereditary cystic kidney disease, which is classified into autosomal dominant polycystic kidney disease and autosomal recessive polycystic kidney disease, in which a great number of cysts are formed in the kidney to cause impaired renal function. It is suggested that when administered to pathologic animals developing polycystic kidney disease, 20-HETE inhibitors not only block intracellular growth signals but also exhibit an ameliorating effect on renal cysts (Non Patent Literature 4). Moreover, increased renal volume and decreased renal function are shown to correlate with increased plasma 20-HETE levels in patients with autosomal dominant polycystic kidney disease, suggesting that 20-HETE is associated with the progression of polycystic kidney disease (Non Patent Literature 5).

Previously reported inhibitors of 20-HETE producing enzymes include, for example, a hydroxyformamidine derivative (Patent Literature 1), a heterocycle derivative as a compound having the phenylazoleskeleton (Patent Literature 2), and a phenylazole compound (Patent Literature 3). Patent Literature 2 discloses a heteroaryl-substituted pyridine compound that is substituted with heteroaryl such as pyrazolyl at the 3-position of pyridine. However, the compound of the present invention or an azole-substituted pyridine compound that is a compound substituted with azole such as pyrazolyl at the 2-position of pyridine is yet to be disclosed.

### CITATION LIST

### PATENT LITERATURE

PTL 1: WO01/032164
PTL2: WO03/022821
PTL 3: WO2004/092163

### NON PATENT LITERATURE

NPL 1: Journal of Vascular Research, Vol. 32, p. 79, 1995
NPL 2: The American Journal of Physiology, Vol. 277, p. R607, 1999
NPL 3: Physiological Reviews, Vol. 82, p. 131, 2002
NPL 4: American Journal of Physiology Renal Physiology, Vol. 296, p. F575, 2009
NPL 5: Journal of Lipid Research, Vol. 55, p. 1139, 2013

### SUMMARY OF INVENTION

### TECHNICAL PROBLEM

An object of the present invention is to provide a novel compound that inhibits 20-HETE producing enzymes.

SOLUTION TO PROBLEM

As a result of intensive studies to solve the above problem, the present inventors found that a compound represented by formula [I'] shown below (hereinafter also referred to as the compound [I']) has an inhibitory effect on 20-HETE producing enzymes.
The present invention will be described in detail below.
Briefly, the following are embodiments of the present invention.

(1) In one embodiment, the present invention provides
A compound represented by formula [I'] shown below:

[Formula 1]

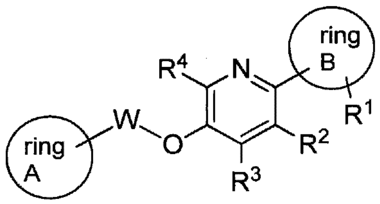

[I']

wherein
the structure represented by formula [III] shown below:

[Formula 2]

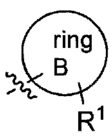

[III]

represents any of the structures represented by formula group [IV] shown below:

[Formula 3]

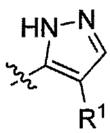 , 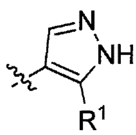 ,

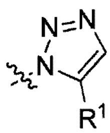 , 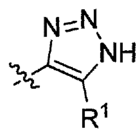 ,

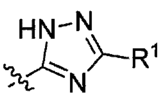 , 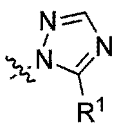 , 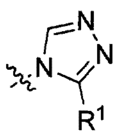 ,

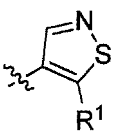 , 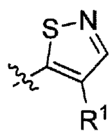

[IV]

wherein

$R^1$ represents a hydrogen atom, hydroxy, carbamoyl, cyano, a fluorine atom, a chlorine atom, a bromine atom, methyl, hydroxymethyl, methoxymethyl, difluoromethyl, trifluoromethyl, methoxy, or cyclopropylaminocarbonyl;
$R^2$, $R^3$, and $R^4$ each independently represent a hydrogen atom, a fluorine atom, or methyl;
W represents a single bond, $C_{1-3}$alkanediyl, or the formula $-O-CH_2CH_2-$;
ring A represents

(a) $C_{4-6}$cycloalkyl, wherein the $C_{4-6}$cycloalkyl is substituted with one substituent selected from substituent group A11,
(b) 4- to 6-membered saturated nitrogen-containing heterocyclyl, wherein the 4- to 6-membered saturated nitrogen-containing heterocyclyl is substituted with one substituent selected from substituent group A21 and may be further substituted with one substituent selected from substituent group A22,
(c) phenyl, wherein the phenyl is substituted with one substituent selected from substituent group A31 and may be further substituted with one substituent selected from substituent group A32,
(d) pyridyl, wherein the pyridyl is substituted with one substituent selected from substituent group A41,
(e) naphthyl,
(f) 2,3-dihydrobenzofuran, wherein the 2,3-dihydrobenzofuran may be substituted with one to three substituents selected from substituent group A51,
(g) 2H-chromenyl, wherein the 2H-chromenyl may be substituted with one oxo,
(h) quinolyl, wherein the quinolyl may be substituted with one $C_{1-6}$alkoxy,
(j) quinoxalyl,
(k) a group represented by formula [11-1] shown below, wherein the group represented by formula [II-1] is substituted with one $C_{1-6}$alkyl, wherein the $C_{1-6}$alkyl may be substituted with one substituent selected from substituent group B61,
(m) a group represented by formula [II-2] shown below, wherein the group represented by formula [II-2] is substituted with one $C_{1-6}$alkylcarbonyl,
(n) a group represented by formula [II-3] shown below, wherein the group represented by formula [II-3] is substituted with one $C_{1-6}$alkylcarbonyl,
(p) a group represented by formula [II-4] shown below, wherein the group represented by formula [II-4] is substituted with one $C_{1-6}$alkylcarbonyl,
(r) 4- to 6-membered saturated oxygen-containing heterocyclyl, or
(s) 4- to 6-membered saturated sulfur-containing heterocyclyl, wherein the 4- to 6-membered saturated sulfur-containing heterocyclyl may be substituted with one or two oxo;

[Formula 4]

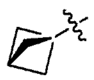
[II−1],
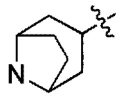
[II−2],
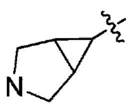
[II−3],
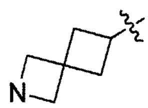
[II−4]

wherein substituent group A11 represents the group consisting of

(i) $C_{1-6}$alkylcarbonylamino and
(ii) $C_{1-6}$alkylcarbonyl($C_{1-6}$alkyl)amino;

substituent group A21 represents the group consisting of

(i) $C_{1-6}$alkylcarbonyl, wherein the $C_{1-6}$alkylcarbonyl may be substituted with one to three substituents selected from substituent group B21,
(ii) $C_{3-8}$cycloalkylcarbonyl, wherein the $C_{3-8}$cycloalkylcarbonyl may be substituted with one or two substituents selected from substituent group B22,

(iii) arylcarbonyl, wherein the arylcarbonyl may be substituted with one substituent selected from substituent group B23,
(iv) saturated heterocyclylcarbonyl, wherein the saturated heterocyclylcarbonyl may be substituted with one or two substituents selected from substituent group B24,
(v) heteroarylcarbonyl, wherein the heteroarylcarbonyl may be substituted with one substituent selected from the group consisting of $C_{1-6}$alkyl, wherein the $C_{1-6}$alkyl may be substituted with one hydroxy,
(vi) $C_{1-6}$alkoxycarbonyl,
(vii) mono$C_{1-6}$alkylaminocarbonyl,
(viii) di$C_{1-6}$alkylaminocarbonyl,
(ix) $C_{3-8}$cycloalkylaminocarbonyl,
(x) $C_{3-8}$cycloalkyl($C_{1-6}$alkyl)aminocarbonyl,
(xi) $C_{1-6}$alkylsulfonyl, wherein the $C_{1-6}$alkylsulfonyl may be substituted with one $C_{1-6}$alkoxycarbonylamino,
(xii) $C_{3-8}$cycloalkylsulfonyl,
(xiii) saturated heterocyclylsulfonyl, wherein the saturated heterocyclylsulfonyl may be substituted with one substituent selected from substituent group B25, and
(xiv) di$C_{1-6}$alkylaminosulfonyl;

substituent group A22 represents the group consisting of

(i) a halogen atom and
(ii) $C_{1-6}$alkyl;

substituent group B21 represents the group consisting of

(i) hydroxy,
(ii) carbamoyl,
(iii) ureide,
(iv) a halogen atom,
(v) $C_{3-8}$cycloalkyl, wherein the $C_{3-8}$cycloalkyl may be substituted with one hydroxy,
(vi) saturated heterocyclyl, wherein the saturated heterocyclyl may be substituted with one or two substituents selected from the group consisting of hydroxy and oxo,
(vii) heteroaryl, wherein the heteroaryl may be substituted with one oxo,
(viii) $C_{1-6}$alkoxy,
(ix) aryloxy,
(x) saturated heterocyclylcarbonyl,
(xi) $C_{1-6}$alkylsulfonyl,
(xii) halo-$C_{1-6}$alkylsulfonyl,
(xiii) arylsulfonyl,
(xiv) $C_{1-6}$alkylcarbonylamino, wherein $C_{1-6}$alkyl in the $C_{1-6}$alkylcarbonylamino may be substituted with one substituent selected from the group consisting of hydroxy and saturated heterocyclyl,
(xv) $C_{1-6}$alkylcarbonyl($C_{1-6}$alkyl)amino,
(xvi) $C_{3-8}$cycloalkylcarbonylamino, wherein $C_{3-8}$cycloalkyl in the $C_{3-8}$cycloalkylcarbonylamino may be substituted with one or two halogen atoms,
(xvii) arylcarbonylamino,
(xviii) saturated heterocyclylcarbonylamino,
(xix) mono$C_{1-6}$alkylaminocarbonyl,
(xx) di$C_{1-6}$alkylaminocarbonyl,
(xxi) $C_{1-6}$alkoxycarbonylamino, wherein $C_{1-6}$alkoxy in the $C_{1-6}$alkoxycarbonylamino may be substituted with one substituent selected from the group consisting of $C_{1-6}$alkoxy and aryl,
(xxii) $C_{1-6}$alkoxycarbonyl($C_{1-6}$alkyl)amino,
(xxiii) $C_{3-8}$cycloalkoxycarbonylamino, wherein $C_{3-8}$cycloalkoxy in the $C_{3-8}$cycloalkoxycarbonylamino may be substituted with one $C_{1-6}$alkyl,
(xxiv) mono$C_{1-6}$alkylaminocarbonylamino, and
(xxv) di$C_{1-6}$alkylaminocarbonylamino;

substituent group B22 represents the group consisting of

(i) hydroxy,

(ii) carbamoyl,
(iii) a halogen atom,
(iv) $C_{1-6}$alkyl, and
(v) $C_{1-6}$alkoxycarbonylamino;

substituent group B23 represents

(i) $C_{1-6}$alkoxy, wherein the $C_{1-6}$alkoxy may be substituted with one carbamoyl;

substituent group B24 represents the group consisting of

(i) oxo,
(ii) a halogen atom,
(iii) $C_{1-6}$alkyl,
(iv) $C_{1-6}$alkylcarbonyl, and
(v) $C_{1-6}$alkoxycarbonyl;

substituent group B25 represents the group consisting of

(i) $C_{1-6}$alkylcarbonyl and
(ii) $C_{1-6}$alkoxycarbonyl, wherein the $C_{1-6}$alkoxycarbonyl may be substituted with one aryl;

substituent group A31 represents the group consisting of

(i) amino,
(ii) $C_{1-6}$alkyl,
(iii) halo-$C_{1-6}$alkyl,
(iv) $C_{2-6}$alkenyl, wherein the $C_{2-6}$alkenyl may be substituted with one substituent selected from substituent group B32,
(v) saturated heterocyclyl, wherein the saturated heterocyclyl may be substituted with one or two substituents selected from substituent group B34,
(vi) $C_{1-6}$alkoxy,
(vii) halo-$C_{1-6}$alkoxy,
(viii) $C_{1-6}$alkylsulfanyl,
(ix) halo-$C_{1-6}$alkylsulfanyl,
(x) saturated heterocyclylcarbonyl, wherein the saturated heterocyclylcarbonyl may be substituted with one or two $C_{1-6}$alkyl,
(xi) $C_{1-6}$alkylsulfonyl, wherein the $C_{1-6}$alkylsulfonyl may be substituted with one substituent selected from substituent group B35,
(xii) $C_{3-8}$cycloalkylsulfonyl,
(xiii) arylsulfonyl, wherein the arylsulfonyl may be substituted with one $C_{1-6}$alkyl,
(xiv) di$C_{1-6}$alkylaminosulfonyl,
(xv) $C_{1-6}$alkoxycarbonylamino, and
(xvi) S-methylsulfonimidoyl

substituent group A32 represents the group consisting of

(i) a halogen atom,
(ii) $C_{1-6}$alkyl,
(iii) halo-$C_{1-6}$alkyl, and
(iv) $C_{1-6}$alkoxy;

substituent group B32 represents

(i) aryl;

substituent group B34 represents the group consisting of

(i) $C_{1-6}$alkylcarbonyl,
(ii) $C_{1-6}$alkoxycarbonyl,
(iii) mono$C_{1-6}$alkylaminocarbonyl, and
(iv) di$C_{1-6}$alkylaminocarbonyl;

substituent group B35 represents the group consisting of

(i) $C_{3-8}$cycloalkyl,
(ii) saturated heterocyclyl, and
(iii) saturated heterocyclylcarbonyl;

substituent group A41 represents the group consisting of

(i) $C_{1-6}$alkyl,
(ii) halo-$C_{1-6}$alkyl,
(iii) triazolyl,
(iv) $C_{1-6}$alkylsulfonyl, wherein the $C_{1-6}$alkylsulfonyl may be substituted with one $C_{3-8}$cycloalkyl, and
(v) $C_{1-6}$alkylcarbonylamino;

substituent group A51 represents the group consisting of

(i) a halogen atom and
(ii) $C_{1-6}$alkyl; and

substituent group B61 represents the group consisting of

(i) $C_{1-6}$alkylcarbonylamino and
(ii) $C_{1-6}$alkylcarbonyl($C_{1-6}$alkyl)amino;

or a pharmaceutically acceptable salt thereof.

(2) In another embodiment, the present invention provides the compound or pharmaceutically acceptable salt thereof according to (1), wherein the structure represented by formula [III] shown below:

[Formula 5]

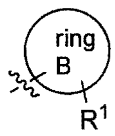
[III]

is any of the structures represented by formula group [V] shown below:

[Formula 6]

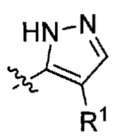 , 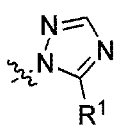 , 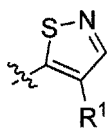 [V]

wherein

$R^1$ is a hydrogen atom, a fluorine atom, a chlorine atom, a bromine atom, or methyl;
$R^2$ is a hydrogen atom, a fluorine atom, or methyl;
$R^3$ is a hydrogen atom or methyl;
$R^4$ is a hydrogen atom;
W is $C_{1-2}$alkanediyl;

ring A is

(a) 4- to 6-membered saturated nitrogen-containing heterocyclyl, wherein the 4- to 6-membered saturated nitrogen-containing heterocyclyl is substituted with one substituent selected from substituent group A21",
(b) phenyl, wherein the phenyl is substituted with one substituent selected from substituent group A31" and may be further substituted with one halogen atom,
(c) pyridyl, wherein the pyridyl is substituted with one substituent selected from substituent group A41",
(d) 2,3-dihydrobenzofuran, wherein the 2,3-dihydrobenzofuran is substituted with one

halogen atom and two $C_{1-6}$alkyl, or
(e) 4- to 6-membered saturated oxygen-containing heterocyclyl;

wherein
substituent group A21" represents the group consisting of

(i) $C_{1-6}$alkylcarbonyl, wherein the $C_{1-6}$alkylcarbonyl may be substituted with one to three substituents selected from substituent group B21",
(ii) $C_{3-8}$cycloalkylcarbonyl, wherein the $C_{3-8}$cycloalkylcarbonyl may be substituted with one $C_{1-6}$alkoxycarbonylamino,
(iii) $C_{1-6}$alkoxycarbonyl,
(iv) mono$C_{1-6}$alkylaminocarbonyl,
(v) di$C_{1-6}$alkylaminocarbonyl,
(vi) $C_{1-6}$alkylsulfonyl, wherein the $C_{1-6}$alkylsulfonyl may be substituted with one $C_{1-6}$alkoxycarbonylamino,
(vii) $C_{3-8}$cycloalkylsulfonyl,
(viii) saturated heterocyclylsulfonyl, wherein the saturated heterocyclylsulfonyl may be substituted with one substituent selected from substituent group B25, and
(ix) di$C_{1-6}$alkylaminosulfonyl;

substituent group B21" represents the group consisting of

(i) a halogen atom,
(ii) $C_{3-8}$cycloalkyl, wherein the $C_{3-8}$cycloalkyl may be substituted with one hydroxy,
(iii) aryloxy,
(iv) $C_{3-8}$cycloalkylcarbonylamino, wherein $C_{3-8}$cycloalkyl in the $C_{3-8}$cycloalkylcarbonylamino may be substituted with one or two halogen atoms,
(v) arylcarbonylamino,
(vi) $C_{1-6}$alkoxycarbonylamino, wherein $C_{1-6}$alkoxy in the $C_{1-6}$alkoxycarbonylamino may be substituted with one substituent selected from the group consisting of aryl, and
(vii) $C_{3-8}$cycloalkoxycarbonylamino, wherein $C_{3-8}$cycloalkoxy in the $C_{3-8}$cycloalkoxycarbonylamino may be substituted with one $C_{1-6}$alkyl;

substituent group B25 represents the group consisting of

(i) $C_{1-6}$alkylcarbonyl and
(ii) $C_{1-6}$alkoxycarbonyl, wherein the $C_{1-6}$alkoxycarbonyl may be substituted with one aryl;

substituent group A31" represents the group consisting of

(i) halo-$C_{1-6}$alkyl,
(ii) halo-$C_{1-6}$alkoxy,
(iii) halo-$C_{1-6}$alkylsulfanyl,
(iv) $C_{1-6}$alkylsulfonyl, wherein the $C_{1-6}$alkylsulfonyl may be substituted with one substituent selected from substituent group B35",
(v) $C_{3-8}$cycloalkylsulfonyl, and
(vi) di$C_{1-6}$alkylaminosulfonyl;

substituent group B35" represents the group consisting of

(i) $C_{3-8}$cycloalkyl and
(ii) saturated heterocyclylcarbonyl; and

substituent group A41" is the group consisting of

(i) halo-$C_{1-6}$alkyl and
(ii) $C_{1-6}$alkylsulfonyl, wherein the $C_{1-6}$alkylsulfonyl may be substituted with one $C_{3-8}$cycloalkyl.

(3) In another embodiment, the present invention provides the compound or pharmaceutically acceptable salt thereof according to (1) or (2), wherein the structure represented by formula [III] shown below is the structure of formula [VI] shown below.

[Formula 7]

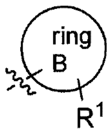
[III]

[Formula 8]

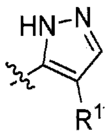
[VI]

(4) In another embodiment, the present invention provides the compound or pharmaceutically acceptable salt thereof according to (3), wherein ring A is 4- to 6-membered saturated nitrogen-containing heterocyclyl, wherein the 4- to 6-membered saturated nitrogen-containing heterocyclyl is substituted with one substituent selected from substituent group A21".
(5) In another embodiment, the present invention provides the compound or pharmaceutically acceptable salt thereof according to (3), wherein ring A is phenyl, wherein the phenyl is substituted with one substituent selected from substituent group A31" and may be further substituted with one halogen atom.
(6) In another embodiment, the present invention provides
the compound or pharmaceutically acceptable salt thereof according to (1), represented by formula [I] shown below:

[Formula 9]

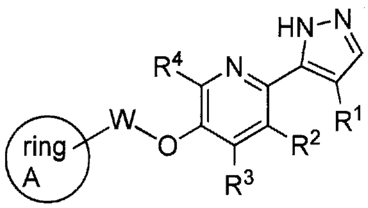
[I]

wherein

$R^1$ represents a hydrogen atom, a fluorine atom, or methyl;
$R^2$, $R^3$, and $R^4$ each independently represent a hydrogen atom, a fluorine atom, or methyl;
W represents a single bond, $C_{1-3}$alkanediyl, or the formula $-O-CH_2CH_2-$;
ring A represents

(a) $C_{4-6}$cycloalkyl, wherein the $C_{4-6}$cycloalkyl is substituted with one substituent selected from substituent group A11,
(b) 4- to 6-membered saturated nitrogen-containing heterocyclyl, wherein the 4- to 6-membered saturated nitrogen-containing heterocyclyl is substituted with one substituent selected from substituent group A21 and may be further substituted with one substituent selected from substituent group A22,

(c) phenyl, wherein the phenyl is substituted with one substituent selected from substituent group A31 and may be further substituted with one substituent selected from substituent group A32,
(d) pyridyl, wherein the pyridyl is substituted with one substituent selected from substituent group A41,
(e) naphthyl,
(f) 2,3-dihydrobenzofuran, wherein the 2,3-dihydrobenzofuran may be substituted with one to three substituents selected from substituent group A51,
(g) 2H-chromenyl, wherein the 2H-chromenyl may be substituted with one oxo,
(h) quinolyl, wherein the quinolyl may be substituted with one $C_{1-6}$alkoxy,
(j) quinoxalyl,
(k) a group represented by formula [11-1] shown below, wherein the group represented by formula [II-1] is substituted with one $C_{1-6}$alkyl, wherein the $C_{1-6}$alkyl may be substituted with one substituent selected from substituent group B61,
(m) a group represented by formula [II-2] shown below, wherein the group represented by formula [II-2] is substituted with one $C_{1-6}$alkylcarbonyl,
(n) a group represented by formula [II-3] shown below, wherein the group represented by formula [II-3] is substituted with one $C_{1-6}$alkylcarbonyl, or
(p) a group represented by formula [II-4] shown below, wherein the group represented by formula [II-4] is substituted with one $C_{1-6}$alkylcarbonyl;

[Formula 10]

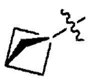 [II-1], 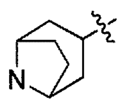 [II-2],

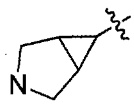 [II-3], 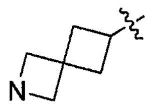 [II-4]

wherein substituent group A11 represents the group consisting of

(i) $C_{1-6}$alkylcarbonylamino and
(ii) $C_{1-6}$alkylcarbonyl($C_{1-6}$alkyl)ammo;

substituent group A21 represents the group consisting of

(i) $C_{1-6}$alkylcarbonyl, wherein the $C_{1-6}$alkylcarbonyl may be substituted with one to three substituents selected from substituent group B21,
(ii) $C_{3-8}$cycloalkylcarbonyl, wherein the $C_{3-8}$cycloalkylcarbonyl may be substituted with one or two substituents selected from substituent group B22,
(iii) arylcarbonyl, wherein the arylcarbonyl may be substituted with one substituent selected from substituent group B23,
(iv) saturated heterocyclylcarbonyl, wherein the saturated heterocyclylcarbonyl may be substituted with one or two substituents selected from substituent group B24,
(v) heteroarylcarbonyl, wherein the heteroarylcarbonyl may be substituted with one substituent selected from the group consisting of $C_{1-6}$alkyl, wherein the $C_{1-6}$alkyl may be substituted with one hydroxy,
(vi) $C_{1-6}$alkoxycarbonyl,
(vii) mono$C_{1-6}$alkylaminocarbonyl,
(viii) di$C_{1-6}$alkylaminocarbonyl,
(ix) $C_{3-8}$cycloalkylaminocarbonyl,
(x) $C_{3-8}$cycloalkyl($C_{1-6}$alkyl)aminocarbonyl,
(xi) $C_{1-6}$alkylsulfonyl, wherein the $C_{1-6}$alkylsulfonyl may be substituted with one $C_{1-6}$alkoxycarbonylamino,
(xii) $C_{3-8}$cycloalkylsulfonyl,
(xiii) saturated heterocyclylsulfonyl, wherein the saturated heterocyclylsulfonyl may be substituted with one substituent selected from substituent group B25, and
(xiv) di$C_{1-6}$alkylaminosulfonyl;

substituent group A22 represents the group consisting of

(i) a halogen atom and
(ii) $C_{1-6}$alkyl;

substituent group B21 represents the group consisting of

(i) hydroxy,
(ii) carbamoyl,
(iii) ureide,
(iv) a halogen atom,
(v) $C_{3-8}$cycloalkyl, wherein the $C_{3-8}$cycloalkyl may be substituted with one hydroxy,
(vi) saturated heterocyclyl, wherein the saturated heterocyclyl may be substituted with one or two substituents selected from the group consisting of hydroxy and oxo,
(vii) heteroaryl, wherein the heteroaryl may be substituted with one oxo,
(viii) $C_{1-6}$alkoxy,
(ix) aryloxy,
(x) saturated heterocyclylcarbonyl,
(xi) $C_{1-6}$alkylsulfonyl,
(xii) halo-$C_{1-6}$alkylsulfonyl,
(xiii) arylsulfonyl,
(xiv) $C_{1-6}$alkylcarbonylamino, wherein $C_{1-6}$alkyl in the $C_{1-6}$alkylcarbonylamino may be substituted with one substituent selected from the group consisting of hydroxy and saturated heterocyclyl,
(xv) $C_{1-6}$alkylcarbonyl($C_{1-6}$alkyl)amino,
(xvi) $C_{3-8}$cycloalkylcarbonylamino, wherein $C_{3-8}$cycloalkyl in the $C_{3-8}$cycloalkylcarbonylamino may be substituted with one or two halogen atoms,
(xvii) arylcarbonylamino,
(xviii) saturated heterocyclylcarbonylamino,
(xix) mono$C_{1-6}$alkylaminocarbonyl,
(xx) di$C_{1-6}$alkylaminocarbonyl,
(xxi) $C_{1-6}$alkoxycarbonylamino, wherein $C_{1-6}$alkoxy in the $C_{1-6}$alkoxycarbonylamino may be substituted with one substituent selected from the group consisting of $C_{1-6}$alkoxy and aryl,
(xxii) $C_{1-6}$alkoxycarbonyl($C_{1-6}$alkyl)amino,
(xxiii) $C_{3-8}$cycloalkoxycarbonylamino, wherein $C_{3-8}$cycloalkoxy in the $C_{3-8}$cycloalkoxycarbonylamino may be substituted with one $C_{1-6}$alkyl,
(xxiv) mono$C_{1-6}$alkylaminocarbonylamino, and
(xxv) di$C_{1-6}$alkylaminocarbonyl;

substituent group B22 represents the group consisting of

(i) hydroxy,
(ii) carbamoyl,
(iii) a halogen atom,
(iv) $C_{1-6}$alkyl, and
(v) $C_{1-6}$alkoxycarbonylamino;

substituent group B23 represents

(i) $C_{1-6}$alkoxy, wherein the $C_{1-6}$alkoxy may be substituted with one carbamoyl;

substituent group B24 represents the group consisting of

(i) oxo,
(ii) a halogen atom,
(iii) $C_{1-6}$alkyl,
(iv) $C_{1-6}$alkylcarbonyl, and
(v) $C_{1-6}$alkoxycarbonyl;

substituent group B25 represents the group consisting of

(i) $C_{1-6}$alkylcarbonyl and
(ii) $C_{1-6}$alkoxycarbonyl, wherein the $C_{1-6}$alkoxycarbonyl may be substituted with one aryl;

substituent group A31 represents the group consisting of

(i) amino,
(ii) $C_{1-6}$alkyl,
(iii) halo-$C_{1-6}$alkyl,
(iv) $C_{2-6}$alkenyl, wherein the $C_{2-6}$alkenyl may be substituted with one substituent selected from substituent group B32,
(v) $C_{1-6}$alkoxy,
(vi) halo-$C_{1-6}$alkoxy,
(vii) $C_{1-6}$alkylsulfanyl,
(viii) halo-$C_{1-6}$alkylsulfanyl,
(ix) saturated heterocyclylcarbonyl, wherein the saturated heterocyclylcarbonyl may be substituted with one or two $C_{1-6}$alkyl,
(x) $C_{1-6}$alkylsulfonyl, wherein the $C_{1-6}$alkylsulfonyl may be substituted with one substituent selected from substituent group B35,
(xi) $C_{3-8}$cycloalkylsulfonyl,
(xii) arylsulfonyl, wherein the arylsulfonyl may be substituted with one $C_{1-6}$alkyl,
(xiii) di$C_{1-6}$alkylaminosulfonyl, and
(xiv) $C_{1-6}$alkoxycarbonylamino;

substituent group A32 represents the group consisting of

(i) a halogen atom,
(ii) $C_{1-6}$alkyl,
(iii) halo-$C_{1-6}$alkyl, and
(iv) $C_{1-6}$alkoxy;

substituent group B32 represents

(i) aryl;

substituent group B35 represents the group consisting of

(i) $C_{3-8}$cycloalkyl,
(ii) saturated heterocyclyl, and
(iii) saturated heterocyclylcarbonyl;

substituent group A41 represents the group consisting of

(i) $C_{1-6}$alkyl,
(ii) halo-$C_{1-6}$alkyl,
(iii) triazolyl,
(iv) $C_{1-6}$alkylsulfonyl, wherein the $C_{1-6}$alkylsulfonyl may be substituted with one $C_{3-8}$cycloalkyl, and
(v) $C_{1-6}$alkylcarbonylamino;

substituent group A51 represents the group consisting of

(i) a halogen atom and
(ii) $C_{1-6}$alkyl; and

substituent group B61 represents the group consisting of

(i) $C_{1-6}$alkylcarbonylamino and

(ii) $C_{1-6}$alkylcarbonyl($C_{1-6}$alkyl)amino.

(7) In another embodiment, the present invention provides the compound or pharmaceutically acceptable salt thereof according to (1) or (6), wherein
$R^2$ is a hydrogen atom, a fluorine atom, or methyl;
$R^3$ is a hydrogen atom or methyl;
$R^4$ is a hydrogen atom;
W is $C_{1-2}$alkanediyl;
ring A is

(a) 4- to 6-membered saturated nitrogen-containing heterocyclyl, wherein the 4- to 6-membered saturated nitrogen-containing heterocyclyl is substituted with one substituent selected from substituent group A21",
(b) phenyl, wherein the phenyl is substituted with one substituent selected from substituent group A31" and may be further substituted with one halogen atom,
(c) pyridyl, wherein the pyridyl is substituted with one substituent selected from substituent group A41", or
(d) 2,3-dihydrobenzofuran, wherein the 2,3-dihydrobenzofuran is substituted with one halogen atom and two $C_{1-6}$alkyl;

wherein substituent group A21" represents the group consisting of

(i) $C_{1-6}$alkylcarbonyl, wherein the $C_{1-6}$alkylcarbonyl may be substituted with one to three substituents selected from substituent group B21",
(ii) $C_{3-8}$cycloalkylcarbonyl, wherein the $C_{3-8}$cycloalkylcarbonyl may be substituted with one $C_{1-6}$alkoxycarbonylamino,
(iii) $C_{1-6}$alkoxycarbonyl,
(iv) mono$C_{1-6}$alkylaminocarbonyl,
(v) di$C_{1-6}$alkylaminocarbonyl,
(vi) $C_{1-6}$alkylsulfonyl, wherein the $C_{1-6}$alkylsulfonyl may be substituted with one $C_{1-6}$alkoxycarbonylamino,
(vii) $C_{3-8}$cycloalkylsulfonyl,
(viii) saturated heterocyclylsulfonyl, wherein the saturated heterocyclylsulfonyl may be substituted with one substituent selected from substituent group B25, and
(ix) di$C_{1-6}$alkylaminosulfonyl;

substituent group B21" represents the group consisting of

(i) a halogen atom,
(ii) $C_{3-8}$cycloalkyl, wherein the $C_{3-8}$cycloalkyl may be substituted with one hydroxy,
(iii) aryloxy,
(iv) $C_{3-8}$cycloalkylcarbonylamino, wherein $C_{3-8}$cycloalkyl in the $C_{3-8}$cycloalkylcarbonylamino may be substituted with one or two halogen atoms,
(v) arylcarbonylamino,
(vi) $C_{1-6}$alkoxycarbonylamino, wherein $C_{1-6}$alkoxy in the $C_{1-6}$alkoxycarbonylamino may be substituted with one substituent selected from the group consisting of aryl, and
(vii) $C_{3-8}$cycloalkoxycarbonylamino, wherein $C_{3-8}$cycloalkoxy in the $C_{3-8}$cycloalkoxycarbonylamino may be substituted with one $C_{1-6}$alkyl;

substituent group B25 represents the group consisting of

(i) $C_{1-6}$alkylcarbonyl and
(ii) $C_{1-6}$alkoxycarbonyl, wherein the $C_{1-6}$alkoxycarbonyl may be substituted with one aryl;

substituent group A31" represents the group consisting of

(i) halo-$C_{1-6}$alkyl,
(ii) halo-$C_{1-6}$alkoxy,
(iii) halo-$C_{1-6}$alkylsulfanyl,
(iv) $C_{1-6}$alkylsulfonyl, wherein the $C_{1-6}$alkylsulfonyl may be substituted with one substituent selected from substituent group B35",

(v) $C_{3-8}$cycloalkylsulfonyl, and
(vi) di$C_{1-6}$alkylaminosulfonyl;

substituent group B35" represents the group consisting of

(i) $C_{3-8}$cycloalkyl and
(ii) saturated heterocyclylcarbonyl; and

substituent group A41" represents the group consisting of

(i) halo-$C_{1-6}$alkyl and
(ii) $C_{1-6}$alkylsulfonyl, wherein the $C_{1-6}$alkylsulfonyl may be substituted with one $C_{3-8}$cycloalkyl.

(8) In another embodiment, the present invention provides the compound or pharmaceutically acceptable salt thereof according to (1), (6), or (7), wherein W is $C_{1-2}$alkanediyl.
(9) In another embodiment, the present invention provides the compound or pharmaceutically acceptable salt thereof according to any of (1) and (6) to (8), wherein ring A is 4- to 6-membered saturated nitrogen-containing heterocyclyl, wherein the 4- to 6-membered saturated nitrogen-containing heterocyclyl is substituted with one substituent selected from substituent group A21";
wherein substituent group A21" represents the group consisting of

(i) $C_{1-6}$alkylcarbonyl, wherein the $C_{1-6}$alkylcarbonyl may be substituted with one to three substituents selected from substituent group B21",
(ii) $C_{3-8}$cycloalkylcarbonyl, wherein the $C_{3-8}$cycloalkylcarbonyl may be substituted with one $C_{1-6}$alkoxycarbonylamino,
(iii) $C_{1-6}$alkoxycarbonyl,
(iv) mono$C_{1-6}$alkylaminocarbonyl,
(v) di$C_{1-6}$alkylaminocarbonyl,
(vi) $C_{1-6}$alkylsulfonyl, wherein the $C_{1-6}$alkylsulfonyl may be substituted with one $C_{1-6}$alkoxycarbonylamino,
(vii) $C_{3-8}$cycloalkylsulfonyl,
(viii) saturated heterocyclylsulfonyl, wherein the saturated heterocyclylsulfonyl may be substituted with one substituent selected from substituent group B25, and
(ix) di$C_{1-6}$alkylaminosulfonyl;

substituent group B21" represents the group consisting of

(i) a halogen atom,
(ii) $C_{3-8}$cycloalkyl, wherein the $C_{3-8}$cycloalkyl may be substituted with one hydroxy,
(iii) aryloxy,
(iv) $C_{3-8}$cycloalkylcarbonylamino, wherein C3-scycloalkyl in the $C_{3-8}$cycloalkylcarbonylamino may be substituted with one or two halogen atoms,
(v) arylcarbonylamino,
(vi) $C_{1-6}$alkoxycarbonylamino, wherein $C_{1-6}$alkoxy in the $C_{1-6}$alkoxycarbonylamino may be substituted with one substituent selected from the group consisting of aryl, and
(vii) $C_{3-8}$cycloalkoxycarbonylamino, wherein $C_{3-8}$cycloalkoxy in the $C_{3-8}$cycloalkoxycarbonylamino may be substituted with one $C_{1-6}$alkyl; and

substituent group B25 represents the group consisting of

(i) $C_{1-6}$alkylcarbonyl and
(ii) $C_{1-6}$alkoxycarbonyl, wherein the $C_{1-6}$alkoxycarbonyl may be substituted with one aryl.

(10) In another embodiment, the present invention provides the compound or pharmaceutically acceptable salt thereof according to any of (1) and (6) to (9), wherein ring A is piperidin-4-yl, wherein the piperidin-4-yl is substituted with one substituent selected from the group consisting of $C_{1-6}$alkylcarbonyl and $C_{1-6}$alkoxycarbonyl.
(11) In another embodiment, the present invention provides the compound or pharmaceutically acceptable salt thereof according to any of (1) and (6) to (8), wherein ring A is phenyl, wherein the phenyl is substituted with one substituent selected from substituent group A31" and may be further substituted with one halogen atom;

wherein substituent group A31" represents the group consisting of

(i) halo-$C_{1-6}$alkyl,
(ii) halo-$C_{1-6}$alkoxy,
(iii) halo-$C_{1-6}$alkylsulfanyl,
(iv) $C_{1-6}$alkylsulfonyl, wherein the $C_{1-6}$alkylsulfonyl may be substituted with one substituent selected from substituent group B35",
(v) $C_{3-8}$cycloalkylsulfonyl, and
(vi) di$C_{1-6}$alkylaminosulfonyl; and

substituent group B35" represents the group consisting of

(i) $C_{3-8}$cycloalkyl and
(ii) saturated heterocyclylcarbonyl.

(12) In another embodiment, the present invention provides the compound or pharmaceutically acceptable salt thereof according to any of (1) and (6) to (8),
wherein
$R^1$ is a hydrogen atom or methyl;
$R^2$ is a hydrogen atom or a fluorine atom;
$R^3$ is a hydrogen atom;
$R^4$ is a hydrogen atom;
W is $C_{1-2}$alkanediyl; and
ring A is

(a) piperidin-4-yl substituted with one substituent selected from the group consisting of $C_{1-6}$alkylcarbonyl and $C_{1-6}$alkoxycarbonyl, or
(b) phenyl substituted with one substituent selected from the group consisting of $C_{1-6}$alkylsulfonyl and $C_{3-8}$cycloalkylsulfonyl.

(13) In another embodiment, the present invention provides the compound or pharmaceutically acceptable salt thereof according to any of (1), (6) to (8), and (12), wherein
$R^1$ is a hydrogen atom or methyl;
$R^2$ is a hydrogen atom or a fluorine atom; wherein one of $R^1$ and $R^2$ is a hydrogen atom;
$R^3$ is a hydrogen atom;
$R^4$ is a hydrogen atom;
W is methanediyl or ethane-1,2-diyl; and
ring A is

(a) piperidin-4-yl substituted at the 1-position with one substituent selected from the group consisting of acetyl and methoxycarbonyl or
(b) phenyl substituted at the 3-position with a substituent selected from the group consisting of methylsulfonyl and cyclopropylsulfonyl.

(14) In another embodiment, the present invention provides the compound or pharmaceutically acceptable salt thereof according to any of (1), (4), (5), (6), and (13), which is shown below:

[Formula 11]

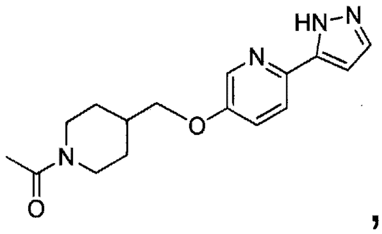

,

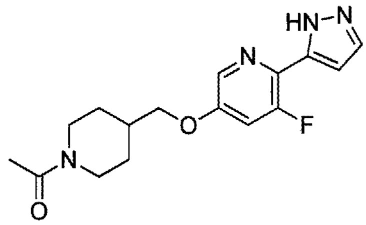

,

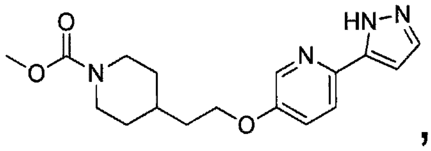

,

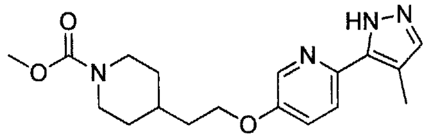

,

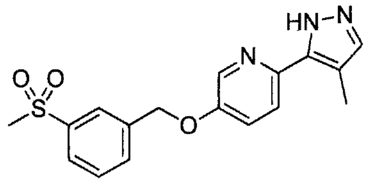

,

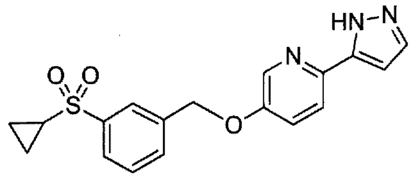

.

(15) In another embodiment, the present invention provides the compound or pharmaceutically acceptable salt thereof according to any of (1), (4), (5), (6), (13), and (14), which is shown below:

[Formula 12]

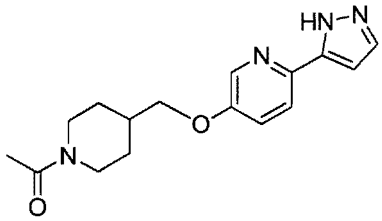

.

(16) In another embodiment, the present invention provides the compound or pharmaceutically acceptable salt thereof according to any of (1), (4), (5), (6), (13), and (14), which is shown below:

[Formula 13]

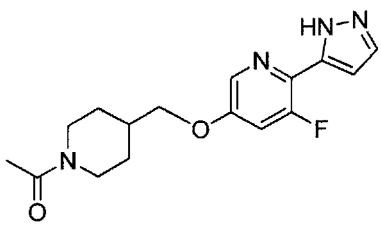

.

(17) In another embodiment, the present invention provides the compound or pharmaceutically acceptable salt thereof according to any of (1), (4), (5), (6), (13), and (14) which is shown below:

[Formula 14]

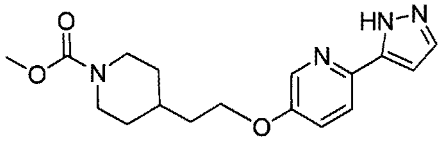

.

(18) In another embodiment, the present invention provides the compound or pharmaceutically acceptable salt thereof according to any of (1), (4), (5), (6), (13), and (14) which is shown below:

[Formula 15]

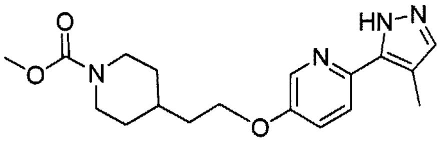

.

(19) In another embodiment, the present invention provides the compound or pharmaceutically acceptable salt thereof according to any of (1), (4), (5), (6), (13), and (14) which is shown below:

[Formula 16]

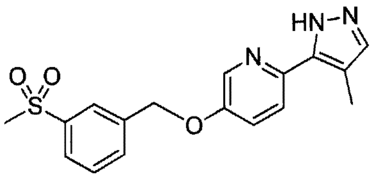

.

(20) In another embodiment, the present invention provides the compound or pharmaceutically acceptable salt thereof according to any of (1), (4), (5), (6), (13), and (14) which is shown below:

[Formula 17]

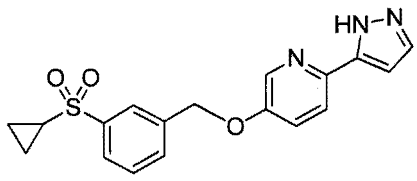

.

(21) In another embodiment, the present invention provides a pharmaceutical comprising the compound or pharmaceutically acceptable salt thereof according to any of (1) to (20) as an active ingredient.

(22) In another embodiment, the present invention provides an agent that inhibits 20-HETE producing enzyme, wherein the agent comprises the compound or pharmaceutically acceptable salt thereof according to any of (1) to (20) as an active ingredient.

(23) In another embodiment, the present invention provides an agent that prevents or ameliorates polycystic kidney disease, wherein the agent comprises the compound or pharmaceutically acceptable salt thereof according to any of (1) to (20) as an active ingredient.

ADVANTAGEOUS EFFECTS OF INVENTION

The compound of the present invention (hereinafter also referred to as "the inventive compound") has an inhibitory effect on 20-HETE producing enzymes.

DESCRIPTION OF EMBODIMENTS

The present invention provides a compound represented by formula [I] shown above that has an inhibitory effect on 20-HETE producing enzymes or a pharmaceutically acceptable salt thereof.

The compounds of the present invention will be described in more detail below, but the present invention is not limited to the exemplary embodiments.

The term "halogen atom" refers to a fluorine atom, a chlorine atom, a bromine atom, or an iodine atom.

The term "$C_{1-6}$alkyl" refers to a straight or branched alkyl group having one to six carbon atoms. Examples of $C_{1-6}$alkyl include methyl, ethyl, n-propyl, isopropyl, n-butyl, isobutyl, sec-butyl, tert-butyl, n-pentyl, isopentyl, neopentyl, 2-methylbutyl, n-hexyl, isohexyl, and the like.

The term "halo-$C_{1-6}$alkyl" refers to a straight or branched alkyl group that is substituted with a halogen atom and has one to six carbon atoms. The halo-$C_{1-6}$alkyl is preferably substituted with one to five halogen atoms, and the halogen atom is preferably a fluorine atom. Examples of halo-$C_{1-6}$alkyl include monofluoromethyl, difluoromethyl, trifluoromethyl, 1-fluoroethyl, 1,1-difluoroethyl, 1,1,2,2,2-pentafluoroethyl, 2-fluoroethyl, 2-fluoro-2-methylpropyl, 2,2-difluoropropyl, 1-fluoro-2-methylpropan-2-yl, 1,1-difluoro-2-methylpropan-2-yl, 1-fluoropentyl, 1-fluorohexyl, 2,2,2-trifluoro-1-methylethyl, and the like.

The term "$C_2$-6alkenyl" refers to a straight or branched alkenyl group having two to six carbon atoms. Examples of $C_{2-6}$alkenyl include ethenyl, (E)-prop-1-en-1-yl, (Z)-prop-1-en-1-yl, prop-2-en-1-yl, (Z)-but-2-en-1-yl, (Z)-pent-3-en-1-yl, (Z)-hex-4-en-1-yl, (Z)-hept-5-en-1-yl, (Z)-oct-6-en-1-yl, and the like.

The term "$C_{3-8}$cycloalkyl" refers to a cyclic alkyl group having three to eight carbon atoms. Examples of $C_{3-8}$cycloalkyl include cyclopropyl, cyclobutyl, cyclopentyl, cyclohexyl, cycloheptyl, and cyclooctyl.

The term "$C_{4-6}$cycloalkyl" refers to a cyclic alkyl group having four to six carbon atoms. Examples of $C_{4-6}$cycloalkyl include cyclobutyl, cyclopentyl, and cyclohexyl.

The term "hydroxy$C_{1-6}$alkyl" refers to the above-mentioned $C_{1-6}$alkyl that is substituted with hydroxy. Examples of hydroxy$C_{1-6}$alkyl include hydroxymethyl, 2-hydroxyethyl, 1-hydroxyethyl, and 3-hydroxypropyl.

The term "aryl" refers to a monocyclic or fused polycyclic aromatic hydrocarbon group having 6 to 14 carbon atoms. Examples of aryl include phenyl, naphthyl, anthryl, and the like.

Also, partially-saturated aryl groups are included in "aryl". The term "partially-saturated aryl group" refers to a partially-saturated fused polycyclic heterocyclic group among the monocyclic or fused polycyclic aromatic hydrocarbon group having 6 to 14 carbon atoms. Examples of partially-saturated aryl groups include dihydroindenyl and the like.

The term "saturated heterocyclyl" refers to a 3- to 8-membered monocyclic saturated heterocyclic group consisting of one to seven carbon atoms and one or more atoms which may be the same or different and are selected from the group consisting of an oxygen atom, a sulfur atom, and a nitrogen atom. Examples of saturated heterocyclyl include oxetanyl, tetrahydrofuranyl, tetrahydropyranyl, oxepanyl, azetidinyl, pyrrolidinyl, piperidinyl, azepanyl, tetrahydrothiopyranyl, piperazinyl, pyrazolidinyl, morpholinyl, piperazinyl, thiomorpholinyl, 1,3-oxazinanyl, isothiazolidinyl, and the like.

The term "4- to 6-membered saturated oxygen-containing heterocyclyl" refers to the above-mentioned "saturated heterocyclyl" that is 4- to 6-membered and contains one oxygen atom in the ring. Examples of 4- to 6-membered saturated oxygen-containing heterocyclyl include oxetanyl, tetrahydrofuranyl, tetrahydropyranyl, and the like.

The term "4- to 6-membered saturated sulfur-containing heterocyclyl" refers to the above-mentioned "saturated heterocyclyl" that is 4- to 6-membered and contains one sulfur atom in the ring. Examples of 4- to 6-membered saturated sulfur-containing heterocyclyl include thietanyl, tetrahydrothiophenyl, tetrahydrothiopyranyl, and the like.

The term "4- to 6-membered saturated nitrogen-containing heterocyclyl" refers to the above-mentioned "saturated heterocyclyl" that is 4- to 6-membered, contains one nitrogen atom in the ring, and may further contain one heteroatom selected from the group consisting of a nitrogen atom, an oxygen atom, and a sulfur atom. Examples of 4- to 6-membered saturated nitrogen-containing heterocyclyl include azetidinyl, pyrrolidinyl, piperidinyl, piperazinyl, morpholinyl, thiomorpholinyl, and the like.

The term "heteroaryl" refers to a 5- to 7-membered monocyclic aromatic heterocyclic group consisting of one to six carbon atoms and one or more atoms which may be the same or different and are selected from the group consisting of an oxygen atom, a sulfur atom, and a nitrogen atom or a fused polycyclic aromatic heterocyclic group that is composed of 9 to 14 atoms consisting of 1 to 13 carbon atoms and one or more atoms which may be the same or different and are selected from the group consisting of an oxygen atom, a sulfur atom, and a nitrogen atom. Examples of heteroaryl include imidazolyl, pyrazolyl, thiazolyl, isothiazolyl, thiadiazolyl, oxazolyl, isooxazolyl, oxadiazolyl, pyrrolyl, triazolyl, tetrazolyl, pyridyl, pyrimidinyl, pyrazinyl, pyridazinyl, triazinyl, indolyl, benzopyrazolyl, benzotriazolyl, benzofuranyl, benzothiophenyl, quinolyl, isoquinolyl, quinoxalyl, and the like.

Also, partially-saturated heteroaryl groups are included in "heteroaryl". The term "partially-saturated heteroaryl group" refers to a 5- to 7-membered partially-saturated monocyclic heterocyclic group consisting of one to six carbon atoms and one or more atoms which may be the same or different and are selected from the group consisting of an oxygen atom, a sulfur atom, and a nitrogen atom or a partially-saturated fused polycyclic heterocyclic group that is

composed of 9 to 14 atoms consisting of 1 to 13 carbon atoms and one or more atoms which may be the same or different and are selected from the group consisting of an oxygen atom, a sulfur atom, and a nitrogen atom. Examples of partially-saturated heteroaryl groups include oxazolidinyl, thiazolinyl, dihydropyridinyl, dihydrobenzofuranyl, chromanyl, dihydropyranopyridinyl, dihydrofuropyridinyl, tetrahydroquinolyl, tetrahydroquinolyl, dihydrobenzodioxinyl, tetrahydrotriazoloazepinyl, and the like.

The term "$C_{1-6}$alkoxy" refers to a straight or branched alkoxy group having one to six carbon atoms. Examples of $C_{1-6}$alkoxy include methoxy, ethoxy, n-propoxy, isopropoxy, n-butoxy, isobutoxy, sec-butoxy, tert-butoxy, n-pentyloxy, isopentyloxy, neopentyloxy, 2-methylbutoxy, n-hexyloxy, isohexyloxy, and the like.

The term "halo-$C_{1-6}$alkoxy" refers to a straight or branched alkoxy group that is substituted with a halogen atom and has one to six carbon atoms. The halo-$C_{1-6}$alkoxy is preferably substituted with one to five halogen atoms, and the halogen atom is preferably a fluorine atom. Examples of halo-$C_{1-6}$alkoxy include monofluoromethoxy, difluoromethoxy, trifluoromethoxy, 1-fluoroethoxy, 1,1-difluoroethoxy, 1,1,2,2-tetrafluoroethoxy, 2-fluoroethoxy, 2,2,2-trifluoroethoxy, 3,3,3-trifluoropropoxy, 1,3-difluoropropan-2-yloxy, 2-fluoro-2-methylpropoxy, 2,2-difluoropropoxy, 1-fluoro-2-methylpropan-2-yloxy, 1,1-difluoro-2-methylpropan-2-yloxy, 4,4,4-trifluorobutoxy, and the like.

The term "$C_{3-8}$cycloalkoxy" refers to a cyclic alkoxy group having three to eight carbon atoms. The $C_{3-8}$cycloalkoxy includes cyclopropoxy, cyclobutoxy, cyclopentyloxy, cyclohexyloxy, cycloheptyloxy, and cyclooctyloxy.

The term "aryloxy" refers to a group consisting of the above-mentioned "aryl" which is bound to an oxygen atom. Examples of aryloxy include phenoxy, naphthyloxy, and the like.

The term "mono$C_{1-6}$alkylamino" refers to an amino group having, as a substituent, one "$C_{1-6}$alkyl" group mentioned above. Examples of mono$C_{1-6}$alkylamino include methylamino, ethylamino, n-propylamino, isopropylamino, n-butylamino, isobutylamino, sec-butylamino, tert-butylamino, n-pentylamino, isopentylamino, neopentylamino, 2-methylbutylamino, n-hexylamino, isohexylamino, and the like.

The term "di$C_{1-6}$alkylamino" refers to an amino group having, as substituents, two "$C_{1-6}$alkyl" groups mentioned above, wherein the $C_{1-6}$alkyl groups may be the same or different. Examples of di$C_{1-6}$alkylamino include dimethylamino, diethylamino, di(n-propyl)amino, di(isopropyl)amino, ethylmethylamino, methyl(n-propyl)amino, and the like.

The term "$C_{1-6}$alkylsulfanyl" refers to a group consisting of the above-mentioned "$C_{1-6}$alkyl" which is bound to a sulfur atom. Examples of $C_{1-6}$alkylsulfanyl include methylsulfanyl, ethylsulfanyl, n-propylsulfanyl, isopropylsulfanyl, n-butylsulfanyl, isobutylsulfanyl, sec-butylsulfanyl, tert-butylsulfanyl, n-pentylsulfanyl, isopentylsulfanyl, neopentylsulfanyl, 2-methylbutylsulfanyl, n-hexylsulfanyl, isohexylsulfanyl, and the like.

The term "halo-$C_{1-6}$alkylsulfanyl" refers to a group consisting of the above-mentioned "halo-$C_{1-6}$alkyl" which is bound to a sulfur atom. Examples of halo-$C_{1-6}$alkylsulfanyl include monofluoromethylsulfanyl, difluoromethylsulfanyl, trifluoromethylsulfanyl, 1-fluoroethylsulfanyl, 1,1-difluoroethylsulfanyl, 1,1,2,2,2-pentafluoroethylsulfanyl, 2-fluoroethylsulfanyl, 2-fluoro-2-methylpropylsulfanyl, 2,2-difluoropropylsulfanyl, 1 -fluoro-2-methylpropan-2-ylsulfanyl, 1,1-difluoro-2-methylpropan-2-ylsulfanyl, 1-fluoropentylsulfanyl, 1-fluorohexylsulfanyl, and the like.

The term "$C_{1-6}$alkylcarbonyl" refers to a group consisting of the above-mentioned "$C_{1-6}$alkyl" which is bound to carbonyl. Examples of $C_{1-6}$alkylcarbonyl include acetyl, ethylcarbonyl, n-propylcarbonyl, isopropylcarbonyl, n-butylcarbonyl, isobutylcarbonyl, sec-butylcarbonyl, tert-butylcarbonyl, n-pentylcarbonyl, isopentylcarbonyl, neopentylcarbonyl, 2-methylbutylcarbonyl, n-hexylcarbonyl, isohexylcarbonyl, and the like.

The term "$C_{3-8}$cycloalkylcarbonyl" refers to a group consisting of the above-mentioned "$C_{3-8}$cycloalkyl" which is bound to carbonyl. The $C_{3-8}$cycloalkylcarbonyl includes cyclopropylcarbonyl, cyclobutylcarbonyl, cyclopentylcarbonyl, cyclohexylcarbonyl, cycloheptylcarbonyl, and cyclooctylcarbonyl.

The term "arylcarbonyl" refers to a group consisting of the above-mentioned "aryl" which is bound to carbonyl. Examples of arylcarbonyl include benzoyl, naphthylcarbonyl, and the like.

The term "saturated heterocyclylcarbonyl" refers to a group consisting of the above-mentioned "saturated heterocyclyl" which is bound to carbonyl. Examples of saturated heterocyclylcarbonyl include oxetanylcarbonyl, tetrahydrofuranylcarbonyl, tetrahydropyranylcarbonyl, oxepanylcarbonyl, azetidinylcarbonyl, pyrrolidinylcarbonyl, piperidinylcarbonyl, azepanylcarbonyl, tetrahydrothiopyranylcarbonyl, morpholinylcarbonyl, piperazinylcarbonyl, thiomorpholinylcarbonyl, isothiazolidinylcarbonyl, and the like.

The term "heteroarylcarbonyl" refers to a group consisting of the above-mentioned "heteroaryl" which is bound to carbonyl. Examples of heteroarylcarbonyl include furanylcarbonyl, pyrazolylcarbonyl, thiophenylcarbonyl, pyridinylcarbonyl, pyridazinylcarbonyl, pyrimidinylcarbonyl, pyrazinylcarbonyl, and the like.

The term "$C_{1-6}$alkylsulfonyl" refers to a group consisting of the above-mentioned "$C_{1-6}$alkyl" which is bound to sulfonyl. Examples of $C_{1-6}$alkylsulfonyl include methylsulfonyl, ethylsulfonyl, n-propylsulfonyl, isopropylsulfonyl, n-butylsulfonyl, isobutylsulfonyl, sec-butylsulfonyl, tert-butylsulfonyl, n-pentylsulfonyl, isopentylsulfonyl, neopentylsulfonyl, 2-methylbutylsulfonyl, n-hexylsulfonyl, isohexylsulfonyl, and the like.

The term "halo-$C_{1-6}$alkylsulfbnyl" refers to a group consisting of the above-mentioned "halo-$C_{1-6}$alkyl" which is bound to sulfonyl. Examples of halo-$C_{1-6}$alkylsulfonyl include monofluoromethylsulfonyl, difluoromethylsulfonyl, trifluoromethylsulfonyl, 1-fluoroethylsulfonyl, 1,1-difluoroethylsulfonyl, 1,1,2,2,2-pentafluoroethylsulfonyl, 2-fluoroethylsulfonyl,

2-fluoro-2-methylpropylsulfonyl, 2,2-difluoropropylsulfonyl, 1-fluoro-2-methylpropan-2-ylsulfonyl, 1,1-difluoro-2-methylpropan-2-ylsulfonyl, 1-fluoropentylsulfonyl, 1-fluorohexylsulfonyl, and the like.

The term "$C_{3-8}$cycloalkylsulfonyl" refers to a group consisting of the above-mentioned "$C_{3-8}$cycloalkyl" which is bound to sulfonyl. Examples of $C_{3-8}$cycloalkylsulfonyl include cyclopropylsulfonyl, cyclobutylsulfonyl, cyclopentylsulfonyl, cyclohexylsulfonyl, cycloheptylsulfonyl, and cyclooctylsulfonyl.

The term "arylsulfonyl" refers to a group consisting of the above-mentioned "aryl" which is bound to sulfonyl. Examples of arylsulfonyl include phenylsulfonyl, naphthylsulfonyl, and the like.

The term "saturated heterocyclylsulfonyl" refers to a group consisting of the above-mentioned "saturated heterocyclyl" which is bound to sulfonyl. Examples of saturated heterocyclylsulfonyl include azetidinylsulfonyl, pyrrolidinylsulfonyl, piperidinylsulfonyl, morpholinylsulfonyl, and the like.

The term "$C_{1-6}$alkylcarbonylamino" refers to an amino group having, as a substituent, one "$C_{1-6}$alkylcarbonyl" mentioned above. Examples of $C_{1-6}$alkylcarbonylamino include acetylamino, ethylcarbonylamino, n-propylcarbonylamino, isopropylcarbonylamino, n-butylcarbonylamino, isobutylcarbonylamino, tert-butylcarbonylamino, n-pentylcarbonylamino, n-hexylcarbonylamino, and the like.

The term "$C_{1-6}$alkylcarbonyl($C_{1-6}$alkyl)amino" refers to an amino group having, as substituents, one "$C_{1-6}$alkylcarbonyl" mentioned above and one "$C_{1-6}$alkyl" mentioned above. Examples of $C_{1-6}$alkylcarbonyl($C_{1-6}$alkyl)amino include acetyl(methyl)amino, acetyl(ethyl)amino, ethylcarbonyl(methyl)amino, n-propylcarbonyl(methyl)amino, isopropylcarbonyl(methyl)amino, n-butylcarbonyl(methyl)amino, isobutylcarbonyl(methyl)amino, tert-butylcarbonyl(methyl)amino, n-pentylcarbonyl(methyl)amino, n-hexylcarbonyl(methyl)amino, and the like.

The term "$C_{3-8}$cycloalkylcarbonylamino" refers to an amino group having, as a substituent, one "$C_{3-8}$cycloalkylcarbonyl" mentioned above. The $C_{3-8}$cycloalkylcarbonylamino includes cyclopropylcarbonylamino, cyclobutylcarbonylamino, cyclopentylcarbonylamino, cyclohexylcarbonylamino, cycloheptylcarbonylamino, and cyclooctylcarbonylamino.

The term "arylcarbonylamino" refers to an amino group having, as a substituent, one "arylcarbonyl" mentioned above. Examples of arylcarbonylamino include phenylcarbonylamino, naphthylcarbonylamino, and the like.

The term "saturated heterocyclylcarbonylamino" refers to an amino group having, as a substituent, one "saturated heterocyclylcarbonyl" mentioned above. Examples of saturated heterocyclylcarbonylamino include oxetanylcarbonylamino, tetrahydrofuranylcarbonylamino, tetrahydropyranylcarbonylamino, oxepanylcarbonylamino, azetidinylcarbonylamino, pyrrolidinylcarbonylamino, piperidinylcarbonylamino, azepanylcarbonylamino, tetrahydrothiopyranylcarbonylamino, morpholinylcarbonylamino, piperazinylcarbonylamino, thiomorpholinylcarbonylamino, and the like.

The term "$C_{1-6}$alkoxycarbonyl" refers to a group consisting of the above-mentioned "$C_{1-6}$alkoxy" which is bound to carbonyl. Examples of $C_{1-6}$alkoxycarbonyl include methoxycarbonyl, ethoxycarbonyl, n-propoxycarbonyl, isopropoxycarbonyl, n-butoxycarbonyl, isobutoxycarbonyl, n-pentyloxycarbonyl, n-hexyloxycarbonyl, and the like.

The term "mono$C_{1-6}$alkylaminocarbonyl" refers to a group consisting of the above-mentioned "mono$C_{1-6}$alkylamino" which is bound to carbonyl. Examples of mono$C_{1-6}$alkylaminocarbonyl include methylaminocarbonyl, ethylaminocarbonyl, n-propylaminocarbonyl, isopropylaminocarbonyl, n-butylaminocarbonyl, isobutylaminocarbonyl, n-pentylaminocarbonyl, n-hexylaminocarbonyl, and the like.

The term "di$C_{1-6}$alkylaminocarbonyl" refers to a group consisting of the above-mentioned "di$C_{1-6}$alkylamino" which is bound to carbonyl. Examples of di$C_{1-6}$alkylaminocarbonyl include dimethylaminocarbonyl, diethylaminocarbonyl, di(n-propyl)aminocarbonyl, di(isopropyl)aminocarbonyl, ethylmethylaminocarbonyl, methyl(n-propyl)aminocarbonyl, and the like.

The term "$C_{3-8}$cycloalkylaminocarbonyl" refers to a group consisting of an amino group that has, as a substituent, one "$C_{3-8}$cycloalkyl" mentioned above and which is bound to carbonyl. The C3-scycloalkylaminocarbonyl includes cyclopropylaminocarbonyl, cyclobutylaminocarbonyl, cyclopentylaminocarbonyl, cyclohexylaminocarbonyl, cycloheptylaminocarbonyl, and cyclooctylaminocarbonyl.

The term "$C_{3-8}$cycloalkyl($C_{1-6}$alkyl)aminocarbonyl" refers to a group consisting of an amino group that has, as substituents, one "$C_{1-6}$alkyl" and one "$C_{3-8}$cycloalkyl" mentioned above and which is bound to carbonyl. The $C_{3-8}$cycloalkyl($C_{1-6}$alkyl)aminocarbonyl includes cyclopropyl(methyl)aminocarbonyl, cyclopropyl(ethyl)aminocarbonyl, cyclobutyl(methyl)aminocarbonyl, cyclopentyl(methyl)aminocarbonyl, cyclohexyl(methyl)aminocarbonyl, cycloheptyl(methyl)aminocarbonyl, and cyclooctyl(methyl)aminocarbonyl.

The term "saturated heterocyclylaminocarbonyl" refers to a group consisting of an amino group that has, as a substituent, one "saturated heterocyclyl" mentioned above and which is bound to carbonyl. Examples of saturated heterocyclylaminocarbonyl include oxetanylaminocarbonyl, tetrahydrofuranylaminocarbonyl, tetrahydropyranylaminocarbonyl, oxepanylaminocarbonyl, azetidinylaminocarbonyl, pyrrolidinylaminocarbonyl, piperidinylaminocarbonyl, azepanylaminocarbonyl, tetrahydrothiopyranylaminocarbonyl, morpholinylaminocarbonyl, piperazinylaminocarbonyl, thiomorpholinylaminocarbonyl, and the like.

The term "di$C_{1-6}$alkylaminosulfonyl" refers to a group consisting of an amino group that has, as substituents, two "$C_{1-6}$alkyl" groups mentioned above and which is bound to sulfonyl, wherein the $C_{1-6}$alkyl groups may be the same

or different. Examples of di$C_{1-6}$alkylaminosulfonyl include dimethylaminosulfonyl, diethylaminosulfonyl, di(n-propyl)aminosulfonyl, di(isopropyl)aminosulfonyl, ethylmethylaminosulfonyl, methyl(n-propyl)aminosulfonyl, and the like.

The term "$C_{1-6}$alkoxycarbonylamino" refers to an amino group having, as a substituent, one "$C_{1-6}$alkoxycarbonyl" mentioned above. Examples of $C_{1-6}$alkoxycarbonylamino include methoxycarbonylamino, ethoxycarbonylamino, n-propoxycarbonylamino, isopropoxycarbonylamino, n-butoxycarbonylamino, isobutoxycarbonylamino, tert-butoxycarbonylamino, n-pentyloxycarbonylamino, n-hexyloxycarbonylamino, and the like.

The term "$C_{1-6}$alkoxycarbonyl($C_{1-6}$alkyl)amino" refers to an amino group having, as substituents, one "$C_{1-6}$alkoxycarbonyl" mentioned above and one "$C_{1-6}$alkyl" group mentioned above. Examples of $C_{1-6}$alkoxycarbonyl($C_{1-6}$alkyl)amino include methoxycarbonyl(methyl)amino, methoxycarbonyl(ethyl)amino, ethoxycarbonyl(methyl)amino, n-propoxycarbonyl(methyl)amino, isopropoxycarbonyl(methyl)amino, n-butoxycarbonyl(methyl)amino, isobutoxycarbonyl(methyl)amino, tert-butoxycarbonyl(methyl)amino, n-pentyloxycarbonyl(methyl)amino, n-hexyloxycarbonyl(methyl)amino, and the like.

The term "$C_{3-8}$cycloalkoxycarbonylamino" refers to an amino group having, as a substituent, a group consisting of the above-mentioned "$C_{3-8}$cycloalkoxy" which is bound to carbonyl. The $C_{3-8}$cycloalkoxycarbonylamino includes cyclopropoxycarbonylamino, cyclobutoxycarbonylamino, cyclopentyloxycarbonylamino, cyclohexyloxycarbonylamino, cycloheptyloxycarbonylamino, and cyclooctyloxycarbonylamino.

The term "mono$C_{1-6}$alkylaminocarbonylamino" refers to a group consisting of the above-mentioned "mono$C_{1-6}$alkylamino", carbonyl, and amino which are bound together. Examples of mono$C_{1-6}$alkylaminocarbonylamino include methylaminocarbonylamino, ethylaminocarbonylamino, n-propylaminocarbonylamino, isopropylaminocarbonylamino, n-butylaminocarbonylamino, isobutylaminocarbonylamino, tert-butylaminocarbonylamino, n-pentylaminocarbonylamino, n-hexylaminocarbonylamino, and the like.

The term "di$C_{1-6}$alkylaminocarbonylamino" refers to a group consisting of the above-mentioned "di$C_{1-6}$alkylamino", carbonyl, and amino which are bound together. Examples of di$C_{1-6}$alkylaminocarbonylamino include dimethylaminocarbonylamino, diethylaminocarbonylamino, di(n-propyl)aminocarbonylamino, di(isopropyl)aminocarbonylamino, ethylmethylaminocarbonylamino, methyl(n-propyl)aminocarbonylamino, and the like.

The term "oxo" refers to a substituent (=O) which involves substitution of the oxygen atom via a double bond. Accordingly, when an oxo group is substituted by a carbon atom, the oxo group and the carbon atom taken together form carbonyl. When one oxo group is substituted by one sulfur atom, the oxo group and the sulfur atom taken together form sulfinyl. When two oxo groups are substituted by one sulfur atom, the oxo groups and the sulfur atom taken together form sulfonyl. When oxo is substituted with saturated heterocyclyl in the present invention, oxo-substituting saturated heterocyclyl forms and specific examples of such oxo-substituting saturated heterocyclyl include 2-oxopyrrolidinyl, 2-oxopiperidinyl, 2-oxopiperazinyl, 3-oxopiperazinyl, 1,1-dioxidotetrahydrothiophenyl, 1-oxidotetrahydro-2H-thiopyranyl, 1,1-dioxidotetrahydro-2H-thiopyranyl, 1,1-dioxidoisothiazolidinyl, 2-oxo-1,3-oxazolidinyl, 2-oxo-1,3-oxazinanyl, 6-oxo-1,1-dihydropyridazinyl, and the like.

The term "$C_{1-2}$alkanediyl" refers to a divalent hydrocarbon group formed by removing one hydrogen atom from an alkyl group having one or two carbon atoms. The $C_{1-2}$alkanediyl includes methanediyl, ethane-1,1-diyl, and ethane-1,2-diyl.

The term "$C_{1-3}$alkanediyl" refers to a divalent hydrocarbon group formed by removing one hydrogen atom from an alkyl group having one to three carbon atoms. The $C_{1-3}$alkanediyl includes methanediyl, ethane-1,1-diyl, ethane-1,2-diyl, propane-1,1-diyl, propane-1,2-diyl, propane-1,3-diyl, and propane-2,2-diyl.

The following is one preferred embodiment of compounds of the present invention.

Among the structures represented by formula [III] shown below,

[Formula 18]

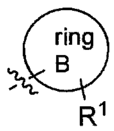

[III]

preferred is any of the structures of formula group [VII] shown below:

[Formula 19]

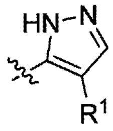 , 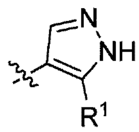 ,

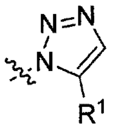 ,

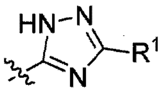 , 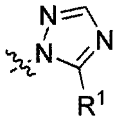 ,

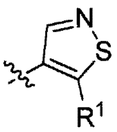 , 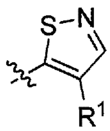 [VII]

More preferred is any of the structures of formula group [V] shown below:

[Formula 20]

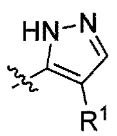 , 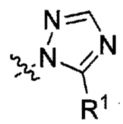 , 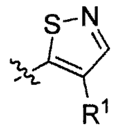 [V]

Even more preferred is the structure of formula [VI] shown below:

[Formula 21]

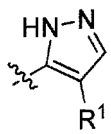 [VI]

Preferably, $R^1$ is a hydrogen atom, hydroxy, cyano, a fluorine atom, a chlorine atom, a bromine atom, methyl, hydroxymethyl, or methoxy. More preferably, $R^1$ is a hydrogen atom, a fluorine atom, a chlorine atom, a bromine atom, or methyl. Even more preferably, $R^1$ is a hydrogen atom or methyl. Particularly preferably, $R^1$ is a hydrogen atom.

Preferably, $R^2$ is a hydrogen atom, a fluorine atom, or methyl. More preferably, $R^2$ is a hydrogen atom or methyl. Even more preferably, $R^2$ is a hydrogen atom.

Preferably, $R^3$ is a hydrogen atom or methyl. More preferably, $R^3$ is a hydrogen atom.

Preferably, $R^4$ is a hydrogen atom.

Preferably, W is a single bond or $C_{1-3}$alkanediyl. More preferably, W is $C_{1-2}$alkanediyl. Even more preferably, W is methanediyl or ethane-1,2-diyl. Particularly preferably, W is methanediyl.

Preferably, ring A is

(a) $C_{4-6}$cycloalkyl, wherein the $C_{4-6}$cycloalkyl is substituted with one substituent selected from substituent group A11',
(b) 4- to 6-membered saturated nitrogen-containing heterocyclyl, wherein the 4- to 6-membered saturated nitrogen-containing heterocyclyl is substituted with one substituent selected from substituent group A21' and may be further

substituted with one substituent selected from substituent group A22,

(c) phenyl, wherein the phenyl is substituted with one substituent selected from substituent group A31' and may be further substituted with one substituent selected from substituent group A32,

(d) pyridyl, wherein the pyridyl is substituted with one substituent selected from substituent group A41',

(e) naphthyl,

(f) 2,3-dihydrobenzofuran, wherein the 2,3-dihydrobenzofuran may be substituted with one to three substituents selected from substituent group A51,

(g) 2H-chromenyl, wherein the 2H-chromenyl may be substituted with one oxo,

(h) quinolyl, wherein the quinolyl may be substituted with one $C_{1-6}$alkoxy,

(j) quinoxalyl,

(k) a group represented by formula [11-1] shown below, wherein the group represented by formula [II-1] is substituted with one $C_{1-6}$alkyl, wherein the $C_{1-6}$alkyl may be substituted with one substituent selected from substituent B61,

(m) a group represented by formula [II-2] shown below, wherein the group represented by formula [II-2] is substituted with one $C_{1-6}$alkylcarbonyl,

(n) a group represented by formula [II-3] shown below, wherein the group represented by formula [II-3] is substituted with one $C_{1-6}$alkylcarbonyl,

(p) a group represented by formula [II-4] shown below, wherein the group represented by formula [II-4] is substituted with one $C_{1-6}$alkylcarbonyl,

(r) 4- to 6-membered saturated oxygen-containing heterocyclyl, or

(s) 4- to 6-membered saturated sulfur-containing heterocyclyl, wherein the 4- to 6-membered saturated sulfur-containing heterocyclyl may be substituted with one or two oxo;

[Formula 22]

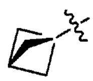 [II−1], 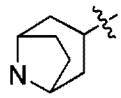 [II−2],

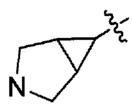 [II−3], 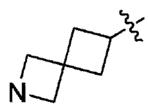 [II−4]

wherein substituent group A11' represents the group consisting of

(i) $C_{1-6}$alkylcarbonylamino and

(ii) $C_{1-6}$alkylcarbonyl($C_{1-6}$alkyl)amino;

substituent group A21' represents the group consisting of

(i) $C_{1-6}$alkylcarbonyl, wherein the $C_{1-6}$alkylcarbonyl may be substituted with one to three substituents selected from substituent group B21',

(ii) $C_{3-8}$cycloalkylcarbonyl, wherein the $C_{3-8}$cycloalkylcarbonyl may be substituted with one or two substituents selected from substituent group B22,

(iii) arylcarbonyl, wherein the arylcarbonyl may be substituted with one substituent selected from substituent group B23,

(iv) saturated heterocyclylcarbonyl, wherein the saturated heterocyclylcarbonyl may be substituted with one or two substituents selected from substituent group B24',

(v) heteroarylcarbonyl, wherein the heteroarylcarbonyl may be substituted with one substituent selected from the group consisting of $C_{1-6}$alkyl, wherein the $C_{1-6}$alkyl may be substituted with one hydroxy,

(vi) $C_{1-6}$alkoxycarbonyl,

(vii) mono$C_{1-6}$alkylaminocarbonyl,

(viii) di$C_{1-6}$alkylaminocarbonyl,

(ix) $C_{3-8}$cycloalkylaminocarbonyl,

(x) $C_{3-8}$cycloalkyl($C_{1-6}$alkyl)aminocarbonyl,

(xi) $C_{1-6}$alkylsulfonyl, wherein the $C_{1-6}$alkylsulfonyl may be substituted with one $C_{1-6}$alkoxycarbonylamino,

(xii) $C_{3-8}$cycloalkylsulfonyl,

(xiii) saturated heterocyclylsulfonyl, wherein the saturated heterocyclylsulfonyl may be substituted with one substituent selected from substituent group B25, and
(xiv) di$C_{1-6}$alkylaminosulfonyl;

substituent group A22 represents the group consisting of

(i) a halogen atom and
(ii) $C_{1-6}$alkyl;

substituent group B21' represents the group consisting of

(i) hydroxy,
(ii) ureide,
(iii) a halogen atom,
(iv) $C_{3-8}$cycloalkyl, wherein the $C_{3-8}$cycloalkyl may be substituted with one hydroxy,
(v) saturated heterocyclyl, wherein the saturated heterocyclyl may be substituted with one or two substituents selected from the group consisting of hydroxy and oxo,
(vi) heteroaryl, wherein the heteroaryl may be substituted with one oxo,
(vii) $C_{1-6}$alkoxy,
(viii) aryloxy,
(ix) saturated heterocyclylcarbonyl,
(x) $C_{1-6}$alkylsulfonyl,
(xi) halo-$C_{1-6}$alkylsulfonyl,
(xii) arylsulfonyl,
(xiii) $C_{1-6}$alkylcarbonylamino, wherein $C_{1-6}$alkyl in the $C_{1-6}$alkylcarbonylamino may be substituted with one substituent selected from the group consisting of hydroxy and saturated heterocyclyl,
(xiv) $C_{1-6}$alkylcarbonyl($C_{1-6}$alkyl)amino,
(xv) $C_{3-8}$cycloalkylcarbonylamino, wherein $C_{3-8}$cycloalkyl in the $C_{3-8}$cycloalkylcarbonylamino may be substituted with one or two halogen atoms,
(xvi) arylcarbonylamino,
(xvii) saturated heterocyclylcarbonylamino,
(xviii) mono$C_{1-6}$alkylaminocarbonyl,
(xix) di$C_{1-6}$alkylaminocarbonyl,
(xx) $C_{1-6}$alkoxycarbonylamino, wherein $C_{1-6}$alkoxy in the $C_{1-6}$alkoxycarbonylamino may be substituted with one substituent selected from the group consisting of $C_{1-6}$alkoxy and aryl,
(xxi) $C_{1-6}$alkoxycarbonyl($C_{1-6}$alkyl)amino,
(xxii) $C_{3-8}$cycloalkoxycarbonylamino, wherein $C_{3-8}$cycloalkoxy in the $C_{3-8}$cycloalkoxycarbonylamino may be substituted with one $C_{1-6}$alkyl,
(xxiii) mono$C_{1-6}$alkylaminocarbonylamino, and
(xxiv) di$C_{1-6}$alkylaminocarbonylamino;

substituent group B22 represents the group consisting of

(i) hydroxy,
(ii) carbamoyl,
(iii) a halogen atom,
(iv) $C_{1-6}$alkyl, and
(v) $C_{1-6}$alkoxycarbonylamino;

substituent group B23 represents

(i) $C_{1-6}$alkoxy, wherein the $C_{1-6}$alkoxy may be substituted with one carbamoyl,

substituent group B24' represents the group consisting of

(i) oxo,
(ii) a halogen atom,
(iii) $C_{1-6}$alkyl,
(iv) $C_{1-6}$alkylcarbonyl, and

(iv) $C_{1-6}$alkoxycarbonyl;

substituent group B25 represents the group consisting of

(i) $C_{1-6}$alkylcarbonyl and
(ii) $C_{1-6}$alkoxycarbonyl, wherein the $C_{1-6}$alkoxycarbonyl may be substituted with one aryl;

substituent group A31' represents the group consisting of

(i) amino,
(ii) halo-$C_{1-6}$alkyl,
(iii) $C_{2-6}$alkenyl, wherein the $C_{2-6}$alkenyl may be substituted with one substituent selected from substituent group B32,
(iv) halo-$C_{1-6}$alkoxy,
(v) halo-$C_{1-6}$alkylsulfanyl,
(vi) saturated heterocyclylcarbonyl, wherein the saturated heterocyclylcarbonyl may be substituted with one or two $C_{1-6}$alkyl groups,
(vii) $C_{1-6}$alkylsulfonyl, wherein the $C_{1-6}$alkylsulfonyl may be substituted with one substituent selected from substituent group B35,
(viii) $C_{3-8}$cycloalkylsulfonyl,
(ix) arylsulfonyl, wherein the arylsulfonyl may be substituted with one $C_{1-6}$alkyl,
(x) di$C_{1-6}$alkylaminosulfonyl, and
(xi) $C_{1-6}$alkoxycarbonylamino;

substituent group A32 represents the group consisting of

(i) a halogen atom,
(ii) $C_{1-6}$alkyl,
(iii) halo-$C_{1-6}$alkyl, and
(iv) $C_{1-6}$alkoxy;

substituent group B32 represents

(i) aryl;

substituent group B35 represents the group consisting of

(i) $C_{3-8}$cycloalkyl,
(ii) saturated heterocyclyl, and
(iii) saturated heterocyclylcarbonyl;

substituent group A41' represents the group consisting of

(i) halo-$C_{1-6}$alkyl,
(ii) triazolyl,
(iii) $C_{1-6}$alkylsulfonyl, wherein the $C_{1-6}$alkylsulfonyl may be substituted with one $C_{3-8}$cycloalkyl, and
(iv) $C_{1-6}$alkylcarbonylamino;

substituent group A51 represents the group consisting of

(i) a halogen atom and
(ii) $C_{1-6}$alkyl;

substituent group B61 represents the group consisting of

(i) $C_{1-6}$alkylcarbonylamino and
(ii) $C_{1-6}$alkylcarbonyl($C_{1-6}$alkyl)amino;

more preferably, ring A is

(a) 4- to 6-membered saturated nitrogen-containing heterocyclyl, wherein the 4- to 6-membered saturated nitrogen-containing heterocyclyl is substituted with one substituent selected from substituent group A21",
(b) phenyl, wherein the phenyl is substituted with one substituent selected from substituent group A31" and may be further substituted with one halogen atom,
(c) pyridyl, wherein the pyridyl is substituted with one substituent selected from substituent group A41",
(d) 2,3-dihydrobenzofuran, wherein the 2,3-dihydrobenzofuran is substituted with one halogen atom and two $C_{1-6}$alkyl, or
(e) 4- to 6-membered saturated oxygen-containing heterocyclyl;

wherein substituent group A21" represents the group consisting of

(i) $C_{1-6}$alkylcarbonyl, wherein the $C_{1-6}$alkylcarbonyl may be substituted with one to three substituents selected from substituent group B21",
(ii) $C_{3-8}$cycloalkylcarbonyl, wherein the $C_{3-8}$cycloalkylcarbonyl may be substituted with one $C_{1-6}$alkoxycarbonylamino,
(iii) $C_{1-6}$alkoxycarbonyl,
(iv) mono$C_{1-6}$alkylaminocarbonyl,
(v) di$C_{1-6}$alkylaminocarbonyl,
(vi) $C_{1-6}$alkylsulfonyl, wherein the $C_{1-6}$alkylsulfonyl may be substituted with one $C_{1-6}$alkoxycarbonylamino,
(vii) $C_{3-8}$cycloalkylsulfonyl,
(viii) saturated heterocyclylsulfonyl, wherein the saturated heterocyclylsulfonyl may be substituted with one substituent selected from substituent group B25, and
(ix) di$C_{1-6}$alkylaminosulfonyl;

substituent group B21" represents the group consisting of

(i) a halogen atom,
(ii) $C_{3-8}$cycloalkyl, wherein the $C_{3-8}$cycloalkyl may be substituted with one hydroxy,
(iii) aryloxy,
(iv) $C_{3-8}$cycloalkylcarbonylamino, wherein $C_{3-8}$cycloalkyl in the $C_{3-8}$cycloalkylcarbonylamino may be substituted with one or two halogen atoms,
(v) arylcarbonylamino,
(vi) $C_{1-6}$alkoxycarbonylamino, wherein $C_{1-6}$alkoxy in the $C_{1-6}$alkoxycarbonylamino may be substituted with one aryl, and
(vii) $C_{3-8}$cycloalkoxycarbonylamino, wherein $C_{3-8}$cycloalkoxy in the $C_{3-8}$cycloalkoxycarbonylamino may be substituted with one $C_{1-6}$alkyl;

substituent group B25 represents the group consisting of

(i) $C_{1-6}$alkylcarbonyl and
(ii) $C_{1-6}$alkoxycarbonyl, wherein the $C_{1-6}$alkoxycarbonyl may be substituted with one aryl;

substituent group A31" represents the group consisting of

(i) halo-$C_{1-6}$alkyl,
(ii) halo-$C_{1-6}$alkoxy,
(iii) halo-$C_{1-6}$alkylsulfanyl,
(iv) $C_{1-6}$alkylsulfonyl, wherein the $C_{1-6}$alkylsulfonyl may be substituted with one substituent selected from substituent group B35",
(v) $C_{3-8}$cycloalkylsulfonyl, and
(vi) di$C_{1-6}$alkylaminosulfonyl;

substituent group B35" represents the group consisting of

(i) $C_{3-8}$cycloalkyl and
(ii) saturated heterocyclylcarbonyl;

substituent group A41" represents the group consisting of

(i) halo-$C_{1-6}$alkyl and
(ii) $C_{1-6}$alkylsulfonyl, wherein the $C_{1-6}$alkylsulfonyl maybe substituted with one $C_{3-8}$cycloalkyl;

even more preferably, ring A is

(a) piperidin-4-yl substituted with one substituent selected from the group consisting of $C_{1-6}$alkylcarbonyl and $C_{1-6}$alkoxycarbonyl or
(b) phenyl substituted with one substituent selected from the group consisting of $C_{1-6}$alkylsulfonyl and $C_{3-8}$cycloalkylsulfonyl; and

particularly preferably, ring A is

(a) piperidin-4-yl substituted at the 1-position with one substituent selected from the group consisting of acetyl and methoxycarbonyl or
(b) phenyl substituted at the 3-position with one substituent selected from the group consisting of methylsulfonyl and cyclopropylsulfonyl.

The following are other preferred embodiments of compounds of the present invention.

Preferably, $R^1$ is a hydrogen atom, a fluorine atom, or methyl. More preferably, $R^1$is a hydrogen atom or methyl. Even more preferably, $R^1$ is a hydrogen atom.

Preferably, $R^2$ is a hydrogen atom or a fluorine atom. More preferably, $R^2$ is a hydrogen atom.

Preferably, $R^3$ is a hydrogen atom or methyl. More preferably, $R^3$ is a hydrogen atom.

Preferably, $R^4$ is a hydrogen atom.

Preferably, W is a single bond or $C_{1-3}$alkanediyl. More preferably, W is $C_{1-2}$alkanediyl. Even more preferably, W is methanediyl or ethane-1,2-diyl. Particularly preferably, W is methanediyl.

Preferably, ring A is

(a) $C_{4-6}$cycloalkyl, wherein the $C_{4-6}$cycloalkyl is substituted with one substituent selected from substituent group A11',
(b) 4- to 6-membered saturated nitrogen-containing heterocyclyl, wherein the 4- to 6-membered saturated nitrogen-containing heterocyclyl is substituted with one substituent selected from substituent group A21' and may be further substituted with one substituent selected from substituent group A22,
(c) phenyl, wherein the phenyl is substituted with one substituent selected from substituent group A31' and may be further substituted with one substituent selected from substituent group A32,
(d) pyridyl, wherein the pyridyl is substituted with one substituent selected from substituent group A41',
(e) naphthyl,
(f) 2,3-dihydrobenzofuran, wherein the 2,3-dihydrobenzofuran may be substituted with one to three substituents selected from substituent group A51,
(g) 2H-chromenyl, wherein the 2H-chromenyl may be substituted with one oxo,
(h) quinolyl, wherein the quinolyl may be substituted with one $C_{1-6}$alkoxy,
(j) quinoxalyl,
(k) a group represented by formula [II-1] shown below, wherein the group represented by formula [II-1] is substituted with one $C_{1-6}$alkyl, wherein the $C_{1-6}$alkyl may be substituted with one substituent selected from substituent B61,
(m) a group represented by formula [II-2] shown below, wherein the group represented by formula [II-2] is substituted with one $C_{1-6}$alkylcarbonyl,
(n) a group represented by formula [II-3] shown below, wherein the group represented by formula [II-3] is substituted with one $C_{1-6}$alkylcarbonyl, or
(p) a group represented by formula [II-4] shown below, wherein the group represented by formula [II-4] is substituted with one $C_{1-6}$alkylcarbonyl;

[Formula 23]

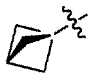 [II−1], 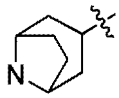 [II−2],

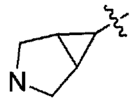 [II−3], 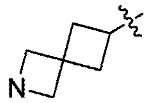 [II−4]

wherein substituent group A11' represents the group consisting of

(i) $C_{1-6}$alkylcarbonylamino and
(ii) $C_{1-6}$alkylcarbonyl($C_{1-6}$alkyl)amino;

substituent group A21' represents the group consisting of

(i) $C_{1-6}$alkylcarbonyl, wherein the $C_{1-6}$alkylcarbonyl may be substituted with one to three substituents selected from substituent group B21',
(ii) $C_{3-8}$cycloalkylcarbonyl, wherein the $C_{3-8}$cycloalkylcarbonyl may be substituted with one or two substituents selected from substituent group B22,
(iii) arylcarbonyl, wherein the arylcarbonyl may be substituted with one substituent selected from substituent group B23,
(iv) saturated heterocyclylcarbonyl, wherein the saturated heterocyclylcarbonyl may be substituted with one or two substituents selected from substituent group B24',
(v) heteroarylcarbonyl, wherein the heteroarylcarbonyl may be substituted with one substituent selected from the group consisting of $C_{1-6}$alkyl, wherein the $C_{1-6}$alkyl may be substituted with one hydroxy,
(vi) $C_{1-6}$alkoxycarbonyl,
(vii) mono$C_{1-6}$alkylaminocarbonyl,
(viii) di$C_{1-6}$alkylaminocarbonyl,
(ix) $C_{3-8}$cycloalkylaminocarbonyl,
(x) $C_{3-8}$cycloalkyl($C_{1-6}$alkyl)aminocarbonyl,
(xi) $C_{1-6}$alkylsulfonyl, wherein the $C_{1-6}$alkylsulfonyl may be substituted with one $C_{1-6}$alkoxycarbonylamino,
(xii) $C_{3-8}$cycloalkylsulfonyl,
(xiii) saturated heterocyclylsulfonyl, wherein the saturated heterocyclylsulfonyl may be substituted with one substituent selected from substituent group B25, and
(xiv) di$C_{1-6}$alkylaminosulfonyl;

substituent group A22 represents the group consisting of

(i) a halogen atom and
(ii) $C_{1-6}$alkyl;

substituent group B21' represents the group consisting of

(i) hydroxy,
(ii) ureide,
(iii) $C_{3-8}$cycloalkyl, wherein the $C_{3-8}$cycloalkyl may be substituted with one hydroxy,
(iv) saturated heterocyclyl, wherein the saturated heterocyclyl may be substituted with one or two substituents selected from the group consisting of hydroxy and oxo,
(v) $C_{1-6}$alkoxy,
(vi) aryloxy,
(vii) $C_{1-6}$alkylsulfonyl,
(viii) halo-$C_{1-6}$alkylsulfonyl,
(ix) arylsulfonyl,
(x) $C_{1-6}$alkylcarbonylamino, wherein $C_{1-6}$alkyl in the $C_{1-6}$alkylcarbonylamino may be substituted with one substituent selected from the group consisting of hydroxy and saturated heterocyclyl,

(xi) $C_{1-6}$alkylcarbonyl($C_{1-6}$alkyl)amino,
(xii) $C_{3-8}$cycloalkylcarbonylamino, wherein $C_{3-8}$cycloalkyl in the $C_{3-8}$cycloalkylcarbonylamino may be substituted with one or two halogen atoms,
(xiii) arylcarbonylamino,
(xiv) saturated heterocyclylcarbonylamino,
(xv) $C_{1-6}$alkoxycarbonylamino, wherein $C_{1-6}$alkoxy in the $C_{1-6}$alkoxycarbonylamino may be substituted with one substituent selected from the group consisting of $C_{1-6}$alkoxy and aryl,
(xvi) $C_{1-6}$alkoxycarbonyl($C_{1-6}$alkyl)amino,
(xvii) $C_{3-8}$cycloalkoxycarbonylamino, wherein $C_{3-8}$cycloalkoxy in the $C_{3-8}$cycloalkoxycarbonylamino may be substituted with one $C_{1-6}$alkyl,
(xviii) mono$C_{1-6}$alkylaminocarbonylamino, and
(xix) di$C_{1-6}$alkylaminocarbonylamino;

substituent group B22 represents the group consisting of

(i) hydroxy,
(ii) carbamoyl,
(iii) a halogen atom,
(iv) $C_{1-6}$alkyl, and
(v) $C_{1-6}$alkoxycarbonylamino;

substituent group B23 represents

(i) $C_{1-6}$alkoxy, wherein the $C_{1-6}$alkoxy may be substituted with one carbamoyl;

substituent group B24' represents the group consisting of

(i) oxo,
(ii) a halogen atom,
(iii) $C_{1-6}$alkyl,
(iv) $C_{1-6}$alkylcarbonyl, and
(v) $C_{1-6}$alkoxycarbonyl;

substituent group B25 represents the group consisting of

(i) $C_{1-6}$alkylcarbonyl and
(ii) $C_{1-6}$alkoxycarbonyl, wherein the $C_{1-6}$alkoxycarbonyl may be substituted with one aryl;

substituent group A31' represents the group consisting of

(i) amino,
(ii) halo-$C_{1-6}$alkyl,
(iii) $C_{2-6}$alkenyl, wherein the $C_{2-6}$alkenyl may be substituted with one substituent selected from substituent group B32,
(iv) halo-$C_{1-6}$alkoxy,
(v) halo-$C_{1-6}$alkylsulfanyl,
(vi) saturated heterocyclylcarbonyl, wherein the saturated heterocyclylcarbonyl may be substituted with one or two $C_{1-6}$alkyl,
(vii) $C_{1-6}$alkylsulfonyl, wherein the $C_{1-6}$alkylsulfonyl may be substituted with one substituent selected from substituent group B35,
(viii) $C_{3-8}$cycloalkylsulfonyl,
(ix) arylsulfonyl, wherein the arylsulfonyl may be substituted with one $C_{1-6}$alkyl,
(x) di$C_{1-6}$alkylaminosulfonyl, and
(xi) $C_{1-6}$alkoxycarbonylamino;

substituent group A32 represents the group consisting of

(i) a halogen atom,
(ii) $C_{1-6}$alkyl,

(iii) halo-$C_{1-6}$alkyl, and
(iv) $C_{1-6}$alkoxy;

substituent group B32 represents

(i) aryl;

substituent group B35 represents the group consisting of

(i) $C_{3-8}$cycloalkyl,
(ii) saturated heterocyclyl, and
(iii) saturated heterocyclylcarbonyl;

substituent group A41' represents the group consisting of

(i) halo-$C_{1-6}$alkyl,
(ii) triazolyl,
(iii) $C_{1-6}$alkylsulfonyl, wherein the $C_{1-6}$alkylsulfonyl may be substituted with one $C_{3-8}$cycloalkyl, and
(iv) $C_{1-6}$alkylcarbonylamino;

substituent group A51 represents the group consisting of

(i) a halogen atom and
(ii) $C_{1-6}$alkyl;

substituent group B61 represents the group consisting of

(i) $C_{1-6}$alkylcarbonylamino and
(ii) $C_{1-6}$alkylcarbonyl($C_{1-6}$alkyl)amino;

more preferably, ring A is

(a) 4- to 6-membered saturated nitrogen-containing heterocyclyl, wherein the 4- to 6-membered saturated nitrogen-containing heterocyclyl is substituted with one substituent selected from substituent group A21",
(b) phenyl, wherein the phenyl is substituted with one substituent selected from substituent group A31" and may be further substituted with one halogen atom,
(c) pyridyl, wherein the pyridyl is substituted with one substituent selected from substituent group A41", or
(d) 2,3-dihydrobenzofuran, wherein the 2,3-dihydrobenzofuran is substituted with one halogen atom and two $C_{1-6}$alkyl;

wherein substituent group A21" represents the group consisting of

(i) $C_{1-6}$alkylcarbonyl, wherein the $C_{1-6}$alkylcarbonyl may be substituted with one to three substituents selected from substituent group B21",
(ii) $C_{3-8}$cycloalkylcarbonyl, wherein the $C_{3-8}$cycloalkylcarbonyl may be substituted with one $C_{1-6-6}$alkoxycarbonylamino,
(iii) $C_{1-6}$alkoxycarbonyl,
(iv) mono$C_{1-6}$alkylaminocarbonyl,
(v) di$C_{1-6}$alkylaminocarbonyl,
(vi) $C_{1-6}$alkylsulfonyl, wherein the $C_{1-6}$alkylsulfonyl may be substituted with one $C_{1-6}$alkoxycarbonylamino,
(vii) $C_{3-8}$cycloalkylsulfonyl,
(viii) saturated heterocyclylsulfonyl, wherein the saturated heterocyclylsulfonyl may be substituted with one substituent selected from substituent group B25, and
(ix) di$C_{1-6}$alkylaminosulfonyl;

substituent group B21" represents the group consisting of

(i) a halogen atom,

(ii) $C_{3-8}$cycloalkyl, wherein the $C_{3-8}$cycloalkyl may be substituted with one hydroxy,
(iii) aryloxy,
(iv) $C_{3-8}$cycloalkylcarbonylamino, wherein $C_{3-8}$cycloalkyl in the $C_{3-8}$cycloalkylcarbonylamino may be substituted with one or two halogen atoms,
(v) arylcarbonylamino,
(vi) $C_{1-6}$alkoxycarbonylamino, wherein $C_{1-6}$alkoxy in the $C_{1-6}$alkoxycarbonylamino may be substituted with one aryl, and
(vii) $C_{3-8}$cycloalkoxycarbonylamino, wherein $C_{3-8}$cycloalkoxy in the $C_{3-8}$cycloalkoxycarbonylamino may be substituted with one Ci-6alkyl;

substituent group B25 represents the group consisting of

(i) $C_{1-6}$alkylcarbonyl and
(ii) $C_{1-6}$alkoxycarbonyl, wherein the $C_{1-6}$alkoxycarbonyl may be substituted with one aryl;

substituent group A31" represents the group consisting of

(i) halo-$C_1$-6alkyl,
(ii) halo-$C_{1-6}$alkoxy,
(iii) halo-$C_{1-6}$alkylsulfanyl,
(iv) $C_{1-6}$alkylsulfonyl, wherein the $C_{1-6}$alkylsulfonyl may be substituted with one substituent selected from substituent group B35",
(v) $C_{3-8}$cycloalkylsulfonyl, and
(vi) di$C_{1-6}$alkylaminosulfonyl;

substituent group B35" represents the group consisting of

(i) $C_{3-8}$cycloalkyl and
(ii) saturated heterocyclylcarbonyl;

substituent group A41" represents the group consisting of

(i) halo-$C_{1-6}$alkyl and
(ii) $C_{1-6}$alkylsulfonyl, wherein the $C_{1-6}$alkylsulfonyl may be substituted with one $C_{3-8}$cycloalkyl;

even more preferably, ring A is

(a) piperidin-4-yl substituted with one substituent selected from the group consisting of $C_{1-6}$alkylcarbonyl and $C_{1-6}$alkoxycarbonyl, or
(b) phenyl substituted with one substituent selected from the group consisting of $C_{1-6}$alkylsulfonyl and $C_{3-8}$cycloalkylsulfonyl; and

particularly preferably, ring A is

(a) piperidin-4-yl substituted at the 1-position with one substituent selected from the group consisting of acetyl and methoxycarbonyl, or
(b) phenyl substituted at the 3-position with one substituent selected from the group consisting of methylsulfonyl and cyclopropylsulfonyl.

One preferred embodiment of the compound of the present invention is a compound represented by formula [I'-a] shown below or a pharmaceutically acceptable salt thereof.

[Formula 24]

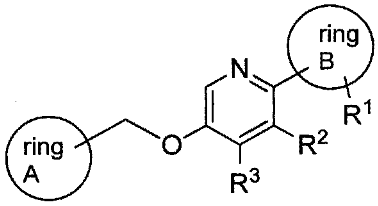

[I'-a]

Here, preferred embodiments of the structure represented by formula [III], $R^1$, $R^2$, $R^3$, and ring A are as described above.

In a more preferred embodiment of formula [I'-a] shown above,
the structure represented by formula [III] shown below

[Formula 25]

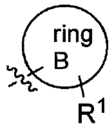

[III]

is any of the structures represented by formula group [V] shown below:

[Formula 26]

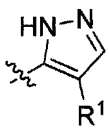

,

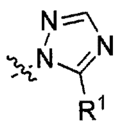

,

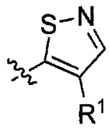

[V]

wherein

$R^1$ is a hydrogen atom, a fluorine atom, a chlorine atom, a bromine atom, or methyl;
$R^2$ is a hydrogen atom, a fluorine atom, or methyl;
$R^3$ is a hydrogen atom or methyl;
ring A is

(a) pyrrolidin-3-yl, wherein the pyrrolidin-3-yl is substituted with one substituent selected from the group consisting of $C_{1-6}$alkylsulfonyl, $C_{3-8}$cycloalkylsulfonyl, and di$C_{1-6}$alkylaminosulfonyl,
(b) piperidin-3-yl, wherein the piperidin-3-yl is substituted with one substituent selected from substituent group A21",
(c) piperidin-4-yl, wherein the piperidin-4-yl is substituted with one $C_{1-6}$alkylcarbonyl,
(d) phenyl, wherein the phenyl is substituted with one substituent selected from substituent group A31" and may be further substituted with one halogen atom,
(e) pyridyl, wherein the pyridyl is substituted with one substituent selected from substituent group A41", or
(f) 2,3-dihydrobenzofuran, wherein the 2,3-dihydrobenzofuran is substituted with one halogen atom and two $C_{1-6}$alkyl;

wherein substituent group A21" represents the group consisting of

(i) $C_{1-6}$alkylcarbonyl, wherein the $C_{1-6}$alkylcarbonyl may be substituted with one to three substituents selected from substituent group B21",
(ii) $C_{3-8}$cycloalkylcarbonyl, wherein the $C_{3-8}$cycloalkylcarbonyl may be substituted with one $C_{1-6}$alkoxycarbonylamino,
(iii) $C_{1-6}$alkoxycarbonyl,
(iv) mono$C_{1-6}$alkylaminocarbonyl,

(v) di$C_{1-6}$alkylaminocarbonyl,
(vi) $C_{1-6}$alkylsulfonyl, wherein the $C_{1-6}$alkylsulfonyl may be substituted with one $C_{1-6}$alkoxycarbonylamino,
(vii) $C_{3-8}$cycloalkylsulfonyl,
(viii) saturated heterocyclylsulfonyl, wherein the saturated heterocyclylsulfonyl may be substituted with one substituent selected from substituent group B25, and
(ix) di$C_{1-6}$alkylaminosulfonyl;

substituent group B21" represents the group consisting of

(i) a halogen atom,
(ii) $C_{3-8}$cycloalkyl, wherein the $C_{3-8}$cycloalkyl may be substituted with one hydroxy,
(iii) aryloxy,
(iv) $C_{3-8}$cycloalkylcarbonylamino, wherein $C_{3-8}$cycloalkyl in the $C_{3-8}$cycloalkylcarbonylamino may be substituted with one or two halogen atoms,
(v) arylcarbonylamino,
(vi) $C_{1-6}$alkoxycarbonylamino, wherein $C_{1-6}$alkoxy in the $C_{1-6}$alkoxycarbonylamino may be substituted with one substituent selected from the group consisting of aryl, and
(vii) $C_{3-8}$cycloalkoxycarbonylamino, wherein $C_{3-8}$cycloalkoxy in the $C_{3-8}$cycloalkoxycarbonylamino may be substituted with one $C_{1-6}$alkyl;

substituent group B25 represents the group consisting of

(i) $C_{1-6}$alkylcarbonyl and
(ii) $C_{1-6}$alkoxycarbonyl, wherein the $C_{1-6}$alkoxycarbonyl may be substituted with one aryl;

substituent group A31" represents the group consisting of

(i) halo-$C_1$-6alkyl,
(ii) halo-$C_{1-6}$alkoxy,
(iii) halo-$C_{1-6}$alkylsulfanyl,
(iv) $C_{1-6}$alkylsulfonyl, wherein the $C_{1-6}$alkylsulfonyl may be substituted with one substituent selected from substituent group B35",
(v) $C_{3-8}$cycloalkylsulfonyl, and
(vi) di$C_{1-6}$alkylaminosulfonyl;

substituent group B35" represents the group consisting of

(i) $C_{3-8}$cycloalkyl and
(ii) saturated heterocyclylcarbonyl; and

substituent group A41" represents the group consisting of

(i) halo-$C_{1-6}$alkyl and
(ii) $C_{1-6}$alkylsulfonyl, wherein the $C_{1-6}$alkylsulfonyl may be substituted with one $C_{3-8}$cycloalkyl.

In a further preferable embodiment of formula [I'-a] shown above, the structure represented by formula [III] shown below

[Formula 27]

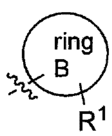

[III]

is the structure represented by formula [VI] shown below:

[Formula 28]

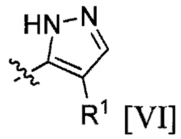

wherein

$R^1$ is a hydrogen atom or methyl;
$R^2$ is a hydrogen atom or a fluorine atom;
$R^3$ is a hydrogen atom; and
ring A is

(a) piperidin-4-yl substituted with one $C_{1-6}$alkylcarbonyl, or
(b) phenyl substituted with one substituent selected from the group consisting of $C_{1-6}$alkylsulfonyl and $C_{3-8}$cycloalkylsulfonyl.

In a particularly preferred embodiment of formula [I'-a] shown above, the structure represented by formula [III] shown below

[Formula 29]

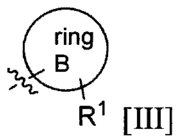

is the structure represented by formula [VI] shown below:

[Formula 30]

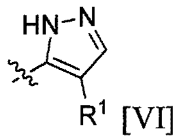

wherein

$R^1$ is a hydrogen atom or methyl, and $R^2$ is a hydrogen atom or a fluorine atom, provided that one of $R^1$ and $R^2$ is a hydrogen atom,
$R^3$ is a hydrogen atom; and
ring A is

(a) piperidin-4-yl substituted at 1-position with one acetyl, or
(b) phenyl substituted at 3-position with one substituent selected from the group consisting of methylsulfonyl and cyclopropylsulfonyl.

Another preferred embodiment of the compound of the present invention is a compound represented by formula [I-a] shown below or a pharmaceutically acceptable salt thereof.

[Formula 31]

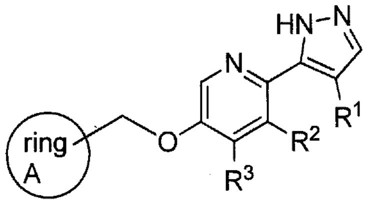

[I-a]

Here, preferred embodiments of $R^1$, $R^2$, $R^3$, and ring A are as described above.

In a more preferred embodiment of formula [I-a] shown above,
$R^1$ is a hydrogen atom, a fluorine atom, or methyl;
$R^2$ is a hydrogen atom or a fluorine atom;
$R^3$ is a hydrogen atom or methyl;
ring A is

(a) pyrrolidin-3-yl, wherein the pyrrolidin-3-yl is substituted with one substituent selected from the group consisting of $C_{1-6}$alkylsulfonyl, $C_{3-8}$cycloalkylsulfonyl, and di$C_{1-6}$alkylaminosulfonyl,
(b) piperidin-3-yl, wherein the piperidin-3-yl is substituted with one substituent selected from substituent group A21",
(c) piperidin-4-yl, wherein the piperidin-4-yl is substituted with one $C_{1-6}$alkylcarbonyl,
(d) phenyl, wherein the phenyl is substituted with one substituent selected from substituent group A31" and may be further substituted with one halogen atom,
(e) pyridyl, wherein the pyridyl is substituted with one substituent selected from substituent group A41", or
(f) 2,3-dihydrobenzofuran, wherein the 2,3-dihydrobenzofuran is substituted with one halogen atom and two $C_{1-6}$alkyl;

wherein substituent group A21" represents the group consisting of

(i) $C_{1-6}$alkylcarbonyl, wherein the $C_{1-6}$alkylcarbonyl may be substituted with one to three substituents selected from substituent group B21",
(ii) $C_{3-8}$cycloalkylcarbonyl, wherein the $C_{3-8}$cycloalkylcarbonyl may be substituted with one $C_{1-6}$alkoxycarbonylamino,
(iii) $C_{1-6}$alkoxycarbonyl,
(iv) mono$C_{1-6}$alkylaminocarbonyl,
(v) di$C_{1-6}$alkylaminocarbonyl,
(vi) $C_{1-6}$alkylsulfonyl, wherein the $C_{1-6}$alkylsulfonyl may be substituted with one $C_{1-6}$alkoxycarbonylamino,
(vii) $C_{3-8}$cycloalkylsulfonyl,
(viii) saturated heterocyclylsulfonyl, wherein the saturated heterocyclylsulfonyl may be substituted with one substituent selected from substituent group B25, and
(ix) di$C_{1-6}$alkylaminosulfonyl;

substituent group B21" represents the group consisting of

(i) a halogen atom,
(ii) $C_{3-8}$cycloalkyl, wherein the $C_{3-8}$cycloalkyl may be substituted with one hydroxy,
(iii) aryloxy,
(iv) $C_{3-8}$cycloalkylcarbonylamino, wherein $C_{3-8}$cycloalkyl in the $C_{3-8}$cycloalkylcarbonylamino may be substituted with one or two halogen atoms,
(v) arylcarbonylamino,
(vi) $C_{1-6}$alkoxycarbonylamino, wherein $C_{1-6}$alkoxy in the $C_{1-6}$alkoxycarbonylamino may be substituted with one substituent selected from the group consisting of aryl, and
(vii) $C_{3-8}$cycloalkoxycarbonylamino, wherein $C_{3-8}$cycloalkoxy in the $C_{3-8}$cycloalkoxycarbonylamino may be substituted with one $C_{1-6}$alkyl;

substituent group B25 represents the group consisting of

(i) $C_{1-6}$alkylcarbonyl and
(ii) $C_{1-6}$alkoxycarbonyl, wherein the $C_{1-6}$alkoxycarbonyl may be substituted with one aryl;

substituent group A31" represents the group consisting of

(i) halo-$C_1$-6alkyl,
(ii) halo-$C_{1-6}$alkoxy,
(iii) halo-$C_{1-6}$alkylsulfanyl,
(iv) $C_{1-6}$alkylsulfonyl, wherein the $C_{1-6}$alkylsulfonyl may be substituted with one substituent selected from substituent group B35",
(v) $C_{3-8}$cycloalkylsulfonyl, and
(vi) di$C_{1-6}$alkylaminosulfonyl;

substituent group B35" represents the group consisting of

(i) $C_{3-8}$cycloalkyl and
(ii) saturated heterocyclylcarbonyl; and

substituent group A41" represents the group consisting of

(i) halo-$C_{1-6}$alkyl and
(ii) $C_{1-6}$alkylsulfonyl, wherein the $C_{1-6}$alkylsulfonyl may be substituted with one $C_{3-8}$cycloalkyl.

In a further preferred embodiment of formula [I-a] shown above,
$R^1$ is a hydrogen atom or methyl;
$R^2$ is a hydrogen atom or a fluorine atom;
$R^3$ is a hydrogen atom; and
ring A is

(a) piperidin-4-yl substituted with one $C_{1-6}$alkylcarbonyl, or
(b) phenyl substituted with one substituent selected from the group consisting of $C_{1-6}$alkylsulfonyl and $C_{3-8}$cycloalkylsulfonyl.

In a particularly preferred embodiment of formula [I-a] shown above,
$R^1$ is a hydrogen atom or methyl, and $R^2$ is a hydrogen atom or a fluorine atom,
provided that one of $R^1$ and $R^2$ is a hydrogen atom,
$R^3$ is a hydrogen atom; and
ring A is

(a) piperidin-4-yl substituted at 1-position with one acetyl, or
(b) phenyl substituted at 3-position with one substituent selected from the group consisting of methylsulfonyl and cyclopropylsulfonyl.

One preferred embodiment of the compound of the present invention is a compound represented by formula [I'-b] shown below or a pharmaceutically acceptable salt thereof.

[Formula 32]

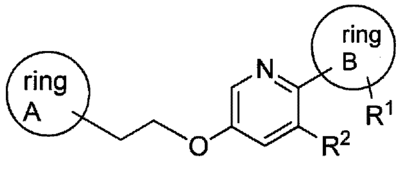

[I'-b]

Here, preferred embodiments of the structure represented by formula [III], $R^1$, $R^2$, and ring A are as described above.
In a more preferred embodiment of formula [I'-b],
the structure represented by formula [III] shown below

[Formula 33]

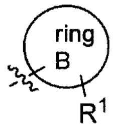

[III]

is the structure represented by formula [VI] shown below:

[Formula 34]

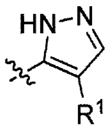

[VI]

wherein

$R^1$ is a hydrogen atom, a chlorine atom, or methyl;
$R^2$ is a hydrogen atom or a fluorine atom; and
ring A is
piperidin-4-yl, wherein the piperidin-4-yl is substituted with one $C_{1-6}$alkoxycarbonyl, or 4- to 6-membered saturated oxygen-containing heterocyclyl.

In a further preferred embodiment of formula [I'-b], the structure represented by formula [III] show below

[Formula 35]

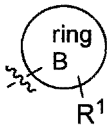

[III]

is the structure represented by formula [VI] shown below:

[Formula 36]

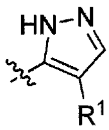

[VI]

wherein

$R^1$ is a hydrogen atom or methyl;
$R^2$ is a hydrogen atom; and
ring A is piperidin-4-yl substituted with one $C_{1-6}$alkoxycarbonyl.

In a particularly preferred embodiment of formula [I'-b] shown above, the structure represented by formula [III] shown below:

[Formula 37]

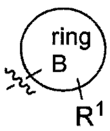

[III]

is the structure represented by formula [VI] shown below:

[Formula 38]

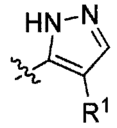

[VI]

wherein

$R^1$ is a hydrogen atom or methyl;
$R^2$ is a hydrogen atom; and
ring A is piperidin-4-yl substituted at 1-position with one methoxycarbonyl.

Another preferred embodiment of the compound of the present invention is a compound represented by formula [I-b] shown below or a pharmaceutically acceptable salt thereof.

[Formula 39]

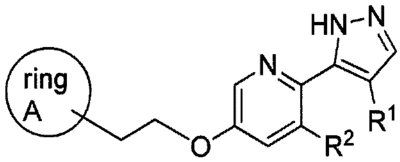

[I-b]

Here, preferred embodiments of $R^1$, $R^2$, and ring A are as described above.
In a more preferred embodiment of formula [I-b],
$R^1$ is a hydrogen atom or methyl;
$R^2$ is a hydrogen atom or a fluorine atom; and
ring A is piperidin-4-yl, wherein the piperidin-4-yl is substituted with one $C_{1-6}$alkoxycarbonyl.
In a further preferred embodiment of formula [I-b],
$R^1$ is a hydrogen atom or methyl;
$R^2$ is a hydrogen atom; and
ring A is piperidin-4-yl substituted with one $C_{1-6}$alkoxycarbonyl.
In a particularly preferred embodiment of formula [I-b] shown above,
$R^1$ is a hydrogen atom or methyl;
$R^2$ is a hydrogen atom; and
ring A is piperidin-4-yl substituted at 1-position with one methoxycarbonyl.
One preferred embodiment of the compound of the present invention is a compound represented by formula [I'-c] shown below or a pharmaceutically acceptable salt thereof.

[Formula 40]

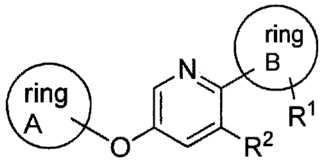

[I'-c]

Here, preferred embodiments of the structure represented by formula [III], $R^1$, $R^2$, and ring A are as described above.
Another preferred embodiment of the compound of the present invention is a compound represented by formula [I-c] shown below or a pharmaceutically acceptable salt thereof.

[Formula 41]

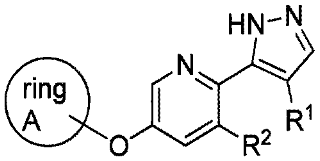

[I-c]

Here, preferred embodiments of $R^1$, $R^2$, and ring A are as described above.

Another preferred embodiment of the compound of the present invention is a compound represented by formula [I'-d] shown below or a pharmaceutically acceptable salt thereof.

[Formula 42]

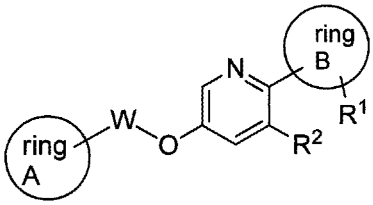

[I'-d]

Here, preferred embodiments of the structure represented by formula [III], $R^1$, $R^2$, W, and ring A are as described above.

In a more preferred embodiment of formula [I'-d] shown above,
the structure represented by formula [III] shown below

[Formula 43]

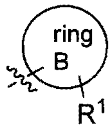

[III]

is the structure represented by formula [VI] shown below:

[Formula 44]

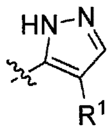

[VI]

wherein

$R^1$ is a hydrogen atom or methyl;
$R^2$ is a hydrogen atom or a fluorine atom;
W is $C_{1-2}$alkanediyl; and
ring A is

(a) piperidin-4-yl substituted with one substituent selected from the group consisting of $C_{1-6}$alkylcarbonyl and $C_{1-6}$alkoxycarbonyl, or
(b) phenyl substituted with one substituent selected from the group consisting of $C_{1-6}$alkylsulfonyl and $C_{3-8}$cycloalkylsulfonyl.

In a further preferred embodiment of formula [I'-d] shown above,
the structure represented by formula [III] shown below:

[Formula 45]

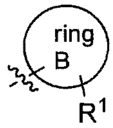
[III]

is the structure represented by formula [VI] shown below:

[Formula 46]

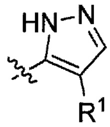
[VI]

wherein

$R^1$ is a hydrogen atom or methyl, and
$R^2$ is a hydrogen atom or a fluorine atom,
provided that one of $R^1$ and $R^2$ is a hydrogen atom;
W is methanediyl or ethane-1,2-diyl; and
ring A is

(a) piperidin-4-yl substituted at 1-position with one substituent selected from the group consisting of acetyl and methoxycarbonyl, or
(b) phenyl substituted at 3-position with one substituent selected from the group consisting of methylsulfonyl and cyclopropylsulfonyl.

Another preferred embodiment of the compound of the present invention is a compound represented by formula [I-d] shown below or a pharmaceutically acceptable salt thereof.

[Formula 47]

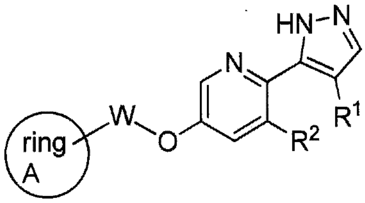
[I-d]

Here, preferred embodiments of $R^1$, $R^2$, W, and ring A are as described above.
In a more preferred embodiment of formula [I-d] shown below,
$R^1$ is a hydrogen atom or methyl;
$R^2$ is a hydrogen atom or a fluorine atom;
W is $C_{1-2}$alkanediyl; and
ring A is

(a) piperidin-4-yl substituted with one substituent selected from the group consisting of $C_{1-6}$alkylcarbonyl and $C_{1-6}$alkoxycarbonyl, or
(b) phenyl substituted with one substituent selected from the group consisting of $C_{1-6}$alkylsulfonyl and $C_{3-8}$cycloalkylsulfonyl.

In a further preferred embodiment of formula [I-d] shown above,
$R^1$ is a hydrogen atom or methyl, and
$R^2$ is a hydrogen atom or a fluorine atom,
provided that one of $R^1$ and $R^2$ is a hydrogen atom;

W is methanediyl or ethane-1,2-diyl; and
ring A is

(a) piperidin-4-yl substituted at 1-position with one substituent selected from the group consisting of acetyl and methoxycarbonyl, or
(b) phenyl substituted at 3-position with one substituent selected from the group consisting of methylsulfonyl and cyclopropylsulfonyl.

In particularly preferred embodiments of formula [I-d] or [I'-d] shown above, the following compounds may be mentioned:

[Formula 48]

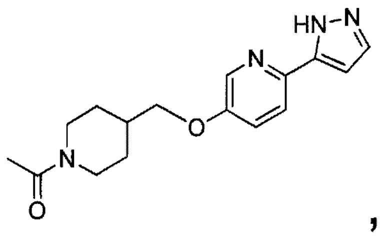

,

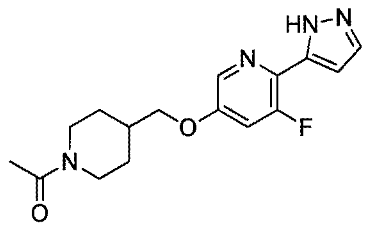

,

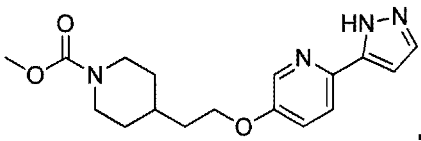

,

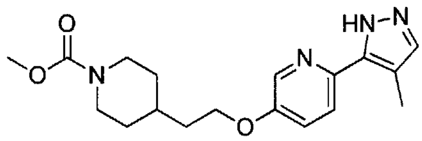

,

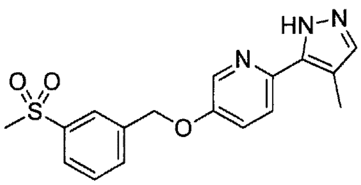

,

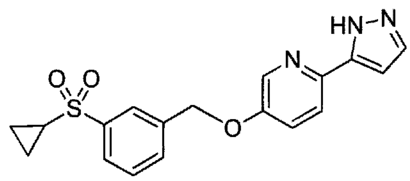

In another particularly preferred embodiment of formula [I-d] or [I'-d] shown above, the following compound may be mentioned:

[Formula 49]

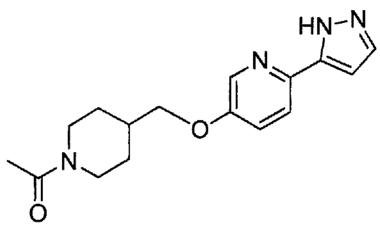

In another particularly preferred embodiment of formula [I-d] or [I'-d] shown above, the following compound may be mentioned:

[Formula 50]

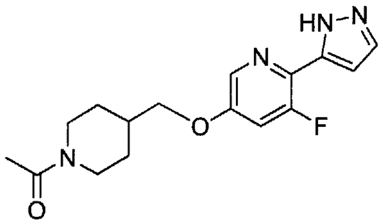

In another particularly preferred embodiment of formula [I-d] or [I'-d] shown above, the following compound may be mentioned:

[Formula 51]

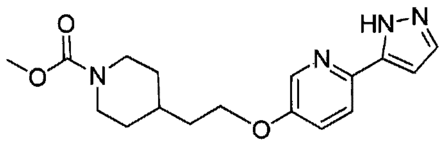

In another particularly preferred embodiment of formula [I-d] or [I'-d] shown above, the following compound may be mentioned:

[Formula 52]

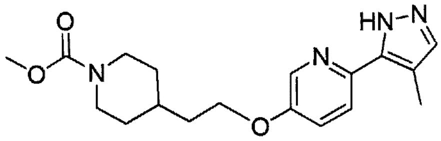

In another particularly preferred embodiment of formula [I-d] or [I'-d] shown above, the following compound may be mentioned:

[Formula 53]

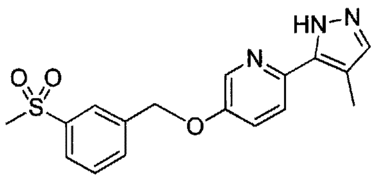

In another particularly preferred embodiment of formula [I-d] or [I'-d] shown above, the following compound may be mentioned:

[Formula 54]

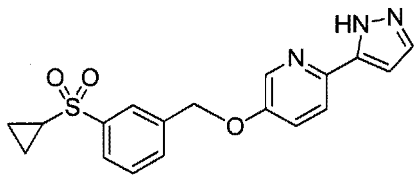

The compounds of the present invention are those the basic skeleton of which is pyridine substituted with an azole such as pyrazolyl, and pharmaceutically acceptable salt of such compounds may also be used.

The compounds of the present invention also include tautomers. To give an example of tautomersism, the structure represented by formula [III] shown below:

[Formula 55]

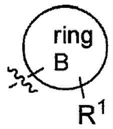

[III]

assumes the structure represented by formula [VI] shown below:

[Formula 56]

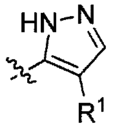

[VI]

to form a compound (hereinafter referred to as compound [I]) and a tautomer thereof (hereinafter referred to as a compound [I-α]), both being shown below.

[Formula 57]

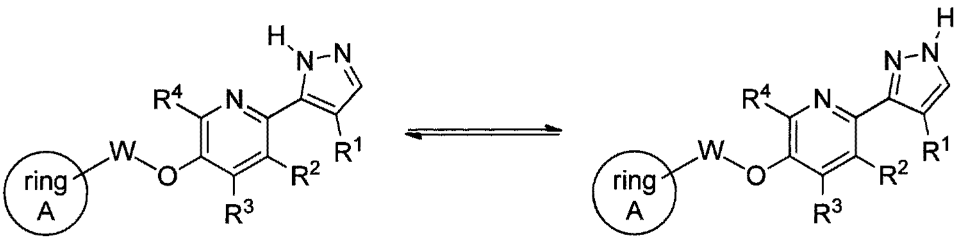

[I]

[I−α]

Examples of the pharmaceutically acceptable salt include: acid addition salts such as mineral acid salts such as hydrochloride, hydrobromide, hydroiodide, phosphate, sulfate, and nitrate, sulfonic acid salts such as methanesulfonate, ethanesulfonate, benzenesulfonate, p-toluenesulfonate, and trifluoromethanesulfonate, and organic acid salts such as oxalate, tartrate, citrate, maleate, succinate, acetate, trifluoroacetate, benzoate, mandelate, ascorbate, lactate, gluconate, and malate; amino acid salts such as glycine salt, lysine salt, arginine salt, ornithine salt, glutamate, and aspartate; or inorganic salts such as lithium salt, sodium salt, potassium salt, calcium salt, and magnesium salt or salts formed with organic bases, such as ammonium salt, triethylamine salt, diisopropylamine salt, and cyclohexylamine salt. Note that salts include hydrous salts.

The compound of the present invention may have an asymmetric center, and in that case, various optical isomers occur. Accordingly, the compound of the present invention may occur as a separate optically active substance (R) or (S), and as a racemate or an (RS) mixture. Also, when the compound has two or more asymmetric centers, diastereomers also result from the respective optical isomerisms. The compound of the present invention also includes mixtures containing all these forms in any proportions. For example, a diastereomer can be separated by methods well known to those skilled in the art such as a fractional crystallization method, and, also, an optically active substance can be obtained by techniques in organic chemistry that are well known for this purpose. Also, the compound of the present invention may occur as geometric isomers such as a cis and a trans form. Moreover, the compound of the present invention has tautomerism and occurs as various tautomers. The compound of the present invention also includes those isomers as well as mixtures containing those isomers in any proportions.

Moreover, when the compound of the present invention or a salt thereof forms a hydrate or a solvate, these are also included within the scope of the compound of the present invention or a salt thereof.

The 20-HETE producing enzymes refer to cytochrome P450 4A11 and 4F2 that catalyze hydroxylation at ω-position of arachidonic acid to produce 20-HETE using arachidonic acid as a substrate.

Now, as already described above, 20-HETE displays diverse functions in a living body and is involved in the onset of polycystic kidney disease and the pathologies of various cerebrovascular diseases, renal diseases, cardiovascular diseases and the like.

Accordingly, by inhibiting the 20-HETE producing enzymes, it is possible to prevent or ameliorate polycystic

kidney disease, diseases associated with polycystic kidney disease, and symptoms associated with polycystic kidney disease. Also, it is possible to prevent or ameliorate hypertension, cerebrovascular diseases, ischemic heart diseases, chronic renal failure, arteriosclerosis, fatty liver, and cancer.

The compound of the present invention acts to inhibit the 20-HETE producing enzymes. Thus, the compound of the present invention can be used as a 20-HETE producing enzyme inhibitor or an active ingredient of a prophylactic or ameliorating agent for polycystic kidney disease.

Also, it is possible to use the compound of the present invention as an active ingredient of a prophylactic or ameliorating agent for hypertension, cerebrovascular diseases, ischemic heart diseases, chronic renal failure, arteriosclerosis, fatty liver, and cancer.

Here, "polycystic kidney disease" includes "autosomal dominant polycystic kidney disease" in which a great number of cysts progressively develop and increase in both kidneys due to genetic mutation, and "autosomal recessive polycystic kidney disease". Examples of "diseases associated with polycystic kidney disease" include chronic renal failure, hypertension, vascular disorders, hepatic and pancreatic cysts, urinary tract infections, hepatobiliary infections, urolithiasis, and the like. Also, "symptoms associated with polycystic kidney disease" include pain, hematuria, and abdominal distention.

The action of the compound of the present invention for inhibiting the 20-HETE producing enzymes can be evaluated by known procedures such as the method described in the following Test Examples of the present specification.

Concerning the pharmaceutical according to the present invention, the contained inventive compound, i.e., the compound inhibiting the 20-HETE producing enzymes, or a pharmaceutically acceptable salt thereof, can be administered either alone or in combination with a pharmaceutically or pharmacologically acceptable additive.

Additives that can be used include excipients or diluents in common use, and if necessary, binders, disintegrants, lubricants, coating agents, sugar coating agents, pH adjusters, solubilizers, or aqueous or nonaqueous solvents that are in general use. Specific examples include water, lactose, dextrose, fructose, sucrose, sorbitol, mannitol, polyethylene glycol, propylene glycol, starch, cornstarch, gum, gelatin, alginate, calcium silicate, calcium phosphate, cellulose, water syrup, methylcellulose, polyvinylpyrrolidone, alkylparahydroxybenzoate, talc, stearic acid, magnesium stearate, agar, pectin, gum arabic, glycerin, sesame oil, olive oil, soybean oil cacao butter, ethylene glycol, low viscosity hydroxypropylcellulose (HPC-L), microcrystalline cellulose, carboxymethylcellulose (CMC), sodium carboxymethylcellulose (CMC-Na), and other commonly used additives.

The pharmaceutical according to the present invention may be in any form selected from a solid composition, a liquid composition, and other compositions, and an optimum form is selected as necessary.

The pharmaceutical according to the present invention can be produced by adding the above-mentioned additives to the compound of the present invention and preparing a tablet, pill, capsule, granule, dust, powder, liquid, emulsion, suspension, injection, or the like by a commonly used formulating technique.

Also, the pharmaceutical according to the present invention can be formulated by forming a clathrate from the compound of the present invention and α, β, or γ-cyclodextrin or methylated cyclodextrin or the like.

The pharmaceutical according to the present invention can be a single preparation (a combination drug) containing the compound of the present invention and concomitantly usable compounds, or two or more preparations (concomitant drugs) obtained by separately formulating the respective compounds.

When these compounds are separately formulated as two or more preparations, the individual preparations can be administered simultaneously or at certain time intervals. In the latter case, whichever may be administered earlier. The two or more preparations can each be administered a different number of times a day. Also, the two or more preparations can be administered through different routes as well.

When these compounds are separately formulated as two preparations, they may be administered simultaneously or at extremely short intervals, and in such a case, it is preferred that a package insert, a sales brochure, or the like of a commercially available pharmaceutical state to the effect that the preparations are used in combination.

It is also preferred to formulate these active ingredients separately and form a kit composed of two preparations.

When the compound of the present invention is used as a 20-HETE producing enzyme inhibitor or the like, the compound of the present invention may be orally administered as it is. Alternatively, the compound of the present invention may be orally administered in the form of an agent containing the compound as an active ingredient.

When the compound of the present invention is used as a prophylactic or ameliorating agent or the like for polycystic kidney disease, the compound of the present invention may be orally administered as it is. Alternatively, the compound of the present invention may be orally administered in the form of an agent containing the compound as an active ingredient.

The dosage of the compound of the present invention varies with the subject to which it is administered, the route of administration, the disease to be treated, the symptoms, and the like; take, for example, the case of oral administration to an adult patient, and the dosage is normally 0.1 mg to 1000 mg, preferably 1 mg to 200 mg, as a single dose and this dosage is desirably administered 1 to 3 times a day or once every 2 to 3 days.

Examples of producing preparations of the compound of the present invention are described below.

Preparation Example 1

Granules containing the following ingredients are produced.

Ingredients: Compound represented by formula [I] or pharmaceutically acceptable salt thereof, lactose, cornstarch, HPC-L.

The compound represented by formula [I] or a pharmaceutically acceptable salt thereof and lactose are sieved. Cornstarch is sieved. These are mixed in a mixer. An aqueous solution of HPC-L is added to the mixed powder, and the mixture is kneaded, granulated (extrusion-granulated), and then dried. The resulting dried granules are passed through a vibrating sieve to give granules.

Preparation Example 2

A powder for encapsulation containing the following ingredients is produced.

Ingredients: Compound represented by formula [I] or pharmaceutically acceptable salt thereof, lactose, cornstarch, magnesium stearate.

The compound represented by formula [I] or a pharmaceutically acceptable salt thereof and lactose are sieved. Cornstarch is sieved. These and magnesium stearate are mixed in a mixer to give a powder. Capsules can be filled with the resulting powder.

Preparation Example 3

Granules for encapsulation containing the following ingredients are produced.

Ingredients: Compound represented by formula [I] or pharmaceutically acceptable salt thereof, lactose, cornstarch, HPC-L.

The compound represented by formula [I] or a pharmaceutically acceptable salt thereof and lactose are sieved. Cornstarch is sieved. These are mixed in a mixer. An aqueous solution of HPC-L is added to the mixed powder, and the mixture is kneaded, granulated, and then dried. The resulting dried granules are passed through a vibrating sieve for classification to give granules. Capsules can be filled with the resulting granules.

Preparation Example 4

A tablet containing the following ingredients is produced.

Ingredients: Compound represented by formula [I] or pharmaceutically acceptable salt thereof, lactose, microcrystalline cellulose, magnesium stearate, CMC-Na.

The compound represented by formula [I] or a pharmaceutically acceptable salt thereof, lactose, microcrystalline cellulose, and CMC-Na are sieved and mixed. Magnesium stearate is added to the mixed powder to give a mixed powder for pharmaceutical preparation. This mixed powder is directly pressed to give a tablet.

Hereinafter, the production method for compound [I'] according to the present invention will be described in detail, but the production method is not particularly limited to the examples given below. The solvent which is used in the reaction may be any solvent as long as it does not interfere with the respective reactions, and it is not particularly limited to the following description.

Compound [I'] of the present invention can be produced by methods known per se, for example, production methods 1 to 9, 11 and 18 to 32 shown below, or modifications thereof.

In addition, in the production of compound [I'] of the present invention, the order of the respective steps in each production method can be appropriately changed.

In each of the following production methods, the starting compound may be used in a salt form thereof; and examples of the salt include the previously described "pharmaceutically acceptable salts".

Among compound [I'] of the present invention, methods for producing the compound (compound [I]) in which the structure represented by the following formula [III] is a structure of the following formula [VI] are shown in production methods 1 to 9, and 11; and methods for producing compound [I'] of the present invention are shown in production methods 18 to 32.

[Formula 58]

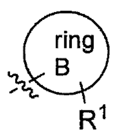
[III]

[Formula 59]

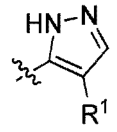

[VI]

Compound [1-e] which is an intermediate in the production of compound [I] of the present invention can be produced, for example, by the following production method 1 or modifications thereof.

Production method 1:

[Formula 60]

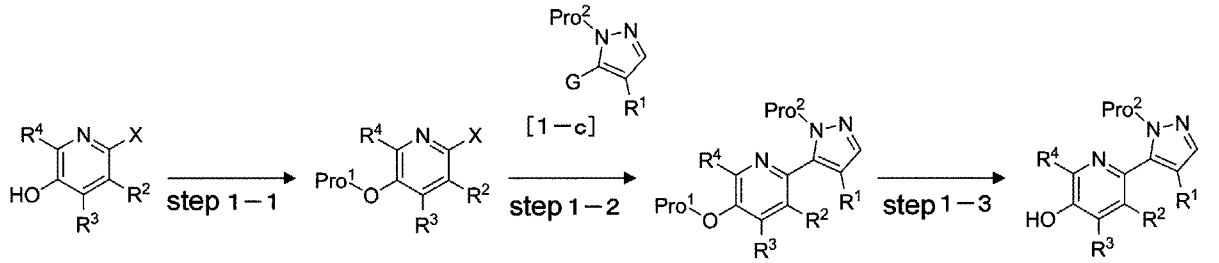


[1−a] [1−b] [1−d] [1−e]

[In the scheme,

$R^1$, $R^2$, $R^3$ and $R^4$ are the same as defined above;
X represents a chlorine atom, a bromine atom or an iodine atom; and
G represents boronic acid, a boronic acid ester, or an N-methyliminodiacetic acid (MIDA) boronate ester; and
$Pro^1$ represents a protecting group for a hydroxy group, as exemplified by (i) benzyl, 4-methoxybenzyl and the like (protecting groups which form a benzyl ether structure together with the hydroxy group, and herein also referred to as "benzyl ether-based protecting group"), (ii) methoxymethyl, tetrahydropyranyl and the like (protecting groups which form an acetal structure together with the hydroxy group, and herein also referred to as "acetal-based protecting group"), (iii) trimethylsilyl, triisopropylsilyl, tert-butyldimethylsilyl and the like (protecting groups which form a silyl ether structure together with the hydroxy group, and herein also referred to as "silyl ether-based protecting group"); and
$Pro^2$ represents a protecting group for azoles which are typified by pyrazolyl, for example, tetrahydropyranyl, triphenylmethyl, benzyl, and tert-butyl.]

[Step 1-1]

This step is a method of producing compound [1-b] by protecting the hydroxy group of compound [1-a] with the protecting group $Pro^1$.

This reaction can be carried out by the methods described in the literature (Protective Groups in Organic Synthesis, 4th edition, 2007, edited by G. M. Wuts and T. W. Greene), or modifications thereof.

[Step 1-2]

This step is a method of producing compound [1-d] by reacting compound [1-b] with compound [1-c].

This reaction is a so-called Suzuki-Miyaura coupling reaction that can be carried out by a process which is described in the literature (Tetrahedron Letters, Vol. 20, page 3437, 1979, Chemical Reviews, Volume 95, page 2457, 1995) in the presence of a palladium catalyst and a base, or a process pursuant thereto.

The amount of compound [1-c] which is used in the present reaction is 1 to 5 equivalents, and preferably 1 to 3 equivalents, with respect to 1 equivalent of compound [1-b].

Examples of the palladium catalysts include

tetrakis(triphenylphosphine)palladium(0), a [1,1'-bis(diphenylphosphino)ferrocene]palladium(II)dichloride dichloromethane adduct, and bis(triphenylphosphine)palladium(II)dichloride. The amount of the palladium catalyst to be used is usually 0.001 to 0.5 equivalents, and preferably 0.001 to 0.3 equivalents, with respect to 1 equivalent of compound [1-b].

Examples of the base include: alkali metal carbonates such as potassium carbonate, cesium carbonate and sodium carbonate, or aqueous solutions thereof; potassium fluoride; cesium fluoride; and triethylamine. The amount of the base to be used is usually 1 to 5 equivalents, and preferably 1 to 3 equivalents, with respect to 1 equivalent of compound [1-b].

In addition, copper salts such as copper acetate may also be used as additives.

Examples of the reaction solvent include solvents that do not interfere with reactions, such as N,N-dimethylformamide, dimethylsulfoxide, toluene, 1,4-dioxane, tetrahydrofuran, 1,2-dimethoxyethane and ethanol; and these solvents may be mixed with each other at an appropriate ratio and used.

These reactions can be carried out usually at room temperature to reflux temperature for 1 to 24 hours, and can be also carried out under microwave irradiation.

As described above, as compound [I'] of the present invention includes a tautomer thereof, compound [1-d] and the like, which are substituted with protecting group $Pro^2$ of pyrazolyl, may include an isomer thereof. As examples of the isomers, compound [1-d] and its isomer [1-d-α] are shown below.

[Formula 61]

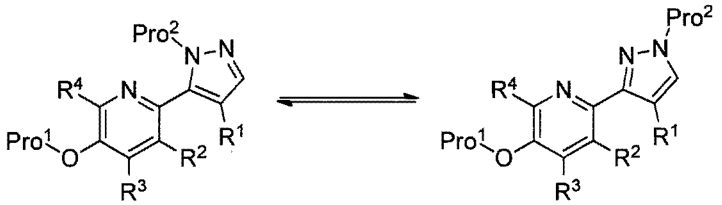

[1-d] [1-d-α]

[Step 1-3]

This step is a method of producing compound [1-e] by deprotecting the hydroxy group of compound [1-d] by removing protecting group $Pro^1$.

(i) When $Pro^1$ is a benzyl ether-based protecting group such as benzyl and 4-methoxybenzyl, the present reaction can be carried out in a solvent which does not interfere with the reaction, in the presence of a metal catalyst and a hydrogen source.
Examples of the metal catalyst which is used in the present reaction include palladium-carbon and palladium hydroxide-carbon. The amount of the metal catalyst to be used is 0.001 to 1 equivalent and preferably 0.01 to 0.5 equivalents, with respect to 1 equivalent of compound [1-d].
A hydrogen pressure which is used in the present reaction is ordinary pressure to 10 atm, and preferably ordinary pressure to 4 atm.
Examples of the solvent which is used in the present reaction include methanol, ethanol, water, tetrahydrofuran and ethyl acetate, and these solvents may be mixed with each other at an appropriate ratio and used.
The present reaction can be carried out usually at room temperature to reflux temperature for 1 to 24 hours.
(ii) When $Pro^1$ is an acetal-based protecting group such as methoxymethyl and tetrahydropyranyl, the present reaction can be carried out in a solvent which does not interfere with the reaction in the presence of an acid.
Examples of the acid which is used in the present reaction include hydrochloric acid, acetic acid and trifluoroacetic acid. The amount of the acid to be used is 1 to 5 equivalents and preferably 1 to 3 equivalents, with respect to 1 equivalent of compound [1-d].
Examples of the solvent which is used in the present reaction include solvents which do not interfere with reactions, such as methanol, ethanol, water, methylene chloride and chloroform; and these solvents may be mixed with each other at an appropriate ratio and used.
The present reaction can be carried out usually at room temperature to reflux temperature for 1 to 24 hours.
(iii) When $Pro^1$ is a silyl ether-based protecting group such as trimethylsilyl, triisopropylsilyl and tert-butyldimethylsilyl, the present reaction can be carried out in a solvent which does not interfere with the reaction in the presence of an acid.

Examples of the acid which is used in the present reaction include hydrochloric acid, acetic acid and trifluoroacetic acid. The amount of the acid to be used is 1 to 5 equivalents and preferably 1 to 3 equivalents, with respect to 1

equivalent of compound [1-d].

Examples of the solvent which is used in the present reaction include solvents that do not interfere with reactions, such as tetrahydrofuran, methanol, ethanol and water; and these solvents may be mixed with each other at an appropriate ratio and used.

The present reaction can be carried out usually at room temperature to reflux temperature for 1 to 24 hours.

In addition, the present reaction can also be carried out in a solvent which does not interfere with the reaction in the presence of a fluoride ion.

Examples of the fluoride ion source which is used in the present reaction include potassium fluoride and tetrabutylammonium fluoride. The amount of the fluoride ion source to be used is 1 to 5 equivalents and preferably 1 to 3 equivalents, with respect to 1 equivalent of compound [1-d].

Examples of the solvent which is used in the present reaction include solvents that do not interfere with reactions, such as tetrahydrofuran, N,N-dimethylformamide, methanol and ethanol; and these solvents may be mixed with each other at an appropriate ratio and used.

The present reaction can be carried out usually at room temperature to reflux temperature for 1 to 24 hours.

Compound [1-e] thus obtained can be isolated and purified by known separation and purification means such as concentration, concentration under reduced pressure, reprecipitation, solvent extraction, crystallization and chromatography.

Incidentally, compounds [1-a] and [1-c] which are used as starting compounds in the above described production method 1 can be produced by a method known per se, or can be obtained by purchasing commercial products.

Compounds [2-c], [3-c] and [4-f] which are compound [I] of the present invention can be produced, for example, by the following production methods 2 - 4 or a method pursuant thereto.

Production method 2:

[Formula 62]

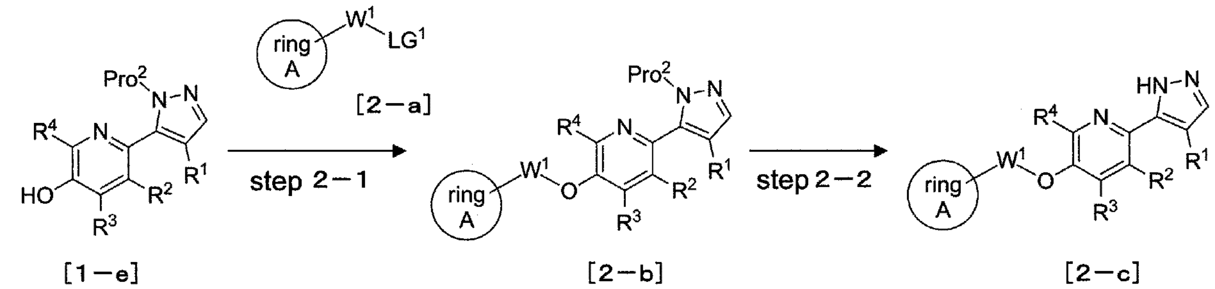


[In the scheme,

$R^1$, $R^2$, $R^3$, $R^4$, ring A, $Pro^2$ are the same as defined above;
$W^1$ represents $C_{1-3}$ alkanediyl; and
$LG^1$ represents a hydroxy group or a leaving group;
the "leaving group" represented by $LG^1$ refers to, for example, a halogen atom such as a chlorine atom and a bromine atom, $C_{1-6}$ alkylsulfonyloxy such as methanesulfonyloxy, or arylsulfonyloxy such as p-toluenesulfonyloxy.]

[Step 2-1]

This step is a method of producing compound [2-b] by reacting compound [1-e] with compound [2-a].

(i) When $LG^1$ in compound [2-a] is a hydroxy group, the present reaction can be carried out by a known method, i.e., the so-called "Mitsunobu reaction" (page 1, Synthesis, 1981).
The amount of compound [2-a] which is used in the present reaction is 1 to 5 equivalents, and preferably 1 to 3 equivalents, with respect to 1 equivalent of compound [1-e].
Examples of the azo compound which is used in the present reaction include diethyl azodicarboxylate, diisopropyl azodicarboxylate and 1,1'-azobis(N,N-dimethylformamide). The amount of the azo compound to be used is 1 to 5 equivalents, and preferably 1 to 3 equivalents, with respect to 1 equivalent of compound [1-e].
Examples of the phosphine compound which is used in the present reaction include triphenylphosphine and tributylphosphine. The amount of the phosphine compound to be used is 1 to 5 equivalents, and preferably 1 to 3 equivalents, with respect to 1 equivalent of compound [1-e].
Examples of the solvent which is used in the present reaction include solvents that do not interfere with reactions, such as tetrahydrofuran, 1,4-dioxane, diethyl ether, chloroform, dichloromethane, toluene, N,N-dimethylformamide and dimethyl sulfoxide; and these solvents may be mixed with each other at an appropriate ratio and used.
The present reaction can be carried out usually at room temperature to reflux temperature for 1 to 24 hours.
The present reaction may also be carried out by the method described in Tetrahedron Letters, Vol. 36, page 2531, 1995 and Tetrahedron Letters, Vol. 37, page 2463, 1996.
Examples of the reagent which is used in the present reaction include cyanomethylenetrimethylphosphorane and cyanomethylenetributylphosphorane. The amount of the reagent to be used is 1 to 5 equivalents, and preferably 1 to 3 equivalents, with respect to 1 equivalent of compound [1-e].
Examples of the solvent to be used in the present reaction are the same as those used in the Mitsunobu reaction described above.
The present reaction can be carried out usually at room temperature to reflux temperature for 1 to 24 hours.
(ii) When $LG^1$ in compound [2-a] is a leaving group, the present reaction can be carried out in the presence of a base.

The amount of compound [2-a] which is used in the present reaction is 1 to 5 equivalents, and preferably 1 to 3 equivalents, with respect to 1 equivalent of compound [1-e].
Examples of the base which is used in the present reaction include amines such as triethylamine, N,N-diisopropylethylamine and 1,8-diazabicyclo[4,3,0]undec-7-ene, alkali metal hydrides such as sodium hydride, alkali metal hydroxides such as potassium hydroxide, alkali metal carbonates such as cesium carbonate, potassium carbonate and sodium carbonate and alkoxy alkali metal such as potassium tert-butoxide. The amount of the base to be used is 1 to 5 equivalents, and preferably 1 to 3 equivalents, with respect to 1 equivalent of compound [1-e].
Examples of the solvent which is used in the present reaction include solvents that do not interfere with reactions, such as tetrahydrofuran, dimethyl sulfoxide, N,N-dimethylformamide, N, N-dimethylacetamide and N-methylpyrrolidone; and these solvents may be mixed with each other at an appropriate ratio and used.
The present reaction can be carried out usually at room temperature to reflux temperature for 1 to 24 hours.

[Step 2-2]

(i) This step is a method of producing compound [2-c] by deprotecting the pyrazolyl of compound [2-b] by removing protecting group $Pro^2$ under an acidic condition when $Pro^2$ is a protecting group represented by tetrahydropyranyl, triphenylmethyl and tert-butyl.
Examples of the acid which is used in the present reaction include hydrochloric acid, formic acid and trifluoroacetic acid. The amount of the acid to be used is 1 equivalent to the amount of the solvent, and preferably 1 to 10 equivalents, with respect to 1 equivalent of compound [2-b].
Examples of the solvent which is used in the present reaction include solvents which do not interfere with reactions, such as methanol, ethanol, tetrahydrofuran, water, ethyl acetate and 1,4-dioxane; and these solvents may be mixed with each other at an appropriate ratio and used.
The present reaction can be carried out usually at 0°C to reflux temperature for 1 to 24 hours.
(ii) When $Pro^2$ is a protecting group represented by benzyl, this step can be carried out by acting a base thereon with aerating with oxygen according to the method described in the literature (Tetrahedron Letters, Vol. 43, page 399, 2002)

Examples of the base which is used in the present reaction include alkoxy alkali metal such as potassium tert-butoxide. The amount of the base to be used is 2 to 20 equivalents, and preferably 5 to 15 equivalents, with respect to 1 equivalent of compound [2-b].
Examples of the solvent which is used in the present reaction include solvents which do not interfere with reactions, such as dimethyl sulfoxide, dimethylformamide, tetrahydrofuran and 1,4-dioxane; and these solvents may be mixed with each other at an appropriate ratio and used.
The present reaction can be carried out usually at 0°C to reflux temperature for 1 to 24 hours.
Compound [2-c] thus obtained can be isolated and purified by known separation and purification means such as concentration, concentration under reduced pressure, reprecipitation, solvent extraction, crystallization and chromatography.
Compounds [1-e] and [2-a] which are used as starting compounds in the above described production method 2 can be produced by the above production method 1, a method pursuant thereto, or a method known per se, or can

be obtained by purchasing commercial products.

Production method 3:

[Formula 63]

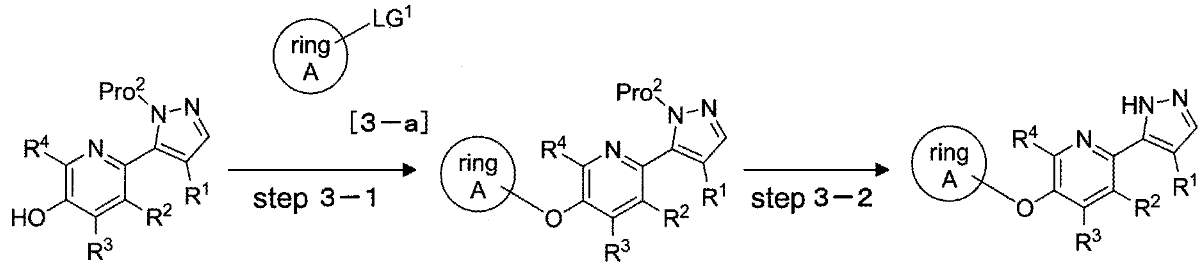


[1−e] [3−a] [3−b] [3−c]

[In the scheme, $R^1$, $R^2$, $R^3$, $R^4$, ring A, $LG^1$, $Pro^2$ are the same as defined above.]

[Step 3-1]

This step is a method of producing compound [3-b] by reacting compound [1-e] with compound [3-a].

(i) When ring A is (a) $C_{4-6}$ cycloalkyl, (b) 4- to 6-membered nitrogen-containing heterocyclyl, (c) a group represented by formula [II-2] above, (d) a group represented by formula [II-3] above or (e) a group represented by formula [II-4] above, the present reaction can be carried out by the method described in step 2-1 of production method 2 or a method pursuant thereto.

(ii) When ring A is (a) phenyl, (b) pyridyl, (c) naphthyl, (d) 2H-chromenyl, (e) quinolyl or (f) quinoxalyl, this reaction can be carried out by the method described in the literature (Tetrahedron, Vol. 40, page 1433, 1984), or a method pursuant thereto.

[Step 3-2]

This step is a method of producing compound [3-c] by deprotecting the pyrazolyl of compound [3-b] by removing protecting group $Pro^2$ under an acidic condition.

This reaction can be carried out by the method described in step 2-2 of production method 2 or a method pursuant thereto.

Compound [3-c] thus obtained can be isolated and purified by known separation and purification means such as concentration, concentration under reduced pressure, reprecipitation, solvent extraction, crystallization and chromatography.

Compounds [1-e] and [3-a] which are used as starting compounds in the above described production method 3 can be produced by the above production method 1, a method pursuant thereto, or a method known per se, or can be obtained by purchasing commercial products.

Production method 4:

[Formula 64]

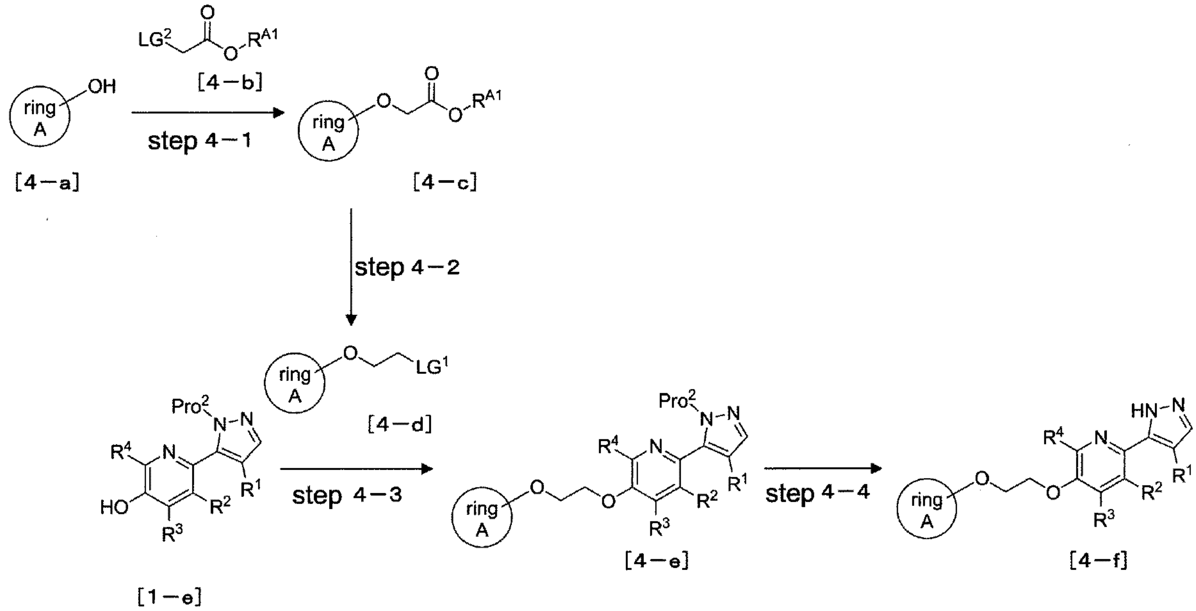


[In the scheme, $R^1$, $R^2$, $R^3$, $R^4$, ring A, $LG^1$, $Pro^2$ are the same as defined above;
$R^{A1}$ represents $C_{1-6}$ alkyl, $C_{3-8}$ alkyl or aryl; and
$LG^2$ represents a hydroxy group or a leaving group;
the "leaving group" represented by $LG^2$ refers to, for example, a halogen atom such as a chlorine atom and a bromine atom, $C_{1-6}$ alkylsulfonyloxy such as methanesulfonyloxy, or arylsulfonyloxy such as p-toluenesulfonyloxy.]

[Step 4-1]

This step is a method of producing compound [4-c] by reacting compound [4-a] with compound [4-b].
This reaction can be carried out in the presence of a base.
The amount of compound [4-b] which is used in the present reaction is 1 to 5 equivalents, and preferably 1 to 3 equivalents, with respect to 1 equivalent of compound [4-a].
Examples of the base which is used in the present reaction include amines such as triethylamine, N,N-diisopropylethylamine and 1,8-diazabicyclo[4,3,0]undec-7-ene, alkali metal hydrides such as sodium hydride, alkali metal hydroxides such as potassium hydroxide, alkali metal carbonates such as cesium carbonate, potassium carbonate and sodium carbonate and alkoxy alkali metals such as potassium tert-butoxide. The amount of the base to be used is 1 to 5 equivalents, and preferably 1 to 3 equivalents, with respect to 1 equivalent of compound [4-a].
Examples of the solvent which is used in the present reaction include solvents that do not interfere with reactions, such as tetrahydrofuran, dimethyl sulfoxide, N,N-dimethylformamide, N, N-dimethylacetamide and N-methylpyrrolidone; and these solvents may be mixed with each other at an appropriate ratio and used.
The present reaction can be carried out usually at room temperature to reflux temperature for 1 to 24 hours.

[Step 4-2]

This step is a method of producing compound [4-d] from compound [4-c].

(i) When $LG^1$ in compound [4-d] is a hydroxy group, the present reaction can be carried out by acting a reducing agent on compound [4-c].
Examples of the reducing agent used in the present reaction include lithium aluminum hydride, diisobutyl aluminum hydride, sodium boron hydride and diborane. The amount of the reducing agent to be used is 1 to 5 equivalents, and preferably 1 to 3 equivalents, with respect to 1 equivalent of compound [4-c].
Examples of the solvent which is used in the present reaction include solvents which do not interfere with reactions, such as diethyl ether, tetrahydrofuran, 1,2-dimethoxyethane, toluene and xylene; and these solvents may be mixed with each other at an appropriate ratio and used.
(ii) When $LG^1$ in compound [4-d] is a leaving group, the present reaction can be carried out by further converting the hydroxy group of the compound prepared in (i) of the present [step 4-2] to a leaving group.

The hydroxy group may be converted to a leaving group by a usual method. For example, compound [4-d] in which $LG^1$ is a leaving group may be prepared by the reaction of a sulfonate esterification reagent in the presence of (a) a halogenating agent or (b) a base in a solvent which does not interfere with the reaction.
Examples of (a) halogenating agent used in the present reaction include thionyl chloride and phosphoryl chloride. The amount of the halogenating agent to be used is 1 to 5 equivalents, and preferably 1 to 3 equivalents, with respect to 1 equivalent of the compound prepared in (i) of the present [Step 4-2].
Furthermore, instead of the above halogenating agent, a reagent which serves as a halogen source, such as N-chlorosuccinimide, N-bromosuccinimide, carbon tetrabromide or bromine may be used in combination with a phosphine reagent such as triphenyl phosphine.
The amount of the reagent which serves as a halogen source and the phosphine reagent to be used is 1 to 5 equivalents, and preferably 1 to 2 equivalents, with respect to 1 equivalent of the compound prepared in (i) of the present [Step 4-2].
Examples of the solvent which is used in the present reaction include solvents that do not interfere with reactions, such as chloroform and dichloromethane; and these solvents may be mixed with each other at an appropriate ratio and used.
The present reaction can be carried out usually at room temperature to reflux temperature for 1 to 24 hours.
Furthermore, examples of (b) sulfonate esterification reagent used in the present reaction include methanesulfonic acid chloride, trifluoromethanesulfonic acid chloride and p-toluenesulfonic acid chloride. The amount of the sulfonate esterification reagent to be used is 1 to 5 equivalents, and preferably 1 to 3 equivalents, with respect to 1 equivalent of the compound prepared in (i) of the present [Step 4-2].
Examples of the base which is used in the present reaction include triethylamine, N,N-diisopropylethylamine, pyridine and N,N-dimethylaminopyridine. The amount of the base to be used is 1 to 5 equivalents, and preferably 1 to 3 equivalents, with respect to 1 equivalent of the sulfonate esterification reagent to be used.
Examples of the solvent which is used in the present reaction include solvents that do not interfere with reactions, such as chloroform and dichloromethane; and these solvents may be mixed with each other at an appropriate ratio and used.
The present reaction can be carried out usually at room temperature to reflux temperature for 1 to 24 hours.

[Step 4-3]

This step is a method of producing compound [4-e] by reacting compound [1-e] with compound [4-d].
This reaction can be carried out by the method described in step 2-1 of production method 2 or a method pursuant thereto.

[Step 4-4]

This step is a method of producing compound [4-f] by deprotecting the pyrazolyl of compound [4-e] by removing protecting group $Pro^2$ under an acidic condition.
This reaction can be carried out by the method described in step 2-2 of production method 2 or a method pursuant thereto.
Compound [4-f] thus obtained can be isolated and purified by known separation and purification means such as concentration, concentration under reduced pressure, reprecipitation, solvent extraction, crystallization and chromatography.
Compounds [1-e], [4-a] and [4-b] which are used as starting compounds in the above described production method 4 can be produced by the above production method 1, a method pursuant thereto, or a method known per se, or can be obtained by purchasing commercial products.
Compounds [2-c], [3-c] and [4-f] which are compound [I] of the present invention can also be produced, for example, by the following production methods 5 - 7 or a method pursuant thereto.

Production method 5:

[Formula 65]

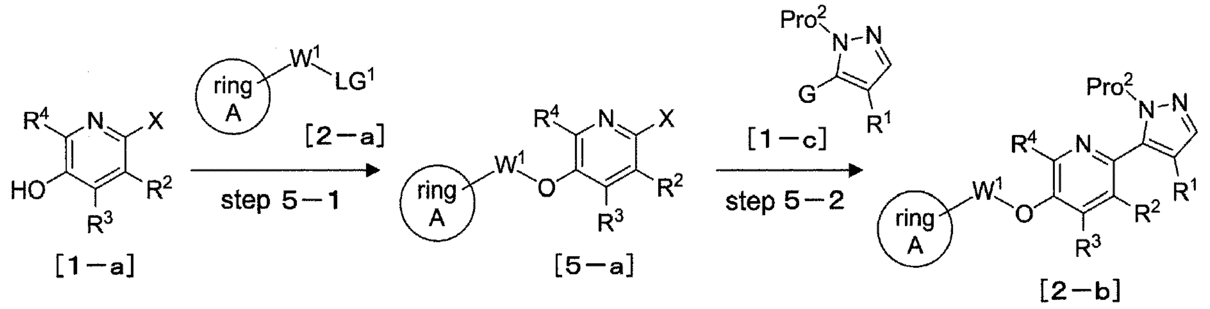


[In the scheme,
$R^1$, $R^2$, $R^3$, $R^4$, ring A, X, G, $W^1$, $LG^1$, $Pro^2$ are the same as defined above.]

[Step 5-1]

This step is a method of producing compound [5-a] by reacting compound [1-a] with compound [2-a].

This reaction can be carried out by the method described in step 2-1 of production method 2 or a method pursuant thereto.

[Step 5-2]

This step is a method of producing compound [2-b] by reacting compound [5-a] with compound [1-c].

This reaction can be carried out by the method described in step 1-2 of production method 1 or a method pursuant thereto.

Compound [2-c] may be derived from compound [2-b] thus obtained by the method described in step 2-2 of production method 2 or a method pursuant thereto.

Compounds [1-a], [2-a] and [1-c] which are used as starting compounds in the above described production method 5 can be produced by a method known per se, or can be obtained by purchasing commercial products.

Production method 6:

[Formula 66]

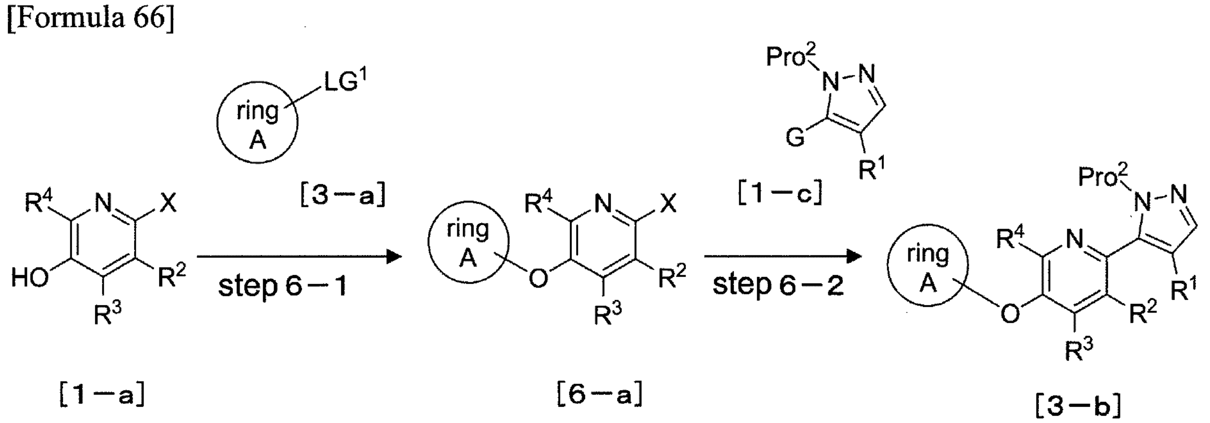


[In the scheme,
$R^1$, $R^2$, $R^3$, $R^4$, ring A, X, G, $LG^1$, $Pro^2$ are the same as defined above.]

[Step 6-1]

This step is a method of producing compound [6-a] by reacting compound [1-a] with compound [3-a].

This reaction can be carried out by the method described in step 3-1 of production method 3 or a method

pursuant thereto.

[Step 6-2]

This step is a method of producing compound [3-b] by reacting compound [6-a] with compound [1-c].
This reaction can be carried out by the method described in step 1-2 of production method 1 or a method pursuant thereto.
Compound [3-c] may be derived from compound [3-b] thus obtained by the method described in step 3-2 of production method 3 or a method pursuant thereto.
Compounds [1-a], [3-a] and [1-c] which are used as starting compounds in the above described production method 6 can be produced by a method known per se, or can be obtained by purchasing commercial products.

Production method 7:

[Formula 67]

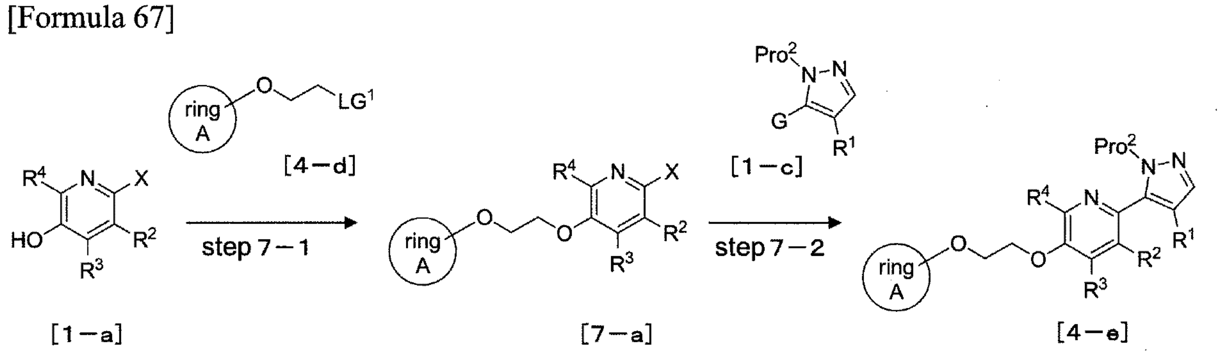


[1-a] [4-d] [7-a] [1-c] [4-e]

[In the scheme,
$R^1$, $R^2$, $R^3$, $R^4$, ring A, X, G, $LG^1$, $Pro^2$ are the same as defined above.]

[Step 7-1]

This step is a method of producing compound [7-a] by reacting compound [1-a] with compound [4-d].
This reaction can be carried out by the method described in step 4-3 of production method 4 or a method pursuant thereto.

[Step 7-2]

This step is a method of producing compound [4-e] by reacting compound [7-a] with compound [1-c].
This reaction can be carried out by the method described in step 1-2 of production method 1 or a method pursuant thereto.
Compound [4-f] may be derived from compound [4-e] thus obtained by the method described in step 4-4 of production method 4 or a method pursuant thereto.
Compounds [1-a], [4-d] and [1-c] which are used as starting compounds in the above described production method 7 can be produced by the above production method 4, a method pursuant thereto, or a method known per se, or can be obtained by purchasing commercial products.
Of compounds [I] of the present invention, compound [8-b] can be produced by the following production method 8 or a method pursuant thereto.

Production method 8:

[Formula 68]

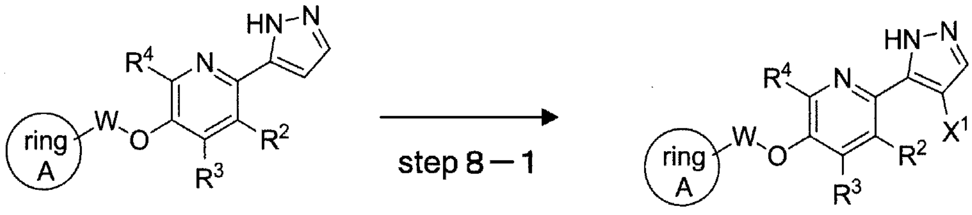


[8−a] [8−b]

[In the scheme,
$R^2$, $R^3$, $R^4$, W, ring A are the same as defined above and $X^1$ represents a halogen atom.]

[Step 8-1]

This step is a method of producing compound [8-b] by reacting compound [8-a] with a halogenating agent.

Examples of the halogenating agent used in the present reaction include Selectfluor (registered trademark), N-fluorobenzenesulfonimide (NFSI), N-chlorosuccinimide (NCS), N-bromosuccinimide (NBS) and N-iodosuccinimide (NIS). The amount of the reagent to be used is 1 to 5 equivalents, and preferably 1 to 3 equivalents, with respect to 1 equivalent of compound [8-a].

Examples of the solvent which is used in the present reaction include solvents that do not interfere with reactions, such as chloroform, dichloromethane, acetonitrile, ethyl acetate, N,N-dimethylformamide and water; and these solvents may be mixed with each other at an appropriate ratio and used.

The present reaction can be carried out usually at 0°C to reflux temperature for 1 to 48 hours.

Compound [8-a] which is used as a starting compound in the above described production method 8 can be produced by the above production methods 2 -7, a method pursuant thereto, or a method known per se.

Of compounds [I] of the present invention, compounds [9-e], [9-g], [9-j], [9-m] can be produced, for example, by the following production method 9 or a method pursuant thereto.

Production method 9:

[Formula 69]

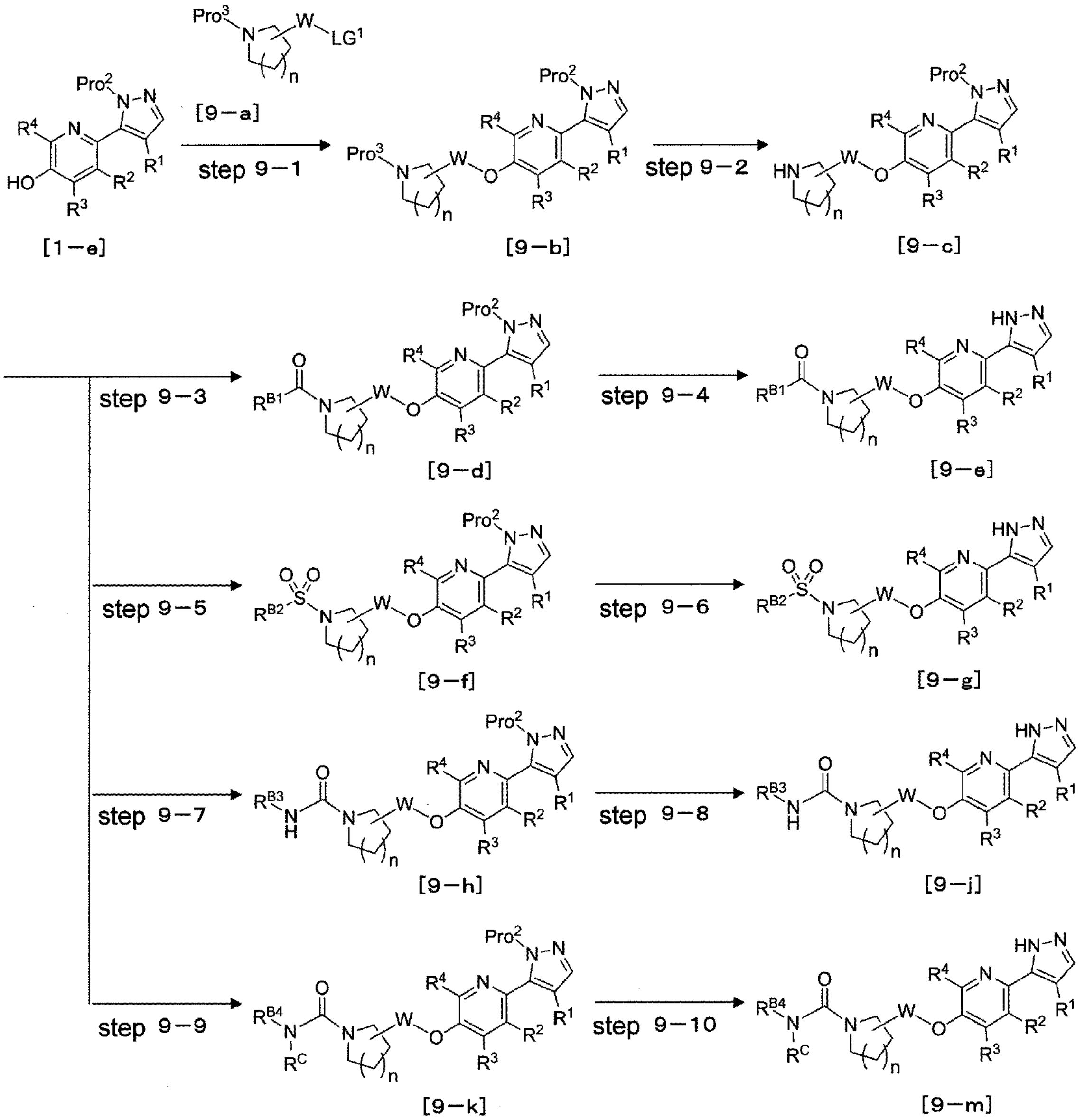


[In the scheme,

$R^1$, $R^2$, $R^3$, $R^4$, W, $LG^1$, $Pro^2$ are the same as defined above;
n represents an integer of 0 - 2;
$R^{B1}$, $R^{B2}$, $R^{B3}$, $R^{B4}$ independently represent $C_{1-6}$ alkyl, $C_{3-8}$ cycloalkyl, aryl, heteroaryl, heterocyclyl or $C_{1-6}$ alkoxy;
$R^c$ represents a hydrogen atom or $C_{1-6}$ alkyl; and
$Pro^3$ represents a protecting group of a nitrogen atom in 4- to 6-membered saturated nitrogen-containing heterocyclyl, e.g., (i) tert-butoxycarbonyl or (ii) benzyloxycarbonyl.]

[Step 9-1]

This step is a method of producing compound [9-b] by reacting compound [1-e] with compound [9-a].

This reaction can be carried out by the method described in step 2-1 of production method 2, step 3-1 of production method 3, step 4-3 of production method 4 or a method pursuant thereto.

[Step 9-2]

This step is a method of producing compound [9-c] by deprotecting the nitrogen atom in 4- to 6-membered saturated nitrogen-containing heterocyclyl of compound [9-b] by removing protecting group $Pro^3$.

(i) When protecting group $Pro^3$ is tert-butoxycarbonyl, the present reaction can be carried out in a solvent which does not interfere with the reaction, in the presence of an acid.
Examples of the reagent used in the present reaction include mineral acid such as hydrochloric acid and organic acid such as trifluoroacetic acid. The amount of the reagent to be used is 1 to 5 equivalents, and preferably 1 to 3 equivalents, with respect to 1 equivalent of compound [9-b].
Examples of the solvent which is used in the present reaction include methanol, ethanol, water, tetrahydrofuran and ethyl acetate; and these solvents may be mixed with each other at an appropriate ratio and used.
The present reaction can be carried out usually at room temperature to reflux temperature for 1 to 24 hours.
The present reaction can also be carried out in a solvent which does not interfere with the reaction in the presence of a Lewis acid.
Examples of the reagent used in the present reaction include trimethylsilyl trifluoromethanesulfonate and tert-butyldimethylsilyl trifluoromethanesulfonate. The amount of the reagent to be used is 1 to 5 equivalents, and preferably 1 to 3 equivalents, with respect to 1 equivalent of compound [9-b].
2,6-Lutidine may be used as an additive in the present reaction. The amount to be used is 1 to 10 equivalents, and preferably 2 to 5 equivalents, with respect to 1 equivalent of compound [9-b].
Examples of the solvent which is used in the present reaction include dichloromethane, chloroform and toluene; and these solvents may be mixed with each other at an appropriate ratio and used.
The present reaction can be carried out usually at -80°C to room temperature for 1 to 24 hours.
(ii) When protecting group $Pro^3$ is benzyloxycarbonyl, this step can be carried out in a solvent which does not interfere with the reaction, in the presence of a metal catalyst and a hydrogen source.

Examples of the metal catalyst which is used in the present reaction include palladium-carbon. The amount of the metal catalyst to be used is 0.1 to 1 equivalent and preferably 0.1 to 0.5 equivalents, with respect to 1 equivalent of compound [9-b].
A hydrogen pressure which is used in the present reaction is ordinary pressure to 10 atm, and preferably ordinary pressure to 4 atm.
Examples of the solvent which is used in the present reaction include methanol, ethanol, water, tetrahydrofuran and ethyl acetate; and these solvents may be mixed with each other at an appropriate ratio and used.
The present reaction can be carried out usually at room temperature to reflux temperature for 1 to 24 hours.

[Step 9-3]

This step is a method of producing compound [9-d] by reacting compound [9-c] with a corresponding carboxylic acid or acid chloride.

(i) When the reagent used in the present reaction is a carboxylic acid, the present reaction can be carried out by a known method, for example, by using a condensation agent in the presence or absence of a base and an additive.
The amount of the carboxylic acid used in the present reaction is 1 to 5 equivalents, and preferably 1 to 3 equivalents, with respect to 1 equivalent of compound [9-c].
Examples of the condensation agent include O-(7-azabenzotriazol-1-yl)-N,N,N',N'-tetramethyluronium hexafluorophosphate (HATU), O-benzotriazol-1-yl-N,N,N',N'-tetramethyluronium hexafluorophosphate (HBTU), 1-(3-dimethylaminopropyl)-3-ethylcarbodiimide hydrochloride (EDCI), dicyclohexylcarbodiimide (CDI), (1H-benzotriazol-1-yloxy)(tripyrrolidin-1-yl)phosphonium hexafluorophosphate (PyBOP), propylphosphonic anhydride (T3P) and 4-(4,6-dimethoxy-1,3,5-triazin-2-yl)-4-methylmorpholinium chloride (DMT-MM). The amount of the condensation agent to be used is 1 to 5 equivalents, and preferably 1 to 3 equivalents, with respect to 1 equivalent of compound [9-c].
Examples of the additive which is used in the present reaction include N-hydroxybenzotriazole monohydrate (HOBt) and N-hydroxysuccinimide. The amount of the additive to be used is 1 to 5 equivalents, and preferably 1 to 2 equivalents, with respect to 1 equivalent of compound [9-c].
Examples of the base which is used in the present reaction include N,N-diisopropylethylamine, triethylamine and pyridine. The amount of the base to be used is 1 to 5 equivalents, and preferably 1 to 2 equivalents, with respect to 1 equivalent of compound [9-c].
Examples of the solvent which is used in the present reaction include solvents that do not interfere with reactions, such as N,N-dimethylformamide, dichloromethane, chloroform, 1,2-dichloroethane, toluene and tetrahydrofuran;

and these solvents may be mixed with each other at an appropriate ratio and used.

The present reaction can be carried out usually at 0°C to reflux temperature for 1 to 24 hours.

(ii) When the reagent used in this step is acid chloride, the present reaction can be carried out by a known method, for example, in the presence of a base.

The amount of the acid chloride used in the present reaction is 1 to 5 equivalents, and preferably 1 to 3 equivalents, with respect to 1 equivalent of compound [9-c].

Examples of the base which is used in the present reaction include triethylamine, pyridine, N,N-dimethyl-4-aminopyridine and N,N-diisopropylethylamine. The amount of the base to be used is usually 1 to 5 equivalents, and preferably 1 to 3 equivalents, with respect to 1 equivalent of compound [9-c].

Examples of the solvent which is used in the present reaction include solvents that do not interfere with reactions, such as chloroform, dichloromethane, toluene, diethyl ether, tetrahydrofuran, ethyl acetate, N,N-dimethylformamide and acetonitrile; and these solvents may be mixed with each other at an appropriate ratio and used.

The present reaction can be carried out usually at 0°C to room temperature for 1 to 24 hours.

[Step 9-4]

This step is a method of producing compound [9-e] by deprotecting the pyrazolyl of compound [9-d] by removing protecting group $Pro^2$ under an acidic condition.

This reaction can be carried out by the method described in step 2-2 of production method 2 or a method pursuant thereto.

Compound [9-e] thus obtained can be isolated and purified by known separation and purification means such as concentration, concentration under reduced pressure, reprecipitation, solvent extraction, crystallization and chromatography.

[Step 9-5]

This step is a method of producing compound [9-f] by reacting compound [9-c] with the corresponding sulfonyl chloride.

The amount of the sulfonyl chloride used in the present reaction is 1 to 5 equivalents, and preferably 1 to 3 equivalents, with respect to 1 equivalent of compound [9-c].

Examples of the base which is used in the present reaction include triethylamine, pyridine, N,N-dimethyl-4-aminopyridine and N,N-diisopropylethylamine. The amount of the base to be used is usually 1 to 5 equivalents, and preferably 1 to 3 equivalents, with respect to 1 equivalent of compound [9-c].

Examples of the solvent which is used in the present reaction include solvents that do not interfere with reactions, such as chloroform, dichloromethane, toluene, diethyl ether, tetrahydrofuran, ethyl acetate, N,N-dimethylformamide and acetonitrile; and these solvents may be mixed with each other at an appropriate ratio and used.

The present reaction can be carried out usually at 0°C to room temperature for 1 to 24 hours.

[Step 9-6]

This step is a method of producing compound [9-g] by deprotecting the pyrazolyl of compound [9-f] by removing protecting group $Pro^2$ under an acidic condition.

This reaction can be carried out by the method described in step 2-2 of production method 2 or a method pursuant thereto.

Compound [9-g] thus obtained can be isolated and purified by known separation and purification means such as concentration, concentration under reduced pressure, reprecipitation, solvent extraction, crystallization and chromatography.

[Step 9-7]

This step is a method of producing compound [9-h] by reacting compound [9-c] with the corresponding isocyanate.

This step can be carried out in a solvent which does not interfere with the reaction, in the presence or absence of a base.

The amount of the isocyanate used in the present reaction is 1 to 5 equivalents, and preferably 1 to 3 equivalents, with respect to 1 equivalent of compound [9-c].

Examples of the base which is used in the present reaction include triethylamine, pyridine, N,N-dimethyl-4-aminopyridine and N,N-diisopropylethylamine. The amount of the base to be used is usually 1 to 5 equivalents, and preferably 1 to 3 equivalents, with respect to 1 equivalent of compound [9-c].

Examples of the solvent which is used in the present reaction include solvents that do not interfere with reactions, such as chloroform, dichloromethane, diethyl ether, tetrahydrofuran, ethyl acetate, N,N-dimethylformamide and dimethyl sulfoxide; and these solvents may be mixed with each other at an appropriate ratio and used.

The present reaction can be carried out usually at 0°C to room temperature for 1 to 24 hours.

[Step 9-8]

This step is a method of producing compound [9-j] by deprotecting the pyrazolyl of compound [9-h] by removing protecting group $Pro^2$ under an acidic condition.

This reaction can be carried out by the method described in step 2-2 of production method 2 or a method pursuant thereto.

Compound [9-j] thus obtained can be isolated and purified by known separation and purification means such as concentration, concentration under reduced pressure, reprecipitation, solvent extraction, crystallization and chromatography.

[Step 9-9]

This step is a method of producing compound [9-k] by reacting compound [9-c] with the corresponding amine.

This step can be carried out by acting 4-nitrophenyl chloroformate, dicyclohexylcarbodiimide (CDI), triphosgene, etc., thereon in a solvent that do not interfere with reactions, in the presence of a base.

The amount of the amine used in the present reaction is 1 to 5 equivalents, and preferably 1 to 3 equivalents, with respect to 1 equivalent of compound [9-c].

Examples of the base which is used in the present reaction include triethylamine, pyridine, N,N-dimethyl-4-aminopyridine and N,N-diisopropylethylamine. The amount of the base to be used is usually 1 to 5 equivalents, and preferably 1 to 3 equivalents, with respect to 1 equivalent of compound [9-c].

The amount of 4-nitrophenyl chloroformate or dicyclohexylcarbodiimide (CDI) to be used in the present reaction is 1 to 5 equivalents, and preferably 1 to 3 equivalents, with respect to 1 equivalent of compound [9-c].

Examples of the solvent which is used in the present reaction include solvents that do not interfere with reactions, such as chloroform, dichloromethane, diethyl ether, tetrahydrofuran, ethyl acetate and acetonitrile; and these solvents may be mixed with each other at an appropriate ratio and used.

The present reaction can be carried out usually at 0°C to reflux temperature for 1 to 24 hours.

[Step 9-10]

This step is a method of producing compound [9-m] by deprotecting the pyrazolyl of compound [9-k] by removing protecting group $Pro^2$ under an acidic condition.

This reaction can be carried out by the method described in step 2-2 of production method 2 or a method pursuant thereto.

Compound [9-m] thus obtained can be isolated and purified by known separation and purification means such as concentration, concentration under reduced pressure, reprecipitation, solvent extraction, crystallization and chromatography.

Compounds [1-e] and [9-a] which are used as starting compounds in the above described production method 9 can be produced by the above production method 1, a method pursuant thereto, or a method known per se, or can be obtained by purchasing commercial products.

The reaction of steps 9-1 to 9-10 may be carried out even when the pyrazolyl is not protected with protecting group $Pro^2$.

Of compounds [I] of the present invention, compound [11-g] can be produced by the following production method 11 or a method pursuant thereto.

Production method 11:

[Formula 70]

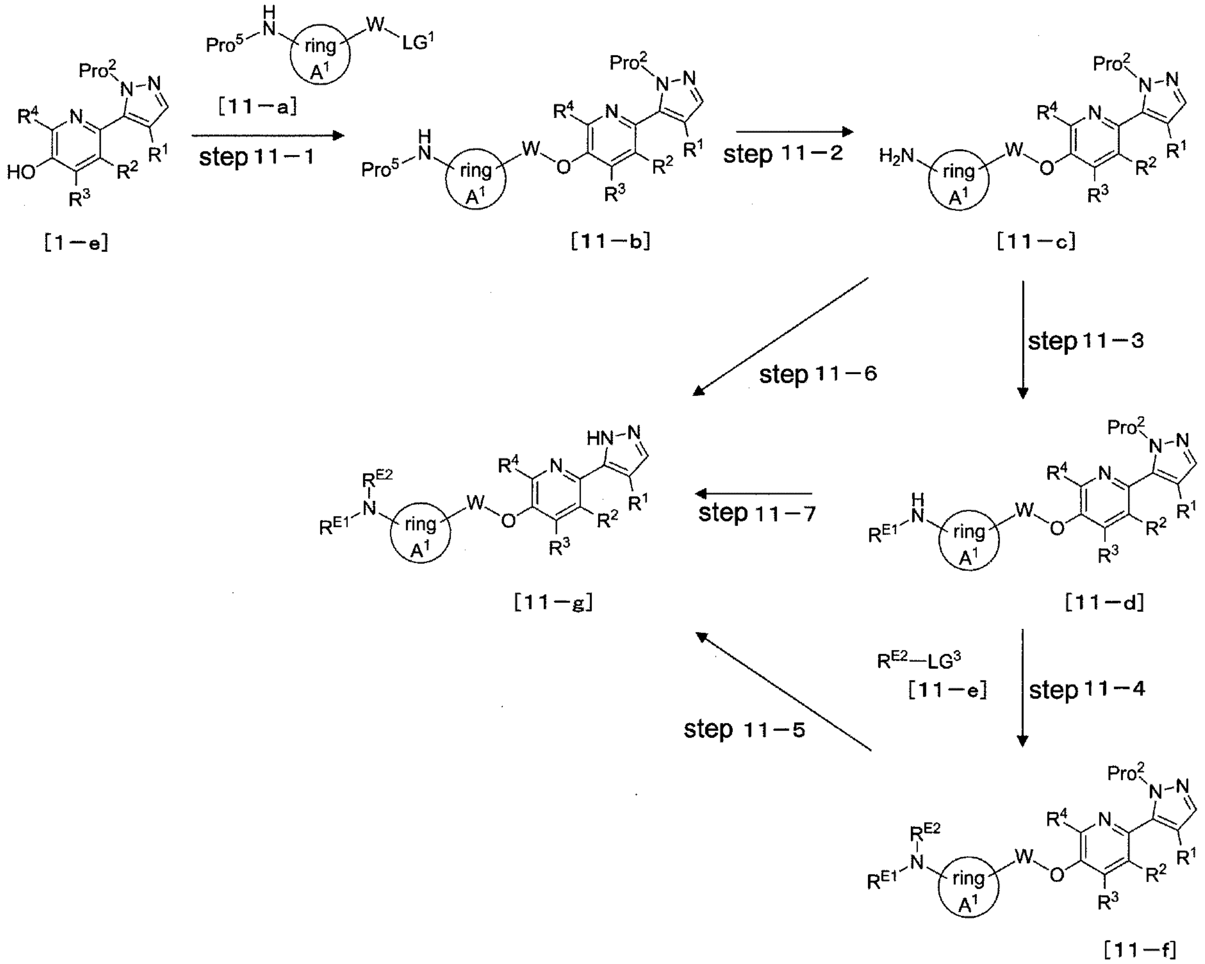


[In the scheme,

$R^1$, $R^2$, $R^3$, $R^4$, W, $LG^1$, $Pro^2$ are the same as defined above;
$R^{E1}$ represents a hydrogen atom or $C_{1-6}$ alkylcarbonyl;
$R^{E2}$ represents a hydrogen atom or $C_{1-6}$ alkyl;
ring $A^1$ represents $C_{4-6}$ cycloalkyl or phenyl;
$LG^3$ represents a leaving group;
$Pro^5$ represents a protecting group of amino;
the "leaving group" represented by $LG^3$ refers to, for example, a halogen atom such as a chlorine atom and a bromine atom, $C_{1-6}$ alkylsulfonyloxy such as methanesulfonyloxy, or arylsulfonyloxy such as p-toluenesulfonyloxy; and
$Pro^5$ represents, for example, tert-butoxycarbonyl, benzyloxycarbonyl, etc.]

[Step 11-1]

This step is a method of producing compound [11-b] by reacting compound [1-e] with compound [11-a].

This reaction can be carried out by the method described in step 2-1 of production method 2, step 3-1 of production method 3, step 4-3 of production method 4 or a method pursuant thereto.

[Step 11-2]

This step is a method of producing compound [11-c] by deprotecting the amino of compound [11-b] by removing protecting group $Pro^5$.

tert-Butoxycarbonyl, benzyloxycarbonyl, etc., which are protecting group $Pro^5$, can be removed by the method described in step 9-2 of production method 9 or a method pursuant thereto.

[Step 11-3]

This step is a method of producing compound [11-d] from compound [11-c].
This reaction can be carried out by the method described in step 9-3 of production method 9 or a method pursuant thereto.

[Step 11-4]

This step is a method of producing compound [11-f] by reacting compound [11-d] with compound [11-e].
This reaction can be carried out by the method described in step 4-1 of production method 4 or a method pursuant thereto.

[Step 11-5]

This step is a method of producing compound [11-g] by deprotecting the pyrazolyl of compound [11-f] by removing protecting group $Pro^2$ under an acidic condition.
This reaction can be carried out by the method described in step 2-2 of production method 2 or a method pursuant thereto.

[Step 11-6]

This step is a method of producing compound [11-g] in which both $R^{E1}$ and $R^{E2}$ are hydrogen atoms from compound [11-c].
This reaction can be carried out by the method described in step 2-2 of production method 2 or a method pursuant thereto.

[Step 11-7]

This step is a method of producing compound [11-g] in which $R^{E2}$ is a hydrogen atom from compound [11-d].
This reaction can be carried out by the method described in step 2-2 of production method 2 or a method pursuant thereto.
Compound [11-g] thus obtained can be isolated and purified by known separation and purification means such as concentration, concentration under reduced pressure, reprecipitation, solvent extraction, crystallization and chromatography.
Compounds [1-e], [11-a] and [11-e] which are used as starting compounds in the above described production method 11 can be produced by the above production method 1, a method pursuant thereto, or a method known per se, or can be obtained by purchasing commercial products.
Production of compounds [2-a], [3-a] and [4-d] which are used as starting compounds in the above described production methods 2-7 may be carried out by considering the respective steps of production method 9 or 11.
Formula [I'] shown below may be derived from compounds [18-c], [19-c], [20-c], [21-c], [22-c], [23-e], [24-e] which are prepared by production methods 18 - 24 described below as a starting material by the method described in production methods 2 - 4, 8, 9 and 11, or a method pursuant thereto. When intermediate [19-b] does not have $Pro^2$, the step corresponding to step 2-2 of production method 2 may be omitted.

[Formula 71]

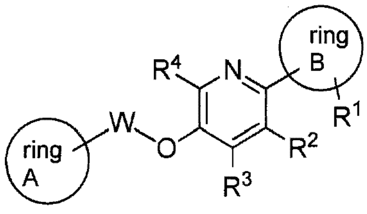

[I']

Of compounds [I'] of the present invention, compound [18-c] which is an intermediate in the production of a compound in which the structure represented by the following formula [III] is a structure of the following formula [VIII] can be produced, for example, by the following production method 18 or a method pursuant thereto.

[Formula 72]

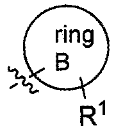

[III]

[Formula 73]

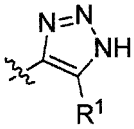

[VIII]

Production method 18:

[Formula 74]

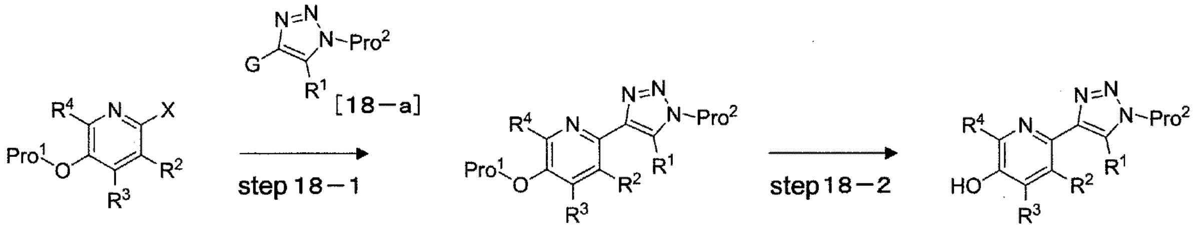


[1−b] [18−b] [18−c]

[In the scheme,
$R^1$, $R^2$, $R^3$, $R^4$, X, $Pro^1$, $Pro^2$ and G are the same as defined above.]

[Step 18-1]

This step is a method for synthesizing compound [18-b] using compound [1-b] by a so-called cross-coupling reaction.

Using compound [18-a], compound [18-b] can be obtained by the method described in step 1-2 in production method 1, or a method pursuant thereto.

[Step 18-2]

This step is a method for producing compound [18-c] by deprotecting the hydroxy group of compound [18-b] by removing protecting group $Pro^1$.

The present reaction can be carried out by the method described in step 1-3 in production method 1, or a method pursuant thereto.

Compound [18-c] thus obtained can be isolated and purified by known separation and purification means such as concentration, concentration under reduced pressure, reprecipitation, solvent extraction, crystallization and chromatography.

Compound [19-c] which is an intermediate in the production of that type of compound [I'] of the present invention in which the structure represented by the following formula [III] is a structure represented by the following formula [IX] can be produced, for example, by the following production method 19 or a method pursuant thereto.

[Formula 75]

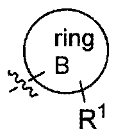

[III]

[Formula 76]

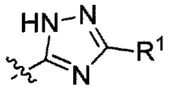

[IX]

Production method 19:

[Formula 77]

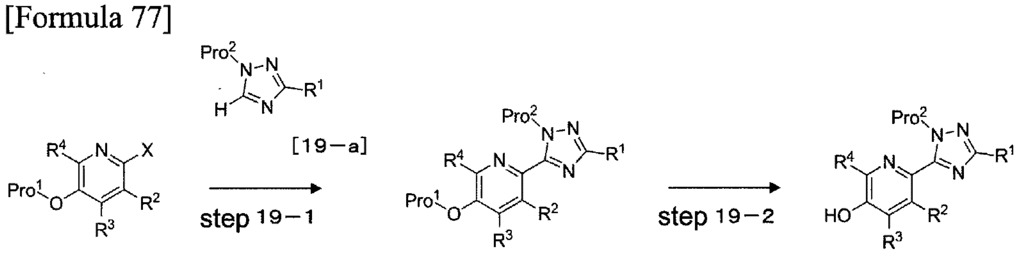


[1−b] [19−b] [19−c]

[In the scheme,
$R^1$, $R^2$, $R^3$, $R^4$, X, $Pro^1$ and $Pro^2$ are the same as defined above.]

[Step 19-1]

This step is a C-H activation reaction, which can be carried out by using a catalyst such as palladium (II), rhodium (I), iridium (I), ruthenium (II), copper (II) and iron (II) in the presence of an appropriate ligand and a base.

The amount of compound [19-a] which is used in the present reaction is 0.5 to 3 equivalents, and preferably 0.5 to 1.5 equivalents, with respect to 1 equivalent of compound [1-b].

Examples of the combination of the catalyst and the ligand to be used in the present reaction include palladium acetate-butyl di-1-adamantylphosphine, iron acetate-bathophenanthroline, and copper iodide-1,10-phenanthroline. The amount of the catalyst to be used is usually 0.001 to 1 equivalent, and preferably 0.005 to 0.5 equivalents, with respect to 1 equivalent of compound [1-b]. The amount of the ligand to be used is usually 0.0001 to 2 equivalents, and preferably 0.01 to 1 equivalent, with respect to 1 equivalent of compound [1-b].

Examples of the base to be used in the present reaction include salts such as silver (I) carbonate, tert-butoxy potassium, and tert-butoxy sodium. The amount of the base to be used is usually 1 to 5 equivalents, and preferably 1 to 3 equivalents, with respect to 1 equivalent of compound [1-b].

Examples of the reaction solvent to be used in the present reaction include solvents that do not interfere with reactions, such as N,N-dimethylformamide, dimethylsulfoxide, toluene, xylene, 1,4-dioxane, tetrahydrofuran, and 1,2-dimethoxyethane; and these solvents may be mixed with each other at an appropriate ratio and used.

The present reaction can be carried out usually at room temperature to reflux temperature for 1 to 24 hours, and can be also carried out under microwave irradiation.

[Step 19-2]

This step is a method for producing compound [19-c] by deprotecting the hydroxy group of compound [19-b] by removing protecting group $Pro^1$.

The present reaction can be carried out by the method described in step 1-3 in production method 1, or a method pursuant thereto.

Compound [19-c] thus obtained can be isolated and purified by known separation and purification means such as concentration, concentration under reduced pressure, reprecipitation, solvent extraction, crystallization and chromatography.

Compound [20-c] which is an intermediate in the production of that type of compound [I'] of the present invention in which the structure represented by the following formula [III] is any of the structures shown in following formula group [X] can be produced, for example, by the following production method 20 or a method pursuant thereto.

[Formula 78]

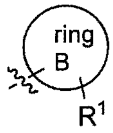

[III]

[Formula 79]

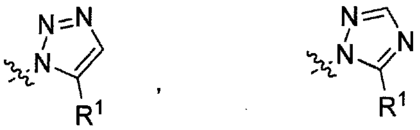

[X]

Production method 20:

[Formula 80]

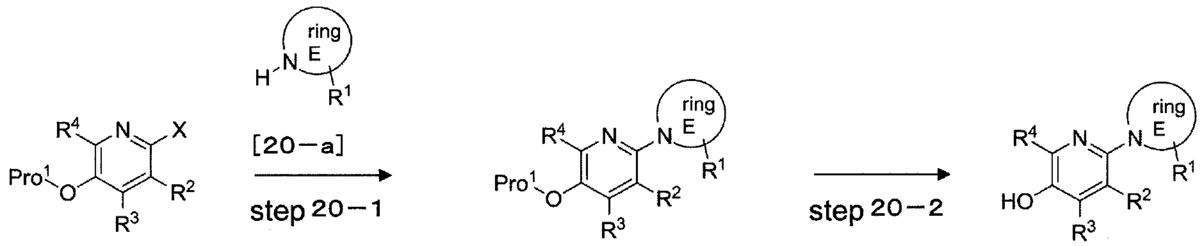


[1−b] [20−b] [20−c]

[In the scheme,
$R^1$, $R^2$, $R^3$, $R^4$, X and $Pro^1$ are the same as defined above, and the structure represented by the following formula [XI] indicates any structure shown in the following formula group [X]].

[Formula 81]

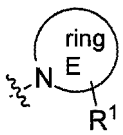

[XI]

[Formula 82]

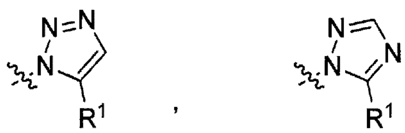

[X]

[Step 20-1]

This step is a coupling reaction that involves forming a C-N bond in the presence of a copper salt and a base.
The amount of compound [20-a] to be used in the present invention is 1 to 3 equivalents, and preferably 1.2 to 1.5 equivalents, with respect to 1 equivalent of compound [1-b].
Examples of the catalyst to be used in the present reaction include copper (I) iodide and copper (II) acetate. The amount of the catalyst to be used is usually 0.1 to 1 equivalent, and preferably 0.1 to 0.5 equivalents, with respect to 1 equivalent of compound [1-b].
Examples of the base include an alkali metal salt such as potassium phosphate, cesium carbonate and potassium carbonate. The amount of the base to be used is usually 1 to 5 equivalents, and preferably 1 to 3 equivalents, with respect to 1 equivalent of compound [1-b].

Examples of the reaction solvent include solvents that do not interfere with reactions, such as N,N-dimethylformamide, dimethylsulfoxide, toluene, xylene, 1,4-dioxane, tetrahydrofuran, and 1,2-dimethoxyethane; and these solvents may be mixed with each other at an appropriate ratio and used.

In these reactions, a divalent amine such as N,N'-dimethyl ethylene diamine may be added as an additive. These reactions can be carried out usually at room temperature to reflux temperature for 1 to 24 hours, and can be also carried out under microwave irradiation.

[Step 20-2]

This step is a method for producing compound [20-c] by deprotecting the hydroxy group of compound [20-b] by removing protecting group $Pro^1$.

The present reaction can be carried out by the method described in step 1-3 in production method 1, or a method pursuant thereto.

Compound [20-c] thus obtained can be isolated and purified by known separation and purification means such as concentration, concentration under reduced pressure, reprecipitation, solvent extraction, crystallization and chromatography.

Compound [21-c] which is an intermediate in the production of that type of compound [I'] of the present invention in which the structure represented by the following formula [III] is a structure shown in the following formula [XII] can be produced, for example, by the following production method 21 or a method pursuant thereto.

[Formula 83]

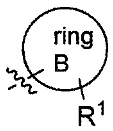

[III]

[Formula 84]

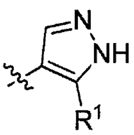

[XII]

Production method 21:

[Formula 85]

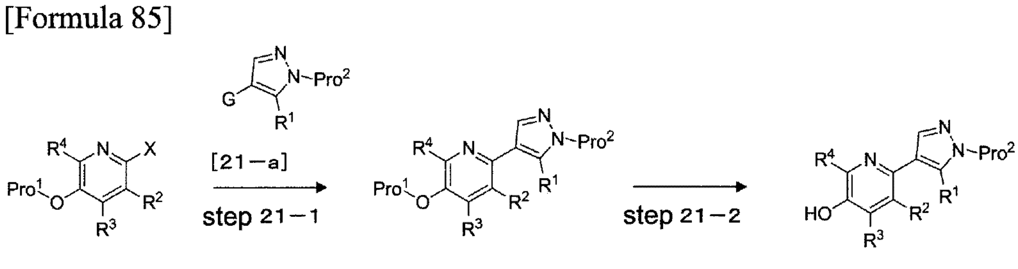


[1−b] [21−b] [21−c]

[In the scheme,
$R^1$, $R^2$, $R^3$, $R^4$, X, $Pro^1$, $Pro^2$ and G are the same as defined above.]

[Step 21-1]

This step is a method of producing compound [21-b] by reacting compound [1-b] with compound [21-a].

The present reaction can be carried out by the method described in step 1-2 in production method 1, or a method pursuant thereto.

[Step 21-2]

This step is a method for producing compound [21-c] by deprotecting the hydroxy group of compound [21-b] by removing protecting group $Pro^1$.
The present reaction can be carried out by the method described in step 1-3 in production method 1, or a method pursuant thereto.
Compound [21-c] thus obtained can be isolated and purified by known separation and purification means such as concentration, concentration under reduced pressure, reprecipitation, solvent extraction, crystallization and chromatography.
Compound [22-c] which is an intermediate in the production of that type of compound [I'] of the present invention in which the structure represented by the following formula [III] is a structure shown in the following formula [XIII] can be produced, for example, by the following production method 22 or a method pursuant thereto.

[Formula 86]

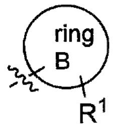
[III]

[Formula 87]

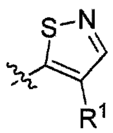
[XIII]

Production method 22:

[Formula 88]

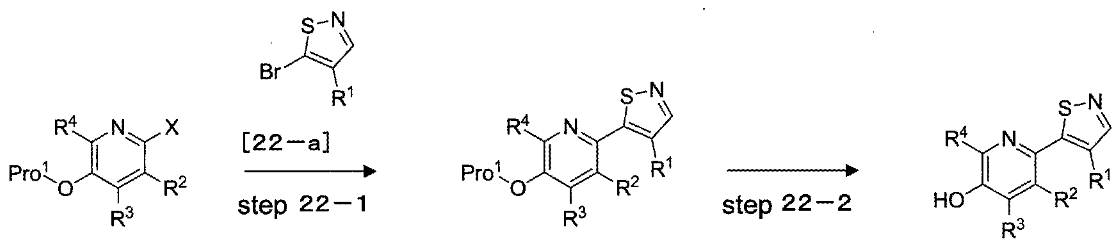


[1−b] [22−b] [22−c]

[In the scheme,
$R^1$, $R^2$, $R^3$, $R^4$, X and $Pro^1$ are the same as defined above.]

[Step 22-1]

This step is a method of producing compound [22-b] by reacting compound [1-b] with compound [22-a].
The present reaction can be carried out by the method described in Patent Literature (International Publication No. WO 2008051406A2), or a method pursuant thereto.
The present reaction is an application of a so-called Stille-Kelly reaction to intermolecular hetero coupling, which can be carried out in the presence of a palladium catalyst and an organodistannane.
The type and the amount of the catalyst to be used in the present reaction are the same as in step 1-2 in production method 1 or pursuant thereto.
Examples of the organodistannane to be used in the present reaction include bis(trimethylstannane) and bis(tributylstannane). The amount of the organodistannane to be used is 1 to 3 equivalents, and preferably 1 to 1.5 equivalents, with respect to compound [1-b].

Examples of the reaction solvent to be used in the present reaction include solvents that do not interfere with reactions, such as toluene, xylene, 1,4-dioxane, tetrahydrofuran, and 1,2-dimethoxyethane; and these solvents may be mixed with each other at an appropriate ratio and used.
These reactions can be carried out usually at room temperature to reflux temperature for 1 to 24 hours, and can be also carried out under microwave irradiation.

[Step 22-2]

This step is a method for producing compound [22-c] by deprotecting the hydroxy group of compound [22-b] by removing protecting group $Pro^1$.
The present reaction can be carried out by the method described in step 1-3 in production method 1, or a method pursuant thereto.
Compound [22-c] thus obtained can be isolated and purified by known separation and purification means such as concentration, concentration under reduced pressure, reprecipitation, solvent extraction, crystallization and chromatography.
Compound [23-e] having $R^1$ of difluoromethyl, which is an intermediate [1-e] in the production of compound [I] of the present invention (that type of compound [I'] in which the structure represented by the following formula [III] is a structure shown in the following formula [VI]), can be produced, for example, by the following production method 23 or a method pursuant thereto.

[Formula 89]

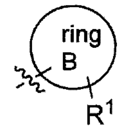

[III]

[Formula 90]

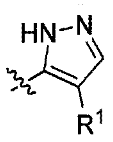

[VI]

Production method 23:

[Formula 91]

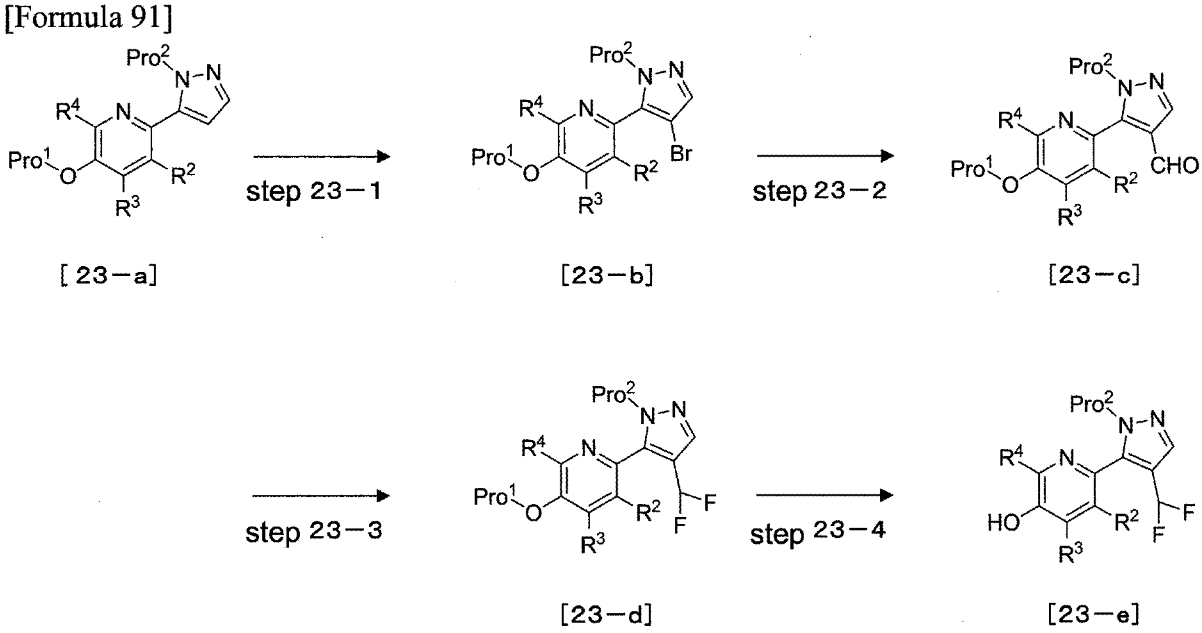


[23−a] [23−b] [23−c] [23−d] [23−e]

[In the scheme,
$R^2$, $R^3$, $R^4$, $Pro^1$ and $Pro^2$ are the same as defined above.]

[Step 23-1]

This step is a method for producing compound [23-b] by regioselectively brominating compound [23-a].

The present reaction can be carried out by the method described in step 8-1 in production method 8, or a method pursuant thereto.

[Step 23-2]

This step is a method for producing compound [23-c] by reacting compound [23-b] with an alkyl lithium compound and then reacting the resulting reaction intermediate with a formamide compound.

Examples of the alkyl lithium compound for use in the reaction with compound [23-b] include n-butyl lithium. The amount of the alkyl lithium compound to be used is 1 to 5 equivalents, and preferably 1 to 3 equivalents, with respect to 1 equivalent of compound [23-b].

Examples of the solvent to be used in the present reaction include solvents that do not interfere with reactions, such as diethyl ether, tetrahydrofuran, 1,4-dioxane, toluene, and xylene; and these solvents may be mixed with each other at an appropriate ratio and used.

The present reaction can be carried out usually at -80°C to -50°C for 0.1 to 1 hour.

Examples of the formamide compound to be used in the reaction with the reaction intermediate include N,N-dimethyl formamide and N-methoxy-N-methylformamide. The amount of the formamide compound to be used is 1 to 5 equivalents, and preferably 1 to 3 equivalents, with respect to 1 equivalent of compound [23-b].

The present reaction can be carried out usually at -80°C to room temperature for 0.1 to 24 hours.

[Step 23-3]

This step is a method for producing compound [23-d] by fluorinating the formyl of compound [23-c].

Examples of the fluorinating reagent to be used in the reaction with compound [23-c] include tetrafluoro sulfur (IV), (N,N-diethylamino)sulfur trifluoride (DAST), and bis(2-methoxyethyl)aminosulfur trifluoride (BAST). The amount of the fluorinating reagent to be used is 1 to 5 equivalents, and preferably 1 to 3 equivalents, with respect to 1 equivalent of compound [23-c].

Examples of the solvent to be used in the present reaction include solvents that do not interfere with reactions, such as dichloromethane, chloroform, acetonitrile, tetrahydrofuran, 1,4-dioxane, toluene, and xylene; and these solvents may be mixed with each other at an appropriate ratio and used.

The present reaction can be carried out usually at room temperature to reflux temperature for 1 to 24 hours.

[Step 23-4]

This step is a method for producing compound [23-e] by deprotecting compound [23-d] by removing protecting group $Pro^1$.
This step can be carried out by step 1-3 in production method 1, or a method pursuant thereto.
Compound [23-e] thus obtained can be isolated and purified by known separation and purification means such as concentration, concentration under reduced pressure, reprecipitation, solvent extraction, crystallization and chromatography.
Incidentally, compound [23-a] to be used as a starting compound in production method 23 can be obtained by steps 1-1 and 1-2 in production method 1, or a method pursuant thereto.
Compound [24-e] which is an intermediate in the production of that type of compound [I'] of the present invention in which the structure represented by the following formula [III] is a structure shown in the following formula [XIV] can be produced, for example, by the following production method 24 or a method pursuant thereto.

[Formula 92]

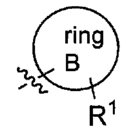
[III]

[Formula 93]

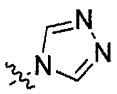
[XIV]

Production method 24:

[Formula 94]

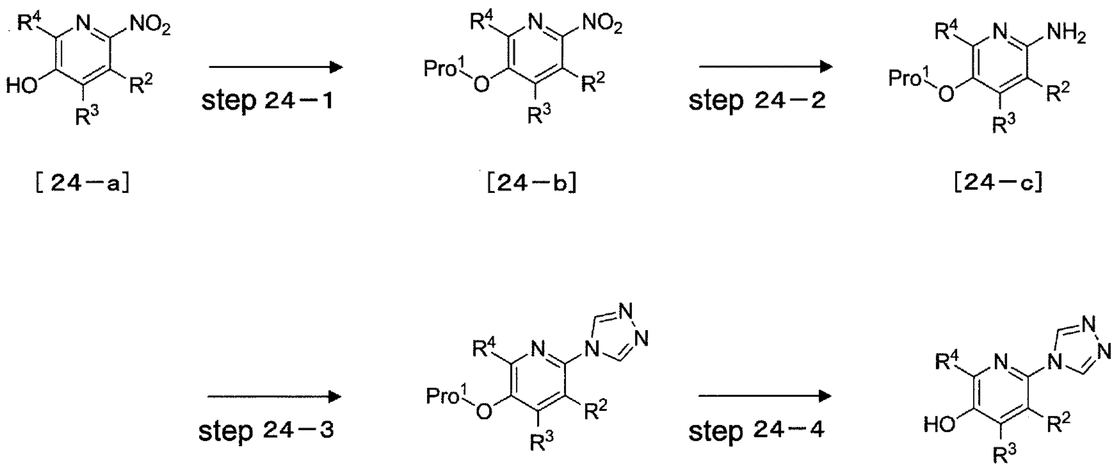

[24−a] [24−b] [24−c] [24−d] [24−e]

[In the scheme,
$R^2$, $R^3$, $R^4$ and $Pro^1$ are the same as defined above.]

[Step 24-1]

This step is a method for producing compound [24-b] by protecting the hydroxy group of compound [24-a] with protecting group $Pro^1$.
This step can be carried out by step 1-1 in production method 1, or a method pursuant thereto.

[Step 24-2]

This step is a method for producing compound [24-c] by reducing the nitro of compound [24-b] using a metal reagent under an acidic condition.
Examples of the metal reagent to be used in this step include iron, zinc, and tin. The amount of the metal reagent to be used is 1 to 10 equivalents, and preferably 2 to 5 equivalents, with respect to 1 equivalent of compound [24-b].
Examples of the acid to be used in this step include hydrochloric acid and ammonium chloride. The amount of the acid to be used is 1 to 5 equivalents, and preferably 1 to 3 equivalents, with respect to 1 equivalent of compound [24-b].
Examples of the solvent to be used in the present reaction include solvents that do not interfere with reactions, such as methanol, ethanol, ethylene glycol, water, and tetrahydrofuran; and these solvents may be mixed with each other at an appropriate ratio and used.
The present reaction can be carried out usually at reflux temperature for 0.5 to 8 hours.

[Step 24-3]

This step is a method for producing compound [24-d] from compound [24-c] and 1,2-diformylhydrazine in the presence of a trialkylsilyl chloride and an amine.
The amount of 1,2-diformylhydrazine to be used in this step is 1 to 5 equivalents, and preferably 2 to 4 equivalents, with respect to 1 equivalent of compound [24-c].
Examples of the trialkylsilyl chloride to be used in this step include trimethylsilyl chloride, triethylsilyl chloride, and triisopropylsilyl chloride. The amount of the trialkylsilyl chloride to be used is 4 to 30 equivalents, and preferably 10 to 20 equivalents, with respect to 1 equivalent of compound [24-c].
Examples of the amine to be used in this step include triethylamine and diisopropylethylamine. The amount of the amine to be used is 2 to 15 equivalents, and preferably 5 to 10 equivalents, with respect to 1 equivalent of compound [24-c].
Examples of the solvent to be used in the present reaction include solvents that do not interfere with reactions, such as toluene, N,N-dimethylformamide, and pyridine; and these solvents may be mixed with each other at an appropriate ratio and used. Alternatively the present reaction may be carried out without a solvent.
The present reaction can be carried out usually at reflux temperature for 0.5 to 8 hours.

[Step 24-4]

This step is a method for producing compound [24-e] by deprotecting the hydroxy group of compound [24-d] by removing protecting group $Pro^1$.
The present step can be carried out by the same method described in step 1-3 in production method 1, or a method pursuant thereto.
Compound [24-e] thus obtained can be isolated and purified by known separation and purification means such as concentration, concentration under reduced pressure, reprecipitation, solvent extraction, crystallization and chromatography.
Compound [24-a] to be used as a starting compound in the production method 24 can be produced by a method known per se, or can be obtained by purchasing commercial products.
Using compounds [18-c], [19-c], [20-c], [21-c], [22-c], [23-e] and [24-e] obtained in the production methods 18 to 24 (compound [25-a] in the following production method 25) as a starting material, compound [I'] can be synthesized by a method described in production methods 2 to 4, 8, 9 and 11 or a method pursuant thereto.

Production method 25:

[Formula 95]

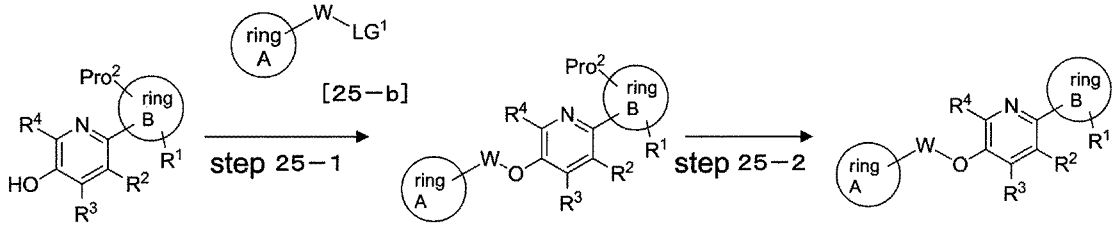


[25−a] [25−c] [I′]

[In the scheme,

$R^1$, $R^2$, $R^3$, $R^4$, W, ring A, the structure represented by formula [III], $Pro^2$ and $LG^1$ are the same as defined above.

Also, the structure represented by the following formula [XV] indicates any structure shown in the following formula group [XVI].

[Formula 96]

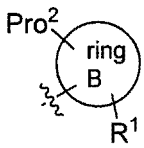
[XV]

[Formula 97]

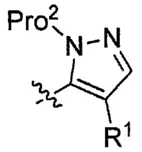 , 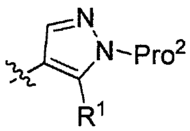 ,

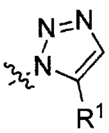 , 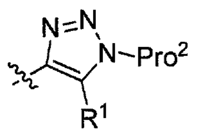 ,

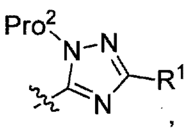 , 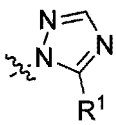 , 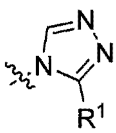 ,

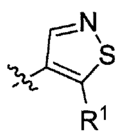 , 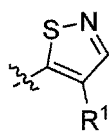

[XVI] ]

[Step 25-1]

This step is a method for producing compound [25-c] by reacting compound [25-a] with compound [25-b].

(i) In the case of a compound with W being a $C_{1-3}$ alkanediyl, compound [25-c] can be produced by the method described in step 2-1 in production method 2, or a method pursuant thereto.

(ii) In the case of a compound with W being a single bond, compound [25-c] can be produced by the method described in step 3-1 in production method 3, or a method pursuant thereto.

(iii) In the case of a compound with W being a formula $-O-CH_2CH_2-$, compound [25-c] can be produced by the method described in step 4-3 in production method 4, or a method pursuant thereto.

[Step 25-2]

This step is a method for producing compound [I'] by deprotecting compound [25-c] by removing protecting group $Pro^2$.

For example, compound [I'] can be synthesized by the method described in step 2-2 in production method 2, or a method pursuant thereto.

Compound [I'] thus obtained can be isolated and purified by known separation and purification means such as concentration, concentration under reduced pressure, reprecipitation, solvent extraction, crystallization and chromatography.

Using production intermediate [5-a] obtained in the production method 5 as a starting material, compound [26-b], [26-c], [26-d], [26-e] and [26-f] can be produced by a method described in production methods 5 and 18 to 22, or a method pursuant thereto.

Production method 26:

[Formula 98]

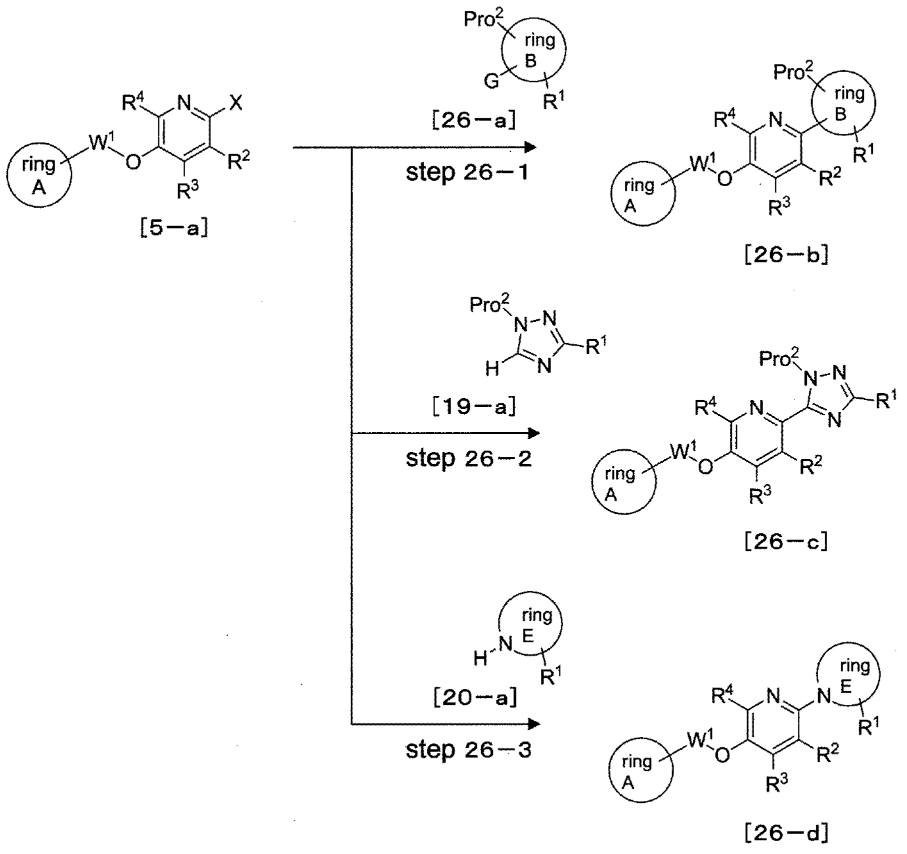

[Formula 99]

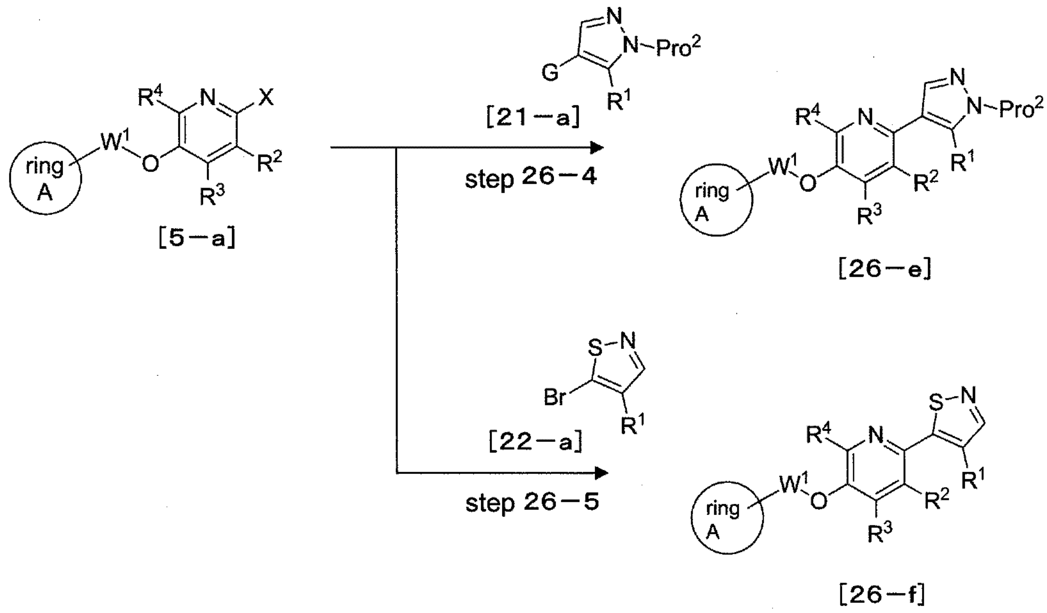


[5−a] [21−a] [26−e] [22−a] [26−f]

[In the scheme,
$R^1$, $R^2$, $R^3$, $R^4$, X, $W^1$, ring A, the structure represented by formula [XV], the structure represented by formula [XI], G, and $Pro^2$ are the same as defined above.

Also, in the present scheme, ring B may not be protected with protecting group $Pro^2$.]

[Step 26-1]

This step is a method for producing compound [26-b] by reacting compound [5-a] with compound [26-a].

(i) When the structure represented by the following formula [XV] is a structure shown in the following formula [XVI], compound [26-b] can be produced by the method described in step 5-2 in production method 5, or a method pursuant thereto, as described above.

[Formula 100]

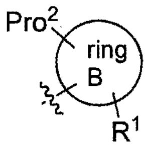
[XV]

[Formula 101]

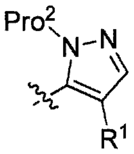
[XVI]

(ii) When the structure represented by the forgoing formula [XV] is any structure shown in the following formula group [XVIII], compound [26-b] can be produced by the method described in step 18-1 in production method 18, or a method pursuant thereto.

[Formula 102]

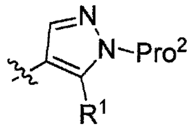

,

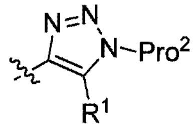

[XVIII]

[Step 26-2]

This step is a method for producing compound [26-c] by reacting compound [5-a] with compound [19-a].

As described above, compound [26-c] can be produced by the method described in step 19-1 in production method 19, or a method pursuant thereto.

[Step 26-3]

This step is a method for producing compound [26-d] by reacting compound [5-a] with compound [20-a].

As described above, compound [26-d] can be produced by the method described in step 20-1 in production method 20, or a method pursuant thereto.

[Step 26-4]

This step is a method for producing compound [26-e] by reacting compound [5-a] with compound [21-a].

As described above, compound [26-e] can be produced by the method described in step 21-1 in production method 21, or a method pursuant thereto.

[Step 26-5]

This step is a method for producing compound [26-f] by reacting compound [5-a] with compound [22-a].

As described above, compound [26-f] can be produced by the method described in step 22-1 in production method 22, or a method pursuant thereto.

Compounds [26-b], [26-c], [26-d], [26-e] and [26-f] thus obtained can be isolated and purified by known separation and purification means such as concentration, concentration under reduced pressure, reprecipitation, solvent extraction, crystallization and chromatography.

Compounds [26-b], [26-c], [26-d], [26-e] and [26-f] can be led to compound [I'] by the method described in step 2-2 in production method 2, or a method pursuant thereto.

Also, using production intermediate [6-a] obtained in the production method 6 as a starting material, compounds [27-a], [27-b], [27-c], [27-d] and [27-e] can be produced by a method described in production methods 6 and 18 to 22, or a method pursuant thereto.

Production method 27:

[Formula 103]

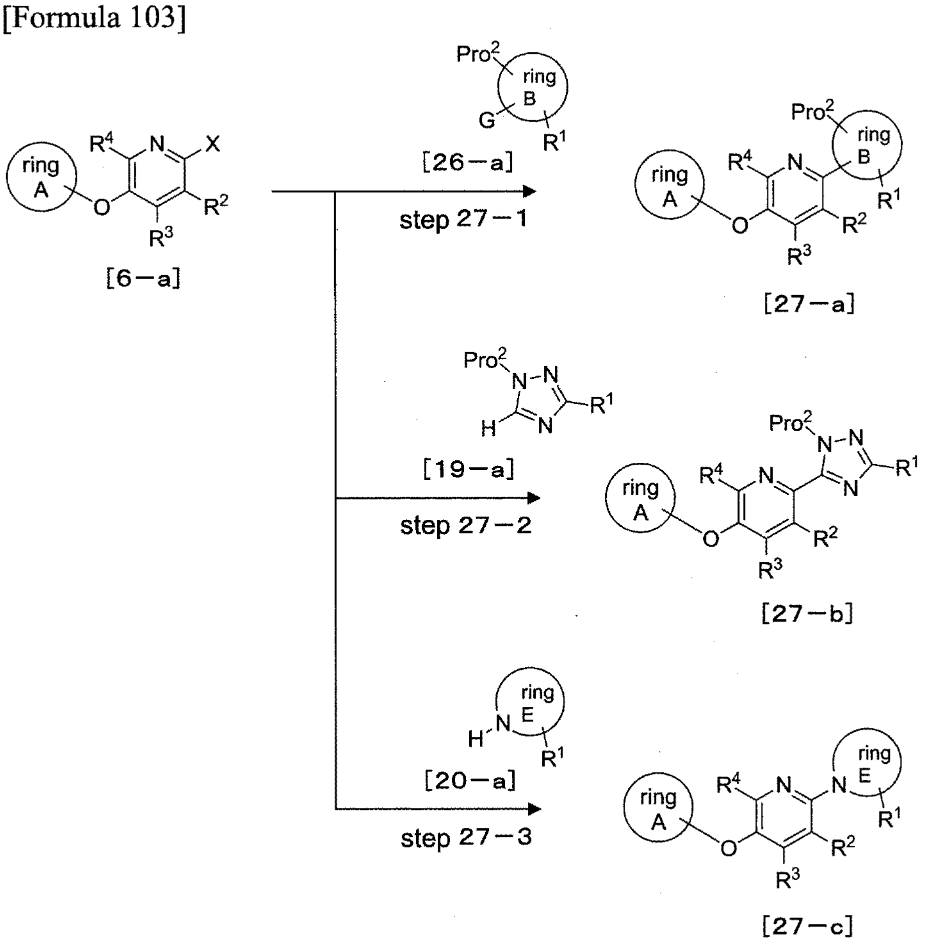


[Formula 104]

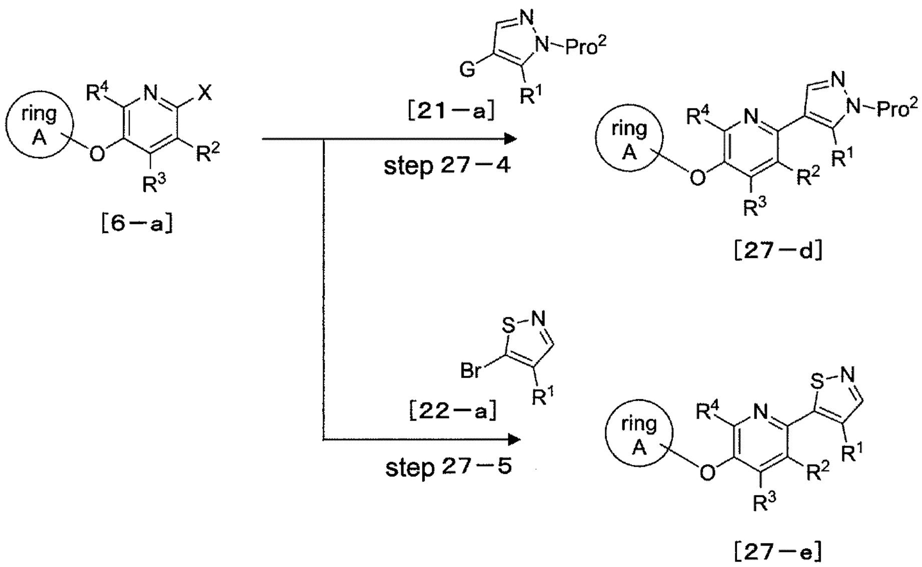


[In the scheme,
$R^1$, $R^2$, $R^3$, $R^4$, X, ring A, the structure represented by formula [XV], the structure represented by formula [XI], G, and $Pro^2$ are the same as defined above.

Also, in the present scheme, ring B may not be protected with protecting group $Pro^2$.]

[Step 27-1]

This step is a method for producing compound [27-a] by reacting compound [6-a] with compound [26-a].

(i) When the structure represented by the following formula [XV] is a structure shown in the following formula [XVI], compound [27-a] can be produced by the method described in step 6-2 in production method 6, or a method pursuant thereto, as described above.

[Formula 105]

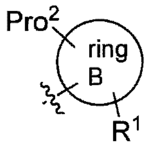

[XV]

[Formula 106]

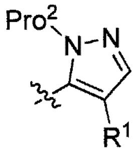

[XVI]

(ii) When the structure represented by the forgoing formula [XV] is any structure shown in the following formula group [XVIII], compound [27-a] can be produced by the method described in step 18-1 in production method 18, or a method pursuant thereto.

[Formula 107]

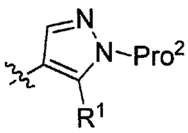

,

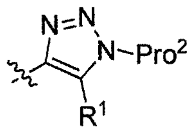

[XVIII]

[Step 27-2]

This step is a method for producing compound [27-b] by reacting compound [6-a] with compound [19-a].
As described above, compound [27-b] can be produced by the method described in step 19-1 in production method 19, or a method pursuant thereto.

[Step 27-3]

This step is a method for producing compound [27-c] by reacting compound [6-a] with compound [20-a].
As described above, compound [27-c] can be produced by the method described in step 20-1 in production method 20, or a method pursuant thereto.

[Step 27-4]

This step is a method for producing compound [27-d] by reacting compound [6-a] with compound [21-a].
As described above, compound [27-d] can be produced by the method described in step 21-1 in production method 21, or a method pursuant thereto.

[Step 27-5]

This step is a method for producing compound [27-e] by reacting compound [6-a] with compound [22-a].
Compound [27-e] can be produced by the method described in step 22-1 in production method 22, or a method

pursuant thereto.

Compounds [27-a], [27-b], [27-c], [27-d] and [27-e] thus obtained can be isolated and purified by known separation and purification means such as concentration, concentration under reduced pressure, reprecipitation, solvent extraction, crystallization and chromatography.

Compounds [27-a], [27-b], [27-c], [27-d] and [27-e] can be led to compound [I'] by the method described in step 3-2 in production method 3, or a method pursuant thereto.

Furthermore, using production intermediate [7-a] obtained in the production method 7 as a starting material, compounds [28-a], [28-b], [28-c], [28-d] and [28-e] can be produced by a method described in production methods 7 and 18 to 22, or a method pursuant thereto.

Production method 28:

[Formula 108]

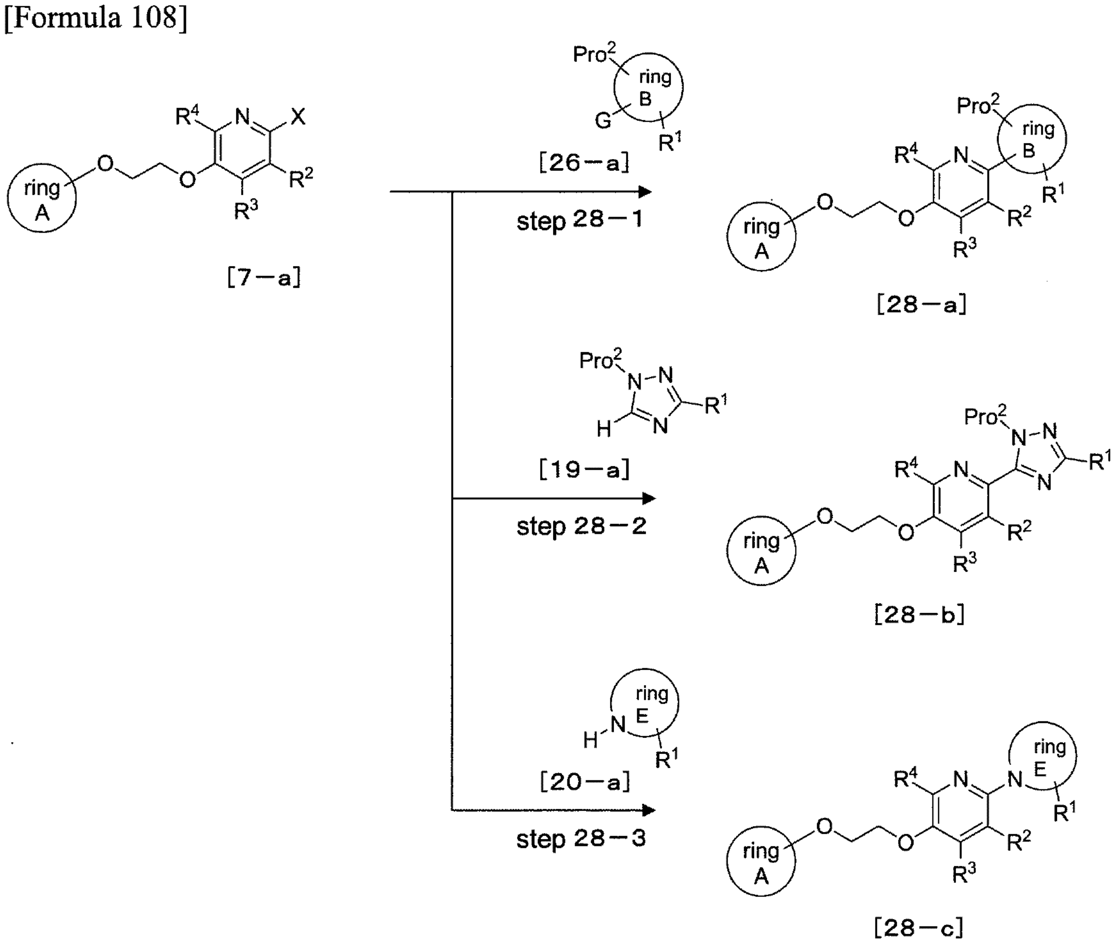

[Formula 109]

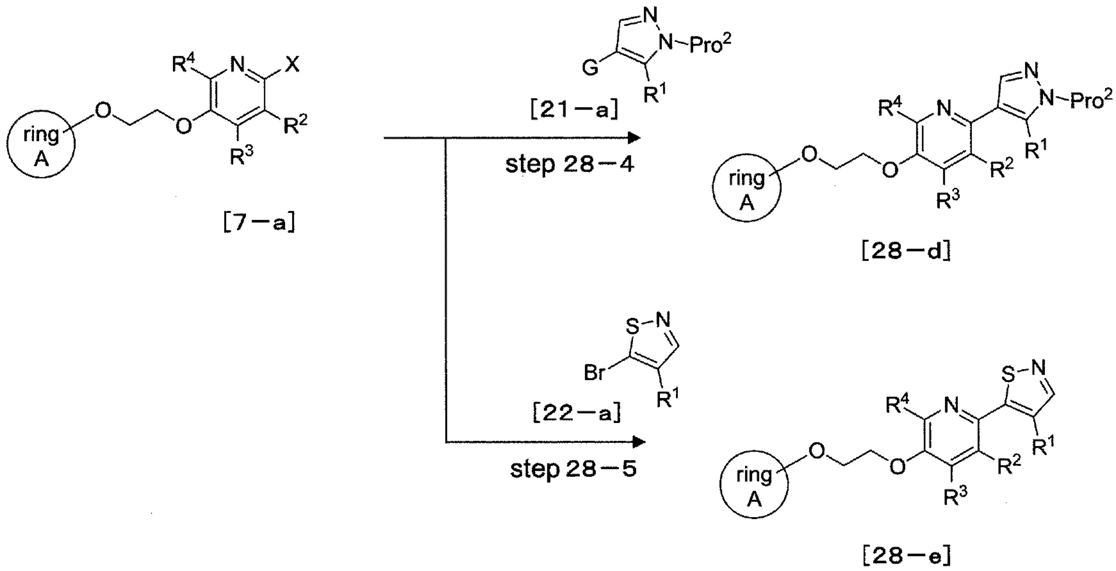


[In the scheme,
$R^1$, $R^2$, $R^3$, $R^4$, X, ring A, the structure represented by formula [XV], the structure represented by formula [XI], G, and $Pro^2$ are the same as defined above.

Moreover, in this scheme, ring B may not be protected with protecting group $Pro^2$.]

[Step 28-1]

This step is a method of producing compound [28-a] by reacting compound [7-a] with compound [26-a].

(i) When the structure represented by the following formula [XV] is a structure represented by the following formula [XVI], compound [28-a] can be produced by the method described in step 7-2 in production method 7 or a method pursuant thereto, as described above.

[Formula 110]

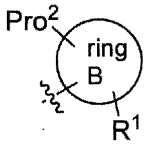
[XV]

[Formula 111]

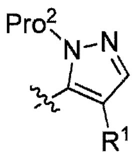
[XVI]

(ii) When the structure represented by the forgoing formula [XV] is any structure of the following formula group [XVIII], compound [28-a] can be produced by the method described in step 18-1 of production method 18 or a method pursuant thereto.

[Formula 112]

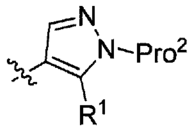

,

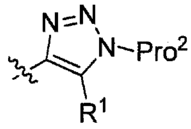

[XVIII]

[Step 28-2]

This step is a method of producing compound [28-b] by reacting compound [7-a] with compound [19-a].

As described above, compound [28-b] can be produced by the method described in step 19-1 of production method 19 or a method pursuant thereto.

[Step 28-3]

This step is a method of producing compound [28-c] by reacting compound [7-a] with compound [20-a].

As described above, compound [28-c] can be produced by the method described in step 20-1 of production method 20 or a method pursuant thereto.

[Step 28-4]

This step is a method of producing compound [28-d] by reacting compound [7-a] with compound [21-a].

As described above, compound [28-d] can be produced by the method described in step 21-1 of production method 21 or a method pursuant thereto.

[Step 28-5]

This step is a method of producing compound [28-e] by reacting compound [7-a] with compound [22-a].

As described above, compound [28-e] can be produced by the method described in step 22-1 of production method 22 or a method pursuant thereto.

Compounds [28-a], [28-b], [28-c], [28-d], and [28-e] thus obtained can be isolated and purified by known separation and purification means such as concentration, concentration under reduced pressure, reprecipitation, solvent extraction, crystallization, and chromatography.

Compounds [28-a], [28-b], [28-c], [28-d], and [28-e] thus obtained can be converted into compound [I'] by the method described in step 4-4 of production method 4 or a method pursuant thereto.

Among compound [I'] of the present invention, compound [29-b] in which the structure represented by the following formula [III] is a structure represented by the following formula [VIII] can be also produced by production method 29 described below or a method pursuant thereto, for example, using, as a starting material, production intermediate [5-a] obtained in production method 2.

[Formula 113]

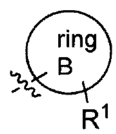

[III]

[Formula 114]

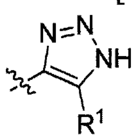

[VIII]

Production method 29:

[Formula 115]

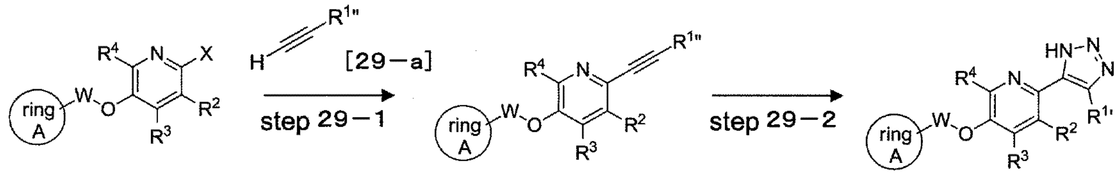


[5−a] [29−b] [29−c]

[In the scheme,

$R^2$, $R^3$, $R^4$, W, ring A, and X are the same as defined above;
$R^{1'}$ represents a hydrogen atom or methyl; and $R^{1''}$ represents trimethylsilyl (TMS) or methyl.]

[Step 29-1]

This step is a method of producing compound [29-b] by reacting compound [5-a] with compound [29-a].

This reaction is Sonogashira reaction that can be carried out using compound [29-a] in the presence of a palladium catalyst, a copper (I) salt, and a base by a process which is described in the literature (Handbook of Organopalladium Chemistry for Organic Synthesis, Chapter III.2.8., page 493) or a process pursuant thereto.

The amount of compound [29-a] which is used in the present reaction is usually 1 to 5 equivalents, and preferably 1 to 2 equivalents, with respect to 1 equivalent of compound [5-a].

Examples of the palladium catalysts which are used in the present reaction include tetrakis(triphenylphosphine)palladium(0), a [1,1'-bis(diphenylphosphino)ferrocene]palladium(II)dichloride dichloromethane adduct, and bis(triphenylphosphine)palladium(II)dichloride. The amount of the palladium catalyst to be used is usually 0.001 to 0.5 equivalents, and preferably 0.005 to 0.3 equivalents, with respect to 1 equivalent of compound [5-a].

Examples of the copper (I) salt which is used in the present reaction include copper (I) iodide. The amount of the copper (I) salt to be used is usually 0.01 to 1 equivalent, and preferably 0.02 to 0.3 equivalents, with respect to 1 equivalent of compound [5-a].

Examples of the base which is used in the present reaction include amine such as triethylamine and diisopropylethylamine. The amount of the base to be used is usually 2 equivalents to a solvent amount, and preferably 2 to 5 equivalents, with respect to 1 equivalent of compound [5-a].

Examples of the reaction solvent which is used in the present reaction include solvents that do not interfere with reactions, such as N,N-dimethylformamide, diethyl ether, 1,4-dioxane, tetrahydrofurane, and 1,2-dimethoxyethane; and these solvents maybe mixed with each other at an appropriate ratio and used.

These reactions can be carried out usually at room temperature to reflux temperature for 1 to 24 hours, and can be also carried out under microwave irradiation.

[Step 29-2]

This step, which is a so-called Huisgen cyclization reaction, is a method of producing compound [29-c] from compound [29-b].

This reaction can be carried out using an azide compound in the presence of a copper catalyst by a process which is described in the literature (Angewandte Chemie International Edition in English, Vol. 2, page 565, 1963) or a process pursuant thereto.

The azide compound which is used in the present reaction is sodium azide, and the amount to be used is usually 1 to 5 equivalents, and preferably 1 to 2 equivalents, with respect to compound [29-b].

Examples of the copper catalyst which is used in the present reaction include copper sulfate, copper iodide, copper acetate, and a copper trifluoromethanesulfonate benzene complex. The amount of the copper catalyst to be used is usually 0.01 to 0.5 equivalents, and preferably 0.05 to 0.2 equivalents, with respect to 1 equivalent of compound [29-b].

Examples of the reaction solvent which is used in the present reaction include solvents that do not interfere with reactions, such as N,N-dimethylformamide, ethanol, methanol, 1,4-dioxane, tetrahydrofurane, and water; and these solvents may be mixed with each other at an appropriate ratio and used.

These reactions can be carried out usually at room temperature to reflux temperature for 1 to 24 hours.

Compound [29-c] thus obtained can be isolated and purified by known separation and purification means such as concentration, concentration under reduced pressure, reprecipitation, solvent extraction, crystallization, and chromatography.

Among compound [I'] of the present invention, that type of compound [30-c] in which the structure represented by the following formula [III] is a structure of the following formula [VI] and $R^1$ is trifluoromethyl can be also produced by production method 30 described below or a method pursuant thereto, for example, using, as a starting material, production intermediate [2-b] obtained in production method 2.

[Formula 116]

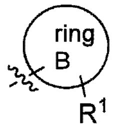
[III]

[Formula 117]

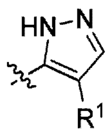
[VI]

Production method 30:

[Formula 118]

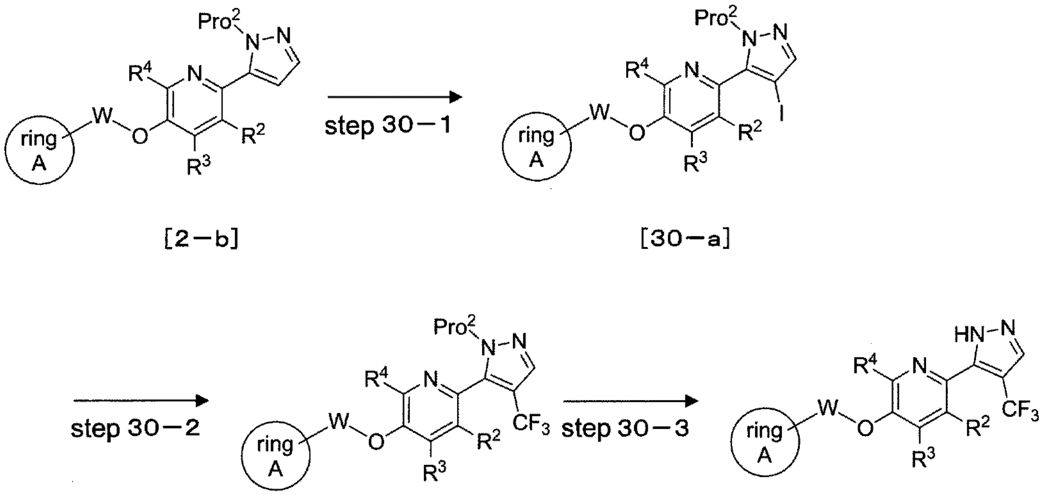

[2−b] [30−a] [30−b] [30−c]

[In the scheme,
$R^2$, $R^3$, $R^4$, W, ring A, and $Pro^2$ are the same as defined above.]

[Step 30-1]

This step is a method of producing compound [30-a] by regioselectively iodinating compound [2-b].

This reaction can be carried out by the method described in step 8-1 of production method 8 or a method pursuant thereto.

[Step 30-2]

This step is a method of producing compound [30-b] by converting an iodine atom of compound [30-a] into trifluoromethyl.

This reaction uses Trifluoromethylator (registered trademark) as a trifluoromethylation reagent. The amount of the reagent to be used is 1 to 10 equivalents, and preferably 3 to 5 equivalents, with respect to compound [30-a].
Examples of the reaction solvent which is used in the present reaction include solvents that do not interfere with reactions, such as N,N-dimethylformamide, N,N-dimethylacetamide, and dimethyl sulfoxide; and these solvents may be mixed with each other at an appropriate ratio and used.
This reaction can be carried out usually at room temperature to reflux temperature for 1 to 24 hours, and can be also carried out under microwave irradiation.

[Step 30-3]

This step is a method of producing compound [30-c] by deprotecting the pyrazolyl of compound [30-b] by removing protecting group $Pro^2$ under an acidic condition.
This reaction can be carried out by the method described in step 2-2 of production method 2 or a method pursuant thereto.
Compound [30-c] thus obtained can be isolated and purified by known separation and purification means such as concentration, concentration under reduced pressure, reprecipitation, solvent extraction, crystallization, and chromatography.
Among compound [I'] of the present invention, that type of compound [31-j] in which the structure represented by the following formula [III] is a structure of the following formula [VI] and $R^1$ is carboxy can be also produced, for example, by production method 31 described below or a method pursuant thereto.

[Formula 119]

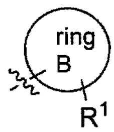
[III]

[Formula 120]

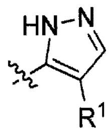
[VI]

Production method 31:

[Formula 121]

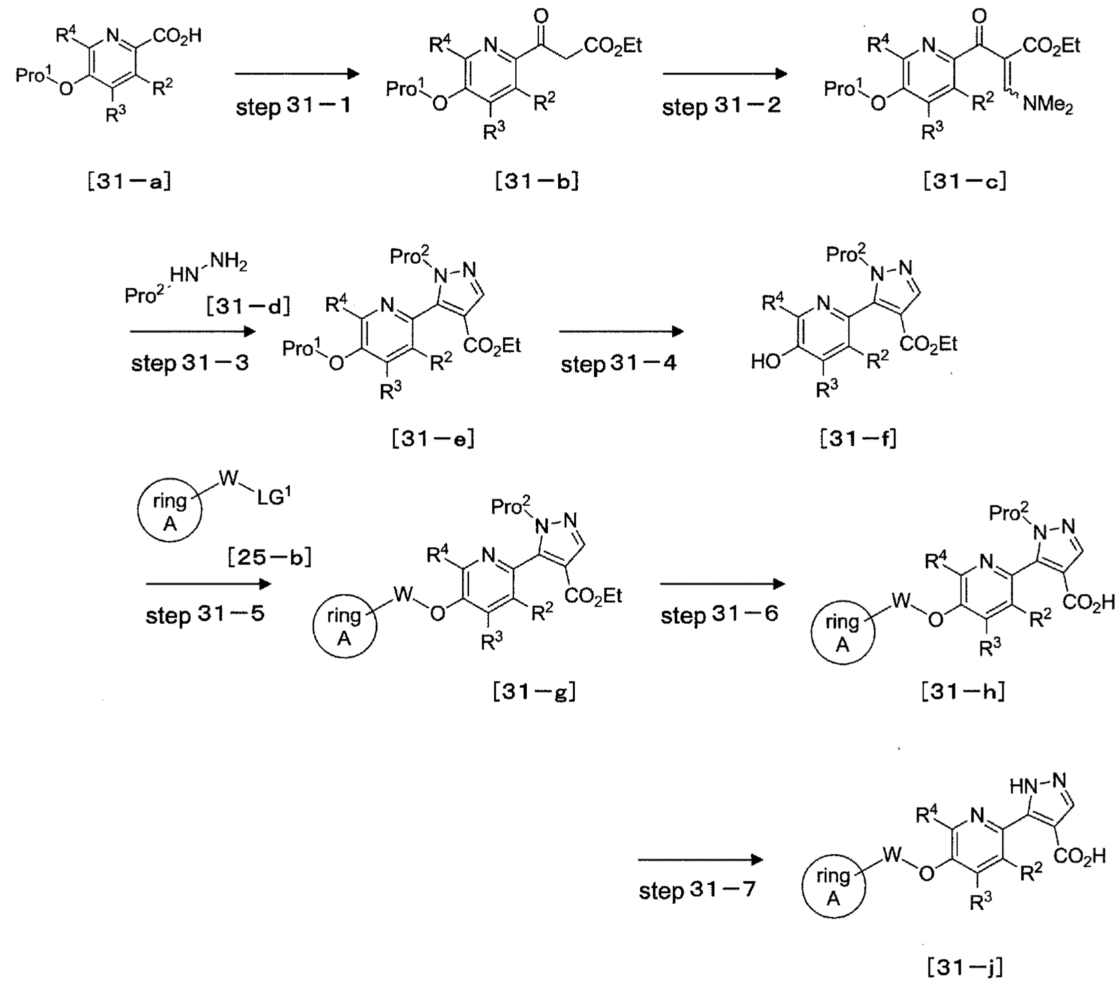


[31−a] [31−b] [31−c] [31−e] [31−f] [31−g] [31−h] [31−j]

[In the scheme, $R^2$, $R^3$, $R^4$, W, ring A, $LG^1$, $Pro^1$, and $Pro^2$ are the same as defined above.]

[Step 31-1]

This step is a method of producing compound [31-b] by converting the carboxy of compound [31-a] into β-ketoester.

The present reaction, which is a so-called Masamune reaction in which a malonic acid monoester magnesium salt and an amine are reacted with an active intermediate obtained from a carboxylic acid and 1,1'-carbonyl diimidazole (CDI), can be carried out by a process which is described in the literature (Angewandte Chemie Intarnational Edition in English, Vol. 18, page 72, 1979).

The amount of the CDI which is used in the present reaction is 1 to 2 equivalents, and preferably 1 to 1.4 equivalents, with respect to 1 equivalent of compound [31-a].

Examples of the solvent which is used in the present reaction include solvents that do not interfere with reactions, such as tetrahydrofurane and acetonitrile; and these solvents may be mixed with each other at an appropriate ratio and used.

This reaction can be carried out usually at room temperature for 0.5 to 6 hours.

The malonic acid monoester magnesium salt which is used in the present reaction can be obtained by purchasing commercial products, and more generally obtained by stirring a malonic acid monoester alkaline metal salt and magnesium chloride in the presence of an amine in a solvent that does not interfere with the reaction.

Examples of the malonic acid monoester alkaline metal salt which is used in the present reaction include a malonic acid monoethyl ester potassium salt. The amount of the malonic acid monoester alkaline metal salt to be used is 1 to 5 equivalents, and preferably 1 to 3 equivalents, with respect to 1 equivalent of compound [31-a].

The amount of the magnesium chloride which is used in the present reaction is 0.5 to 3 equivalents, and more

preferably 0.5 to 1.5 equivalents, with respect to the malonic acid monoester alkaline metal salt.

Examples of the amine which is used in the present reaction include triethylamine and diisopropylethylamine. The amount of the amine to be used is 1 to 5 equivalents, and preferably 1 to 2 equivalents, with respect to the malonic acid monoester alkaline metal salt.

Examples of the solvent which is used in the present reaction include solvents that do not interfere with reactions, such as tetrahydrofurane and acetonitrile; and these solvents may be mixed with each other at an appropriate ratio and used.

This reaction can be carried out usually at room temperature to reflux temperature for 0.5 to 12 hours.

The resulting active intermediate solution can be mixed with a solution of malonic acid monoester magnesium salt to give compound [31-b].

This reaction can be carried out usually at room temperature to reflux temperature for 1 to 24 hours.

[Step 31-2]

This step is a method of producing compound [31-c] from compound [31-b] and N,N-dimethylformamide dimethylacetal.

The amount of the N,N-dimethylformamide dimethylacetal which is used in the present reaction is from 1 equivalent to a solvent amount, and preferably 1 to 1.5 equivalents, with respect to compound [31-b].

Examples of the solvent which is used in the present reaction include solvents that do not interfere with reactions, such as chloroform, toluene, and xylene; and these solvents may be mixed with each other at an appropriate ratio and used, and the present reaction may be carried out in the absence of solvents.

This reaction can be carried out usually at a temperature from 60°C to reflux temperature for 0.5 to 6 hours.

[Step 31-3]

This step is a method of producing compound [31-e] by reacting compound [31-d] with compound [31-c].

Examples of compound [31-d] which is used in the present reaction include benzylhydrazine and tert-butylhydrazine. The amount of compound [31-d] to be used is 1 to 2 equivalents, and preferably 1 to 1.2 equivalents, with respect to compound [31-c].

Examples of the solvent which is used in the present reaction include solvents that do not interfere with reactions, such as ethanol, 2-propanol, and water; and these solvents may be mixed with each other at an appropriate ratio and used.

This reaction can be carried out usually at room temperature to reflux temperature for 0.5 to 6 hours.

[Step 31-4]

This step is a method of producing compound [31-f] by deprotecting the hydroxy of compound [31-e] by removing protecting group $Pro^1$.

Compound [31-f] can be synthesized, for example, by the method described in step 1-3 of production method 1 or a method pursuant thereto.

[Step 31-5]

This step is a method of producing compound [31-g] by reacting compound [31-f] with compound [25-b].

Compound [31-g] can be synthesized, for example, by the method described in step 25-1 of production method 25 or a method pursuant thereto.

[Step 31-6]

This step is a method of producing compound [31-h] by ester hydrolysis of compound [31-g] in the presence of a base.

Examples of the base which is used in the present reaction include aqueous lithium hydroxide, aqueous sodium hydroxide, and aqueous potassium hydroxide. The amount of the base to be used is usually 1 to 10 equivalents, and preferably 1 to 5 equivalents, with respect to 1 equivalent of compound [31-g].

Examples of the solvent which is used in the present reaction include solvents that do not interfere with reactions, such as methanol, ethanol, water, and tetrahydrofurane; and these solvents may be mixed with each other at an appropriate ratio and used.

This reaction can be carried out usually at a temperature from 0°C to room temperature for 1 to 24 hours.

[Step 31-7]

This step is a method of producing compound [31-j] from compound [31-h].

Compound [31-j] can be synthesized, for example, by the method described in step 2-2 of production method 2 or a method pursuant thereto.

Compound [31-j] thus obtained can be isolated and purified by known separation and purification means such as concentration, concentration under reduced pressure, reprecipitation, solvent extraction, crystallization, and chromatography.

Incidentally, compound [31-a] which is used as a starting compound in the above described production method 31 can be produced by a method known per se, or can be obtained by purchasing commercial products.

Among compound [I'] of the present invention, that type of compound [32-e] or compound [32-h] in which the structure represented by the following formula [III] is a structure of the following formula [VI] wherein $R^1$ is hydroxy for compound [32-e] or methoxy for compound [32-h] can be also produced, for example, by production method 32 described below or a method pursuant thereto.

[Formula 122]

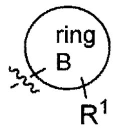
[III]

[Formula 123]

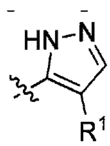
[VI]

Production method 32:

[Formula 124]

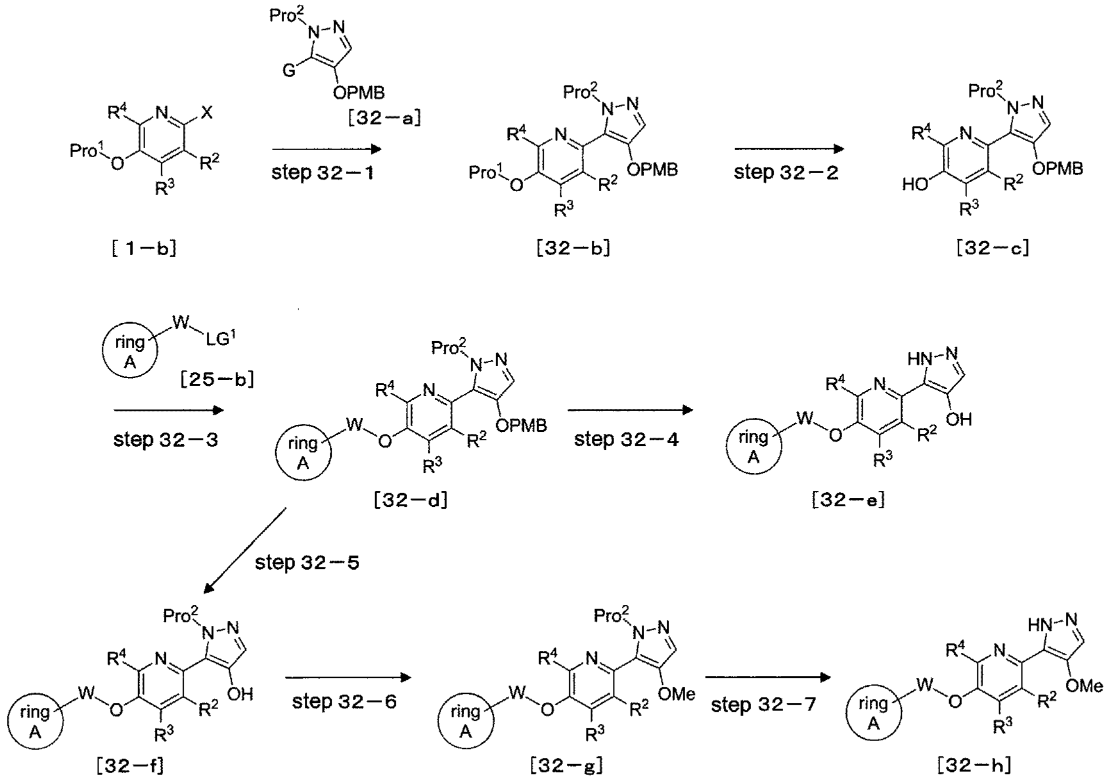


[In the scheme,

$R^2$, $R^3$, $R^4$, W, ring A, $LG^1$, $Pro^1$, $Pro^2$, and G are the same as defined above; and PMB represents p-methoxybenzyl.]

[Step 32-1]

This step is a method of producing compound [32-b] by reacting compound [1-b] with compound [32-a].

This reaction can be carried out by the method described in step 1-2 of production method 1 or a method pursuant thereto.

[Step 32-2]

This step is a method of producing compound [32-c] by deprotecting the hydroxy of compound [32-b] by removing protecting group $Pro^1$.

This reaction can be carried out by the method described in step 1-3 of production method 1 or a method pursuant thereto.

[Step 32-3]

This step is a method of producing compound [32-d] by reacting compound [32-c] with compound [25-b].

This reaction can be carried out by the method described in step 25-1 of production method 25 or a method pursuant thereto.

[Step 32-4]

This step is a method of producing compound [32-e] from compound [32-d].

Compound [32-e] can be synthesized, for example, by the method described in step 2-2 of production method 2 or a method pursuant thereto.

[Step 32-5]

This step is a method of producing compound [32-f] by removing the p-methoxybenzyl of compound [32-d].

This reaction can be carried out, for example, by the method described in step 1-3 of production method 1 or a process which is described in the literature (Protecting Groups in Organic Synthesis, 4th edition, 2007, edited by G. M. Wuts and T. W. Greene; pages 402-403), or a process pursuant thereto.

[Step 32-6]

This step is a method of producing compound [32-g] by methylating the hydroxy of compound [32-f].

This reaction can be carried out by a well-known method using various methylating agents.

[Step 32-7]

This step is a method of producing compound [32-h] from compound [32-g].

Compound [32-h] can be synthesized, for example, by the method described in step 2-2 of production method 2 or a method pursuant thereto.

Compounds [32-e] and [32-h] thus obtained can be isolated and purified by known separation and purification means such as concentration, concentration under reduced pressure, reprecipitation, solvent extraction, crystallization, and chromatography.

Incidentally, compound [32-a] which is used as a starting compound in the above described production method 32 can be produced by a method known per se.

EXAMPLES

The present invention will be described in more detail with reference to the following Reference Examples, Examples, and Test Examples, but these examples do not limit the present invention, and may be varied in such a range as not to deviate from the scope of the present invention.

In the following Reference Examples and Examples, packed columns (Reveleris (registered trademark) Flash Cartridges Silica manufactured by Grace or Biotage (registered trademark) SNAP Cartridge HP-Sphere manufactured by Biotage AB) were used for silica gel column chromatography. Packed columns (Reveleris (registered trademark) Flash Cartridges Amino manufactured by Grace or Biotage (registered trademark) SNAP Cartridge KP-NH manufactured by Biotage AB) were used for NH silica gel column chromatography. PLC plate 20 × 20 cm silica gel $60F_{254}$, 2 mm manufactured by Merck KGaA was used for preparative thin-layer chromatography. Unless otherwise stated, the ratio of eluent solvents is expressed as a volume ratio. The phase separator used was ISOLUTE (registered trademark) Phase Separator manufactured by Biotage AB.

Abbreviations used herein have the following meanings:

s: singlet
d: doublet
t: triplet
q: quartet
quin: quintet
sxt: sextet
spt: septet
dd: double doublet
dt: double triplet
td: triple doublet
tt: triple triplet
qd: quarter doublet
m: multiplet
br: broad
J: coupling constant
Hz: Hertz
CHLOROFORM-d: deuterated chloroform
DMSO-d6: deuterated dimethyl sulfoxide
MeOH-$d_4$: deuterated methanol
ACETONE-$d_6$: deuterated acetone

$D_2O$: deuterated water
THP: tetrahydropyranyl
TMS: trimethylsilyl
$^1$H-NMR (proton nuclear magnetic resonance spectrum) was measured using tetramethylsilane as an internal standard with Fourier transformed NMR as described below, and all $\delta$ values are expressed in ppm.
200 MHz: Gemini2000 (Agilent Technologies, Inc.)
300 MHz: Inova300 (Agilent Technologies, Inc.)
400 MHz: AVANCE III HD400 (Bruker Corporation)
500 MHz: JNM-ECA500 (JEOL Ltd.)
600 MHz: JNM-ECA600 (JEOL Ltd.)

ACD/Spectrus Processor 2015 ACD/Labs 2015 Release (File Version S30S41, Build 76327, 28 Feb 2014) (trade name) or the like was used for analysis. Very broad proton peaks shown by hydroxy, amino, amide, pyrazole, or the like are not indicated.

Mass Spectrum (MS) was measured on the following devices:

PlatformLC (Waters Corporation)
LCMS-2010EV (Shimadzu Corporation)
LCMS-IT-TOF (Shimadzu Corporation)
Agilent 6130 (Agilent Technologies, Inc.)
Agilent 6150 (Agilent Technologies, Inc.)

Ionization techniques used were Electrospray Ionization (ESI), Electron Ionization (EI), and dual ionization of ESI and Atmospheric Pressure Chemical Ionization (APCI). The values actually measured (which are described as "Found") are reported. Generally, molecular ion peaks are detected. However, for compounds having tert-butoxycarbonyl (-Boc), fragment ion peaks, which are peaks derived from the compounds that have lost tert-butoxycarbonyl or tert-butyl, may be detected. For compounds having tetrahydropyranyl (THP), fragment ion peaks, which are peaks derived from the compounds that have lost tetrahydropyranyl, may be also detected. For compounds having hydroxy (-OH), fragment peaks, which are peaks derived from compounds that have lost $H_2O$, may be also detected. For salts, molecular ion peaks of free forms or fragment ion peaks are typically observed.

LC-MS was performed in the Examples and Reference Examples under the following conditions:

HPLC: Agilent 1290 Infinity
MS: Agilent 6130 or 6150
[HPLC conditions]
Column: Acquity UPLC CSH C18, 1.7 μm, 2.1x × 50 mm (WATERS Corporation)
Solvent: solution A; water with 0.1% formic acid, solution B; acetonitrile with 0.1% formic acid

(Method A)

Gradient: 0.00 min (solution A/solution B = 80/20), 1.20 min (solution A/solution B = 1/99), 1.40 min (solution A/solution B = 1/99), 1.41 min (solution A/solution B = 80/20), 1.50 min (solution A/solution B = 80/20)

(Method B)

Gradient: 0.00 min (solution A/solution B = 95/5), 0.80 min (solution A/solution B = 60/40), 1.08 min (solution A/solution B = 1/99), 1.38 min (solution A/solution B = 1/99), 1.41 min (solution A/solution B = 95/5), 1.50 min (solution A/solution B = 80/20)

(Method C)

Gradient: 0.00 min (solution A/solution B = 70/30), 0.80 min (solution A/solution B = 1/99), 1.40 min (solution A/solution B = 1/99), 1.42 min (solution A/solution B = 70/30), 1.50 min (solution A/solution B = 70/30)
Injection volume: 0.5 μL; Flow rate: 0.8 mL/min
Detection: UV 210 nm, 254 nm
HPLC equipped with ELSD: Agilent 385-ELSD
MS condition
Ionization: ESI or ESI/APCI multimode

Purification by preparative HPLC was performed in the Examples and Reference Examples under the following conditions:

Equipment: High-throughput purification system from Gilson, Inc.
Column: Triart C18, 5 μm, 30 × 50 mm (YMC Co., Ltd.) or X-Bridge Prep C18 5 um OBD, 30 × 50 (Waters Corporation)
Solvent: solution A; water with 0.1% formic acid, solution B; acetonitrile with 0.1% formic acid, or solution A; water with 0.1% trifluoroacetic acid, solution B; acetonitrile with 0.1% trifluoroacetic acid

(Method A)

Gradient: 0.00 min (solution A/solution B = 90/10), 2.00 min (solution A/solution B = 90/10), 11.0 min (solution A/solution B = 20/80), 12.0 min (solution A/solution B = 5/95), 13.52 min (solution A/solution B = 5/95), 15.0 min (solution A/solution B = 90/10)

(Method B)

Gradient: 0.00 min (solution A/solution B = 95/5), 3.00 min (solution A/solution B = 95/5), 8.53 min (solution A/solution B = 80/20), 10.0 min (solution A/solution B = 80/20), 11.0 min (solution A/solution B = 50/50), 12.02 min (solution A/solution B = 5/95), 13.5 min (solution A/solution B = 5/95), 13.65 min (solution A/solution B = 95/5), 15.0 min (solution A/solution B = 95/5)

(Method C)

Gradient: 0.00 min (solution A/solution B = 80/20), 2.00 min (solution A/solution B = 80/20), 10.0 min (solution A/solution B = 5/95), 11.5 min (solution A/solution B = 1/99), 13.5 min (solution A/solution B = 1/99), 13.55 min (solution A/solution B = 80/20), 15.0 min (solution A/solution B = 5/95), 15.0 min (solution A/solution B = 95/5)
Flow rate: 40 mL/min
Detection: UV 210 nm, UV 254 nm
HPLC equipped with ELSD: SofTA MODEL 300S ELSD

Purification by preparative LC-MS was performed in the Examples and Reference Examples under the following conditions:

HPLC: Agilent 1260 Infinity
[HPLC conditions]
Column: X-SELECT CSH C18, 5 μm, OBD, 30 × 50 (Waters Corporation)
Solvent: solution A; water with 0.1% formic acid, solution B; acetonitrile with 0.1% formic acid, or solution A; water with 0.1% trifluoroacetic acid, solution B; acetonitrile with 0.1% trifluoroacetic acid

(Method A)

Gradient: 0.00 min (solution A/solution B = 90/10), 0.50 min (solution A/solution B = 90/10), 7.50 min (solution A/solution B = 20/80), 7.95 min (solution A/solution B = 20/80), 8.00 min (solution A/solution B = 5/95), 9.00 min (solution A/solution B = 5/95), 9.05 min (solution A/solution B = 90/10), 10.0 min (solution A/solution B = 90/10)

(Method B)

Gradient: 0.00 min (solution A/solution B = 95/5), 0.50 min (solution A/solution B = 95/5), 7.50 min (solution A/solution B = 50/50), 7.95 min (solution A/solution B = 50/50), 8.00 min (solution A/solution B = 5/95), 9.00 min (solution A/solution B = 5/95), 9.05 min (solution A/solution B = 95/5), 10.00 min (solution A/solution B = 95/5)

(Method C)

Gradient: 0.00 min (solution A/solution B = 80/20), 0.50 min (solution A/solution B = 80/20), 7.00 min (solution A/solution B = 5/95), 7.45 min (solution A/solution B = 5/95), 7.50 min (solution A/solution B = 1/99), 9.00 min (solution A/solution B = 1/99), 9.20 min (solution A/solution B = 80/20), 10.0 min (solution A/solution B = 80/20)
Flow rate: 50 mL/min
Detection: UV 210 nm, UV 254 nm
MS: Agilent 6130

HPLC equipped with ELSD: Agilent 385 ELSD

MS condition

Ionization: ESI or ESI/APCI multimode

Chiral HPLC analysis was performed in the Examples and Reference Examples under the following conditions:

HPLC: Nexera Quaternary System from Shimadzu Corporation
[HPLC conditions]
Column: CHIRALPAK AY-3, 3 μm, 4.6x × 150 mm (Daicel Corporation)
Solvent: solution A; n-hexane, solution B; ethanol
Elution condition: solution A/solution B = 80/20 (isocratic)
Injection volume: 3 μL; Flow rate: 1.0 mL/min
Detection: UV 210 nm, 254 nm

Preparative chiral HPLC was performed in the Examples and Reference Examples under the following conditions:
HPLC: High-throughput purification system from Gilson, Inc.

[HPLC conditions]

Column: CHIRALPAK AY-H, 5 μm, 20x × 250 mm (Daicel Corporation)
Solvent: solution A; n-hexane, solution B; ethanol
Elution condition: solution A/solution B = 80/20 (isocratic)
Flow rate: 10.0 mL/min
Detection: UV 210 nm, 254 nm

The microwave reactor used was Initiator from Biotage AB or MONOWAVE 300 from Anton-Paar GmbH.

Compound names were designated using ACD/Name (ACD/Labs 2015, Advanced Chemistry Development Inc.).

Conformations of compounds in the Reference Examples and Examples are shown in the absolute configuration of its asymmetric carbon. A compound with the designation of absolute configuration of its asymmetric carbon is an optically active substance.

The present invention will be described in more detail with reference to the following Reference Examples, Examples, Test Examples and Preparation Examples, but these examples do not limit the present invention, and may be varied in such a range as not to deviate from the scope of the present invention.

Reference Example 1-1

6-[1-(Oxan-2-yl)-1H-pyrazol-5-yl]pyridin-3-ol

[Formula 125]

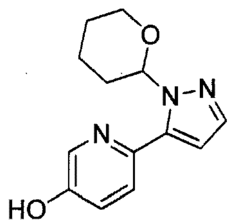

(1) Potassium carbonate (20.65 g) and benzyl bromide (10.6 mL) were added to a solution of 6-bromopyridin-3-ol

(13.00 g) in acetone (250 mL) under ice cooling, and the mixture was stirred at room temperature for 2 hours. After the solvent was distilled off under reduced pressure, water was added to the residue, and the mixture was extracted with ethyl acetate. The organic layer was separated by a phase separator, and the solvent was distilled off under reduced pressure. The obtained residue was purified by silica gel column chromatography (n-hexane/ethyl acetate = 9:1 to 4:1) to give 5-(benzyloxy)-2-bromopyridine (16.71 g) as a colorless powder.

(2) Dimethoxyethane (120 mL) and water (60 mL) were added to a mixture of the compound (10.00 g) obtained in the above described (1), 1-(2-tetrahydropyranyl)1H-pyrazole-5-boronic acid pinacol ester (15.80 g), sodium carbonate (12.04 g) and a 1,1'-bis (diphenylphosphino)ferrocene palladium(II) dichloride dichloromethane adduct (1.55 g); and the resultant mixture was heated to reflux at 100°C under a nitrogen atmosphere for 7 hours. After cooling to room temperature, the resultant solution was passed through Celite (registered trademark) to remove insolubles, and the filtrate was concentrated under reduced pressure. Water was added to the residue, and the mixture was extracted with chloroform. The organic layer was separated by a phase separator, and the solvent was distilled off under reduced pressure. The obtained residue was purified by silica gel column chromatography (n-hexane/ethyl acetate = 7:3 to 1:1) to give 5-(benzyloxy)-2-[1-(oxan-2-yl)-1H-pyrazol-5-yl]pyridine (11.04 g) as a pale orange oily substance.

(3) To a solution of the compound (11.04 g) obtained in the above described (2) in ethanol (40 mL) and ethyl acetate (40 mL), 10% palladium-carbon (1.10 g) was added; and the mixture was stirred under a hydrogen atmosphere at room temperature for 2 hours. After the reaction solution was filtered through Celite (registered trademark), the filtrate was concentrated; the obtained residue was purified by silica gel column chromatography (chloroform only, to chloroform/methanol = 92:8), and was powdered from diethyl ether/n-hexane to give the title compound (6.18 g) as a colorless powder.

$^1$H NMR (400 MHz, DMSO-$d_6$) $\delta$ ppm 1.43 - 1.70 (m, 3 H) 1.83 - 1.91 (m, 1 H) 1.94 - 2.03 (m, 1 H) 2.30 - 2.42 (m, 1 H) 3.41 - 3.54 (m, 1 H) 3.81 - 3.88 (m, 1 H) 6.11 - 6.17 (m, 1 H) 6.56 - 6.60 (m, 1 H) 7.24 - 7.29 (m, 1 H) 7.48 - 7.59 (m, 2 H) 8.20 - 8.24 (m, 1 H) 10.21 (s, 1 H).

MS ESI/APCI Multi posi: 246 [M+H]$^+$.

The compounds of the following Reference Examples 1-2 and 1-5 were synthesized using a commercially available corresponding boronic acid ester or the compound obtained in Reference Example 65-1 described later, according to the method described in Reference Examples 1-1-(2) to 1-1-(3). Reference Examples 1-3 to 1-4 were synthesized using a commercially available corresponding hydroxypyridine according to the method described in Reference Example 1-1. These structures, NMR data and MS data are shown in Tables 1-1 to 1-2.

[Table 1-1]

| Reference Example No. | Structure | Analytical Data |
|---|---|---|
| 1-2 | 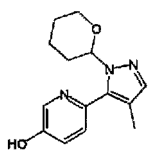 | $^1$H NMR (400 MHz. DMSO-$d_6$) $\delta$ ppm 1:41 - 1.62 (m, 3 H) 1.75 - 1.85 (m, 1 H) 1.90 - 2.03 (n, 4 H) 2. 25 - 2.37 (m, 1 H) 3.77 - 3.84 (m, 1 H) 5.50 - 5.56 (m, 1 H) 7.26 - 7.32 (m, 1 H) 7.35 - 7.40 (m, 2 H) 8.24 - 8.28 (m, 1 H) 10.23 (br s, 1 H). MS ESI/APCI Multi posi: 260[M+H]$^+$. MS ESI/APCI Multi nega: 258[M-H]$^-$. |
| 1-3 | 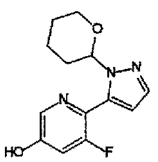 | $^1$H NMR (400 MHz, DMSO-$d_6$) $\delta$ ppm 1.42 - 1.68 (m, 3 H) 1.85 - 2.03 (m, 2 H) 2.26 - 2.39 (m, 1 H) 3.34 - 3.43 (m, 1 H) 3.73 - 3.81 (m, 1 H) 5.91 - 5.97 (m, 1 H) 6.52 - 6.57 (m, 1 H) 7.18 - 7.25 (m, 1 H) 7.56 - 7.60 (m, 1 H) 8.12 - 8.17 (m, 1 H) 10.81 (br s, 1 H). MS ESI nega: 262[M-H]$^-$. |
| 1-4 | 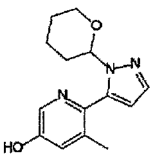 | $^1$H NMR (600 MHz, CHLOROFORM-d) $\delta$ ppm 1.45 - 1.50 (m, 1 H) 1.51 - 1.69 (m, 2 H) 1.92 - 2. 07 (m, 2 H) 2.19 (s, 3 H) 2.37 - 2.47 (m, 1 H) 3.34 - 3.41 (m, 1 H) 3.91 - 3.95 (m, 1 H) 5.21 - 5.24 (m, 1 H) 6.33 (d, J=1.7 Hz, 1 H) 7.06 (d, J=2. 5 Hz. 1 H) 7.63 (d, J=1. 7 Hz, 1 H) 8.13 (d, J=2.5 Hz, 1 H). MS ESI/APCI Multi posi: 260 [M+H]$^+$. MS ESI/APCI Multi nega: 258[M-H]$^-$. |

[Table 1-2]

| Reference Example No. | Structure | Analytical Data |
|---|---|---|
| 1-5 | 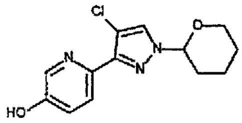 | $^1$H NMR (400 MHz, CHLOROFORM-d) δ ppm 1.37 - 1.77 (m, 4 H) 1.92 - 2. 17 (m 2 H) 3.60 - 3.75 (m, 1 H) 4.00 - 4.12 (m, 1 H) 5.32 - 6.46 (m, 1 H) 7.16 - 7.33 (m, 1 H) 7.65 - 7.73 (m, 1 H) 7.80 - 7.90 (m, 1 H) 8.28 - 8.38 (m, 1 H). MS ESI/APCI Multi posi: 280[M+H]$^+$. MS ESI/APCI Multi nega: 278[M-H]$^-$. |

Reference Example 1-6

6-[1-(Oxan-2-yl)pyrazol-4-yl]pyridin-3-ol

[Formula 126]

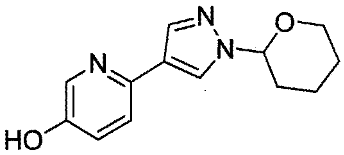

(1) 4-(5,5-Dimethyl-1,3,2-dioxaborinan-2-yl)-1-(oxan-2-yl)pyrazole (500 mg) and sodium carbonate (505 mg) were added to a solution of the compound (420 mg) obtained in Reference Example 1-1-(1) in toluene (3 mL), ethanol (3 mL) and water (3 mL), which is followed by purging with nitrogen. Tetrakis(triphenylphosphine)palladium(0) (91.8 mg) was added thereto, and the resultant mixture was stirred at 90°C for 2 hours under a nitrogen atmosphere. Chloroform was added thereto, and the organic layer was separated by a phase separator, and was then concentrated. The concentrate was purified by silica gel column chromatography (n-hexane only to ethyl acetate only) to give a mixture (707 mg) containing 2-[1-(oxan-2-yl)pyrazol-4-yl]-5-phenylmethoxypyridine as a yellow oily substance.
(2) The mixture (707 mg) obtained in the above described (1) was used to perform the synthesis process according to the method described in Reference Example 1-1-(3) thereby giving the title compound (390 mg) as a colorless amorphous substance.
$^1$H NMR (400 MHz, CHLOROFORM-d) δ ppm 1.50 - 1.77 (m, 3 H) 1.99 - 2.19 (m, 3 H) 3.65 - 3.78 (m, 1 H) 4.03 - 4.09 (m, 1 H) 5.41 (dd, J=9.2, 2.9 Hz, 1 H) 7.19 (dd, J=8.6, 2.8 Hz, 1 H) 7.37 (d, J=8.6 Hz, 1 H) 7.94 (s, 1 H) 8.08 (s, 1 H) 8.19 (d, J=2.8 Hz, 1 H). MS ESI/APCI Multi posi: 246 [M+H]$^+$.

Reference Example 1-7

6-(1-Benzyltriazol-4-yl)pyridin-3-ol

[Formula 127]

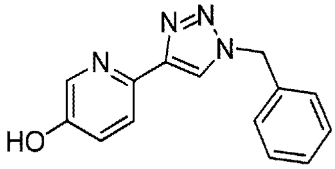

(1) The compound (527 mg) obtained in Reference Example 1-1-(1), 2-(1-benzyltriazol-4-yl)-6-methyl-1,3,6,2-dioxazaborocan-4,8-dione (502 mg), XPhosPdG2 (125 mg), copper(II) acetate monohydrate (159 mg) and potassium carbonate (1.54 g) were mixed in acetonitrile (8 mL) and 2-propanol (2 mL); and the mixture was stirred at 120°C for 30 minutes and at 140°C for 2 hours, under a nitrogen atmosphere and under microwave irradiation. The reaction mixture was purified by silica gel column chromatography (n-hexane only to n-hexane/ethyl acetate = 1:1), subsequently by NH silica gel chromatography (n-hexane only to ethyl acetate only), and subsequently by NH silica gel chromatography (n-hexane only to n-hexane/ethyl acetate = 1:1) to give a mixture (502 mg) containing 2-(1-benzyltriazol-4-yl)-5-phenylmethoxypyridine as a pale yellow powder.

(2) The mixture (153 mg) obtained in the above described (1) was used to perform the synthesis process according to the method described in Reference Example 1-1-(3) thereby giving the title compound (52.7 mg) as a colorless powder.

$^1$H NMR (400 MHz, DMSO-$d_6$) δ ppm 5.63 (s, 2 H) 7.25 (dd, J=8.6, 2.9 Hz, 1 H) 7.30 - 7.42 (m, 5 H) 7.85 (d, J=8.6 Hz, 1 H) 8.12 (d, J=2.9 Hz, 1 H) 8.46 (s, 1 H) 9.72 - 10.43 (m, 1 H).

MS ESI/APCI Multi posi: 253 [M+H]$^+$.

Reference Example 2-1

2-[1-(Oxan-2-yl)-1H-pyrazol-5-yl]-5-[(piperidin-4-yl)methoxy]pyridine

[Formula 128]

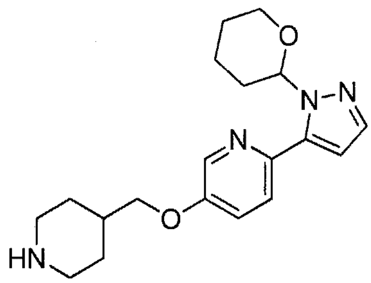

(1) The compound (1.11 g) obtained in Reference Example 19-1 described later, tributylphosphine (1.50 mL) and 1,1'-azobis(N,N-dimethylformamide) (1.04 g) were added to a solution of the compound (1.00 g) obtained in Reference Example 1-1 in tetrahydrofuran (40 mL), the mixture was stirred at 60°C for 3 hours, and then was stirred at room temperature overnight. After the reaction solution was concentrated, water was added to the concentrated solution, and the mixture was extracted with ethyl acetate. After the extracted substance was dried over anhydrous sodium sulfate, the drying agent was filtered off, and the solvent was distilled off under reduced pressure. The obtained residue was purified by silica gel column chromatography (n-hexane/ethyl acetate = 9:1 to 0:1) to give 4-[({6-[1-(oxan-2-yl)-1H-pyrazol-5-yl]pyridin-3-yl}oxy)methyl]piperidine-1-benzyl carboxylate (1.01 g) as a colorless oily substance.

(2) To a solution of the compound (1.01 g) obtained in the above described (1) in methanol (15 mL), 20% palladium-carbon (200 mg) was added, and the mixture was stirred under a hydrogen atmosphere at room temperature for 30 minutes. After the reaction solution was filtered through Celite (registered trademark), the filtrate was concentrated, the obtained residue was purified by NH silica gel column chromatography (n-hexane/ethyl acetate = 1:1, to ethyl acetate only, to chloroform/methanol = 19:1 to 9:1) to give the title compound (561 mg) as a colorless oily substance.

$^1$H NMR (600 MHz, CHLOROFORM-d) δ ppm 1.23 - 1.38 (m, 3 H) 1.51 - 1.80 (m, 3 H) 1.82 - 1.87 (m, 2 H) 1.93 - 2.04 (m, 2 H) 2.06 - 2.12 (m, 1 H) 2.49 - 2.57 (m, 1 H) 2.67 (td, J=12.2, 2.5 Hz, 2 H) 3.12 - 3.17 (m, 2 H) 3.59 (td, J=11.6, 2.5 Hz, 1 H) 3.89 (d, J=6.6 Hz, 2 H) 4.02 - 4.07 (m, 1 H) 6.08 (dd, J=9.9, 2.5 Hz, 1 H) 6.49 (d, J=1.7 Hz, 1 H) 7.23 - 7.27 (m, 1 H) 7.53 (d, J=8.7 Hz, 1 H) 7.59 (d, J=1.7 Hz, 1 H) 8.36 (d, J=2.9 Hz, 1 H).

MS ESI/APCI Multi posi: 343 [M+H]$^+$.

The compounds of the following Reference Examples 2-2 to 2-14 were synthesized using the compound obtained in Reference Example 1 and the compounds obtained in Reference Examples 19, 20 and 21 described later, according to the method described in Reference Example 2-1. These structures, NMR data and MS data are shown in Tables 2-1 to 2-2.

[Table 2-1]

| Reference Example No. | Structure | Analytical Data |
|---|---|---|
| 2-2 | 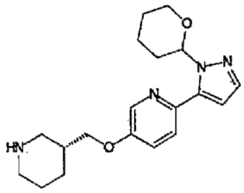 | $^1$H NMR (600 MHz, CHLOROFORM-d) δ ppm 1.23 - 1.35 (m, 1 H) 1.49 - 1.80 (m, 5 H) 1. 87 - 1.96 (m, 1 H) 1.99 - 2.11 (m, 3 H) 2.47 - 2.57 (m, 2 H) 2.59 - 2.66 (m, 1 H) 3.00 - 3.08 (m, 1 H) 3.21 - 3.29 (m, 1 H) 3.55 - 3.63 (m, 1 H) 3.86 - 3.94 (m, 2 H) 4.00 - 4.09 (m, 1 H) 6.04 - 6.10 (m, 1 H) 6.49 (d, J=1.9 Hz, 1 H) 7.25 (dd, J=8.7, 3.1 Hz, 1 H) 7.52 (d, J=8. 7 Hz, 1 H) 7.59 (d, J=1.9 Hz, 1 H) 8.36 (d, J=3.1 Hz, 1 H). MS ESI/APCI Multi posi: 343[M+H]$^+$. |
| 2-3 | 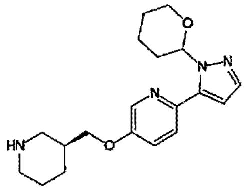 | $^1$H NMR (600 MHz, CHLOROFORM-d) δ ppm 1.23 - 1.35 (m, 1 H) 1.49 - 1.80 (m, 5 H) 1.87 - 1.96 (m, 1 H) 1.99 - 2.11 (m, 3 H) 2.47 - 2.57 (m, 2 H) 2.59 - 2.66 (m, 1 H) 3.00 - 3.08 (m, 1 H) 3.21 - 3.29 (m. 1 H) 3.55 - 3.63 (m, 1 H) 3.86 - 3.94 (m, 2 H) 4.00 - 4.09 (m, 1 H) 6.04 - 6.10 (m, 1 H) 6.49 (d, J=1. 9 Hz, 1 H) 7.25 (dd, J=8.7, 3.1 Hz, 1 H) 7.52 (d, J=8. 7 Hz, 1 H) 7.59 (d, J=1.9 Hz, 1 H) 8.36 (d, J=3.1 Hz, 1 H). MS ESI/APCI Multi posi: 343[M+H]$^+$. |
| 2-4 | 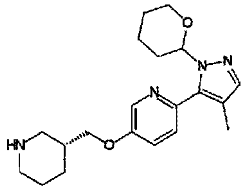 | MS ESI/APCI Multi posi: 357[M+H]$^+$. |
| 2-5 | 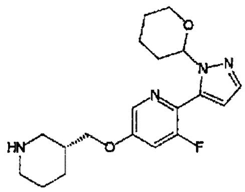 | MS ESI/APCI Multi posi: 361[M+H]$^+$. |
| 2-6 | 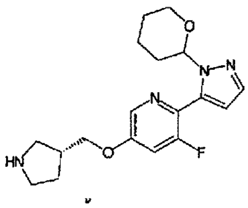 | $^1$H NMR (400 MHz, CHLOROFORM-d) δ ppm 1.50 - 1.77 (m, 5 H) 1.97 - 2.13 (m, 3 H) 2.47 - 2.67 (m, 2 H) 2.78 - 2.85 (m, 1 H) 2.92 - 3.06 (m, 2 H) 3.12 - 3.19 (m, 1 H) 3.50 - 3.59 (m, i H) 3.93 - 4.04 (m, 3 H) 6.09 - 6.14 (m, 1 H) 6.62 - 6.65 (m, 1 H) 7.02 - 7.07 (m, 1 H) 7.62 - 7.65 (m, 1 H) 8.22 - 8.25 (m, 1 H).<br>MS ESI/APCI Multi posi: 347[M+H]$^+$. |
| 2-7 | 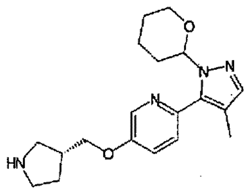 | $^1$H NMR (400 MHz, CHLOROFORM-d) δ ppm 1.41 - 1.77 (m, 3 H) 1.91 - 2.11 (m, 8 H) 2.45 - 2.69 (m, 2 H) 2.80 - 2.87 (m, 1 H) 2.92 - 3.07 (m, 2 H) 3.13 - 3.20 (m, 1 H) 3.43 - 3.50 (m, 1 H) 3.95 - 4.06 (m, 3 H) 5.49 - 5.54 (m, 1 H) 7.27 - 7.31 (m, 1 H) 7.39 - 7.47 (m, 2 H) 8.41 - 8.44 (m, 1 H).<br>MS ESI/APCI Multi posi: 343[M+H]$^+$. |

[Table 2-2]

| Reference Example No. | Structure | Analytical Data |
|---|---|---|
| 2-9 | 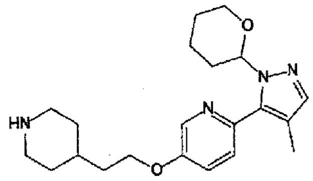 | MS ESI/APCI Multi posi: 371 $[M+H]^+$. |
| 2-10 | 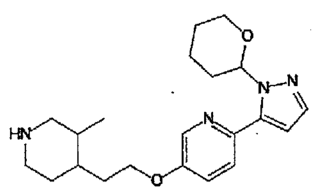 | $^1$H NMR (300 MHz, CHLOROFORM-d) δ ppm 0.91 - 1.00 (m, 3 H) 1.44 - 1.84 (m, 10 H) 1.98 - 2.14 (m, 2 H) 2.47 - 2.70 (m, 2 H) 2.81 - 2.83 (m, 1 H) 2.98 - 3.12 (m, 1 H) 3.55 - 3.64 (m, 1 H) 4.01 - 4.16 (m, 3 H) 6.05 - 6.11 (m. 1 H) 6.49 - 6.50 (m, 1 H) 7.21 - 7.25 (m, 1 H) 7.51 - 7.55 (m, 1 H) 7.58 - 7.61 (m, 1 H) 8.35 - 8.38 (m, 1 H). MS ESI/APCI Multi posi: 371$[M+H]^+$. |
| 2-11 | 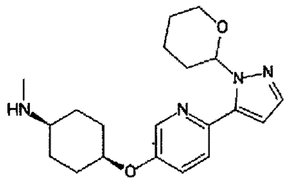 | $^1$H NMR (400 MHz, CHLOROFORM-d) δ ppm 1.04 - 2.57 (m, 17 H) 3.57 - 3.67 (m, 2 H) 4.02 - 4.08 (m, 1 H) 4.54 - 4.60 (m, 1 H) 6.07 - 6.12 (m, 1 H) 6.49 (d, J=1.8 Hz, 1 H) 7.24 (br d, J=2.9 Hz, 1 H) 7.52 (d, J=8.6 Hz, 1 H) 7.58 (d, J=1. 8 Hz, 1 H) 8.36 (d, J=2.9 Hz, 1 H). MS ESI/APCI Multi posi: 357$[M+H]^+$. |
| 2-12 | 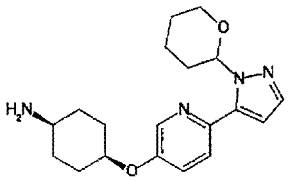 | $^1$H NMR (400 MHz, CHLOROFORM-d) δ ppm 1.46 - 2.24 (m, 13 H) 2.47 - 2.58 (m, 1 H) 3.18 - 3.26 (m, 1 H) 3.56 - 3.64 (m, 1 H) 4.01 - 4.07 (m, 1 H) 4.59 - 4.63 (m, 1 H) 6.07 - 6.12 (m, 1 H) 6.48 (d, J=1.8 Hz, 1 H) 7.27 - 7.31 (m, 1 H) 7.50 - 7.60 (m, 2 H) 8.39 (d, J=2.5 Hz, 1 H). MS ESI/APCI Multi posi: 343$[M+H]^+$. |
| 2-13 | 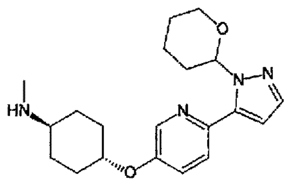 | $^1$H NMR (400 MHz, CHLOROFORM-d) δ ppm 1.44 - 2.29 (m, 13 H) 2.48 - 2.61 (m, 4 H) 2.67 - 2.77 (m, 1 H) 3.53 - 3.70 (m, 1 H) 3.98 - 4.08 (m, 1 H) 4.21 - 4.40 (m, 1 H) 6.07 - 6.12 (m, 1 H) 6.49 (d, J=1. 8 Hz, 1 H) 7.25 (dd, J=8.8, 2.8 Hz, 1 H) 7.53 (d, J=8.8 Hz, 1 H) 7.58 (d, J=1.8 Hz, 1 H) 8.35 (d, J=2.B Hz, 1 H). MS ESI/APCI Multi posi: 357$[M+H]^+$. |
| 2-14 | 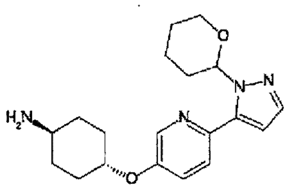 | 'H NMR (400 MHz, CHLOROFORM-d) δ ppm 1.48 - 2.38 (m, 13 H) 2.45 - 2.59 (m, 1 H) 3.24 - 3.32 (m, 1 H) 3.56 - 3.63 (m, 1 H) 4.01 - 4.07 (m, 1 H) 4.34 - 4.41 (m, 1 H) 6.06 - 6.11 (m, 1 H) 6.50 (d, J=1. 8 Hz, 1 H) 7.24 (dd, J=8. 7, 2.8 Hz, 1 H) 7.53 (d, J=8.7 Hz, 1 H) 7.59 (d, J=1. 8 Hz, 1 H) 8.35 (d, J=2.8 Hz, 1 H). MS ESI/APCI Multi posi: 343$[M+H]^+$. |

Reference Example 3-1

5-[(Azetidin-3-yl)methoxy]-2-[1-(oxan-2-yl)-1H-pyrazol-5-yl]pyridine

[Formula 129]

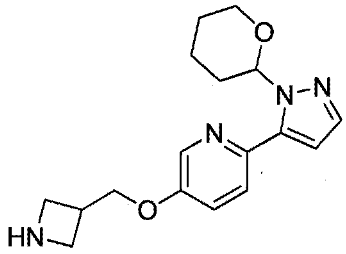

(1) The compound (397 mg) obtained in Reference Example 1-1 and a commercially available compound (1-benzhydrylazetidin-3-yl)methanol (533 mg) were used to perform the synthesis process according to the method described in Reference Example 2-1-(1) thereby giving a mixture (612 mg) containing 5-{[1-(diphenylmethyl)azetidin-3-yl]methoxy}-2-[1-(oxan-2-yl)-1H-pyrazol-5-yl]pyridine as a yellow amorphous substance.

(2) To a solution of the mixture (612 mg) obtained in the above described (1) in methanol (5 mL), 20% palladium-carbon (30 mg) was added, and the mixture was stirred under a hydrogen atmosphere at room temperature for 1 hour and at 60°C for 3 hours. The reaction solution was filtered through Celite (registered trademark), and the filtrate was concentrated to thereby give a mixture (288 mg) containing the title compound as a colorless oily substance.

$^1$H NMR (600 MHz, CHLOROFORM-d) δ ppm 1.49 - 1.60 (m, 1 H) 1.61 - 1.80 (m, 3 H) 1.97 - 2.04 (m, 1 H) 2.04 - 2.13 (m, 1 H) 2.44 - 2.59 (m, 1 H) 3.24 - 3.42 (m, 1 H) 3.54 - 3.69 (m, 1 H) 4.02 - 4.12 (m, 3 H) 4.22 - 4.34 (m, 4 H) 6.05 - 6.10 (m, 1 H) 6.51 (d, J=1.7 Hz, 1 H) 7.43 (dd, J=8.6, 3.1 Hz, 1 H) 7.57 (d, J=8.6 Hz, 1 H) 7.60 (d, J=1.7 Hz, 1 H) 8.48 (d, J=3.1 Hz, 1 H).

MS ESI/APCI Multi posi: 315 $[M+H]^+$.

Reference Example 4-1

5-[(3-Bromophenyl)methoxy]-2-[1-(oxan-2-yl)-1H-pyrazol-5-yl]pyridine

[Formula 130]

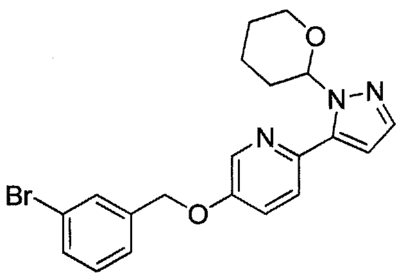

3-Bromobenzyl alcohol (2.75 g) and cyanomethylene tributylphosphorane (9.63 mL) were added to a solution of the compound (3.00 g) obtained in Reference Example 1-1 in toluene (31 mL), and the mixture was stirred at 100°C for 1.5 hours. The reaction mixture was concentrated, the obtained residue was purified by silica gel column chromatography (n-hexane only, to ethyl acetate only) to give the title compound (4.70 g) as a brown oily substance. $^1$H NMR (300 MHz, CHLOROFORM-d) δ ppm 1.51 - 1.83 (m, 3 H) 1.97 - 2.13 (m, 2 H) 2.45 - 2.61 (m, 1 H) 3.52 - 3.68 (m, 1 H) 3.97 - 4.08 (m, 1 H) 5.13 (s, 2 H) 5.99 - 6.21 (m, 1 H) 6.50 (d, J=1.9 Hz, 1 H) 7.23 - 7.42 (m, 3 H) 7.47 - 7.52 (m, 1 H) 7.52 - 7.57 (m, 1 H) 7.59 (d, J=1.9 Hz, 1 H) 7.60 - 7.65 (m, 1 H) 8.41 - 8.46 (m, 1 H).

MS ESI/APCI Multi posi: 414 $[M+H]^+$.

Reference Example 7-1

2-{(3R)-3-[({6-[1-(Oxan-2-yl)-1H-pyrazol-5-yl]pyridin-3-yl}oxy)methyl]piperidine-1-sulfonyl}ethane-1 -amine

[Formula 131]

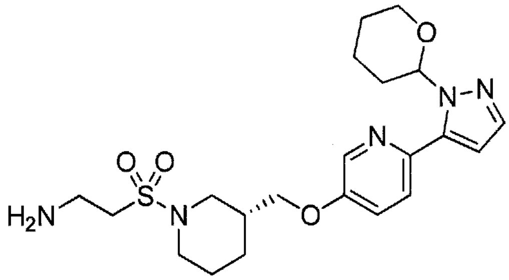

(1) Triethylamine (162 μL) and benzyl N-(2-chlorosulfonylethyl)carbamate (194 mg) were added to a solution of the compound (200 mg) obtained in Reference Example 2-2 in tetrahydrofuran (5 mL), and the mixture was stirred at room temperature for 2 hours. After the reaction solution was concentrated, the obtained residue was purified by silica gel column chromatography (n-hexane/ethyl acetate = 1:1, to ethyl acetate only, and subsequently chloroform/methanol = 19:1 to 9:1) to give benzyl (2-{(3R)-3-[({6-[1-(oxan-2-yl)-1H-pyrazol-5-yl]pyridin-3-yl}oxy)methyl]piperidine-1-sulfonyl}ethyl)carbamate (318 mg) as a colorless amorphous substance.
(2) The compound (318 mg) obtained in the above described (1) was used to perform the synthesis process according to the method described in Reference Example 2-1-(2) thereby giving the title compound (121 mg) as a colorless amorphous substance.
MS ESI/APCI Multi posi: 450[M+H]$^+$.

The compounds of the following Reference Examples 7-2 to 7-4 were synthesized according to the method described in Reference Example 7-1, using the compound obtained in Reference Example 2-2 or Reference Example 2-3. These structures, NMR data and MS data are shown in Table 3-1.

[Table 3-1]

| Reference Example No. | Structure | Analytical Data |
|---|---|---|
| 7-2 | 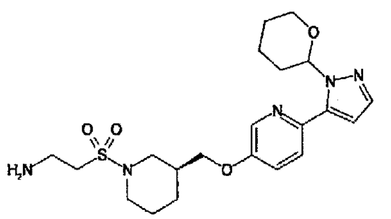 | $^1$H NMR (600 MHz, CHLOROFORM-d) δ ppm 1.34 - 1. 43 (m, 1 H) 1.60 - 1.77 (m, 4 H) 1.81 - 1.86 (m, 1 H) 1.86 - 1.92 (m, 1 H) 1.96 - 2.01 (m, 1 H) 2.03 - 2.10 (m, 1 H) 2.17 - 2.25 (m, 1 H) 2.46 - 2.56 (m, 1 H) 2.81 (dd, J=11.6, 9.9 Hz, 1 H) 2.86 - 2.92 (m, 1 H) 3.01 - 3.05 (m, 2 H) 3.20 (t, J=6.2 Hz, 2 H) 3.57 (td, J=11.5, 2.3 Hz, 1 H) 3. 63 - 3.68 (m, 1 H) 3. 83 - 3.88 (m, 1 H) 3.88 - 3.93 (m, 1 H) 3.98 (dd, J=9.1, 5.4 Hz, 1 H) 4.00 - 4.05 (m, 1 H) 6.03 - 6.07 (m, 1 H) 6.47 (d, J=1.7 Hz, 1 H) 7.20 - 7.26 (m, 1 H) 7.51 (d, J=8.7 Hz, 1 H) 7.57 (d, J=1.7 Hz, 1 H) 8.34 (d, J=2.9 Hz, 1 H).<br>MS ESI/APCI Multi posi: 450[M+H]$^+$. |
| 7-3 | 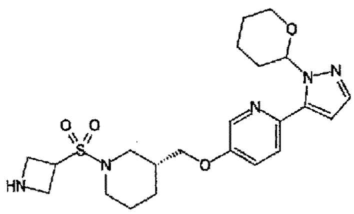 | $^1$H NMR (600 MHz. CHLOROFORM-d) δ ppm 1.31 - 1.43 (m, 1 H) 1.60 - 1.84 (m, 5 H) 1.84 - 1.92 (m. 1 H) 1.95 - 2.01 (m, 1 H) 2.03 - 2.10 (m, 1 H) 2.11 - 2.22 (m, 1 H) 2.45 - 2. 56 (m, 1 H) 2.74 - 2.93 (m, 2 H) 3.42 - 3. 68 (m, 3 H) 3.74 - 4.18 (m, 8 H) 6.05 (dd, J=10.1, 2.3 Hz, 1 H) 6.47 (d, J=1.7 Hz, 1 H) 7.19 - 7.27 (m, 1 H) 7.51 (d, J=8.7 Hz, 1 H) 7.57 (d, J=1.7 Hz, 1 H) 8.34 (d, J=2.9 Hz, 1 H).<br>MS ESI/APCI Multi posi: 462[M+H]$^+$. |

(continued)

| Reference Example No. | Structure | Analytical Data |
|---|---|---|
| 7-4 | 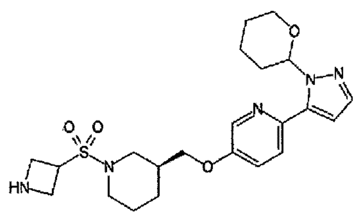 | $^1$H NMR (600 MHz, CHLOROFORM-d) δ ppm 1.37 - 1.45 (m, 1 H) 1.65 - 1.79 (m, 4 H) 1.80 - 1.87 (m, 1 H) 1.88 - 1.94 (m, 1 H) 1.98 - 2.04 (m, 1 H) 2.06 - 2.12 (m, 1 H) 2.16 - 2.24 (m, 1 H) 2.49 - 2.58 (m, 1 H) 2.83 (dd, J=12.0, 9.5 Hz, 1 H) 2.88 - 2.95 (m, 1 H) 3.60 (td, J=11. 4, 2.5 Hz, 1 H) 3. 64 - 3.71 (m, 1 H) 3.77 - 3.83 (m, 2 H) 3.85 - 3.90 (m, 1 H) 3.92 (dd, J=9.5, 7.4 Hz, 1 H) 3.99 (dd. J=9.5, 5.2 Hz, 1 H) 4.02 - 407 (m, 1 H) 4.08 - 4.13 (m, 2 H) 4. 13 - 4.20 (m, 1 H) 6.08 (dd, J=10.1, 2.3 Hz, 1 H) 6.50 (d, J=1.7 Hz, 1 H) 7.22 - 7.30 (m, 1 H) 7.54 (d, J=8.7 Hz, 1 H) 7.59 (d, J=1.7 Hz, 1 H) 8.37 (d, J=2.9 Hz, 1 H). MS ESI/APCI Multi posi: 462[M+H]$^+$. |

Reference Example 8-1

(trans-3-Aminocyclobutyl)[(3R)-3-({[6-(1H-pyrazol-5-yl)pyridin-3-yl]oxy}methyl)piperidin-1-yl]methanone

[Formula 132]

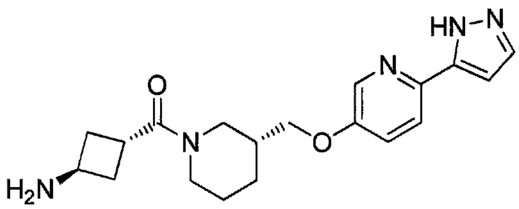

(1) trans-3-[(tert-Butoxycarbonyl)amino]cyclobutane-1-carboxylic acid (302 mg), diisopropylethyl amine (397 μL) and anhydrous propylphosphonic acid (1.6 mol/L N,N-dimethylformamide solution, 1.46 mL) were added to a solution of the compound (400 mg) obtained in Reference Example 2-2 in N,N-dimethylformamide (10 mL), and the mixture was stirred at room temperature overnight. Ethyl acetate and an aqueous solution of 10% ammonium chloride were added to the reaction solution, and the resultant solution was separated. After the organic layer was washed with an aqueous solution of 10% ammonium chloride, the organic layer was separated by a phase separator, and the solvent was distilled off under reduced pressure. The obtained residue was purified by silica gel column chromatography (chloroform only, to chloroform/methanol = 19:1) to give tert-butyl {trans-3-[(3R)-3-({[6-(1H-pyrazol-5-yl)pyridin-3-yl]oxy}methyl)piperidine-1-carbonyl]cyclobutyl}carbamate (290 mg) as a colorless oily substance.

(2) The compound (290 mg) obtained in the above described (1) was dissolved in ethyl acetate (5 mL) and ethanol (5 mL), a solution of 4 mol/L hydrogen chloride in ethyl acetate (5 mL) was added thereto, and the mixture was stirred at room temperature for 2 hours. After the solvent was distilled off under reduced pressure, an aqueous solution of 1 mol/L sodium hydroxide was added to the residue, and the mixture was extracted with chloroform. The organic layer was separated by a phase separator, and the solvent was distilled off under reduced pressure to give the title compound (227 mg) as a colorless amorphous substance.

$^1$H NMR (600 MHz, CHLOROFORM-d) δ ppm 1.38 - 1.80 (m, 4 H) 1.87 - 2.06 (m, 4 H) 2.54 - 2.70 (m, 2.5 H) 2.83 - 3.05 (m, 1.5 H) 3.20 - 3.29 (m, 1 H) 3.58 - 3.67 (m, 1.5 H) 3.76 - 3.97 (m, 2.5 H) 4.31 - 4.37 (m, 0.5 H) 4.58 - 4.63 (m, 0.5 H) 6.64 - 6.75 (m, 1 H) 7.23 - 7.26 (m, 1 H) 7.59 - 7.69 (m, 2 H) 8.26 - 8.30 (m, 1 H).

MS ESI/APCI Multi posi: 356 [M+H]$^+$.

The compound of the following Reference Example 8-2 was synthesized using a commercially available reagent according to the method described in Reference Example 8-1. The structure, NMR data and MS data are shown in Table 4-1.

[Table 4-1]

| Reference Example No. | Structure | Analytical Data |
|---|---|---|
| 8-2 | 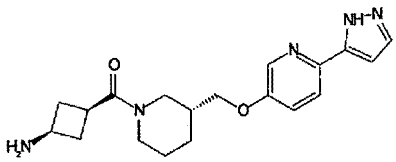 | $^1$H NMR (600 MHz, CHLOROFORM-d) δ ppm 1.38 - 2.08 (m, 8 H) 2.43 - 2.72 (m, 2.5 H) 2.78 - 2.90 (m, 1.5 H) 2.94 - 3.08 (m, 1 H) 3. 32 - 3. 41 (m, 1. H) 3. 66 - 3. 75 (m, 0. 5 H) 3.84 - 3.98 (m, 2.5 H) 4.28 - 4.35 (m, 0.5 H) 4.55 - 4.62 (m, 0.5 H) 6.65 - 6.74 (m, 1 H) 7.22 - 7.26 (m, 1 H) 7.61 - 7.70 (m, 2 H) 8.27 - 8.31 (m, 1 H).<br>MS ESI/APCI Multi posi: 356[M+H]$^+$. |

Reference Example 9-1

3-{(3R)-3-[({6-[1-(Oxan-2-yl)-1H-pyrazol-5-yl]pyridin-3-yl}oxy)methyl]piperidin-1-yl}-3-oxopropanoic acid

[Formula 133]

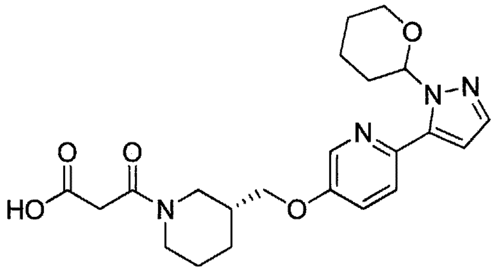

(1) Triethylamine (163 μL) and ethylmalonyl chloride (74.8 μL) were added to a solution of the compound (200 mg) obtained in Reference Example 2-2 in chloroform (4 mL), and the mixture was stirred at room temperature for 1 hour. Water was added to the reaction solution, and the mixture was extracted twice with chloroform. The organic layer was dried over magnesium sulfate, the drying agent was filtered off, and then the solvent was distilled off under reduced pressure. The obtained residue was purified by silica gel column chromatography (chloroform only, to chloroform/methanol = 19:1) to give ethyl 3-[(3R)-3-({6-[2-(oxan-2-yl)pyrazol-3-yl]pyridin-3-yl}oxymethyl)piperidin-1-yl]-3-oxopropanate (163 mg).
(2) An aqueous solution (2 mL) of 1 mol/L sodium hydroxide was added to a solution of the compound (163 mg) obtained in the above described (1) in tetrahydrofuran (2 mL) and methanol (2 mL), and the mixture was stirred at room temperature overnight. After the end of the reaction, pH was adjusted to 7 with 2 mol/L hydrochloric acid; the resultant solution was diluted with water; and then the diluted solution was extracted twice with chloroform. The organic layer was dried over magnesium sulfate, the drying agent was filtered off, and then the filtrate was concentrated under reduced pressure to give a mixture (104 mg) containing the title compound.
$^1$H NMR (600 MHz, CHLOROFORM-d) δ ppm 1.48 - 1.80 (m, 5 H) 1.89 - 2.20 (m, 4 H) 2.48 - 2.92 (m, 2 H) 2.92 - 3.23 (m, 2 H) 3.55 - 3.65 (m, 1 H) 3.71 - 3.81 (m, 2 H) 3.86 - 4.08 (m, 4 H) 4.32 - 4.71 (m, 1 H) 6.04 - 6.15 (m, 1 H) 6.47 - 6.54 (m, 1 H) 7.24 - 7.31 (m, 1 H) 7.53 - 7.58 (m, 1 H) 7.58 - 7.62 (m, 1 H) 8.38 (t, J=2.7 Hz, 1 H).
MS ESI/APCI Multi posi: 429 [M+H]$^+$.

Reference Example 10-1

3-Ammo-1-{(3R)-3-[({6-[1-(oxan-2-yl)-1H-pyrazol-5-yl]pyridin-3-yl}oxy)methyl]piperidin-1-yl}propan-1-one

[Formula 134]

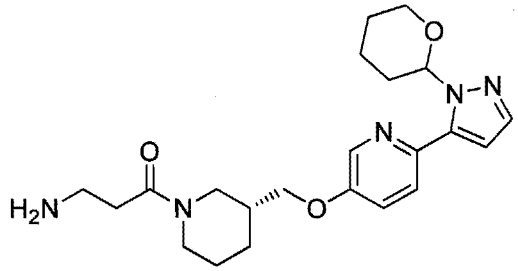

(1) N-(tert-butoxycarbonyl)-β-alanine (833 mg), diisopropylethylamine (1.15 mL) and anhydrous propylphosphonic acid (1.6 mol/L N,N-dimethylformamide solution, 3.18 mL) were added to a solution of the compound (1.16 g) obtained in Reference Example 2-2 in N,N-dimethylformamide (10 mL), and the mixture was stirred at room temperature for 20 hours. Water was added to the reaction solution, and the mixture was extracted with ethyl acetate. The organic layer was washed with water and brine, and then was dried over anhydrous sodium sulfate. The drying agent was filtered off, and the filtrate was concentrated under reduced pressure. The obtained residue was purified by silica gel column chromatography (n-hexane/ethyl acetate = 2:3) to give tert-butyl (3-{(3R)-3-[({6-[1-(oxan-2-yl)-1H-pyrazol-5-yl]pyridin-3-yl}oxy)methyl]piperidin-1-yl}-3-oxopropyl)carbamate (1.73 g) as a colorless amorphous substance.

(2) 2,6-Lutidine (1.17 mL) and trimethylsilyl trifluoromethanesulfonate (912 μL) were added to a solution of the compound (1.73 g) obtained in the above described (1) in chloroform (15 mL), and the mixture was stirred at room temperature for 2 hours. 2,6-Lutidine (1.17 mL) and trimethylsilyl trifluoromethanesulfonate (912 μL) were added, and the mixture was further stirred for 1 hour. An aqueous solution of saturated sodium hydrogen carbonate was added to the reaction mixture, and the mixture was extracted with chloroform. After the organic layer was separated by a phase separator, the solvent was distilled off under reduced pressure. The obtained residue was purified by NH silica gel column chromatography (chloroform only, to chloroform/methanol = 4:1, and subsequently ethyl acetate only, to ethyl acetate/methanol = 9:1) to give the title compound (650 mg) as a pale yellow oily substance.

MS ESI/APCI Multi posi: 414[M+H]$^+$.

Reference Example 11-1

2-[1-(Oxan-2-yl)-1H-pyrazol-5-yl]-5-{2-[(2R)-pyrrolidin-2-yl]ethoxy}pyridine

[Formula 135]

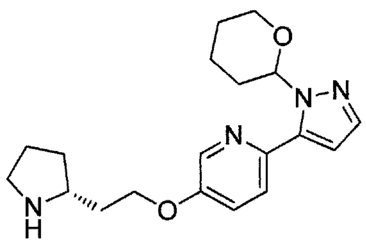

(1) A borane-tetrahydrofuran complex (0.98 mol/L tetrahydrofuran solution, 1.5 mL) was added to a solution of [(2R)-1-{[(9H-fluoren-9-yl)methoxy]carbonyl}pyrrolidin-2-yl]acetic acid (400 mg) in tetrahydrofuran (5.7 mL) under ice cooling, the ice bath was removed, and the mixture was stirred overnight. Water was added to the reaction solution under ice cooling, the mixture was extracted with chloroform, and the organic layer was separated by a phase separator. The obtained organic layer was concentrated under reduced pressure, and then the obtained residue was purified by silica gel column chromatography (n-hexane/ethyl acetate = 1:1 to 1:4) to give (9H-fluoren-9-yl)methyl (2R)-2-(2-hydroxyethyl)pyrrolidine-1-carboxylate (378 mg) as a colorless oily substance.

(2) The compound (70 mg) obtained in Reference Example 1-1 and triphenylphosphine (97 mg) were added to a

solution of the compound (125 mg) obtained in the above described (1) in tetrahydrofuran (1.4 mL), then diethyl azodicarboxylate (2.2 mol/L toluene solution, 168 μL) was added thereto under ice cooling. After the ice bath was removed, the mixture was stirred overnight, and the reaction solution was concentrated under reduced pressure. The obtained residue was purified by silica gel column chromatography (chloroform/methanol = 99:1 to 95:5) to give (9H-fluoren-9-yl)methyl (2R)-2-[2-({6-[1-(oxan-2-yl)-1H-pyrazol-5-yl]pyridin-3-yl}oxy)ethyl]pyrrolidine-1-carboxylate (435 mg) as a colorless oily substance.

(3) Piperidine (370 μL) was added to a solution of the compound (435 mg) obtained in the above described (2) in chloroform (3.7 mL), and the mixture was stirred at room temperature for 5 hours, and further stirred under heated reflux for 5 hours. An aqueous solution of saturated sodium hydrogen carbonate was added to the reaction solution, the mixture was extracted with chloroform, and the obtained organic layer was subjected to washing with brine; and drying over anhydrous magnesium sulfate, followed by filtration. The filtrate was concentrated under reduced pressure, and the obtained residue was purified by NH silica gel column chromatography (ethyl acetate). The obtained oily substance was dissolved in ethyl acetate, and the solution was subjected to washing sequentially with water and brine, and drying over anhydrous magnesium sulfate, followed by filtration. The filtrate was concentrated under reduced pressure to give the title compound (42 mg) as a colorless oily substance.

$^1$H NMR (600 MHz, CHLOROFORM-d) δ ppm 1.23 - 2.13 (m, 12 H) 2.49 - 2.59 (m, 1 H) 2.88 - 2.95 (m, 1 H) 3.01 - 3.07 (m, 1 H) 3.23 - 3.30 (m, 1 H) 3.56 - 3.63 (m, 1 H) 4.02 - 4.08 (m, 1 H) 4.13 - 4.24 (m, 2 H) 6.06 - 6.11 (m, 1 H) 6.48 - 6.51 (m, 1 H) 7.24 - 7.30 (m, 3 H) 7.51 - 7.55 (m, 1 H) 7.58 - 7.61 (m, 1 H) 8.36 - 8.40 (m, 1 H).

MS ESI/APCI Multi posi: 343 [M+H]$^+$.

Reference Example 12-1

1-{3-[({6-[1-(Oxan-2-yl)-1H-pyrazol-5-yl]pyridin-3-yl}oxy)methyl]bicyclo[1.1.1]pentan-1-yl}methanamine

[Formula 136]

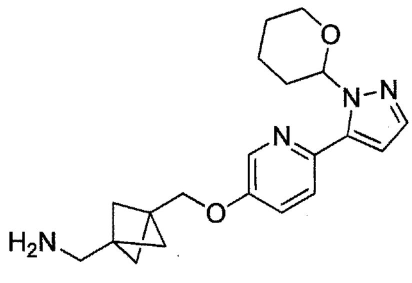

(1) The compound (421 mg) obtained in Reference Example 1-1 and methyl 1-(hydroxymethyl)bicyclo[1.1.1]pentane-3-carboxylate (295 mg) was used to perform the synthesis process according to the method described in Reference Example 2-1-(1) thereby giving methyl 3-[({6-[1-oxan-2-yl)-1H-pyrazol-5-yl]pyridin-3-yl}oxy)methyl]bicyclo[1.1.1]pentane-1-carboxylate (684 mg) as a colorless oily substance.

(2) To a solution of the compound (684 mg) obtained in the above described (1) in methanol (10 mL), lithium borohydride (148 mg) was added, and the mixture was stirred at room temperature for 17 hours. Lithium borohydride (74.0 mg) was further added thereto, and the mixture was stirred at room temperature for 2 hours. An aqueous solution of saturated ammonium chloride was added to the reaction solution, and the solvent was distilled off. The residue was extracted with chloroform, and the organic layer was separated by a phase separator and then was concentrated under reduced pressure to give {3-[(6-[1-(oxan-2-yl)-1H-pyrazol-5-yl]pyridin-3-yl}oxy)methyl]bicyclo[1.1.1]pentan-1-yl}methanol (495 mg) as a colorless oily substance. The obtained compound was used for the next reaction without being purified.

(3) Triethylamine (201 μL) and methanesulfonyl chloride (94.8 μL) were added to a solution of the compound (395 mg) obtained in the above described (2) in ethyl acetate (2 mL), and the mixture was stirred at room temperature for 1 hour. After impurities were filtered off, the filtrate was concentrated to give {3-[({6-[1-(oxan-2-yl)-1H-pyrazol-5-yl]pyridin-3-yl}oxy)methyl]bicyclo[1.1.1]pentan-1-yl}methyl methanesulfonate (481 mg) as a colorless oily substance. The obtained compound was used for the next reaction without being purified.

(4) Sodium azide (216 mg) was added to a solution of the compound (481 mg) obtained in the above described (3) in N,N-dimethylformamide (5 mL), and the mixture was stirred at 80°C for 1 hour. Water was added to the reaction

mixture, and the resultant mixture was extracted with ethyl acetate. The organic layer was washed with water and brine, and then was dried over anhydrous magnesium sulfate. The drying agent was filtered off, and the filtrate was concentrated under reduced pressure to give 5-{[3-(azidomethyl)bicyclo[1.1.1]pentan-1-yl]methoxy}-2-[1-(oxan-2-yl)1H-pyrazol-5-yl]pyridine (422 mg) as a brown oily substance. The obtained compound was used for the next reaction without being purified.

(5) To a solution of the compound (422 mg) obtained in the above described (4) in methanol (6 mL), 10% palladium-carbon (42.2 mg) was added, and the mixture was stirred under a hydrogen atmosphere at room temperature for 2 hours. The reaction mixture was filtered through Celite (registered trademark), and then the filtrate was concentrated under reduced pressure. The obtained residue was purified by NH silica gel column chromatography (chloroform only, to chloroform/methanol = 9:1) to give the title compound (358 mg) as a brown oily substance.

$^1$H NMR (400 MHz, CHLOROFORM-d) δ ppm 1.49 - 1.58 (m, 1 H) 1.62 - 1.80 (m, 7 H) 1.87 - 2.25 (m, 3 H) 2.47 - 2.60 (m, 1 H) 2.72 - 2.79 (m, 2 H) 3.54 - 3.63 (m, 1 H) 4.02 - 4.09 (m, 3 H) 6.05 - 6.10 (m, 1 H) 6.49 (d, J=1.8 Hz, 1 H) 7.24 (dd, J=8.7, 3.0 Hz, 1 H) 7.52 (d, J=8.7 Hz, 1 H) 7.59 (d, J=1.8 Hz, 1 H) 8.37 (d, J=3.0 Hz, 1 H).

MS ESI/APCI Multi posi: 355 $[M+H]^+$.

Reference Example 13-1

2-[1-(Oxan-2-yl)-1H-pyrazol-5-yl]-5-{[(3R)-pyrrolidin-3-yl]methoxy}pyridine

[Formula 137]

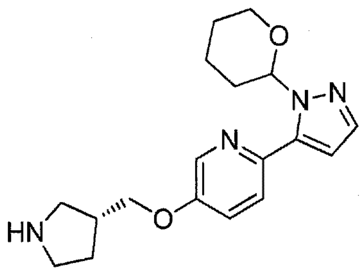

The compound (501 mg) obtained in Reference Example 1-1 and (R)-3-(hydroxymethyl)pyrrolidine (248 mg) were used to perform the synthesis process according to the method described in Reference Example 2-1-(1) thereby giving the title compound (584 mg) as a yellow amorphous substance.

$^1$H NMR (600 MHz, CHLOROFORM-d) δ ppm 1.55 - 1.58 (m, 1 H) 1.69 - 1.78 (m, 4 H) 1.98 - 2.12 (m, 3 H) 2.49 - 2.58 (m, 1 H) 2.58 - 2.65 (m, 1 H) 2.80 - 2.88 (m, 1 H) 2.93 - 2.97 (m, 1 H) 3.00 - 3.08 (m, 1 H) 3.13 - 3.18 (m, 1 H) 3.54 - 3.64 (m, 1 H) 3.92 - 4.07 (m, 3 H) 6.06 - 6.10 (m, 1 H) 6.49 (d, J=1.7 Hz, 1 H) 7.24 - 7.26 (m, 1 H) 7.52 - 7.55 (m, 1 H) 7.59 (d, J=1.7 Hz, 1 H) 8.36 - 8.39 (m, 1 H).

MS ESI/APCI Multi posi: 329 $[M+H]^+$.

The compound of the following Reference Example 13-2 was synthesized using the compound obtained in Reference Example 1-1, according to the method described in Reference Example 13-1. The structure, NMR data and MS data are shown in Table 5-1.

[Table 5-1]

| Reference Example No. | Structure | Analytical Data |
|---|---|---|
| 13-2 | 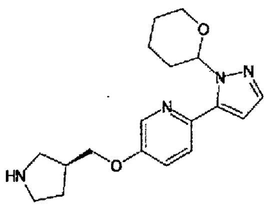 | $^1$H MMR (600 MHz, CHLOROFORM-d) δ ppm 1.55 - 1.58 (m, 1 H) 1.69 - 1.78 (m, 4 H) 1.98 - 2.12 (m, 3 H) 2.49 - 2.58 (m, 1 H) 2.58 - 2.65 (m, 1 H) 2.80 - 2.88 (m, 1 H) 2.93 - 2.97 (m, 1 H) 3.00 - 3.08 (m, 1 H) 3.13 - 3.18 (m, 1 H) 3.54 - 3.64 (m, 1 H) 3.92 - 4.07 (m. 3 H) 6.06 - 6.10 (m, 1 H) 6.49 (d, J=1.7 Hz, 1 H) 7.24 - 7.26 (m, 1 H) 7.52 - 7.55 (m, 1 H) 7.69 (d, J=1.7 Hz, 1 H) 8.36 - 8.39 (m, 1 H). MS ESI/APCI Multi posi: 329$[M+H]^+$. |

Reference Example 14-1

5-{[(3R)-Piperidin-3-yl]methoxy}-2-(1H-pyrazol-5-yl)pyridine

[Formula 138]

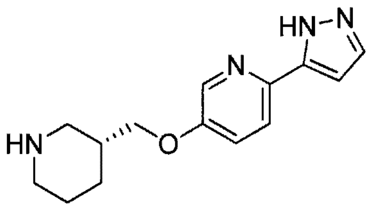

Water (4 mL) and trifluoroacetic acid (1 mL) were added to a solution of the compound (2.00 g) obtained in Reference Example 2-2 in methanol (20 mL), and the mixture was stirred at 60°C for 3 hours. After the reaction solution was concentrated, the residue was neutralized with saturated sodium hydrogen carbonate and was concentrated again. The obtained residue was purified by NH silica gel column chromatography (n-hexane/ethyl acetate = 1:1, to ethyl acetate only, and subsequently chloroform/methanol = 9:1) to give the title compound (625 mg) as a colorless powder.
$^1$H NMR (600 MHz, DMSO-d6) δ ppm 1.15 - 1.25 (m, 1 H) 1.30 - 1.42 (m, 1 H) 1.53 - 1.61 (m, 1 H) 1.76 - 1.89 (m, 2 H) 2.07 (br s, 1 H) 2.33 (dd, J=11.8, 9.7 Hz, 1 H) 2.41 - 2.47 (m, 1 H) 2.82 (dt, J=11.9, 3.6 Hz, 1 H) 3.02 (dd, J=11.8, 2.7 Hz, 1 H) 3.91 (d, J=6.6 Hz, 2 H) 6.72 (d, J=2.1 Hz, 1 H) 7.37 - 7.47 (m, 1 H) 7.60 - 7.76 (m, 1 H) 7.78 - 7.88 (m, 1 H) 8.26 (d, J=2.9 Hz, 1 H) 12.94 (br s, 1 H).
MS ESI/APCI Multi posi: 259 $[M+H]^+$.

Reference Example 14-2

5-[2-(Piperidin-4-yl)ethoxy]-2-(1H-pyrazol-5-yl)pyridine

[Formula 139]

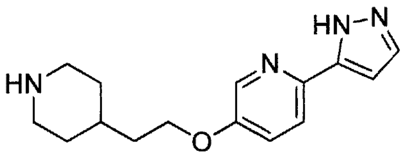

(1) The compound (6.13 g) obtained in Reference Example 1-1 and 2-(1-benzylpiperidin-4-yl)ethanol (3.60 g) were used to perform the synthesis process according to the method described in Reference Example 4-1 thereby giving a mixture (4.44 g) containing 5-[2-(1-benzylpiperidin-4-yl)ethoxy]-2-[2-(oxan-2-yl)pyrazol-3-yl)pyridine as a light brown oily substance.

(2) 1-Chloroethyl chloroformate (1.64 mL) and proton sponge (registered trademark) (1.49 g) were added to a solution of the mixture (4.44 g) obtained in the above described (1) in 1,2-dichloroethane (50 mL). The reaction solution was heated to reflux for 1 hour, and then the resultant solution was concentrated under reduced pressure. The obtained residue was dissolved in methanol (50 mL), and the solution was stirred at 50°C for 1 hour. After the reaction solution was concentrated, a solution of 4 mol/L hydrogen chloride in ethyl acetate was added to the concentrated solution, and the produced precipitate was collected by filtration and was washed with ethyl acetate. This solid was purified by NH silica gel column chromatography (n-hexane/ethyl acetate = 1:1, to ethyl acetate only, and subsequently chloroform/methanol = 19:1 to 9:1) to give the title compound (310 mg) as a light brown powder.
$^1$H NMR (300 MHz, DMSO-d6) δ ppm 0.97 - 1.17 (m, 2 H) 1.46 - 1.73 (m, 4 H) 2.37 - 2.48 (m, 3 H) 2.83 - 2.96 (m, 2 H) 4.11 (t, J=6.5 Hz, 2 H) 6.72 (d, J=2.0 Hz, 1 H) 7.44 (dd, J=8.7, 2.8 Hz, 1 H) 7.60 - 7.76 (m, 1 H) 7.84 (d, J=8.7 Hz, 1 H) 8.27 (d, J=2.8 Hz, 1 H) 8.32 (s, 1 H).
MS ESI/APCI Multi posi: 273 $[M+H]^+$.

Reference Example 14-3

2-(4-Methyl-1H-pyrazol-5-yl)-5-[2-(piperidin-4-yl)ethoxy]pyridine

[Formula 140]

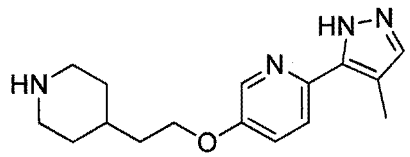

(1) 6-Bromopyridin-3-ol (1.00 g) and the compound (1.97 g) obtained in Reference Example 19-5 described later were used to perform the synthesis process according to the method described in Reference Example 4-1 thereby giving benzyl 4-[2-(6-bromopyridin-3-yl)oxyethyl]piperidine-1-carboxylate (1.72 g) as a light brown oily substance.
(2) 4-Methyl-5-(4,4,5,5-tetramethyl-1,3,2-dioxaborolan-2-yl)-1H-pyrazole (150 mg) and an aqueous solution (0.72 mL) of 2 mol/L potassium carbonate were added to a solution of the compound (200 mg) obtained in the above described (1) in 1,4-dioxane (10 mL), and the interior of the reaction container was purged with nitrogen. A 1,1'-Bis(diphenylphosphino)ferrocene palladium(II)dichloride dichloromethane adduct (118 mg) was added to the above described mixture, and the resultant mixture was stirred at 100°C for 4 hours. After the end of the reaction, water was added to the mixture, and the resultant mixture was extracted with ethyl acetate. After the organic layer was separated, the organic layer was dried over anhydrous sodium sulfate, the drying agent was filtered off, and then the solvent was distilled off under reduced pressure. The obtained residue was purified twice by NH silica gel column chromatography (n-hexane/ethyl acetate = 1:1, to ethyl acetate only, and subsequently chloroform/methanol = 19:1) to give benzyl 4-{2-[6-(4-methyl-1H-pyrazol-5-yl)pyridin-3-yl]oxyethyl}piperidine-1-carboxylate (20 mg) as a light brown oily substance.
(3) The compound (20 mg) obtained in the above described (2) was used to perform the reaction according to the method described in Reference Example 2-1-(2) thereby giving the title compound (16 mg) as a light brown amorphous substance.
MS ESI/APCI Multi posi: 287[M+H]$^+$.

Reference Example 15-1

3-Amino-1-[(3R)-3-({[6-(1H-pyrazol-5-yl)pyridin-3-yl]oxy}methyl)piperidin-1-yl] propan-1-one

[Formula 141]

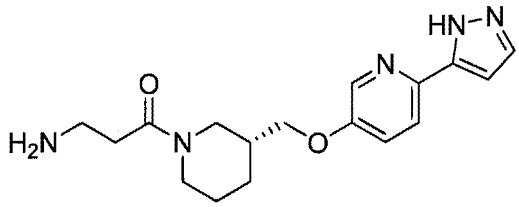

(1) The compound (327 mg) obtained in Reference Example 14-1 and 3-[(2-methylpropan-2-yl)oxycarbonylamino]propanoic acid (288 mg) were used to perform the synthesis process according to the method described in Reference Example 8-1-(1) to give tert-butyl N-{3-oxo-3-[(3R)-3-{[6-(1H-pyrazol-5-yl)pyridm-3-yl]oxymethyl}piperidin-1-yl]propyl} carbamate (542 mg) as a colorless amorphous substance.
(2) The compound (542 mg) obtained in the above described (1) was used to perform the synthesis process according to the method described in Reference Example 8-1-(2) thereby giving the title compound (309 mg) as a colorless solid.

$^1$H NMR (600 MHz, DMSO-d6) δ ppm 1.29 - 1.50 (m, 2 H) 1.61 - 1.76 (m, 1 H) 1.80 - 2.02 (m, 2 H) 2.34 - 2.43 (m, 2 H) 2.54 - 2.60 (m, 0.5 H) 2.71 (q, J=6.6 Hz, 2 H) 2.78 - 2.85 (m, 0.5 H) 2.97 - 3.10 (m, 1 H) 3.74 - 3.90 (m, 1 H) 3.90 - 4.05 (m, 2 H) 4.05 - 4.47 (m, 1 H) 6.70 - 6.75 (m, 1 H) 7.41 - 7.51 (m, 1 H) 7.61 - 7.77 (m, 1 H) 7.79 - 7.92 (m, 1 H) 8.25 - 8.33 (m, 1 H) 12.98 (br s, 1 H).
MS ESI/APCI Multi posi: 330 [M+H]+.

Reference Example 16-1

1-(4-{[(6-Chloro-5-fluoropyridin-3-yl)oxy]methyllpiperidin-1-yl)ethan-1-one

[Formula 142]

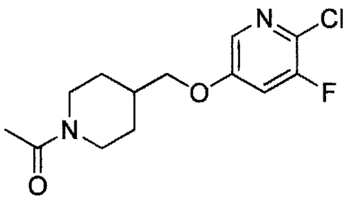

Toluene (10 mL), the compound (320 mg) obtained in Reference Example 25-1 described later, and cyanomethylene tributylphosphorane (1.33 mL) were added to 6-chloro-5-fluoropyridin-3-ol (250 mg), and the mixture was heated to 80°C and was stirred for 2 hours. The solvent was distilled off under reduced pressure, and the obtained residue was purified by NH silica gel column chromatography (n-hexane/ethyl acetate = 7:3 to 1:1). Diethyl ether was added to the obtained crude product, and the precipitated powder was collected by filtration to give the title compound (409 mg) as a colorless powder.
$^1$H NMR (600 MHz, CHLOROFORM-d) δ ppm 1.26 - 1.36 (m, 2 H) 1.79 - 1.95 (m, 2 H) 2.03 - 2.13 (m, 4 H) 2.56 - 2.62 (m, 1 H) 3.05 - 3.15 (m, 1 H) 3.82 - 3.92 (m, 3 H) 4.67 - 4.74 (m, 1 H) 7.05 (dd, J=9.1, 2.5 Hz, 1 H) 7.92 (d, J=2.5 Hz, 1 H).
MS ESI/APCI Multi posi: 287 [M+H]+.

The compounds of the following Reference Examples 16-2 to 16-3 were synthesized using a commercially available reagent, according to the method described in Reference Example 16-1. These structures, NMR data and MS data are shown in Table 6-1.

[Table 6-1]

| Reference Example No. | Structure | Analytical Data |
|---|---|---|
| 16-2 | 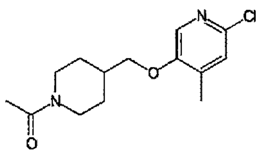 | $^1$H NMR (600 MHz, CHLOROFORM-d) δ ppm 1.29 - 1.39 (m, 2 H) 1.83 - 1.94 (m, 2 H) 2.07 - 2.12 (m, 4 H) 2.21 (s, 3 H) 2.56 - 2.64 (m, 1 H) 3.08 - 3.15 (m, 1 H) 3.85 - 3.93 (m, 3 H) 4.67 - 4.75 (m, 1 H) 7.10 (s, 1 H) 7.85 (s, 1 H).<br>MS ESI/APCI Multi posi: 283[M+H]+. |
| 16-3 | 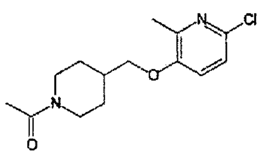 | $^1$H NMR (600 MHz, CHLOROFORM-d) δ ppm 1.25 - 1.37 (m, 2 H) 1.81 - 1.90 (m, 1 H) 1.90 - 1.97 (m, 1 H) 2.05 - 2.14 (m, 4 H) 2.43 (s, 3 H) 2.56 - 2.65 (m. 1 H) 3.06 - 3.17 (m, 1 H) 3.74 - 3.80 (m, I H) 3.80 - 3.85 (m, 1 H) 3.86 - 3.93 (m, 1 H) 4.66 - 4.77 (m, 1 H) 7.03 (d, J=8.7 Hz, 1 H) 7.10 (d, J=8.7 Hz, 1 H).<br>MS ESI/APCI Multi posi: 283[M+H]+. |

Reference Example 17-1

(3S)-3-{[(6-Chloro-5-fluoropyridin-3-yl)oxy]methyl}-N,N-dimethylpiperidine-1-sulfonamide

[Formula 143]

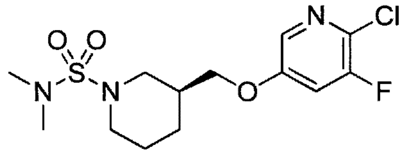

(1) 6-Chloro-5-fluoropyridin-3-ol (990 mg) was used to perform the synthesis process according to the method described in Reference Example 2-1-(1) thereby giving tert-butyl (3S)-3-{[(6-chloro-5-fluoropyridin-3-yl)oxy]methyl}piperidine-1-carboxylate (2.04 g) as a colorless oily substance.
(2) A solution of 2 mol/L hydrogen chloride in methanol (8.9 mL) was added to a solution of the compound (2.04 g) obtained in the above described (1) in methanol (10 mL) and chloroform (10 mL), and the mixture was stirred at room temperature for 1 hour. A solution of 2 mol/L hydrogen chloride in methanol (8.9 mL) was further added thereto, and the mixture was stirred at room temperature for 18 hours. After the reaction mixture was concentrated, ethyl acetate was added thereto. The produced solid was collected by filtration and was dried under reduced pressure to give 2-chloro-3-fluoro-5-{[(3S)-piperidin-3-yl]methoxy}pyridine hydrochloride (1.31 g) as a colorless powder. The obtained compound was used for the next reaction without being purified.
(3) Triethylamine (525 $\mu$L) and dimethylsulfamoyl chloride (150 $\mu$L) were added to a solution of the compound (299 mg) obtained in the above described (2) in chloroform (10 mL), and the mixture was stirred at room temperature for 18 hours. Water was added to the reaction mixture, the organic layer was separated by a phase separator, and then the solvent was distilled off under reduced pressure. The obtained residue was purified by silica gel column chromatography (n-hexane only, to n-hexane/ethyl acetate = 7:3) to give the title compound (363 mg) as a colorless powder.
$^1$H NMR (400 MHz, CHLOROFORM-d) $\delta$ ppm 1.29 - 1.40 (m, 1 H) 1.59 - 1.72 (m, 1 H) 1.76 - 1.91 (m, 2 H) 2.13 - 2.23 (m, 1 H) 2.77 - 2.86 (m, 7 H) 2.87 - 2.97 (m, 1 H) 3.54 - 3.61 (m, 1 H) 3.71 - 3.78 (m, 1 H) 3.85 - 3.95 (m, 2 H) 7.06 (dd, J=9.2, 2.3 Hz, 1 H) 7.92 (d, J=2.3 Hz, 1 H).

Reference Example 18-1

Methyl 4-{2-[(6-Chloro-5-fluoropyridin-3-yl)oxy]ethyl}piperidine-1-carboxylate

[Formula 144]

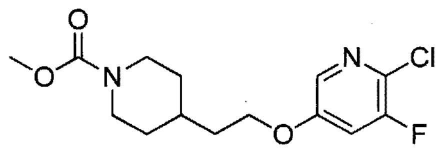

(1) 6-Chloro-5-fluoropyridin-3-ol (292 mg) and tert-butyl 4-(2-hydroxyethyl) piperidine-1-carboxylate (500 mg) were used to perform the synthesis process according to the method described in Reference Example 4-1 thereby giving tert-butyl 4-[2-(6-chloro-5-fluoropyridin-3-yl)oxyethyl]piperidine-1-carboxylate (735 mg) as a light brown oily substance.
(2) The compound (735 mg) obtained in the above described (1) was used to perform the synthesis process according to the method described in Reference Example 17-1-(2) thereby giving 2-chloro-3-fluoro-5-(2-piperidin-4-ylethoxy)pyridine hydrochloride monohydrate (560 mg) as a colorless powder.
(3) Diisopropylethylamine (523 $\mu$L) and methyl chloroformate (70 $\mu$L) were added to a solution of the compound (250 mg) obtained in the above described (2) in tetrahydrofuran (3 mL), and the mixture was stirred at room temperature for 1 hour. After the reaction solution was concentrated, the obtained residue was purified by silica gel column chromatography (n-hexane/ethyl acetate = 9:1 to 1:1) to give the title compound (279 mg) as a colorless solid.
$^1$H NMR (600 MHz, CHLOROFORM-d) $\delta$ ppm 1.14 - 1.24 (m, 2 H) 1.66 - 1.80 (m, 5 H) 2.71 - 2.83 (m, 2 H) 3.69 (s,

3 H) 4.00 - 4.31 (m, 4 H) 7.05 (dd, J=9.3, 2.5 Hz, 1 H) 7.92 (d, J=2.5 Hz, 1 H).
MS ESI/APCI Multi posi: 317 $[M+H]^+$.

Reference Example 19-1

Benzyl 4-(hydroxymethyl)piperidine-1-carboxylate

[Formula 145]

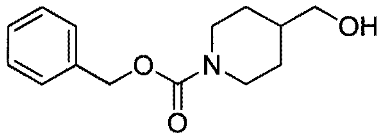

A solution of sodium hydroxide (880 mg)/water (10 mL) was added to a solution of commercially available 4-piperidine methanol (2.00 g) in tetrahydrofuran (25 mL), and benzyl chloroformate (3.14 mL) was added thereto dropwise under ice cooling, and the mixture was stirred at room temperature overnight. After the reaction solution was concentrated, water and brine were added thereto, and the mixture was extracted with chloroform. The organic layer was separated by a phase separator, and the solvent was distilled off under reduced pressure. The obtained residue was purified by silica gel column chromatography (n-hexane/ethyl acetate = 9:1 to 1:1, to chloroform/methanol = 19:1) to give the title compound (4.27 g) as a colorless oily substance.
$^1$H NMR (600 MHz, CHLOROFORM-d) δ ppm 1.12 - 1.23 (m, 2 H) 1.38 (t, J=5.4 Hz, 1 H) 1.63 - 1.77 (m, 3 H) 2.70 - 2.88 (m, 2 H) 3.47 - 3.53 (m, 2 H) 4.11 - 4.34 (m, 2 H) 5.13 (s, 2 H) 7.29 - 7.40 (m, 5 H).
MS ESI/APCI Multi posi: 250 $[M+H]^+$.
The compounds of the following Reference Examples 19-2 to 19-8 were synthesized using a commercially available corresponding amino alcohol, according to the method described in Reference Example 19-1. These structures, NMR data and MS data are shown in Table 7-1.

[Table 7-1]

| Reference Example No. | Structure | Analytical Data |
|---|---|---|
| 19-2 | 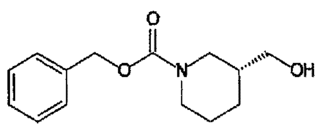 | $^1$H NMR (400 MHz, CHLOROFORM-d) δ ppm 1.20 - 1. 54 (m, 2 H) 1.61 - 1.87 (m, 3 H) 2.76 - 3.23 (m, 2 H) 3.47 - 3.55 (m, 2 H) 3.68 - 4.08 (m, 2 H) 5.08 - 5.17 (m, 2 H) 7.28 - 7.39 (m, 5 H).<br>MS ESI/APCI Multi posi: 250$[M+H]^+$. |
| 19-3 | 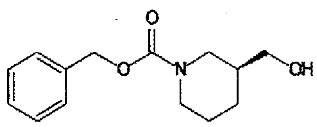 | $^1$H NMR (600 MHz, CHLOROFORM-d) δ ppm 1.20 - 1.54 (m, 2 H) 1.61 - 1.87 (m, 3 H) 2.75 - 3.23 (m, 2 H) 3.47 - 3.55 (m, 2 H) 3.68 - 4.08 (m, 2 H) 5.14 (s, 2 H) 7.28 - 7.39 (m, 5 H).<br>MS ESI/APCI Multi posi: 250$[M+H]^+$. |
| 19-4 | 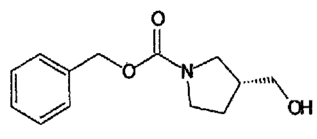 | $^1$H NMR (400 MHz, CHLOROFORM-d) δ ppm 1.41 - 1.52 (m, 1 H) 1.63 - 1.78 (m, 1 H) 1.95 - 2.06 (m, 1 H) 2.37 - 2.48 (m, 1 H) 3.16 - 3.24 (m, 1 H) 3.35 - 3.69 (m, 5 H) 5.13 (s, 2 H) 7.29 - 7.39 (m, 5 H).<br>MS ESI/APCI Multi posi: 236$[M+H]^+$. |
| 19-5 | 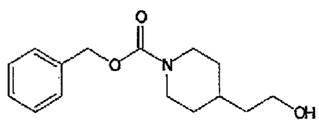 | $^1$H NMR (300 MHz, CHLOROFORM-d) δ ppm 1. 07 - 1.25 (m, 2 H) 1.48 - 1.75 (m, 6 H) 2.70 - 2.87 (m, 2 H) 3.71 (t, J=6.5 Hz, 2 H) 4.06 - 4.28 (m, 2 H) 5.12 (s, 2 H) 7. 27 - 7.38 (m, 5 H).<br>MS ESI/APCI Multi posi: 264 $[M+H]^+$. |

(continued)

| Reference Example No. | Structure | Analytical Data |
|---|---|---|
| 19-6 | 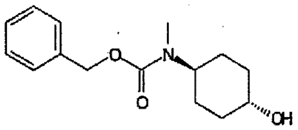 | $^1$H NMR (400 MHz, CHLOROFORM-d) δ ppm 1.40 - 1. 59 (m, 4 H) 1.68 - 1. 76 (m, 2 H) 2.00 - 2.06 (m, 2 H) 2.79 (s, 3 H) 3.52 - 3.61 (m, 1 H) 3.85 - 4. 06 (m, 1 H) 5.14 (s, 2 H) 7.26 - 7.38 (m, 5 H). MS ESI/APCI Multi posi: 264[M+H]$^+$. |
| 19-7 | 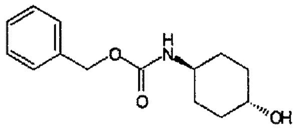 | $^1$H NMR (400 MHz, CHLOROFORM-d) δ ppm 1.14 - 1.26 (m, 2 H) 1.32 - 1.45 (m, 3 H) 1.94 - 2.08 (m, 4 H) 3.44 - 3.55 (m, 1 H) 3.55 - 3.66 (m, 1 H) 4.46 - 4.64 (m, 1 H) 5.09 (s, 2 H) 7.29 - 7.39 (m, 5 H). MS ESI/APCI Multi posi: 250[M+H]$^+$. |
| 19-8 | 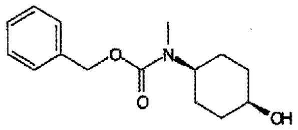 | $^1$H NMR (400 MHz, CHLOROFORM-d) δ ppm 1.16 - 1.23 (m, 1 H) 1.45 - 1.53 (m, 2 H) 1.59 - 1.70 (m, 2 H) 1.81 - 1.94 (m, 4 H) 2.84 (s, 3 H) 3.90 - 4.07 (m, 2 H) 5.14 (s, 2 H) 7.28 - 7.39 (m, 5 H). MS ESI/APCI Multi posi: 264[M+H]$^+$. |

Reference Example 20-1

Benzyl 4-(2-hydroxyethyl)-3-methylpiperidme-1-carboxylate

[0815] [Formula 146]

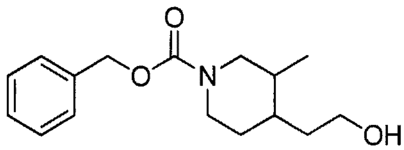

(1) Sodium hydride (2.95 g) was added to a solution of triethyl phosphonoacetate (16.5 g) in tetrahydrofuran (100 mL) under ice cooling, and the mixture was stirred at room temperature for 1 hour. 1-Benzyl-3-methylpiperidin-4-one (10.00 g) was added to the reaction solution, and the mixture was stirred at room temperature for 20 hours. The solvent was distilled off under reduced pressure, water was added to the residue, and the mixture was extracted with ethyl acetate. The organic layer was washed with brine, and was dried over anhydrous magnesium sulfate. The drying agent was filtered off, and the filtrate was concentrated under reduced pressure. The obtained residue was purified by silica gel column chromatography (n-hexane/ethyl acetate = 9:1 to 3:2). Diethyl ether was added to the obtained crude product, and the precipitated powder was collected by filtration to give ethyl (2E)-(1-benzyl-3-methylpiperidin-4-ylidene)acetate (9.38 g) as a pale yellow oily substance.
(2) To a solution of the compound (3.60 g) obtained in the above described (1) in ethanol (26 mL), 20% palladium hydroxide-carbon (360 mg) was added, and the mixture was stirred under a hydrogen atmosphere at room temperature for 20 hours. The reaction solution was passed through Celite (registered trademark), and the filtrate was concentrated under reduced pressure. The resultant solution was dried under reduced pressure to give ethyl 2-(3-methylpiperidin-4-yl)acetate (2.44 g) as a pale yellow oily substance.
(3) Triethylamine (2.75 mL) was added to a solution of the compound (2.44 g) obtained in the above described (2) in chloroform (26 mL), benzyl chloroformate (2.04 mL) was added thereto dropwise under ice cooling, and the mixture was stirred at room temperature for 2 hours. To the reaction solution, 1 mol/L hydrochloric acid was added, and the mixture was extracted with chloroform. The organic layer was dried over anhydrous magnesium sulfate, and the drying agent was filtered off. The filtrate was concentrated under reduced pressure, and the obtained residue was purified by silica gel column chromatography (n-hexane/ethyl acetate = 7:3) to give benzyl 4-(2-ethoxy-2-oxoethyl)-3-methylpiperidine-1-carboxylate (3.21 g) as a colorless oily substance.

(4) Lithium borohydride (1.31 g) was added to a solution of the compound (3.21 g) obtained in the above described (3) in tetrahydrofuran (50 mL) under ice cooling, and the mixture was stirred at room temperature for 20 hours. Water, methanol and an aqueous solution of saturated sodium potassium tartrate were added to the reaction solution, and the mixture was stirred at room temperature for 1 hour. The resultant mixture was extracted with chloroform, the organic layer was dried over anhydrous magnesium sulfate, and the drying agent was filtered off. The filtrate was concentrated under reduced pressure to give the title compound (3.12 g) as a colorless oily substance.
$^1$H NMR (300 MHz, CHLOROFORM-d) $\delta$ ppm 0.81 - 0.96 (m, 3 H) 1.11 - 1.61 (m, 6 H) 1.67 - 1.97 (m, 2 H) 2.69 - 3.04 (m, 1 H) 3.62 - 4.24 (m, 4 H) 5.07 - 5.17 (m, 2 H) 7.30 - 7.39 (m, 5 H).
MS ESI/APCI Multi posi: 278 [M+H]$^+$.

Reference Example 21-1

Benzyl (cis-4-hydroxycyclohexyl)carbamate

[Formula 147]

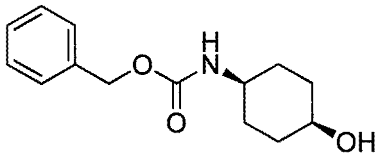

(1) Triphenylphosphine (421 mg) and di-tert-butyl azodicarboxylate (370 mg) were added to a solution of the compound (200 mg) obtained in Reference Example 19-7 and 4-nitrobenzoic acid (268 mg) in tetrahydrofuran (3 mL), and the mixture was stirred at 60°C for 2 hours. To the above mixture, 6 mol/L hydrochloric acid was added, and the resultant mixture was stirred for 2 hours and then was extracted with ethyl acetate. The organic layer was washed with an aqueous solution of saturated sodium hydrogen carbonate and brine, and then was dried over anhydrous magnesium sulfate. After the drying agent was filtered off, the filtrate was concentrated under reduced pressure, and the obtained residue was purified by silica gel column chromatography (n-hexane only, to n-hexane/ethyl acetate=1:1) to give cis-4-{[(benzyloxy)carbonyl]amino}cyclohexyl 4-nitrobenzoate (161 mg) as a colorless solid.
(2) Potassium carbonate (111 mg) was added to a solution of the compound (161 mg) obtained in the above described (1) in methanol (2 mL), and the mixture was stirred at room temperature for 3 hours. Water was added thereto, and the mixture was extracted with ethyl acetate, and then was washed with water and brine. The resultant mixture was dried over anhydrous magnesium sulfate, the drying agent was filtered off, then the filtrate was concentrated under reduced pressure, and the obtained residue was purified by silica gel column chromatography (chloroform only, to chloroform/methanol = 20:1) to give the title compound (35 mg) as a colorless solid.
$^1$H NMR (400 MHz, CHLOROFORM-d) $\delta$ ppm 1.32 (s, 1 H) 1.62 - 1.74 (m, 8 H) 3.53 - 3.68 (m, 1 H) 3.86 - 3.95 (m, 1 H) 4.60 - 4.85 (m, 1 H) 5.09 (s, 2 H) 7.26 - 7.43 (m, 5 H). MS ESI/APCI Multi posi: 250 [M+H]$^+$.

Reference Example 23-1

4-(Bromomethyl)-N,N-dimethylbenzene-1-sulfonamide

[Formula 148]

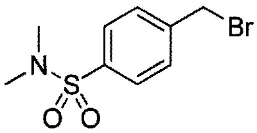

N, N-Diisopropylethylamine (1.36 mL) and dimethylamine (9.5 mol/L methanol solution, 820 $\mu$L) were added to a solution of commercially available 4-(bromomethyl)benzenesulfonyl chloride (2.00 g) in chloroform (30 mL), and the

mixture was stirred at room temperature. After the end of the reaction was confirmed by thin layer chromatography, water was added to the mixture, and the resultant mixture was extracted with ethyl acetate. The organic layer was washed with brine and was dried over anhydrous magnesium sulfate, then the drying agent was filtered off, and the filtrate was concentrated under reduced pressure. The obtained residue was purified by column chromatography (hexane/ethyl acetate = 1:0 to 7:3) to give the title compound (956 mg) as a colorless powder. $^1$H NMR (300 MHz, CHLOROFORM-d) δ ppm 2.73 (s, 6 H) 4.51 (s, 2 H) 7.53 - 7.61 (m, 2 H) 7.69 - 7.84 (m, 2 H).
MS ESI/APCI Multi posi: 278 $[M+H]^+$.

Reference Example 24-1

[3-(Methanesulfonyl)phenyl]methyl methanesulfonate

[Formula 149]

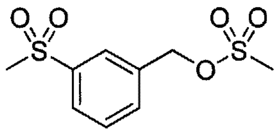

Commercially available 3-(methylsulfonyl)benzyl alcohol (128 mg) was used to perform the synthesis process according to the method described in Reference Example 12-1-(3) thereby giving the title compound (218 mg) as a yellow oily substance.
$^1$H NMR (400 MHz, CHLOROFORM-d) δ ppm 3.04 - 3.10 (m, 6 H) 5.31 (s, 2 H) 7.60 - 7.68 (m, 1 H) 7.68 - 7.75 (m, 1 H) 7.94 - 8.02 (m, 2 H).

Reference Example 25-1

1 -[4-(Hydroxymethyl)piperidin-1-yl]ethan-1 -one

[Formula 150]

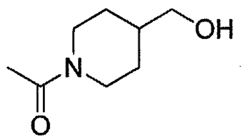

Triethylamine (6.41 mL) and acetic acid anhydride (3.49 mL) were added to a solution of commercially available 4-piperidine methanol (3.98 g) in chloroform (50 mL), and the mixture was stirred at room temperature for 2 hours. An aqueous solution of saturated sodium hydrogen carbonate was added to the reaction mixture, and the solvent was distilled off under reduced pressure. The obtained residue was purified by silica gel column chromatography (chloroform/methanol = 99:1 to 4:1) and then was purified by NH silica gel column chromatography (ethyl acetate/ methanol = 99:1 to 4:1) again to give the title compound (4.87 g) as a colorless oily substance. $^1$H NMR (600 MHz, CHLOROFORM-d) δ ppm 1.11 - 1.27 (m, 2 H) 1.71 - 1.79 (m, 2 H) 1.81 - 1.86 (m, 1 H) 2.10 (s, 3 H) 2.52 - 2.59 (m, 1 H) 2.99 - 3.10 (m, 1 H) 3.47 - 3.56 (m, 2 H) 3.81 - 3.87 (m, 1 H) 4.62 - 4.67 (m, 1 H).
MS ESI/APCI Multi posi: 158 $[M+H]^+$.

The compounds of the following Reference Examples 25-2 to 25-7 were synthesized using a commercially available corresponding alcohol, according to the method described in Reference Example 25-1. These structures, NMR data and MS data are shown in Table 8-1.

[Table 8-1]

| Reference Example No. | Structure | Analytical Data |
|---|---|---|
| 25-2 | 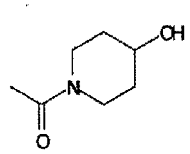 | $^1$H NMR (400 MHz, CHLOROFORM-d) δ ppm 1.40 - 1.64 (m, 3 H) 1.81 - 1.97 (m, 2 H) 2.10 (s, 3 H) 3.13 - 3.29 (m, 2 H) 3.65 - 3.78 (m, 1 H) 3. 88 - 4.00 (m, 1 H) 4.02 - 4.14 (m, 1 H).<br>MS ESI/APCI Multi posi: 144[M+H]$^+$, 166[M+Na]$^+$. |
| 25-3 | 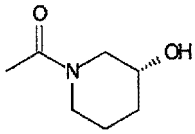 | $^1$H NMR (400 MHz, CHLOROFORM-d) δ ppm 1.40 - 2.02 (m, 5 H) 2.04 - 2.23 (m, 3 H) 3.18 - 3.94 (m, 5 H).<br>MS ESI/APCI Multi posi: 144[M+H]$^+$, 166[M+Na]$^+$. |
| 25-4 | 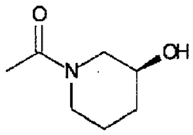 | $^1$H NMR (400 MHz, CHLOROFORM-d) δ ppm 1.41 - 2.04 (m, 5 H) 2.06 - 2.25 (m. 3 H) 3.17 - 3. 94 (m, 5 H).<br>MS ESI/APCI Multi posi: 144[M+H]$^+$, 166[M+Na]$^+$. |
| 25-5 | 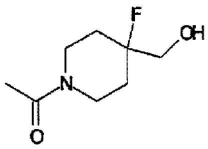 | $^1$H NMR (600 MHz, CHLOROFORM-d) δ ppm 1.47 - 1.65 (m, 1 H) 1.76 - 1.81 (m, 1 H) 1.87 - 1.94 (m, 1 H) 1.96 - 2.04 (m, 1 H) 2. 10 (s, 3 H) 2.90 - 2.98 (m, 1 H) 3.35 - 3.44 (m, 1 H) 3.51 - 3.74 (m, 3 H) 4.43 - 4.50 (m, 1 H).<br>MS ESI/APCI Multi posi: 176[M+H]$^+$, 198[M+Na]$^+$. |
| 25-6 | 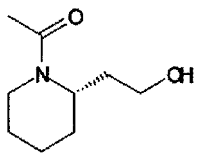 | $^1$H NMR (600 MHz, CHLOROFORM-d) δ ppm 1.42 - 1.78 (m, 8 H) 1.87 - 1.96 (m, 1 H) 2.14 (s, 3 H) 2.94 - 3.02 (m, 1 H) 3.24 - 3.31 (m, 1 H) 3.56 - 3.68 (m, 2 H) 4.81 - 4.89 (m, 1 H).<br>MS ESI/APCI Multi posi: 172[M+H]$^+$, 194[M+Na]$^+$. |
| 25-7 | 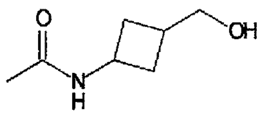 | $^1$H NMR (600 MHz, DMSO-$d_6$) δ ppm 1.43 - 2.28 (m, 8 H) 3.32 - 3.45 (m, 2 H) 3.93 - 4.26 (m, 1 H) 4.36 - 4.62 (m, 1 H) 7.82 - 8.26 (m, 1 H).<br>MS ESI/APCI Multi posi: 144[M+H]$^+$. |

Reference Example 26-1

1-[4-(Hydroxymethyl)-4-methylpiperidin-1-yl]ethan-1-one

[Formula 151]

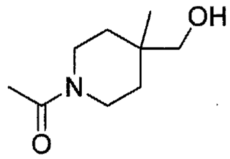

(1) Borane-tetrahydrofuran complex (0.98 mol/L tetrahydrofuran solution, 345 μL) was added to a solution of 4-methyl-1-[(2-methylpropan-2-yl)oxycarbonyl]piperidine-4-carboxylic acid (500 mg) in tetrahydrofuran (10 mL) under ice cooling, the ice bath was removed, and the mixture was stirred overnight. Water was added to the reaction solution under ice cooling, the mixture was extracted with chloroform, and the organic layer was separated by a

phase separator. The obtained organic layer was concentrated under reduced pressure to give tert-butyl 4-(hydroxymethyl)-4-methylpiperidine-1-carboxylate (522 mg) as a colorless oily substance.
(2) A solution of 4 mol/L hydrogen chloride in ethyl acetate (2 mL) was added to a solution of the compound (522 mg) obtained in the above described (1) in ethyl acetate (2 mL), and the mixture was stirred at room temperature overnight. The reaction solution was concentrated under reduced pressure to give (4-methylpiperidin-4-yl)methanol hydrochloride (322 mg) as a colorless solid.
(3) Acetic acid anhydride (322 μL) and triethylamine (631 μL) were added to a suspension of the compound (322 mg) obtained in the above described (2) in chloroform (10 mL), and the mixture was stirred at room temperature for 4 hours. Water was added to the reaction solution, the mixture was extracted with chloroform, and the organic layer was separated by a phase separator. The obtained organic layer was concentrated under reduced pressure, and the obtained residue was purified by silica gel column chromatography (chloroform/methanol = 49:1 to 9:1) to give the title compound (263 mg) as a colorless oily substance.
$^1$H NMR (600 MHz, CHLOROFORM-d) δ ppm 1.02 (s, 3 H) 1.28 - 1.39 (m, 2 H) 1.41 - 1.49 (m, 2 H) 1.50 - 1.57 (m, 1 H) 2.08 (s, 3 H) 3.11 - 3.18 (m, 1 H) 3.27 - 3.34 (m, 1 H) 3.37 - 3.43 (m, 2 H) 3.50 - 3.58 (m, 1 H) 3.99 - 4.06 (m, 1 H).
MS ESI/APCI Multi posi: 172 [M+H]$^+$, 194 [M+Na]$^+$.

The compound of the following Reference Example 26-2 was synthesized using a commercially available corresponding carboxylic acid, according to the method described in Reference Example 26-1. The structure, NMR data and MS data are shown in Table 9-1.

[Table 9-1]

| Reference Example No. | Structure | Analytical Data |
|---|---|---|
| 26-2 | 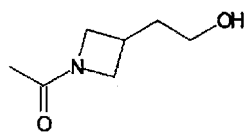 | MS ESI/APCI Multi posi: 144[M+H]$^+$. |

Reference Example 27-1

1-[(2R)-2-(2-Hydroxyethyl)piperidin-1-yl]ethanone

[Formula 152]

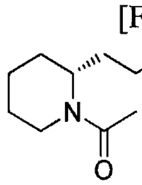

(1) tert-Butyl (2R)-2-(2-Hydroxyethyl)piperidine-1-carboxylate (500 mg) was dissolved in a solution of 2 mol/L hydrogen chloride in methanol (11 mL), and the resultant solution was stirred at room temperature overnight. The reaction solution was concentrated under reduced pressure to give a mixture containing 2-[(2R)-piperidin-2-yl]ethanol hydrochloride.
(2) Acetic acid anhydride (216 μL) and triethylamine (663 μL) were added to a suspension of the mixture obtained in the above described (1) in chloroform (11 mL), and the mixture was stirred at room temperature for 3 hours. The reaction solution was concentrated under reduced pressure, and then the obtained residue was purified by silica gel column chromatography (chloroform: methanol=97:3 to 92:8) to give the title compound (320 mg) as a colorless oily substance.
$^1$H NMR (600 MHz, CHLOROFORM-d) δ ppm 1.34 - 1.70 (m, 8 H) 1.80 - 1.88 (m, 1 H) 2.06 (s, 3 H) 2.85 - 2.95 (m, 1 H) 3.16 - 3.24 (m, 1 H) 3.48 - 3.60 (m, 2 H) 4.74 - 4.82 (m, 1H).
MS ESI/APCI Multi posi: 172 [M+H]$^+$, 194 [M+Na]$^+$.

The following Reference Example 27-2 was synthesized using a commercially available corresponding alcohol,

according to the method described in Reference Example 27-1. The structure, NMR data and MS data are shown in Table 10-1.

[Table 10-1]

| Reference Example No. | Structure | Analytical Data |
|---|---|---|
| 27-2 | 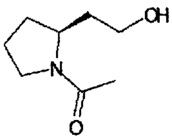 | $^1$H NMR (600 MHz, CHLOROFORM-d) δ ppm 1. 42 - 1. 51 (m, 1 H) 1.61 - 1.76 (m, 2 H) 1. 91 - 2.06 (n, 3 H) 2.08 (s, 3 H) 3.37 - 3.52 (m, 3 H) 3.54 - 3.63 (m, 1 H) 4.38 - 4.47 (m, 1 H).<br>MS ESI/APCI Multi posi: 158[M+H]$^+$, 180[M+Na]$^+$. |

Reference Example 28-1

1-[(3S)-3-(2-Hydroxyethyl)piperidin-1-yl]ethan-1-one

[Formula 153]

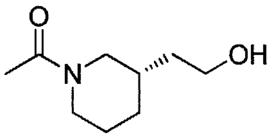

(1) A solution of tert-butyl (3R)-3-(hydroxymethyl)piperidine-1-carboxylate (2.3 g) in chloroform (43 mL) was added to a suspension of Dess-Martin periodinane (5.89 g) in chloroform (43 mL) under ice cooling, the ice bath was removed, and the mixture was stirred for 1 hour. An aqueous solution of saturated sodium thiosulfate and an aqueous solution of saturated sodium hydrogen carbonate were added thereto under ice cooling, the ice bath was removed, and the mixture was stirred for a while. The reaction solution was extracted with chloroform, and the organic layer was separated by a phase separator. The obtained organic layer was concentrated under reduced pressure to give tert-butyl (3R)-3-formylpiperidine-1-carboxylate.

(2) Potassium tert-butoxide (1.8 g) was added to a suspension of methyl triphenylphosphonium bromide (5.7 g) in tetrahydrofuran (33 mL) under ice cooling under a nitrogen atmosphere. After the ice bath was removed, the mixture was stirred for 1 hour. Then a suspension of the compound obtained in the above described (1) in tetrahydrofuran (10 mL) was added thereto under ice cooling, and after the ice bath was removed, the mixture was stirred overnight. An aqueous solution of saturated ammonium chloride was added thereto, and the mixture was extracted with a mixed solution of n-hexane/ethyl acetate, and the organic layer was separated by a phase separator. The obtained organic layer was concentrated under reduced pressure, and then the obtained residue was purified by silica gel column chromatography (n-hexane/ethyl acetate = 49:1 to 9:1) to give tert-butyl (3S)-3-ethenylpiperidine-1-carboxylate (1.16 g) as a colorless oily substance.

(3) Under ice cooling, 9-borabicyclo[3.3.1]nonane (0.5 mol/L tetrahydrofuran solution, 13.2 mL) was added to a solution of the compound (1.16 g) obtained in the above described (2) in tetrahydrofuran (22 mL), the ice bath was removed, and the mixture was stirred for 3 hours. Under ice cooling, water (15 mL) and sodium peroxoborate tetrahydrate (4.2 g) were added thereto, the ice bath was removed, and the mixture was stirred overnight. An aqueous solution of saturated ammonium chloride was added thereto, the mixture was extracted with chloroform, and the organic layer was separated by a phase separator. The obtained organic layer was concentrated under reduced pressure, and then the obtained residue was purified by silica gel column chromatography (n-hexane/ethyl acetate = 13:7 to 7:13) to give tert-butyl (3S)-3-(2-hydroxyethyl)piperidine-1-carboxylate (920 mg) as a colorless oily substance.

(4) The compound (920 mg) obtained in the above described (3) was dissolved in a solution of 2 mol/L hydrogen chloride in methanol (9.2 mL), and the resultant solution was stirred at room temperature overnight. The reaction solution was concentrated under reduced pressure to give 2-[(3S)-piperidin-3-yl]ethanol hydrochloride.

(5) Triethylamine (1.4 mL) was added to a suspension of the compound obtained in the above described (4) in

chloroform (10 mL), a solution of acetic acid anhydride (400 μL) in chloroform (10 mL) was added thereto under ice cooling. After the ice bath was removed, the mixture was stirred overnight. The reaction solution was concentrated under reduced pressure, and the obtained residue was purified by silica gel column chromatography (chloroform/methanol=49:1 to 91:9) to give the title compound (491 mg) as a colorless oily substance.
$^1$H NMR (600 MHz, CHLOROFORM-d) δ ppm 1.13 - 1.26 (m, 1 H) 1.36 - 1.76 (m, 6 H) 1.82 - 1.92 (m, 1 H) 2.07 (s, 3 H) 2.46 - 3.12 (m, 2 H) 3.60 - 3.79 (m, 3 H) 4.26 - 4.40 (m, 1 H).
MS ESI/APCI Multi posi: 172 [M+H]$^+$, 194 [M+Na]$^+$.

The compounds of the following Reference Examples 28-2 to 28-4 were synthesized using a commercially available corresponding alcohol, according to the method described in Reference Example 28-1. These structures, NMR data and MS data are shown in Table 11-1.

[Table 11-1]

| Reference Example No. | Structure | Analytical Data |
|---|---|---|
| 28-2 | 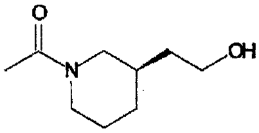 | $^1$H NMR (600 MHz, CHLOROFORM-d) δ ppm 1.12 - 1.27 (m, 1 H) 1.37 - 1.77 (m, δ H) 1.82 - 1.93 (m, 1 H) 2.08 (s, 3 H) 2.48 - 3.14 (m, 2 H) 3.60 - 3.79 (m, 3 H) 4.27 - 4.41 (m, 1 H).<br>MS ESI/APCI Multi posi: 172[M+H]$^+$, 194[M+Na]$^+$. |
| 28-3 | 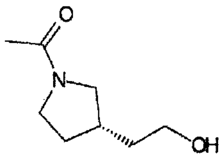 | $^1$H NMR (600 MHz, CHLOROFORM-d) δ ppm 1.39 - 1. 66 (m, 4 H) 1.92 - 2.10 (m, 4 H) 2.13 - 2.36 (m, 1 H) 2.86 - 3.00 (m, 1 H) 3.20 - 3.36 (m, 1 H) 3.40 - 3.72 (m, 4 H).<br>MS ESI/APCI Multi posi: 158[M+H]$^+$, 180[M+Na]$^+$. |
| 28-4 | 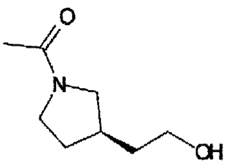 | $^1$H NMR (600 MHz, CHLOROFORM-d) δ ppm 1. 45 - 1.84 (m, 4 H) 1.95 - 2.16 (m, 4 H) 2.20 - 2.42 (m, 1 H) 2.93 - 3.07 (m, 1 H) 3.26 - 3.42 (m, 1 H) 3.47 - 3.79 (m, 4 H).<br>MS ESI/APCI Multi posi: 158[M+H]$^+$, 180[M+Na]$^+$. |

Reference Example 29-1

N-Cyclopropyl-1H-imidazole-1-carboxamide

[Formula 154]

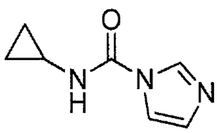

1,1'-Carbonyldiimidazole (5.11 g) was added to a solution of cyclopropanamine (1.20 g) in tetrahydrofuran (30 mL) under ice cooling, and the mixture was stirred at room temperature for 2 hours. The solvent was distilled off under reduced pressure, and the obtained residue was purified by silica gel column chromatography (chloroform only, to chloroform/methanol = 9:1) to give the title compound (3.40 g) as a colorless solid.
$^1$H NMR (400 MHz, CHLOROFORM-d) δ ppm 0.66 - 0.75 (m, 2 H) 0.87 - 0.93 (m, 2 H) 2.81 - 2.88 (m, 1 H) 6.21 (br s, 1 H) 7.08 (s, 1 H) 7.31 (s, 1 H) 8.09 (s, 1 H).

Reference Example 30-1

1-Methylcyclopropyl 4-nitrophenyl carbonate

[Formula 155]

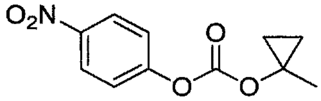

Triethylamine (271 μL), N,N-dimethylaminopyridine (1 mg) and (4-nitrophenyl) chlorocarbonate (167 mg) were added to a solution of 1-methylcyclopropan-1-ol (54 mg) in chloroform (3 mL), the mixture was stirred at room temperature overnight, water was added to the reaction solution, and the mixture was extracted with chloroform. The organic layer was separated by a phase separator, and the solvent was distilled off under reduced pressure. The obtained residue was purified by silica gel column chromatography (n-hexane/ethyl acetate = 9:1, to 4:1, and to 11: 9) to give the title compound (84.4 mg) as a colorless oily substance.
$^1$H NMR (600 MHz, CHLOROFORM-d) δ ppm 0.71 - 0.79 (m, 2 H) 1.03 - 1.14 (m, 2 H) 1.66 (s, 3 H) 7.36 - 7.41 (m, 2 H) 8.26 - 8.30 (m, 2 H).

Reference Example 31-1

2-[2-(Methanesulfonyl)phenyl]ethan-1-ol

[Formula 156]

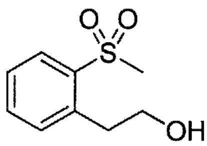

(1) In a test tube for a microwave reaction, potassium disulfite (591 mg), tetrabutylammonium bromide (471 mg), sodium formate (199 mg), palladium (II) acetate (14.9 mg), triphenylphosphine (52.3 mg), 1,10-phenanthroline (35.9 mg) and dimethylsulfoxide (4.43 mL) were mixed, and nitrogen gas was passed therethrough for 10 minutes. Commercially available methyl (2-iodophenyl)acetate (367 mg) was added to the mixture, the test tube was sealed, and then the mixture was stirred at 100°C for 30 minutes under microwave irradiation. After the mixture was cooled to room temperature, the test tube was opened, methyl iodide (82.8 μL) was added to the mixture, and the resultant mixture was stirred at room temperature for 25 hours. This mixture was poured into water, and the resultant mixture was extracted three times with chloroform. The organic layers were combined, washed with brine, and then was separated by a phase separator, and the solvent was distilled off under reduced pressure. The obtained residue was purified by silica gel column chromatography (n-hexane/ethyl acetate = 19:1 to 9:11) to give methyl [2-(methanesulfonyl)phenyl]acetate (117 mg) as a light brown oily substance.

(2) The compound (117 mg) obtained in the above described (1) was used to perform the reaction according to the method described in Reference Example 20-1-(4) thereby giving the title compound (103 mg) as a colorless oily substance.
$^1$H NMR (400 MHz, CHLOROFORM-d) δ ppm 3.13 (s, 3 H) 3.31 (t, J=6.3 Hz, 2 H) 3.98 (t, J=6.3 Hz, 2 H) 7.37 - 7.51 (m, 2 H) 7.51 - 7.70 (m, 1 H) 8.06 (dd, J=8.0, 1.2 Hz, 1 H).
MS ESI/APCI Multi posi: 201 $[M+H]^+$, 223 $[M+Na]^+$.

The compound of the following Reference Example 31-3 was synthesized using a commercially available corresponding iodobenzene analogue and a corresponding alkyl halide, according to the method described in Reference Example 31-1. The structure, NMR data and MS data are shown in Table 12-1.

[Table 12-1]

| Reference Example No. | Structure | Analytical Data |
|---|---|---|
| 31-3 | 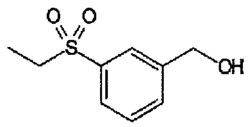 | $^1$H NMR (600 MHz, CHLOROFORM-d) δ ppm 1.28 (t, J=7.4 Hz, 3 H) 1.95 (t, J=5.8 Hz, 1 H) 3.13 (q, J=7.4 Hz, 2 H) 4.81 (d, J=5.8 Hz, 2 H) 7.54 - 7.59 (m, 1 H) 7.65 - 7.68 (m, 1 H) 7.81 - 7.85 (m, 1 H) 7.92 (s, 1 H).<br>MS ESI/APCI Multi posi: 201[M+H]$^+$, 223 [M+Na]$^+$.<br>MS ESI/APCI Multi nega: 199[M-H]$^-$, 235 [M+Cl]$^-$. |

Reference Example 32-1

N-[4-(Hydroxymethyl)pyridin-2-yl]acetamide

[Formula 157]

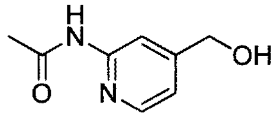

Isobutyl chlorocarbonate (144 μL) was added dropwise to a solution of commercially available 2-acetamidopyridine-4-carboxylic acid (200 mg) and N-methylmorpholine (122 μL) in tetrahydrofuran (3.70 mL) under ice cooling. After the end of the dropwise addition, the mixture was stirred for 30 minutes under ice cooling, and then the precipitate was filtered off. The filtrate was slowly added to a solution of sodium borohydride (84.0 mg) in water (1.11 mL) and tetrahydrofuran (3.70 mL) under ice cooling, the mixture was stirred for 40 minutes under ice cooling, water was added to the mixture, and then the resultant mixture was extracted with ethyl acetate three times. The combined organic layer was washed with water, then the organic layer was separated by a phase separator, and the solvent was distilled off under reduced pressure. The obtained residue was purified by silica gel column chromatography (n-hexane/ethyl acetate = 1:1, to ethyl acetate only) to give the title compound (36.0 mg) as a colorless powder.
$^1$H NMR (600 MHz, DMSO-$d_6$) δ ppm 2.08 (s, 3 H) 4.51 (d, J=5.8 Hz, 2 H) 5.40 (t, J=5.8 Hz, 1 H) 7.00 - 7.02 (m, 1 H) 8.05 (br s, 1 H) 8.20 (d, J=5.0 Hz, 1 H) 10.39 (br s, 1 H). MS ESI/APCI Multi nega: 165 [M-H]$^-$.

Reference Example 33-1

[2-(Ethanesulfonyl)pyridin-4-yl]methanol

[Formula 158]

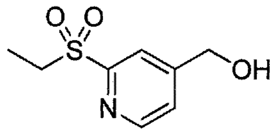

(1) Lawesson's reagent (6.34 g) was added to a solution of commercially available methyl 2-oxo-1,2-dihydropyridine-4-carboxylate (2.00 g) in toluene (26.1 mL), and the mixture was stirred under heated reflux for 40 minutes. The mixture was cooled to room temperature, then the precipitate was collected by filtration, and the solid was roughly purified by silica gel column chromatography (chloroform only, to chloroform/methanol = 19:1). The obtained roughly purified substance was suspended in ethyl acetate (1 mL), and the suspension was stirred under heated reflux for

30 minutes. The suspension was cooled to room temperature, and then the precipitate was collected by filtration to give methyl 2-sulfanylidene-1,2-dihydropyridine-4-carboxylate (227 mg) as an orange solid.
(2) Potassium carbonate (180 mg) and ethyl iodide (57.2 μL) were added to a solution of the compound (110 mg) obtained in the above described (1) in acetone (3.25 mL), and the mixture was stirred at 65°C for 2 hours. After cooling to room temperature, the mixture was diluted with ethyl acetate, and the solid matter was filtered off. The filtrate was concentrated under reduced pressure, and the obtained residue was purified by silica gel column chromatography (n-hexane/ethyl acetate = 9:1 to 1:4) to give methyl 2-(ethylsulfanyl)pyridine-4-carboxylate (114 mg) as a light gray oily substance.
(3) m-Chloroperbenzoic acid (356 mg) was carefully added to a solution of the compound (114 mg) obtained in the above described (2) in chloroform (2.89 mL), and the mixture was stirred at room temperature for 3 hours. An aqueous solution of saturated sodium thiosulfate was added to the mixture to stop the reaction, and the mixture was further diluted with an aqueous solution of saturated sodium hydrogen carbonate, and the resultant solution was stirred vigorously at room temperature for 40 minutes. After the organic layer was separated, the aqueous layer was extracted with chloroform twice. The organic layers were combined, washed with brine, and then was separated by a phase separator, and the solvent was distilled off under reduced pressure. The obtained residue was purified by NH silica gel column chromatography (n-hexane only, to n-hexane/ethyl acetate = 1:1) to give methyl 2-(ethanesulfonyl)pyridine-4-carboxylate (126 mg) as a colorless oily substance.
(4) The compound (126 mg) obtained in the above described (3) was used to perform the reaction according to the method described in Reference Example 20-1-(4) thereby giving the title compound (97.0 mg) as a colorless oily substance.
$^1$H NMR (400 MHz, CHLOROFORM-d) δ ppm 1.30 (t, J=7.5 Hz, 3 H) 3.43 (q, J=7.5 Hz, 2 H) 4.86 (s, 2 H) 7.55 - 7.59 (m, 1 H) 8.06 - 8.09 (m, 1 H) 8.70 (d, J=4.9 Hz, 1 H). MS ESI/APCI Multi posi: 202 [M+H]$^+$.

The compound of the following Reference Example 33-2 was synthesized using the compound obtained in Reference Example 33-1-(1) and bromomethylcyclopropane, according to the method described in Reference Example 33-1-(2) to (4). The structure, NMR data and MS data are shown in Table 13-1.

[Table 13-1]

| Reference Example No. | Structure | Analytical Data |
|---|---|---|
| 33-2 | 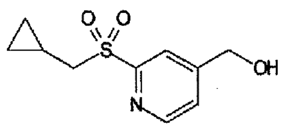 | $^1$H NMR (400 MHz, CHLOROFORM-d) δ ppm 0.12 - 0.24 (m, 2 H) 0.49 - 0.59 (m, 2 H) 0.99 - 1.10 (m, 1 H) 3.33 (d, J=7.2 Hz, 2 H) 4.87 (s, 2 H) 7.55 - 7.59 (m, 1 H) 8. 10 - 8.12 (m, 1 H) 8.70 (d, J=4.9 Hz, 1 H).<br>MS ESI/APCI Multi posi: 228[M+H]$^+$. |

Reference Example 34-1

[3-(Cyclopropanesulfonyl)phenyl]methanol

[Formula 159]

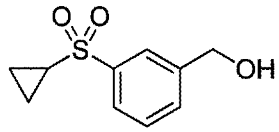

(1) Diisopropylethylamine (38.4 mL) was added to a solution of commercially available (3-bromophenyl)methanol (10.2 g) in chloroform (54.0 mL) under ice cooling, and then chloromethyl methyl ether (8.37 mL) was slowly added thereto. The mixture was slowly heated to room temperature and was stirred at room temperature for 17 hours, then chloromethyl methyl ether (4.00 mL) was further added thereto, and the resultant mixture was stirred at room temperature for 30 minutes. An aqueous solution of saturated sodium hydrogen carbonate was added to the above

mixture under ice cooling to stop the reaction, and the organic layer was distilled off under reduced pressure. The remaining aqueous layer was extracted with ethyl acetate; the organic layer was subjected to washing with 1 mol/L hydrochloric acid twice, with an aqueous solution of saturated sodium hydrogen carbonate and subsequently with brine, and drying over magnesium sulfate, followed by filtration; and then the solvent was distilled off under reduced pressure. The obtained residue was purified by silica gel column chromatography (n-hexane only, to n-hexane/ethyl acetate = 4:1) to give 1-bromo-3-[(methoxymethoxy)methyl] benzene (12.0 g) as a colorless oily substance.

(2) The compound (1.38 g) obtained in the above described (1) and 1-chloro-3-iodopropane (641 μL) were used to perform the reaction according to the method described in Reference Example 31-1-(1) thereby giving 1-(3-chloropropane-1-sulfonyl)-3-[(methoxymethoxy)methyl]benzene (923 mg) as a pale yellow oily substance.

(3) Potassium tert-butoxide (46.8 mg) was added to a solution of the compound (122 mg) obtained in the above described (2) in tetrahydrofuran (4.17 mL), and the mixture was stirred at 60°C for 2.5 hours under a nitrogen atmosphere. After cooling to room temperature, water was added to the mixture, and the resultant mixture was extracted with ethyl acetate three times. The combined organic layer was washed with water, and was separated by a phase separator, and then the solvent was distilled off under reduced pressure to give 1-(cyclopropanesulfonyl)-3-[(methoxymethoxy)methyl]benzene (104 mg) as a pale yellow oily substance. The obtained compound was used for the next reaction without being purified.

(4) Water (30.0 μL) and trifluoroacetic acid (2.00 mL) were added to a solution of the compound (104 mg) obtained in the above described (3) in chloroform (2.00 mL), and the mixture was stirred at room temperature for 15.5 hours. After the solvent was distilled off under reduced pressure, the obtained residue was purified by NH silica gel column chromatography (n-hexane/ethyl acetate = 7:3, to ethyl acetate only) to give the title compound (73.0 mg) as a colorless gummy substance.

$^1$H NMR (600 MHz, CHLOROFORM-d) δ ppm 1.02 - 1.06 (m, 2 H) 1.35 - 1.38 (m, 2 H) 2.45 - 2.50 (m, 1 H) 4.81 (s, 2 H) 7.52 - 7.59 (m, 1 H) 7.63 - 7.67 (m, 1 H) 7.79 - 7.85 (m, 1 H) 7.92 (s, 1 H).

MS ESI/APCI Multi posi: 213 $[M+H]^+$, 235 $[M+Na]^+$.

MS ESI/APCI Multi nega: 211 $[M-H]^-$.

Reference Example 35-1

{3-[3-(Pyrrolidin-1-yl)propane-1-sulfonyl]phenyl}methanol

[Formula 160]

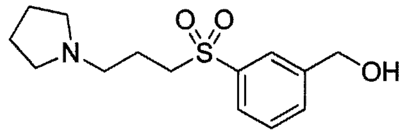

(1) In a test tube for a microwave reaction, potassium carbonate (69.7 mg), sodium iodide (75.6 mg) and pyrrolidine (38.4 μL) were added to a solution of the compound (123 mg) obtained in Reference Example 34-1-(2) in 1,4-dioxane (2.10 mL), and the test tube was sealed. The mixture was stirred at 130°C under microwave irradiation for 30 minutes, and then was stirred using an oil bath at 80°C for 17.5 hours. After cooling to room temperature, the mixture was poured into an aqueous solution of saturated sodium hydrogen carbonate, and the mixture was extracted with ethyl acetate three times. The combined organic layer was washed with brine, and was separated from the aqueous layer by a phase separator, and then the solvent was distilled off under reduced pressure. The obtained residue was purified by NH silica gel column chromatography (n-hexane only, to n-hexane/ethyl acetate = 3:7) to give 1-(3-{3-[(methoxymethoxy)methyl]benzene-1-sulfonyl}propyl)pyrrolidine (102 mg) as a pale orange oily substance.

(2) The compound (102 mg) obtained in the above described (1) was used to perform the reaction according to the method described in Reference Example 34-1-(4) thereby giving the title compound (71.0 mg) as a colorless gummy substance.

$^1$H NMR (400 MHz, CHLOROFORM-d) δ ppm 1.71 - 1.77 (m, 4 H) 1.87 - 1.96 (m, 2 H) 2.38 - 2.46 (m, 4 H) 2.49 (t, J=7.03 Hz, 2 H) 3.16 - 3.22 (m, 2 H) 4.79 (s, 2 H) 7.52 - 7.58 (m, 1 H) 7.62 - 7.67 (m, 1 H) 7.80 - 7.85 (m, 1 H) 7.92 (s, 1 H).

MS ESI/APCI Multi posi: 284 $[M+H]^+$.

The compound of the following Reference Example 35-2 was synthesized using morpholine, according to the method described in Reference Example 35-1. The structure, NMR data and MS data are shown in Table 14-1.

[Table 14-1]

| Reference Example No. | Structure | Analytical Data |
|---|---|---|
| 35-2 | 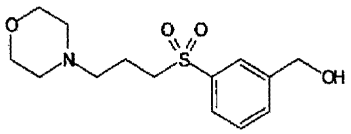 | $^1$H NMR (400 MHz, CHLOROFORM-d) $\delta$ ppm 1.85 - 1. 97 (m, 2 H) 2.33 - 2.45 (m, $\delta$ H) 3.17 - 3.24 (m, 2 H) 3.64 - 3.69 (m, 4 H) 4.81 (s, 2 H) 7.54 - 7.59 (m, 1 H) 7.64 - 7.68 (m, 1 H) 7.81 - 7.86 (m, 1 H) 7.94 (s, 1 H).<br>MS ESI/APCI Multi posi: 300[M+H]$^+$. |

Reference Example 36-1

[3-(Methanesulfonyl)-4-methylphenyl]methanol

[Formula 161]

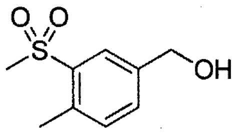

In a test tube for a microwave reaction, sodium methanesulfinate (370 mg), copper(I) trifluoromethanesulfonate benzene complex (152 mg) and N,N'-dimethylethylenediamine (65.1 $\mu$L) were added to a solution of commercially available (3-iodo-4-methylphenyl)methanol (300 mg) in dimethylsulfoxide (4.03 mL); the air in the test tube was purged with nitrogen, and then the test tube was sealed; and then the mixture was stirred at 150°C under microwave irradiation for 1 hour. After cooling to room temperature, the mixture was poured into an aqueous solution of saturated sodium chloride, and the resultant mixture was stirred at room temperature for 1.5 hours. The organic layer was separated, and the aqueous layer was extracted with ethyl acetate. The combined organic layer was washed with brine, and was separated by a phase separator, and then the solvent was distilled off under reduced pressure. The obtained residue was purified by NH silica gel column chromatography (n-hexane/ethyl acetate = 4:1 to 1:9) to give the title compound (170 mg) as a colorless oily substance.
$^1$H NMR (400 MHz, CHLOROFORM-d) $\delta$ ppm 2.71 (s, 3 H) 3.08 (s, 3 H) 4.75 (s, 2 H) 7.34 (d, J=7.8 Hz, 1 H) 7.55 (dd, J=7.8, 1.2 Hz, 1 H) 8.02 (s, 1 H).
MS ESI/APCI Multi posi: 201 [M+H]$^+$, 223 [M+Na]$^+$.
MS ESI/APCI Multi nega: 199 [M-H]$^-$.

Reference Example 37-1

2-[3-(Methanesulfonyl)phenyl]ethan-1-ol

[Formula 162]

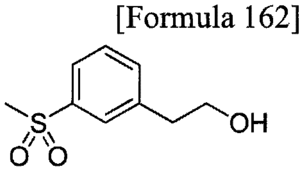

Under a nitrogen atmosphere, borane-tetrahydrofuran complex (0.98 mol/L tetrahydrofuran solution, 1.91 mL) was added to a solution of commercially available [3-(methanesulfonyl)phenyl]acetic acid (200 mg) in tetrahydrofuran

(1.87 mL) under ice cooling, and the mixture was stirred at room temperature for 14.5 hours. The saturated sodium hydrogen carbonate was carefully added to the mixture to stop the reaction, and then the mixture was extracted with ethyl acetate three times. The combined organic layer was washed with water, and was separated by a phase separator, and the solvent was distilled off under reduced pressure. The obtained residue was purified by NH silica gel column chromatography (n-hexane/ethyl acetate = 4: 1, to ethyl acetate only) to give the title compound (160 mg) as a colorless oily substance. $^1$H NMR (600 MHz, CHLOROFORM-d) δ ppm 1.44 (t, J=5.6 Hz, 1 H) 2.97 (t, J=6.4 Hz, 2 H) 3.06 (s, 3 H) 3.89 - 3.96 (m, 2 H) 7.48 - 7.57 (m, 2 H) 7.77 - 7.87 (m, 2 H).
MS ESI/APCI Multi posi: 223 $[M+Na]^+$.
MS ESI/APCI Multi nega: 235 $[M+Cl]^-$.

The compounds of the following Reference Examples 37-2 and 37-3 were synthesized using respective corresponding benzoic acid analogs, according to the method described in Reference Example 37-1. These structures, NMR data and MS data are shown in Table 15-1.

[Table 15-1]

| Reference Example No. | Structure | Analytical Data |
|---|---|---|
| 37-2 | 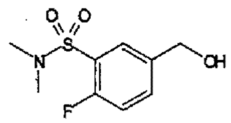 | $^1$H NMR (300 MHz, CHLOROFORM-d) 5 ppm 2.01 (t, J=5.5 Hz, 1 H) 2.83 - 2.86 (m, 6 H) 4.74 (d, J=5.6 Hz, 2 H) 7.18 - 7.25 (m, 1 H) 7.43 - 7.68 (m, 1 H) 7.75 - 7.93 (m, 1 H). |
| 37-3 | 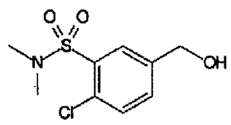 | $^1$1H NMR (300 MHz, CHLOROFORM-d) $\delta$ ppm 1.93 - 2.02 (m, 1 H) 2.90 (s, 6 H) 4.70 - 4.78 (m, 2 H) 7.48 - 7.54 (m, 2 H) 7.97 - 8.11 (m, 1 H). |

Reference Example 38-1

2-[2-(Methanesulfonyl)phenoxy]ethan-1-ol

[Formula 163]

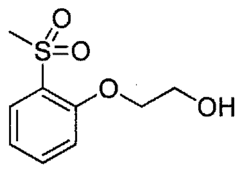

(1) Palladium(II) acetate (8.15 g), tri(o-tolyl)phosphine (33.2 mg) and potassium carbonate (836 mg) were added to a solution of isopropyl bromoacetate (188 μL) in tetrahydrofuran (4.03 mL). A solution of 2-(methanesulfonyl)phenol (242 mg) and water (54.5 μL) in tetrahydrofuran (4.03 mL) was added to the mixture for 30 minutes or more. The mixture was stirred at room temperature for 19.5 hours, and then was poured into water, and the resultant mixture was extracted with chloroform three times. The combined organic layer was washed with water, and was separated by a phase separator, and the solvent was distilled off under reduced pressure. The obtained residue was purified by silica gel column chromatography (n-hexane only, to n-hexane/ethyl acetate = 2:3) to give isopropyl [2-(methanesulfonyl)phenoxy]acetate (319 mg) as a yellow oily substance.

(2) The compound (319 mg) obtained in the above described (1) was used to perform the reaction according to the method described in Reference Example 20-1-(4) thereby giving the title compound (175 mg) as a colorless oily substance.
$^1$H NMR (600 MHz, CHLOROFORM-d) δ ppm 3.25 (s, 3 H) 3.36 (t, J=6.4 Hz, 1 H) 3.92 - 3.97 (m, 2 H) 4.33 - 4.37

(m, 2 H) 7.09 (d, J=7.8 Hz, 1 H) 7.14 - 7.18 (m, 1 H) 7.59 - 7.64 (m, 1 H) 7.96 (dd, J=7.8, 1.7 Hz, 1 H).
MS ESI/APCI Multi posi: 217 $[M+H]^+$, 239 $[M+Na]^+$.
MS ESI/APCI Multi nega: 251 $[M+Cl]^-$.

Reference Example 39-1

1-[6-(Hydroxymethyl)-3-azabicyclo[3.1.0]hexan-3-yl]ethan-1-one

[Formula 164]

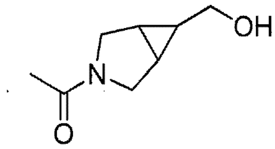

(1) Commercially available ethyl 3-azabicyclo[3.1.0]hexane-6-carboxylate hydrochloride (871 mg) was used to perform the reaction according to the method described in Reference Example 25-1 thereby giving ethyl 3-acetyl-3-azabicyclo[3.1.0]hexane-6-carboxylate (896 mg) as a colorless oily substance. The obtained compound was used for the next reaction without being purified.
(2) The compound (896 mg) obtained in the above described (1) was used to perform the reaction according to the method described in Reference Example 20-1-(4) thereby giving the title compound (647 mg) as a colorless oily substance.
$^1$H NMR (600 MHz, CHLOROFORM-d) δ ppm 0.91 - 0.97 (m, 1 H) 1.49 - 1.59 (m, 2 H) 2.00 (s, 3 H) 2.97 - 3.85 (m, 7 H).
MS ESI/APCI Multi posi: 156 $[M+H]^+$.

Reference Example 40-1

1-[3-(Hydroxymethyl)-8-azabicyclo[3.2.1]octan-8-yl]ethan-1-one

[Formula 165]

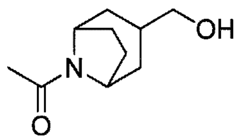

(1) A solution of 8-benzyl-8-azabicyclo[3.2.1]octane-3-carboxylic acid (478 mg) in tetrahydrofuran (6.50 mL) was added dropwise to a suspension of lithium aluminum hydride (89.0 mg) in tetrahydrofuran (6.50 mL) under ice cooling. The mixture was heated to reflux under a nitrogen atmosphere for 2 hours, and then water (90.0 μL), an aqueous solution (90.0 μL) of 15% sodium hydroxide and water (270 μL) were added to the mixture in this order under ice cooling. After the resultant mixture was stirred for a while, anhydrous magnesium sulfate was added thereto, and the solid was filtered off, and was washed with diethyl ether (60 mL). The filtrate and the washing solution were combined, and the solvent was distilled off under reduced pressure to give (8-benzyl-8-azabicyclo[3.2.1]octan-3-yl)methanol (432 mg) as a colorless oily substance. The obtained compound was used for the next reaction without being purified.
(2) The compound (432 mg) obtained in the above described (1) was used to perform the reaction according to the method described in Reference Example 20-1-(2) thereby giving (8-azabicyclo[3.2.1]octan-3-yl)methanol (270 mg) as a colorless oily substance. The obtained compound was used for the next reaction without being purified.
(3) The compound (270 mg) obtained in the above described (2) was used to perform the reaction according to the method described in Reference Example 25-1 thereby giving the title compound (233 mg) as a colorless solid.
$^1$H NMR (600 MHz, CHLOROFORM-d) δ ppm 1.32 - 1.40 (m, 1 H) 1.43 - 1.51 (m, 1 H) 1.59 - 1.64 (m, 1 H) 1.66 -

1.72 (m, 1 H) 1.73 - 1.81 (m, 2 H) 1.88 - 1.98 (m, 1 H) 1.99 - 2.22 (m, 5 H) 3.40 - 3.52 (m, 2 H) 4.10 - 4.17 (m, 1 H) 4.67 - 4.74 (m, 1 H).
MS ESI/APCI Multi posi: 184 $[M+H]^+$.

Reference Example 41-1

1-[6-(Hydroxymethyl)-2-azaspiro[3.3]heptan-2-yl]ethan-1-one

[Formula 166]

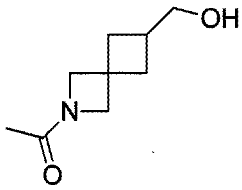

(1) Commercially available 2-(tert-butoxycarbonyl)-2-azaspiro[3.3]heptane-6-carboxylic acid (503 mg) was used to perform the reaction according to the method described in Reference Example 32-1 thereby giving tert-butyl 6-(hydroxymethyl)-2-azaspiro[3.3]heptane-2-carboxylate (486 mg) as a colorless oily substance. The obtained compound was used for the next reaction without being purified.

(2) The compound (486 mg) obtained in the above described (1) was used to perform the reaction according to the method described in Reference Example 17-1-(2) thereby giving a mixture mainly containing (2-azaspiro[3.3]heptan-6-yl)methanol hydrochloride as a colorless solid. The obtained substance was used for the next reaction without being purified.

(3) The mixture obtained in the above described (2) was used to perform the reaction according to the method described in Reference Example 25-1 using pyridine as a solvent, whereby the title compound (265 mg) was obtained as a colorless oily substance.
$^1$H NMR (600 MHz, CHLOROFORM-d) $\delta$ ppm 1.83 - 1.85 (m, 3 H) 1.98 - 2.02 (m, 2 H) 2.25 - 2.30 (m, 2 H) 2.36 - 2.44 (m, 1 H) 3.57 - 3.60 (m, 2 H) 3.90 (s, 1 H) 4.00 (s, 1 H) 4.02 (s, 1 H) 4.11 (s, 1 H).
MS ESI/APCI Multi posi: 170 $[M+H]^+$, 192 $[M+Na]^+$.

The compounds of the following Reference Examples 41-2 and 41-3 were synthesized using a corresponding carboxylic acid, according to the method described in Reference Example 41-1. These structures, NMR data and MS data are shown in Table 16-1.

[Table 16-1]

| Reference Example No. | Structure | Analytical Data |
|---|---|---|
| 41-2 | 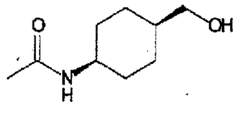 | $^1$H NMR (600 MHz, DMSO-$d_6$) $\delta$ ppm 1. 26 - 1. 52 (m, 9 H) 1.79 (s, 3 H) 3.24 - 3.28 (m, 2 H) 3.72 - 3.77 (m, 1 H) 4.37 (t, J=5.4 Hz, 1 H) 7.60 - 7.66 (m, 1 H).<br>MS ESI/APCI Multi nega: 170$[M-H]^-$, 216 $[M+HC00]^-$. |
| 41-3 | 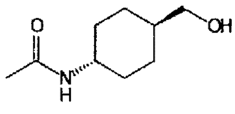 | $^1$H NMR (600 MHz, DMSO-$d_6$) $\delta$ ppm 0.82 - 0.91 (m, 2 H) 1.01 - 1.10 (m, 2 H) 1.19 - 1.28 (m, 1 H) 1.66 - 1.79 (m, 7 H) 3.14 - 3.18 (m, 2 H) 3.37 - 3.41 (m, 1 H) 4.34 (t, J=5.16 Hz, 1 H) 7.61 - 7.67 (m, 1 H).<br>MS ESI/APCI Multi nega: 170$[M-H]^-$. |

Reference Example 42-1

1-[3-(3-Hydroxypropyl)azetidin-1-yl]ethan-1-one

[Formula 167]

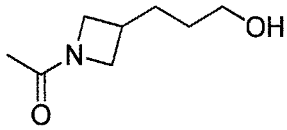

(1) Commercially available tert-butyl 3-(hydroxymethyl)azetidine-1-carboxylate (500 mg) was used to perform the reaction according to the method described in Reference Example 28-1-(1) thereby giving tert-butyl 3-formylazetidine-1-carboxylate (410 mg) as a colorless oily substance.

(2) 1,8-Diazabicyclo[5.4.0]undec-7-ene (364 μL) and lithium chloride (103 mg) were added to a solution of trimethyl phosphonoacetate (484 mg) in tetrahydrofuran (5 mL) under ice cooling, and the mixture was stirred for 10 minutes. A solution of the compound (410 mg) obtained in the above described (1) in tetrahydrofuran (5 mL) was added thereto under ice cooling, and the mixture was stirred at room temperature for 30 minutes. An aqueous solution of saturated ammonium chloride and chloroform were added to the reaction mixture, the organic layer was separated by a phase separator, and then the solvent was distilled off under reduced pressure. The obtained residue was purified by silica gel column chromatography (n-hexane only, to n-hexane/ethyl acetate = 7:3) to give tert-butyl 3-(3-methoxy-3-oxoprop-1-en-1-yl)azetidine-1-carboxylate (501 mg) as a colorless oily substance.

(3) To a solution of the compound (501 mg) obtained in the above described step (2) in methanol (10 mL), 20% palladium-carbon (50.1 mg) was added, and the mixture was stirred under a hydrogen atmosphere at room temperature for 1 hour. The reaction solution was filtered through Celite (registered trademark), and then the filtrate was concentrated to give tert-butyl 3-(3-methoxy-3-oxopropyl)azetidine-1-carboxylate (447 mg) as a colorless oily substance. The obtained compound was used for the next reaction without being purified.

(4) The compound (447 mg) obtained in the above described (3) was used to perform the synthesis process according to the method described in Reference Example 20-1-(4) thereby giving tert-butyl 3-(3-hydroxypropyl)azetidine-1-carboxylate (393 mg) as a colorless oily substance.

(5) Trifluoroacetic acid (1.00 mL) was added to a solution of the compound (140 mg) obtained in the above described (4) in chloroform (2.5 mL), and the mixture was stirred at room temperature for 1 hour. The reaction mixture was concentrated. Triethylamine (180 μL) and acetic acid anhydride (73.7 μL) were added to a solution of the obtained residue in chloroform (2.5 mL), and the mixture was stirred at room temperature for 2 hours. Methanol and potassium carbonate were added to the reaction mixture, and the resultant mixture was stirred at room temperature for 24 hours, and then was concentrated under reduced pressure. The obtained residue was purified by silica gel column chromatography (chloroform only, to chloroform/methanol = 1:1) to give the title compound (22 mg) as a colorless oily substance.

$^1$H NMR (400 MHz, CHLOROFORM-d) δ ppm 1.49 - 1.59 (m, 2 H) 1.65 - 1.74 (m, 2 H) 1.85 (s, 3 H) 2.50 - 2.65 (m, 1 H) 3.58 - 3.68 (m, 3 H) 3.68 - 3.75 (m, 1 H) 4.04 - 4.11 (m, 1 H) 4.17 - 4.24 (m, 1 H)

MS ESI/APCI Multi posi: 158 $[M+H]^+$.

Reference Example 43-2

(3-Fluoro-5-methylsulfonylphenyl)methanol

[Formula 168]

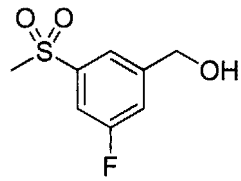

(1) Borane-tetrahydrofuran complex (0.98 mol/L tetrahydrofuran solution, 9.3 mL) was added to a solution of 3-bromo-5-fluorobenzoic acid (1.0 g) in tetrahydrofuran (23 mL) under ice cooling, the ice bath was removed, and the mixture was stirred overnight. Water was added to the reaction solution under ice cooling, the mixture was extracted with chloroform, and the organic layer was separated by a phase separator. The obtained organic layer was concentrated under reduced pressure, and the obtained residue was purified by silica gel column chromatography (chloroform/methanol = 99:1 to 47:3) to give (3-bromo-5-fluorophenyl)methanol (966 mg) as a colorless oily substance.

(2) In a test tube for a microwave reaction, sodium methanesulfinate (1.44 g), copper(I) trifluoromethanesulfonate benzene complex (711 mg) and N,N'-dimethylethylenediamine (304 μL) were added to a solution of the compound (966 mg) obtained in the above described (1) in dimethylsulfoxide (19 mL), the air in the test tube was purged with nitrogen, then the test tube was sealed, and the mixture was stirred at 150°C under microwave irradiation for 1 hour. After cooling to room temperature, the mixture was poured into an aqueous solution of saturated ammonium chloride, and the resultant mixture was stirred at room temperature for a while and then was extracted with ethyl acetate. The organic layer was washed with water and subsequently with brine, and was separated by a phase separator, and the solvent was concentrated under reduced pressure. The obtained residue was purified by silica gel column chromatography (chloroform/methanol = 97:3 to 91:9) to give the title compound (767 mg) as a colorless oily substance.

$^1$H NMR (400 MHz, CHLOROFORM-d) δ ppm 2.07 (t, J=5.81 Hz, 1 H) 3.07 (s, 3 H) 4.81 (d, J=5.75 Hz, 2 H) 7.37 - 7.43 (m, 1 H) 7.52 - 7.58 (m, 1 H) 7.72 - 7.76 (m, 1 H). MS ESI/APCI Multi posi: 227 $[M+Na]^+$.

Reference Example 44-1

[3-(Cyclopropylmethanesulfonyl)phenyl]methanol

[Formula 169]

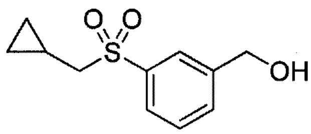

(1) Commercially available 3-sulfanylbenzoic acid (400 mg) and bromomethyl cyclopropane were used to perform the reaction according to the method described in Reference Example 33-1-(2) thereby giving cyclopropylmethyl 3-[(cyclopropylmethyl)sulfanyl]benzoate (285 mg) as a colorless oily substance.

(2) The compound (285 mg) obtained in the above described (1) was used to perform the reaction according to the method described in Reference Example 33-1-(3) thereby giving cyclopropylmethyl 3-(cyclopropylmethanesulfonyl)benzoate (334 mg) as a colorless oily substance.

(3) The compound (334 mg) obtained in the above described (2) was used to perform the reaction according to the method described in Reference Example 20-1-(4) thereby giving the title compound (241 mg) as a colorless oily substance.

$^1$H NMR (600 MHz, CHLOROFORM-d) δ ppm 0.10 - 0.19 (m, 2 H) 0.51 - 0.62 (m, 2 H) 0.96 - 1.05 (m, 1 H) 1.88 - 1.94 (m, 1 H) 3.03 (d, J=7.0 Hz, 2 H) 4.81 (d, J=5.8 Hz, 2 H) 7.53 - 7.59 (m, 1 H) 7.66 (d, J=7.8 Hz, 1 H) 7.86 (d, J=7.4 Hz, 1 H) 7.95 (s, 1 H).

MS ESI/APCI Multi posi: 227 $[M+H]^+$, 249 $[M+Na]^+$.

MS ESI/APCI Multi nega: 263 $[M+Cl]^-$.

Reference Example 45-1

1-(Azetidin-1-yl)-2-[3-(hydroxymethyl)benzene-1-sulfonyl]ethan-1-one

[Formula 170]

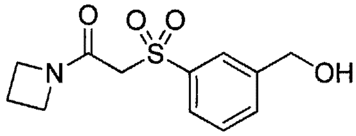

(1) Commercially available ethyl 3-iodobenzoate (415 mg) and tert-butyl bromoacetate (214 μL) were used to perform the reaction according to the method described in Reference Example 31-1-(1) thereby giving ethyl 3-(2-tert-butoxy-2-oxoethanesulfonyl)benzoate (332 mg) as a brown oily substance.
(2) Trifluoroacetic acid (1.65 mL) was added to a solution of the compound (163 mg) obtained in the above described (1) in chloroform (1.65 mL), and the mixture was stirred at room temperature for 14.5 hours. The mixture was concentrated under reduced pressure to give a mixture (149 mg) mainly containing [3-(ethoxycarbonyl)benzene-1-sulfonyl]acetic acid as a yellow oily substance. The obtained mixture was used for the next reaction without being purified.
(3) Azetidine hydrochloride (38.6 mg), 1-hydroxybenzotriazole monohydrate (63.2 mg), triethylamine (76.6 μL) and 1-(3-dimethylaminopropyl)-3-ethylcarbodiimide hydrochloride (79.1 mg) were added to a solution of the mixture (87.0mg) obtained in the above described step (2) in chloroform (1.38 mL), and the mixture was stirred at room temperature for 15.5 hours. An aqueous solution of saturated ammonium chloride was added to the mixture, and the resultant mixture was extracted three times with chloroform. The combined organic layer was washed with water, and was separated by a phase separator, and then the solvent was distilled off under reduced pressure. The obtained residue was purified by silica gel column chromatography (n-hexane/ethyl acetate = 7:3, to ethyl acetate only) to give ethyl 3-[2-(azetidin-1-yl)-2-oxoethanesulfonyl]benzoate (68.0 mg) as a colorless solid.
(4) The compound (65.0 mg) obtained in the above described (3) was used to perform the synthesis process according to the method described in Reference Example 20-1-(4) thereby giving the title compound (48.0 mg) as a colorless oily substance.
$^1H$ NMR (600 MHz, CHLOROFORM-d) δ ppm 2.26 - 2.32 (m, 2 H) 3.92 (s, 2 H) 4.05 (t, J=7.8 Hz, 2 H) 4.35 (t, J=7.8 Hz, 2 H) 4.79 (d, J=5.8 Hz, 2 H) 7.53 - 7.58 (m, 1 H) 7.67 (d, J=7.4 Hz, 1 H) 7.85 (d, J=7.8 Hz, 1 H) 7.95 (s, 1 H).
MS ESI/APCI Multi posi: 270 $[M+H]^+$.

The compound of the following Reference Example 45-2 was synthesized using the compound obtained in Reference Example 45-1-(2) and pyrrolidine, according to the method described in Reference Example 45-1-(3) to (4). The structure, NMR data and MS data are shown in Table 17-1.

[Table 17-1]

| Reference Example No. | Structure | Analytical Data |
|---|---|---|
| 45-2 | 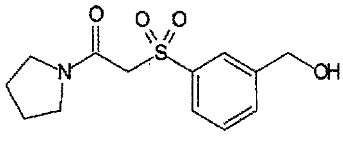 | $^1H$ NMR (600 MHz, CHLOROFORM-d) δ ppm 1.87 - 1.92 (m, 2 H) 1.96 - 2.02 (m, 2 H) 3.44 (t, J=7.0 Hz, 2 H) 3.64 (t, J=7.0 Hz, 2 H) 4.16 (s, 2 H) 4.79 (d, J=5.0 Hz, 2 H) 7.53 - 7.58 (m, 1 H) 7.67 (d, J=7.8 Hz, 1 H) 7.85 (d, J=7.8 Hz, 1 H) 7.94 (s, 1 H). MS ESI/APCI Multi posi: 284$[M+H]^+$. |

Reference Example 46-1

(2R)-2-Methoxypropan-1-amine

[Formula 171]

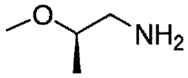

(1) Triethylamine (8.35 mL) was added to a solution of (R)-(-)-1-amino-2-propanol (3.00 g) and di-tert-butyl dicarbonate (9.59 g) in tetrahydrofuran (50 mL), and the mixture was stirred at room temperature for 20 hours. The solvent was distilled off under reduced pressure, an aqueous solution of 20% citric acid was added thereto, and the mixture was extracted with ethyl acetate. The organic layer was washed with an aqueous solution of saturated sodium hydrogen carbonate and was separated by a phase separator. The filtrate was concentrated under reduced pressure to give tert-butyl N-[(2R)-2-hydroxypropyl]carbamate (8.30 g) as a pale yellow oily substance.
(2) Sodium hydride (1.10 g) and methyl iodide (1.56 mL) were added to a solution of the compound (4.00 g) obtained in the above described (1) in tetrahydrofuran (50 mL) under ice cooling, and the mixture was stirred at the same temperature for 2 hours. Water was added to the reaction solution, and the mixture was extracted with ethyl acetate. The organic layer was washed with water, and was separated by a phase separator. The filtrate was concentrated under reduced pressure, and the obtained residue was purified by silica gel column chromatography (n-hexane/ethyl acetate = 4:1 to 1:1) to give tert-butyl N-[(2R)-2-methoxypropyl]carbamate (700 mg) as a pale yellow oily substance.
(3) A solution of 4 mol/L hydrogen chloride in ethyl acetate (5 mL) was added to a solution of the compound (700 mg) obtained in the above described (2) in ethyl acetate (5 mL), and the mixture was stirred at room temperature for 20 hours. The solvent was distilled off under reduced pressure, an aqueous solution of 1 mol/L sodium hydroxide was added to the residue, and the mixture was extracted with a solution of chloroform/methanol = 9:1. The organic layer was separated by a phase separator, and the solvent was distilled off under reduced pressure to give the title compound (435 mg) as a pale yellow amorphous substance.
$^1$H NMR (600 MHz, CHLOROFORM-d) $\delta$ ppm 1.21 (d, J=6.2 Hz, 3 H) 2.88 - 2.93 (m, 1 H) 3.11 - 3.16 (m, 1 H) 3.40 (s, 3 H) 3.72 - 3.78 (m, 1 H).
MS ESI/APCI Multi posi: 90 [M+H]$^+$.

Reference Example 48-1

2-Amino-1-(pyrrolidin-1-yl)ethan-1-one

[Formula 172]

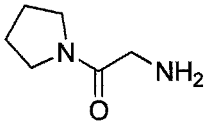

(1) Pyrrolidine (614 $\mu$L), diisopropylethylamine (1.46 mL) and anhydrous propylphosphonic acid (1.7 mol/L ethyl acetate solution, 5.04 mL) were added to a solution of N-(tert-butoxycarbonyl)glycine (1.00 g) in ethyl acetate (20 mL), and the mixture was stirred at room temperature for 20 hours. To the reaction solution, 0.5 mol/L hydrochloric acid was added, and the mixture was extracted with ethyl acetate. The organic layer was sequentially washed with 0.5 mol/L hydrochloric acid, an aqueous solution of 1 mol/L sodium hydroxide and water, and was separated by a phase separator. The solvent was distilled off under reduced pressure to give tert-butyl N-(2-oxo-2-pyrrolidin-1-ylethyl)carbamate (1.00 g) as a colorless powder.
(2) A solution of 4 mol/L hydrogen chloride in ethyl acetate (5 mL) was added to a solution of the compound (1.00

g) obtained in the above described (1) in ethyl acetate (5 mL), and the mixture was stirred at room temperature for 20 hours. The solvent was distilled off under reduced pressure, an aqueous solution of 1 mol/L sodium hydroxide was added to the residue, and the mixture was extracted with a solution of chloroform/methanol = 9:1. The organic layer was separated by a phase separator, and the solvent was distilled off under reduced pressure to give the title compound (98 mg) as a pale yellow oily substance. $^1$H NMR (600 MHz, CHLOROFORM-d) δ ppm 1.81 - 1.91 (m, 2 H) 1.92 - 2.01 (m, 2 H) 3.33 (t, J=6.8 Hz, 2 H) 3.37 (s, 2 H) 3.50 (t, J=6.8 Hz, 2 H).
MS ESI/APCI Multi posi: 129 $[M+H]^+$.

Reference Example 53-1

5-1[3-(Cyclopent-1-en-1-yl)phenyl]methoxy}-2-(1H-pyrazol-5-yl)pyridine

[Formula 173]

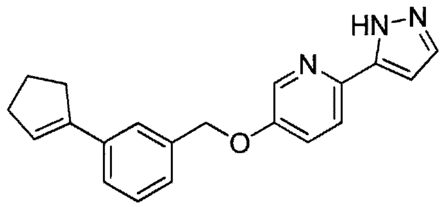

(1) An aqueous solution of 2 mol/L potassium carbonate (1.3 mL), cyclopenten-1-ylboronic acid (35 mg) and tetrakis(triphenylphosphine)palladium(0) (60 mg) were added to a solution of the compound (106 mg) obtained in Reference Example 4-1 in 1,4-dioxane (3 mL), and the mixture was stirred at 120°C under microwave irradiation for 20 minutes. After cooling to room temperature, the reaction solution was concentrated under reduced pressure, and the obtained residue was purified by silica gel column chromatography (n-hexane/ethyl acetate = 9:1, to ethyl acetate only) to give 5-{[3-(cyclopenten-1-yl)phenyl]methoxy}-2-[2-(oxan-2-yl)pyrazol-3-yl]pyridine (73 mg) as a pale yellow oily substance.
(2) The compound (73 mg) obtained in the above described (1) was used to perform the synthesis process according to the method described in Reference Example 14-1 thereby giving the title compound (39 mg) as a colorless powder. $^1$H NMR (300 MHz, CHLOROFORM-d) δ ppm 1.97 - 2.09 (m, 2 H) 2.48 - 2.59 (m, 2 H) 2.66 - 2.78 (m, 2 H) 5.14 (s, 2 H) 6.19 - 6.24 (m, 1 H) 6.68 (d, J=2.0 Hz, 1 H) 7.27 - 7.38 (m, 3 H) 7.40 - 7.45 (m, 1 H) 7.48 - 7.52 (m, 1 H) 7.58 - 7.68 (m, 2 H) 8.36 (d, J=2.8 Hz, 1 H). MS ESI/APCI Multi posi: 318 $[M+H]^+$.

Reference Example 53-2

5-[(3-Ethylphenyl)methoxy]-2-(1H-pyrazol-5-yl)pyridine

[Formula 174]

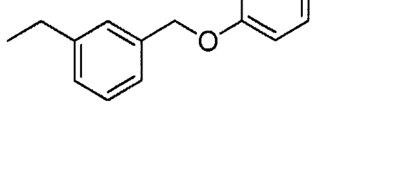

(1) Commercially available 2-ethenyl-4,4,5,5-tetramethyl-1,3,2-dioxaborolane (44 μL) was used to perform the synthesis process according to the method described in Reference Example 53-1-(1) thereby giving 5-[(3-ethylphenyl)methoxy]-2-[2-(oxan-2-yl)pyrazol-3-yl]pyridine (73 mg) as a brown oily substance.
(2) Concentrated hydrochloric acid (2 drops) and 10% palladium-carbon (10 mg) were added to a solution of the compound (73 mg) obtained in the above described (1) in methanol (4 mL), and the mixture was stirred under a hydrogen atmosphere at room temperature for 3 hours. The reaction solution was filtered through Celite (registered trademark), and then the filtrate was concentrated under reduced pressure. The obtained residue was purified by silica gel column chromatography (n-hexane/ethyl acetate = 9: 1, to ethyl acetate only). The obtained purified substance was powderized in diethyl ether/n-hexane to give the title compound (17 mg) as a pale yellow powder.
$^1$H NMR (300 MHz, CHLOROFORM-d) δ ppm 1.26 (t, J=7.6 Hz, 3 H) 2.68 (q, J=7.6 Hz, 2 H) 5.13 (s, 2 H) 6.68 (d, J=1.9 Hz, 1 H) 7.17 - 7.35 (m, 5 H) 7.60 - 7.68 (m, 2 H) 8.37 (d, J=2.8 Hz, 1 H).
MS ESI/APCI Multi posi: 280 [M+H]$^+$.

The compounds of the following Reference Examples 53-3 to 53-5 were synthesized using a commercially available corresponding alkenylboronic acid or alkenylboronic acid ester, respectively, according to the method described in Reference Example 53-2. These structures, NMR data and MS data are shown in Table 18-1.

[Table 18-1]

| Reference Example No. | Structure | Analytical Data |
|---|---|---|
| 53-3 | 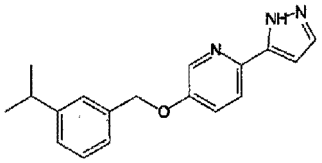 | $^1$H NMR (300 MHz, CHLOROFORM-d) δ ppm 1.28 (d, J=6. 8 Hz, 6 H) 2.89 - 3.01 (m, 1 H) 5.14 (s, 2 H) 6.66 - 6.73 (m, 1 H) 7.21 - 7.41 (m, 5 H) 7.61 - 7.72 (m, 2 H) 8.38 (d, J=2.6 Hz, 1 H).<br>MS ESI/APCI Multi posi: 294[M+H]$^+$. |
| 53-4 | 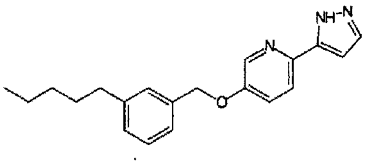 | $^1$H NMR (300 MHz, CHLOROFORM-d) δ ppm 0.86 - 0.93 (m, 3 H) 1.30 - 1.38 (m, 4 H) 1.63 - 1.67 (m, 2 H) 2. 59 - 2.69 (m, 2 H) 5.14 (s, 2 H) 6.65 - 6.73 (m, 1 H) 7.15 - 7.38 (m, 5 H) 7.60 - 7.69 (m, 2 H) 8.37 (d, J=2.8 Hz, 1 H).<br>MS ESI/APCI Multi posi: 322[M+H]$^+$. |
| 53-5 | 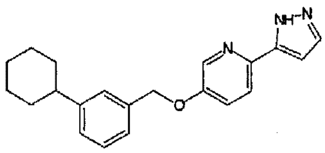 | $^1$H NMR (600 MHz, CHLOROFORM-d) δ ppm 1.33 - 1.48 (m, 4 H) 1.71 - 1.81 (m, 2 H) 1.81 - 1.92 (m, 4 H) 2.49 - 2.58 (m, 1 H) 5.12 (s, 2 H) 6.68 (s, 1 H) 7.18 - 7.29 (m, 3 H) 7.30 - 7.36 (m, 2 H) 7.60 - 7.67 (m, 2 H) 8.36 (d, J=2.9 Hz, 1 H).<br>MS ESI/APCI Multi posi: 334[M+H]$^+$. |

Reference Example 54-1

5-[(3-Methylphenyl)methoxy]-2-(1H-pyrazol-5-yl)pyridine

[Formula 175]

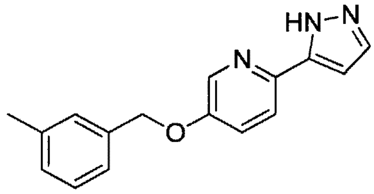

(1) (3-Methylphenyl)methanol (18 mg), triphenylphosphine (63 mg) and diisopropyl azodicarboxylate (1.9 mol/L toluene solution, 1.1 mL) were added to a solution of the compound (40 mg) obtained in Reference Example 1-1 in

tetrahydrofuran (1 mL), at 0°C, and then the temperature of the mixture was increased to room temperature and the mixture was stirred for 1.5 hours. The reaction solution was concentrated, and the obtained residue was purified by silica gel column chromatography (n-hexane/ethyl acetate = 4:1 to 1:1) to give 5-[(3-methylphenyl)methoxy]-2-[2-(oxan-2-yl)pyrazol-3-yl]pyridine (106 mg).

(2) The compound (106 mg) obtained in the above described (1) was dissolved in 2 mol/L hydrogen chloride-methanol (2 mL), water (0.3 mL) was added to the mixture, and the resultant mixture was stirred at room temperature for 30 minutes. After the end of the reaction was confirmed by LC-MS, an aqueous solution of saturated sodium hydrogen carbonate was added thereto, and the mixture was extracted with ethyl acetate. The organic layer was dried over magnesium sulfate, the drying agent was filtered off, and the filtrate was concentrated under reduced pressure. The obtained residue was purified by silica gel column chromatography (chloroform/methanol = 99:1 to 95:5). The obtained roughly purified substance was purified by preparative HPLC to give the title compound (16.6 mg) as a colorless solid.
$^1$H NMR (300 MHz, CHLOROFORM-d) $\delta$ ppm 2.38 (s, 3 H) 5.11 (s, 2 H) 6.65 - 6.70 (m, 1 H) 7.12 - 7.35 (m, 5 H) 7.59 - 7.69 (m, 2 H) 8.36 (d, J=2.3 Hz, 1 H).
MS ESI/APCI Multi posi: 266 [M+H]$^+$.

Reference Example 54-2

5-[(4-Methylphenyl)methoxy]-2-(1H-pyrazol-5-yl)pyridine

[Formula 176]

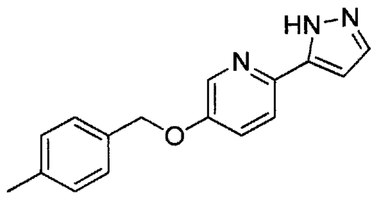

(1) 1-(Chloromethyl)-4-methylbenzene (26 μL) and potassium carbonate (45 mg) were added to a solution of the compound (40 mg) obtained in Reference Example 1-1 in N,N-dimethylformamide (820 μL), and the mixture was stirred overnight at room temperature. Water was added to the reaction solution, the mixture was extracted with ethyl acetate, and the obtained organic layer was sequentially washed with water and brine. The organic layer was dried over anhydrous magnesium sulfate, and was then filtered, and the filtrate was concentrated under reduced pressure. The obtained residue was purified by silica gel column chromatography (n-hexane/ethyl acetate = 7:3 to 2:3) to give 5-[(4-methylphenyl)methoxy]-2-[2-(oxan-2-yl)pyrazol-3-yl]pyridine (64 mg) as a colorless oily substance.
(2) Water (400 μL) and trifluoroacetic acid (200 μL) were added to a solution of the compound (64 mg) obtained in the above described (1) in methanol (800 μL), and the mixture was stirred at 60°C for 3 hours. An aqueous solution of saturated sodium hydrogen carbonate was added to the reaction solution; the mixture was extracted with chloroform; the organic layer was dried over anhydrous magnesium sulfate, and then was filtered; and the filtrate was concentrated under reduced pressure. The obtained solid was recrystallized in ethyl acetate/n-hexane to give the title compound (30 mg) as a colorless solid.
$^1$H NMR (300 MHz, CHLOROFORM-d) $\delta$ ppm 2.37 (s, 3 H) 5.11 (s, 2 H) 6.65 - 6.71 (m, 1 H) 7.18 - 7.24 (m, 2 H) 7.27 - 7.36 (m, 3 H) 7.59 - 7.69 (m, 2 H) 8.34 - 8.39 (m, 1 H). MS ESI/APCI Multi posi: 266 [M+H]$^+$.

The compounds of the following Reference Examples 54-3 to 54-21 were synthesized using a commercially available corresponding benzyl halide according to the method described in Reference Example 54-2. These structures and MS data are shown in Tables 19-1 to 19-3.

[Table 19-1]

| Reference Example No. | Structure | Analytical Data |
| --- | --- | --- |
| 54-3 | 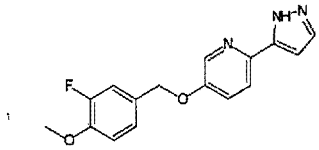 | MS ESI posi: 300[M+H]+. |
| 54-4 | 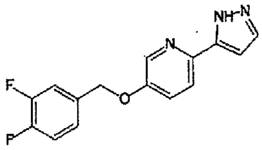 | MS ESI posi: 288[M+H]+. |
| 54-5 | 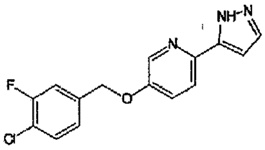 | MS ESI posi: 304[M+H]+. |
| 54-6 | 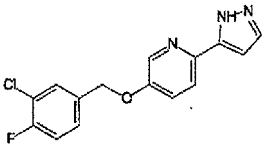 | MS ESI posi: 304[M+H]+. |
| 54-7 | 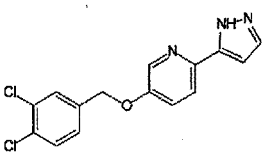 | MS ESI posi: 320[M+H]+. |
| 54-8 | 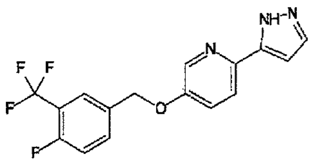 | MS ESI posi: 338[M+H]+. |
| 54-9 | 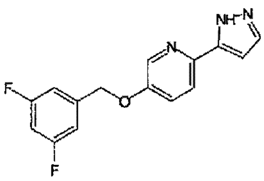 | MS ESI posi: 288[M+H]+. |
| 54-10 | 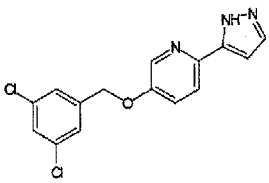 | MS ESI posi: 320[M+H]+. |

[Table 19-2]

| Reference Example No. | Structure | Analytical Data |
|---|---|---|
| 54-11 | 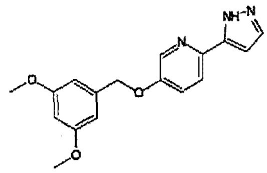 | MS ESI posi: 312[M+H]+. |
| 54-12 | 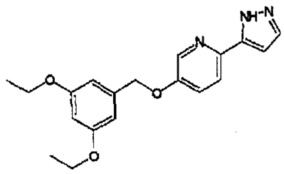 | MS ESI posi: 340[M+H]+. |
| 54-13 | 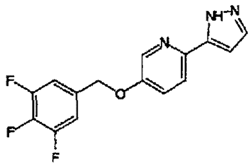 | MS ESI posi: 306[M+H]+. |
| 54-14 | 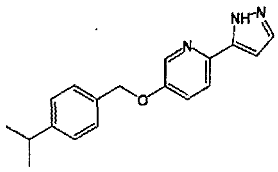 | MS ESI posi: 294[M+H]+. |
| 54-15 | 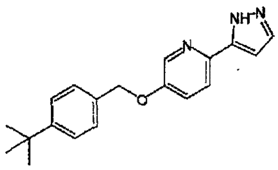 | MS ESI posi: 308[9+H]+. |
| 54-16 | 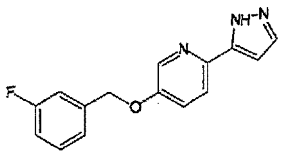 | MS ESI posi: 270[M+H]+. |
| 54-17 | 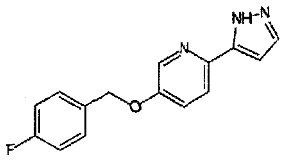 | MS ESI posi: 270[M+H]+. |
| 54-18 | 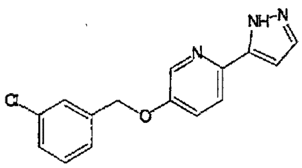 | MS ESI posi: 286[M+H]+. |
| 54-19 | 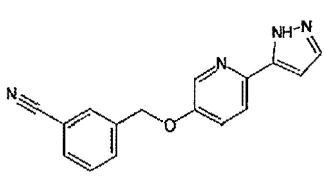 | MS ESI posi: 277[M+H]+. |

[Table 19-3]

| Reference Example No. | Structure | Analytical Data |
|---|---|---|
| 54-20 | 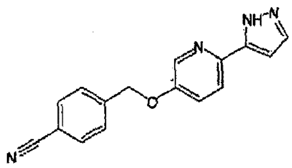 | MS ESI posi: 277[M+H]+. |
| 54-21 | 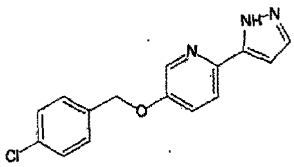 | MS ESI posi: 286[M+H]+. |

The above described compounds of Reference Examples 53-1 to 53-5 and Reference Examples 54-1 to 54-21 have the following human CYP4F2 and CYP4A11 inhibitory activity in Test Example 1 described below. Table 20-1 shows the 50% inhibitory concentration ($IC_{50}$ value) of each compound.

[Table 20-1]

| Reference Example No. | $IC_{50}$ value [nM] | |
|---|---|---|
| | Human CYP4F2 | Human CYP4A11 |
| 53-1 | 25 | 210 |
| 53-2 | 88 | 120 |
| 53-3 | 140 | 53 |
| 53-4 | 32 | 460 |
| 53-5 | 130 | 660 |
| 54-1 | 88 | 340 |
| 54-2 | 10 | 560 |
| 54-3 | 12 | 660 |
| 54-4 | 24 | 350 |
| 54-5 | 13 | 640 |
| 54-6 | 29 | 88 |
| 54-7 | 19 | 170 |
| 54-8 | 21 | 1100 |
| 54-9 | 130 | 670 |
| 54-10 | 290 | 68 |
| 54-11 | 320 | 690 |
| 54-12 | 640 | 3600 |
| 54-13 | 170 | 480 |
| 54-14 | 33 | 1200 |
| 54-15 | 440 | 6300 |
| 54-16 | 22 | 520 |
| 54-17 | 21 | 370 |

(continued)

| Reference Example No. | $IC_{50}$ value [nM] | |
|---|---|---|
| | Human CYP4F2 | Human CYP4A11 |
| 54-18 | 23 | 130 |
| 54-19 | 15 | 390 |
| 54-20 | 6.5 | 1600 |
| 54-21 | 9.3 | 650 |

Reference Example 56-1

6-[4-(Difluoromethyl)-2-(oxan-2-yl)pyrazol-3-yl]pyridin-3-ol

[Formula 177]

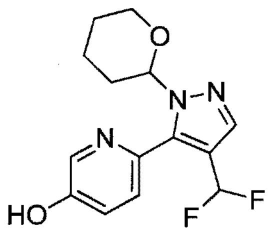

(1) N-bromosuccinimide (557 mg) was added to a solution of the compound (700 mg) obtained in Reference Example 1-1-(2) in chloroform (10 mL) at room temperature, and the mixture was stirred for 1 hour. The reaction solution was poured into an aqueous solution of saturated sodium hydrogen carbonate and extracted with chloroform. The organic layer was separated by a phase separator and concentrated under reduced pressure. The residue was purified by silica gel column chromatography (n-hexane/ethyl acetate = 7:3) to give 2-[4-bromo-2-(oxan-2-yl)pyrazol-3-yl]-5-phenylmethoxypyridine (710 mg) as a light yellow oily substance.

(2) n-Butyl lithium (1.60 mol/L n-hexane solution, 1.18 mL) was added dropwise to a solution of the compound (710 mg) obtained in the above described (1) in tetrahydrofuran (10 mL) under a nitrogen atmosphere at -78°C and the mixture was stirred for 45 minutes. Then, N,N-dimethylformamide (0.15 mL) was added dropwise to the reaction solution. After the dropwise addition, the temperature of the solution was gradually increased to room temperature and the solution was stirred for 1 hour. Water was added to the reaction solution, and the mixture was extracted with ethyl acetate. The organic layer was separated by a phase separator and concentrated under reduced pressure. The residue was purified by silica gel column chromatography (n-hexane/ethyl acetate = 4:1 to 1:1) to give a mixture (630 mg) containing 1-(oxan-2-yl)-5-(5-phenylmethoxypyridin-2-yl)pyrazole-4-carbaldehyde as a light yellow oily substance.

(3) Bis(2-methoxyethyl)aminosulfur trifluoride (491 μL) and ethanol (7 μL) were added to a solution of the compound (622 mg) obtained in the above described (2) in chloroform (10 mL), and the mixture was stirred at room temperature for 1 hour, and then at 50°C for 3 hours. The reaction solution was poured into an aqueous solution of saturated sodium hydrogen carbonate and extracted with chloroform. The organic layer was separated by a phase separator and concentrated under reduced pressure. The residue was purified by silica gel column chromatography (n-hexane/ethyl acetate = 9:1 to 13:7) to give 2-[4-(difluoromethyl)-2-(oxan-2-yl)pyrazol-3-yl]-5-phenylmethoxypyridine (196 mg) as a light yellow oily substance.

(4) The compound (196 mg) obtained in the above described (3) was used to perform the synthesis process according to the method described in Reference Example 1-1-(3) thereby giving the title compound (149 mg) as a colorless amorphous substance.

$^1$H NMR (400 MHz, CHLOROFORM-d) δ ppm 1.50 - 1.80 (m, 3 H) 1.89 - 2.15 (m, 2 H) 2.44 - 2.55 (m, 1 H) 3.47 - 3.58 (m, 1 H) 4.00 - 4.09 (m, 1 H) 5.41 - 5.52 (m, 1 H) 6.50 - 6.83 (m, 1 H) 7.28 - 7.33 (m, 1 H) 7.49 - 7.55 (m, 1 H) 7.83 (s, 1 H) 8.36 - 8.42 (m, 1 H). MS ESI/APCI Multi posi: 296 $[M+H]^+$.

MS ESI/APCI Multi nega: 294 $[M-H]^-$.

Reference Example 57-1

6-(Triazol-1-yl)pyridin-3-ol

[Formula 178]

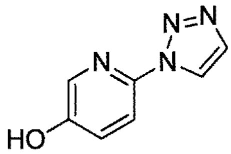

(1) The compound (501 mg) obtained in Reference Example 1-1-(1), 1,2,3-triazole (196 mg), copper iodide (I) (72.3 mg), N,N'-dimethylethylenediamine (0.102 mL), and potassium phosphate (1.20 g) were suspended in N,N-dimethylformamide (9 mL), and the mixture was deaerated, so that the air in the container was purged with nitrogen. The mixture was stirred under microwave irradiation at 120°C for 1 hour, cooled to room temperature, and then subjected to removal of insolubles by celite filtration and washing with ethyl acetate. The filtrate and washing solution were mixed, and the mixture was washed with an aqueous solution of saturated ammonium chloride, water, and then brine, and the organic layer was separated by a phase separator and then concentrated under reduced pressure. The obtained residue was purified by preparative HPLC and then preparative thin layer chromatography (n-hexane/ethyl acetate = 7:3) to give 5-phenylmethoxy-2-(triazol-1-yl)pyridine (39.7 mg) as a colorless powder.
(2) The compound (38.2 mg) obtained in the above described (1) was used to perform the synthesis process according to the method described in Reference Example 1-1-(3) thereby giving the title compound (24.0 mg) as a colorless powder.
$^1H$ NMR (400 MHz, ACETONE-$d_6$) $\delta$ ppm 7.54 (dd, J=8.8, 2.9 Hz, 1 H) 7.80 - 7.85 (m, 1 H) 8.01 (d, J=8.8 Hz, 1 H) 8.14 (d, J=2.9 Hz, 1 H) 8.56 - 8.61 (m, 1 H).
MS ESI/APCI Multi posi: 163 $[M+H]^+$.
MS ESI/APCI Multi nega: 161 $[M-H]^-$.

Reference Example 58-1

6-(1,2,4-Triazol-1-yl)pyridin-3-ol

[Formula 179]

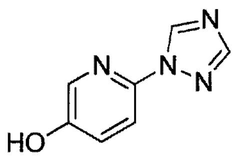

(1) The compound (201 mg) obtained in Reference Example 1-1-(1), 4H-1,2,4-triazole (63.1 mg), copper (II) acetate monohydrate (76.0 mg), and cesium carbonate (744 mg) were suspended in N,N-dimethylformamide (4 mL), and the mixture was deaerated, so that the air in the container was purged with nitrogen. The mixture was stirred under microwave irradiation at 120°C for 1 hour and then stirred in oil bath at 160°C for 7 hours. The mixture was cooled to room temperature, and then subjected to removal of insolubles by celite filtration and washing with ethyl acetate. An aqueous solution of saturated ammonium chloride was added to the mixture of filtrate and washing solution, the mixture was shaken, the organic layer was separated, and the aqueous layer was extracted with ethyl acetate. The

obtained organic layer was washed with water and then brine, and was separated by a phase separator, and concentrated under reduced pressure. The obtained residue was purified by silica gel column chromatography (n-hexane/ethyl acetate = 1:1 to ethyl acetate only) to give 5-phenylmethoxy-2-(1,2,4-triazol-1-yl)pyridine (129 mg) as a colorless powder.

(2) The compound (129 mg) obtained in the above described (1) was used to perform the synthesis process according to the method described in Reference Example 1-1-(3) thereby giving the title compound (59.6 mg) as a pale yellow powder.

$^1$H NMR (400 MHz, DMSO-$d_6$) δ ppm 7.40 - 7.47 (m, 1 H) 7.66 - 7.75 (m, 1 H) 8.01 - 8.08 (m, 1 H) 8.20 - 8.23 (m, 1 H) 9.12 - 9.21 (m, 1 H) 10.20 - 10.48 (m, 1 H).

MS ESI/APCI Multi posi: 163 $[M+H]^+$.

MS ESI/APCI Multi nega: 161 $[M-H]^-$.

Reference Example 59-1

6-(1,2,4-Triazol-1-yl)pyridin-3-ol

[Formula 180]

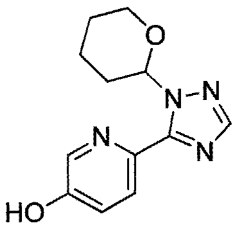

(1) 1-(Oxan-2-yl)-1,2,4-triazole (638 mg) was added to a suspension of the compound (1.00 g) obtained in Reference Example 1-1-(1) in toluene (5 mL) so that the interior of the container was purged with nitrogen. Palladium acetate (II) (42.5 mg), butyldi-1-adamantylphosphine (102 mg), and tert-butoxy sodium (728 mg) were added to the reaction solution, and the mixture was stirred at 110°C for 5 hours. After cooling to room temperature, the reaction solution was subjected to celite filtration, the residue was subjected to toluene washing, and the filtrate was concentrated under reduced pressure. The residue was purified by silica gel column chromatography (n-hexane/ethyl acetate = 7:3 to 1:1) to give 2-[2-(oxan-2-yl)-1,2,4-triazol-3-yl]-5-phenylmethoxypyridine (0.28 g) as a pale brown oily substance.

(2) The compound (0.28 mg) obtained in the above described (1) was used to perform the synthesis process according to the method described in Reference Example 1-1-(3) thereby giving the title compound (208 mg) as a light yellow powder.

$^1$H NMR (400 MHz, METHANOL-$d_4$) δ ppm 1.56 - 1.83 (m, 3 H) 1.96 - 2.15 (m, 2 H) 2.27 - 2.42 (m, 1 H) 3.59 - 3.76 (m, 1 H) 3.94 - 4.09 (m, 1 H) 6.54 - 6.61 (m, 1 H) 7.32 (dd, J=8.6, 2.7 Hz, 1 H) 7.91 (d, J=8.6 Hz, 1 H) 7.98 (s, 1 H) 8.27 (d, J=2.7 Hz, 1 H).

MS ESI/APCI Multi posi: 247 $[M+H]^+$.

MS ESI/APCI Multi nega: 245 $[M-H]^-$.

Reference Example 60-1

6-(1,2,4-Triazol-4-yl)pyridin-3-ol

[Formula 181]

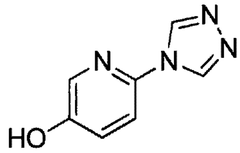

(1) 6-Nitropyridin-3-ol (499 mg) was used to perform the synthesis process according to the method described in Reference Example 1-1-(1) thereby giving 2-nitro-5-phenylmethoxypyridine (570 mg) as a yellow powder.
(2) Ammonium chloride (264 mg) was added to a suspension of the compound (570 mg) obtained in the above described (1) and iron powder (691 mg) in tetrahydrofuran (10 mL) and water (5 mL), the mixture was stirred while being heated to reflux for 1 hour, and the reaction mixture was concentrated. The obtained residue was purified by NH silica gel chromatography (n-hexane/ethyl acetate = 7:3 to 1:4) to give 5-phenylmethoxypyridin-2-amine (471 mg) as a dark green powder.
(3) Trimethylsilyl chloride (830 mg) was added to triethylamine (497 mL) under ice cooling. To the mixture, the compound (102 mg) obtained in the above described (2) and a suspension of 1,2-diformylhydrazine (112 mg) in pyridine (4 mL) were added, and the mixture was stirred while being heated to reflux for 9 hours. After left standing to cool, an aqueous solution of saturated sodium hydrogen carbonate and chloroform were added to the mixture, and the organic layer was separated by a phase separator and concentrated. The obtained residue was purified by silica gel column chromatography (ethyl acetate only, to ethyl acetate/methanol = 4:1) to give 5-phenylmethoxy-2-(1,2,4-triazol-4-yl)pyridine (99.5 mg) as a red powder.
(4) The compound (99.5 mg) obtained in the above described (3) was used to perform the synthesis process according to the method described in Reference Example 1-1-(3) thereby giving the title compound (48.0 mg) as a pale yellow powder.
$^1$H NMR (400 MHz, DMSO-$d_6$) δ ppm 7.42 (dd, J=8.8, 2.8 Hz, 1 H) 7.69 (d, J=8.8 Hz, 1 H) 8.06 (d, J=2.8 Hz, 1 H) 9.10 - 9.14 (m, 2 H) 10.21 - 10.48 (m, 1 H).
MS ESI/APCI Multi posi: 163 $[M+H]^+$.
MS ESI/APCI Multi nega: 161 $[M+Cl]^-$.

Reference Example 61-1

1-tert-Butyl-5-[5-{(3-methylsulfonylphenyl)methoxy}pyridin-2-yl]pyrazole-4-carboxylic acid

[Formula 182]

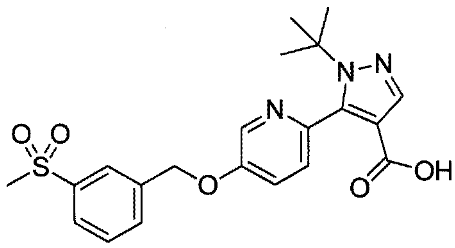

(1) Triethylamine (1.16 mL) and magnesium chloride (953 mg) were added to a suspension of potassium malonate monoethyl ester (1.42 g) in acetonitrile (8.34 mL), and the mixture was stirred at room temperature for 40 minutes, stirred at 50°C for 2 hours, and then cooled to room temperature. In another flask, under ice cooling, 1,1'-carbonyldiimidazole (811 mg) was added to a solution of 5-phenylmethoxypyridin-2-carboxylic acid (975 mg) in acetonitrile (8.34 mL), and the solution was stirred for 3 hours while gradually increasing its temperature to room temperature. The solution was then added to the aforementioned mixture and the resultant mixture was stirred at room temperature overnight. After the solvent was distilled off under reduced pressure, the residue was distributed into chloroform

and 1 mol/L hydrochloric acid. The organic layer was washed with an aqueous solution of saturated sodium hydrogen carbonate, 1 mol/L hydrochloric acid, and then brine, separated by a phase separator, and concentrated under reduced pressure. The obtained residue was purified by silica gel column chromatography (n-hexane only, to n-hexane/ethyl acetate = 1:1) to give ethyl 3-oxo-3-(5-phenylmethoxypyridin-2-yl)propanoate (1.18 g) as a light yellow oily substance.

(2) The compound (1.18 g) obtained in the above described (1) and N,N-dimethylformamide dimethyl acetal (616 μL) were mixed, and the mixture was heated to reflux for 2 hours. The mixture was then cooled to room temperature and then distributed into ethyl acetate and water. The organic layer was separated, and the aqueous layer was then extracted twice with ethyl acetate. The obtained organic layer was collected, washed with brine, separated by a phase separator, and concentrated under reduced pressure. The residue was dissolved in ethanol (12.8 mL), and tert-butylhydrazine hydrochloride (528 mg) was added to the solution which was then heated to reflux for 1 hour, cooled to room temperature, and distilled off under reduced pressure. The obtained residue was distributed into ethyl acetate and water, the organic layer was separated, and the aqueous layer was then extracted twice with ethyl acetate. The obtained organic layer was collected, washed with brine, separated by a phase separator, and concentrated under reduced pressure. The obtained residue was purified by silica gel column chromatography (n-hexane only, to n-hexane/ethyl acetate = 13:3) to give ethyl 1-tert-butyl-5-(5-phenylmethoxypyridin-2-yl)pyrazole-4-carboxylate (1.34 g) as a yellow oily substance.

(3) The compound (1.34 g) obtained in the above described (2) was used to perform the synthesis process according to the method described in Reference Example 1-1-(3), and the obtained crude product was then recrystallized from ethyl acetate/n-hexane thereby giving ethyl 1-tert-butyl-5-(5-hydroxypyridin-2-yl)pyrazole-4-carboxylate (764 mg) as a brown solid.

(4) The compound (668 mg) obtained in Reference Example 24-1 and potassium carbonate (386 mg) were added to a solution of the compound (550 mg) obtained in the above described (3) in N,N-dimethylformamide (9.32 mL), and the mixture was stirred overnight at room temperature. The mixture was diluted with water and extracted with ethyl acetate three times. The obtained organic layer was collected, washed with brine, separated by a phase separator, and concentrated under reduced pressure. The obtained residue was purified by silica gel column chromatography (n-hexane/ethyl acetate = 19:1 to 3:7) to give ethyl 1-tert-butyl-5-[5-{(3-methylsulfonylphenyl)methoxy}pyridin-2-yl]pyrazole-4-carboxylate (803 mg) as a colorless solid.

(5) The compound (803 mg) obtained in the above described (4) was used to perform the synthesis process according to the method described in Reference Example 9-1-(2) thereby giving the title compound (689 mg) as a colorless powder.

$^1$H NMR (400 MHz, DMSO-$d_6$) δ ppm 1.35 (s, 9 H) 3.24 (s, 3 H) 5.38 (s, 2 H) 7.44 - 7.51 (m, 1 H) 7.57 (dd, J=8.7, 2.9 Hz, 1 H) 7.68 - 7.77 (m, 1 H) 7.84 (s, 1 H) 7.86 - 7.90 (m, 1 H) 7.91 - 7.99 (m, 1 H) 8.08 (s, 1 H) 8.47 (d, J=2.9 Hz, 1 H) 11.97 (br s, 1 H).

MS ESI/APCI Multi posi: 430 $[M+H]^+$.

MS ESI/APCI Multi nega: 428 $[M-H]^-$.

Reference Example 62-1

1-[4-{(6-Bromopyridin-3-yl)oxymethyl}piperidin-1-yl]ethanone

[Formula 183]

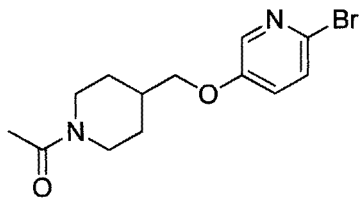

Triethylamine (1.16 mL) and methanesulfonyl chloride (805 mg) were added to a solution of the compound (1.00 g) obtained in Reference Example 25-1 in ethyl acetate (25 mL) under ice cooling, and the mixture was stirred at room temperature for 3 hours. The precipitated salt was filtered off and washed with ethyl acetate, and the filtrate and washing solution were mixed and concentrated. 2-Bromo-6-hydroxypyridine (1.01 g), potassium carbonate (1.75 g), and N,N-dimethylformamide (20 mL) were added to the obtained residue, and the mixture was stirred at 90°C for 10 hours. The reaction mixture was filtered, the residue was washed with ethyl acetate, water was added to a mixture of the filtrate

and the washing solution, and the mixture was subjected to extraction with ethyl acetate. The obtained organic layer was washed with an aqueous solution of saturated sodium hydrogen carbonate three times and then with brine once, and then dried over magnesium sulfate and then filtered, and the filtrate was concentrated. The obtained residue was purified by silica gel column chromatography (chloroform only, to chloroform/methanol = 9:1), and was solidified from ether to give the title compound (1.22 g) as a colorless powder.
$^1$H NMR (400 MHz, CHLOROFORM-d) δ ppm 1.22 - 1.40 (m, 2 H) 1.80 - 1.96 (m, 2 H) 2.00 - 2.13 (m, 4 H) 2.54 - 2.64 (m, 1 H) 3.05 - 3.15 (m, 1 H) 3.79 - 3.92 (m, 3 H) 4.66 - 4.75 (m, 1 H) 7.08 (dd, J=8.7, 3.1 Hz, 1 H) 7.37 (d, J=8.7 Hz, 1 H) 8.04 (d, J=3.1 Hz, 1 H). MS ESI/APCI Multi posi: 313 $[M+H]^+$.

Reference Example 63-1

2-Bromo-5-[(3-methylsulfonylphenyl)methoxy]pyridine

[Formula 184]

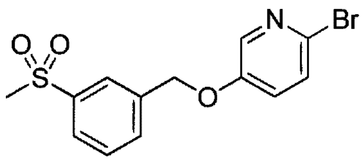

The compound (1.42 mg) obtained in the above described Reference Example 24-1 was used to perform the synthesis process according to the method described in Reference Example 1-1-(1) thereby giving the title compound (1.20 g) as a colorless powder.
$^1$H NMR (400 MHz, CHLOROFORM-d) δ ppm 3.08 (s, 3 H) 5.17 (s, 2 H) 7.19 (dd, J=8.7, 3.2 Hz, 1 H) 7.41 (d, J=8.7 Hz, 1 H) 7.60 - 7.66 (m, 1 H) 7.72 (d, J=7.8 Hz, 1 H) 7.95 (d, J=7.8 Hz, 1 H) 8.03 (s, 1 H) 8.14 (d, J=3.2 Hz, 1 H).
MS ESI/APCI Multi posi: 342 $[M+H]^+$.

Reference Example 64-1

2-Bromo-5-[2-(2-methylsulfonylphenyl)ethoxy]pyridine

[Formula 185]

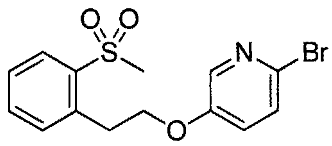

The compound (69.3 mg) obtained in the above described Reference Example 31-1 and 2-bromo-6-hydroxypyridine (50.2 mg) were used to perform the synthesis process according to the method described in Reference Example 14-1-(1) thereby giving the title compound (44.7 mg) as a colorless powder.
$^1$H NMR (400 MHz, CHLOROFORM-d) δ ppm 3.13 (s, 3 H) 3.52 (t, J=6.6 Hz, 2 H) 4.32 (t, J=6.6 Hz, 2 H) 7.07 - 7.12 (m, 1 H) 7.32 - 7.37 (m, 1 H) 7.45 - 7.52 (m, 2 H) 7.58 - 7.64 (m, 1 H) 8.03 - 8.11 (m, 2 H).
MS ESI/APCI Multi posi: 356 $[M+H]^+$.

Reference Example 65-1

4-Chloro-1-(oxan-2-yl)-3-(4,4,5,5-tetramethyl-1,3,2-dioxaborolan-2-yl)pyrazole

[Formula 186]

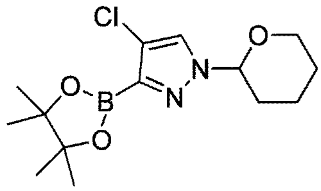

N-chlorosuccinimide (1.6 g) was added to a solution of 1-(2-tetrahydropyranyl)1H-pyrazole-5-boronic acid pinacol ester (3.4 g) in tetrahydrofuran (6 mL), the mixture was stirred at 70°C for 2 hours, and the reaction solution was concentrated under reduced pressure. A n-hexane/ethyl acetate mixed solution was added to the obtained residue and the mixture was filtered, and the filtrate was concentrated under reduced pressure. The obtained residue was purified by silica gel column chromatography (ethyl acetate/n-hexane = 99:1) to give a mixture (3.39 g) containing the title compound as a colorless oily substance.
MS ESI/APCI Multi posi: 313[M+H]$^+$.

Reference Example 66-1

4-[(4-Methoxyphenyl)methoxy]-1-(oxan-2-yl)-5-(4,4,5,5-tetramethyl-1,3,2-dioxaborolan-2-yl)pyrazole

[Formula 187]

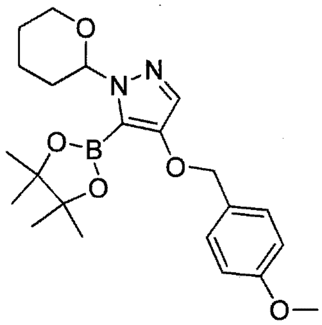

(1) 3,4-Dihydro-2H-pyran (4.00 mL) and p-toluenesulfonic acid monohydrate (421 mg) were added to a solution of 4-(4,4,5,5-tetramethyl-1,3,2-dioxaborolan-2-yl)-1H-pyrazole (4.29 g) in chloroform (44.2 mL), and the mixture was stirred at 50°C for 3.5 hours. The mixture was cooled to room temperature, diluted with chloroform, and then washed sequentially with an aqueous solution of saturated sodium hydrogen carbonate and brine. The organic layer was separated by a phase separator and concentrated under reduced pressure. The obtained residue was purified by column chromatography (n-hexane/ethyl acetate = 19:1 to ethyl acetate only) to give 1-(oxan-2-yl)-4-(4,4,5,5-tetramethyl-1,3,2-dioxaborolan-2-yl)pyrazole (5.49 g) as a light brown oily substance.

(2) An aqueous solution (17.8 mL) of 2 mol/L sodium hydroxide and a 30% hydrogen peroxide solution (3.56 mL) were added to a solution of the compound (5.49 g) obtained in the above described (1) in tetrahydrofuran (17.8 mL) under ice cooling, and the mixture was stirred at room temperature for 1.5 hours. One mol/L hydrochloric acid was added to this mixture to adjust the pH to 6, and the mixture was extracted with ethyl acetate three times. The obtained organic layer was collected, washed with brine, separated by a phase separator, and concentrated under reduced pressure. The residue was dissolved in N,N-dimethylformamide (35.7 mL), and potassium carbonate (3.21 g), sodium iodide (3.21 g), and p-methoxybenzyl chloride (2.89 mL) were added to the solution which was then stirred at room temperature for 2 hours. Potassium carbonate (3.21 g) and p-methoxybenzyl chloride (2.89 mL) were added to this mixture which was then stirred at room temperature for 1.5 hours, and then at 80°C for 1 hour. Potassium carbonate (3.21 g) and p-methoxybenzyl chloride (2.89 mL) were further added to the mixture which was then stirred at room temperature overnight. Potassium carbonate (3.21 g) and p-methoxybenzyl chloride (2.89 mL) were added again to this mixture which was then stirred at 80°C for 1 hour. After cooling to room temperature, the mixture was diluted with water and then extracted with a mixed solution (n-hexane/ethyl acetate = 1:1) three times. The obtained

organic layer was collected, washed with brine, separated by a phase separator, and concentrated under reduced pressure. The obtained residue was purified by column chromatography (n-hexane only, to n-hexane/ethyl acetate = 3:2), NH silica gel column chromatography (n-hexane only, to hexane/ethyl acetate = 3:2), and then NH silica gel column chromatography (n-hexane/ethyl acetate = 19:1 to 13:7) again to give 4-[(4-methoxyphenyl)methoxy]-1-(oxan-2-yl)pyrazole (1.34 g) as a colorless oily substance.

(3) Under an argon atmosphere and at -78°C, n-butyl lithium (1.60 mol/L n-hexane solution, 665 μL) was added dropwise to a solution of the compound (289 mg) obtained in the above described (2) in tetrahydrofuran (3.87 mL), and the mixture was stirred at the same temperature for 1 hour. Triisopropyl boronate (268 μL) was added to this mixture, and the mixture was stirred overnight while its temperature was slowly increased to room temperature. Pinacol (137 mg) and acetic acid (83.0 μL) were added to the mixture, the mixture was stirred at room temperature for 2 hours, and pinacol (137 mg) was further added to the mixture. The mixture was stirred at room temperature for 2 hours, acetic acid (83.0 μL) was added to this mixture, and the mixture was stirred at room temperature for 1.5 hours. The mixture was diluted with toluene, insolubles were filtered off, and the solvent was distilled off under reduced pressure. The residue was purified by column chromatography twice (first with chloroform only, to chloroform/ethyl acetate = 4:1, and second with chloroform/ethyl acetate = 97:3 to 9:1) to give the title compound (169 mg) as a colorless solid.

$^1$H NMR (400 MHz, CHLOROFORM-d) δ ppm 1.35 (s, 12 H) 1.50 - 1.58 (m, 1 H) 1.60 - 1.74 (m, 2 H) 1.90 - 1.99 (m, 1 H) 1.99 - 2.13 (m, 1 H) 2.33 - 2.47 (m, 1 H) 3.59 - 3.68 (m, 1 H) 3.80 (s, 3 H) 3.96 - 4.04 (m, 1 H) 4.92 - 5.02 (m, 2 H) 5.73 (dd, J=10.0, 2.3 Hz, 1 H) 6.87 (d, J=8.6 Hz, 2 H) 7.27 - 7.31 (m, 1 H) 7.35 (d, J=8.6 Hz, 2 H).

MS ESI/APCI Multi posi: 415 $[M+H]^+$.

Reference Example 66-2

4-Methoxy-1-(oxan-2-yl)-5-(4,4,5,5-tetramethyl-1,3,2-dioxaborolan-2-yl)pyrazole

[Formula 188]

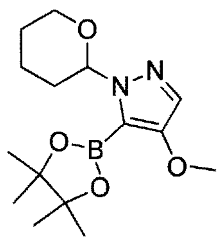

(1) 4-Methoxy-lH-pyrazole (579 mg) was used to perform the synthesis process according to the method described in Reference Example 66-1-(1) thereby giving 4-methoxy-1-(oxan-2-yl)pyrazole (1.14 g) as a light yellow oily substance.

(2) The compound (1.14 g) obtained in the above described (1) was used to perform the synthesis process according to the method described in Reference Example 66-1-(3) thereby giving the title compound (1.57 g) as a light yellow oily substance.

$^1$H NMR (400 MHz, CHLOROFORM-d) δ ppm 1.35 (s, 12 H) 1.50 - 1.58 (m, 1 H) 1.62 - 1.78 (m, 2 H) 1.89 - 2.15 (m, 2 H) 2.34 - 2.47 (m, 1 H) 3.64 (td, J=11.4, 2.4 Hz, 1 H) 3.82 (s, 3 H) 3.97 - 4.05 (m, 1 H) 5.76 (dd, J=10.1, 2.4 Hz, 1 H) 7.33 (s, 1 H).

MS ESI/APCI Multi posi: 309 $[M+H]^+$.

Reference Example 67-1

Ethyl N-[{3-(hydroxymethyl)phenyl}-methyl-oxo-λ^{6}-sulfanylidene]carbamate

[Formula 189]

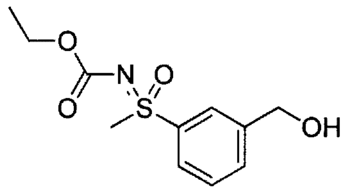

(1) Under an argon atmosphere, an aqueous solution (6.77 mL) of sodium periodate (421 mg) was added dropwise to a solution of 3-methylsulfanylbenzoic acid (569 mg) in methanol (6.77 mL) under ice cooling. This mixture was stirred at the same temperature for 2 hours, and then at room temperature for 2 hours, and the precipitate was filtered off and washed with a small amount of methanol. The filtrate and washing solution were combined and concentrated under reduced pressure, and brine (10 mL) was added to the residue and the mixture was extracted with a mixed solution (chloroform/methanol = 9:1) three times. The obtained organic layer was collected, dehydrated by a phase separator, and concentrated under reduced pressure. 1,1'-Carbonyldiimidazole (594 mg) was added to a suspension of the obtained residue in tetrahydrofuran (13.3 mL) and the mixture was stirred at room temperature for 30 minutes. Methanol (1.1 mL) was added to this mixture, the container was heated with a drier to reflux for a short time and left standing to cool, and the mixture was then stirred at room temperature for 30 minutes. An aqueous solution of saturated sodium hydrogen carbonate was added to the mixture and the mixture was extracted with ethyl acetate three times. The obtained organic layer was collected, washed sequentially with brine, 1 mol/L hydrochloric acid, and then brine, separated by a phase separator, and concentrated under reduced pressure. The residue was purified by column chromatography (n-hexane/ethyl acetate = 4:1 to ethyl acetate only) to give methyl 3-methylsulfinylbenzoate (465 mg) as a light yellow oily substance.

(2) The compound (172 mg) obtained in the above described (1), 2,2,2-trifluoroacetamide (186 mg), magnesium oxide (99.7 mg), rhodium (II) acetate dimer (9.11 mg), and iodobenzene diacetate (398 mg) were mixed in a three-neck flask, and evacuation and nitrogen introduction were performed three times to fill the container with nitrogen. A syringe was used to add chloroform (4.12 mL), and the suspension was stirred at room temperature overnight. 2,2,2-Trifluoroacetamide (186 mg), magnesium oxide (99.7 mg), rhodium (II) acetate dimer (9.11 mg), and iodobenzene diacetate (398 mg) were added to the mixture, the mixture was stirred at room temperature for 2 hours, and the precipitate was filtered off and washed with chloroform. The filtrate and washing solution were combined and concentrated under reduced pressure, and the obtained residue was dissolved in methanol (6.59 mL). Potassium carbonate (342 mg) was added to this solution, the solution was stirred at room temperature for 3 hours, the solid was filtered off and washed with methanol and ethyl acetate, and the filtrate and washing solution were combined and the mixture was concentrated under reduced pressure. Dilute hydrochloric acid was added to the residue and the mixture was extracted with a mixed solution (n-hexane/ethyl acetate = 7:3) three times. The aqueous layer was adjusted to pH9 with sodium carbonate, and then extracted with chloroform three times. The obtained organic layer was collected, washed with brine, separated by a phase separator, and concentrated under reduced pressure to give a mixture (137 mg) containing methyl 3-(methylsulfonimidoyl)benzoate.

(3) Ethyl chloroformate (296 μL) was added dropwise to a solution of the mixture (136 mg) obtained in the above described (2) in pyridine (6.19 mL), and the mixture was then stirred at room temperature for 2 hours. After the solvent was distilled off under reduced pressure, water was added to the residue, and the mixture was extracted with ethyl acetate three times. The obtained organic layer was collected, dehydrated by a phase separator, and concentrated under reduced pressure. The residue was purified by column chromatography (n-hexane/ethyl acetate = 9:1 to 3:7) to give methyl 3-(N-ethoxycarbonyl-S-methylsulfonimidoyl)benzoate (169 mg) as a colorless oily substance.

(4) The compound (169 mg) obtained in the above described (3) was used to perform the synthesis process according to the method described in Reference Example 9-1-(2) thereby giving carboxylic acid intermediate (94.9 mg). This intermediate was used to perform the synthesis process according to the method described in Reference Example 32-1 thereby giving a mixture (91.5 mg) containing the title compound.

$^1$H NMR (400 MHz, CHLOROFORM-d) δ ppm 1.21 - 1.27 (m, 3 H) 1.89 (t, J=5.9 Hz, 1 H) 3.32 (s, 3 H) 4.05 - 4.16 (m, 2 H) 4.82 (d, J=5.9 Hz, 2 H) 7.57 - 7.63 (m, 1 H) 7.69 (d, J=7.5 Hz, 1 H) 7.91 (d, J=7.8 Hz, 1 H) 8.01 (s, 1 H).

MS ESI/APCI Multi posi: 258 $[M+H]^+$.

Reference Example 68-1

(5-Methylsulfonylpyridin-3-yl)methanol

[Formula 190]

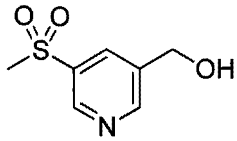

(1) 2-Bromo-4-hydroxymethylpyridine (540 mg), copper iodide (I) (110 mg), L-proline (123 mg), sodium hydroxide (43 mg), and sodium methanesulfinate (543 mg) were suspended in dimethylsulfoxide (4.5 mL), and the suspension was stirred under microwave irradiation at 160°C for 30 minutes. The mixture was cooled, water and ethyl acetate were added to the mixture to filter off insolubles, and the filtrate was extracted with ethyl acetate. The obtained organic layer was washed with brine, dried over anhydrous magnesium sulfate, and, after the drying agent was filtered off, concentrated under reduced pressure. The obtained residue was purified by column chromatography (chloroform/methanol = 99:1 to 22:3) to give the title compound (193 mg) as a light brown solid.
$^1$H NMR (400 MHz, CHLOROFORM-d) $\delta$ ppm 2.05 - 2.16 (m, 1 H) 3.12 (s, 3 H) 4.84 - 4.93 (m, 2 H) 8.24 - 8.31 (m, 1 H) 8.83 - 8.91 (m, 1 H) 9.04 - 9.11 (m, 1 H).
MS ESI/APCI Multi posi: 188 $[M+H]^+$.

Reference Example 69-1

2-(1,1-Dioxothian-4-yl)ethanol

[Formula 191]

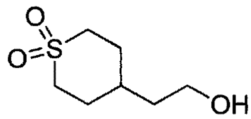

(1) Tetrahydrothiopyran-4-one (500 mg) was used to perform the synthesis process according to the method described in Reference Example 20-1-(1) thereby giving ethyl 2-(thian-4-ylidene)acetate (800 mg) as a colorless oily substance.
(2) The compound (800 mg) obtained in the above described (1) was used to perform the synthesis process according to the method described in Reference Example 42-1-(3) thereby giving ethyl 2-(thian-4-yl)acetate (621 mg) as a colorless oily substance.
(3) The compound (621 mg) obtained in the above described (2) was used to perform the synthesis process according to the method described in Reference Example 33-1-(3) thereby giving ethyl 2-(1,1-dioxothian-4-yl)acetate (680 mg) as a light brown solid.
(4) The compound (673 mg) obtained in the above described (3) was used to perform the synthesis process according to the method described in Reference Example 40-1-(1) thereby giving the title compound (539 mg) as a light brown oily substance.
$^1$H NMR (400 MHz, CHLOROFORM-d) $\delta$ ppm 1.54 - 1.63 (m, 2 H) 1.73 - 1.95 (m, 3 H) 2.09 - 2.18 (m, 2 H) 2.93 - 3.09 (m, 4 H) 3.70 - 3.76 (m, 2 H).
MS ESI/APCI Multi posi: 201 $[M+Na]^+$.

The compound of the following Reference Example 69-2 was synthesized using a commercially available

tetrahydrothiopyran-3-one, according to the method described in Reference Examples 69-1-(1) to (4). The structure, NMR data and MS data are shown in Table 21-1.

[Table 21-1]

| Reference Example No. | Structure | Analytical Data |
|---|---|---|
| 69-2 | 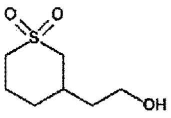 | $^1H$ NMR (400 MHz, CHLOROFORM-d) δ ppm 1.13 - 1.29 (m, 1 H) 1.53 - 1.71 (m, 2 H) 1.83 - 1.98 (m, 1 H) 2.02 - 2.17 (m, 2 H) 2.30 - 2.41 (m, 1 H) 2.67 - 2.75 (m, 1 H) 2.82 - 2.91 (m, 1 H) 3.01 - 3.18 (m, 2 H) 3.69 - 3.78 (m, 2 H).<br>MS ESI/APCI Multi posi: 201[M+Na]$^+$. |

Reference Example 70-1

2-(3,5-Dimethyl-1H-pyrazol-4-yl)-5-[(3-methylsulfonylphenyl)methoxy]pyridine

[Formula 192]

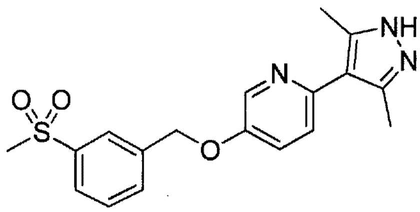

(1) Commercially available 3,5-dimethyl-4-(4,4,5,5-tetramethyl-1,3,2-dioxaborolan-2-yl)-1H-pyrazole (50.6 mg) was used to perform the synthesis process according to the method described in Example 41-1, which will be described later, thereby giving the title compound (17.9 mg) as a colorless solid.
$^1H$ NMR (400 MHz, CHLOROFORM-d) δ ppm 2.41 (s, 6 H) 3.09 (s, 3 H) 5.22 (s, 2 H) 7.27 - 7.35 (m, 2 H) 7.61 - 7.66 (m, 1 H) 7.75 - 7.78 (m, 1 H) 7.93 - 7.96 (m, 1 H) 8.05 - 8.08 (m, 1 H) 8.43 - 8.45 (m, 1 H).
MS ESI/APCI Multi posi: 358 [M+H]$^+$.

Reference Example 70-2

2-[4-(Difluoromethyl)-1H-pyrazol-5-yl]-5-[(5-methylsulfonylpyridin-3-yl)methoxy]pyridine

[Formula 193]

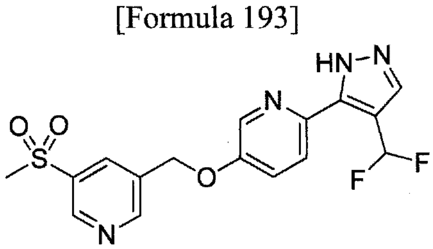

(1) The compound (35 mg) obtained in Reference Example 56-1 and the compound (22 mg) obtained in Reference Example 68-1 were used to perform the synthesis process according to the method described in Examples 46-1-(1) to (3), which will be described later, thereby giving the title compound (47 mg) as a light brown oily substance.

$^1$H NMR (400 MHz, DMSO-$d_6$) δ ppm 3.37 (s, 3 H) 5.43 (s, 2 H) 7.34 - 8.24 (m, 3 H) 8.39 - 8.56 (m, 2 H) 8.91 - 9.28 (m, 2 H).
MS ESI/APCI Multi posi: 380 [M+H]$^+$.

Reference Example 70-3

Azetidin-1-yl-(5-[5-1(3-methylsulfonylphenyl)methoxylpyridin-2-yl]-1H-pyrazol-4-yl)methanone

[Formula 194]

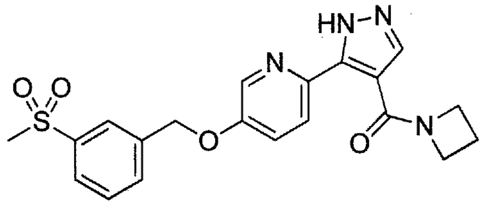

(1) The compound (74.2 mg) obtained in Reference Example 61-1 and commercially available azetidine (11.8 mg) were used to perform the synthesis process according to the method described in Examples 47-1-(1) and (2), which will be described later, thereby giving the title compound (9.12 mg) as a colorless high-viscosity substance.
$^1$H NMR (400 MHz, METHANOL-$d_4$) δ ppm 2.22 - 2.34 (m, 2 H) 3.14 (s, 3 H) 4.02 - 4.19 (m, 4 H) 5.35 (s, 2 H) 7.56 (dd, J=8.8, 2.8 Hz, 1 H) 7.65 - 7.72 (m, 1 H) 7.82 - 7.92 (m, 3 H) 7.92 - 7.98 (m, 1 H) 8.10 (s, 1 H) 8.42 (d, J=2.8 Hz, 1 H).
MS ESI/APCI Multi posi: 413 [M+H]$^+$.

Example 1-1

1-[4-({[6-(1H-Pyrazol-5-yl)pyridin-3-yl]oxylmethyl)piperidin-1-yl]ethan-1-one

[Formula 195]

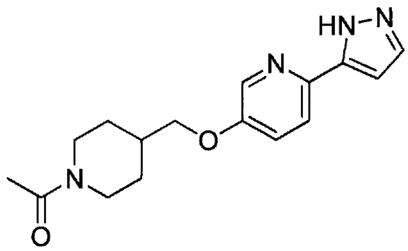

(1) N,N-Diisopropylethylamine (70 μL) and acetyl chloride (17 μL) were added to a solution of the compound (70 mg) obtained in Reference Example 2-1 in tetrahydrofuran (2 mL) under ice cooling, and the mixture was stirred at room temperature for 1 hour. After the reaction solution was concentrated, the obtained residue was purified by silica gel column chromatography (n-hexane/ethyl acetate = 7:3 to ethyl acetate only, subsequently chloroform/methanol = 19:1) to give 1-{4-[({6-[1-(oxan-2-yl)-1H-pyrazol-5-yl]pyridin-3-yl}oxy)methyl]piperidin-1-yl}ethan-1-one (80 mg) as a colorless oily substance.
(2) Water (2 mL) and trifluoroacetic acid (1 mL) were added to a solution of the compound (80 mg) obtained in the above described (1) in methanol (4 mL), and the mixture was stirred at 60°C for 3 hours. An aqueous solution of saturated sodium hydrogen carbonate was added to the reaction solution, and the mixture was extracted with chloroform. The organic layer was separated by a phase separator, and the solvent was distilled off under reduced pressure. After the obtained residue was purified by NH silica gel column chromatography (n-hexane/ethyl acetate

= 9:1 to 1:1, subsequently chloroform/methanol = 19:1 to 9:1), the residue was powdered from diethyl ether/n-hexane to give the title compound (37 mg) as a colorless powder.
$^1$H NMR (400 MHz, DMSO-$d_6$) δ ppm 1.06 - 1.33 (m, 2 H) 1.73 - 1.86 (m, 2 H) 1.97 - 2.08 (m, 4 H) 2.52 - 2.60 (m, 1 H) 2.99 - 3.11 (m, 1 H) 3.79 - 3.91 (m, 1 H) 3.95 (d, J=6.4 Hz, 2 H) 4.36 - 4.46 (m, 1 H) 6.73 (d, J=2.0 Hz, 1 H) 7.40 - 7.51 (m, 1 H) 7.58 - 7.95 (m, 2 H) 8.24 - 8.33 (m, 1 H) 12.78 - 13.44 (m, 1 H).
MS ESI/APCI Multi posi: 301 $[M+H]^+$.
The title compound can also be synthesized by a method shown below.
(3) A solution of 4 mol/L of hydrogen chloride in ethyl acetate (849 mL) was added dropwise to a solution of the compound (435.43 g) obtained in the above described (1) in methanol (870 mL) under ice cooling, and the mixture was stirred with immersion in a water bath for 2 hours. The precipitated solid was collected by filtration to give the title compound dihydrochloride (397.74 g) as a pale brown powder.
$^1$H NMR (400 MHz, DMSO-$d_6$) δ ppm 1.08 - 1.34 (m, 2 H) 1.74 - 1.86 (m, 2 H) 1.97 - 2.12 (m, 4 H) 2.52 - 2.61 (m, 1 H) 3.01 - 3.11 (m, 1 H) 3.80 - 3.91 (m, 1 H) 4.07 (d, J=6.2 Hz, 2 H) 4.36 - 4.46 (m, 1 H) 7.05 - 7.14 (m, 1 H) 7.87 - 7.97 (m, 2 H) 8.15 - 8.22 (m, 1 H) 8.33 - 8.37 (m, 1 H).
(4) The compound (395.92 g) obtained in the above described (3) was dissolved in water (1.99 L), an aqueous solution (273 mL) of 8 mol/L sodium hydroxide was added thereto under ice cooling, and the mixture was stirred overnight with immersion in a water bath. The precipitated solid was collected by filtration to give the title compound (349.11 g) as a pale brown powder.
(5) Ethanol (6.69 L) was added to the compound (318.58 g) obtained in the above described (4), and the mixture was heated to reflux at 110°C and stirred until it was dissolved. The mixture was cooled to room temperature, and then immersed in a water bath and stirred overnight. The precipitated solid was collected by filtration to give the title compound (250.06 g) as a colorless powder (melting point: 197°C).

Purification by recrystallization in the above described (5) can also be carried out using methanol instead of ethanol.

The compounds of the following Examples 1-2 to 1-26 were synthesized using the compound obtained in Reference Example 2-1 to 2-3, 2-10, 3-1, or 7-1 to 7-4, and a corresponding acid chloride according to the method described in Examples 1-1-(1) to (2). The structures, NMR data, MS data, and the like of these compounds are shown in Tables 22-1 to 22-4.

[Table 22-1]

| Example No. | Structure | Analytical Data |
|---|---|---|
| 1-2 | 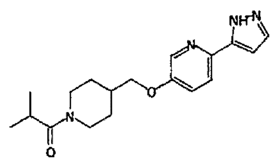 | $^1$H NMR (600 MHz, CHLOROFORM-d) δ ppm 1.11 - 1.17 (m, 6 H) 1.25 - 1.37 (m, 2 H) 1.88 (d, J=12.0 Hz, 1 H) 1.98 (d, J=12.0 Hz, 1 H) 2.06 - 2.15 (m, 1 H) 2.60 (t, J=11. 8 Hz, 1 H) 2.83 (spt, J=6.8 Hz, 1 H) 3.09 (t, J=12.4 Hz, 1 H) 3. 84 - 3.96 (m, 2 H) 4.02 (d, J=13.2 Hz, 1 H) 4.74 (d, J=12.0 Hz, 1 H) 6.69 (br s, 1 H) 7.23 (dd, J=8.7, 2.9 Hz, 1 H) 7.60 - 7.71 (m, 2 H) 8.28 (d, J=2.5 Hz, 1 H).<br>MS ESI/APCI Multil posi: 329$[M+H]^+$. |
| 1-3 | 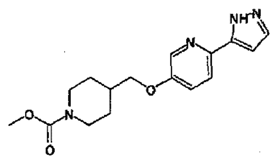 | $^1$H NMR (600 MHz, CHLOROFORM-d) δ ppm 1.27 - 1.38 (m, 2 H) 1.86 (d, J=13.2 Hz, 2 H) 1.97 - 2.06 (m, 1 H) 2.77 - 2.88 (m, 2 H) 3.71 (s, 3 H) 3.89 (d, J=6.2 Hz, 2 H) 4.10 - 4.37 (m, 2 H) 6.68 (br s, 1 H) 7.23 (dd, J=8.7, 2.9 Hz, 1 H) 7.61 - 7.67 (m, 2 H) 8.27 (d, J=2.9 Hz, 1 H).<br>MS ESI/APCI Multi posi: 317$[M+H]^+$. |
| 1-4 | 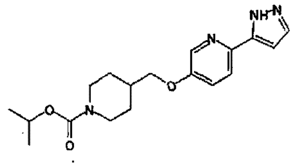 | $^1$H NMR (600 MHz, CHLOROFORM-d) δ ppm 1.25 (d, J=6.2 Hz, 6 H) 1.27 - 1.37 (m, 2 H) 1.85 (d, J=12.4 Hz, 2 H) 1.97 - 2.06 (m, 1 H) 2.79 (t, J=12.4 Hz, 2 H) 3.89 (d, J=6.6 Hz, 2 H) 4.14 - 4. 32 (m, 2 H) 4.93 (spt, J=6.3 Hz, 1 H) 6.68 (br s, 1 H) 7.23 (dd, J=8.7, 2.9 Hz, 1 H) 7.60 - 7.68 (m, 2 H) 8.28 (d. J=2.5 Hz, 1 H).<br>MS ESI/APCI Multi posi: 345$[M+H]^+$. |

(continued)

| Example No. | Structure | Analytical Data |
|---|---|---|
| 1-5 | 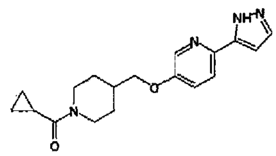 | $^1$H NMR (600 MHz, CHLOROFORM-d) δ ppm 0.69 - 0.79 (m, 2 H) 0.93 - 1.02 (m, 2 H) 1.14 - 2.03 (m, 5 H) 2.04 - 2.16 (m, 1 H) 2.57 - 2.70 (m, 1 H) 3.07 - 3.21 (m, 1 H) 3.81 - 3.97 (m, 2 H) 4.24 - 4.34 (m, 1 H) 4.62 - 4.74 (m, 1 H) 6.68 (br s, 1 H) 7.18 - 7.27 (m. 1 H) 7.58 - 7.69 (m, 2 H) 8.24 - 8.30 (m, 1 H).<br>MS ESI/APCI Multi posi: 327[M+H]$^+$. |
| 1-6 | 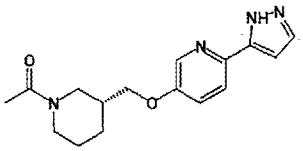 | $^1$H NMR (600 MHz, CHLOROFORM-d) δ ppm 1.39 - 2.17 (m, 8 H) 2.62 - 2.71 (m, 0.5 H) 2.91 - 3.06 (m, 0.5 H) 3.06 - 3.21 (m, 1 H) 3.71 - 3.81 (m, 0.5 H) 3.86 - 3.99 (m, 2.5 H) 4.09 - 4.24 (m, 0.5 H) 4.53 - 4.64 (m, 0.5 H) 6.67 - 6.77 (m, 1 H) 7.23 - 7.30 (m, 1 H) 7.58 - 7.75 (m, 2 H) 8.25 - 8.36 (m, 1 H).<br>MS ESI/APCI Multi posi: 301[M+H]$^+$. |
| 1-7 | 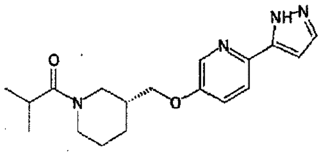 | $^1$H NMR (600 MHz, CHLOROFORM-d) δ ppm 1.00 - 2.23 (m, 11 H) 2.60 - 2.74 (m, 0.5 H) 2.75 - 2.97 (m, 1.5 H) 2.99 - 3.17 (m, 1 H) 3.77 - 4.12 (m, 3 H) 4.24 - 4.42 (m, 0.5 H) 4.47 - 4.66 (m, 0.5 H) 6.64 - 6.83 (m. 1 H) 7.21 - 7.33 (m, 1 H) 7.60 - 7.72 (m, 2 H) 8.24 - 8.37 (m, 1 H).<br>MS ESI/APCI Multi posi: 329[M+H]$^+$. |
| 1-8 | 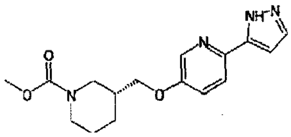 | $^1$H NMR (600 MHz, CHLOROFORM-d) δ ppm 1.32 - 2.22 (m, 5 H) 2. 77 - 2. 96 (m, 2 H) 3. 70 (s, 3 H) 3. 86 - 4.04 (m, 3 H) 4.05 - 4.37 (m, 1 H) 6.79 (d, J=2.1 Hz, 1 H) 7.30 (dd, J=8.5, 2.7 Hz, 1 H) 7.64 (d, J=2.1 Hz, 1 H) 7.70 (br d, J=8.5 Hz, 1 H) 8.29 (d, J=2.7 Hz, 1 H).<br>MS ESI/APCI Multi posi: 317[M+H]$^+$. |

[Table 22-2]

| Example No. | Structure | Analytical Data |
|---|---|---|
| 1-9 | 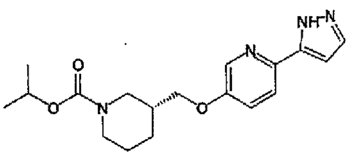 | $^1$H NMR (600 MHz, CHLOROFORM-d) δ ppm 1.19 - 1.32 (m, 6 H) 1.34 - 1.50 (m, 1 H) 1.61 - 1.66 (m, 1 H) 1.64 - 1.80 (m, 1 H) 1.89 - 1.98 (m, 1 H) 2.02 - 2.14 (m, 1 H) 2.65 - 3.09 (m, 2.5 H) 3.87 - 3.98 (m, 3 H) 4.13 - 4.27 (m, 0.5 H) 4.87 - 4.96 (m, 1 H) 6.65 - 6.77 (m, 1 H) 7.23 - 7.28 (m, 1 H) 7.59 - 7.70 (m, 2 H) 8.25 - 8.32 (m, 1 H).<br>MS ESI/APCI Multi posi: 345[M+H]$^+$. |
| 1-10 | 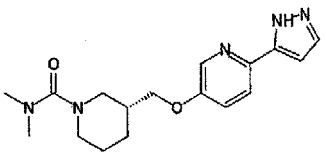 | $^1$H NMR (600 MHz, CHLOROFORM-d) δ ppm 1.30 - 1.42 (m, 1 H) 1.56 - 1.64 (m, 1 H) 1.71 - 1.80 (m, 1 H) 1.89 - 1.95 (m, 1 H) 2.09 - 2.17 (m, 1 H) 2.69 - 2.77 (m, 1 H) 2.82 - 2.87 (m, 7 H) 3.49 - 3.61 (m, 1 H) 3.66 - 3.83 (m, 1 H) 3.88 - 3.99 (m. 2 H) 6.77 (d, J=1. 7 Hz, 1 H) 7.29 (dd, J=8.5, 2.7 Hz, 1 H) 7.64 (d, J=1. 7 Hz, 1 H) 7.70 (br d, J=8.5 Hz, 1 H) 8.28 (d, J=2.7 Hz, 1 H).<br>MS ESI/APCI Multi posi: 330[M+H]$^+$. |
| 1-11 | 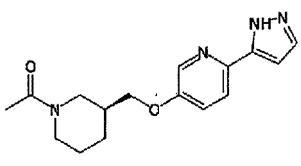 | $^1$H NMR (600 MHz, CHLOROFORM-d) δ ppm 1.41 - 1.62 (m, 2 H) 1.70 - 1.85 (m, 1 H) 1.89 - 2.15 (m, 5 H) 2.67 (dd, J=12.8, 10. 3 Hz, 0.5 H) 2.97 - 3.03 (m, 0.5 H) 3.08 - 3.19 (m. 1 H) 3.72 - 3.79 (m, 0.5 H) 3.84 - 4.00 (m, 2.5 H) 4.16 - 4.22 (m, 0.5 H) 4.56 - 4.62 (m, 0.5 H) 6.73 (br s, 1 H) 7.25 - 7.29 (m, 1 H) 7.62 - 7.72 (m, 2 H) 8.29 (dd, J=7.6, 2.7 Hz, 1 H).<br>MS ESI/APCI Multi posi: 301[M+H]$^+$. |

(continued)

| Example No. | Structure | Analytical Data |
|---|---|---|
| 1-12 | 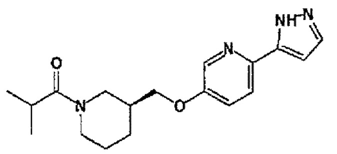 | $^1$H NMR (600 MHz, CHLOROFORM-d) δ ppm 1.05 - 1.18 (m, 6 H) 1.39 - 1.62 (m, 2 H) 1.70 - 1.87 (m, 1 H) 1.89 - 2. 13 (m, 2 H) 2.63 - 2.73 (m, 0.5 H) 2.78 - 2.94 (m, 1.5 H) 3.05 - 3.17 (m, 1 H) 3.81 - 4.07 (m, 3 H) 4.34 (br d, J=12. 8 Hz, 0.5 H) 4.61 (br d, J=14. 0 Hz, 0.5 H) 6.72 (br s, 1 H) 7. 22 - 7.31 (m, 1 H) 7.59 - 7.74 (m, 2 H) 8.29 (br d, J=8.7 Hz, 1 H).<br>MS ESI/APCI Multi posi: 329[M+H]$^+$. |
| 1-13 | 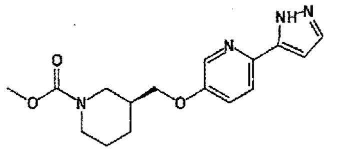 | $^1$H NMR (500 MHz, CHLOROFORM-d, 55°C) δ ppm 1.36 - 1. 46 (m, 1 H) 1.48 - 1.60 (m, 1 H) 1.68 - 1.77 (m, 1 H) 1.88 - 1.95 (m, 1 H) 2.01 - 2.11 (m, 1 H) 2.81 - 2.91 (m, 1 H) 2.95 (ddd, J=13.5, 10.7, 3.3 Hz, 1 H) 3.69 (s, 3 H) 3.87 - 3.98 (m, 3 H) 4.07 - 4.20 (m, 1 H) 6.70 (s, 1 H) 7.22 - 7.27 (m. 1 H) 7.59 - 7.68 (m, 2 H) 8.28 (d, J=2.7 Hz, 1 H).<br>MS ESI/APCI Multi posi: 317[M+H]$^+$. |
| 1-14 | 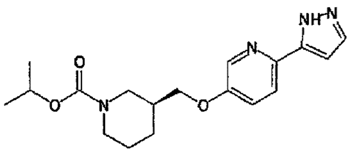 | $^1$H NMR (500 MHz, CHLOROFORM-d, 55°C) δ ppm 1.19 - 1.27 (m, 6 H) 1.36 - 1.47 (m, 1 H) 1.47 - 1.59 (m, 1 H) 1.67 - 1.75 (m, 1 H) 1.87 - 1.96 (m, 1 H) 2.00 - 2.10 (m, 1 H) 2.82 - 3.02 (m, 2 H) 3.87 - 3.97 (m, 3 H) 4.03 - 4.16 (m, 1 H) 4.92 (spt, J=6.2 Hz, 1 H) 6.71 (s, 1 H) 7.22 - 7.28 (m, 1 H) 7.59 - 7.68 (m, 2 H) 8.28 (d, J=2.7 Hz, 1 H).<br>MS ESI/APCI Multi posi: 345[M+H]$^+$. |
| 1-15 | 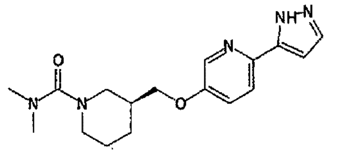 | $^1$H NMR (600 MHz, CHLOROFORM-d) δ ppm 1. 32 - 1.40 (m, 1 H) 1.55 - 1.64 (m, 1 H) 1.72 - 1.79 (m, 1 H) 1.89 - 1.96 (m, 1 H) 2.09 - 2.17 (m, 1 H) 2.72 (dd, J=12. 8, 9.9 Hz, 1 H) 2.81 - 2. 85 (m, 7 H) 3.53 - 3. 59 (m, 1 H) 3. 75 - 3.81 (m, 1 H) 3.87 - 3.97 (m, 2 H) 6.71 (d, J=1.7 Hz, 1 H) 7.24 - 7.27 (m, 1 H) 7.62 - 7.68 (m, 2 H) 8.28 (d, J=2. 9 Hz, 1 H).<br>MS ESI/APCI Multi posi: 330[M+H]$^+$. |

[Table 22-3]

| Example No. | Structure | Analytical Data |
|---|---|---|
| 1-16-1 | 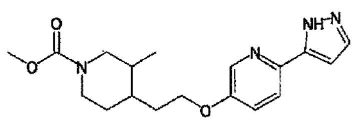 | $^1$H NMR (500 MHz, CHLOROFORM-d) δ ppm 0.84 - 0.98 (m, 3 H) 1.23 - 1.32 (m, 1 H) 1.45 - 1.55 (m, 2 H) 1.72 - 1.93 (m, 3 H) 2.81 - 2.96 (m, 1 H) 2.96 - 3.10 (m, 1 H) 3.69 (s, 3 H) 3.76 - 4.14 (m, 4 H) 6.69 - 6.73 (m, 1 H) 7.23 - 7.28 (m, 1 H) 7.62 - 7.69 (m, 2 H) 8.27 8.32 (m, 1 H).<br>MS ESI/APCI Multi posi: 345[M+H]$^+$.<br>LC-MS retention time : 1.19 min (condition: method B) |
| 1-16-2 | 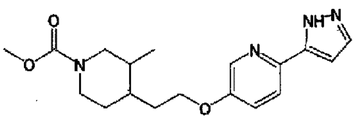 | $^1$H NMR (500 MHz, CHLOROFORM-d) δ ppm 0.96 (d, J=6. 2 Hz, 3 H) 1.20 - 1.41 (m, 3 H) 1.50 - 1.61 (m, 1 H) 1.74 - 1.85 (m, 1 H) 2.10 - 2.23 (m, 1 H) 2. 33 - 2.53 (m, 1 H) 2. 66 - 2.86 (m, 1 H) 3.69 (s, 3 H) 3.74 - 4.22 (m, 4 H) 6.62 - 6.78 (m, 1 H) 7.17 - 7.33 (m, 1 H) 7.57 - 7.71 (m, 2 H) 8.21 - 8.36 (m, 1 H).<br>MS ESI/APCI Multi posi: 345[M+H]$^+$.<br>LC-MS retention time : 1. 22 min (condition: method B) |

(continued)

| Example No. | Structure | Analytical Data |
|---|---|---|
| 1-17 | 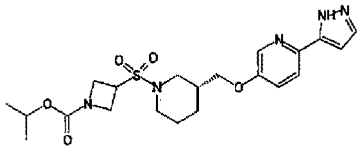 | $^1$H NMR (600 MHz, CHLOROFORM-d) δ ppm 1.23 (d, J=6.2 Hz, 6 H) 1.36 - 1.45 (m, 1 H) 1.62 - 1.71 (m, 1 H) 1.81 - 1.88 (m, 1 H) 1.89 - 1.95 (m, 1 H) 2.14 - 2.21 (m, 1 H) 2.85 (dd, J=12. 2, 10.1 Hz, 1 H) 2.90 - 2.97 (m, 1 H) 3.74 (br d, J=12. 4 Hz, 1 H) 3.86 - 4.00 (m, 4 H) 4.20 (t, J=8.9 Hz, 2 H) 4.26 - 4.30 (m, 2 H) 4.86 - 4.93 (m, 1 H) 6.69 (br s, 1 H) 7.22 - 7.28 (m, 1 H) 7.61 - 7.68 (m, 2 H) 8.27 (d, J=2.9 Hz, 1 H). MS ESI/APCI Multi posi: 464[M+H]$^+$. |
| 1-18 | 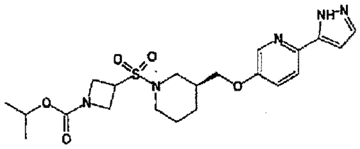 | $^1$H NMR (600 MHz, CHLOROFORM-d) δ ppm 1.23 (d, J=6.2 Hz, 6 H) 1.36 - 1.45 (m, 1 H) 1.62 - 1.71 (m, 1 H) 1.81 - 1.88 (m. 1 H) 1.89 - 1.96 (m, 1 H) 2.14 - 2.21 (m, 1 H) 2.85 (dd, J=12. 2, 10. 1 Hz, 1 H) 2.90 - 2.97 (m, 1 H) 3.74 (br d, J=12. 4 Hz, 1 H) 3.86 - 4.00 (m, 4 H) 4.20 (t, J=8.9 Hz, 2 H) 4.25 - 4.30 (m, 2 H) 4.86 - 4.93 (m, 1 H) 6.69 (br s, 1 H) 7.22 - 7.28 (m, 1 H) 7.61 - 7.68 (m, 2 H) 8.27 (d, J=2. 9 Hz, 1 H). MS ESI/APCI Multi posi: 464[M+H]$^+$. |
| 1-19 | 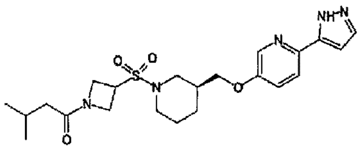 | $^1$H NMR (600 MHz, CHLOROFORM-d) δ ppm 0.91 - 1.00 (m, 6 H) 1.35 - 1.46 (m, 1 H) 1.61 - 1.72 (m, 1 H) 1.81 - 1.89 (m, 1 H) 1.89 - 2.01 (m, 3 H) 2.09 - 2.21 (m, 2 H) 2.82 - 2.90 (m, 1 H) 2.94 (br t, J=12.0 Hz, 1 H) 3.70 - 3.78 (m, 1 H) 3.86 - 4.01 (m, 4 H) 4.19 - 4.25 (m, 1 H) 4.26 - 4.31 (m, 1 H) 4.36 (td, J=8.8, 2.3 Hz, 1 H) 4.41 - 4.47 (m, 1 H) 6.70 (br s, 1 H) 7.23 - 7.30 (m, 1 H) 7.61 - 7.69 (m, 2 H) 8.28 (t, J=2.7 Hz, 1 H) MS ESI/APCI Multi posi: 462[M+H]$^+$. |
| 1-20 | 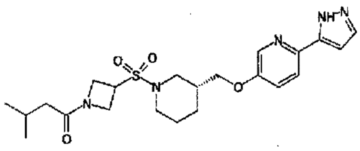 | $^1$H NMR (600 MHz, CHLOROFORM-d) δ ppm 0.92 - 1.01 (m, 6 H) 1.36 - 1.47 (m, 1 H) 1.61 - 1.72 (m, 1 H) 1.81 - 1.89 (m, 1 H) 1.90 - 2.01 (m, 3 H) 2.08 - 2.21 (m. 2 H) 2.82 - 2.90 (m, 1 H) 2.94 (br t, J=11.6 Hz, 1 H) 3.70 - 3.77 (m, 1 H) 3.86 - 4.00 (m, 4 H) 4.19 - 4.25 (m, 1 H) 4.26 - 4.31 (m, 1 H) 4.36 (td, J=8.8, 2.3 Hz, 1 H) 4.41 - 4.47 (m, 1 H) 6.70 (br s, 1 H) 7.23 - 7.28 (m, 1 H) 7.61 - 7.69 (m, 2 H) 8.28 (t, J=2.7 Hz, 1 H). MS ESI/APCI Multi posi: 462[M+H]$^+$. |
| 1-21 | 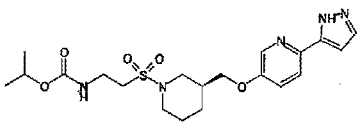 | $^1$H NMR (600 MHz. DMSO-$d_6$) δ ppm 1. 11 - 1.20 (m, 7 H) 1.21 - 1.31 (m, 1 H) 1.47 - 1.57 (m, 1 H) 1.73 - 1.87 (m, 2 H) 2.01 - 2.10 (m, 1 H) 2.72 - 2.78 (m, 1 H) 2.83 (td, J=11.6, 2.9 Hz, 1 H) 3. 16 (dd, J=8.3, 6.2 Hz, 2 H) 3.27 - 3.38 (m, 1 H) 3.46 - 3.53 (m, 1 H) 3.67 - 3.73 (m, 1 H) 3.92 - 4.07 (m, 2 H) 4.70 - 4.79 (m, 1 H) 6.73 (s, 1 H) 7.16 (br t. J=5.6 Hz, 1 H) 7.41 - 7.55 (m, 1 H) 7.72 - 7.93 (m, 1 H) 8.29 (br s, 1 H). MS ESI/APCI Multi posi: 452[M+H]$^+$. |

[Table 22-4]

| Example No. | Structure | Analytical Data |
|---|---|---|
| 1-22 | 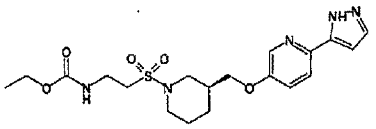 | $^1$H NMR (600 MHz, DMSO-$d_6$) δ ppm 1.14 (t, J=7.2 Hz, 3 H) 1.22 - 1.31 (m, 1 H) 1.48 - 1.58 (m, 1 H) 1.73 - 1.86 (m, 2 H) 2.01 - 2.10 (m, 1 H) 2.72 - 2.78 (m, 1 H) 2.83 (td, J=11.6, 2.9 Hz, 1 H) 3.16 (t, J=7.0 Hz, 2 H) 3.28 - 3.38 (m, 3 H) 3.47 - 3.53 (m, 1 H) 3.67 - 3.73 (m, 1 H) 3.93 - 4.06 (m, 4 H) 6.73 (s, 1 H) 7.23 (br t, J=5. 6 Hz, 1 H) 7.42 - 7.52 (m, 1 H) 7.72 - 7.92 (m, 1 H) 8.29 (br s, 1 H).<br>MS ESI/APCI Multi posi: 438[M+H]$^+$. |
| 1-23 | 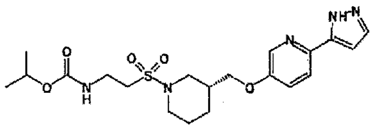 | $^1$H NMR (600 MHz, DMSO-$d_6$) δ ppm 1.15 (d, J=6.2 Hz, 6 H) 1.21 - 1.31 (m, 1 H) 1.48 - 1.58 (m, 1 H) 1.73 - 1.87 (m, 2 H) 2.01 - 2.10 (m, 1 H) 2.72 - 2.78 (m, 1 H) 2.83 (td, J=11.6, 2.5 Hz, 1 H) 3.13 - 3.18 (m, 2 H) 3.27 - 3.37 (m, 2 H) 3.47 - 3.53 (m, 1 H) 3.67 - 3.73 (m, 1 H) 3.93 - 4.06 (m, 2 H) 4.70 - 4.79 (m, 1 H) 6.73 (d, J=1.7 Hz, 1 H) 7.15 (br t, J=5.6 Hz, 1 H) 7.43 - 7.50 (m, 1 H) 7.71 - 7.91 (m, 1 H) 8.29 (br s, 1 H).<br>MS ESI/APCI Multi posi: 452 [M+H]$^+$. |
| 1-24 | 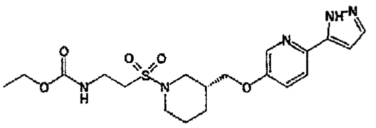 | $^1$H NMR (600 MHz, DMSO-$d_6$) δ ppm 1.14 (t, J=7.2 Hz, 3 H) 1.22 - 1.31 (m, 1 H) 1.47 - 1.58 (m, 1 H) 1.73 - 1.87 (m, 2 H) 2.01 - 2.10 (m, 1 H) 2.72 - 2.78 (m, 1 H) 2.83 (td, J=11. 6, 2.5 Hz, 1 H) 3.16 (t, J=7.0 Hz, 2 H) 3.28 - 3.38 (m, 3 H) 3.47 - 3.53 (m, 1 H) 3. 67 - 3. 73 (m, 1 H) 3. 93 - 4.06 (m, 4 H) 6.73 (d, J=1.7 Hz, 1 H) 7.23 (br t, J=5.6 Hz, 1 H) 7.41 - 7.51 (m, 1 H) 7.68 - 7.93 (m, 1 H) 8.29 (br s, 1 H).<br>MS ESI/APCI Multi posi: 438[M+H]$^+$. |
| 1-25 | 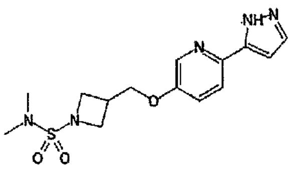 | $^1$H NMR (600 MHz. CHLOROFORM-d) δ ppm 2.80 - 2.88 (m, 6 H) 3.02 - 3.13 (m, 1 H) 3.80 - 3. 87 (m, 2 H) 4.04 - 4.11 (m, 2 H) 4.19 (br s, 2 H) 6.71 (br s, 1 H) 7.26 - 7.29 (m, 1 H) 7.56 - 7.64 (m, 1 H) 7.67 (br s, 1 H) 8.26 - 8.34 (m, 1 H) 10.73 (br s, 1 H).<br>MS ESI/APCI Multi posi: 338[M+H]$^+$. |
| 1-26 | 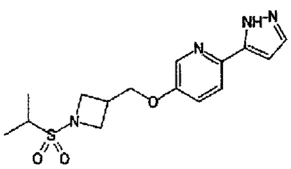 | $^1$H NMR (600 MHz, CHLOROFORM-d) δ ppm 1.36 (d, J=7.0 Hz, 6 H) 3.05 - 3.16 (m, 2 H) 3.89 - 3.94 (m, 2 H) 4.08 - 4. 19 (m, 2 H) 4.20 - 4.22 (m, 2 H) 6.71 (br s, 1 H) 7.26 - 7.29 (m, 1 H) 7.58 - 7.64 (m, 1 H) 7.67 (br s, 1 H) 8.28 (br s, 1 H) 10.86 (br s, 1 H).<br>MS ESI/APCI Multi posi: 337[M+H]$^+$. |

Example 1-29

Propan-2-yl 4-(2-{[6-(1H-pyrazol-5-yl)pyridin-3-yl]oxy}ethyl)piperidin-1-carboxylate

[Formula 196]

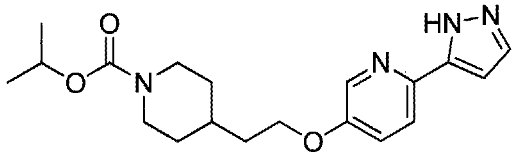

N,N-Diisopropylethylamine (63 μL) and isopropyl chloroformate (25 μL) were added to a suspension of the compound (50 mg) obtained in Reference Example 14-2 in tetrahydrofuran (2 mL), and the mixture was stirred at room temperature for 3 hours. After the reaction solution was concentrated, water was added, and the mixture was extracted with chloroform. The organic layer was separated by a phase separator, and the solvent was distilled off under reduced pressure. The obtained residue was purified by silica gel column chromatography (n-hexane/ethyl acetate = 7:3 to ethyl acetate only, subsequently chloroform/methanol = 19:1) to give the title compound (57 mg) as a colorless amorphous substance.
$^1$H NMR (500 MHz, DMSO-$d_6$) δ ppm 1.02 - 1.14 (m, 2 H) 1.18 (d, J=6.2 Hz, 6 H) 1.64 - 1.76 (m, 5 H) 2.66 - 2.83 (m, 2 H) 3.97 (br d, J=11.7 Hz, 2 H) 4.13 (br t, J=5.8 Hz, 2 H) 4.76 (spt, J=6.3 Hz, 1 H) 6.73 (d, J=2.1 Hz, 1 H) 7.38 - 7.52 (m, 1 H) 7.68 - 7.95 (m, 2 H) 8.28 (br s, 1 H).
MS ESI/APCI Multi posi: 359 [M+H]$^+$.

The compounds of the following Examples 1-30 to 1-40 were synthesized using the compound obtained in each of Reference Example 8-1, 8-2, 14-2, 14-3 or 15-1, and a corresponding acid chloride according to the method described in Example 1-29. The structures, NMR data, MS data of these compounds are shown in Tables 22-5 to 22-6.

[Table 22-5]

| Example No. | Structure | Analytical Data |
|---|---|---|
| 1-30 | 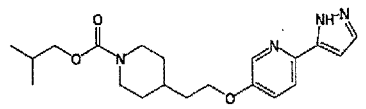 | $^1$H NMR (300 MHz, DMSO-$d_6$) δ ppm 0.88 (d, J=6. 7 Hz, 6 H) 1.00 - 1.18 (m, 2 H) 1.63 - 1. 77 (m, 5 H) 1.79 - 1.94 (m, 1 H) 2.68 - 2.90 (m, 2 H) 3. 77 (d, J=6. 5 Hz, 2 H) 3. 91 - 4. 03 (m, 2 H) 4.07 - 4.17 (m, 2 H) 6.72 (d, J=1. 9 Hz, 1 H) 7.37 - 7.54 (m, 1 H) 7.69 - 7.92 (m, 2 H) 8.27 (br s, 1 H).<br>MS ESI/APCI Multi posi: 373[M+H]$^+$. |
| 1-31 | 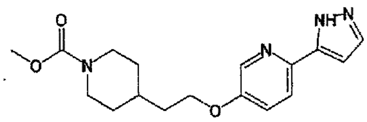 | $^1$H NMR (500 MHz, DMSO-$d_6$, 80°C) δ ppm 1.05 - 1.21 (m, 2 H) 1.64 - 1.79 (m, 5 H) 2.81 (br t, J=12.0 Hz, 2 H) 3.60 (s, 3 H) 3.96 (br d, J=13.4 Hz, 2 H) 4.15 (t, J=5.8 Hz, 2 H) 6.71 (s, 1 H) 7.42 (br d, J=7.2 Hz, 1 H) 7.57 - 7.92 (m, 2 H) 8.27 (br s, 1 H).<br>MS ESI/APCI Multi posi: 331[M+H]$^+$. |
| 1-32 | 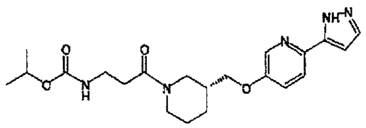 | $^1$H NMR (600 MHz, DMSO-$d_6$) δ ppm 1.07 - 1.20 (m, 6 H) 1.28 - 1.51 (m, 2 H) 1.60 - 1.76 (m, 1 H) 1.81 - 2.03 (m, 2 H) 2.41 - 2.64 (m, 1.5 H) 2.75 - 2.83 (m, 0.5 H) 2.98 - 3.07 (m, 1 H) 3.12 - 3.21 (m, 2 H) 3.69 - 3.78 (m, 0.5 H) 3.82 - 3.88 (m, 0.5 H) 3.90 - 4.03 (m, 2 H) 4.06 - 4.12 (m, 0.5 H) 4.36 - 4.45 (m, 0.5 H) 4. 69 - 4. 76 (m, 1 H) 6.73 (br s, 1 H) 6.87 - 6.94 (m, 1 H) 7.39 - 7.93 (m, 3 H) 8.26 - 8.35 (m, 1 H).<br>MS ESI/APCI Multi posi: 416[M+H]$^+$. |
| 1-33 | 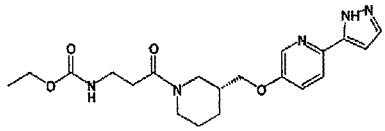 | $^1$H NMR (600 MHz, DMSO-$d_6$) δ ppm 1.09 - 1.19 (m, 3 H) 1.29 - 1.50 (m, 2 H) 1.60 - 1. 75 (m, 1 H) 1.81 - 2.03 (m, 2 H) 2.40 - 2.64 (m, 1.5 H) 2.79 (br t, J=11. 4 Hz, 0.5 H) 2.97 - 3.07 (m, 1 H) 3.13 - 3. 23 (m, 2 H) 3. 69 - 3. 78 (m, 0.5 H) 3.80 - 3.89 (m, 0.5 H) 3.89 - 4.04 (m, 4 H) 4.05 - 4.13 (m, 0.5 H) 4.37 - 4.45 (m, 0.5 H) 6.73 (br s, 1 H) 6.99 (br t, J=5. 6 Hz, 1 H) 7.38 - 7.94 (m, 3 H) 8.23 - 8.36 (m, 1 H).<br>MS ESI/APCI Multi posi: 402[H+H]$^+$. |
| 1-34 | 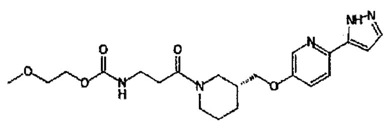 | $^1$H NMR (600 MHz, DMSO-$d_6$) δ ppm 1.29 - 1.52 (m, 2 H) 1.61 - 1.75 (m, 1 H) 1.82 - 2.03 (m, 2 H) 2.41 - 2.64 (m, 3.5 H) 2.75 - 2.83 (m, 0.5 H) 2.98 - 3.07 (m, 1 H) 3.15 - 3.27 (m, 4 H) 3.43 - 3.49 (m, 2 H) 3.71 - 3.77 (m, 0.5 H) 3.82 - 3.88 (m, 0.5 H) 3.91 - 4.13 (m, 3.5 H) 4.38 - 4.46 (m, 0.5 H) 6.73 (s, 1 H) 7.11 (br t, J=5.4 Hz, 1 H) 7.40 - 7.94 (m, 3 H) 8.24 - 8.35 (m, 1 H).<br>MS ESI/APCI Multi posi: 432[H+H]$^+$. |

(continued)

| Example No. | Structure | Analytical Data |
|---|---|---|
| 1-35 | 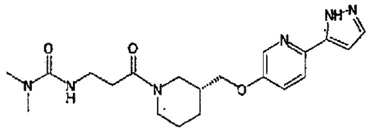 | $^1$H NMR (600 MHz, DMSO-$d_6$) δ ppm 1.29 - 1.48 (m, 2 H) 1.61 - 1.75 (m, 1 H) 1.82 - 2.01 (m, 2 H) 2.39 - 2.64 (m, 2 H) 2.70 - 2.80 (m, 6 H) 3.03 (br dd, J=13. 2, 10.3 Hz, 1 H) 3.16 - 3.24 (m, 2 H) 3.75 - 4.03 (m, 3 H) 4.09 - 4.15 (m, 0.5 H) 4.38 - 4.45 (m, 0.5 H) 6.28 (q, J=5.8 Hz, 1 H) 6.73 (d, J=2.1 Hz, 1 H) 7.41 - 7.94 (m, 3 H) 8.25 - 8.35 (m, 1 H). MS ESI/APCI Multi posi: 401(M+H)$^+$. |
| 1-36 | 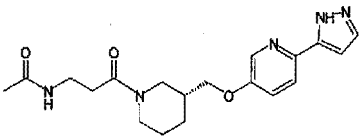 | $^1$H NMR (600 MHz, CHLOROFORM-d) δ ppm 1.40 - 1.71 (m, 3 H) 1.74 - 1.87 (m, 1 H) 1.89 - 2.13 (m, 4 H) 2.51 - 2.60 (m, 2 H) 2.62 - 2.67 (m, 0.5 H) 2.85 - 2.91 (m, 0.5 H) 3.00 - 3.09 (m, 1 H) 3.50 - 3. 59 (m, 2 H) 3.72 - 3. 78 (m, 0.5 H) 3.84 - 3.99 (m, 2.5 H) 4.30 - 4.34 (m, 0.5 H) 4.61 - 4.66 (m, 0.5 H) 6.36 - 6.47 (m, 0.5 H) 6.67 - 6.73 (m, 0.5 H) 7.23 - 7. 26 (m, 1 H) 7.61 - 7. 69 (m, 2 H) 8.27 - 8.30 (m, 1 H). MS ESI/APCI Multi posi: 372[M+H]$^+$. |

[Table 22-6]

| Example No. | Structure | Analytical Data |
|---|---|---|
| 1-37 | 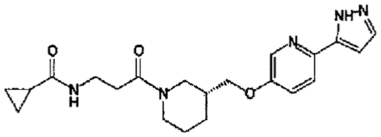 | $^1$H NMR (600 MHz, CHLOROFORM-d) δ ppm 0. 66 - 0.74 (m, 2 H) 0.89 - 0.98 (m, 2 H) 1.25 - 1.37 (m, 1 H) 1. 41 - 1.64 (m, 2 H) 1.75 - 1.84 (m, 1 H) 1.92 - 2.10 (m, 2 H) 2.52 - 2.60 (m, 2 H) 2.64 - 2.69 (m, 0.5 H) 2.85 - 2.90 (m, 0.5 H) 3.01 - 3.09 (m, 1 H) 3.52 - 3.65 (m, 2 H) 3.73 - 3.78 (m, 0.5 H) 3.85 - 3.98 (m, 2.5 H) 4.32 - 4.36 (m, 0.5 H) 4.62 - 4.66 (m, 0.5 H) 6.52 - 6.60 (m, 0.5 H) 6. 65 - 6.73 (m, 0.5 H) 7.22 - 7.26 (m, 1 H) 7.60 - 7.69 (m, 2 H) 8.27 - 8.30 (m, 1 H). MS ESI/APCI Multi posi: 398[M+H]$^+$. |
| 1-38 | 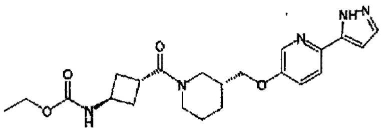 | $^1$H NMR (600 MHz, DMSO-$d_6$) δ ppm 1.14 (t, J=7.0 Hz, 3 H) 1.30 - 1.43 (m, 2 H) 1.62 - 1.71 (m, 1 H) 1.82 - 1.99 (m, 2 H) 2.04 - 2.18 (m, 2 H) 2.32 - 2.44 (m, 2 H) 2.53 - 2.85 (m, 2 H) 2.87 - 2.99 (m, 1 H) 3.14 - 3.23 (m, 1 H) 3.48 - 3.72 (m, 1 H) 3.88 - 4.02 (m, 4 H) 4.06 - 4.17 (m, 0.5 H) 4.39 - 4.47 (m, 0.5 H) 6.70 - 6.76 (m, 1 H) 7.41 - 7.55 (m 2 H) 7.65 - 7.95 (m, 2 H) 8.25 - 8.34 (m, 1 H) 12.85 - 13.43 (m, 1 H). MS ESI/APCI Multi posi: 428[M+H]$^+$. |
| 1-39 | 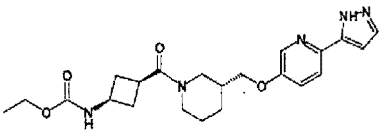 | $^1$H NMR (600 MHz, DMSO-$d_6$) δ ppm 1.08 - 1.19 (m, 3 H) 1.29 - 1.42 (m, 2 H) 1.60- 1.72 (m, 1 H) 1.79 - 1.90 (m, 2 H) 1.96 - 2.09 (m, 2 H) 2.23 - 2.43 (m, 2 H) 2.51 - 2.81 (m, 2 H) 2.87 - 3.00 (m, 2 H) 3. 61 - 3. 71 (m, 0.5 H) 3.75 - 3.84 (m, 0.5 H) 3.87 - 4.04 (m, 4 H) 4.06 - 4.13 (m, 0.5 H) 4.34 - 4.40 (m, 0.5 H) 6.71 - 6.76 (m, 1 H) 7.31 - 7.56 (m, 2 H) 7.71 - 7.95 (m, 2 H) 8.25 - 8.35 (m, 1 H) 12.86 - 13.38 (m, 1 H). MS ESI/APCI Multi posi: 428[M+H]$^+$. |
| 1-40 | 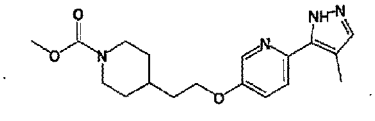 | $^1$H NMR (500 MHz, DMSO-$d_6$, 100°C) δ ppm 1.03 - 1.20 (m, 2 H) 1.61 - 1.78 (m, 5 H) 2.28 (s, 3 H) 2.80 (br t, J=12. 3 Hz, 2 H) 3.58 (s, 3 H) 3.94 (br d, J=13.4 Hz, 2 H) 4.14 (br t, J=5.5 Hz, 2 H) 7.34 - 7.44 (m, 2 H) 7.67 - 7.81 (m, 1 H) 8.29 (br s, 1 H). MS ESI/APCI Multi posi: 345[M+H]$^+$. |

Example 2-1

(1-Methylcyclopropyl)[4-({[6-(1H-pyrazol-5-yl)pyridin-3-yl]oxy}methyl)piperidin-1-yl]methanone

[Formula 197]

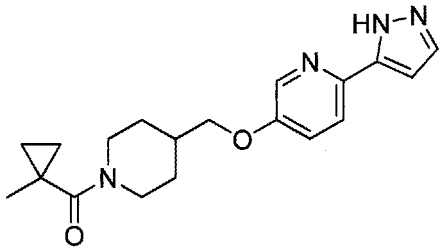

(1) N,N-Diisopropylethylamine (102 μL), propylphosphonic anhydride (1.7 mol/Lethyl acetate solution, 174 μL) and 1-methylcyclopropan-1-carboxylic acid (43 mg) were added to a solution of the compound (50 mg) obtained in Reference Example 2-1 in ethyl acetate (1.5 mL), and the mixture was stirred at room temperature overnight. An aqueous solution of saturated sodium hydrogen carbonate was added to the reaction solution, the mixture was extracted with ethyl acetate, and the organic layer was sequentially washed with water and brine, and then separated by a phase separator. The obtained organic layer was concentrated under reduced pressure to give (1-methylcyclopropyl)-[4-[[6-[2-(oxan-2-yl)pyrazol-3-yl]pyridin-3-yl]oxymethyl]piperidin-1-yl]methanone.

(2) The compound obtained in the above described (1) was used to perform the synthesis process according to the method described in Example 1-1-(2) thereby giving the title compound (43 mg).

$^1$H NMR (600 MHz, CHLOROFORM-d) δ ppm 0.54 - 0.60 (m, 2 H) 0.90 - 0.95 (m, 2 H) 1.19 - 1.35 (m, 4 H) 1.31 (s, 3 H) 1.86 - 1.96 (m, 2 H) 2.04 - 2.14 (m, 1 H) 3.86 - 3.92 (m, 2 H) 4.44 - 4.58 (m, 2 H) 6.68 (s, 1 H) 7.19 - 7.27 (m, 1 H) 7.59 - 7.69 (m, 2 H) 8.25 - 8.30 (m, 1 H).

MS ESI/APCI Multi posi: 341 $[M+H]^+$.

The compounds of the following Examples 2-2 to 2-10 were synthesized using the compound obtained in Reference Example 2-1, and a corresponding carboxylic acid according to the method described in Example 2-1. The structures, NMR data, MS data of these compounds are shown in Tables 23-1 to 23-2.

[Table 23-1]

| Example No. | Structure | Analytical Data |
|---|---|---|
| 2-2 | 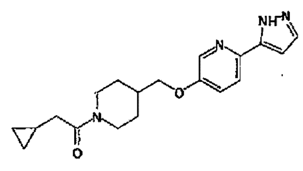 | $^1$H NMR (600 MHz, CHLOROFORM-d) δ ppm 0.13 - 0.22 (m, 2 H) 0.49 - 0.60 (m, 2 H) 0.99 - 1. 39 (m, 3 H) 1.81 - 1.98 (m, 2 H) 2.02 - 2.14 (m, 1 H) 2.25 - 2.32 (m, 2 H) 2.56 - 2.65 (m, 1 H) 3.02 - 3.11 (m, 1 H) 3.82 - 3.96 (m, 3 H) 4.69 - 4.77 (m, 1 H) 6.68 (s, 1 H) 7.19 - 7.24 (m. 1 H) 7.58 - 7.68 (m, 2 H) 8.24 - 8. 29 (m, 1 H).<br>MS ESI/APCI Multi posi: 341$[H+H]^+$. |
| 2-3 | 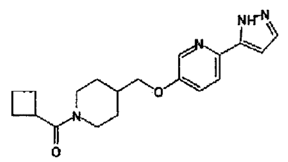 | $^1$H NMR (600 MHz, CHLOROFORM-d) δ ppm 1.16 - 2.20 (m, 9 H) 2.30 - 2.40 (m, 2 H) 2.55 - 2.64 (m, 1 H) 2.93 - 3.02 (m, 1 H) 3.21 - 3.31 (m, 1 H) 3.72 - 3.94 (m, 3 H) 4.63 - 4.72 (m, 1 H) 6.68 (s, 1 H) 7.19 - 7. 24 (m, 1 H) 7.59 - 7. 69 (m, 2 H) 8.23 - 8.29 (m, 1 H).<br>MS ESI/APCI Multi posi: 341$[M+H]^+$. |

(continued)

| Example No. | Structure | Analytical Data |
|---|---|---|
| 2-4 | 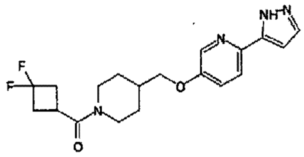 | $^1$H NMR (600 MHz, CHLOROFORM-d) δ ppm 1.20 - 1.37 (m, 2 H) 1.85 - 1.99 (m, 2 H) 2.04 - 2.16 (m, 1 H) 2.61 - 2.77 (m, 3 H) 2.85 - 2.99 (m, 2 H) 3.02 - 3.11 (m, 2 H) 3.75 - 3.81 (m, 1 H) 3.83 - 3.94 (m, 2 H) 4.64 - 4.72 (m, 1 H) 6.68 (br s, 1 H) 7.19 - 7.24 (m, 1 H) 7.58 - 7.69 (m, 2 H) 8.24 - 8.29 (m, 1 H).<br>MS ESI/APCI Multi posi: 377[M+H]$^+$. |
| 2-5 | 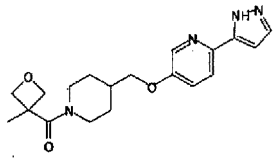 | $^1$H NMR (600 MHz, CHLOROFORM-d) δ ppm 1.25 - 1.39 (m, 2 H) 1.66 (s, 3 H) 1.86 - 1.97 (m, 2 H) 2.03 - 2.14 (m, 1 H) 2.57 - 2.68 (m, 1 H) 2.97 - 3.10 (m, 2 H) 3.83 - 3.96 (m, 2 H) 4.31 - 4.38 (m, 2 H) 4.63 - 4.71 (m, 1 H) 4. 95 - 5.02 (m, 2 H) 6.68 (br s, 1 H) 7.20 - 7.24 (m, 1 H) 7.58 - 7.70 (m, 2 H) 8.24 - 8.29 (m, 1 H).<br>MS ESI/APCI Multi posi: 357[M+H]$^+$. |
| 2-6 | 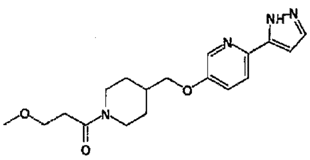 | $^1$H NMR (600 MHz, CHLOROFORM-d) δ ppm 1.20 - 1.38 (m, 2 H) 1.82 - 1.97 (m, 2 H) 2.02 - 2.13 (m, 1 H) 2.56 - 2.67 (m, 3 H) 3.02 - 3.10 (m, 1 H) 3.36 (s, 3 H) 3.68 - 3.74 (m, 2 H) 3.83 - 3.93 (m, 2 H) 3.93 - 4.00 (m, 1 H) 4.68 - 4.75 (m, 1 H) 6.68 (s, 1 H) 7.20 - 7.24 (m, 1 H) 7.59 - 7.68 (m, 2 H) 8. 24 - 8.29 (m, 1 H).<br>MS ESI/APCI Multi posi: 345[M+H]$^+$. |
| 2-7 | 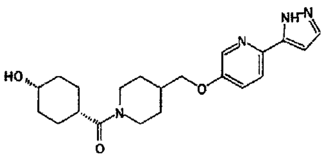 | $^1$H NMR (600 MHz, CHLOROFORM-d) δ ppm 0.68 - 2.07 (m, 14 H) 2.42 - 2.55 (m, 2 H) 2.95 - 3.05 (m, 1 H) 3.74 - 3.98 (m, 4 H) 4.60 - 4.70 (m, 1 H) 6.58 - 6.63 (m, 1 H) 7.12 - 7.17 (m, 1 H) 7.51 - 7.59 (m, 2 H) 8.16 - 8.20 (m, 1 H).<br>MS ESI/APCI Multi posi: 385[M+H]$^+$. |
| 2-8 | 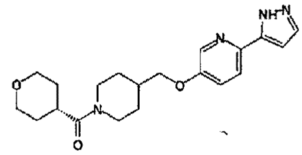 | $^1$H NMR (600 MHz, CHLOROFORM-d) δ ppm 1.22 - 1.37 (m, 2 H) 1.51 - 1.64 (m, 2 H) 1. 83 - 2.03 (m, 4 H) 2.04 - 2.15 (m, 1 H) 2.55 - 2.65 (m, 1 H) 2.70 - 2.79 (m, 1 H) 3.04 - 3.14 (m, 1 H) 3.40 - 3.50 (m, 2 H) 3.82 - 4.06 (m, 5 H) 4.67 - 4. 76 (m, 1 H) 6.62 - 6.74 (m. 1 H) 7.19 - 7.24 (m, 1 H) 7.58 - 7.70 (m, 2 H) 8.24 - 8.28 (m, 1 H).<br>MS ESI/APCI Multi posi: 371[M+H]$^+$. |

[Table 23-2]

| Example No. | Structure | Analytical Data |
|---|---|---|
| 2-9 | 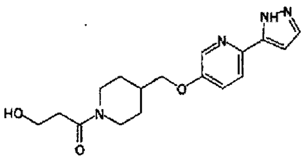 | $^1$H NMR (600 MHz, CHLOROFORM-d) δ ppm 1.26 - 1. 39 (m, 2 H) 1.86 - 1.92 (m, 1 H) 1.92 - 1.98 (m, 1 H) 2.05 - 2.14 (m, 1 H) 2.52 - 2.58 (m, 2 H) 2.59 - 2.67 (m, 1 H) 3.02 - 3.13 (m, 1 H) 3.83 - 3.94 (m, 5 H) 4.68 - 4.74 (m, 1 H) 6. 65 - 6.71 (m, 1 H) 7.22 (dd, J=8.67, 2.89 Hz, 1 H) 7. 59 - 7.68 (m, 2 H) 8.24 - 8.29 (m, 1 H).<br>MS ESI/APCI Multi posi: 331[M+H]$^+$. |
| 2-10 | 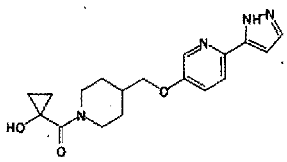 | $^1$H NMR (600 MHz, CHLOROFORM-d) δ ppm 0.94 - 1.00 (m, 2 H) 1.09 - 1.15 (m, 2 H) 1.31 - 1.41 (m, 2 H) 1. 89 - 1.95 (m, 2 H) 2.06 - 2. 16 (m, 1 H) 2.78 - 2.98 (m, 2 H) 3.05 - 3.13 (m, 1 H) 3.85 - 3.91 (m, 2 H) 4.56 - 4.64 (m, 2 H) 6.63 - 6.72 (m, 1 H) 7.19 - 7.24 (m, 1 H) 7.57 - 7.68 (m, 2 H) 8.23 - 8.29 (m, 1 H).<br>MS ESI/APCI Multi posi: 343[M+H]$^+$. |

Example 2-11

tert-Butyl methyl{3-oxo-3-[(3R)-3-({[6-(1H-pyrazol-5-yl)pyridin-3-yl]oxy}methyl)piperidin-1-yl]propyl}carbamate

[Formula 198]

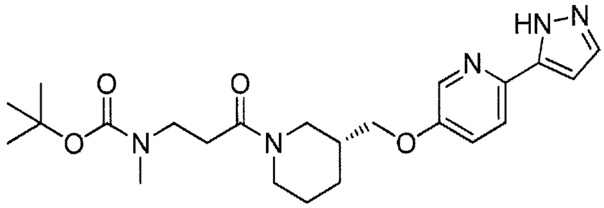

(1) 3-{Methyl-[(2-methylpropan-2-yl)oxycarbonyl]amino}propanoic acid (59.4 mg), diisopropylethylamine (102 μL), and propylphosphonic anhydride (1.6 mol/L N,N-dimethylformamide solution, 183 μL) were added to a solution of the compound (50 mg) obtained in Reference Example 2-2 in N,N-dimethylformamide (1 mL), and the mixture was stirred at room temperature for 2 hours. Water was added to the reaction solution, the mixture was extracted with ethyl acetate twice, and the organic layer was dried over magnesium sulfate. After the drying agent was filtered off, the filtrate was concentrated under reduced pressure to give a mixture (87 mg) containing tert-butyl N-methyl-N-{3-[(3R)-3-({6-[2-(oxan-2-yl)pyrazol-3-yl]pyridin-3-yl}oxymethyl)piperdin-1-yl]-3-oxopropyl}carbamate.

(2) Two mol/L hydrochloric acid (0.5 mL) was added to a solution of the compound (87 mg) obtained in the above described (1) in methanol (1 mL), and the mixture was stirred at room temperature for 2 hours. After the end of the reaction was confirmed by LC-MS, triethylamine (140 μL) was added to adjust the pH to 8. The mixture was purified by preparative HPLC to give the title compound (17 mg) as a colorless oily substance. $^1$H NMR (600 MHz, CHLOROFORM-d) δ ppm 1.42 - 1.49 (m, 9 H) 1.50 - 1.60 (m, 1 H) 1.72 - 2.15 (m, 4 H) 2.53 - 2.69 (m, 3 H) 2.85 - 2.91 (m, 3 H) 3.04 - 3.17 (m, 1 H) 3.41 - 3.62 (m, 2 H) 3.80 - 4.05 (m, 3 H) 4.23 - 4.68 (m, 1 H) 6.65 - 6.74 (m, 1 H) 7.22 - 7.31 (m, 1 H) 7.59 - 7.71 (m, 2 H) 8.26 - 8.32 (m, 1 H).
MS ESI/APCI Multi posi: 444 [M+H].

The compounds of the following Examples 2-12 to 2-18 were synthesized using the compound obtained in Reference Example 2-2, and a corresponding commercially available carboxylic acid, or the compound obtained in Reference Example 9-1 and a corresponding commercially available amine according to the method described in Example 2-11. The structures, NMR data, MS data of these compounds are shown in Table 23-3.

[Table 23-3]

| Example No. | Structure | Analytical Data |
|---|---|---|
| 2-12 | 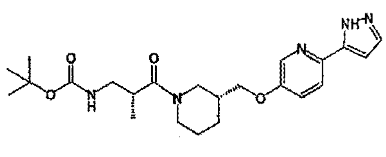 | $^1$H NMR (600 MHz, CHLOROFORM-d) δ ppm 1.03 - 1.14 (m, 3 H) 1.39 - 1.59 (m, 11 H) 1.74 - 2.09 (m, 3 H) 2.70 - 2.87 (m, 1 H) 3.00 - 3.18 (m, 2 H) 3.21 - 3.34 (m, 2 H) 3.79 - 4.06 (m, 3 H) 4.35 - 4.66 (m, 1 H) 5.10 - 5.25 (m, 1 H) 6.65 - 6.74 (m, 1 H) 7.21 - 7.32 (m, 1 H) 7.58 - 7.72 (m, 2 H) 8.24 - 8.33 (m, 1 H). MS ESI/APCI Multi posi: 444[M+H]$^+$. |
| 2-13 | 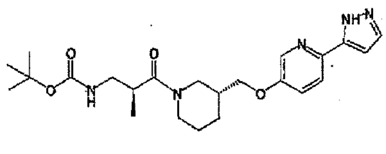 | $^1$H NMR (600 MHz, CHLOROFORM-d) δ ppm 1.05 - 1.14 (m, 3 H) 1. 36 - 1.44 (m, 9 H) 1.46 - 1.60 (m, 2 H) 1.73 - 2.08 (m, 3 H) 2.64 - 2.77 (m, 1 H) 2.93 - 3.14 (m, 2 H) 3.21 - 3.34 (m, 2 H) 3.82 - 3.93 (m, 2 H) 3.94 - 4.11 (m, 1 H) 4.44 - 4.65 (m, 1 H) 5.12 - 5.23 (m, 1 H) 6.63 - 6.73 (m, 1 H) 7.19 - 7.31 (m, 1 H) 7.60 - 7.70 (m, 2 H) 8.24 - 8.31 (m, 1 H). MS ESI/APCI Multi posi : 444[M+H)$^+$. |

(continued)

| Example No. | Structure | Analytical Data |
|---|---|---|
| 2-14 | 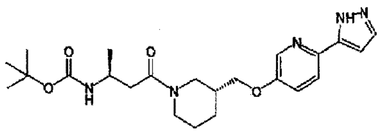 | $^1$H NMR (600 MHz, CHLOROFORM-d) δ ppm 1.23 - 1.28 (m, 3 H) 1.40 - 1.45 (m, 9 H) 1.45 - 1.63 (m, 2 H) 1.74 - 1.86 (m, 1 H) 1.88 - 2.14 (m, 2 H) 2.40 - 2.54 (m, 1 H) 2.61 - 2.69 (m, 1 H) 2.69 - 2.94 (m, 1 H) 3.05 - 3.16 (m, 1 H) 3. 79 - 3. 94 (m, 2 H) 3.95 - 4.06 (m, 2 H) 4.26 - 4.66 (m, 1 H) 5.19 - 5.40 (m, 1 H) 6.66 - 6.74 (m, 1 H) 7.21 - 7.31 (m, 1 H) 7.59 - 7.70 (m, 2 H) 8.25 - 8.33 (m, 1 H).<br>MS ESI/APCI Multi posi: 444[M+H]$^+$. |
| 2-15 | 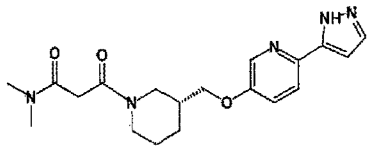 | $^1$H NMR (600 MHz, CHLOROFORM-d) δ ppm 1.43 - 1.64 (m, 2 H) 1.74 - 1.86 (m, 1 H) 1.89 - 1.99 (m, 1 H) 2.04 - 2.19 (m, 1 H) 2. 76 - 2.93 (m, 1 H) 2.93 - 3.00 (m, 3 H) 3.07 - 3.12 (m, 3 H) 3.13 - 3.21 (m, 1 H) 3. 50 - 3. 60 (m, 2 H) 3. 88 - 4. 07 (m, 3 H) 4.26 - 4.55 (m, 1 H) 6.67 - 6.71 (m, 1 H) 7.22 - 7.29 (m, 1 H) 7.61 - 7.69 (m, 2 H) 8.28 (d, J=2.9 Hz, 1 H).<br>MS ESI/APCI Multi posi : 372[M+H]$^+$. |
| 2-16 | 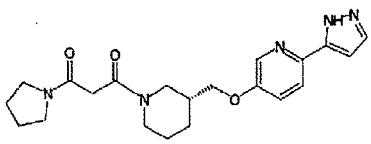 | $^1$H NMR (600 MHz, CHLOROFORM-d) δ ppm 1.43 - 1.67 (m, 2 H) 1.73 - 2.00 (m, 6 H) 2.03 - 2.21 (m, 1 H) 2.78 - 2.98 (m, 1 H) 3.12 - 3.24 (m, 1 H) 3.42 - 3.59 (m, 6 H) 3.89 - 4.09 (m, 3 H) 4.25 - 4.54 (m, 1 H) 6.70 (dd, J=11.6, 2.1 Hz, 1 H) 7.20 - 7.26 (m, 1 H) 7.58 - 7.72 (m, 2 H) 8.29 (d, J=1.2 Hz, 1 H).<br>MS ESI/APCI Multi posi: 398[M+H]$^+$. |
| 2-17 | 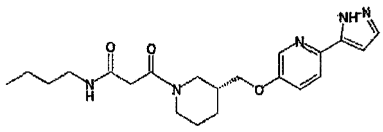 | $^1$H NMR (600 MHz, CHLOROFORM-d) δ ppm 0.88 - 0.94 (m, 3 H) 1.30 - 1.39 (m, 2 H) 1.44 - 1.62 (m, 4 H) 1.77 - 1.88 (m, 1 H) 1.92 - 2.00 (m, 1 H) 2.01 - 2.13 (m, 1 B) 2.69 - 2.94 (m, 1 H) 3.11 - 3.19 (m, 1 H) 3.21 - 3.32 (m, 2 H) 3.33 - 3.44 (m, 2 H) 3.86 - 4.10 (m, 3 H) 4.32 - 4.65 (m, 1 H) 6.67 - 6.74 (m, 1 H) 7.22 - 7.30 (m, 1 H) 7.58 - 7.71 (m, 3 H) 8.27 - 8.33 (m, 1 H).<br>MS ESI/APCI Multi posi: 400[M+H]$^+$. |
| 2-18 | 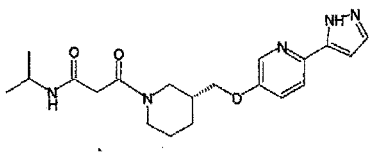 | $^1$H NMR (600 MHz, CHLOROFORM-d) δ ppm 1.14 - 1.17 (m, 6 H) 1.43 - 1.62 (m, 2 H) 1.76 - 1.88 (m, 1 H) 1.92 - 2.00 (m, 1 H) 2.01 - 2.13 (m, 1 H) 2.68 - 2.92 (m, 1 H) 3.10 - 3.18 (m, 1 H) 3.31 - 3.43 (m, 2 H) 3.86 - 3.92 (m, 1 H) 3.92 - 4.12 (m, 3 H) 4.32 - 4.66 (m, 1 H) 6.68 - 6.73 (m, 1 H) 7.22 - 7.26 (m, 1 H) 7.40 - 7.52 (m, 1 H) 7.63 (t, J=2.1 Hz, 1 H) 7.65 - 7.70 (m, 1 H) 8.28 - 8.32 (m, 1 H).<br>MS ESI/APCI Multi posi: 386[M+H]$^+$. |

Example 3-2

(4-Hydroxycyclohexyl)-((3R)-3-{[6-(1H-pyrazol-5-yl)pyridin-3-yl]oxymethyl}piperidin-1-yl)methanone(trans isomer)

[Formula 199]

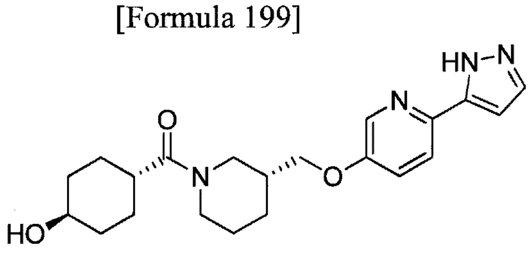

Example 3-3

(4-Hydroxycyclohexyl)-((3R)-3-{[6-(1H-pyrazol-5-yl)pyridin-3-yl]oxymethyl}piperidin-1-yl)methanone (cis isomer)

[Formula 200]

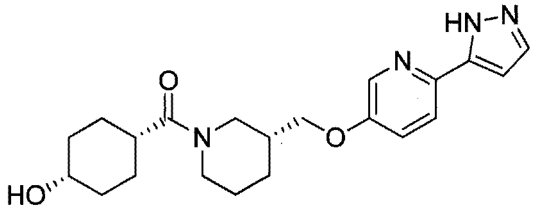

N,N-Diisopropylethylamine (132 μL), 4-hydroxycyclohexane-1-carboxylic acid (33 mg) and propylphosphonic anhydride (1.6 mol/L N,N-dimethylformamide solution, 238 μL) were added to a solution of the compound (50 mg) obtained in Reference Example 14-1 in N,N-dimethylformamide (2 mL), and the mixture was stirred at room temperature overnight. The mixture was purified by preparative LC-MS to give the title compound (Example 3-2) (25 mg) which was a trans isomer as a highly polar compound, as a colorless amorphous substance.
$^1$H NMR (500 MHz, DMSO-$d_6$) δ ppm 1.09 - 2.03 (m, 14.5 H) 2.85 - 2.98 (m, 0.5 H) 3.00 - 3.20 (m, 1.5 H) 3.21 - 3.40 (m, 1 H) 3.78 - 4.08 (m, 3.5 H) 4.35 - 4.45 (m, 0.5 H) 4.48 - 4.58 (m, 1 H) 6.73 (s, 1 H) 7.36 - 7.96 (m, 3 H) 8.23 - 8.33 (m, 1 H). MS ESI/APCI Multi posi: 385 [M+H]$^+$.

The title compound (Example 3-3) (23.7 mg) which was a cis isomer as a less polar compound was obtained as a colorless amorphous substance.
$^1$H NMR (600 MHz, DMSO-$d_6$) δ ppm 1.13 - 2.02 (m, 12 H) 2.45 - 2.63 (m, 1.5 H) 2.84 - 2.95 (m, 0.5 H) 2.99 - 3.17 (m, 1 H) 3.26 - 3.37 (m, 1 H) 3.71 - 4.09 (m, 4.5 H) 4.19 - 4.30 (m, 1 H) 4.38 - 4.46 (m, 0.5 H) 6.73 (br s, 1 H) 7.38 - 7.93 (m, 3 H) 8.24 - 8.35 (m, 1 H). MS ESI/APCI Multi posi: 385 [M+H]$^+$.

The compounds of the following Examples 3-4 to 3-5, 3-9 to 3-15, 3-17 to 3-21, and 3-23 to 3-47 were synthesized using the compound obtained in Reference Example 14-1, and a corresponding carboxylic acid according to the method described in Example 3-2. The structures, NMR data, MS data of these compounds are shown in Tables 24-1 to 24-7.

[Table 24-1]

| Example No. | Structure | Analytical Data |
|---|---|---|
| 3-4 | 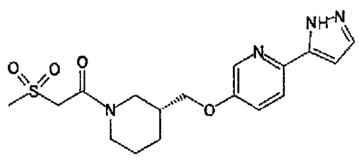 | $^1$H NMR (600 MHz, DMSO-$d_6$) δ ppm 1.31 - 1.58 (m, 2 H) 1.66 - 1.76 (m,1 H) 1.81 - 1.97 (m, 2 H) 2.74 - 2.86 (m, 1 H) 3.04 - 3.19 (m, 4 H) 3.83 - 4.08 (m, 3.5 H) 4.32 - 4.39 (m, 0.5 H) 4.42 - 4.53 (m, 2 H) 6.74 (br s, 1 H) 7.39 - 7.95 (m, 3 H) 8.24 - 8.36 (m, 1 H). MS ESI/APCI Multi posi: 379[M+H]$^+$. MS ESI/APCI Multi nega: 377[M-H]$^-$. |
| 3-5 | 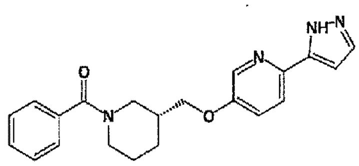 | MS ESI/APCI Multi posi: 363[M+H]$^+$. |

[Table 24-2]

| Example No. | Structure | Analytical Data |
|---|---|---|
| 3-9 | 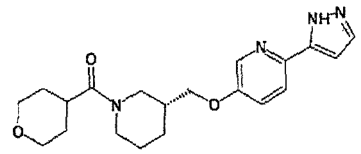 | MS ESI/APCI Multi posi : 371[M+H]+. |
| 3-10 | 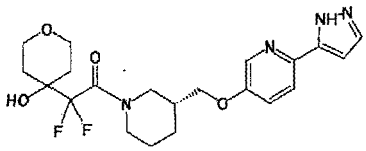 | MS ESI/APCI Multi posi : 437[M+H]+. |
| 3-11 | 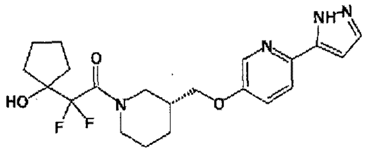 | MS ESI/APCI Multi posi: 421[M+H]+. |
| 3-12 | 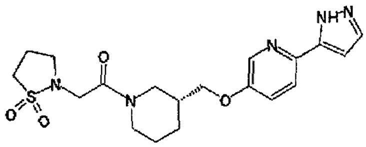 | MS ESI/APCI Multi posi : 420[M+H]+.<br>MS ESI/APCI Multi nega: 418[M-H]-. |
| 3-13 | 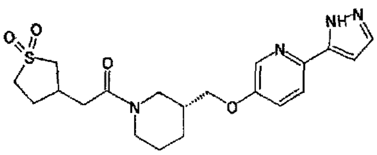 | MS ESI/APCI Multi posi: 419[M+H]+. |
| 3-14 | 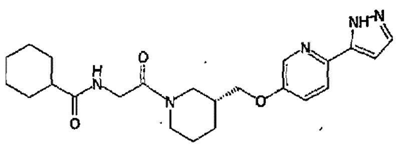 | MS ESI/APCI Multi posi: 425[M+H]+. |
| 3-15 | 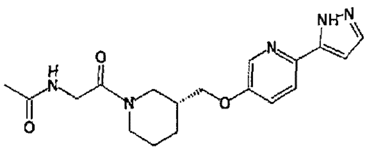 | MS ESI/APCI Multi posi: 358[M+H]+. |

[Table 24-3]

| Example No. | Structure | Analytical Data |
|---|---|---|
| 3-17 | 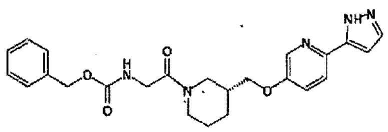 | MS ESI/APCI Multi posi: 450[M+H]+. |
| 3-18 | 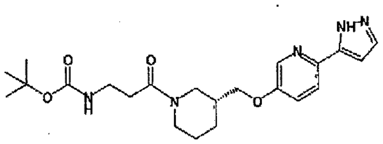 | MS ESI/APCI Multi posi: 430 [M+H]+. |

(continued)

| Example No. | Structure | Analytical Data |
|---|---|---|
| 3-19 | 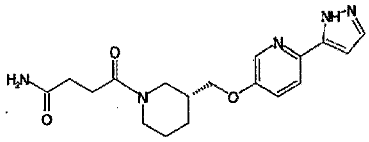 | MS ESI/APCI Multi posi: 358[M+H]$^+$. |
| 3-20 | 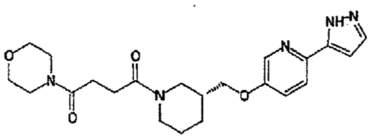 | MS ESI/APCI Multi posi: 428[M+H]$^+$. |
| 3-21 | 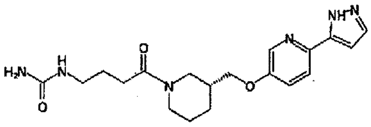 | MS ESI/APCI Multi posi: 387[M+H)$^+$. |

[Table 24-4]

| Example No. | Structure | Analytical Data |
|---|---|---|
| 3-23 | 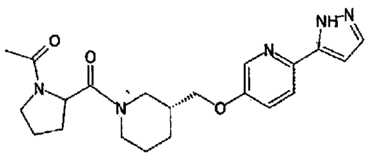 | MS ESI/APCI Multi posi: 398(M+H]$^+$.<br>MS ESI/APCI Multi nega: 396[M-H]$^-$. |
| 3-24 | 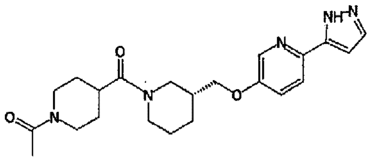 | MS ESI/APCI Multi posi: 412[M+H]$^+$.<br>MS ESI/APCI Multi nega: 410[M-H]$^-$. |
| 3-25 | 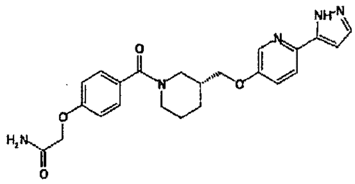 | MS ESI/APCI Multi posi: 436[M+H]$^+$.<br>MS ESI/APCI Multi nega: 434[M-H]$^-$. |
| 3-26 | 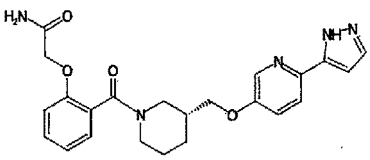 | MS ESI/APCI Multi posi: 436[M+H]$^+$.<br>MS ESI/APCI Multi nega: 434[M-H]$^-$. |
| 3-27 | 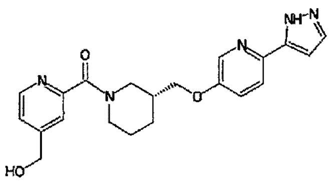 | MS ESI/APCI Multi posi: 394[M+H]$^+$.<br>MS ESI/APCI Multi nega: 392[M-H]$^-$. |

(continued)

| Example No. | Structure | Analytical Data |
| --- | --- | --- |
| 3-28 | 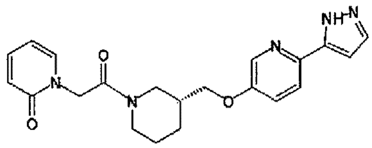 | MS ESI/APCI Multi posi: 394[M+H]$^+$. MS ESI/APCI Multi nega: 392[M-H]$^-$. |
| 3-29 | 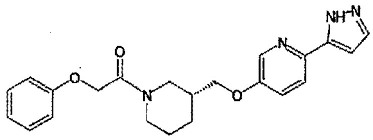 | MS ESI/APCI Multi posi : 393[M+H]$^+$. MS ESI/APCI Multi nega: 391[M-H]$^-$. |

[Table 24-5]

| Example No. | Structure | Analytical Data |
| --- | --- | --- |
| 3-30 | 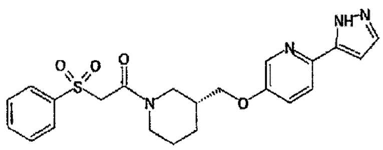 | MS ESI/APCI Multi posi: 441[M+H]$^+$. MS ESI/APCI Multi nega: 439[M-H]$^-$. |
| 3-31 | 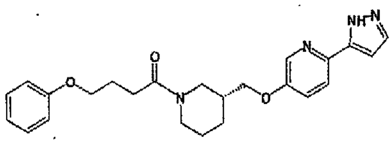 | MS ESI/APCI Multi posi: 421[M+H]$^+$. MS ESI/APCI Multi nega: 419[M-H]$^-$. |
| 3-32 | 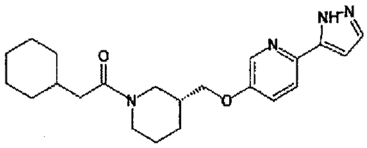 | MS ESI/APCI Multi posi : 383[M+H]$^+$. MS ESI/APCI Multi nega: 381[M-H]$^-$. |
| 3-33 | 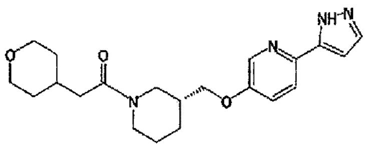 | MS ESI/APCI Multi posi : 385[M+H]$^+$. MS ESI/APCI Multi nega: 383[M-H]$^-$. |
| 3-34 | 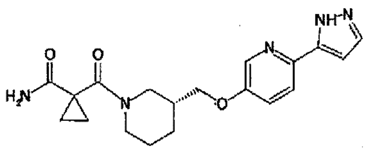 | MS ESI/APCI Multi posi : 370[M+H]$^+$. MS ESI/APCI Multi nega: 366[M-H]$^-$. |
| 3-35 | 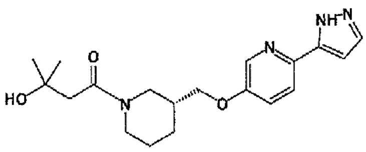 | MS ESI/APCI Multi posi: 359[M+H]$^+$. |
| 3-36 | 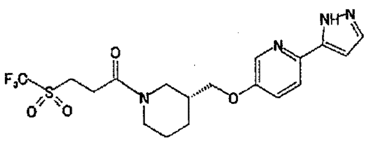 | MS ESI/APCI Multi posi: 447[M+H]$^+$. MS ESI/APCI Multi nega: 445[M-H]$^-$. |

[Table 24-6]

| Example No. | Structure | Analytical Data |
|---|---|---|
| 3-37 | 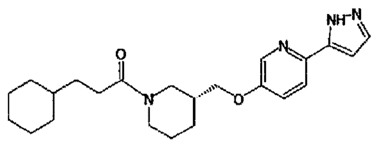 | MS ESI/APCI Multi posi: 397[M+H]+.<br>MS ESI/APCI Multi nega: 395[M-H]-. |
| 3-38 | 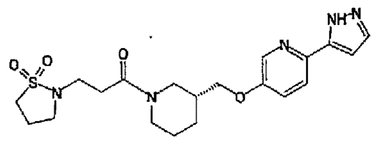 | MS ESI/APCI Multi posi: 434[M+H]+.<br>MS ESI/APCI Multi nega: 432[M-H]-. |
| 3-39 | 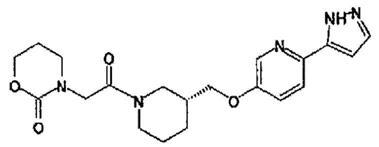 | MS ESI/APCI Multi posi : 400[M+H]+.<br>MS ESI/APCI Multi nega: 398[M-H]-. |
| 3-40 | 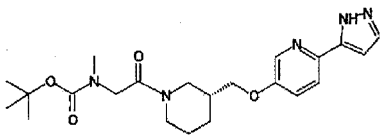 | MS ESI/APCI Multi posi : 430[M+H]+.<br>MS ESI/APCI Multi nega: 428[M-H]-. |
| 3-41 | 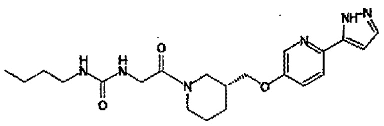 | MS ESI/APCI Multi posi: 415[M+H]+. |
| 3-42 | 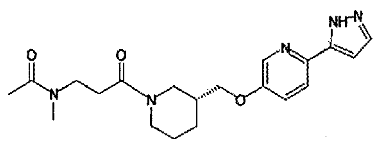 | MS ESI/APCI Multi posi: 386[M+H]+.<br>MS ESI/APCI Multi nega: 384[M-H]-. |
| 3-43 | 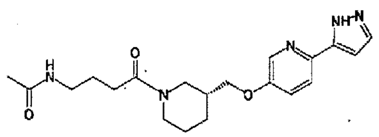 | MS ESI/APCI Multi posi : 386[M+H]+.<br>MS ESI/APCI Multi nega: 384[M-H]-. |

[Table 24-7]

| Example No. | Structure | Analytical Data |
|---|---|---|
| 3-44 | 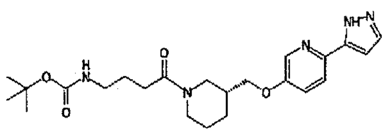 | MS ESI/APCI Multi posi: 444[M+H]+. |
| 3-45 | 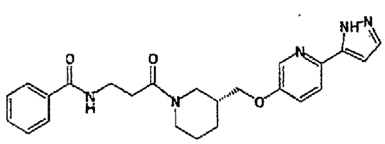 | MS ESI/APCI Multi posi: 434[M+H]+.<br>MS ESI/APCI Multi nega: 432[M-H]-. |

(continued)

| Example No. | Structure | Analytical Data |
|---|---|---|
| 3-46 | 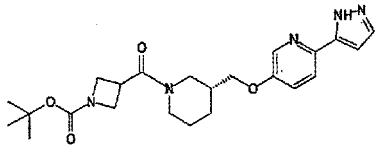 | MS ESI/APCI Multi posi : 442[M+H]$^+$. MS ESI/APCI Multi nega: 440[M-H]$^-$. |
| 3-47 | 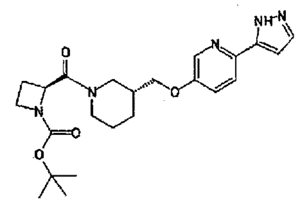 | MS ESI/APCI Multi posi: 442[M+H]$^+$. |

Example 4-1

1-[4-(2-{[6-(1H-Pyrazol-5-yl)pyridin-3-yl]oxy}ethyl)piperidin-1-yl]ethan-1-one

[Formula 201]

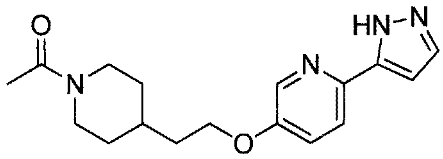

N,N-Diisopropylethylamine (94 μL), acetic acid (12 μL), and O-(7-azabenzotriazol-1-yl)-N,N,N',N'-tetramethyluronium hexafluorophosphate (HATU) (68 mg) were added to a solution of the compound (50 mg) obtained in Reference Example 14-2 in N,N-dimethylformamide (2 mL), and the mixture was stirred at room temperature overnight. Water and brine were added to the reaction solution, and the mixture was extracted with ethyl acetate. After the obtained organic layer was dried over sodium sulfate, the drying agent was filtered off, and the solvent was distilled off under reduced pressure. After the obtained residue was purified by silica gel column chromatography (n-hexane/ethyl acetate = 7:3 to 1:1 to ethyl acetate only, to chloroform/methanol = 19:1 to 9:1), the residue was powdered from diethyl ether/n-hexane to give the title compound (48 mg) as a colorless powder. $^1$H NMR (300 MHz, CHLOROFORM-d) δ ppm 1.04 - 1.37 (m, 2 H) 1.69 - 1.91 (m, 5 H) 2.10 (s, 3 H) 2.46 - 2.68 (m, 1 H) 2.95 - 3.18 (m, 1 H) 3.73 - 3.93 (m, 1 H) 4.10 (t, J=6.0 Hz, 2 H) 4.54 - 4.72 (m, 1 H) 6.70 (d, J=2.0 Hz, 1 H) 7.16 - 7.31 (m, 1 H) 7.57 - 7.72 (m, 2 H) 8.28 (d, J=2.5 Hz, 1 H).
MS ESI/APCI Multi posi: 315 [M+H]$^+$.

The compounds of the following Examples 4-2 to 4-10 were synthesized using the compound obtained in Reference Example 14-1, 14-2 or 15-1, and a corresponding carboxylic acid according to the method described in Example 4-1. The structures, NMR data, MS data of these compounds are shown in Tables 25-1 to 25-2.

[Table 25-1]

| Example No. | Structure | Analytical Data |
|---|---|---|
| 4-2 | 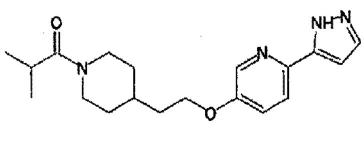 | $^1$H NMR (500 MHz, CHLOROFORM-d) δ ppm 1.05 - 1.29 (m, 8 H) 1.70 - 1.95 (m, 5 H) 2.47 - 2.64 (m, 1 H) 2.81 (spt, J=6. 7 Hz, 1 H) 3.04 (br t, J=12. 3 Hz, 1 H) 3.95 (br d, J=13. 4 Hz, 1 H) 4.10 (t, J=6.0 Hz, 2 H) 4.66 (br d, J=12. 7 Hz, 1 H) 6.70 (br s, 1 H) 7.18 - 7.32 (m, 1 H) 7. 57 - 7. 71 (m, 2 H) 8.28 (d, J=2. 7 Hz, 1 H). MS ESI/APCI Multi posi: 343[M+H]$^+$. |

(continued)

| Example No. | Structure | Analytical Data |
|---|---|---|
| 4-3 | 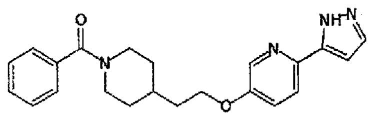 | $^1$H NMR (600 MHz, CHLOROFORM-d) δ ppm 1.16 - 1.99 (m, 7 H) 2.71 - 3.13 (m, 2 H) 3.69 - 3.88 (m, 1 H) 4.11 (t, J=6.2 Hz, 2 H) 4.67 - 4.84 (m, 1 H) 6.70 (s, 1 H) 7.20 - 7.29 (m, 1 H) 7.36 - 7.41 (m, 5 H) 7.59 - 7.70 (m, 2 H) 8.28 (d, J=2.9 Hz, 1 H). MS ESI/APCI Multi posi: 377[M+H]$^+$. |
| 4-4 | 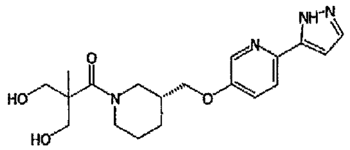 | $^1$H NMR (600 MHz, CHLOROFORM-d) δ ppm 1.10 (s, 3 H) 1.31 - 1.48 (m, 2 H) 1.65 - 1.73 (m, 1 H) 1.82 - 1.89 (m, 1 H) 1.92 - 2.01 (m, 1 H) 2.73 - 2.91 (m, 2 H) 3.50 - 3.59 (m, 4 H) 3.92 - 4.05 (m, 2 H) 4.15 - 4.20 (m, 1 H) 4.30 - 4.37 (m, 1 H) 6.74 (d, J=2.1 Hz, 1 H) 7.46 (br d, J=6.2 Hz, 1 H) 7.65 - 7.74 (m, 1 H) 7.86 (br d, J=8.7 Hz, 1 H) 8.28 (d, J=2.9 Hz, 1 H). MS ESI/APCI Multi posi: 375[M+H]$^+$. |
| 4-5 | 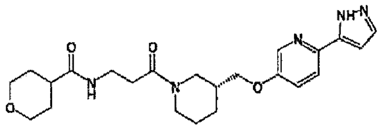 | $^1$H NMR (600 MHz, CHLOROFORM-d) δ ppm 1.38 - 1.86 (m, 8 H) 1.92 - 2.10 (m, 2 H) 2.26 - 2.32 (m, 1 H) 2.51 - 2.59 (m, 1.5 H) 2.64 - 2.69 (m, 0.5 H) 2.87 - 2.92 (m, 0.5 H) 3.03 - 3.09 (m, 0.5 H) 3.35 - 3.41 (m, 2 H) 3.53 - 3.62 (m, 2 H) 3.72 - 3.77 (m, 0.5 H) 3.85 - 4.02 (m, 4.5 H) 4. 28 - 4. 32 (m, 0.5 H) 4.60 - 4.64 (m, 0.5 H) 6.49 - 6.56 (m, 1 H) 6.68 - 6.72 (m, 1 H) 7.62 - 7.69 (m, 2 H) 8.27 - 8.30 (m, 1 H). MS ESI/APCI Multi posi: 442[M+H]$^+$. |
| 4-6 | 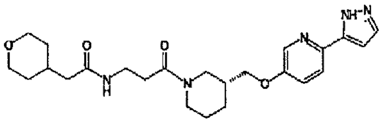 | $^1$H NMR (600 MHz, CHLOROFORM-d) δ ppm 1.26 - 1.34 (m, 2 H) 1.40 - 1.64 (m, 4 H) 1.75 - 1.86 (m, 1 H) 1.90 - 2.09 (m, 5 H) 2.51 - 2.61 (m, 2 H) 2.64 - 2.69 (m, 0.5 H) 2.82 - 2.96 (m, 0.5 H) 2.97 - 3.11 (m, 1 H) 3.36 - 3.42 (m, 2 H) 3.52 - 3.60 (m, 2 H) 3.71 - 3.77 (m, 0.5 H) 3.84 - 3.98 (m, 4.5 H) 4.29 - 4.33 (m, 0.5 H) 4.60 - 4.64 (m, 0.5 H) 6.40 - 6.48 (m, 1 H) 6. 68 - 6. 71 (m, 1 H) 7.24 - 7.26 (m, 1 H) 7.62 - 7.69 (m, 2 H) 8.27 - 8.29 (m, 1 H). MS ESI/APCI Multi posi : 456[M+H]$^+$. |
| 4-7 | 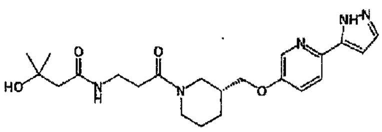 | $^1$H NMR (600 MHz, CHLOROFORM-d) δ ppm 1.24 (s, 6 H) 1. 42 - 1.58 (m, 2 H) 1.14 - 1.86 (m, 1 H) 1.91 - 2.12 (m, 2 H) 2.24 - 2.31 (m, 2 H) 2.52 - 2.70 (m, 2.5 H) 2.87 - 2.94 (m, 0.5 H) 3.04 - 3.10 (m, 1 H) 3.54 - 3.63 (m, 2 H) 3.72 - 3.78 (m, 0.5 H) 3.84 - 3.99 (m, 2.5 H) 4.26 - 4.31 (m, 0.5 H) 4.60 - 4.64 (m, 0.5 H) 6.60 - 6.74 (m, 2 H) 7.23 - 7.25 (m, 1 H) 7.62 - 7.68 (m, 2 H) 8.27 - 8.30 (m, 1 H). MS ESI/APCI Multi posi : 430[M+H]$^+$. |
| 4-8 | 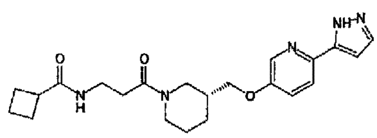 | $^1$H NMR (600 MHz, CHLOROFORM-d) δ ppm 1.41 - 1.57 (m, 2 H) 1.74 - 2.28 (m, 8 H) 2.49 - 2.61 (m, 2 H) 2.63 - 2.72 (m, 0.5 H) 2.82 - 2.90 (m, 0.5 H) 2.92 - 3.08 (m, 2 H) 3. 51 - 3.60 (m, 2 H) 3.72 - 4.00 (m, 3 H) 4.29 - 4.37 (m, 0.5 H) 4. 54 - 4.65 (m, 0.5 H) 6.25 - 6.39 (m, 1 H) 6.66 - 6.80 (m, 1 H) 7.22 - 7.26 (m, 1 H) 7.68 - 7.71 (m, 2 H) 8.18 - 8.37 (m, 1 H). MS ESI/APCI Multi posi : 412[M+H]$^+$. |

[Table 25-2]

| Example No. | Structure | Analytical Data |
|---|---|---|
| 4-9 | 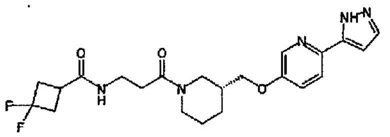 | $^1H$ NMR (600 MHz, CHLOROFORM-d) δ ppm 1.42 - 1.58 (m, 2 H) 1.74 - 1.87 (m, 1 H) 1.92 - 2.10 (m, 2 H) 2.52 - 2.58 (m, 2 H) 2.63 - 2.75 (m, 4 H) 2.79 - 2.91 (m, 2 H) 2.99 - 3.12 (m, 1 H) 3.51 - 3.64 (m, 2 H) 3.71 - 3.79 (m, 0.5 H) 3.83 - 3.99 (m, 2.5 H) 4.15 - 4.33 (m, 0.5 H) 4.51 - 4.79 (m, 0.5 H) 6.54 - 6.64 (m, 0.6 H) 6.65 - 6.75 (m, 1.6 H) 7.23 - 7.26 (m, 1 H) 7.60 - 7.71 (m, 2 H) 8.25 - 8.32 (m, 1 H).<br>MS ESI/APCI Multi posi: 448[M+H]$^+$. |
| 4-10 | 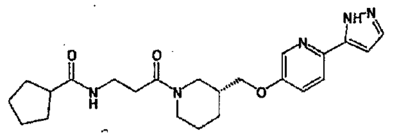 | $^1H$ NMR (600 MHz, CHLOROFORM-d) δ ppm 1.40 - 1.60 (m, 4 H) 1.67 - 1.87 (m, 7 H) 1.92 - 2.10 (m, 2 H) 2.44 - 2.62 (m, 3 H) 2.64 - 2.69 (m, 0.5 H) 2.85 - 2.92 (m, 0.5 H) 3.02 - 3.09 (m, 1 H) 3.21 (br s, 1 H) 3.51 - 3.60 (m, 2 H) 3.72 - 3.78 (m, 0.5 H) 3.84 - 4.00 (m, 2.5 H) 4.25 - 4.36 (m, 0.5 H) 4.55 - 4.67 (m, 0.5 H) 6.32 - 6.48 (m, 1 H) 6.67 - 6.75 (m, 1 H) 7.24 - 7.26 (m, 1 H) 7.58 - 7.72 (m, 2 H) 8.23 - 8.34 (m, 1 H).<br>MS ESI/APCI Multi posi: 426[M+H]$^+$. |

Example 5-1

N-(Propan-2-yl)-4-({[6-(1H-pyrazol-5-yl)pyridin-3-yl]oxy}methyl)piperdin-1-carboxamide

[Formula 202]

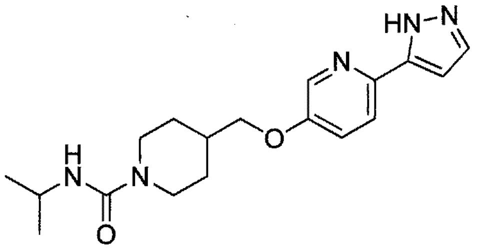

(1) N,N-Diisopropylethylamine (54 μL) and 2-isocyanatepropane (30 μL) were added to a solution of the compound (70 mg) obtained in Reference Example 2-1 in chloroform (2 mL) under ice cooling, and the mixture was stirred at room temperature for 1 hour. After the reaction solution was concentrated, the obtained residue was purified by silica gel column chromatography (n-hexane/ethyl acetate = 7:3 to ethyl acetate only, subsequently chloroform/methanol = 19:1 to 9:1) to give 4-[({6-[1-(oxan-2-yl)-1H-pyrazol-5-yl]pyridin-3-yl}oxy)methyl]-N-(propan-2-yl)piperidin-1-carboxamide (91 mg) as a colorless amorphous substance.

(2) Water (2 mL) and trifluoroacetic acid (1 mL) were added to a solution of the compound (91 mg) obtained in the above described (1) in methanol (4 mL), and the mixture was stirred at 60°C for 6 hours. An aqueous solution of saturated sodium hydrogen carbonate was added to the reaction solution, and the mixture was extracted with chloroform. The organic layer was separated by a phase separator, and the solvent was distilled off under reduced pressure. After the obtained residue was purified by NH silica gel column chromatography (n-hexane/ethyl acetate = 9:1 to 1:1, subsequently chloroform/methanol = 19:1 to 9:1), the residue was powdered from diethyl ether/n-hexane to give the title compound (57 mg) as a colorless powder.
$^1H$ NMR (600 MHz, CHLOROFORM-d) δ ppm 1.17 (d, J=6.6 Hz, 6 H) 1.35 (qd, J=12.5, 4.1 Hz, 2 H) 1.87 (br d, J=10.7 Hz, 2 H) 1.97 - 2.07 (m, 1 H) 2.81 (td, J=12.8, 2.5 Hz, 2 H) 3.90 (d, J=6.2 Hz, 2 H) 3.95 - 4.04 (m, 3 H) 4.23 (br d, J=7.0 Hz, 1 H) 6.73 (s, 1 H) 7.24 - 7.28 (m, 1 H) 7.63 (d, J=1.7 Hz, 1 H) 7.68 (br d, J=8.7 Hz, 1 H) 8.28 (d,

J=2.9 Hz, 1 H).
MS ESI/APCI Multi posi: 344 [M+H]+.

The compounds of the following Examples 5-2 to 5-3 were synthesized using the compound obtained in Reference Example 2-2 or 2-3, and a corresponding isocyanate according to the method described in Example 5-1. The structures, NMR data, MS data of these compounds are shown in Table 26-1.

[Table 26-1]

| Example No. | Structure | Analytical Data |
|---|---|---|
| 5-2 | 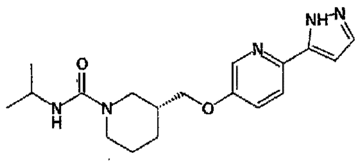 | $^1$H NMR (600 MHz, CHLOROFORM-d) δ ppm 1.10 (d, J=6.6 Hz, 3 H) 1.14 (d, J=6,6 Hz, 3 H) 1.33 - 1.49 (m, 1 H) 1.49 - 1.64 (m, 1 H) 1.66 - 1.82 (m, 1 H) 1.83 - 1.98 (m, 1 H) 1.99 - 2.15 (m, 1 H) 2.79 - 2.97 (m, 1 H) 3.02 - 3.13 (m, 1 H) 3.57 - 3.64 (m, 1 H) 3.89 - 3.99 (m, 4 H) 4.21 - 4.31 (m, 1 H) 6.71 (d, J=1.7 Hz, 1 H) 7.26 (dd, J=8.9, 3.0 Hz, 1 H) 7.63 (d, J=1.7 Hz, 1 H) 7.67 (d, J=8.9 Hz, 1 H) 8.30 (d, J=3.0 Hz, 1 H).<br>MS ESI/APCI Multi posi: 344[M+H]+. |
| 5-3 | 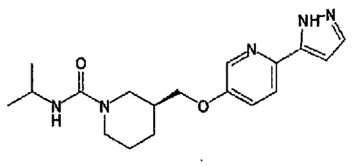 | $^1$H NMR (600 MHz, CHLOROFORM-d) δ ppm 1.10 (d, J=6.6 Hz, 3 H) 1.14 (d, J=6.6 Hz, 3 H) 1.33 - 1.49 (m, 1 H) 1.49 - 1.64 (m, 1 H) 1. 66 - 1.82 (m, 1 H) 1.83 - 1.98 (m, 1 H) 1.99 - 2.15 (m, 1 H) 2.79 - 2.97 (m, 1 H) 3.02 - 3.13 (m, 1 H) 3.57 - 3.64 (m, 1 H) 3.89 - 3.99 (m, 4 H) 4.21 - 4.31 (m, 1 H) 6.71 (d, J=1.7 Hz, 1 H) 7.26 (dd, J=8.9, 3.0 Hz, 1 H) 7.63 (d, J=1.7 Hz, 1 H) 7.67 (d, J=8.9 Hz, 1 H) 8.30 (d, J=3.0 Hz, 1 H).<br>MS ESI/APCI Multi posi: 344[M+H]+. |

Example 6-1

N,N-Dimethyl-4-({[6-(1H-pyrazol-5-yl)pyridin-3-yl]oxy}methyl)piperidin-1-carboxamide

[Formula 203]

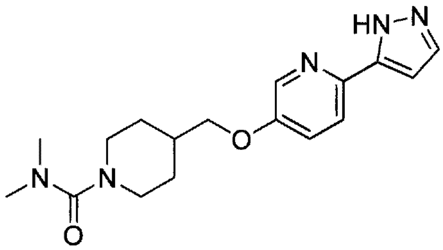

(1) After triethylamine (84 μL) and triphosgene (24 mg) were added to a solution of the compound (70 mg) obtained in Reference Example 2-1 in chloroform (2 mL) under ice cooling and the mixture was stirred at the same temperature for 10 minutes, an aqueous dimethylamine solution (50%) was added thereto, and the mixture was stirred at room temperature for 30 minutes. After the reaction solution was concentrated, the obtained residue was purified by silica gel column chromatography (n-hexane/ethyl acetate = 9:1 to 1:1, subsequently chloroform/methanol = 19:1) to give N,N-dimethyl-4-[({6-[1-(oxan-2-yl)-1H-pyrazol-5-yl]pyridin-3-yl}oxy)methyl]piperidin-1-carboxamide (76 mg) as a colorless oily substance.
(2) Water (2 mL) and trifluoroacetic acid (1 mL) were added to a solution of the compound (76 mg) obtained in the above described (1) in methanol (4 mL), and the mixture was stirred at 60°C for 6 hours. An aqueous solution of saturated sodium hydrogen carbonate was added to the reaction solution, and the mixture was extracted with

chloroform. The organic layer was separated by a phase separator, and the solvent was distilled off under reduced pressure. After the obtained residue was purified by NH silica gel column chromatography (n-hexane/ethyl acetate = 9:1 to 1:1, subsequently chloroform/methanol = 19:1 to 9:1), the residue was powdered from diethyl ether/n-hexane to give the title compound (29 mg) as a colorless powder.
$^1$H NMR (600 MHz, CHLOROFORM-d) δ ppm 1.39 (qd, J=12.4, 4.1 Hz, 2 H) 1.86 (br d, J=10.7 Hz, 2 H) 1.95 - 2.08 (m, 1 H) 2.77 - 2.87 (m, 8 H) 3.74 (br d, J=13.2 Hz, 2 H) 3.90 (d, J=6.2 Hz, 2 H) 6.72 (s, 1 H) 7.21 - 7.29 (m, 1 H) 7.63 (d, J=2.1 Hz, 1 H) 7.67 (br d, J=8.7 Hz, 1 H) 8.28 (d, J=2.9 Hz, 1 H).
MS ESI/APCI Multi posi: 330 [M+H]$^+$.

The compounds of the following Examples 6-2 to 6-5 were synthesized using any of the compound obtained in Reference Examples 2-1 to 2-3, and methylamine or isothiazolidine-1,1-dioxide according to the method described in Example 6-1. The structures, NMR data, MS data of these compounds are shown in Table 27-1.

[Table 27-1]

| Example No. | Structure | Analytical Data |
|---|---|---|
| 6-2 | 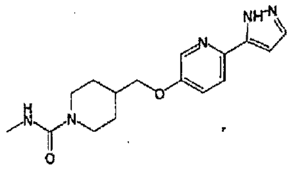 | $^1$H NMR (600 MHz, CHLOROFORM-) δ ppm 1.39 (qd, J=12.4, 4.1 Hz, 2 H) 1.86 (br d, J=10.7 Hz, 2 H) 1.95 - 2.08 (m, 1 H) 2.77 - 2.87 (m, 8 H) 3.74 (br d, J=13. 2 Hz, 2 H) 3.90 (d, J=6.2 Hz, 2 H) 6.72 (s, 1 H) 7.21 - 7.29 (m, 1 H) 7.63 (d, J=2.1 Hz, 1 H) 7.67 (br d, J=8.7 Hz, 1 H) 8.28 (d, J=2.9 Hz, 1 H).<br>MS ESI/APCI Multi posi: 330[M+H]$^+$. |
| 6-3 | 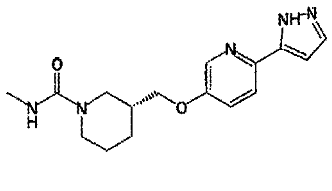 | $^1$H NMR (600 MHz, CHLOROFORM-d) δ ppm 1.33 - 1.48 (m, 1 H) 1.49 - 1.65 (m, 1 H) 1.67 - 1.83 (m, 1 H) 1.84 - 1.99 (m, 1 H) 2.03 - 2.17 (m, 1 H) 2.81 (d, J=3.7 Hz, 3 H) 2.82 - 2.87 (m, 1 H) 2.94 - 3.07 (m, 1 H) 3.68 - 3.81 (m, 1 H) 3.89 - 3.96 (m, 2 H) 3.97 - 4.01 (m, 1 H) 4.49 (br d, J=3.7 Hz, 1 H) 6.71 (d, J=2.1 Hz, 1 H) 7.26 (dd, J=8.7, 2.9 Hz, 1 H) 7.63 (d, J=2. 1 Hz, 1 H) 7.67 (d, J=8.7 Hz, 1 H) 8.29 (d, J=2.9 Hz, 1 H).<br>MS ESI/APCI Multi posi: 316[M+H]$^+$. |
| 6-4 | 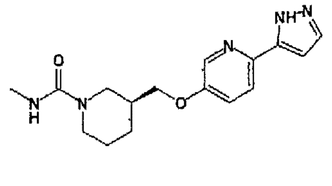 | $^1$H NMR (600 MHz, CHLOROFORM-d) δ ppm 1.34 - 1.46 (m, 1 H) 1.51 - 1.62 (m, 1 H) 1.70 - 1.78 (m, 1 H) 1.87 - 1.97 (m, 1 H) 2.04 - 2.15 (m, 1 H) 2.78 - 2.89 (m, 4 H) 3.01 (ddd, J=13.3, 10.4, 3.1 Hz, 1 H) 3.71 (dt, J=12.8, 4.1 Hz, 1 H) 3.86 - 4.03 (m, 3 H) 4.48 (br s, 1 H) 6.71 (d, J=1.7 Hz, 1 H) 7.21 - 7.33 (m, 1 H) 7.60 - 7.72 (m, 2 H) 8.29 (d, J=2.9 Hz. 1 H).<br>MS ESI/APCI Multi posi: 316[M+H]$^+$. |
| 6-5 | 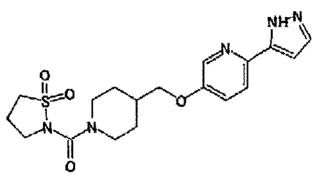 | $^1$H NMR (400 MHz, CHLOROFORM-d) δ ppm 1.47 (qd, J=12.6, 4.2 Hz, 2 H) 1.88 - 1.98 (m, 2 H) 2.03 - 2.17 (m, 1 H) 2.47 (quin, J=7.1 Hz, 2 H) 3.03 (td, J=13.0, 2.6 Hz, 2 H) 3.20 (t, J=7.5 Hz, 2 H) 3.84 (t, J=6.8 Hz, 2 H) 3.91 (d, J=6.4 Hz, 2 H) 4.37 - 4.46 (m, 2 H) 6. 68 (br s, 1 H) 7.20 - 7.28 (m, 1 H) 7.59 - 7.69 (m, 2 H) 8.27 (d, J=2.9 Hz, 1 H).<br>MS ESI/APCI Multi posi: 406[M+H]$^+$. |

Example 7-1

(Azetidin-1-yl)[4-({[6-(1H-pyrazol-5-yl)pyridin-3-yl]oxy}methyl)piperidin-1-yl]methanone

[Formula 204]

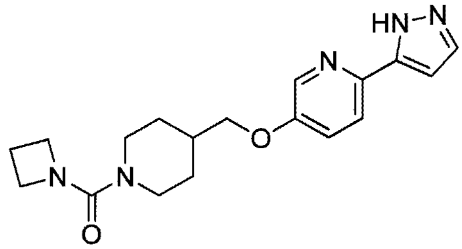

Triphosgene (35 mg) was added to a solution of azetidine hydrochloride (27 mg) and triethylamine (51 μL) in chloroform (5 mL) under ice cooling, and the mixture was stirred at the same temperature for 10 minutes. The compound (50 mg) obtained in Reference Example 2-1 and triethylamine (51 μL) were added to the reaction solution, and the mixture was stirred at room temperature for 1 hour. Water was added to the reaction solution, and the mixture was extracted with chloroform. The organic layer was separated by a phase separator, and the solvent was distilled off under reduced pressure. The obtained residue was dissolved in methanol (4 mL), water (2 mL) and trifluoroacetic acid (1 mL) were added thereto, and the mixture was stirred at room temperature for 2 days. An aqueous solution of saturated sodium hydrogen carbonate was added to the reaction solution, and the mixture was extracted with chloroform. The organic layer was separated by a phase separator, and the solvent was distilled off under reduced pressure. After the obtained residue was purified by preparative HPLC, it was solidified by diethyl ether to give the title compound (4 mg) as a colorless powder.
$^1$H NMR (600 MHz, DMSO-$d_6$) δ ppm 1.14 - 1.23 (m, 2 H) 1.72 - 1.77 (m, 2 H) 1.92 - 2.01 (m, 1 H) 2.10 - 2.16 (m, 2 H) 2.69 - 2.76 (m, 2 H) 3.73 - 3.80 (m, 2 H) 3.82 - 3.99 (m, 6 H) 6.70 - 6.75 (m, 1 H) 7.39 - 7.53 (m, 1 H) 7.72 - 7.93 (m, 2 H) 8.24 - 8.32 (m, 1 H) 12.89 (br s, 0.7 H) 13.31 (br s, 0.3 H).
MS ESI/APCI Multi posi: 342 [M+H]$^+$.

The compound of the following Example 7-2 was synthesized using the compound obtained in Reference Example 2-1, and a corresponding amine according to the method described in Example 7-1. The structure, NMR data, MS data of the compound are shown in Table 28-1.

[Table 28-1]

| Example No. | Structure | Analytical Data |
|---|---|---|
| 7-2 | 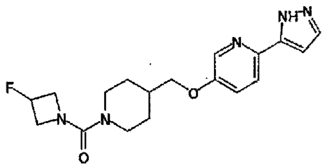 | $^1$H NMR (600 MHz, DMSO-$d_6$) δ ppm 1.15 - 1.24 (m, 2 H) 1.71 - 1.80 (m, 2 H) 1.93 - 2.02 (m, 1 H) 2.72 - 2.81 (m, 2 H) 3.74 - 3.80 (m, 2 H) 3. 89 - 3. 99 (m, 4 H) 4.14 - 4. 24 (m, 2 H) 5.26 - 5.31 (m, 0.5 H) 5.36 - 5.40 (m, 0.5 H) 6.70 - 6.76 (m, 1 H) 7.40 - 7.54 (m, 1 H) 7.72 - 7.91 (m, 2 H) 8. 24 - 8.32 (m, 1 H) 12.88 (br s, 0.7 H) 13.31 (br s, 0.3 H).<br>MS ESI/APCI Multi posi: 360[M+H]$^+$. |

Example 8-1

N-Cyclopropyl-4-({[6-(1H-pyrazol-5-yl)pyridin-3-yl]oxy}methyl)piperidin-1-carboxamide

[Formula 205]

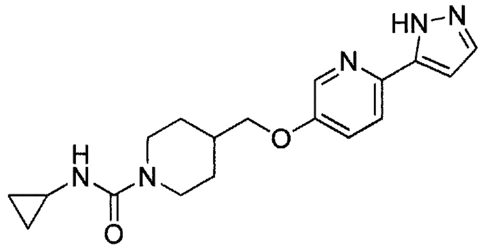

(1) Diisopropylethylamine (183 μL) and the compound (106 mg) obtained in Reference Example 29-1 were added to a solution of the compound (120 mg) obtained in Reference Example 2-1 in tetrahydrofuran (3 mL), and the mixture was stirred at 70°C for 2 hours and at room temperature for 2 days. The solvent was distilled off under reduced pressure, and the obtained residue was purified by silica gel column chromatography (chloroform only, to chloroform/methanol = 19:1) to give N-cyclopropyl-4-[({6-[1-(oxan-2-yl)-1H-pyrazol-5-yl]pyridin-3-yl}oxy)methyl]piperidin-1-carboxamide (140 mg) as a colorless oily substance.

(2) The compound (140 mg) obtained in the above described (1) was used to perform the synthesis process according to the method described in Example 1-1-(2) thereby giving the title compound (35 mg) as a colorless powder.
$^1H$ NMR (400 MHz, DMSO-$d_6$) δ ppm 0.34 - 0.38 (m, 2 H) 0.49 - 0.55 (m, 2 H) 1.09 - 1.20 (m, 2 H) 1.68 - 1.75 (m, 2 H) 1.86 - 1.99 (m, 1 H) 2.60 - 2.69 (m, 2 H) 3.91 - 3.99 (m, 4 H) 6.49 - 6.53 (m, 1 H) 6.71 - 6.74 (m, 1 H) 7.36 - 7.54 (m, 1 H) 7.70 - 7.93 (m, 2 H) 8.25 - 8.32 (m, 1 H) 12.88 (br s, 0.7 H) 13.31 (br s, 0.3 H).
MS ESI/APCI Multi posi: 342 $[M+H]^+$.

Example 8-2

N-Cyclopropyl-N-methyl-4-({[6-(1H-pyrazol-5-yl)pyridin-3-yl]oxy}methyl)piperidin-1-carboxamide

[Formula 206]

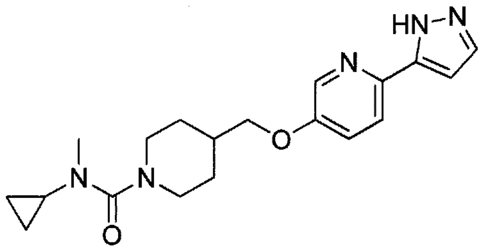

Sodium hydride (14 mg) was added to a solution of the compound (72 mg) obtained in Example 8-1-(1) in tetrahydrofuran (5 mL) under ice cooling, and the mixture was stirred at the same temperature for 5 minutes. Methyl iodide (16 μL) was added to the reaction solution, and the mixture was stirred at room temperature for 1 hour and at 70°C for 1 hour. Water was added to the reaction solution, and the mixture was extracted with chloroform. The organic layer was separated by a phase separator, and the solvent was distilled off under reduced pressure. The obtained residue was dissolved in methanol (4 mL), water (2 mL) and trifluoroacetic acid (1 mL) were added thereto, and the mixture was stirred at room temperature for 15 hours. After an aqueous solution of saturated sodium hydrogen carbonate was added to the reaction solution, the mixture was extracted with chloroform and the organic layer was separated by a phase separator, the solvent was distilled off under reduced pressure. The obtained residue was purified by silica gel column chromatography (chloroform only, to chloroform/methanol = 19:1), and then solidified by diethyl ether to give the title compound (51 mg) as a colorless powder.
$^1H$ NMR (400 MHz, DMSO-$d_6$) δ ppm 0.42 - 0.52 (m, 2 H) 0.59 - 0.68 (m, 2 H) 1.17 - 1.30 (m, 2 H) 1.71 - 1.81 (m, 2 H) 1.88 - 2.01 (m, 1 H) 2.54 - 2.60 (m, 1 H) 2.66 - 2.77 (m, 5 H) 3.69 - 3.80 (m, 2 H) 3.88 - 4.01 (m, 2 H) 6.69 - 6.75 (m, 1 H) 7.39 - 7.60 (m, 1 H) 7.65 - 7.98 (m, 2 H) 8.23 - 8.32 (m, 1 H) 12.88 (br s, 0.7 H) 13.31 (br s, 0.3 H).

MS ESI/APCI Multi posi: 356 $[M+H]^+$.

Example 9-1

N-Methyl-4-(2-1[6-(1H-pyrazol-5-yl)pyridin-3-yl]oxylethyl)piperidine-1-carboxamide

[Formula 207]

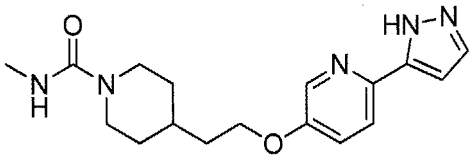

(1) Triethylamine (485 μL) and 4-nitrophenyl chloroformate (228 mg) were added to a solution of the compound (300 mg) obtained in Reference Example 14-2 in tetrahydrofuran, and the mixture was stirred at 40°C for 3 hours. The solvent was distilled off under reduced pressure, and water was added to the residue and the mixture was extracted with chloroform. The organic layer was separated by a phase separator and concentrated under reduced pressure. After the obtained residue was purified by silica gel column chromatography (n-hexane/ethyl acetate =7:3 to ethyl acetate only, subsequently chloroform/methanol = 19:1), 4-nitrophenyl 4-(2-{[6-(1H-pyrazol-5-yl)pyridin-3-yl]oxy}ethyl)piperidine-1-carboxylate (95 mg) was given as colorless powder.

(2) Triethylamine (96 μL), methylamine hydrochloride (47 mg) and N,N-dimethyl-4-aminopyridine (8.4 mg) were added to a solution of the compound (30 mg) obtained in the above described (1) in dimethylsulfoxide (2 mL), and the mixture was stirred under microwave irradiation at 100°C for 1 hour. Water was added to the reaction solution, and the mixture was extracted with ethyl acetate. After the obtained organic layer was dried over sodium sulfate, the drying agent was filtered off, and the solvent was distilled off under reduced pressure. After the obtained residue was purified by NH silica gel column chromatography (n-hexane/ethyl acetate = 6:4 to ethyl acetate only, subsequently chloroform/methanol = 19:1), the residue was powdered from diethyl ether/n-hexane to give the title compound (17 mg) as a colorless powder.

$^1$H NMR (300 MHz, CHLOROFORM-d) δ ppm 1.11 - 1.37 (m, 2 H) 1.67 - 1.87 (m, 5 H) 2.69 - 2.91 (m, 5 H) 3.86 - 4.02 (m, 2 H) 4.10 (t, J=6.1 Hz, 2 H) 4.40 (br s, 1 H) 6.63 - 6.76 (m, 1 H) 7.16 - 7.32 (m, 1 H) 7.57 - 7.71 (m, 2 H) 8.28 (d, J=3.0 Hz, 1 H).

MS ESI/APCI Multi posi: 330 $[M+H]^+$.

The compounds of the following Examples 9-2 to 9-3 were synthesized using a corresponding amines according to the method described in Example 9-1. The structures, NMR data and MS data of these compounds are shown in Table 29-1.

[Table 29-1]

| Example No. | Structure | Analytical Data |
|---|---|---|
| 9-2 | 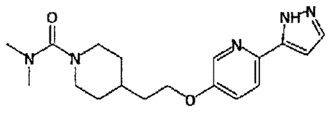 | $^1$H NMR (300 MHz, CHLOROFORM-d) δ ppm 1.19 - 1.37 (m, 2 H) 1.68 - 1.86 (m, 5 H) 2.69 - 2.86 (m, 8 H) 3.62 - 3.75 (m, 2 H) 4.10 (t, J=6. 1 Hz, 2 H) 6.69 (s, 1 H) 7.20 - 7.30 (m, 1 H) 7.59 - 7.69 (m, 2 H) 8.28 (d, J=3.0 Hz, 1 H).<br>MS ESI/APCI Multi posi: 344$[M+H]^+$. |
| 9-3 | 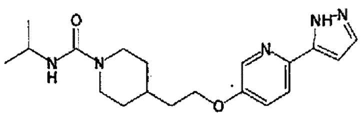 | $^1$H NMR (300 MHz, CHLOROFORM-d) δ ppm 1.16 (d, J=6.4 Hz, 6 H) 1.20 - 1.33 (m, 2 H) 1.66 - 1.86 (m, 5 H) 2.70 - 2.84 (m, 2 H) 3.87 - 4.04 (m, 3 H) 4.10 (t, J=6.0 Hz, 2 H) 4.20 (br d, J=6.8 Hz, 1 H) 6.69 (s, 1 H) 7.20 - 7.29 (m, 1 H) 7.59 - 7.70 (m, 2 H) 8.28 (d, J=2.8 Hz, 1 H).<br>MS ESI/APCI Multi posi: 358$[M+H]^+$. |

Example 10-1

5-{[1-(Methanesulfonyl)piperidin-4-yl]methoxy}-2-(1H-pyrazol-5-yl)pyridine

[Formula 208]

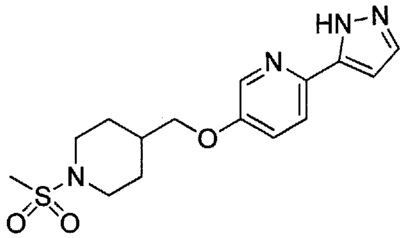

(1) Triethylamine (56 μL) and methanesulfonyl chloride (19 μL) were added to a solution of the compound (70 mg) obtained in Reference Example 2-1 in tetrahydrofuran (2 mL), and the mixture was stirred at room temperature for 1 hour. After the reaction solution was concentrated, the obtained residue was purified by silica gel column chromatography (n-hexane/ethyl acetate = 9:1 to ethyl acetate only, subsequently chloroform/methanol = 19:1 to 9:1) to give 5-{[1-(methanesulfonyl)piperidin-4-yl]methoxy}-2-[1-(oxan-2-yl)-1H-pyrazol-5-yl]pyridine (109 mg) as a colorless oily substance.

(2) Water (2 mL) and trifluoroacetic acid (1 mL) were added to a solution of the compound (109 mg) obtained the above described (1) in methanol (4 mL), and the mixture was stirred at 60°C for 3 hours. An aqueous solution of saturated sodium hydrogen carbonate was added to the reaction solution to collect a precipitated crystal by filtration, and the precipitated crystal was sequentially washed with chloroform, water, acetone and diethyl ether to give the title compound (35 mg) as a colorless powder.

$^1$H NMR (600 MHz,DMSO-$d_6$) δ ppm 1.30 - 1.44 (m, 2 H) 1.81 - 1.99 (m, 3 H) 2.74 (td, J=12.0, 2.1 Hz, 2 H) 2.86 (s, 3 H) 3.60 (br d, J=11.6 Hz, 2 H) 3.99 (br d, J=5.8 Hz, 2 H) 6.73 (d, J=2.1 Hz, 1 H) 7.46 (br s, 1 H) 7.67 - 7.97 (m, 2 H) 8.23 - 8.34 (m, 1 H). MS ESI/APCI Multi posi: 337 [M+H]$^+$.

The compounds of the following Examples 10-2 to 10-25 were synthesized using the compound obtained by Reference Examples 2-2 to 2-8, 2-12, 2-14, 7-3, 13-1 or 13-2, and a corresponding sulfonyl chloride according to the method described in Example 10-1. The structures, NMR data and MS data of these compounds are shown in Tables 30-1 to 30-4.

[Table 30-1]

| Example No. | Structure | Analytical Data |
|---|---|---|
| 10-2 | 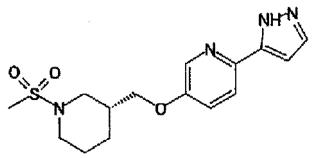 | $^1$H NMR (600 MHz, CHLOROFORM-d) δ ppm 1.35 - 1.45 (m, 1 H) 1.69 - 1.79 (m, 1 H) 1.84 - 1.95 (m, 2 H) 2.21 - 2.30 (m, 1 H) 2.73 (dd, J=11.6, 9.9 Hz, 1 H) 2.78 - 2.86 (m, 4 H) 3.63 - 3.71 (m, 1 H) 3.86 (dd, J=11.6, 3.7 Hz, 1 H) 3.93 (dd, J=9.3, 7.2 Hz, 1 H) 3.98 - 4.04 (m, 1 H) 6.72 (d, J=1. 7 Hz, 1 H) 7.24 - 7.29 (m, 1 H) 7.61 - 7.71 (m, 2 H) 8.29 (d, J=2.9 Hz, 1 H).<br>MS ESI/APCI Multi posi: 337[M+H]$^+$. |
| 10-3 | 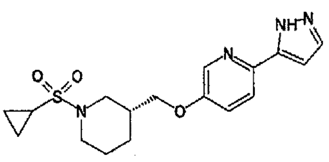 | $^1$H NMR (600 MHz, CHLOROFORM-d) δ ppm 0.93 - 1.00 (m, 2 H) 1.14 - 1.21 (m, 2 H) 1.33 - 1.44 (m, 1 H) 1.66 - 1.76 (m, 1 H) 1.80 - 1.94 (m, 2 H) 2.17 - 2.30 (m, 2 H) 2.84 (dd, J=11. 8, 9.7 Hz, 1 H) 2.93 (td, J=11. 3, 3.1 Hz, 1 H) 3.64 - 3.72 (m, 1 H) 3.83 - 3.94 (m, 2 H) 3.98 (dd, J=9.1, 5.4 Hz, 1 H) 6.68 (br s, 1 H) 7.20 - 7.28 (m, 1 H) 7.58 - 7.68 (m, 2 H) 8.27 (d, J=2.9 Hz, 1 H).<br>MS ESI/APCI Multi posi: 363[M+H]$^+$. |

(continued)

| Example No. | Structure | Analytical Data |
|---|---|---|
| 10-4 | 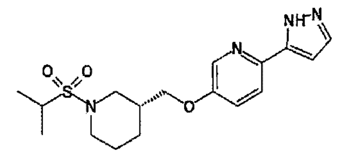 | 1H NMR (600 MHz, CHLOROFORM-d) δ ppm 1.35 (dd, J=6.8, 1.4 Hz, 6 H) 1.38 - 1.47 (m, 1 H) 1.63 - 1.72 (m, 1 H) 1.78 - 1.85 (m, 1 H) 1.89 - 1.96 (m, 1 H) 2.14 - 2.22 (m, 1 H) 2.92 (dd, J=12.4, 9.9 Hz, 1 H) 2.97 - 3.03 (m, 1 H) 3.20 (spt, J=6.8 Hz, 1 H) 3.68 - 3.74 (m, 1 H) 3.87 - 3.93 (m, 2 H) 3.95 - 3.99 (m, 1 H) 6.69 (br s, 1 H) 7.23 - 7.28 (m, 1 H) 7.61 - 7.68 (m, 2 H) 8.28 (d, J=2.9 Hz, 1 H).<br>MS ESI/APCI Multi posi: 365[M+H]+. |
| 10-5 | 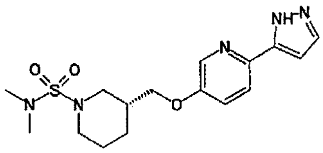 | 1H NMR (600 MHz, CHLOROFORM-d) δ ppm 1.33 - 1.42 (m, 1 H) 1.63 - 1.72 (m, 1 H) 1.78 - 1.85 (m, 1 H) 1.86 - 1.93 (m, 1 H) 2.15 - 2.24 (m, 1 H) 2.80 - 2.86 (m, 7 H) 2.89 - 2.96 (m, 1 H) 3.58 (dt, J=12.3, 4.0 Hz, 1 H) 3.77 (dd, J=12.2, 3.9 Hz, 1 H) 3.89 - 3.94 (m, 1 H) 3.94 - 3.99 (m, 1 H) 6.69 (br s, 1 H) 7.22 - 7.28 (m, 1 H) 7.61 - 7.69 (m, 2 H) 8.28 (d, J=2.9 Hz, 1 H).<br>MS ESI/APCI Multi posi: 366[M+H]+. |
| 10-6 | 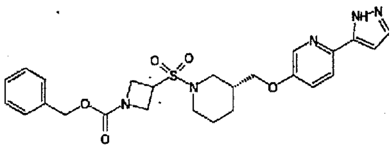 | 1H NMR (600 MHz, CHLOROFORM-d) δ ppm 1. 34 - 1. 44 (m, 1 H) 1.60 - 1.70 (m, 1 H) 1.79 - 1.86 (m, 1 H) 1.87 - 1.94 (m, 1 H) 2.11 - 2.19 (m, 1 H) 2.84 (dd, J=12.2, 10.1 Hz, 1 H) 2.88 - 2.95 (m, 1 H) 3.72 (br d, J=12.4 Hz, 1 H) 3.84 - 4.00 (m, 4 H) 4.21 - 4.27 (m, 2 H) 4.29 - 4.35 (m, 2 H) 5.11 (s, 2 H) 6.69 (br s, 1 H) 7.21 - 7.28 (m, 1 H) 7.30 - 7.39 (m, 5 H) 7.60 - 7.69 (m, 2 H) 8.27 (d, J=2.9 Hz, 1 H).<br>MS ESI/APCI Multi posi: 512[M+H]+. |
| 10-7 | 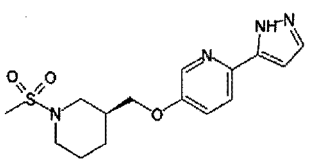 | 1H NMR (600 MHz, CHLOROFORM-d) δ ppm 1.36 - 1.44 (m, 1 H) 1.69 - 1.78 (m, 1 H) 1.84 - 1.95 (m, 2 H) 2.21 - 2.30 (m, 1 H) 2.74 (dd, J=11.6, 9.9 Hz, 1 H) 2.78 - 2.85 (m, 4 H) 3.64 - 3.70 (m, 1 H) 3.86 (dd, J=11.6, 3.7 Hz, 1 H) 3.91 - 3.95 (m, 1 H) 3.98 - 4.03 (m, 1 H) 6.73 (s, 1 H) 7.25 - 7.29 (m, 1 H) 7.63 (d, J=2.1 Hz, 1 H) 7.68 (d, J=8.7 Hz, 1 H) 8.29 (d, J=2.9 Hz, 1 H).<br>MS ESI/APCI Multi posi: 337[M+H]+. |

[Table 30-2]

| Example No. | Structure | Analytical Data |
|---|---|---|
| 10-8 | 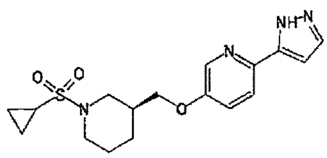 | 1H NMR (600 MHz, CHLOROFORM-d) δ ppm 0.96 - 1.01 (m, 2 H) 1.16 - 1.22 (m, 2 H) 1.36 - 1.44 (m, 1 H) 1.67 - 1.76 (m, 1 H) 1.81 - 1.94 (m, 2 H) 2.19 - 2.31 (m, 2 H) 2.85 (dd, J=11.6, 9.9 Hz, 1 H) 2.94 (td, J=11.3, 3.1 Hz, 1 H) 3.65 - 3.73 (m, 1 H) 3.86 - 3.95 (m, 2 H) 3.97 - 4.02 (m, 1 H) 6.69 (br s, 1 H) 7.22 - 7.28 (m, 1 H) 7.60 - 7.70 (m, 2 H) 8.28 (d, J=2.9 Hz, 1 H).<br>MS ESI/APCI Multi posi: 363[M+H]+. |
| 10-9 | 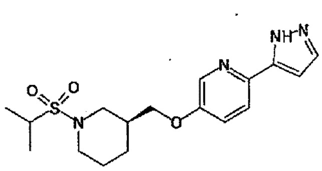 | 1H NMR (600 MHz, CHLOROFORM-d) δ ppm 1.33 - 1.37 (m, 6 H) 1.38 - 1.47 (m, 1 H) 1.63 - 1.72 (m, 1 H) 1.78 - 1.84 (m, 1 H) 1.89 - 1.96 (m, 1 H) 2.14 - 2.23 (m, 1 H) 2.92 (dd, J=12.6, 9.7 Hz, 1 H) 2.97 - 3.04 (m, 1 H) 3.20 (spt, J=6.8 Hz, 1 H) 3.68 - 3.74 (m, 1 H) 3.87 - 3.93 (m, 2 H) 3.94 - 3.99 (m, 1 H) 6.69 (br s, 1 H) 7.23 - 7.28 (m, 1 H) 7. 60 - 7.68 (m, 2 H) 8.28 (d, J=2.9 Hz, 1 H).<br>MS ESI/APCI Multi posi: 365[M+H]+. |

(continued)

| Example No. | Structure | Analytical Data |
|---|---|---|
| 10-10 | 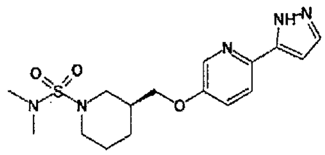 | 1H NMR (600 MHz, CHLOROFORM-d) δ ppm 1.32 - 1.43 (m, 1 H) 1.61 - 1.73 (m, 1 H) 1.77 - 1.85 (m, 1 H) 1.86 - 1.94 (m, 1 H) 2.15 - 2.25 (m, 1 H) 2.78 - 2.87 (m, 7 H) 2.89 - 2.96 (m, 1 H) 3.55 - 3.62 (m, 1 H) 3.77 (dd, J=12. 2, 3.9 Hz, 1 H) 3.88 - 4.00 (m, 2 H) 6.69 (br s, 1 H) 7.21 - 7.30 (m, 1 H) 7.59 - 7.70 (m, 2 H) 8.28 (d, J=2.9 Hz, 1 H).<br>MS ESI/APCI Multi posi: 366[M+H]+. |
| 10-11 | 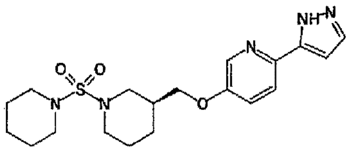 | 1H NMR (600 MHz, CHLOROFORM-d) δ ppm 1.31 - 1.41 (m, 1 H) 1.50 - 1.70 (m, 7 H) 1.77 - 1.84 (m, 1 H) 1.85 - 1.92 (m, 1 H) 2.14 - 2.22 (m, 1 H) 2.81 (dd, J=12.0, 9.9 Hz, 1 H) 2.87 - 2.94 (m, 1 H) 3.17 - 3.24 (m, 4 H) 3.55 - 3.61 (m, 1 H) 3.77 (dd, J=12.2, 3.5 Hz, 1 H) 3.89 - 3.99 (m, 2 H) 6.69 (br s, 1 H) 7.21 - 7.29 (m, 1 H) 7.60 - 7.69 (m, 2 H) 8.27 (d, J=2.5 Hz, 1 H).<br>MS ESI/APCI Multi posi: 406[M+H]+. |
| 10-12 | 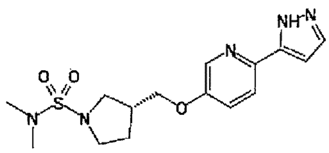 | 1H NMR (600 MHz, CHLOROFORM-d) δ ppm 1.82 - 1.95 (m, 1 H) 2.13 - 2.23 (m, 1 H) 2.76 - 2.83 (m, 1 H) 2.84 (s, 6 H) 3.23 - 3.28 (m, 1 H) 3.35 - 3.42 (m, 1 H) 3.46 - 3.52 (m, 1 H) 3.55 - 3.61 (m, 1 H) 3.97 - 4.02 (m, 1 H) 4.02 - 4.07 (m, 1 H) 6.70 (d, J=2.1 Hz, 1 H) 7.25 (dd, J=8.9, 2.7 Hz, 1 H) 7.63 (d, J=2.1 Hz, 1 H) 7.67 (d, J=8.9 Hz, 1 H) 8.30 (d, J=2.7 Hz, 1 H) 10.96 (br s, 1 H).<br>MS ESI/APCI Multi posi: 352[M+H]+. |
| 10-13 | 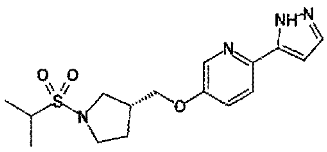 | 1H NMR (600 MHz, CHLOROFORM-d) δ ppm 1.38 (d, J=6.8 Hz, 3 H) 1.39 (d, J=6.8 Hz, 3 H) 1.84 - 1. 96 (m, 1 H) 2.15 - 2.22 (m, 1 H) 2.75 - 2.84 (m, 1 H) 3.25 (spt, J=6.8 Hz, 1 H) 3.32 - 3.39 (m, 1 H) 3.42 - 3.52 (m, 1 H) 3.54 - 3.63 (m, 1 H) 3.63 - 3.70 (m, 1 H) 3. 95 - 4. 03 (m, 1 H) 4. 03 - 4. 08 (m, 1 H) 6.70 (d, J=1.7 Hz, 1 H) 7.25 (dd, J=8.5, 2.7 Hz, 1 H) 7.63 (d, J=1.7 Hz, 1 H) 7.66 (br d, J=8.5 Hz, 1 H) 8.28 (d, J=2.7 Hz, 1 H) 10.74 (br s, 1 H).<br>MS ESI/APCI Multi posi: 351[M+H]+. |

[Table 30-3]

| Example No. | Structure | Analytical Data |
|---|---|---|
| 10-14 | 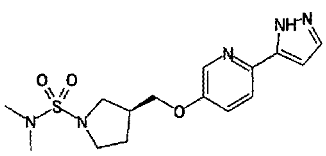 | 1H NMR (600 MHz, CHLOROFORM-d) δ ppm 1.82 - 1.95 (m, 1 H) 2.13 - 2.23 (m, 1 H) 2.76 - 2.83 (m, 1 H) 2.84 (s, 6 H) 3.23 - 3.28 (m, 1 H) 3.35 - 3. 42 (m, 1 H) 3.46 - 3.52 (m, 1 H) 3.55 - 3.61 (m, 1 H) 3.97 - 4.02 (m, 1 H) 4.02 - 4.07 (m, 1 H) 6.70 (d, J=2.1 Hz, 1 H) 7.25 (dd, J=8.9, 2.7 Hz, 1 H) 7.63 (d, J=2.1 Hz, 1 H) 7.67 (d, J=8.9 Hz, 1 H) 8.30 (d, J=2.7 Hz, 1 H) 10.96 (br s, 1 H).<br>MS ESI/APCI Multi posi: 352[M+H]+. |
| 10-15 | 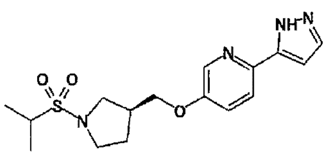 | 1H NMR (600 MHz, CHLOROFORM-d) δ ppm 1.38 (d, J=6.8 Hz, 3 H) 1.39 (d, J=6. 8 Hz, 3 H) 1.84 - 1.95 (m, 1 H) 2.15 - 2.22 (m, 1 H) 2.75 - 2.84 (m, 1 H) 3.25 (spt, J=6.8 Hz, 1 H) 3.32 - 3.39 (m, 1 H) 3.42 - 3.52 (m, 1 H) 3.54 - 3.63 (m, 1 H) 3.63 - 3.70 (m, 1 H) 3.95 - 4.03 (m, 1 H) 4.03 - 4.08 (m, 1 H) 6.70 (d, J=1.7 Hz, 1 H) 7.25 (dd, J=8.5, 2.7 Hz, 1 H) 7.63 (d, J=1. 7 Hz, 1 H) 7.66 (br d, J=8.5 Hz, 1 H) 8.28 (d, J=2.7 Hz, 1 H) 10.74 (br s, 1 H).<br>MS ESI/APCI Multi posi: 351 [M+H]+. |

(continued)

| Example No. | Structure | Analytical Data |
|---|---|---|
| 10-16 | 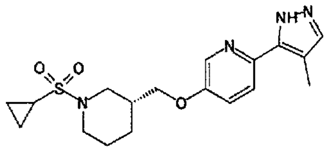 | $^1$H NMR (600 MHz, DMS0-$d_6$) δ ppm 0.89 - 0.99 (m, 4 H) 1.23 - 1.32 (m, 1 H) 1.49 - 1. 60 (m, 1 H) 1.73 - 1.87 (m, 2 H) 2.03 - 2. 12 (m, 1 H) 2.29 (s, 3 H) 2.56 - 2.62 (m, 1 H) 2.78 (dd, J=11.6, 10.3 Hz, 1, H) 2.87 (td, J=11.5, 2.7 Hz, 1 H) 3.49 - 3.55 (m, 1 H) 3.71 (dd, J=11.6, 3.7 Hz, 1 H) 3.96 - 4.08 (m, 2 H) 7.37 - 7.57 (m, 2 H) 7.66 - 7.89 (m, 1 H) 8.27 - 8.39 (m, 1 H).<br>MS ESI/APCI Multi posi: 377 $[M+H]^+$. |
| 10-17 | 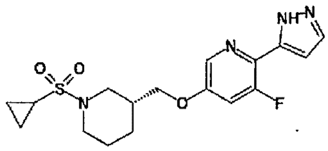 | $^1$H NMR (600 MHz, DMS0-$d_6$) δ ppm 0.85 - 0.97 (m, 4 H) 1.19 - 1.29 (m, 1 H) 1.47 - 1.56 (m, 1 H) 1.71 - 1.84 (m, 2 H) 2.00 - 2.10 (m, 1 H) 2.52 - 2.60 (m, 1 H) 2.75 (t, J=10.9 Hz, 1 H) 2.83 (td, J=11.6, 2.9 Hz, 1 H) 3.45 - 3.52 (m, 1 H) 3.64 - 3.70 (m, 1 H) 3.97 - 4.08 (m, 2 H) 6.65 (br s, 1 H) 7.44-7.77 (m, 2 H) 8.22 (s, 1 H).<br>MS ESI/APCI Multi posi: 381 $[M+H]^+$. |
| 10-18 | 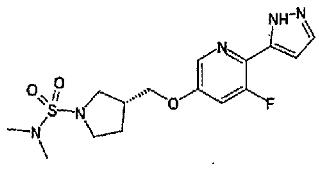 | $^1$H NMR (400 MHz, DMS0-$d_6$) δ ppm 1.73 - 1.83 (m, 1 H) 2.05 - 2.14 (m, 1 H) 2.67 - 2.78 (m, 7 H) 3.08 - 3.16 (m, 1 H) 3.22 - 3.50 (m, 3 H) 4.06 - 4.18 (m, 2 H) 6.69 (s, 1 H) 7.41 - 7.92 (m, 2 H) 8.26 (s, 1 H) 12.94 - 13.53 (m, 1 H).<br>MS ESI/APCI Multi posi: 370 $[M+H]^+$ . |
| 10-19 | 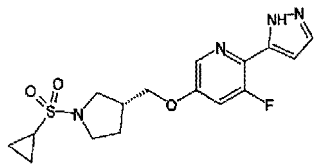 | $^1$H NMR (400 MHz, DHS0-$d_6$) δ ppm 0.91 - 1. 01 (m, 4 H) 1.74 - 1.84 (m, 1 H) 2.07 - 2.16 (m, 1 H) 2.66 - 2.81 (m, 2 H) 3.15 - 3.22 (m, 1 H) 3.33 - 3.55 (m, 3 H) 4.07 - 4.20 (m, 2 H) 6.65 - 6.72 (m, 1 H) 7.47 - 7.86 (m, 2 H) 8.26 (s, 1 H) 12.87 - 13.49 (m, 1 H).<br>MS ESI/APCI Multi posi: 367 $[M+H]^+$. |
| 10-20 | 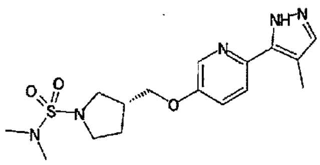 | $^1$H NMR (400 MHz, DMS0-$d_6$) δ ppm 1. 74 - 1. 84 (m, 1 H) 2. 05 - 2.15 (m, 1 H) 2.24 - 2.38 (m, 3 H) 2.66 - 2.77 (m, 7 H) 3.09 - 3.16 (m, 1 H) 3.23 - 3.48 (m, 3 H) 4.03 - 4.14 (m, 2 H) 7. 28 - 7. 94 (m, 3 H) 8. 26 - 8. 40 (m, 1 H) 12.53 - 13. 09 (m, 1 H).<br>MS ESI/APCI Multi posi: 366 $[M+H]^+$ |

[Table 30-4]

| Example No. | Structure | Analytical Data |
|---|---|---|
| 10-21 | 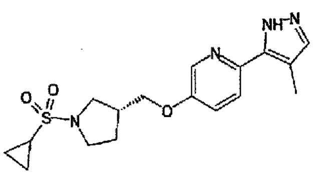 | $^1$H NMR (400 MHz, DMS0-$d_6$) δ ppm 0.90 - 1.00 (m, 4 H) 1. 75 - 1.85 (m, 1 H) 2.06 - 2.16 (m, 1 H) 2.24 - 2.35 (m, 3 H) 2.66 - 2.78 (m, 2 H) 3.16 - 3.22 (m, 1 H) 3.32 - 3.55 (m, 3 H) 4.04 - 4.15 (m, 2 H) 7.32 - 7.92 (m, 3 H) 8.28 - 8.40 (m, 1 H) 12.60 (br s, 0.6 H) 13.00 (br s, 0.4 H).<br>MS ESI/APCI Multi posi: 363 $[M+H]^+$. |
| 10-22 | 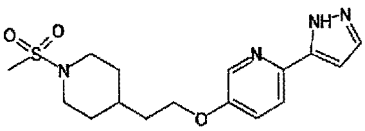 | $^1$H NMR (300 MHz, CHLOROFORM-d) δ ppm 1.32 - 1.94 (m, 7 H) 2.68 (td, J=12.0, 2.7 Hz, 2 H) 2.78 (s, 3 H) 3.78 - 3.88 (m, 2 H) 4.11 (t, J=6.1 Hz, 2 H) 6.69 (d, J=2.0 Hz, 1 H) 7.20 - 7.29 (m, 1 H) 7.60 - 7.69 (m, 2 H) 8.28 (d, J=3.0 Hz, 1 H).<br>MS ESI/APCI Multi posi: 351 $[M+H]^+$. |

(continued)

| Example No. | Structure | Analytical Data |
|---|---|---|
| 10-23 | 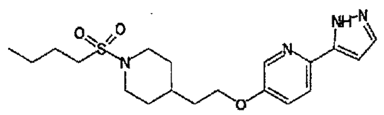 | $^1H$ NMR (600 MHz, CHLOROFORM-d) δ ppm 0.95 (t, J=7.4 Hz, 3 H) 1. 34 - 1.49 (m, 3 H) 1.67 - 1.88 (m, 8 H) 2. 78 (td, J=12. 3, 2.3 Hz, 2 H) 2.87 - 2.92 (m, 2 H) 3.80 - 3.86 (m, 2 H) 4.10 (t, J=6.2 Hz, 2 H) 6.69 (br s, 1 H) 7.21 - 7.28 (m, 1 H) 7.61 - 7.68 (m, 2 H) 8.28 (d, J=2.9 Hz, 1 H).<br>MS ESI/APCI Multi posi: 393 $[M+H]^+$. |
| 10-24 | 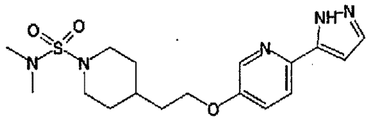 | $^1H$ NMR (600 MHz, CHLOROFORM-d) δ ppm 1. 30 - 1.41 (m, 2 H) 1.64 - 1.76 (m, 1 H) 1.77 - 1.87 (m, 4 H) 2. 79 - 2.88 (m, 8 H) 3.71 (br d, J=12.4 Hz, 2 H) 4.10 (t, J=6.2 Hz, 2 H) 6.69 (br s, 1 H) 7.20 - 7.27 (m, 1 H) 7.60 - 7.69 (m, 2 H) 8.28 (d, J=2.5 Hz, 1 H).<br>MS ESI/APCI Multi posi: 380 $[M+H]^+$. |
| 10-25 | 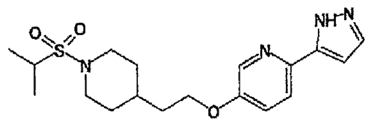 | $^1H$ NMR (600 MHz, CHLOROFORM-d) δ ppm 1.31 - 1.40 (m, 8 H) 1.69 - 1.84 (m, 5 H) 2.89 (td, J=12.5, 2.3 Hz, 2 H) 3.13 - 3.22 (m, 1 H) 3.80 - 3.88 (m, 2 H) 4.10 (t, J=6.0 Hz, 2 H) 6.69 (br s, 1 H) 7.20 - 7.28 (m, 1 H) 7.60 - 7.68 (m, 2 H) 8.27 (d, J=2. 9 Hz, 1 H).<br>MS ESI/APCI Multi posi: 379 $[M+H]^+$. |

Example 11-1

5-{[(3R)-1-(Piperidine-1-sulfonyl)piperidin-3-yl]methoxy}-2-(1H-pyrazol-5-yl)pyridine

[Formula 209]

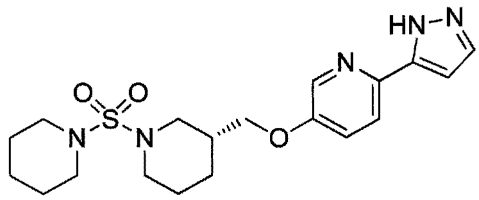

(1) Piperidine (242 μL) was added to a solution of 1-(1H-imidazole-1-sulfonyl)-3-methyl-1H-imidazolium trifluoromethanesulfonate (319 mg) in acetonitrile (3 mL), and the mixture was stirred at room temperature overnight. After the reaction solution was concentrated, the obtained residue was purified by silica gel column chromatography (n-hexane/ethyl acetate =9:1 to ethyl acetate only) to give 1-(1H-imidazole-1-sulfonyl)piperidine (73 mg) as a colorless powder.

(2) Methyl trifluoromethanesulfonate (30 μL) was added to a solution of the compound (39 mg) obtained in the above described (1) in chloroform (2 mL), and the mixture was stirred at room temperature for 30 minutes. The reaction solution was concentrated and then dried under reduced pressure, and N,N-diisopropylethylamine (42 μL) and the compound (42 mg) obtained in Reference Example 2-2 were added to a solution of the obtained residue in acetonitrile (2 mL), and the mixture was stirred at room temperature for 30 minutes. After the reaction solution was concentrated, the obtained residue was purified by silica gel column chromatography (n-hexane/ethyl acetate = 9:1 to ethyl acetate only, subsequently chloroform/methanol = 19:1) to give 2-[1-(oxan-2-yl)-1H-pyrazol-5-yl]-5-{[(3R)-1-(piperidine-1-sulfonyl)piperidin-3-yl]methoxy}pyridine (68.6 mg) as a colorless amorphous substance.

(3) Water (2 mL) and trifluoroacetic acid (1 mL) were added to a solution of the compound (68.6 mg) obtained in the above described (2) in methanol (4mL), and the mixture was stirred at room temperature overnight. An aqueous solution of saturated sodium hydrogen carbonate was added to the reaction solution, and the mixture was extracted

with chloroform. The organic layer was separated by a phase separator, and the solvent was distilled off under reduced pressure. The obtained residue was purified by NH silica gel column chromatography (n-hexane only to n-hexane/ethyl acetate =1:1 to ethyl acetate only, subsequently chloroform/methanol = 19:1 to 9:1) to give the title compound (46.9 mg) as a colorless amorphous substance.
$^1$H NMR (600 MHz, CHLOROFORM-d) δ ppm 1.31 - 1.41 (m, 1 H) 1.50 - 1.72 (m, 7 H) 1.77 - 1.83 (m, 1 H) 1.86 - 1.92 (m, 1 H) 2.14 - 2.23 (m, 1 H) 2.81 (dd, J=12.0, 9.9 Hz, 1 H) 2.87 - 2.94 (m, 1 H) 3.14 - 3.24 (m, 4 H) 3.55 - 3.61 (m, 1 H) 3.77 (dd, J=12.0, 3.7 Hz, 1 H) 3.89 - 3.99 (m, 2 H) 6.69 (br s, 1 H) 7.22 - 7.27 (m, 1 H) 7.61 - 7.69 (m, 2 H) 8.28 (d, J=2.5 Hz, 1 H).
MS ESI/APCI Multi posi: 406 [M+H]$^+$.

The compounds of the following Examples 11-2 to 11-3 were synthesized using the compound obtained in Reference Example 2-2, and a corresponding amine according to the method described in Example 11-1. The structures, NMR data and MS data of these compounds are shown in Table 31-1.

[Table 31-1]

| Example No. | Structure | Analytical Data |
|---|---|---|
| 11-2 | 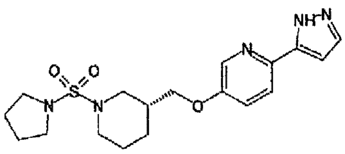 | $^1$H NMR (600 MHz, CHLOROFORM-d) δ ppm 1.32 - 1.41 (m, 1 H) 1.63 - 1.73 (m, 1 H) 1.78 - 1.84 (m, 1 H) 1.85 - 1.94 (m, 5 H) 2.15 - 2.25 (m, 1 H) 2.82 (dd, J=12.2, 9.7 Hz, 1 H) 2.88 - 2.95 (m, 1 H) 3.27 - 3.36 (m, 4 H) 3.55 - 3.62 (m, 1 H) 3.76 (dd, J=12.0, 3.7 Hz, 1 H) 3.90 - 3.99 (m, 2 H) 6.69 (br s, 1 H) 7.22 - 7.28 (m, 1 H) 7.60 - 7.69 (m, 2 H) 8.27 (d, J=2.9 Hz, 1 H).<br>MS ESI/APCI Multi posi: 392 [M+H]$^+$. |
| 11-3 | 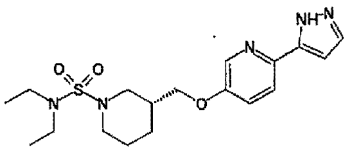 | $^1$H NMR (600 MHz, CHLOROFORM-d) δ ppm 1.18 (t, J=7.2 Hz, 6 H) 1.29 - 1.39 (m, 1 H) 1.61 - 1.71 (m, 1 H) 1.78 - 1.84 (m, 1 H) 1.85 - 1.91 (m, 1 H) 2.14 - 2.23 (m, 1 H) 2.74 (dd, J=12.0, 9.9 Hz, i H) 2.84 (td, J=11.6, 2.9 Hz, 1 H) 3.27 (q, J=7.2 Hz, 4 H). 3.49 - 3.56 (m, 1 H) 3.72 (dd, J=12. 0, 3.7 Hz, 1 H) 3.88 - 3.99 (m, 2 H) 6.69 (br s, 1 H) 7.21 - 7.26 (m, 1 H) 7.60 - 7.70 (m, 2 H) 8. 27 (d, J=2.9 Hz, 1 H).<br>MS ESI/APCI Multi posi: 394 [M+H]$^+$. |

Example 12-1

1-Methylcyclopropyl {3-oxo-3-[(3R)-3-({[6-(1H-pyrazol-5-yl)pyridin-3-yl]oxy}methyl)piperidin-1-yl]propyl}carbamate

[Formula 210]

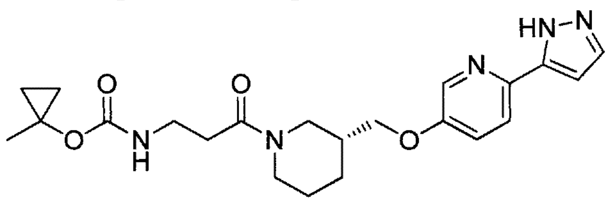

Triethylamine (20 μL) and the compound (19 mg) obtained in Reference Example 30-1 were added to a solution of the compound (30 mg) obtained in Reference Example 10-1 in chloroform (2 mL), and the mixture was stirred at room temperature overnight. An aqueous solution of saturated sodium hydrogen carbonate was added to the reaction solution, and the mixture was extracted with chloroform. The organic layer was separated by a phase separator, and the solvent was distilled off under reduced pressure. The obtained residue was purified with a silica gel column chromatography (n-hexane only to n-hexane/ethyl acetate = 1:1 to ethyl acetate only, subsequently chloroform/methanol = 9:1), and the fractions containing the target substance were collected. The solvent was distilled off under reduced pressure, and water (2 mL) and trifluoroacetic acid (1 mL) were added to a solution of the obtained residue in methanol (4 mL), and the

mixture was stirred at room temperature overnight. An aqueous solution of saturated sodium hydrogen carbonate was added to the reaction solution, and the mixture was extracted with chloroform. The organic layer was separated by a phase separator, and the solvent was distilled off under reduced pressure. After the obtained residue was purified by NH silica gel column chromatography (n-hexane only to n-hexane/ethyl acetate = 1:1 to ethyl acetate only, subsequently chloroform/methanol = 19:1 to 9:1), purification was performed by preparative HPLC to give the title compound (13 mg) as a colorless amorphous substance.
$^1$H NMR (600 MHz, CHLOROFORM-d) δ ppm 0.51 - 0.66 (m, 2 H) 0.79 - 0.92 (m, 2 H) 1.38 - 1.60 (m, 5 H) 1.72 - 1.86 (m, 1 H) 1.88 - 2.12 (m, 2 H) 2.48 - 2.72 (m, 3 H) 3.00 - 3.13 (m, 1 H) 3.40 - 3.53 (m, 2 H) 3.71 - 4.01 (m, 3 H) 4.25 - 4.38 (m, 0.5 H) 4.57 - 4.66 (m, 0.5 H) 5.39 (br s, 1 H) 6.70 (br d, J=11.1 Hz, 1 H) 7.20 - 7.30 (m, 1 H) 7.59 - 7.72 (m, 2 H) 8.29 (br d, J=4.1 Hz, 1 H).
MS ESI/APCI Multi posi: 428 $[M+H]^+$.

Example 13-1

1-[4-Fluoro-4-({[6-(1H-pyrazol-5-yl)pyridin-3-yl]oxylmethyl)piperidin-1-yl]ethan-1-one

[Formula 211]

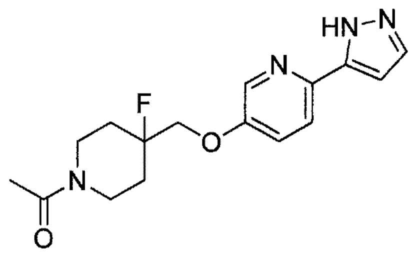

(1) The compound (70 mg) obtained in Reference Example 1-1, the compound (55 mg) obtained in Reference Example 25-5, and a suspension of cyanomethylenetributylphosphorane (150 μl) in toluene (1.4 ml) were stirred at 100°C for 3 hours. The reaction solution was cooled to room temperature and concentrated under reduced pressure. The obtained residue was purified by NH silica gel column chromatography (n-hexane/ethyl acetate = 7:13 to 1:4) to give 1-[4-fluoro-4-({6-[2-(oxan-2-yl)pyrazol-3-yl]pyridin-3-yl)oxymethyl)piperidin-1-yl]ethanone (115 mg) as a brown oily substance.
(2) The compound obtained in the above described (1) was used to perform the synthesis process according to the method described in Example 1-1 (2) thereby giving the title compound (47 mg) as a colorless amorphous substance.
$^1$H NMR (600 MHz, CHLOROFORM-d) δ ppm 1.62 - 1.80 (m, 2 H) 1.91 - 1.99 (m, 1 H) 2.00 - 2.10 (m, 1 H) 2.07 (s, 3 H) 2.87 - 2.95 (m, 1 H) 3.36 - 3.44 (m, 1 H) 3.67 - 3.74 (m, 1 H) 3.92 - 4.05 (m, 2 H) 4.48 - 4.56 (m, 1 H) 6.64 (s, 1 H) 7.18 - 7.24 (m, 1 H) 7.54 - 7.57 (m, 1 H) 7.58 - 7.65 (m, 1 H) 8.21 - 8.27 (m, 1 H).
MS ESI/APCI Multi posi: 319 $[M+H]^+$.

The compounds of the following Examples 13-2 to 13-13 and 13-15 to 13-18 were synthesized using the compound obtained by Reference Example 1-1, 1-4 or 1-5 and the alcohol obtained by Reference Examples 25 to 28 or 68 according to the method described in Example 13-1. The structures, NMR data and MS data of these compounds are shown in Tables 32-1 to 32-3.

[Table 32-1]

| Example No. | Structure | Analytical Data |
|---|---|---|
| 13-2 | 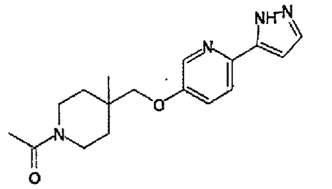 | $^1$H NMR (600 MHz, CHLOROFORM-d) δ ppm 1.17 (s, 3 H) 1.42 - 1.74 (m, 4 H) 2. 10 (s, 3 H) 3.14 - 3.22 (m, 1 H) 3.32 - 3.41 (m, 1 H) 3.55 - 3.64 (m, 1 H) 3.72 - 3.81 (m, 2 H) 4.07 - 4.15 (m, 1 H) 6.53 - 6.73 (m, 1 H) 7.19 - 7.28 (m, 1 H) 7.58 - 7.70 (m, 2 H) 8.24 - 8.32 (m, 1 H).<br>MS ESI/APCI Multi posi: 315 [M+H]$^+$. |
| 13-3 | 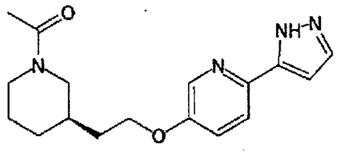 | $^1$H NMR (600 MHz, CHLOROFORM-d) δ ppm 1. 21 - 1. 31 (m, 1 H) 1.41 - 1.86 (m, 5 H) 1.89 - 1.98 (m, 1 H) 2.05 - 2.13 (m, 3 H) 2.50 - 3.13 (m, 2 H) 3.65 - 3.81 (m, 1 H) 4.05 - 4.16 (m, 2 H) 4.33 - 4.44 (m, 1 H) 6.63 - 6.72 (m, 1 H) 7.20 - 7.27 (m, 1 H) 7.59 - 7.70 (m, 2 H) 8.23 - 8.31 (m, 1 H).<br>MS ESI/APCI Multi posi: 315 [M+H]$^+$. |
| 13-4 | 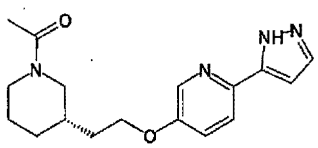 | $^1$H NMR (600 MHz, CHLOROFORM-d) δ ppm 1.20 - 1.31 (m, 1 H) 1.37 - 1.86 (m, 5 H) 1.89 - 1.98 (m, 1 H) 2.04 - 2.11 (m, 3 H) 2.50 - 3.12 (m, 2 H) 3.65 - 3.81 (m, 1 H) 4.04 - 4.16 (m, 2 H) 4.33 - 4.44 (m, 1 H) 6.63 - 6.71 (m, 1 H) 7.20 - 7.26 (m, 1 H) 7.57 - 7.70 (m, 2 H) 8.24 - 8.30 (m, 1 H).<br>MS ESI/APCI Multi posi: 315 [M+H]$^+$. |
| 13-5 | 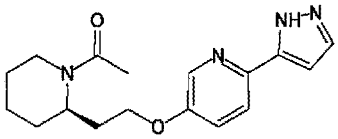 | $^1$H NMR (600 MHz, CHLOROFORM-d) δ ppm 1. 36 - 1. 81 (m, 6 H) 1.89 - 2.04 (m, 1 H) 2.05 - 2.09 (m, 3 H) 2.21 - 2.36 (m, 1 H) 2.52 - 2.61 (m, 0.5 H) 3.16 - 3.24 (m, 0.5 H) 3.60 - 3.68 (m, 0.5 H) 3.92 - 4.02 (m, 1 H) 4.03 - 4.11 (m, 1 H) 4.24 - 4.31 (m, 0.5 H) 4.56 - 4.64 (m, 0.5 H) 4.99 - 5.05 (m, 0.5 H) 6.61 - 6.72 (m, 1 H) 7. 18 - 7.24 (m, 1 H) 7.56 - 7.69 (m, 2 H) 8.22 - 8. 30 (m, 1 H).<br>MS ESI/APCI Multi posi: 315 [M+H]$^+$. |
| 13-6 | 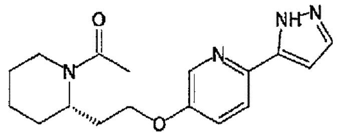 | $^1$H NMR (600 MHz, CHLOROFORM-d) δ ppm 1.36 - 1.79 (m, 6 H) 1.89 - 2.04 (m, 1 H) 2.05 - 2.09 (m, 3 H) 2.20 - 2. 36 (m, 1 H) 2.53 - 2.61 (m, 0.5 H) 3.15 - 3.24 (m, 0.5 H) 3.60 - 3.67 (m, 0.5 H) 3.92 - 4.02 (m, 1 H) 4.03 - 4.12 (m, 1 H) 4. 24 - 4.31 (m, 0.5 H) 4.57 - 4.63 (m, 0.5 H) 4.99 - 5.05 (m, 0.5 H) 6.62 - 6.72 (m, 1 H) 7.18 - 7.23 (m, 1 H) 7.56 - 7.68 (m, 2 H) 8.23 - 8.28 (m, 1 H).<br>MS ESI/APCI Multi posi: 315 [M+H]$^+$. |
| 13-7 | 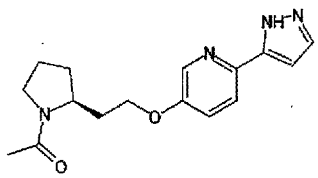 | $^1$H NMR (600 MHz, CHLOROFORM-d) δ ppm 1.82 - 2.16 (m, 8 H) 2.20 - 2.36 (m, 1 H) 3.38 - 3.65 (m, 2 H) 4.00 - 4.33 (m, 3 H) 6.64 - 6.73 (m, 1 H) 7.22 - 7.30 (m, 1 H) 7.58 - 7.71 (m, 2 H) 8.25 - 8.32 (m, 1 H).<br>MS ESI/APCI Multi posi: 301 [M+H]$^+$. |
| 13-8 | 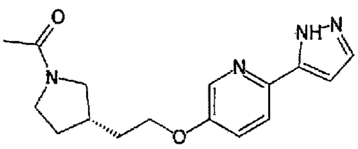 | $^1$H NMR (600 MHz, CHLOROFORM-d) δ ppm 1.51 - 1.77 (m, 1 H) 1.87 - 1.99 (m, 2 H) 2.01 - 2.07 (m, 3 H) 2.07 - 2.22 (m, 1 H) 2.33 - 2.54 (m, 1 H) 3.02 - 3.16 (m, 1 H) 3.31 - 3.86 (m, 3 H) 4.03 - 4.15 (m, 2 H) 6.64 - 6.73 (m, 1 H) 7.19 - 7.27 (m, 1 H) 7.57 - 7.71 (m, 2 H) 8.24 - 8.32 (m, 1 H).<br>MS ESI/APCI Multi posi: 301 [M+H]$^+$. |

[Table 32-2]

| Example No. | Structure | Analytical Data |
|---|---|---|
| 13-9 | 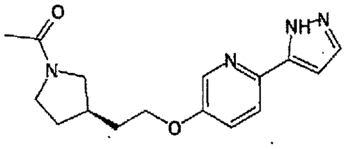 | $^1$H NMR (600 MHz, CHLOROFORM-d) δ ppm 1.47 - 1.69 (m, 1 H) 1.80 - 1.92 (m, 2 H) 1.95 - 2.00 (m, 3 H) 2.02 - 2.16 (m, 1 H) 2.28 - 2.48 (m, 1 H) 2.96 - 3.09 (m, 1 H) 3.25 - 3.79 (m, 3 H) 3.97 - 4.08 (m, 2 H) 6.58 - 6.66 (m, 1 H) 7.13 - 7.20 (m, 1 H) 7.51 - 7.65 (m, 2 H) 8.17 - 8.24 (m, 1 H).<br>MS ESI/APCI Multi posi: 301 [M+H]$^+$. |
| 13-10 | 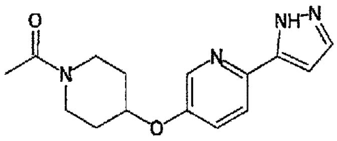 | $^1$H NMR (400 MHz, CHLOROFORM-d) δ ppm 1.77 - 2.05 (m, 4 H) 2.13 (s, 3 H) 3.38 - 3.50 (m, 1 H) 3.60 - 3.86 (m, 3 H) 4.57 - 4.66 (m, 1 H) 6.67 - 6.73 (m, 1 H) 7.27 - 7.30 (m, 1 H) 7.59 - 7.71 (m, 2 H) 8.28 - 8.34 (m, 1 H).<br>MS ESI/APCI Multi posi: 287 [M+H]$^+$. |
| 13-11 | 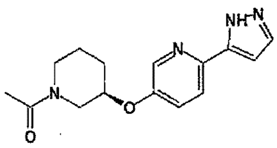 | $^1$H NMR (400 MHz, DMS0-$d_6$) δ ppm 1.36 - 2.10 (m, 7 H) 3.24 - 3.40 (m, 3.5 H) 3.86 - 3.97 (m, 0.5 H) 4.35 - 4.49 (m, 0.5 H) 4.55 - 4.71 (m, 0.5 H) 6.69 - 6.77 (m, 1 H) 7.39 - 7.95 (m, 3 H) 8.23 - 8.34 (m, 1 H).<br>MS ESI/APCI Multi posi: 287 [M+H]$^+$. |
| 13-12 | 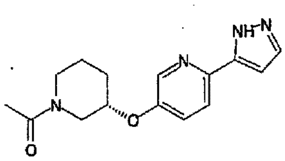 | $^1$H NMR (400 MHz, DMS0-$d_6$) δ ppm 1. 38 - 2.09 (m, 7 H) 3.21 - 3.41 (m, 1.5 H) 3.46 - 3.70 (m, 2 H) 3.87 - 3.97 (m, 0.5 H) 4. 35 - 4. 50 (m, 0.5 H) 4. 57 - 4. 70 (m, 0.5 H) 6. 69 - 6. 77 (m, 1 H) 7.33 - 7.95 (m, 3 H) 8.22 - 8. 34 (m, 1 H).<br>MS ESI/APCI Multi posi: 287 [M+H]$^+$. |
| 13-13 | 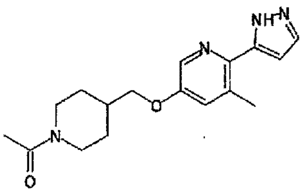 | $^1$H NMR (600 MHz, CHLOROFORM-d) δ ppm 1.25 - 1.43 (m, 2 H) 1.80 - 2.01 (m, 2 H) 2.03 - 2.21 (m, 4 H) 2.50 - 2.67 (m, 4 H) 3.07 - 3.17 (m, 1 H) 3.82 - 3.98 (m, 3 H) 4.65 - 4.78 (m, 1 H) 6.67 (br s, 1 H) 7.11 (s, 1 H) 7.67 (br s, 1 H) 8.17 (br s, 1 H).<br>MS ESI/APCI Multi posi: 315 [M+H]$^+$. |

[Table 32-3]

| Example No. | Structure | Analytical Data |
|---|---|---|
| 13-15 | 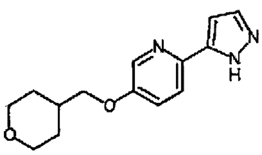 | $^1$H NMR (400 MHz, CHLOROFORM-d) δ ppm 1. 37 - 1.58 (m, 2 H) 1. 68 - 1. 85 (m, 2 H) 2.00 - 2.20 (m, 1 H) 3.36 - 3.52 (m, 2 H) 3.81 - 3.95 (m, 2 H) 3.96 - 4.10 (m, 2 H) 6.71 (s, 1 H) 7.18 - 7.33 (m. 1 H) 7.53 - 7.75 (m, 2 H) 8.28 (s, 1 H).<br>MS ESI/APCI Multi posi: 260[M+H]$^+$. |
| 13-16 | 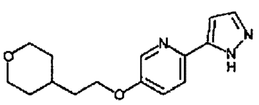 | $^1$H NMR (400 MHz, CHLOROFORM-d) δ ppm 1.18 - 1.73 (m, 4 H) 1.74 - 1.89 (m, 3 H) 3.33 - 3.50 (m, 2 H) 3.92 - 4.02 (m, 2 H) 4.06 - 4.14 (m, 2 H) 6.68 (s, 1 H) 7.18 - 7.31 (m, 1 H) 7.58 - 7.68 (m, 2 H) 8.23 - 8.33 (m, 1 H).<br>MS ESI/APCI Multi posi: 274[M+H]$^+$. |
| 13-17 | 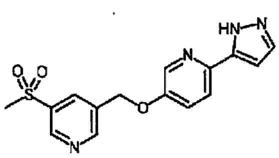 | $^1$H NMR (400 MHz, DMSO-$d_6$) δ ppm 3.37 (s, 3 H) 5.40 (s, 2 H) 6.75 (s, 1 H) 7.51 - 7.98 (m, 3 H) 8.37 - 8.50 (m, 2 H) 8.98 - 9.12 (m, 2 H).<br>MS ESI/APCI Multi posi: 331[M+H]$^+$. |

(continued)

| Example No. | Structure | Analytical Data |
|---|---|---|
| 13-18 | 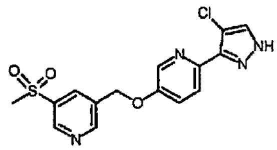 | $^1H$ NMR (400 MHz, DMSO-$d_6$) δ ppm 3. 37 (s, 3 H) 5.43 (s, 2 H) 7.52 - 8.14 (m, 3 H) 8.41 - 8.58 (m, 2 H) 9.00 - 9.13 (m, 2 H).<br>MS ESI/APCI Multi posi: 355[M+H]+. |

Example 14-1

N-[cis-4-({[6-(1H-Pyrazol-5-yl)pyridin-3-yl]oxy}methyl)cyclohexyl]acetamide

[Formula 212]

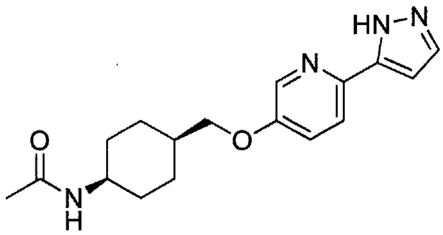

(1) Triphenylphosphine (297 mg) and di(methoxyethyl) azodicarboxylate (133 mg) were added to a solution of the compound (139 mg) obtained in Reference Example 1-1 and the compound (97.0 mg) obtained in Reference Example 41-2 in tetrahydrofuran (2.83 mL). After this mixture was stirred at room temperature for 30 minutes, di(methoxyethyl) azodicarboxylate (133 mg) was further added, and the mixture was stirred at room temperature for 2 hours. The reaction mixture was concentrated under reduced pressure, and the obtained residue was purified by NH silica gel column chromatography (n-hexane/ethyl acetate = 4:1 to ethyl acetate only) to give a partially purified substance (144 mg) of N-{cis-4-[({6-[1-(oxan-2-yl)-1H-pyrazol-5-yl]pyridin-3-yl}oxy)methyl]cyclohexyl}acetamide.

(2) The compound (71 mg) obtained in the above described (1) was used to perform the synthesis process according to the method described in Example 1-1-(2) thereby giving the title compound (20 mg) as a colorless powder.

$^1H$ NMR (600 MHz, DMSO-$d_6$) δ ppm 1.46 - 1.63 (m, 8 H) 1.82 (s, 3 H) 1.84 - 1.92 (m, 1 H) 3.77 - 3.85 (m, 1 H) 3.95 (m, J=6.2 Hz, 2 H) 6.73 (br s, 1 H) 7.36 - 7.99 (m, 4 H) 8.20 - 8.40 (m, 1 H) 12.78 - 13.42 (m, 1 H).

MS ESI/APCI Multi posi: 315 [M+H]+.

The compounds of the following Examples 14-2 to 14-15 were synthesized using the compound obtained in the Reference Example 1-1 and the alcohol obtained in Reference Example 31-1, 32-1, 33-1, 33-2, 34-1, 35-1, 35-2, 36-1, 37-1, 38-1, 39-1, 40-1, 41-1 or 41-3 according to the method described in Example 14-1. The structures, NMR data and MS data of these compounds are shown in Tables 33-1 to 33-2.

[Table 33-1]

| Example No. | Structure | Analytical Data |
|---|---|---|
| 14-2 | 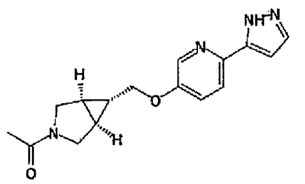 | $^1H$ NMR (600 MHz, DMSO-$d_6$) δ ppm 1.05 (tt, J=6.9, 3.4 Hz, 1 H) 1.62 - 1.68 (m, 1 H) 1.70 - 1. 75 (m, 1 H) 1.89 (s, 3 H) 3.27 (dd, J=11.6, 4.5 Hz, 1 H) 3.53 - 3.65 (m, 3 H) 3.88 - 4.10 (m, 2 H) 6.72 (br s, 1 H) 7.37 - 7.93 (m, 3 H) 8.27 (br s, 1 H) 12.81 - 13.37 (m, 1 H).<br>MS ESI/APCI Multi posi: 299[M+H]+. |

(continued)

| Example No. | Structure | Analytical Data |
|---|---|---|
| 14-3 | 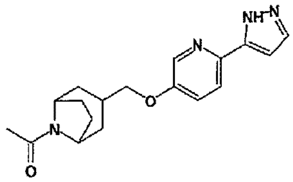 | $^1$H NMR (600 MHz, DMSO-$d_6$) δ ppm 1. 35 - 1.46 (m, 2 H) 1.59 - 1.71 (m, 2 H) 1.72 - 1.84 (m, 3 H) 1.92 - 2.01 (m, 4 H) 2.33 - 2.42 (m, 1 H) 3.81 - 3.93 (m, 2 H) 4.17 - 4.24 (m, 1 H) 4.41 - 4.53 (m, 1 H) 6.72 (s, 1 H) 7.36 - 7.93 (m, 3 H) 8.25 (br s, 1 H) 12.78 - 13.39 (m, 1 H).<br>MS ESI/APCI Multi posi: 327[H+H]$^+$. |
| 14-4 | 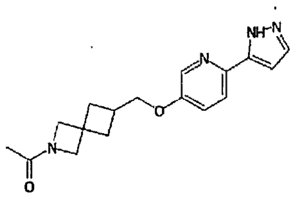 | $^1$H NMR (600 MHz, DMSO-$d_6$) δ ppm 1.68 - 1.75 (m, 3 H) 2.01 - 2.10 (m, 2 H) 2.27 - 2.36 (m, 2 H) 2.55 - 2.63 (m, 1 H) 3.78 (s, 1 H) 3.84 (s, 1 H) 4.00 - 4.04 (m, 2 H) 4.06 (s, 1 H) 4.12 (s, 1 H) 6.72 (s, 1 H) 7.35 - 7.96 (m, 3 H) 8.27 (br s, 1 H) 12.80 - 13.39 (m, 1 H).<br>MS ESI/APCI Multi posi: 313[M+H]$^+$. |
| 14-5 | 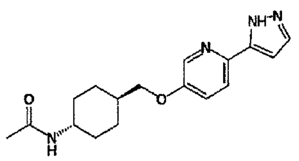 | $^1$H NMR (600 MHz, DUSO-$d_6$) δ ppm 0.97 - 1. 11 (m, 4 H) 1.53 - 1.64 (m, 1 H) 1.66 (s, 3 H) 1.69 - 1.81 (m, 4 H) 3.33 - 3.42 (m, 1 H) 3.72 - 3.84 (m, 2 H) 6.61 (s, 1 H) 7.24 - 7.82 (m, 4 H) 8.15 (br s, 1 H) 12.69 - 13.29 (m, 1 H).<br>MS ESI/APCI Multi posi: 315[M+H]$^+$. |
| 14-6 | 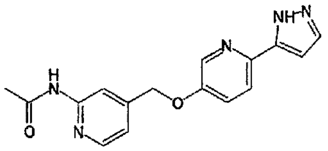 | $^1$H NMR (600 MHz, DMSO-$d_6$) δ ppm 2.06 (s, 3 H) 5.22 - 5.32 (m, 2 H) 6. 67 - 6.75 (m, 1 H) 7.14 (d, J=5.0 Hz, 1 H) 7.43 - 7.90 (m, 3 H) 8.16 (s, 1 H) 8.28 (d, J=5.0 Hz, 1 H) 8.30 - 8.39 (m, 1 H) 10.50 (s, 1 H) 12.80 - 13.36 (m, 1 H).<br>MS ESI/APCI Multi posi: 310[M+H]$^+$. |
| 14-7 | 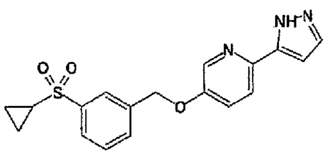 | $^1$H NMR (400 MHz, DMSO-$d_6$) δ ppm 0.99 - 1.08 (m, 2 H) 1.08 - 1.16 (m, 2 H) 2.87 (tt, J=7.8, 4.8 Hz, 1 H) 5.31 - 5.41 (m, 2 H) 6.70 - 6.78 (m, 1 H) 7.45 - 7.95 (m, 6 H) 8.01 (s, 1 H) 8.32 - 8.45 (m, 1 H) 12.66 - 13.55 (m, 1 H).<br>MS ESI/APCI Multi posi: 356[M+H]$^+$. |
| 14-8 | 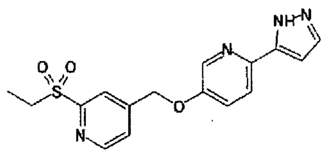 | 'H NMR (400 MHz, DMSO-$d_6$) δ ppm 1.14 (t, J=7.4 Hz, 3 H) 3.47 (q, J=7.4 Hz, 2 H) 5.47 (s, 2 H) 6.75 (br s, 1 H) 7.43 - 8.01 (m, 4 H) 8.14 (d, J=0.61 Hz, 1 H) 8.35 - 8.50 (m, 1 H) 8.78 - 8.87 (m, 1 H) 12.81 - 13.44 (m, 1 H).<br>MS ESI/APCI Multi posi: 345[M+H]$^+$. |

[Table 33-2]

| Example No. | Structure | Analytical Data |
|---|---|---|
| 14-9 | 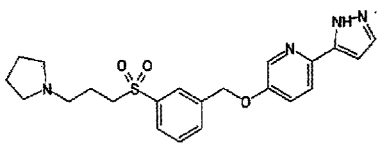 | $^1$H NMR (400 MHz, DMSO-$d_6$) δ ppm 1.78 - 1.89 (m, 2 H) 1.92 - 2.02 (m, 4 H) 2.85 - 2.99 (m, 2 H) 3.14 - 3.23 (m, 2 H) 3.43 - 3.54 (m, 4 H) 5.40 (s, 2 H) 6.81 (d, J=2.2 Hz, 1 H) 7.67 (dd, J=8.8, 2.9 Hz, 1 H) 7.71 - 7.78 (m, 2 H) 7.87 - 7.98 (m, 3 H) 8.03 - 8.07 (m, 1 H) 8.38 - 8.43 (m, 1 H) 10.33 (br s, 1 H).<br>MS ESI/APCI Multi posi: 427[M+H]$^+$. |

(continued)

| Example No. | Structure | Analytical Data |
|---|---|---|
| 14-10 | 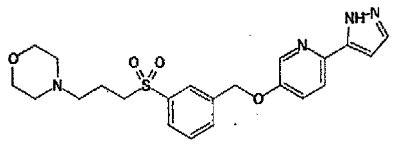 | $^1$H NMR (400 MHz, METHANOL-$d_4$) δ ppm 2.09 - 2.24 (m, 2 H) 3.25 - 3.40 (m, 8 H) 3.70 - 4.03 (m, 4 H) 5.36 (s, 2 H) 6.81 (d, J=2.3 Hz, 1 H) 7.58 (dd, J=8.8, 2.9 Hz, 1 H) 7.68 (d, J=2.2 Hz, 1 H) 7.70 - 7.77 (m, 1 H) 7.85 - 7.92 (m, 2 H) 7.93 - 7.98 (m, 1 H) 8.09 (s, 1 H) 8.31 - 8.38 (m, 1 H). MS ESI/APCI Multi posi: 443[M+H]$^+$. |
| 14-11 | 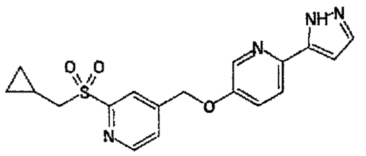 | $^1$H NMR (400 MHz, DMSO-$d_6$) δ ppm 0.08 - 0.15 (m, 2 H) 0.36 - 0.43 (m, 2 H) 0.77 - 0.92 (m, 1 H) 3.42 (d, J=7.1 Hz, 2 H) 5.48 (s, 2 H) 6.74 (br s, 1 H) 7.48 - 7.97 (m, 4 H) 8.12 - 8.20 (m, 1 H) 8.40 (br s, 1 H) 8.82 (dd, J=5.0, 0.4 Hz, 1 H) 12.81 - 13.43 (m, 1 H). MS ESI/APCI Multi posi: 371[M+H]$^+$. |
| 14-12 | 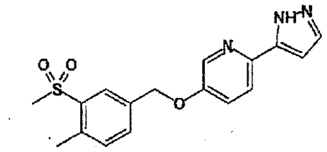 | $^1$H NMR (400 MHz, DMSO-$d_6$) δ ppm 2.65 (s, 3 H) 3.23 (s, 3 H) 5.30 (s, 2 H) 6.73 (d, J=2.1 Hz, 1 H) 7.45 - 7.94 (m, 5 H) 8.02 (d, J=1.7 Hz, 1 H) 8.30 - 8.43 (m, 1 H) 12.66 - 13.44 (m, 1 H). MS ESI/APCI Multi posi: 344[M+H]$^+$. |
| 14-13 | 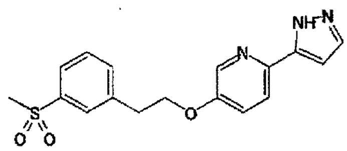 | $^1$H NMR (600 MHz, DMSO-$d_6$) δ ppm 3.18 - 3.24 (m, 5 H) 4.30 - 4.41 (m, 2 H) 6.67 - 6.77 (m, 1 H) 7.40 - 7.90 (m, 6 H) 7.90 - 7.95 (m, 1 H) 8.23 - 8.33 (m, 1 H) 12.83 - 13.37 (m, 1 H). MS ESI/APCI Multi posi: 344[M+H]$^+$. |
| 14-14 | 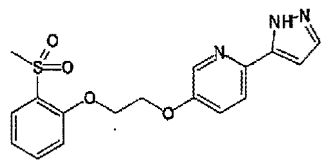 | $^1$H NMR (600 MHz, DMSO-$d_6$) δ ppm 3.28 (s, 3 H) 4.47 - 4.54 (m, 2 H) 4.54 - 4.59 (m, 2 H) 6.73 (br s, 1 H) 7.14 - 7.23 (m, 1 H) 7.37 (d, J=8.3 Hz, 1 H) 7.47 - 7.93 (m, 5 H) 8.31. (br s, 1 H) 12.78 - 13.44 (m, 1 H). MS ESI/APCI Multi posi: 360[M+H]$^+$. |
| 14-15 | 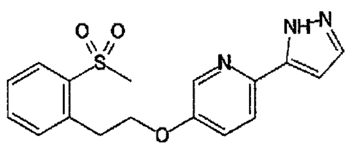 | $^1$H NMR (400 MHz, DMSO-$d_6$) δ ppm 2.50 (s, 3 H) 3.52 (t, J=6.9 Hz, 2 H) 4.39 (t, J=6.9 Hz, 2 H) 6.72 (d, J=2.0 Hz, 1 H) 7.43 - 7.91 (m, 6 H) 7.97 (dd, J=8.0, 1.0 Hz, 1 H) 8.22 - 8.35 (m, 1 H) 12. 77 - 13.42 (m, 1 H) MS ESI/APCI Multi posi: 344[M+H]$^+$. |

Example 15-1

N-Methyl-N-[cis-4-({[6-(1H-pyrazol-5-yl)pyridin-3-yl]oxy}methyl)cyclohexyl]acetamide

[Formula 213]

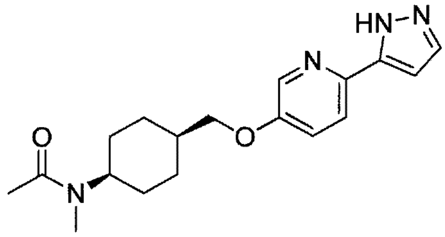

(1) The compound (73 mg) obtained in Example 14-1-(1) was used to perform the synthesis process according to the method described in Example 8-2-(1) thereby giving N-methyl-N-{cis-4-[({6-[1-(oxan-2-yl)-1H-pyrazol-5-yl]pyridin-3-yl}oxy)methyl]cyclohexyl}acetamide (29 mg) as a colorless gum-like substance.

(2) The compound (26 mg) obtained in the above described (1) was used to perform the synthesis process according to the method described in Example 1-1-(2) thereby giving the title compound (13 mg) as a pale yellow gum-like substance.

$^1$H NMR (600 MHz, DMSO-$d_6$) δ ppm 1.26 - 1.33 (m, 1.14 H) 1.41 - 1.50 (m, 0.86 H) 1.54 - 1.77 (m, 4 H) 1.83 - 1.91 (m, 2 H) 1.96 (s, 1.71 H) 2.02 (s, 1.29 H) 2.08 - 2.16 (m, 1 H) 2.70 (s, 1.29 H) 2.83 (s, 1.71 H) 3.54 - 3.63 (m, 0.43 H) 4.15 (d, J=7.84 Hz, 2 H) 4.22 - 4.29 (m, 0.57 H) 6.72 (d, J=2.06 Hz, 1 H) 7.45 - 7.95 (m, 3 H) 8.25 - 8.36 (m, 1 H) 12.68 - 13.51 (m, 1 H)

MS ESI/APCI Multi posi: 329 $[M+H]^+$.

Example 16-1

N-[3-({[6-(1H-Pyrazol-5-yl)pyridin-3-yl]oxy}methyl)cyclobutyl]acetamide

[Formula 214]

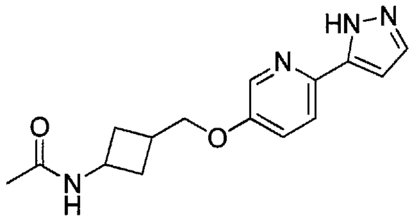

(1) Tributylphosphine (666 μL) and 1,1'-azobis(N,N-dimethylformamide) (459 mg) were added to a solution of the compound (261 mg) obtained in Reference Example 1-1 and the compound (183 mg) obtained in Reference Example 25-7 in tetrahydrofuran (5 mL), and the mixture was stirred at 60°C for 1 hour and at room temperature for two days. After the resulting solid was filtrated off, water was added to the filtrate, and the mixture was extracted with ethyl acetate. The organic layer was washed with water and brine and then dried with anhydrous sodium sulfate. After the drying agent was filtrated off, the filtrate was concentrated under reduced pressure, and the obtained residue was purified by silica gel column chromatography (n-hexane/ethyl acetate = 7:3 to ethyl acetate only, subsequently ethyl acetate/methanol = 20:1) to give N-{3-[({6-[1-(oxan-2-yl)-1H-pyrazol-5-yl]pyridin-3-yl}oxy)methyl]cyclobutyl}acetamide (420 mg) as a colorless oily substance.

(2) The compound (159 mg) obtained in the above described (1) was used to perform the reaction and after-treatment according to the method described in Example 1-1-(2). After isomeric separation of the obtained residue was performed by preparative HPLC, each obtained isomer was recrystallized from acetonitrile/diethyl ether. An isomer having a short retention time was obtained as Example 16-1-1 (9.1 mg, colorless powder), and a compound having a long retention time was obtained as Example 16-1-2 (10.7 mg, colorless powder).

Example 16-1-1

N-[cis-3-({[6-(1H-Pyrazol-5-yl)pyridin-3-yl]oxy}methyl)cyclobutyl]acetamide

$^1$H NMR (600 MHz, ACETONE-$d_6$) δ ppm 1.75 - 1.90 (m, 5 H) 2.41 - 2.52 (m, 3 H) 4.02 - 4.11 (m, 2 H) 4.24 - 4.36 (m, 1 H) 6.79 (d, J=2.1 Hz, 1 H) 7.25 (br s, 1 H) 7.38 (dd, J=8.9, 2.9 Hz, 1 H) 7.66 (d, J=2.1 Hz, 1 H) 7.88 (d, J=8.9 Hz, 1 H) 8.26 (d, J=2.9 Hz, 1 H) 12.21 (br s, 1 H).

MS ESI/APCI Multi posi: 287 $[M+H]^+$.

LC-MS Retention time: 0.719 min. (condition: method B)

Example 16-1-2

N-[trans-3-({[6-(1H-Pyrazol-5-yl)pyridin-3-yl]oxy}methyl)cyclobutyl]acetamide

$^1$H NMR (600 MHz, ACETONE-$d_6$) δ ppm 1.83 (s, 3 H) 2.13 - 2.20 (m, 2 H) 2.22 - 2.32 (m, 2 H) 2.57 - 2.74 (m, 1 H) 4.17 - 4.22 (m, 2 H) 4.45 - 4.54 (m, 1 H) 6.79 (d, J=2.1 Hz, 1 H) 7.33 (br s, 1 H) 7.43 (dd, J=8.9, 2.9 Hz, 1 H) 7.65 (d, J=2.1 Hz, 1 H) 7.88 (d, J=8.9 Hz, 1 H) 8.30 (d, J=2.9 Hz, 1 H) 12.16 (br s, 1 H).
MS ESI/APCI Multi posi: 287 $[M+H]^+$.
LC-MS Retention time: 0.723 min. (condition: method B)

Example 16-2

1-[3-(2-{[6-(1H-Pyrazol-5-yl)pyridin-3-yl]oxy}ethyl)azetidin-1-yl]ethan-1-one

[Formula 215]

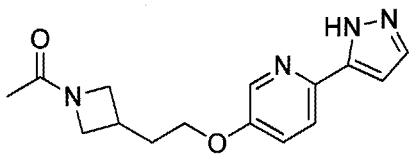

(1) The compound (59.8 mg) obtained in Reference Example 1-1 and the compound (52.4 mg) obtained in Reference Example 26-2 were used to perform the synthesis process according to the method described in Example 16-1-(1) thereby giving 1-{3-[2-({6-[1-(oxan-2-yl)-1H-pyrazol-5-yl]pyridin-3-yl}oxy)ethyl]azetidin-1-yl}ethan-1-one (30.7 mg) as a colorless oily substance.
(2) The compound (30.7 mg) obtained in the above described (1) was used to perform the synthesis process according to the method described in Example 1-1-(2) thereby giving the title compound (11.7 mg) as a colorless powder.
$^1$H NMR (600 MHz, CHLOROFORM-d) δ ppm 1.87 (s, 3 H) 2.11 - 2.20 (m, 2 H) 2.80 - 2.96 (m, 1 H) 3.71 - 3.82 (m, 1 H) 3.82 - 3.94 (m, 1 H) 4.01 - 4.13 (m, 2 H) 4.13 - 4.22 (m, 1 H) 4.24 - 4.36 (m, 1 H) 6.70 (d, J=2.1 Hz, 1 H) 7.22 (dd, J=8.7, 2.9 Hz, 1 H) 7.63 (d, J=2.1 Hz, 1 H) 7.66 (d, J=8.6 Hz, 1 H) 8.26 (d, J=2.9 Hz, 1 H).
MS ESI/APCI Multi posi: 287 $[M+H]^+$.

The compounds of the following Examples 16-3 and 16-5 were synthesized using the compound obtained in the Reference Example 1-1 and the alcohol obtained in Reference Example 42-1 or 43-2 according to the method described in Example 16-1. The structures, NMR data and MS data of these compounds are shown in Table 34-1.

[Table 34-1]

| Example No. | Structure | Analytical Data |
|---|---|---|
| 16-3 | 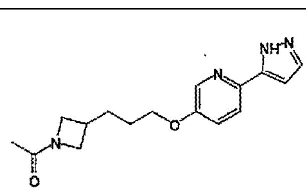 | $^1$H NMR (600 MHz, CHLOROFORM-d) δ ppm 1.75 - 1.86 (m, 4 H) 1.86 (s, 3 H) 2.61 - 2.70 (m, 1 H) 3.63 - 3.69 (m, 1 H) 3.71 - 3.81 (m, 1 H) 4.03 - 4.09 (m, 2 H) 4.09 - 4.14 (m, 1 H) 4.18 - 4.31 (m, 1 H) 6.69 (d, J=2.1 Hz, 1 H) 7.24 (dd, J=8.3, 3.0 Hz, 1 H) 7.63 (d, J=2.1 Hz, 1 H) 7.66 (br d, J=8.3 Hz, 1 H) 8.28 (d, J=3.0 Hz, 1 H).<br>MS ESI/APCI Multi posi: 301$[M+H]^+$ |

(continued)

| Example No. | Structure | Analytical Data |
|---|---|---|
| 16-5 | 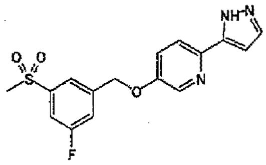 | $^1$H NMR (400 MHz, CHLOROFORM-d) δ ppm 3.10 (s, 3 H) 5.22 (s, 2 H) 6.73 (s, 1 H) 7.29 - 7.40 (m, 1 H) 7.45 - 7.53 (m, 1 H) 7.57 - 7.77 (m, 3 H) 7.85 (s, 1 H) 8.32 - 8.44 (m, 1 H).<br>MS ESI/APCI Multi posi: 348[M+H]$^+$. |

Example 17-1

N-Methyl-N-[3-({[6-(1H-pyrazol-5-yl)pyridin-3-yl]oxy}methyl)cyclobutyl]acetamide

[Formula 216]

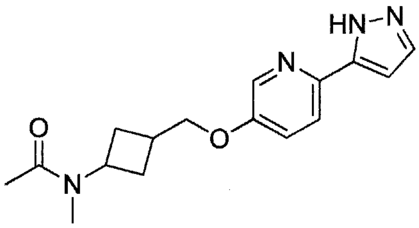

(1) The compound (77.6 mg) obtained in Example 16-1-(1) was used to perform the synthesis process according to the method described in Example 8-2-(1) thereby giving N-methyl-N-{3-[({6-[1-(oxan-2-yl)-1H-pyrazol-5-yl]pyridin-3-yl}oxy)methyl]cyclobutyl}acetamide (66.5 mg) as a colorless oily substance.
(2) The compound (66.5 mg) obtained in the above described (1) was used to perform the reaction and after-treatment according to the method described in Example 1-1-(2). Isomeric separation of the obtained residue was performed by preparative HPLC. The compound of the higher polarity was freeze-dried to give the compound of Example 17-1-1 (22.0 mg, a colorless powder), and the compound of the lower polarity was recrystallized from acetonitrile/diethyl ether to give the compound of Example 17-1-2 (5.2 mg, a colorless powder).

Example 17-1-1

N-Methyl-N-[cis-3-({[6-(1H-pyrazol-5-yl)pyridin-3-yl]oxy}methyl)cyclobutyl]acetamide

$^1$H NMR (600 MHz, CHLOROFORM-d) δ ppm 2.04 - 2.49 (m, 8 H) 2.96 (s, 1.5 H) 2.98 (s, 1.5 H) 4.02 (dd, J=10.1, 5.2 Hz, 2 H) 4.20 - 4.36 (m, 0.5 H) 4.91 - 5.04 (m, 0.5 H) 6.65 - 6.75 (m, 1 H) 7.23 - 7.26 (m, 1 H) 7.60 - 7.70 (m, 2 H) 8.26 - 8.35 (m, 1 H).
MS ESI/APCI Multi posi: 301 [M+H]$^+$.
LC-MS Retention time: 0.84 min (condition: method B)

Example 17-1-2

N-Methyl-N-[trans-3-({[6-(1H-pyrazol-5-yl)pyridin-3-yl]oxy}methyl)cyclobutyl]acetamide

$^1$H NMR (600 MHz, CHLOROFORM-d) δ ppm 2.09 (s, 1.5 H) 2.11 (s, 1.5 H) 2.14 - 2.21 (m, 1 H) 2.23 - 2.29 (m, 1 H) 2.33 - 2.45 (m, 1 H) 2.46 - 2.58 (m, 1 H) 2.58 - 2.76 (m, 1 H) 2.98 (s, 1.5 H) 3.01 (s, 1.5 H) 4.09 - 4.21 (m, 2 H) 4.54 - 4.66 (m, 0.5 H) 5.20 - 5.31 (m, 0.5 H) 6.67 - 6.74 (m, 1 H) 7.26 - 7.29 (m, 1 H) 7.61 - 7.73 (m, 2 H) 8.27 - 8.33 (m, 1 H) MS ESI/APCI Multi posi: 301 [M+H]$^+$.
LC-MS Retention time: 0.86 min. (condition: method B)

Example 18-1

2-Fluoro-N,N-dimethyl-5-({[6-(1H-pyrazol-5-yl)pyridin-3-yl]oxy}methyl)benzene-1-sulfonamide

[Formula 217]

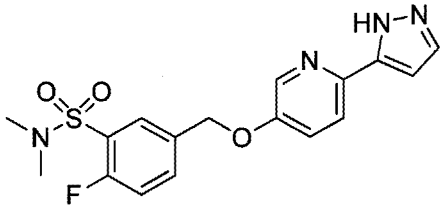

(1) Triphenylphosphine (36.4 mg) and diisopropyl azodicarboxylate (1.9 mol/L solution in toluene, 54.7 μL) were added to a solution of the compound (17.0 mg) obtained in Reference Example 1-1 and the compound (19.4 mg) obtained in Reference Example 37-2 in tetrahydrofuran (1 mL), and the mixture was stirred at 50°C for 2 hours and at 70°C for 1 hour. The reaction mixture was concentrated, and the obtained residue was purified by silica gel column chromatography (chloroform only, to chloroform/methanol = 9:1) to give 2-fluoro-N,N-dimethyl-5-[({6-[1-(oxan-2-yl)-1H-pyrazol-5-yl]pyridin-3-yl}oxy)methyl]benzene-1-sulfonamide (35.2 mg) as a colorless oily substance.

(2) The compound (35.2 mg) obtained in the above described (1) was used to perform the synthesis process according to the method described in Example 1-1-(2) thereby giving the title compound (15.8 mg) as a colorless powder.

$^1$H NMR (300 MHz, CHLOROFORM-d) δ ppm 2.83 - 2.87 (m, 6 H) 5.15 (s, 2 H) 6.72 (d, J=2.0 Hz, 1 H) 7.21 - 7.30 (m, 1 H) 7.32 (dd, J=9.0, 2.7 Hz, 1 H) 7.64 (d, J=2.0 Hz, 1 H) 7.64 - 7.69 (m, 1 H) 7.71 (d, J=9.0 Hz, 1 H) 7.83 - 8.08 (m, 1 H) 8.39 (d, J=2.7 Hz, 1 H). MS ESI/APCI Multi posi: 377 $[M+H]^+$.

The compounds of the following Examples 18-2 to 18-6 were synthesized using the compound obtained in Reference Example 1-1, as well as the alcohol obtained in Reference Examples 31-3, 37-3, 44-1, 45-1, or 45-2, according to the method described in Example 18-1. The structures, NMR data and MS data of these compounds are shown in Table 35-1.

[Table 35-1]

| Example No. | Structure | Analytical Data |
|---|---|---|
| 18-2 | 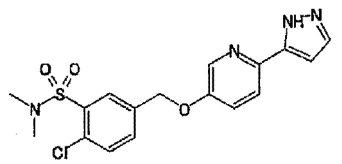 | $^1$H NMR (600 MHz, DMSO-$d_6$) δ ppm 2.80 (s, 6 H) 5.34 (br s, 2 H) 6.73 (br s, 1 H) 7.47 - 7.63 (m, 1.3 H) 7.70 - 7.85 (m, 3 H) 7.87 - 7.92 (m, 0.7 H) 8.03 (s, 1 H) 8.32 - 8.43 (m, 1 H) 12.90 (br s, 0.7 H) 13.32 (br s, 0.3 H).<br>MS ESI/APCI Multi posi: 393$[M+H]^+$. |
| 18-3 | 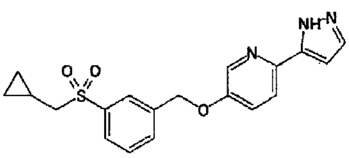 | $^1$H NMR (600 MHz, DMSO-$d_6$) δ ppm 0.04 - 0.09 (m, 2 H) 0.36 - 0.42 (m, 2 H) 0.74 - 0.83 (m, 1 H) 3.27 (d, J=7.43 Hz, 2 H) 5.33 - 5.41 (m, 2 H) 6. 68 - 6.78 (m, 1 H) 7.48 - 7.93 (m, 6 H) 8.01 (s, 1 H) 8.32 - 8.43 (m, 1 H) 12.86 - 13.38 (m, 1 H) .<br>MS ESI/APCI Multi posi: 370$[M+H]^+$. |
| 18-4 | 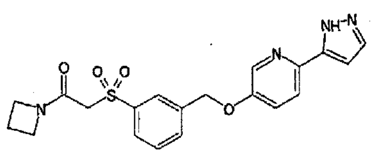 | $^1$H NMR (600 MHz, DMSO-$d_6$) δ ppm 2.04 - 2.13 (m, 2 H) 3.80 (t, J=7.6 Hz, 2 H) 4.06 (t, J=7.6 Hz, 2 H) 4.30 (s, 2 H) 5.30 - 5.40 (m, 2 H) 6.69 - 6.77 (m, 1 H) 7.46 - 7.93 (m, 6 H) 8.02 (s, 1 H) 8.33 - 8.44 (m, 1 H) 12.85 - 13.37 (m, 1 H).<br>MS ESI/APCI Multi posi: 413$[M+H]^+$. |

(continued)

| Example No. | Structure | Analytical Data |
|---|---|---|
| 18-5 | 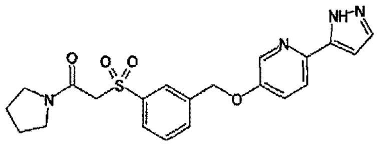 | $^1$H NMR (600 MHz, DMSO-$d_6$) δ ppm 1. 66 - 1.73 (m, 2 H) 1.73 - 1.80 (m, 2 H) 3.20 (t, J=6.8 Hz, 2 H) 3.40 (t, J=6.6 Hz, 2 H) 4.54 (s, 2 H) 5.29 - 5.38 (m, 2 H) 6.70 - 6.78 (m, 1 H) 7.46 - 7.93 (m, 6 H) 8.02 (s, 1 H) 8.33 - 8.44 (m, 1 H) 12.86 - 13.37 (m, 1 H).<br>MS ESI/APCI Multi posi: 427[M+H]$^+$. |
| 18-6 | 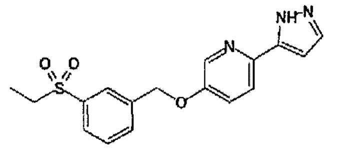 | $^1$H NMR (600 MHz, DMSO-$d_6$) δ ppm 1.08 (t, J=7.4 Hz, 3 H) 3.26 - 3.35 (m, 2 H) 5.36 (s, 2 H) 6.73 (s, 1 H) 7.50 - 7.93 (m, 6 H) 8.00 (s, 1 H) 8.37 (br s, 1 H) 12.83 - 13.42 (m, 1 H).<br>MS ESI/APCI Multi posi: 344[M+H]$^+$. |

Example 18-7

tert-Butyl 3-({[6-(1H-pyrazol-5-yl)pyridin-3-yl]oxy}methyl)phenyl]carbamate

[Formula 218]

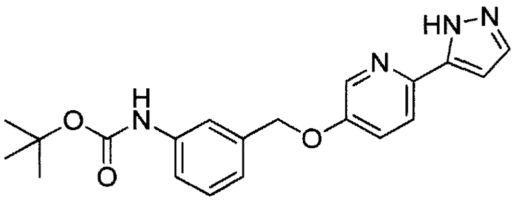

(1) A commercially available corresponding alcohol was used to perform the synthesis process according to the method described in Example 18-1-(1) thereby giving tert-butyl {3-[({6-[1-(oxan-2-yl)-1H-pyrazol-5-yl]pyridin-3-yl}oxyl)methyl]phenyl}carbamate (202 mg).

(2) The compound (41 mg) obtained in the above described (1) was used to perform the synthesis process according to the method described in Example 2-11-(2) thereby giving the title compound (6.8 mg) as a colorless powder.
$^1$H NMR (200 MHz, CHLOROFORM-d) δ ppm 1.52 (s, 9 H) 5.12 (s, 2 H) 6.49 - 6.58 (m, 1 H) 6.68 (d, J=2.0 Hz, 1 H) 7.05 - 7.16 (m, 1 H) 7.23 - 7.35 (m, 3 H) 7.56 (s, 1 H) 7.59 - 7.69 (m, 2 H) 8.34 (d, J=3.1 Hz, 1 H).
MS ESI/APCI Multi posi: 367 [M+H]$^+$.

Example 18-8

5-[(3-Methylsulfonylphenyl)methoxy]-2-(1H-pyrazol-5-yl)pyridine

[Formula 219]

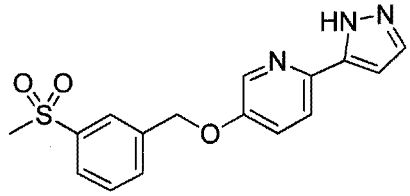

(1) The compound (540 mg) obtained in Reference Example 1-1 and commercially available (3-methylsulfonylphenyl)methanol (448 mg) were used to perform the synthesis process according to the method described in Example 18-1-(1) thereby giving a mixture (920 mg) containing 5-[(3-methylsulfonylphenyl)methoxy]-2-[2-(oxan-2-yl)pyrazol-3-yl]pyridine.
(2) The mixture (920 mg) obtained in the above described (1) was used to perform the synthesis process according to the method described in Example 2-11-(2) thereby giving the title compound (525 mg) as a colorless powder.
$^1$H NMR (200 MHz, CHLOROFORM-d) δ ppm 3.08 (s, 3 H) 5.23 (s, 2 H) 6.71 (d, J=2.2 Hz, 1 H) 7.34 (dd, J=8.8, 3.1 Hz, 1 H) 7.59 - 7.81 (m, 4 H) 7.90 - 7.99 (m, 1 H) 8.03 - 8.09 (m, 1 H) 8.33 - 8.39 (m, 1 H).
MS ESI/APCI Multi posi: 330 $[M+H]^+$.

Example 19-1

3-({[6-(1H-Pyrazol-5-yl)pyridin-3-yl]oxy}methyl)aniline

[Formula 220]

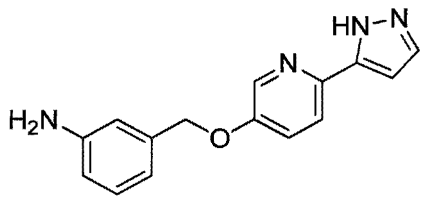

The compound (161 mg) obtained in Example 18-7-(1) was dissolved in trifluoroacetic acid (2 mL). To this mixture, water (0.5 mL) was added, and the mixture was stirred at room temperature for 8 hours. After confirming the end of the reaction by LC-MS, a nitrogen gas was blown onto the mixture to remove volatiles. An operation of dissolving the residue in methanol and concentrating it under reduced pressure was repeated twice, and the obtained residue was purified by NH silica gel column chromatography (chloroform only, to chloroform/methanol = 19:1). The resulting partially purified product was recrystallized from chloroform/methanol/n-hexane to give the title compound (35 mg) as a colorless powder.
$^1$H NMR (200 MHz, CHLOROFORM-d) δ ppm 3.72 (br s, 2 H) 5.06 (s, 2 H) 6.61 - 6.70 (m, 2 H) 6.73 - 6.84 (m, 2 H) 7.11 - 7.34 (m, 2 H) 7.59 - 7.67 (m, 2 H) 8.35 (d, J=2.9 Hz, 1 H). MS ESI/APCI Multi posi: 267 $[M+H]^+$.

Example 20-1

1-[(3R)-3-({[6-(1H-Pyrazol-5-yl)pyridin-3-yl]oxy}methyl)pyrrolidin-1-yl]ethan-1-one

[Formula 221]

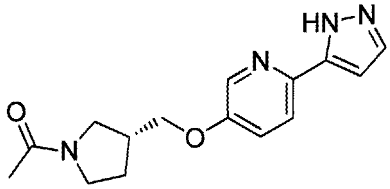

Triethylamine (64.3 μL) and acetic anhydride (35.1 μL) were added to a solution of the compound (102 mg) obtained in Reference Example 13-1 in chloroform (3 mL), and the mixture was stirred at room temperature for 1.5 hours. Hydrochloric acid (6 mol/L) was added under ice cooling, and the mixture was stirred at room temperature for 30 minutes. After adding an aqueous solution of saturated sodium hydrogen carbonate and separating the organic layer by a phase separator, the solvent was distilled off under reduced pressure. The obtained residue was purified by preparative HPLC to give the title compound (20.4 mg) as a colorless powder.
$^1$H NMR (600 MHz, DMSO-$d_6$) δ ppm 1.66 - 1.76 (m, 0.5 H) 1.80 - 1.90 (m, 0.5 H) 1.92 - 1.96 (m, 3 H) 1.98 - 2.04 (m, 0.5 H) 2.07 - 2.13 (m, 0.5 H) 2.57 - 2.67 (m, 0.5 H) 2.67 - 2.77 (m, 0.5 H) 3.12 - 3.19 (m, 0.5 H) 3.24 - 3.31 (m, 1 H) 3.42 - 3.50 (m, 1 H) 3.50 - 3.59 (m, 1 H) 3.63 - 3.72 (m, 0.5 H) 3.99 - 4.18 (m, 2 H) 6.73 (br s, 1 H) 7.28 - 7.63 (m, 1.5 H) 7.69 - 7.94 (m, 1.5 H) 8.20 - 8.46 (m, 1 H) 12.90 (br s, 0.5 H) 13.33 (s, 0.5 H). MS ESI/APCI Multi posi: 287 [M+H]$^+$.

The compound of the following Example 20-2 was synthesized using the compound obtained in Reference Example 13-2, according to the method described in Example 20-1. The structure, NMR data and MS data of the compound are shown in Table 36-1.

[Table 36-1]

| Example No. | Structure | Analytical Data |
|---|---|---|
| 20-2 | 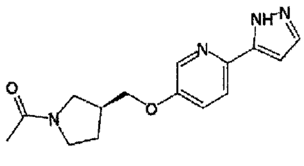 | $^1$H NMR (600 MHz, DMSO-$d_6$) δ ppm 1.66 - 1.76 (m, 0.5 H) 1.80 - 1.90 (m, 0.5 H) 1.92-1.96 (m, 3 H) 1.98 - 2.04 (m, 0.5 H) 2.07 - 2.13 (m, 0.5 H) 2.57 - 2.67 (m, 0.5 H) 2.67 - 2.77 (m, 0.5 H) 3.12 - 3.19 (m, 0.5 H) 3.24 - 3.31 (m, 1 H) 3.42 - 3.50 (m, 1 H) 3.50 - 3.59 (m, 1 H) 3.63 - 3.72 (m, 0.5 H) 3.99 - 4.18 (m, 2 H) 6.73 (br s, 1 H) 7.28 - 7.63 (m. 1.5 H) 7.69 - 7.94 (m, 1.5 H) 8.20 - 8.46 (m, 1 H) 12.90 (br s, 0.5 H) 13.33 (s, 0.5 H).<br>MS ESI/APCI Multi posi: 287[M+H]$^+$. |

Example 20-3

1-[(2R)-2-(2-{[6-(1H-Pyrazol-5-yl)pyridin-3-yl]oxy}ethyl)pyrrolidin-1-yl]ethan-1-one

[Formula 222]

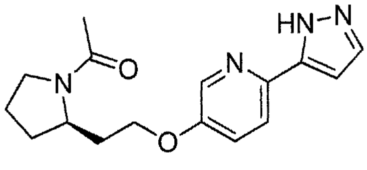

(1) N-Methylmorpholine (20 μL) and acetic anhydride (17 μL) were added to a solution of the compound (42 mg) obtained in Reference Example 11-1 in chloroform (1.2 mL), and the mixture was stirred at room temperature for 2.5 hours. An aqueous solution of saturated sodium hydrogen carbonate was added, and the reaction solution was extracted with ethyl acetate. The obtained organic layer was washed with brine, and then dried over anhydrous magnesium sulfate. The drying agent was filtered off, and the filtrate was concentrated under reduced pressure to

give 1-[(2R)-2-(2-{6-[2-(oxan-2-yl)pyrazol-3-yl]pyridin-3-yl}oxyethyl)pyrrolidin-1-yl]ethanone (51 mg) as a colorless oily substance.

(2) The compound (51 mg) obtained in the above described (1) was used to perform the synthesis process according to the method described in Example 1-1-(2) thereby giving the title compound (31 mg) as a colorless solid.

$^1$H NMR (600 MHz, CHLOROFORM-d) δ ppm 1.47 - 1.69 (m, 1 H) 1.76 - 2.09 (m, 8 H) 2.16 - 2.26 (m, 1 H) 3.30 - 3.59 (m, 2 H) 3.94 - 4.25 (m, 3 H) 6.57 - 6.66 (m, 1 H) 7.15 - 7.21 (m, 1 H) 7.51 - 7.64 (m, 2 H) 8.17 - 8.24 (m, 1 H). MS ESI/APCI Multi posi: 301 [M+H]$^+$.

The compounds of the following Examples 20-4 to 20-7 were synthesized using any of the compounds obtained in Reference Examples 2-11 to 2-14, according to the method described in Example 20-3. The structures, NMR data and MS data of these compounds are shown in Table 36-2.

[Table 36-2]

| Example No. | Structure | Analytical Data |
|---|---|---|
| 20-4 | 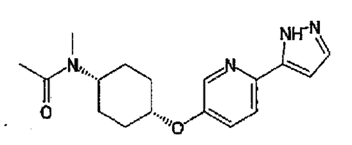 | $^1$H NMR (400 MHz, CHLOROFORM-d) 5 ppm 1.45 - 2.28 (m, 11 H) 2.88 (s, 1 H) 2.91 (s, 2 H) 3.60 - 3.68 (m, 0.3 H) 4.56 - 4. 64 (m, 1.7 H) 6.67 - 6.71 (m, 1 H) 7.25 - 7.29 (m, 1 H) 7.60 - 7.70 (m, 2 H) 8.29 - 8.32 (m, 1 H).<br>MS ESI/APCI Multi posi: 315[M+H]$^+$. |
| 20-5 | 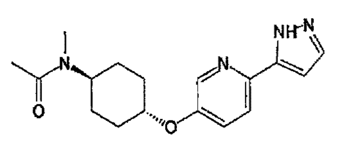 | $^1$H NMR (400 MHz, CHLOROFORM-d) δ ppm 1.41 - 2.33 (m, 11 H) 2.83 (s, 1 H) 2.87 (s, 2 H) 3.60 - 3.70 (m, 0.3 H) 4.16 - 4.26 (m, 1 H) 4.51 - 4.59 (m, 0.7 H) 6.67 - 6.70 (m, 1 H) 7.22 - 7.25 (m, 1 H) 7.61 - 7.67 (m, 2 H) 8.26 - 8.28 (m, 1 H).<br>MS ESI/APCI Multi posi: 315[M+H]$^+$. |
| 20-6 | 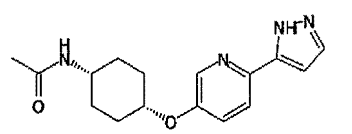 | $^1$H NMR (400 MHz, CHLOROFORM-d) δ ppm 1. 57 - 1.87 (m, 6 H) 1.99 (s, 3 H) 2.01 - 2. 07 (m, 2 H) 3.86 - 3. 95 (m, 1 H) 4. 54 - 4.68 (m, 1 H) 5.34 - 5.45 (m, 1 H) 6. 69 (d, J=2.2 Hz, 1 H) 7.24 (dd, J=8.6, 2.9 Hz, 1 H) 7.62 (d, J=2.2 Hz, 1 H) 7.65 (d, J=8.6 Hz, 1 H) 8.28 (d, J=2.9 Hz, 1 H).<br>MS ESI/APCI Multi posi: 301[M+H]$^+$. |
| 20-7 | 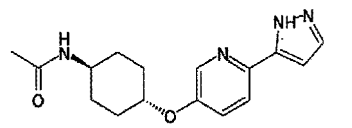 | $^1$H NMR (400 MHz, CHLOROFORM-d) δ ppm 1.21 - 2.20 (m, 11 H) 3.79 - 3.93 (m, 1 H) 4.21 - 4.28 (m, 1 H) 5.20 - 5.33 (m, 1 H) 6.68 (d, J=2.1 Hz, 1H) 7.24 (dd, J=8.7, 2.8 Hz, 1 H) 7.62 (d, J=2.1 Hz, 1H) 7.63 (d, J=8.7 Hz, 1 H) 8.26 (d, J=2.8 Hz, 1 H).<br>MS ESI/APCI Multi posi: 301[M+H]$^+$. |

Example 21-1

N-{[3-({[6-(1H-Pyrazol-5-yl)pyridin-3-yl]oxy}methyl)bicyclo[1.1.1]pentan-1-yl]methyl}acetamide

[Formula 223]

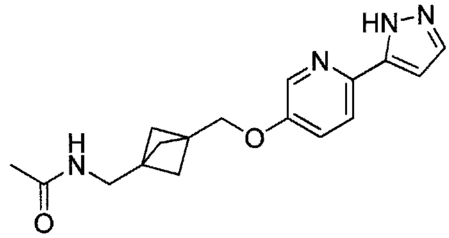

(1) The compound (149 mg) obtained in Reference Example 12-1 was used to perform the synthesis process according to the method described in Example 20-3-(1) thereby giving N-({3-[({6-[1-(oxan-2-yl)-1H-pyrazol-5-yl]pyridin-3-yl}oxy)methyl]bicyclo[1.1.1]pentan-1-yl}methyl)acetamide (146 mg) as a colorless oily substance.

(2) The compound (71.8 mg) obtained in the above described (1) was used to perform the synthesis process according to the method described in Example 1-1-(2) thereby giving the title compound (28.6 mg) as a colorless powder.
$^1$H NMR (600 MHz, CHLOROFORM-d) δ ppm 1.74 (s, 6 H) 2.00 (s, 3 H) 3.38 (d, J=6.2 Hz, 2 H) 4.06 (s, 2 H) 5.39 (br s, 1 H) 6.68 (br s, 1 H) 7.20 - 7.24 (m, 1 H) 7.57 - 7.66 (m, 2 H) 8.23 - 8.31 (m, 1 H).
MS ESI/APCI Multi posi: 313 $[M+H]^+$.

Example 22-1

N-Methyl-N-{[3-({[6-(1H-pyrazol-5-yl)pyridin-3-yl]oxy}methyl)bicyclo[1.1.1]pentan-1-yl]methyl}acetamide

[Formula 224]

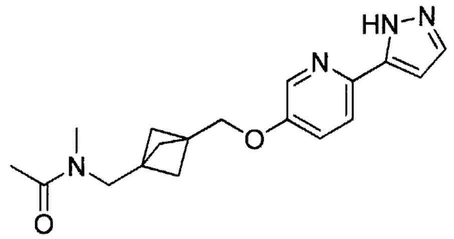

(1) The compound (73.7 mg) obtained in Example 21-1-(1) was used to perform the synthesis process according to the method described in Example 8-2-(1) thereby giving a mixture (120 mg) containing N-methyl-N-({3-[({6-[1-(oxan-2-yl)-1H-pyrazol-5-yl]pyridin-3-yl}oxy)methyl]bicyclo[1.1.1]pentan-1-yl}methyl)acetamide as a yellow oily substance.
(2) The compound (120 mg) obtained in the above described (1) was used to perform the synthesis process according to the method described in Example 1-1-(2) thereby giving the title compound (14.9 mg) as a colorless powder.
$^1$H NMR (400 MHz, CHLOROFORM-d) δ ppm 1.77 - 1.85 (m, 6 H) 2.05 - 2.10 (m, 3 H) 2.94 - 3.04 (m, 3 H) 3.39 - 3.52 (m, 2 H) 4.03 - 4.08 (m, 2 H) 6.66 - 6.70 (m, 1 H) 7.20 - 7.24 (m, 1 H) 7.60 - 7.66 (m, 2 H) 8.25 - 8.29 (m, 1 H).
MS ESI/APCI Multi posi: 327 $[M+H]^+$.

Example 23-1

1-[4-({[6-(4-Fluoro-1H-pyrazol-5-yl)pyridin-3-yl]oxy}methyl)piperidin-1-yl]ethan-1-one

[Formula 225]

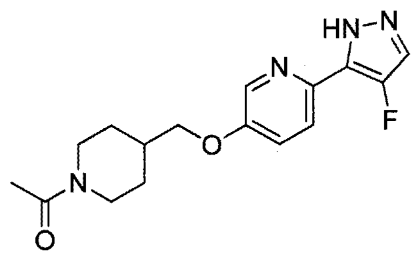

Selectfluor (registered trademark) (429 mg) was added to a suspension of the compound (182 mg) obtained in Example 1-1 in acetonitrile (8 mL), and the mixture was stirred at 60°C for 2 hours. After cooling the reaction solution

to 0°C, an aqueous solution of saturated sodium hydrogen carbonate was added to stop the reaction, and the mixture was extracted with chloroform twice. After drying the organic layer over anhydrous magnesium sulfate and filtering off the drying agent, the filtrate was concentrated under reduced pressure. The obtained residue was purified by preparative HPLC to give the title compound (17 mg) as a yellow powder.

$^1$H NMR (600 MHz, CHLOROFORM-d) δ ppm 1.28 - 1.40 (m, 2 H) 1.84 - 1.90 (m, 1 H) 1.93 - 1.99 (m, 1 H) 2.04 - 2.14 (m, 4 H) 2.58 - 2.65 (m, 1 H) 3.07 - 3.16 (m, 1 H) 3.86 - 3.95 (m, 3 H) 4.69 - 4.74 (m, 1 H) 7.28 (dd, J=8.7, 2.9 Hz, 1 H) 7.51 (d, J=4.1 Hz, 1 H) 7.71 (d, J=8.7 Hz, 1 H) 8.35 (d, J=2.9 Hz, 1 H).

MS ESI/APCI Multi posi: 319 [M+H]$^+$.

The compounds of the following Examples 23-2 to 23-6 were synthesized using the compound obtained in Examples 1-1, 18-8, 13-15, or 13-16, as well as a commercially available halogenating reagent, according to the method described in Example 23-1. The structures, NMR data and MS data of these compounds are shown in Table 37-1.

[Table 37-1]

| Example No. | Structure | Analytical Data |
|---|---|---|
| 23-2 | 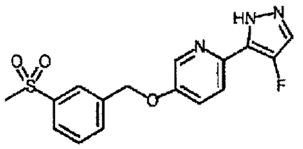 | $^1$H NMR (600 MHz, CHLOROFORM-d) δ ppm 1.17 (s, 3 H) 1.42 - 1.74 (m, 4 H) 2.10 (s, 3 H) 3.14 - 3.22 (m, 1 H) 3.32 - 3.41 (m, 1 H) 3.55 - 3.64 (m, 1 H) 3.72 - 3.81 (m, 2 H) 4.07 - 4.15 (m, 1 H) 6.63 - 6.73 (m, 1 H) 7.19 - 7.28 (m, 1 H) 7.58 - 7.70 (m, 2 H) 8.24 - 8.32 (m, 1 H).<br>MS ESI/APCI Multi posi: 315[M+H]$^+$. |
| 23-3 | 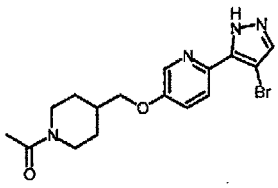 | $^1$H NMR (600 MHz, CHLOROFORM-d) δ ppm 1.21 - 1.31 (m, 1 H) 1.41 - 1.86 (m, 5 H) 1.89 - 1.98 (m, 1 H) 2.05 - 2.13 (m, 3 H) 2.50 - 3.13 (m, 2 H) 3.65 - 3.81 (m, 1 H) 4.05 - 4. 16 (m, 2 H) 4.33 - 4.44 (m, 1 H) 6.63 - 6.72 (m, 1 H) 7.20 - 7.27 (m, 1 H) 7.58 - 7.70 (m, 2 H) 8.23 - 8.31 (m, 1 H).<br>MS ESI/APCI Multi posi: 315[M+H]$^+$. |
| 23-4 | 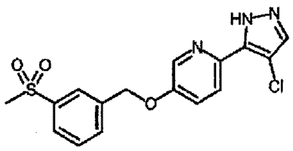 | $^1$H NMR (600 MHz, CHLOROFORM-d) δ ppm 1.20 - 1. 31 (m, 1 H) 1.37 - 1.86 (m, 5 H) 1.89 - 1. 98 (m, 1 H) 2.04 - 2.11 (m, 3 H) 2.50 - 3.12 (m, 2 H) 3.65 - 3.81 (m, 1 H) 4.04 - 4.16 (m, 2 H) 4.33 - 4.44 (m, 1 H) 6.63 - 6.71 (m, 1 H) 7.20 - 7.26 (m, 1 H) 7.57 - 7.70 (m, 2 H) 8.24 - 8.30 (m, 1 H).<br>MS ESI/APCI Multi posi: 315[M+H]$^+$. |
| 23-5 | 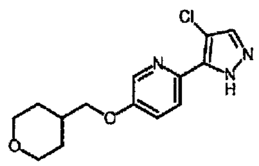 | $^1$H NMR (400 MHz, CHLOROFORM-d) δ ppm 1.40 - 1. 58 (m, 2 H) 1.71 - 1. 84 (m, 2 H) 2.04 - 2.20 (m, 1 H) 3. 38 - 3.52 (m, 2 H) 3.85 - 3.95 (m, 2 H) 4.00 - 4.09 (m, 2 H) 7.19 - 7.36 (m, 1 H) 7.58 (s, 1 H) 8.03 - 8.17 (m, 1 H) 8.30 (s, 1 H).<br>MS ESI/APCI Multi posi: 294[M+H]$^+$. |
| 23-6 | 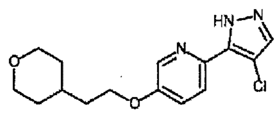 | $^1$H NMR (400 MHz, CHLOROFORM-d) δ ppm 1.28 - 1.90 (m, 7 H) 3.35 - 3.51 (m, 2 H) 3.93 - 4.03 (m, 2 H) 4.06 - 4.17 (m, 2 H) 7.20 -7.34 (m. 1 H) 7.57 (s, 1 H) 8.04 - 8.14 (m, 1 H) 8.25 - 8.35 (m, 1 H) 10.97 (s, 1 H).<br>MS ESI/APCI Multi posi: 308[M+H]$^+$. |

Example 24-1

1-[4-({[5-Fluoro-6-(1H-pyrazol-5-yl)pyridin-3-yl]oxy}methyl)piperidin-1-yl]ethan-1-one

[Formula 226]

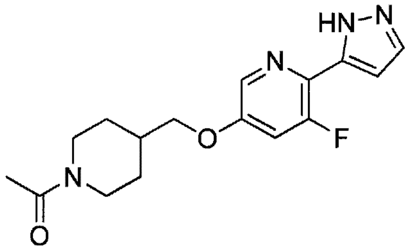

(1) 1-(Tetrahydro-2H-pyran-2-yl)-1H-pyrazole-5-boronic acid pinacol ester (93 mg), [1,1'-bis(diphenylphosphino)ferrocene]palladium(II) dichloride dichloromethane adduct (23 mg) and an aqueous solution (1 mL) of 2 mol/L cesium carbonate were added to a solution of the compound (80 mg) obtained in Reference Example 16-1 in dioxane (3 mL), and the mixture was stirred at 100°C for 6 hours. Water was added to the reaction solution, and the mixture was extracted with chloroform. The organic layer was separated by a phase separator, and the solvent was distilled off under reduced pressure. The obtained residue was purified by silica gel column chromatography (chloroform only, to chloroform/methanol = 19:1) to give 1-{4-[({5-fluoro-6-[1-(oxan-2-yl)-1H-pyrazol-5-yl]pyridin-3-yl}oxy)methyl]piperidin-1-yl}ethan-1-one (99 mg) as a pale brown amorphous substance.

(2) The compound (99 mg) obtained in the above described (1) was used to perform the synthesis process according to the method described in Example 1-1-(2) thereby giving the title compound (52 mg) as a colorless powder.

$^1$H NMR (600 MHz, DMSO-$d_6$) δ ppm 1.08 - 1.17 (m, 1 H) 1.22 - 1.30 (m, 1 H) 1.74 - 1.83 (m, 2 H) 1.95 - 2.07 (m, 4 H) 2.52 - 2.60 (m, 1 H) 3.01 - 3.09 (m, 1 H) 3.82 - 3.88 (m, 1 H) 3.96 - 4.04 (m, 2 H) 4.38 - 4.43 (m, 1 H) 6.65 - 6.72 (m, 1 H) 7.44 - 7.85 (m, 2 H) 8.20 - 8.28 (m, 1 H) 13.02 (br s, 0.4 H) 13.41 (br s, 0.6 H).

MS ESI/APCI Multi posi: 319 [M+H]$^+$.

The compounds of the following Examples 24-2 to 24-8 were synthesized using the compound obtained in Reference Examples 16 to 18 or 63, as well as a commercially available boronic acid ester or the compound obtained in Reference Example 66, according to the method described in Example 24-1. The structures, NMR data and MS data of these compounds are shown in Table 38-1 to 38-2.

[Table 38-1]

| Example No. | Structure | Analytical Data |
|---|---|---|
| 24-2 | 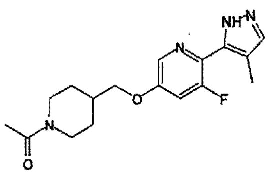 | $^1$H NMR (600 MHz, DMSO-$d_6$) δ ppm 1.09 - 1.17 (m, 1 H) 1.21 - 1.30 (m, 1 H) 1.74 - 1.84 (m, 2 H) 1.99 - 2.07 (m, 4 H) 2.11 (s, 3 H) 2.51 - 2.62 (m, 1 H) 3.00 - 3.10 (m, 1 H) 3.81 - 3.89 (m, 1 H) 3.96 - 4.05 (m, 2 H) 4.37 - 4.44 (m, 1 H) 7.36 - 7.64 (m, 2 H) 8.22 - 8.33 (m, 1 H) 12.72 (br s, 0.6 H) 12.92 (br s, 0.4 H). MS ESI/APCI Multi posi: 333[M+H]$^+$. |
| 24-3 | 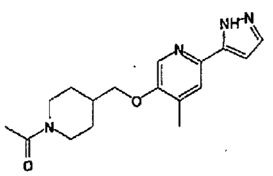 | $^1$H NMR (600 MHz, DMSO-$d_6$) δ ppm 1.11 - 1.19 (m, 1 H) 1.24 - 1.32 (m, 1 H) 1.77 - 1.86 (m, 2 H) 1.97 - 2.09 (m, 4 H) 2.22 (s, 3 H) 2.51 - 2.59 (m, 1 H) 2.99 - 3.13 (m, 1 H) 3.81 - 3.90 (m, 1 H) 3.95 - 4.05 (m, 2 H) 4.39 - 4.45 (m, 1 H) 6.70 - 6.72 (m, 1 H) 7.61 - 7.77 (m, 2 H) 8.18 - 8.23 (m, 1 H). MS ESI/APCI Multi posi: 315[M+H]$^+$. |
| 24-4 | 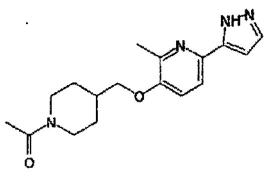 | $^1$H NMR (600 MHz, CHLOROFORM-d) δ ppm 1.29 - 1.41 (m, 2 H) 1.83 - 1.94 (m, 1 H) 1.94 - 2.02 (m, 1 H) 2.06 - 2.19 (m, 4 H) 2.52 (s, 3 H) 2.57 - 2.76 (m, I H) 3.08 - 3.19 (m, 1 H) 3.80 - 3.86 (m, 1 H) 3.86 - 3.95 (m, 2 H) 4.69 - 4.77 (m, 1 H) 6.68 (d, J=1.2 Hz, 1 H) 7.11 (br d, J=8.3 Hz, 1 H) 7.50 (br d, J=8.3 Hz, 1 H) 7.62 (br d, J=1.2 Hz, 1 H). MS ESI/APCI Multi posi: 315[M+H]$^+$. |

(continued)

| Example No. | Structure | Analytical Data |
|---|---|---|
| 24-5 | 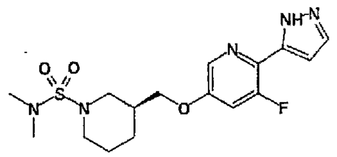 | $^1$H NMR (600 MHz, CHLOROFORM-d) δ ppm 1.31 - 1.47 (m, 1 H) 1.61 - 1.73 (m, 1 H) 1.76 - 1.85 (m, 1 H) 1.88 (br s, 1 H) 2.16 - 2.25 (m, 1 H) 2.76 - 2.89 (m, 7 H) 2.89 - 2.99 (m, 1 H) 3.52 - 3.64 (m, 1 H) 3.69 - 3.83 (m, 1 H) 3.89 - 3.95 (m, 1 H) 3.95 - 3.99 (m, 1 H) 6.77 - 6.84 (m, 1 H) 7.05 (dd, J=11. 8, 2.3 Hz, 1 H) 7.66 - 7.69 (m, 1 H) 8.14 - 8.19 (m, 1 H).<br>MS ESI/APCI Multi posi: 384[M+H]$^+$. |
| 24-6 | 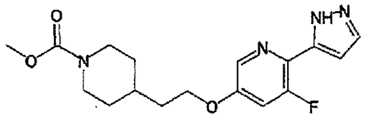 | $^1$H NMR (600 MHz, CHLOROFORM-d) δ ppm 1.14 - 1.28 (m, 2 H) 1.69 - 1.82 (m, 5 H) 2.71 - 2.85 (m, 2 H) 3.70 (s, 3 H) 4.02 - 4.27 (m, 4 H) 6.77 - 6.83 (m, 1 H) 7.03 (dd, J=12.0, 2. 1 Hz, 1 H) 7.67 (d, J=1.7 Hz, 1 H) 8.15 (d, J=1.2 Hz, 1 H).<br>MS ESI/APCI Multi posi: 349[M+H]$^+$. |

[Table 38-2]

| Example No. | Structure | Analytical Data |
|---|---|---|
| 24-7 | 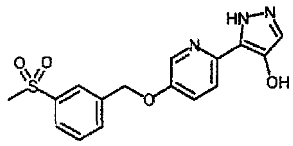 | $^1$H NMR (400 MHz, DMSO-d$_6$) δ ppm 3.25 (s, 3 H) 5.35 (s, 2 H) 7.05 - 7.46 (m, 1 H) 7.59 - 7.66 (m, 1 H) 7.67 - 7.76 (m, 1 H) 7.81 - 7.88 (m, 2 H) 7.93 (d, J=7.7 Hz, 1 H) 8.06 (s, 1 H) 8.38 (d, J=2.8 Hz, 1 H) 8.95 - 9.56 (m, 1 H) 12.20 - 12.97 (m, 1 H).<br>MS ESI/APCI Multi posi: 345[M+H]$^+$. |
| 24-8 | 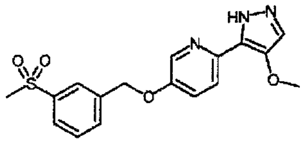 | $^1$H NMR (400 MHz, CHLOROFORM-d) δ ppm 3.08 (s, 3 H) 3.93 (s, 3 H) 5.22 (s, 2 H) 7.32 (dd, J=8.8, 2.9 Hz, 1 H) 7.42 (s, 1 H) 7.69 - 7.67 (m, 1 H) 7.75 (d, J=7.7 Hz, 1 H) 7.88 (d, J=8.8 Hz, 1 H) 7.94 (d, J=7.7 Hz, 1 H) 8.06 (s, 1 H) 8.34 (d, J=2.9 Hz, 1 H) 10.62 (br s, 1 H).<br>MS ESI/APCI Multi posi: 360[M+H]$^+$. |

Example 25-3

N,N-Dimethyl-4-{[6-(1H-pyrazol-5-yl)pyridin-3-yl]oxymethyl}benzenesulfonamide

[Formula 227]

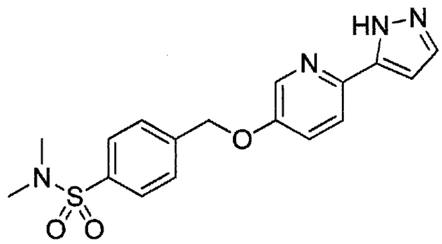

(1) Potassium carbonate (10.3 mg) was added to a solution of the compound (15.3 mg) obtained in Reference Example 1-1 and the compound (20.8 mg) obtained in Reference Example 23-1 in N,N-dimethylformamide (1 mL), and the mixture was stirred at 50°C for 1 hour. After cooling to room temperature, water was added to the reaction mixture, and the mixture was extracted with ethyl acetate. The organic layer was washed with brine, and then dried

over anhydrous magnesium sulfate. The drying agent was filtered off, and the filtrate was concentrated under reduced pressure to give a mixture (87 mg) containing N,N-dimethyl-4-({6-[2-(oxan-2-yl)pyrazol-3-yl]pyridin-3-yl} oxymethyl)benzenesulfonamide.

(2) The mixture (37 mg) obtained in the above described (1) was used to perform the synthesis process according to the method described in Example 1-1-(2) thereby giving the title compound (6.2 mg) as a colorless powder.

$^1$H NMR (600 MHz, DMSO-$d_6$) δ ppm 2.57 - 2.66 (m, 6 H) 5.35 (br s, 2 H) 6.74 (br s, 1 H) 7.48 - 7.66 (m, 1.4 H) 7.71 - 7.84 (m, 4.6 H) 7.86 - 8.05 (m, 1 H) 8.34 - 8.44 (m, 1 H) 12.90 (br s, 0.7 H) 13.35 (br s, 0.3 H).

MS ESI/APCI Multi posi: 359 [M+H]$^+$.

The compounds of the following Examples 25-4 to 25-16, 25-18 to 24, 25 -28, and 25-29 were synthesized using any of the compounds obtained in Reference Examples 1-1 to 1-3, as well as the compound obtained in Reference Example 23-1 or 24-1 or a commercially available benzyl halide, according to the method described in Example 25-3. The structures, NMR data and MS data of these compounds are shown in Table 39-1 to 39-4.

[Table 39-1]

| Example No. | Structure | Analytical Data |
|---|---|---|
| 25-4 | 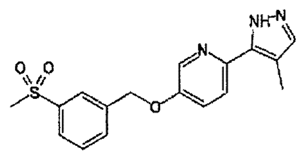 | $^1$H NMR (400 MHz, CHLOROFORM-d) δ ppm 2.36 (s, 3 H) 3.09 (s, 3 H) 5.23 (s, 2 H) 7.35 (dd, J=8. 7, 3.0 Hz, 1 H) 7.42 - 7.47 (m, 1 H) 7.59 - 7.68 (m, 2 H) 7.73 - 7.78 (m, 1 H) 7.93 - 7.97 (m, 1 H) 8.06 - 8.08 (m, 1 H) 8.40 (d, J=3.0 Hz, 1 H) 10.70 (br s, 1 H).<br>MS ESI/APCI Multi posi: 344[M+H]$^+$. |
| 25-5 | 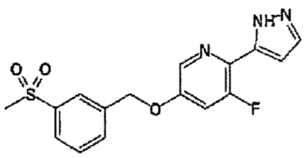 | $^1$H NMR (400 MHz, CHLOROFORM-d) δ ppm 3.09 (s, 3 H) 6.23 (s, 2 H) 6.83 (dd, J=3.8, 2.0 Hz, 1 H) 7.15 (dd, J=11. 6, 2.3 Hz, 1 H) 7.63 - 7.68 (m, 1 H) 7.68 - 7.71 (m, 1 H) 7.71 - 7.77 (m, 1 H) 7.93 - 8.00 (m, 1 H) 8.06 (s, 1 H) 8.26 (dd, J=2.3, 0.9 Hz, 1 H).<br>MS ESI/APCI Multi posi: 348[M+H]$^+$. |
| 25-6 | 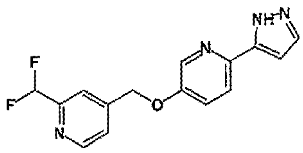 | MS ESI posi: 303[M+H]$^+$. |
| 25-7 | 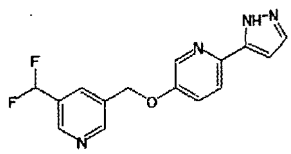 | MS ESI posi: 303[M+H]$^+$. |
| 25-8 | 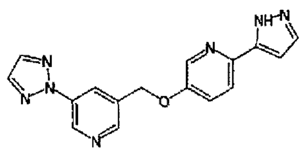 | MS ESI posi: 320[M+H]$^+$. |
| 25-9 | 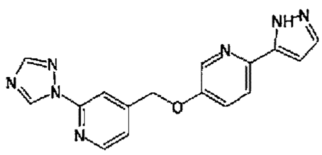 | MS ESI posi: 320[M+H]$^+$. |

[Table 39-2]

| Example No. | Structure | Analytical Data |
|---|---|---|
| 25-10 | 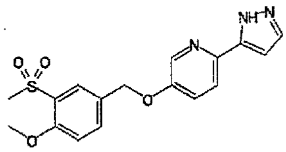 | MS ESI posi: 360[M+H]$^+$. |
| 25-11 | 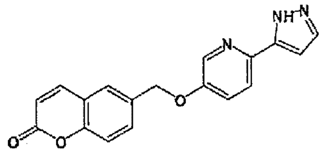 | MS ESI posi: 320[M+H]$^+$. |
| 25-12 | 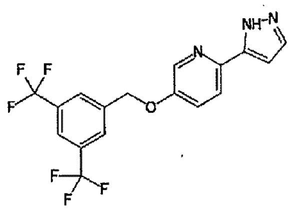 | MS ESI posi: 388[M+H]$^+$. |
| 25-13 | 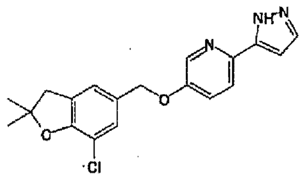 | MS ESI posi: 356[M+H]$^+$. |
| 25-14 | 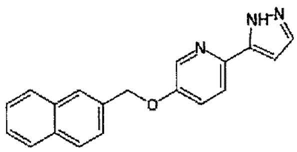 | MS ESI posi: 302[M+H]$^+$. |
| 25-15 | 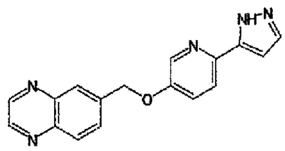 | MS ESI posi: 304[M+H]$^+$. |
| 25-16 | 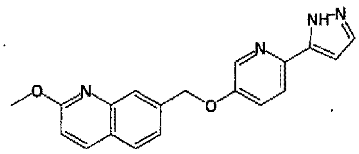 | MS ESI posi: 333[M+H]$^+$. |

[Table 39-3]

| Example No. | Structure | Analytical Data |
|---|---|---|
| 25-18 | 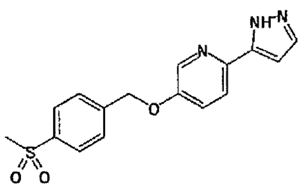 | MS ESI posi: 330[M+H]$^+$. |

(continued)

| Example No. | Structure | Analytical Data |
|---|---|---|
| 25-19 | 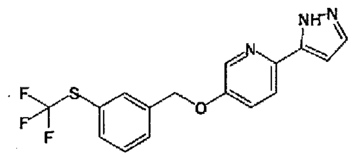 | MS ESI posi: 352[M+H]$^+$. |
| 25-20 | 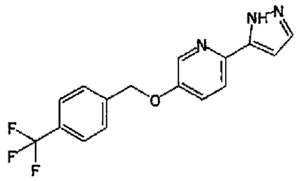 | MS ESI posi: 320[M+H]$^+$. |
| 25-21 | 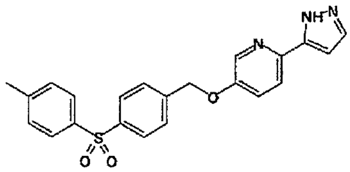 | MS ESI posi: 406[M+H]$^+$. |
| 25-22 | 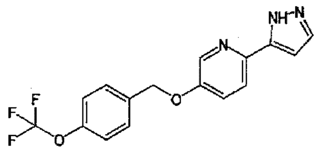 | MS ESI posi: 336[M+H]$^+$. |
| 25-23 | 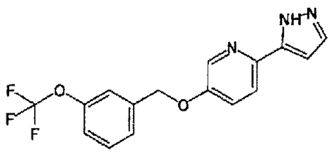 | MS ESI posi: 336[M+H]$^+$. |

[Table 39-4]

| Example No. | Structure | Analytical Data |
|---|---|---|
| 25-24 | 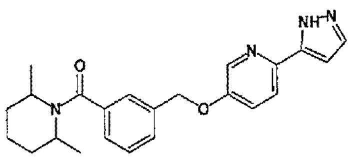 | MS ESI posi: 391[M+H]$^+$. |
| 25-28 | 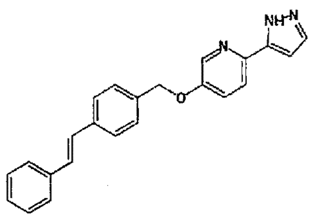 | MS ESI posi: 354[M+H]$^+$. |
| 25-29 | 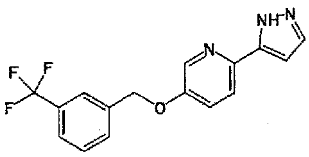 | MS ESI posi: 320[M+H]$^+$. |

Example 39-1

1-(4-{[6-(1H-Pyrazol-4-yl)pyridin-3-yl]oxymethyl}piperidin-1-yl)ethanone

[Formula 228]

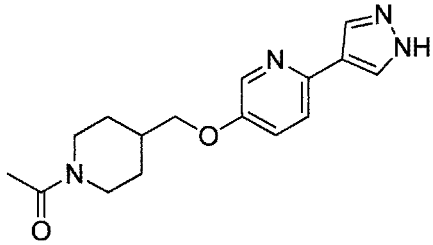

(1) The compound (174 mg) obtained in Reference Example 62-1 was used to perform the synthesis process according to the method described in Reference Example 1-6-(1) thereby giving 1-[4-({6-[1-(oxan-2-yl)pyrazol-4-yl]pyridin-3-yl}oxymethyl)piperidin-1-yl]ethanone (129 mg) as a pale yellow solid.

(2) The compound obtained in the above described (1) was used to perform the synthesis process according to the method described in Example 1-1-(2) thereby giving the title compound (62.0 mg) as a colorless powder.
$^1$H NMR (400 MHz, DMSO-$d_6$) δ ppm 1.04 - 1.19 (m, 1 H) 1.18 - 1.33 (m, 1 H) 1.63 - 1.76 (m, 2 H) 1.94 - 2.07 (m, 4 H) 2.52 - 2.60 (m, 1 H) 2.98 - 3.10 (m, 1 H) 3.79 - 3.88 (m, 1 H) 3.92 (d, J=6.4 Hz, 2 H) 4.35 - 4.45 (m, 1 H) 7.38 (dd, J=8.7, 3.0 Hz, 1 H) 7.60 (d, J=8.7 Hz, 1 H) 7.85 - 8.36 (m, 3 H) 12.92 (br s, 1 H).
MS ESI/APCI Multi posi: 301 $[M+H]^+$.

The compound of the following Example 39-2 was synthesized using the compound obtained in Reference Example 64 and a commercially available boronic acid ester according to the method described in Example 24-1. The structure, NMR data and MS data of the compound are shown in Table 40-1.

[Table 40-1]

| Example No. | Structure | Analytical Data |
|---|---|---|
| 39-2 | 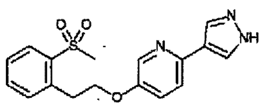 | $^1$H NMR (400 MHz, CHLOROFORM-d) δ ppm 3.15 (s, 3 H) 3.55 (t, J=6.5 Hz, 2 H) 4.37 (t, J=6.5 Hz, 2 H) 7.18 - 7.23 (m, 1 H) 7.33 - 7.68 (m, 4 H) 7.99 - 8.13 (m, 3 H) 8.26 (d, J=2.7 Hz, 1 H). MS ESI/APCI Multi posi: 344$[M+H]^+$. |

Example 40-1

5-[(3-Methylsulfonylphenyl)methoxy]-2-[4-(trifluoromethyl)-1H-pyrazol-5-yl]pyridine

[Formula 229]

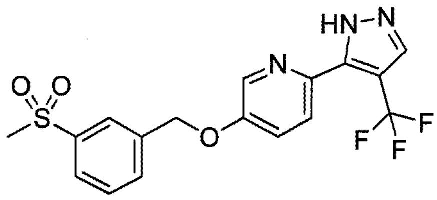

(1) The compound (379 mg) obtained in Example 18-8-(1) and N-iodosuccinimide (252 mg) were used to perform the synthesis process according to the method described in Example 23-1 thereby giving 2-[4-iodo-2-(oxan-2-yl)pyrazol-3-yl]-5-[(3-methylsulfonylphenyl)methoxy]pyridine (455 mg) as a colorless amorphous substance.
(2) The compound (455 mg) obtained in the above described (1) was dissolved in N,N-dimethylformamide (1.8 mL) and the system was placed in a nitrogen atmosphere. Trifluoromethylator (registered trademark) (660 mg) was added, and the mixture was stirred at 70°C for 3 hours. After cooling to room temperature, a sodium chloride solution was added to stop the reaction, and the reaction mixture was extracted with ethyl acetate three times. After removing moisture by a phase separator, the combined organic layers were concentrated under reduced pressure to give a mixture (227 mg) containing 5-[(3-methylsulfonylphenyl)methoxy]-2-[2-(oxan-2-yl)-4-(trifluoromethyl)pyrazol-3-yl]pyridine.
(3) The mixture (227 mg) obtained in the above described (2) was used to perform the synthesis process according to the method described in Example 1-1-(2) thereby giving the title compound (40 mg) as a white powder.
$^1$H NMR (400 MHz, CHLOROFORM-d) δ ppm 3.09 (s, 3 H) 5.25 (s, 2 H) 7.23 - 7.33 (m, 1 H) 7.39 (d, J=8.7 Hz, 1 H) 7.56 - 8.81 (m, 6 H).
MS ESI/APCI Multi posi: 398 $[M+H]^+$.
MS ESI/APCI Multi nega: 396 $[M-H]^-$.

Example 41-1

5-[(3-Methylsulfonylphenyl)methoxy]-2-(1H-pyrazol-4-yl)pyridine

[Formula 230]

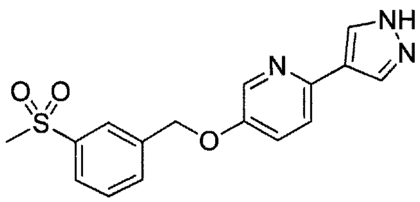

The compound (200 mg) obtained in Reference Example 63-1 and commercially available 4-(4,4,5,5-tetramethyl-1,3,2-dioxaborolan-2-yl)-1H-pyrazole (147 mg) were used to perform the synthesis process according to the method described in Reference Example 1-6-(1) thereby giving the title compound (94.2 mg) as a colorless solid.
$^1$H NMR (400 MHz, CHLOROFORM-d) δ ppm 3.08 (s, 3 H) 5.21 (s, 2 H) 7.30 (dd, J=8.7, 2.9 Hz, 1 H) 7.46 (d, J=8.7 Hz, 1 H) 7.63 (dd, J=7.7, 7.6 Hz, 1 H) 7.72 - 7.81 (m, 1 H) 7.91 - 7.96 (m, 1 H) 8.02 - 8.07 (m, 3 H) 8.35 (d, J=2.9 Hz, 1 H).
MS ESI/APCI Multi posi: 330 $[M+H]^+$.
The compounds of the following Examples 41-2 and 41-3 were synthesized using a commercially available boronic acid ester according to the method described in Example 41-1. The structures, NMR data and MS data of these compounds are shown in Table 41-1.

[Table 41-1]

| Example No. | Structure | Analytical Data |
|---|---|---|
| 41-2 | 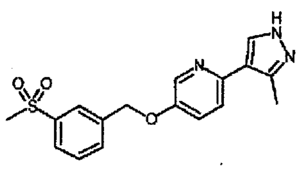 | $^1$H NMR (400 MHz, CHLOROFORM-d) δ ppm 2.59 (s, 3 H) 3.08 (s, 3 H) 5.21 (s, 2 H) 7.30 (dd, J=8.7. 2.9 Hz, 1 H) 7.41 (d, J=8.7 Hz, 1 H) 7.63 (dd, J=7.7, 7.5 Hz, 1 H) 7.74 - 7.77 (m, 1 H) 7.89 (s, 1 H) 7.92 - 7.96 (m, 1 H) 8.05 - 8.07 (m, 1 H) 8.38 (d, J=2.9 Hz, 1 H).<br>MS ESI/APCI Multi posi: 344$[M+H]^+$. |

(continued)

| Example No. | Structure | Analytical Data |
|---|---|---|
| 41-3 | 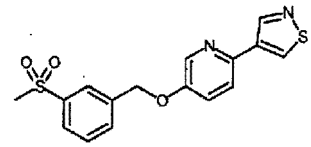 | $^{1}H$ NMR (400 MHz, CHLOROFORM-d) δ ppm 3.09 (s, 3 H) 5.23 (s, 2 H) 7.34 (dd, J=8.7, 2.9 Hz, 1 H) 7.55 - 7.70 (m, 2 H) 7.70 - 7.81 (m, 1 H) 7.89 - 8.01 (m, 1 H) 8.01 - 8.13 (m, 1 H) 8.42 (d, J=2.9 Hz, 1 H) 8.97 - 9.02 (m, 2 H). MS ESI/APCI Multi posi: 347[M+H]$^{+}$. MS ESI/APCI Multi nega: 345[M-H]$^{-}$. |

Example 42-1

1-(4-{[6-(1,2-Thiazol-5-yl)pyridin-3-yl]oxymethyl}piperidin-1-yl)ethanone

[Formula 231]

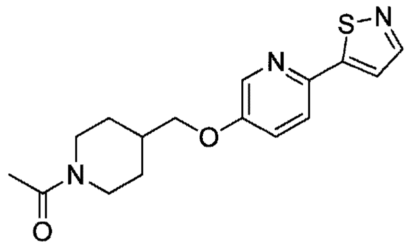

Tetrakis(triphenylphosphine)palladium(0) (18.5 mg) and hexamethyldistannane (57.5 mg) were added to a solution of the compound (50 mg) obtained in Reference Example 62-1 and 5-bromo-1,2-thiazole (28.8 mg) in 1,4-dioxane (4 mL), evacuation and nitrogen introduction were repeated three times, and the air in the vessel was purged with nitrogen. This mixture was stirred at 140°C for 1 hour under microwave irradiation, and after cooling to room temperature, the solvent was distilled off under reduced pressure. The residue was purified by silica gel column chromatography (chloroform/methanol = 1:0 to 19:1) and then by preparative thin layer chromatography (developed three times with ethyl acetate), and solidified from ether to give the title compound (5.24 mg) as a colorless solid.
$^{1}H$ NMR (400 MHz, DMSO-$d_6$) δ ppm 1.09 - 1.31 (m, 2 H) 1.73 - 1.86 (m, 2 H) 1.96 - 2.06 (m, 4 H) 2.56 - 2.66 (m, 1 H) 3.01 - 3.10 (m, 1 H) 3.78 - 3.93 (m, 1 H) 3.93 - 4.08 (m, 2 H) 4.28 - 4.52 (m, 1 H) 7.55 (dd, J=8.7, 2.8 Hz, 1 H) 7.89 (d, J=1.7 Hz, 1 H) 7.99 (d, J=8.7 Hz, 1 H) 8.33 (d, J=2.8 Hz, 1 H) 8.57 (d, J=1.7 Hz, 1 H).
MS ESI/APCI Multi posi: 318 [M+H]$^{+}$.

The compound of the following Example 42-2 was synthesized using the compound obtained in Reference Example 63-1, according to the method described in Example 42-1. The structure, NMR data and MS data of the compound are shown in Table 42-1.

[Table 42-1]

| Example No. | Structure | Analytical Data |
|---|---|---|
| 42-2 | 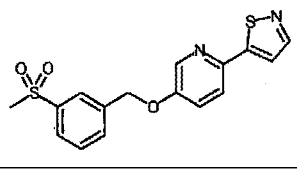 | $^{1}H$ NMR (400 MHz, DMSO-$d_6$) δ ppm 3.25 (s, 3 H) 5.39 (s, 2 H) 7.63 - 1.76 (m, 2 H) 7.83 - 7.96 (m, 3 H) 8.01 - 8.09 (m, 2 H) 8.44 (d, J=2. 8 Hz, 1 H) 8.56 - 8.60 (m, 1 H). MS ESI/APCI Multi posi: 347[M+H]$^{+}$. MS ESI/APCI Multi nega: 345[M-H]$^{-}$. |

Example 43-1

1-(4-{[6-(1H-Triazol-4-yl)pyridin-3-yl]oxymethyl}piperidin-1-yl)ethanone

[Formula 232]

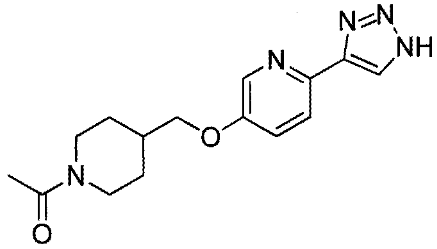

(1) The compound (26.3 mg) obtained in Reference Example 1-7 and the compound (19.7 mg) obtained in Reference Example 25-1 were used to perform the synthesis process according to the method described in Example 13-1-(1) thereby giving 1-(4-{[6-(1-benzyltriazol-4-yl)pyridin-3-yl]oxymethyl}piperidin-1-yl)ethanone (24.7 mg) as a pale yellow solid.

(2) A solution of potassium tert-butoxide (70.8 mg) in tetrahydrofuran (1.5 mL) was added to a solution of the compound (24.7 mg) obtained in the above described (1) in dimethylsulfoxide (0.5 mL) and tetrahydrofuran (1 mL) under ice cooling, and the mixture was stirred for 10 minutes while aerating oxygen. An aqueous solution of saturated ammonium chloride was added under ice cooling, and the mixture was extracted with ethyl acetate. The organic layer was separated and dried over anhydrous magnesium sulfate, and the drying agent was filtered off. After distilling off the solvent under reduced pressure, the residue was purified by preparative HPLC to give the title compound (4.75 mg) as a colorless solid.
$^1$H NMR (400 MHz, DMSO-$d_6$) δ ppm 1.07 - 1.32 (m, 2 H) 1.71 - 1.94 (m, 2 H) 1.95 - 2.10 (m, 4 H) 2.54 - 2.64 (m, 1 H) 2.98 - 3.13 (m, 1 H) 3.76 - 3.90 (m, 1 H) 3.90 - 4.04 (m, 2 H) 4.35 - 4.47 (m, 1 H) 7.50 (dd, J=8.7, 2.8 Hz, 1 H) 7.90 (d, J=8.7 Hz, 1 H) 8.21 (s, 1 H) 8.32 (d, J=2.8 Hz, 1 H).
MS ESI/APCI Multi posi: 302 $[M+H]^+$.

The compounds of the following Examples 43-2 to 43-6 were synthesized using the compound obtained in Reference Examples 1-6 or 59-1, as well as the compound obtained in Reference Examples 25-1, 69, or a commercially available alcohol, according to the method described in Example 43-1-(1) and Examples 1-1-(2) or 2-11-(2). The structures, NMR data and MS data of these compounds are shown in Table 43-1.

[Table 43-1]

| Example No. | Structure | Analytical Data |
|---|---|---|
| 43-2 | 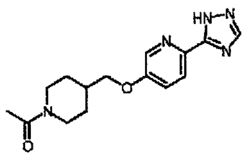 | $^1$H NMR (400 MHz, DMSO-$d_6$) δ ppm 1.07 - 1.37 (m, 2 H) 1.71 - 1.89 (m, 2 H) 1.96 - 2.10 (m, 4 H) 2.55 - 2.59 (m, 1 H) 3.00 - 3.12 (m, 1 H) 3.80 - 3. 90 (m, 1 H) 3. 96 - 4. 05 (m, 2 H) 4.36 - 4.46 (m, 1 H) 7.52 - 7.59 (m, 1 H) 7.98 - 8.23 (m, 2 H) 8.34 - 8.42 (m, 1 H) 14.40 (br s, 1 H).<br>MS ESI/APCI Multi posi: 302$[M+H]^+$. |
| 43-3 | 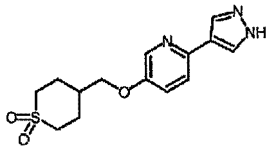 | $^1$H NMR (400 MHz, DMSO-$d_6$) δ ppm 1.70 - 1.86 (m, 2 H) 2.06 - 2.22 (m, 3 H) 3.03 - 3.12 (m, 2 H) 3.14 - 3.25 (m, 2 H) 3.93 - 4.03 (m, 2 H) 7.39 (dd, J=8.7, 2.9 Hz, 1 H) 7.62 (d, J=8.7 Hz, 1 H) 7.90 - 8.07 (m, 1 H) 8. 12 - 8.22 (m, 1 H) 8.23 (d, J=2.9 Hz, 1 H) 12.94 (br s, 1 H).<br>MS ESI/APCI Multi posi: 308$[M+H]^+$. |
| 43-4 | 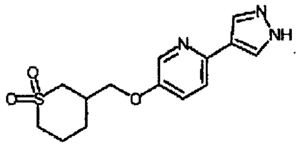 | $^1$H NMR (400 MHz, DMSO-$d_6$) δ ppm 1.32 - 1.51 (m, 1 H) 1.76 - 1.96 (m, 2 H) 1.99 - 2.20 (m, 1 H) 2.37 - 2.51 (m, 1 H) 3.03 - 3.29 (m, 4 H) 3.91 - 4.09 (m, 2 H) 7.39 (dd, J=8.7, 2.8 Hz, 1 H) 7.62 (d, J=8.7 Hz, 1 H) 7.82 - 8.08 (m, 1 H) 8.12 - 8.23 (m, 1 H) 8.24 (d, J=2.8 Hz, 1 H) 12.71 - 13.16 (m, 1 H).<br>MS ESI/APCI Multi posi: 308$[M+H]^+$. |

(continued)

| Example No. | Structure | Analytical Data |
|---|---|---|
| 43-5 | 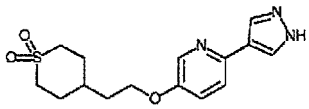 | $^1$H NMR (400 MHz, DMSO-$d_6$) δ ppm 1.62 - 1.89 (m, 5 H) 2.04 - 2.13 (m, 2 H) 2.97 - 3.06 (m, 2 H) 3.09 - 3.21 (m, 2 H) 4.05 - 4.14 (m, 2 H) 7.38 (dd, J=8.7, 2.9 Hz, 1 H) 7.61 (d, J=8.7 Hz, 1 H) 7.93 - 8.01 (m, 1 H) 8.17 - 8.21 (m, 1 H) 8.22 (d, J=2.9 Hz, 1 H) 12.87 - 13.06 (m, 1 H).<br>MS ESI/APCI Multi posi: 322[M+H]$^+$. |
| 43-6 | 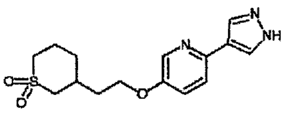 | $^1$H NMR (400 MHz, DMSO-$d_6$) δ ppm 1.20 - 1. 35 (m, 1 H) 1.71 - 1.88 (m, 4 H) 1.95 - 2.10 (m, 1 H) 2.11 - 2.26 (m, 1 H) 2.91 - 3.23 (m, 4 H) 4. 11 (t, J=6.4 Hz, 2 H) 7.38 (dd, J=8.6, 2.9 Hz, 1 H) 7.61 (d, J=8.6 Hz, 1 H) 7.91 - 8.01 (m, 1 H) 8.16 - 8.21 (m, 1 H) 8.22 (d, J=2.9 Hz, 1 H) 12.86 - 12.99 (m, 1 H).<br>MS ESI/APCI Multi posi: 322[M+H]$^+$. |

Example 44-1

5-[(3 -Methylsulfonylphenyl)methoxy]-2-(1H-triazol-4-yl)pyridine

[Formula 233]

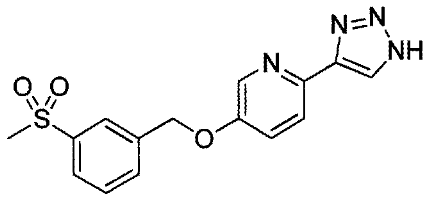

(1) The compound (200 mg) obtained in Reference Example 63-1 and trimethylsilylacetylene (68.9 mg) were dissolved in N,N-dimethylformamide (1.17 mL), and triethylamine (244 μL), tetrakis(triphenylphosphine)palladium(0) (82.1 mg), and copper(I) iodide (22.3 mg) were added. After deaerating the mixed solution and filling the vessel with nitrogen, the mixture was stirred at 100°C for 30 minutes under microwave irradiation. After cooling to room temperature, methanol (1 mL) and an aqueous solution (1 mL) of 1 mol/L sodium hydroxide were added, and the mixture was stirred for 30 minutes at room temperature. An aqueous solution of saturated ammonium chloride and ethyl acetate were added, insolubles were filtered off, and the filtrate was extracted with ethyl acetate. The obtained organic layer was washed with water and then with brine, and dried over anhydrous sodium sulfate. After filtering off the drying agent, the solvent was distilled off under reduced pressure. The obtained residue was purified by silica gel column chromatography (n-hexane only to ethyl acetate only) to give 2-ethynyl-5-[(3-methylsulfonylphenyl)methoxy]pyridine (92.4 mg) as a brown oily substance.

(2) Sodium azide (20.4 mg) and copper(I) iodide (3.99 mg) were added to a solution of the compound (60.2 mg) obtained in the above described (1) in N,N-dimethylformamide (2 mL), and the mixture was stirred at room temperature for 1 hour, at 50°C for 1 hour, and at 100°C for 4 hours. After cooling to room temperature, water was added and the mixture was extracted with ethyl acetate. The organic layer was separated and the solvent was distilled off under reduced pressure. The obtained residue was purified by preparative HPLC to give the title compound (1.51 mg) as a yellow solid.

$^1$H NMR (400 MHz, ACETONE-$d_6$) δ ppm 3.15 (s, 3 H) 5.43 (s, 2 H) 7.56 - 7.66 (m, 1 H) 7.67 - 7.80 (m, 1 H) 7.86 - 8.04 (m, 3 H) 8.08 - 8.30 (m, 2 H) 8.32 - 8.59 (m, 1 H).

MS ESI/APCI Multi posi: 331 [M+H]$^+$.

Example 45-1

1-(4-{[6-(Triazol-1-yl)pyridin-3-yl]oxymethyl}piperidin-1-yl)ethanone

[Formula 234]

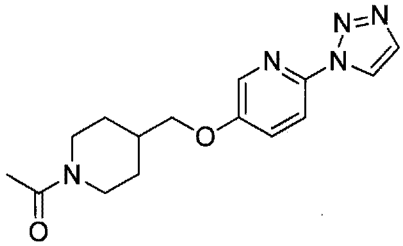

The compound (12.0 mg) obtained in Reference Example 57-1 and the compound (14.0 mg) obtained in Reference Example 25-1 were used to perform the synthesis process according to the method described in Example 13-1-(1) thereby giving the title compound (5.86 mg) as a colorless solid.
$^1$H NMR (400 MHz, CHLOROFORM-d) δ ppm 1.27 - 1.43 (m, 2 H) 1.84 - 2.00 (m, 2 H) 2.05 - 2.16 (m, 4 H) 2.57 - 2.66 (m, 1 H) 3.07 - 3.17 (m, 1 H) 3.85 - 3.98 (m, 3 H) 4.68 - 4.77 (m, 1 H) 7.41 (dd, J=8.9, 2.9 Hz, 1 H) 7.81 (d, J=1.1 Hz, 1 H) 8.10 - 8.18 (m, 2 H) 8.49 (d, J=1.1 Hz, 1 H).
MS ESI/APCI Multi posi: 302 [M+H]$^+$.

The compounds of the following Examples 45-2 to 45-6 were synthesized using the compound obtained in Reference Example 57, 58 or 60, as well as the compound obtained in Reference Example 25-1 or a commercially available alcohol, according to the method described in Example 45-1. The structures, NMR data and MS data of these compounds are shown in Table 44-1.

[Table 44-1]

| Example No. | Structure | Analytical Data |
|---|---|---|
| 45-2 | 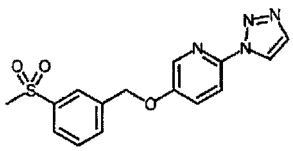 | $^1$H NMR (400 MHz, CHLOROFORM-d) δ ppm 3.09 (s, 3 H) 5.25 (s, 2 H) 7.51 (dd, J=8.9, 2.9 Hz, 1 H) 7.59 - 7.70 (m, 1 H) 7.74 - 7.77 (m, 1 H) 7.82 (d, J=1.0 Hz, 1 H) 7.93 - 8.00 (m, 1 H) 8.04 - 8.10 (m, 1 H) 8.17 (d, J=8.9 Hz, 1 H) 8.24 (d, J=2.9 Hz, 1 H) 8.50 (d, J=1.0 Hz, 1 H).<br>MS ESI/APCI Multi posi: 331[M+H]$^+$. |
| 45-3 | 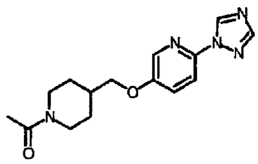 | $^1$H NMR (400 MHz, DMSO-$d_6$) δ ppm 1.07 - 1.33 (m, 2 H) 1.71 - 1.89 (m, 2 H) 1.96 - 2.08 (m, 4 H) 2.52 - 2.61 (m, 1 H) 2.97 - 3.13 (m, 1 H) 3.79 - 3.93 (m, 1 H) 3.95 - 4.05 (m, 2 H) 4.35 - 4.47 (m, 1 H) 7.68 (dd, J=8.9, 2.9 Hz, 1 H) 7.80 (d, J=8.9 Hz, 1 H) 8.18 - 8.30 (m, 2 H) 9.24 (s, 1 H).<br>MS ESI/APCI Multi posi: 302[M+H]$^+$. |
| 45-4 | 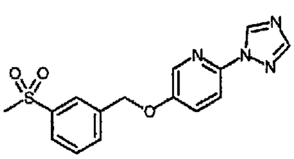 | $^1$H NMR (400 MHz, DMSO-$d_6$) δ ppm 3.25 (s, 3 H) 5.39 (s, 2 H) 7.69 - 7.75 (m, 1 H) 7.80 (dd, J=9.0, 2.9 Hz, 1 H) 7.82 - 7.88 (m, 2 H) 7.89 - 7.97 (m, 1 H) 8.04 - 8.09 (m, 1 H) 8.25 (s, 1 H) 8.35 (d, J=2.9 Hz, 1 H) 9.26 (s, 1 H).<br>MS ESI/APCI Multi posi: 331 [M+H]$^+$. |
| 45-5 | 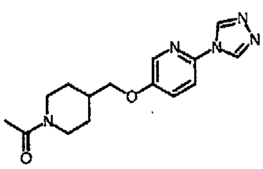 | $^1$H NMR (400 MHz, DMSO-$d_6$) δ ppm 1.08 - 1.32 (m, 2 H) 1.72 - 1.90 (m, 2 H) 1.94 - 2.09 (m, 4 H) 2.55 - 2. 66 (m, 1 H) 3.00 - 3.12 (m, 1 H) 3.80 - 3.92 (m, 1 H) 3.96 - 4.04 (m, 2 H) 4.36 - 4.46 (m, 1 H) 7.71 (dd, J=8.9, 2.9 Hz, 1 H) 7.82 (d, J=8.9 Hz, 1 H) 8.25 (d, J=2.9 Hz, 1 H) 9.19 (s, 2 H).<br>MS ESI/APCI Multi posi: 302[M+H]$^+$. |

(continued)

| Example No. | Structure | Analytical Data |
|---|---|---|
| 45-6 | 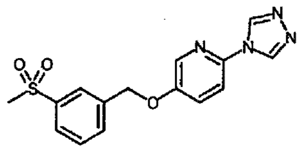 | $^1$H NMR (400 MHz, DMSO-$d_6$) δ ppm 3.24 (s, 3 H) 5.39 (s, 2 H) 7.68 - 7.75 (m, 1 H) 7.80 - 7.88 (m, 3 H) 7.90 - 7.96 (m, 1 H) 8.04 - 8.08 (m, 1 H) 8.34 - 8.38 (m, 1 H) 9.20 (s, 2 H). MS ESI/APCI Multi posi: 331[M+H]$^+$. |

Example 46-1

2-[4-(Difluoromethyl)-1H-pyrazol-5-yl]-5-[(3-methylsulfonylphenyl)methoxy]pyridine

[Formula 235]

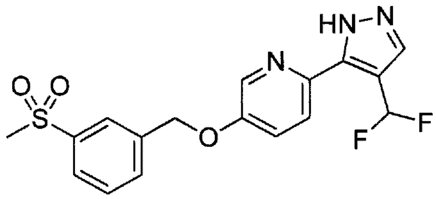

(1) Commercially available (3-methylsulfonylphenyl)methanol (100 mg) was used to perform the synthesis process according to the method described in Reference Example 12-1-(3) thereby giving a mixture containing (3-methylsulfonylphenyl)methyl methanesulfonate. The obtained mixture was used in the next step without purification.
(2) The compound obtained in the above described (1) and the compound (40 mg) obtained in Reference Example 56-1 were used to perform the synthesis process according to the method described in Example 27-1-(1) thereby giving 2-[4-(difluoromethyl)-2-(oxan-2-yl)pyrazol-3-yl]-5-[(3-methylsulfonylphenyl)methoxy]pyridine (70 mg) as a pale yellow oily substance.
(3) To a suspension of the compound (63 mg) obtained in the above described (2) in methanol (2.00 mL), hydrochloric acid (2 mol/L, 1.00 mL) was added and the mixture was stirred at room temperature for 2 hours. An aqueous solution of saturated sodium hydrogen carbonate was added to the reaction solution, and the mixture was extracted with a solution of chloroform/methanol (9:1). The organic layer was concentrated under reduced pressure, and the residue was purified by preparative HPLC to give the title compound (30 mg) as a colorless amorphous substance.
$^1$H NMR (600 MHz, DMSO-$d_6$) δ ppm 3.25 (s, 3 H) 5.37 (s, 2 H) 7.45 - 7.75 (m, 3 H) 7.83 - 7.87 (m, 1 H) 7.91 - 7.97 (m, 2 H) 8.03 - 8.23 (m, 2 H) 8.40 - 8.48 (m, 1 H).
MS ESI/APCI Multi posi: 380 [M+H]$^+$.
MS ESI/APCI Multi nega: 378 [M-H]$^-$.

The compounds of the following Examples 46-2 to 46-4 were synthesized using the compound obtained in Reference Examples 1-6 or 59, as well as a commercially available alcohol, according to the method described in Example 46-1. The structures, NMR data and MS data of these compounds are shown in Table 45-1.

[Table 45-1]

| Example No. | Structure | Analytical Data |
|---|---|---|
| 46-2 | 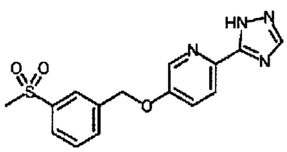 | $^1$H NMR (400 MHz, DMSO-$d_6$) δ ppm 3.25 (s, 3 H) 5.40 (s, 2 H) 7.62 - 7.76 (m, 2 H) 7.82 - 7.98 (m, 2 H) 8.00 - 8.25 (m, 3 H) 8.43 - 8.55 (m, 1 H). MS ESI/APCI Multi posi: 331[M+H]$^+$. MS ESI/APCI Multi nega: 329[M-H]$^-$. |

(continued)

| Example No. | Structure | Analytical Data |
|---|---|---|
| 46-3 | 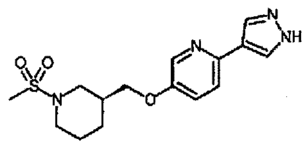 | $^1$H NMR (400 MHz, DMSO-$d_6$) δ ppm 1.14 - 1. 30 (m, 1 H) 1.49 - 1.62 (m, 1 H) 1.72 - 1.91 (m, 2 H) 1.96 - 2.17 (m, 1 H) 2.56 - 2.69 (m, 1 H) 2.70 - 2.78 (m, 1 H) 2.86 (s, 3 H) 3.41 - 3.54 (m, 1 H) 3.60 - 3.72 (m, 1 H) 3.90 - 4.05 (m, 2 H) 7.40 (dd, J=8.7, 2.9 Hz, 1 H) 7.62 (d, J=8. 7 Hz, 1 H) 7.86 - 8.34 (m, 3 H).<br>MS ESI/APCI Multi posi: 337[M+H]$^+$. |
| 46-4 | 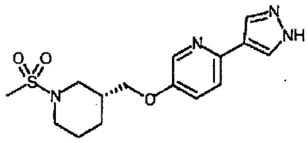 | $^1$H NMR (400 MHz, DMSO-$d_6$) δ ppm 1.14 - 1.30 (m, 1 H) 1.49 - 1.62 (m, 1 H) 1. 72 - 1.91 (m, 2 H) 1.96 - 2.17 (m, 1 H) 2.56 - 2.69 (m, 1 H) 2.70 - 2.78 (m, 1 H) 2.86 (s, 3 H) 3.41 - 3.54 (m, 1 H) 3.60 - 3.72 (m, 1 H) 3.90 - 4.05 (m, 2 H) 7.40 (dd, J=8.7, 2.9 Hz, 1 H) 7.62 (d, J=8.7 Hz, 1 H) 7.85 - 8.34 (m, 3 H).<br>MS ESI/APCI Multi posi: 337[M+H]$^+$. |

Example 47-1

N-Cyclopropyl-5-{5-[(3-methylsulfonylphenyl)methoxy]pyridin-2-yl}-1H-pyrazole-4-carboxamide

[Formula 236]

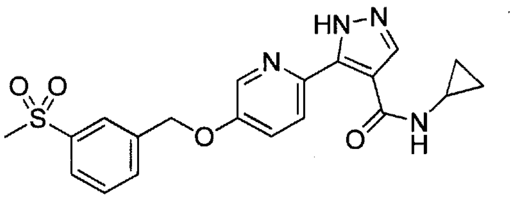

(1) The compound (70.2 mg) obtained in Reference Example 61-1 and commercially available cyclopropylamine (11.2 mg) were used to perform the synthesis process according to the method described in Reference Example 45-1-(3) thereby giving 1-tert-butyl-N-cyclopropyl-5-{5-[(3-methylsulfonylphenyl)methoxy]pyridin-2-yl}pyrazole-4-carboxamide (61.4 mg) as a colorless amorphous substance.

(2) The compound (61.4 mg) obtained in the above described (2) was dissolved in formic acid (1.00 mL), and the mixture was stirred at room temperature overnight. After distilling off the solvent under reduced pressure, the residue was recrystallized from to give the title compound (44.4 mg) as a colorless powder.
$^1$H NMR (400 MHz, DMSO-$d_6$) δ ppm 0.51 - 0.57 (m, 2 H) 0.69 - 0.78 (m, 2 H) 2.79 - 2.89 (m, 1 H) 3.25 (s, 3 H) 5.41 (s, 2 H) 7.67 - 7.76 (m, 2 H) 7.86 (d, J=7.8 Hz, 1 H) 7.94 (d, J=7.8 Hz, 1 H) 8.08 (s, 1 H) 8.14 (d, J=8.9 Hz, 1 H) 8.16 - 8.22 (m, 1 H) 8.46 (d, J=2.9 Hz, 1 H).
MS ESI/APCI Multi posi: 413 [M+H]$^+$.
MS ESI/APCI Multi nega: 411 [M-H]$^-$.

Example 48-1

5-{5-[(3-Methylsulfbnylphenyl)methoxy]pyridin-2-yl}-1H-pyrazole-4-carboxamide

[Formula 237]

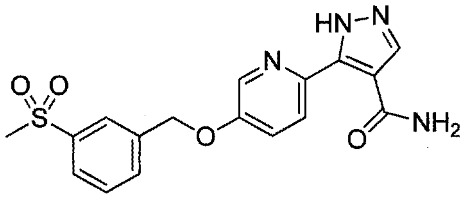

(1) N-Methylmorpholine (46.7 μL) and isobutyl chloroformate (55.6 μL) were sequentially added to a solution of the compound (152 mg) obtained in Reference Example 61-1 in chloroform (1.77 mL) under ice cooling, and the mixture was stirred at the same temperature for 1 hour. A solution of 7 mol/L ammonia in methanol (506 μL) was added to this mixture, and the mixture was additionally stirred for 1.5 hours. 1-Ethyl-3-(3-dimethylaminopropyl)carbodiimide hydrochloride (50.0 mg) was added, and the mixture was stirred under ice cooling for 30 minutes, and then at 50°C for 1 hour. The mixture was cooled on an ice bath, a solution of 7 mol/L ammonia in methanol (506 μL) was added, and the mixture was stirred for 30 minutes. Water was added to the mixture, and the mixture was extracted with chloroform four times. The obtained organic layer was collected, and sequentially washed with an aqueous solution of saturated ammonium chloride and brine. After separation by a phase separator, the solvent was distilled off under reduced pressure. The obtained residue was purified by NH silica gel column chromatography (n-hexane/ethyl acetate = 7:3, to ethyl acetate only) to give 1-tert-butyl-5-{5-[(3-methylsulfonylphenyl)methoxy]pyridin-2-yl}pyrazole-4-carboxamide (91.0 mg) as a colorless solid.
(2) The compound (45.2 mg) obtained in the above described (1) was used to perform the synthesis process according to the method described in Example 47-1-(2) thereby giving the title compound (10.7 mg) as a colorless powder.
$^1$H NMR (400 MHz, DMSO-$d_6$) δ ppm 3.25 (s, 3 H) 5.39 (s, 2 H) 7.63 - 7.77 (m, 2 H) 7.85 (d, J=7.9 Hz, 1 H) 7.93 (d, J=7.9 Hz, 1 H) 8.03 - 8.18 (m, 2 H) 8.20 - 8.27 (m, 1 H) 8.42 - 8.52 (m, 1 H).
MS ESI/APCI Multi posi: 373 $[M+H]^+$.

Example 48-2

5-{5-[(3-Methylsulfonylphenyl)methoxy]pyridin-2-yl}-1H-pyrazole-4-carbonitrile

[Formula 238]

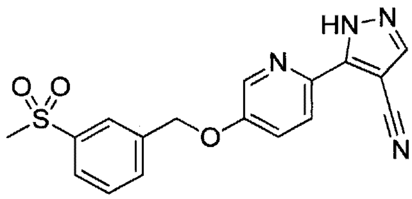

(1) Pyridine (38.8 μL) and p-toluenesulfonic acid chloride (36.7 mg) were added to a suspension of the compound (41.2 mg) obtained in Example 48-1-(1) in chloroform (961 μL), and the mixture was stirred at room temperature for 2 hours and at 50°C for 3 hours. Pyridine (38.8 μL) and p-toluenesulfonic acid chloride (36.7 mg) were further added, and the mixture was stirred at 50°C for 2.5 hours. After cooling to room temperature, an aqueous solution of saturated sodium hydrogen carbonate was added, and the mixture was vigorously stirred for 30 minutes. After left to stand the mixture to separate the organic layer, the aqueous layer was extracted with chloroform twice. The obtained organic layer was collected, and washed with a 1:1 mixed solution consisting of an aqueous solution of saturated sodium hydrogen carbonate and brine. After separation by a phase separator, the solvent was distilled off under reduced pressure. The obtained residue was purified by silica gel column chromatography (n-hexane only, to n-hexane/ethyl acetate = 1:4) to give 1-tert-butyl-5-{5-[(3-methylsulfonylphenyl)methoxy]pyridin-2-yl}pyrazole-4-carbonitrile (38.5 mg) as a colorless solid.

(2) The compound (38.5 mg) obtained in the above described (1) was used to perform the synthesis process according to the method described in Example 47-1-(2) thereby giving the title compound (15.2 mg) as a colorless solid.
$^1$H NMR (400 MHz, DMSO-$d_6$) δ ppm 3.25 (s, 3 H) 5.40 (s, 2 H) 7.66 (dd, J=8.7, 2.9 Hz, 1 H) 7.69 - 7.76 (m, 1 H) 7.86 (d, J=7.8 Hz, 1 H) 7.90 - 7.98 (m, 2 H) 8.07 (s, 1 H) 8.44 - 8.54 (m, 2 H).
MS ESI/APCI Multi posi: 355 [M+H]+.
MS ESI/APCI Multi nega: 353 $[M-H]^-$.

Example 49-1

(5-{5-[(3-Methylsulfonylphenyl)methoxy]pyridin-2-yl}-1H-pyrazol-4-yl)methanol

[Formula 239]

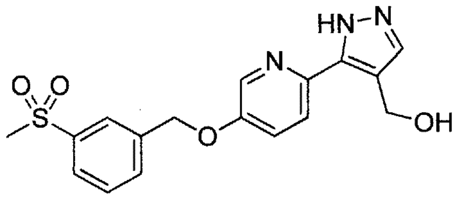

Example 49-2

2-[4-(Methoxymethyl)-1H-pyrazol-5-yl]-5-[(3-methylsulfonylphenyl)methoxy]pyridine

[Formula 240]

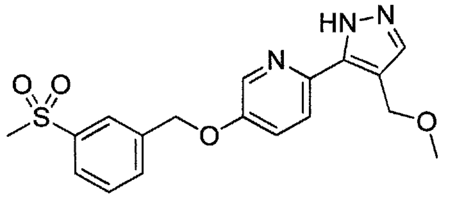

(1) The compound (100 mg) obtained in Reference Example 61-1 was used to perform the synthesis process according to the method described in Reference Example 32-1 thereby giving (1-tert-butyl-5-15-[(3-methylsulfonylphenyl)methoxy]pyridin-2-yl}pyrazol-4-yl)methanol (57.0 mg) as a colorless amorphous substance.
(2) The compound (57.0 mg) obtained in the above described (1) was dissolved in formic acid (1.00 mL), and the mixture was stirred at room temperature overnight. The solvent was distilled off under reduced pressure, and the residue was dissolved in methanol (1.00 mL). Potassium carbonate (70.9 mg) was added to this solution, and the mixture was stirred at room temperature for 4 hours. After filtering off insolubles, the filtrate was purified by preparative HPLC-MS to give the compound of Example 49-1 (1.80 mg), a component of the higher polarity, as a colorless amorphous substance.
$^1$H NMR (400 MHz, ACETONE-$d_6$) δ ppm 3.15 (s, 3 H) 4.52 - 4.63 (m, 2 H) 5.44 (s, 2 H) 5.49 - 5.70 (m, 1 H) 7.60 - 7.66 (m, 1 H) 7.68 (s, 1 H) 7.69 - 7.77 (m, 1 H) 7.91 (d, J=7.5 Hz, 1 H) 7.96 (d, J=7.8 Hz, 1 H) 8.04 - 8.11 (m, 1 H) 8.13 (s, 1 H) 8.42 - 8.49 (m, 1 H).
MS ESI/APCI Multi posi: 360 $[M+H]^+$.
MS ESI/APCI Multi nega: 358 $[M-H]^-$.

In addition, the compound of Example 49-2 (4.73 mg), a component of the lower polarity, was obtained as a colorless amorphous substance.
$^1$H NMR (400 MHz, METHANOL-$d_4$) δ ppm 3.14 (s, 3 H) 3.37 (s, 3 H) 4.51 - 4.76 (m, 2 H) 5.33 (s, 2 H) 7.49 - 7.89 (m,

5 H) 7.94 (d, J=7.8 Hz, 1 H) 8.10 (s, 1 H) 8.38 - 8.48 (m, 1 H). MS ESI/APCI Multi posi: 374 [M+H]+.

Example 50-1

Imino-methyl-oxo-(3-([6-(1H-pyrazol-5-yl)pyridin-3-yl]oxymethyl}phenyl)-λ^{6}-sulfane (racemate)

[Formula 241]

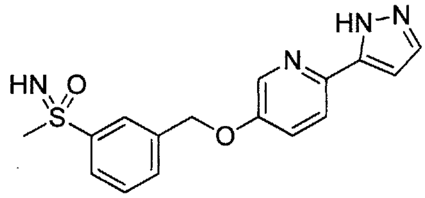

(1) The compound (91.5 mg) obtained in Reference Example 67 and the compound (70.7 mg) obtained in Reference Example 1-1 were used to perform the synthesis process according to the methods described in Reference Examples 46-1-(1) and 46-1-(2) thereby giving ethyl N-({methyl-[3-({6-[2-(oxan-2-yl)pyrazol-3-yl]pyridin-3-yl}oxymethyl)phenyl]-oxo-λ^{6}-sulfanilidene} carbamate (114 mg) as a colorless oily substance.

(2) Sodium ethoxide (2.8 mol/L ethanol solution, 286 μL) was added to a solution of the compound (114 mg) obtained in the above described (1) in ethanol (1.67 mL), and the mixture was stirred at 100°C for 30 minutes under microwave irradiation. After cooling to room temperature, the mixture was diluted with water and extracted with chloroform three times. The obtained organic layer was collected and washed with brine. After separation by a phase separator, the solvent was distilled off under reduced pressure. The obtained residue was dissolved in methanol (2.00 mL), concentrated hydrochloric acid (167 μL) was added, and the mixture was stirred at room temperature overnight. Sodium hydroxide (1 mol/L) was used to neutralize the mixture, and the solvent was distilled off under reduced pressure. An aqueous solution of saturated sodium hydrogen carbonate was added to the residue, and the mixture was extracted with chloroform three times. The obtained organic layer was collected and washed with brine. After separation by a phase separator, the solvent was distilled off under reduced pressure. The obtained residue was purified by NH column chromatography (chloroform/ethyl acetate = 19:1 to 3:17). The crude product obtained was suspended in ethyl acetate (2 mL) under heating. After cooling the suspension to room temperature, n-hexane (2 mL) was added, and the precipitate was filtered and dried to give the title compound (52.2 mg) as a colorless solid.

$^1$H NMR (400 MHz, DMSO-$d_6$) δ ppm 3.07 (s, 3 H) 4.24 (s, 1 H) 5.32 (s, 2 H) 6.73 (d, J=1.8 Hz, 1 H) 7.48 - 7.97 (m, 6 H) 8.05 (s, 1 H) 8.35 - 8.40 (m, 1 H).

MS ESI/APCI Multi posi: 329 [M+H]+.

Example 50-2

Imino-methyl-oxo-(3-{[6-(1H-pyrazol-5-yl)pyridin-3-yl]oxymethyl}phenyl)-λ^{6}-sulfane (optically active substance, high polarity)

[Formula 242]

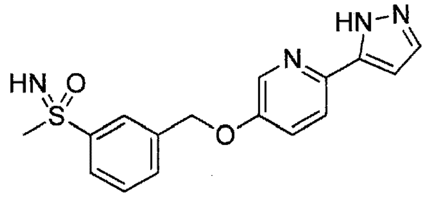

Example 50-3

Imino-methyl-oxo-(3-1[6-(1H-pyrazol-5-yl)pyridin-3-yl]oxymethyllphenyl)-λ^{6}-sulfane (optically active substance, low polarity)

[Formula 243]

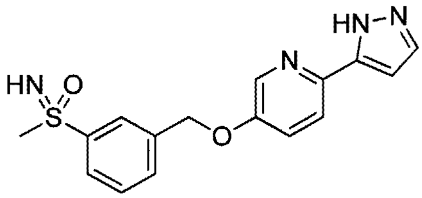

(1) The racemic mixture (41.0 mg) obtained in Example 50-1 was optically resolved by HPLC equipped with a chiral column, and the obtained compound was solidified from ethanol/hexane to give the compound of Example 50-2 (20.5 mg) of the higher polarity as a colorless solid.
$^{1}H$ NMR (400 MHz, DMSO-$d_6$) δ ppm 3.08 (d, J=0.8 Hz, 3 H) 4.26 (s, 1 H) 5.33 (s, 2 H) 6.74 (d, J=2.1 Hz, 1 H) 7.51 - 7.97 (m, 6 H) 8.06 (s, 1 H) 8.38 (d, J=2.7 Hz, 1 H).
MS ESI/APCI Multi posi: 374 $[M+H]^+$.
Chiral HPLC analysis retention time: 7.41 min

In addition, the compound of Example 50-3 (18.6 mg) of the lower polarity was obtained as a colorless solid. Chiral HPLC analysis retention time: 4.69 min

The inhibitory action of the compound of the present invention against 20-HETE producing enzymes was measured by the method described in the following Test Example 1.

Test Example 1

(1) Inhibition test for each compound of the present invention against 20-HETE producing enzymes (CYP4F2 and CYP4A11)

In the CYP4F2 inhibition test, the reaction solution containing each compound [final concentration of 50 mM, $KPO_4$ (pH 7.4), 2.5 μM, luciferine derivative and 1 mM NADPH] was added to an Escherichia coli membrane fraction (100 μg/mL, protein) in which human CYP4F2 had been expressed. In the CYP4A11 inhibition test, the reaction solution containing each compound [final concentration of 100 mM, Tris-HCl (pH 7.5), 60 μM, luciferine derivative, 1.3 mM $NADP^+$, 3.3 mM Glucose 6-Phosphate, 3.3 mM $MgCl_2$ and 0.4 U/mL Glucose 6-Phosphate dehydrogenase] was added to an Escherichia coli membrane fraction (100 μg/mL, protein) in which human CYP4A11 had been expressed. Following this, the membrane fraction was left to stand at room temperature for 60 minutes to perform an enzymatic reaction. After the reaction, a luciferine detection reagent was added, and the luminescence value was measured using a plate reader. By using that value, the percent inhibition of 20-HETE producing enzyme (%) was calculated according to the equation described below, and the 50% inhibitory concentration ($IC_{50}$ value) for each compound was calculated.

$$\text{Percent inhibition of 20-HETE producing enzyme (\%)} = [1-(A-B)/(C-B)]*100$$

A: Luminescence value with addition of compound
B: Luminescence value without addition of compound and enzyme
C: Luminescence value without addition of compound

(2) Results

The inhibitory activity of each compound of the present invention against CYP4F2 and CYP4A11 is shown in

the following Tables 46-1 to 46-4.

[Table 46-1]

| Example No. | $IC_{50}$ value [nM] | |
|---|---|---|
| | Human CYP4F2 | Human CYP4A11 |
| 1-1 | 40 | 140 |
| 1-2 | 180 | 530 |
| 1-3 | 73 | 310 |
| 1-4 | 120 | 510 |
| 1-5 | 110 | 530 |
| 1-6 | 640 | 70 |
| 1-7 | 150 | 87 |
| 1-8 | 190 | 130 |
| 1-9 | 130 | 210 |
| 1-10 | 22 | 130 |
| 1-11 | 180 | 220 |
| 1-12 | 21 | 330 |
| 1-13 | 50 | 310 |
| 1-14 | 39 | 330 |
| 1-15 | 8.0 | 350 |
| 1-16-1 | 240 | 53 |
| 1-16-2 | 260 | 57 |
| 1-17 | 6.3 | 51 |
| 1-18 | 3.6 | 82 |
| 1-19 | 3.7 | 190 |
| 1-20 | 8.6 | 77 |
| 1-21 | 18 | 32 |
| 1-22 | 37 | 44 |
| 1-23 | 26 | 26 |
| 1-24 | 40 | 35 |
| 1-25 | 640 | 1600 |
| 1-26 | 93 | 2300 |
| 1-29 | 85 | 190 |
| 1-30 | 11 | 110 |
| 1-31 | 70 | 140 |
| 1-32 | 27 | 67 |
| 1-33 | 54 | 83 |
| 1-34 | 79 | 310 |
| 1-35 | 320 | 890 |
| 1-36 | 680 | 640 |
| 1-37 | 120 | 160 |

(continued)

| Example No. | $IC_{50}$ value [nM] | |
|---|---|---|
| | Human CYP4F2 | Human CYP4A11 |
| 1-38 | 6.2 | 180 |
| 1-39 | 11 | 270 |
| 1-40 | 23 | 62 |
| 2-1 | 830 | 1100 |
| 2-2 | 78 | 930 |
| 2-3 | 76 | 360 |
| 2-4 | 94 | 1100 |
| 2-5 | 810 | 1600 |
| 2-6 | 290 | 2900 |
| 2-7 | 440 | 5300 |
| 2-8 | 290 | 5700 |
| 2-9 | 440 | 3800 |
| 2-10 | 400 | 1100 |
| 2-11 | 9.5 | 350 |
| 2-12 | 23 | 540 |
| 2-13 | 73 | 100 |
| 2-14 | 29 | 77 |
| 2-15 | 12000 | 5800 |
| 2-16 | 3600 | 7600 |
| 2-17 | 480 | 270 |
| 2-18 | 2900 | 1800 |
| 3-2 | 2000 | 2600 |
| 3-3 | 790 | 1700 |
| 3-4 | 4200 | 500 |
| 3-5 | 440 | 270 |
| 3-9 | 1300 | 2000 |
| 3-10 | 430 | 600 |
| 3-11 | 52 | 190 |
| 3-12 | 2600 | 2800 |
| 3-13 | 280 | 1500 |
| 3-14 | 110 | 280 |
| 3-15 | 7100 | 1200 |
| 3-17 | 17 | 77 |
| 3-18 | 18 | 50 |
| 3-19 | 2400 | 1000 |
| 3-20 | 650 | 3300 |
| 3-21 | 720 | 800 |

(continued)

| Example No. | IC50 value [nM] | |
|---|---|---|
| | Human CYP4F2 | Human CYP4A11 |
| 3-23 | 4700 | 3200 |
| 3-24 | 490 | 2000 |
| 3-25 | 110 | 560 |
| 3-26 | 9200 | 1200 |
| 3-27 | 700 | 310 |
| 3-28 | 3200 | 2400 |
| 3-29 | 50 | 79 |

[Table 46-2]

| Example No. | $IC_{50}$ value [nM] | |
|---|---|---|
| | Human CYP4F2 | Human CYP4A11 |
| 3-30 | 250 | 110 |
| 3-31 | 4.9 | 43 |
| 3-32 | 15 | 460 |
| 3-33 | 220 | 2500 |
| 3-34 | 45000 | 770 |
| 3-35 | 790 | 1300 |
| 3-36 | 90 | 710 |
| 3-37 | 8.3 | 290 |
| 3-38 | 380 | 1800 |
| 3-39 | 3500 | 3400 |
| 3-40 | 370 | 930 |
| 3-41 | 180 | 230 |
| 3-42 | 900 | 1800 |
| 3-43 | 570 | 720 |
| 3-44 | 18 | 100 |
| 3-45 | 24 | 70 |
| 3-46 | 23 | 290 |
| 3-47 | 530 | 2100 |
| 4-1 | 120 | 550 |
| 4-2 | 50 | 380 |
| 4-3 | 210 | 270 |
| 4-4 | 4500 | 310 |
| 4-5 | 93 | 370 |
| 4-6 | 77 | 340 |
| 4-7 | 230 | 380 |

(continued)

| Example No. | IC$_{50}$ value [nM] | |
|---|---|---|
| | Human CYP4F2 | Human CYP4A11 |
| 4-8 | 43 | 91 |
| 4-9 | 16 | 97 |
| 4-10 | 20 | 56 |
| 5-1 | 170 | 1200 |
| 5-2 | 450 | 510 |
| 5-3 | 190 | 1200 |
| 6-1 | 19 | 640 |
| 6-2 | 63 | 1300 |
| 6-3 | 570 | 450 |
| 6-4 | 110 | 1100 |
| 6-5 | 720 | 2000 |
| 7-1 | 22 | 790 |
| 7-2 | 29 | 1100 |
| 8-1 | 110 | 1500 |
| 8-2 | 90 | 930 |
| 9-1 | 74 | 310 |
| 9-2 | 63 | 370 |
| 9-3 | 150 | 360 |
| 10-1 | 640 | 860 |
| 10-2 | 710 | 38 |
| 10-3 | 100 | 32 |
| 10-4 | 72 | 33 |
| 10-5 | 73 | 24 |
| 10-6 | 2.5 | 17 |
| 10-7 | 730 | 41 |
| 10-8 | 130 | 59 |
| 10-9 | 42 | 47 |
| 10-10 | 36 | 29 |
| 10-11 | 3.7 | 22 |
| 10-12 | 210 | 120 |
| 10-13 | 130 | 170 |
| 10-14 | 320 | 160 |
| 10-15 | 100 | 210 |
| 10-16 | 45 | 21 |
| 10-17 | 38 | 19 |
| 10-18 | 110 | 48 |
| 10-19 | 58 | 120 |

(continued)

| Example No. | $IC_{50}$ value [nM] | |
|---|---|---|
| | Human CYP4F2 | Human CYP4A11 |
| 10-20 | 98 | 41 |
| 10-21 | 51 | 93 |
| 10-22 | 960 | 560 |
| 10-23 | 45 | 220 |
| 10-24 | 350 | 390 |
| 10-25 | 340 | 390 |
| 11-1 | 4.3 | 27 |
| 11-2 | 12 | 24 |
| 11-3 | 23 | 30 |
| 12-1 | 45 | 58 |
| 13-1 | 24 | 350 |
| 13-2 | 10 | 640 |
| 13-3 | 550 | 1700 |
| 13-4 | 250 | 1300 |
| 13-5 | 5000 | 3300 |
| 13-6 | 1200 | 530 |
| 13-7 | 2300 | 530 |
| 13-8 | 49 | 6500 |

[Table 46-3]

| Example No. | $IC_{50}$ value [nM] | |
|---|---|---|
| | Human CYP4F2 | Human CYP4A11 |
| 13-9 | 220 | 2000 |
| 13-10 | 11000 | 9300 |
| 13-11 | 12000 | 36000 |
| 13-12 | > 50000 | 19000 |
| 13-13 | 100 | 150 |
| 13-15 | 270 | 140 |
| 13-16 | 890 | 490 |
| 13-17 | 860 | 1300 |
| 13-18 | 93 | 190 |
| 14-1 | 170 | 410 |
| 14-2 | 16 | 2800 |
| 14-3 | 990 | 260 |
| 14-4 | 360 | 4200 |
| 14-5 | 220 | 610 |

(continued)

| Example No. | IC$_{50}$ value [nM] | |
|---|---|---|
| | Human CYP4F2 | Human CYP4A11 |
| 14-6 | 210 | 6100 |
| 14-7 | 57 | 26 |
| 14-8 | 60 | 53 |
| 14-9 | 99 | 2900 |
| 14-10 | 11 | 260 |
| 14-11 | 17 | 30 |
| 14-12 | 31 | 270 |
| 14-13 | 98 | 2500 |
| 14-14 | 1400 | 6100 |
| 14-15 | 1500 | 78 |
| 15-1 | 260 | 690 |
| 16-1-1 | 17 | 5400 |
| 16-1-2 | 64 | 8100 |
| 16-2 | 790 | 10000 |
| 16-3 | 15 | 4600 |
| 16-5 | 310 | 79 |
| 17-1-1 | 20 | 2000 |
| 17-1-2 | 19 | 2700 |
| 18-1 | 140 | 18 |
| 18-2 | 140 | 58 |
| 18-3 | 27 | 24 |
| 18-4 | 85 | 76 |
| 18-5 | 47 | 140 |
| 18-6 | 93 | 27 |
| 18-7 | 95 | 230 |
| 18-8 | 260 | 63 |
| 19-1 | 46 | 620 |
| 20-1 | 240 | 1600 |
| 20-2 | 240 | 1600 |
| 20-3 | 2800 | 1400 |
| 20-4 | 180 | 2400 |
| 20-5 | 150 | 6400 |
| 20-6 | 310 | 22000 |
| 20-7 | 69 | 35000 |
| 21-1 | 150 | 1200 |
| 22-1 | 19 | 390 |
| 23-1 | 13 | 71 |

(continued)

| Example No. | $IC_{50}$ value [nM] | |
|---|---|---|
| | Human CYP4F2 | Human CYP4A11 |
| 23-2 | 85 | 26 |
| 23-3 | 15 | 42 |
| 23-4 | 39 | 15 |
| 23-5 | 61 | 52 |
| 23-6 | 170 | 150 |
| 24-1 | 28 | 87 |
| 24-2 | 450 | 850 |
| 24-3 | 35 | 120 |
| 24-4 | 590 | 8600 |
| 24-5 | 32 | 19 |
| 24-6 | 25 | 64 |
| 24-7 | 420 | 78 |
| 24-8 | 2600 | 440 |
| 25-3 | 140 | 1700 |
| 25-4 | 99 | 24 |
| 25-5 | 110 | 45 |
| 25-6 | 62 | 95 |
| 25-7 | 57 | 910 |
| 25-8 | 230 | 2500 |
| 25-9 | 12 | 1300 |
| 25-10 | 20 | 1100 |
| 25-11 | 3.0 | 1600 |
| 25-12 | 1600 | 75 |
| 25-13 | 190 | 36 |
| 25-14 | 16 | 410 |
| 25-15 | 11 | 2700 |
| 25-16 | 3.2 | 2200 |
| 25-18 | 96 | 6000 |
| 25-19 | 83 | 100 |

[Table 46-4]

| Example No. | $IC_{50}$ value [nM] | |
|---|---|---|
| | Human CYP4F2 | Human CYP4A11 |
| 25-20 | 14 | 1200 |
| 25-21 | 40 | 2400 |
| 25-22 | 9.2 | 1500 |

(continued)

| Example No. | IC$_{50}$ value [nM] | |
|---|---|---|
| | Human CYP4F2 | Human CYP4A11 |
| 25-23 | 52 | 160 |
| 25-24 | 350 | 2500 |
| 25-28 | 5.1 | > 50000 |
| 25-29 | 88 | 67 |
| 39-1 | 240 | 220 |
| 39-2 | 320 | 95 |
| 40-1 | 12000 | 12000 |
| 41-1 | 470 | 120 |
| 41-2 | 4300 | 1100 |
| 41-3 | 1600 | 520 |
| 42-1 | 44 | 110 |
| 42-2 | 140 | 65 |
| 43-1 | 3100 | 4800 |
| 43-2 | 250 | 1300 |
| 43-3 | 3800 | 600 |
| 43-4 | 5400 | 390 |
| 43-5 | 3400 | 290 |
| 43-6 | 3500 | 420 |
| 44-1 | 9000 | 2400 |
| 45-1 | 370 | 1300 |
| 45-2 | 3100 | 640 |
| 45-3 | 26 | 55 |
| 45-4 | 220 | 31 |
| 45-5 | 1600 | 11000 |
| 45-6 | 16000 | 5700 |
| 46-1 | 7500 | 3900 |
| 46-2 | 1500 | 340 |
| 46-3 | 2000 | 33 |
| 46-4 | 1400 | 39 |
| 47-1 | 15000 | > 50000 |
| 48-1 | 33000 | 20000 |
| 48-2 | 1700 | 770 |
| 49-1 | 1800 | 550 |
| 49-2 | 28000 | 13000 |
| 50-1 | 580 | 240 |
| 50-2 | 1200 | 250 |
| 50-3 | 380 | 340 |

(3) Inhibition test for compound A and compound B disclosed in WO03/022821 against 20-HETE producing enzymes (CYP4F2 and CYP4A11)

For compound A (Example 402) and compound B (Example 754) described below, whose inhibitory activity against 20-HETE producing enzymes using human kidney microsomes is disclosed in WO03/022821, the 50% inhibitory concentration ($IC_{50}$ value) against CYP4F2 and CYP4A11 was calculated according to the method described in the present Test Example 1.

Note that compound A and compound B disclosed in WO03/022821 are as follows:

[Formula 244]

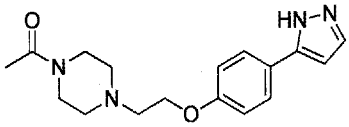

Compound A

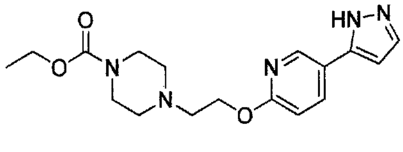

Compound B

(4) Results

The inhibitory activity of compound A and compound B against CYP4F2 and CYP4A11 is shown in the following Table 46-5.

[Table 46-5]

| Compound No. | $IC_{50}$ value [nM] | |
|---|---|---|
| | Human CYP4F2 | Human CYP4A11 |
| Compound A | 1000 | 11000 |
| Compound B | 36000 | > 50000 |

Furthermore, the inhibitory action of the compound of the present invention against 20-HETE producing enzymes was also measured by the method described in the following Test Example 2.

Test Example 2

(1) Inhibition test for each compound of the present invention against 20-HETE producing enzymes using human kidney microsomes

The reaction solution containing each compound [final concentration of 100 mmol/L, $KPO_4$ (pH 7.4), 20μM, Arachidonic acid, 4 mM NADPH] was added to the human kidney microsome (250 μg/mL, protein). Following this, the microsome was left to stand at 37°C for 45 minutes to perform 20-HETE producing reaction. After adding formic acid to stop the reaction, 9 times amount of acetonitrile was added, and deproteinization was carried out by centrifugation (1000 rpm, 4°C, 10 minutes). After that, the peak area value of 20-HETE was measured using a liquid chromatograph-tandem mass spectrometer (LC-MS/MS), and by using that value, the percent inhibition of 20-HETE producing enzyme (%) was calculated according to the equation described below, and the 50% inhibitory concentration ($IC_{50}$ value) for each compound was calculated.

$$\text{Percent inhibition of 20-HETE producing enzyme (\%)} = [1-(A-B)/(C-B)]*100$$

A: Peak area value of 20-HETE/peak area value of internal standard substance with addition of compound
B: Peak area value of 20-HETE/peak area value of internal standard substance without addition of compound and NADPH
C: Peak area value of 20-HETE/peak area value of internal standard substance without addition of compound

(2) Results

The inhibitory activity of each compound of the present invention against 20-HETE producing enzymes is shown in the following Table 47-1.

[Table 47-1]

| Example No. | $IC_{50}$ value [nM] |
|---|---|
| 1-1 | 16.4 |
| 24-1 | 26.0 |
| 1-31 | 28.1 |
| 1-40 | 13.6 |
| 25-4 | 12.7 |
| 14-7 | 11.1 |

(3) Inhibition test for compound A and compound B disclosed in WO03/022821 against 20-HETE producing enzymes using human kidney microsomes

For the above described compound A and compound B disclosed in WO03/022821, the 50% inhibitory concentration ($IC_{50}$ value) against 20-HETE producing enzymes was calculated according to the method described in the present Test Example 2.

(4) Results

The inhibitory activity of compound A and compound B against 20-HETE producing enzymes is shown in the following Table 47-2.

[Table 47-2]

| Compound No. | $IC_{50}$ value [nM] |
|---|---|
| Compound A | 408 |
| Compound B | 15600 |

(5) Comparison of inhibitory activities against 20-HETE producing enzymes between the above described compound A and compound B disclosed in WO03/022821, and the compound of the present invention

Compared to the above described compound A and compound B, six compounds from Examples of the present inventive compounds (Example 1-1, Example 24-1, Example 1-31, Example 1-40, Example 25-4, and Example 14-7) have stronger inhibitory activities against 20-HETE producing enzymes.

Now, explanation will be given regarding the inhibition test against 20-HETE producing enzymes using human kidney microsomes, disclosed in WO03/022821, and the aforementioned Test Example 2.

In the test disclosed in WO03/022821, radiolabelled arachidonic acid is used as a substrate, and the amount of 20-HETE produced is measured using a radio-HPLC. In this case, the concentration of arachidonic acid, the substrate, is 0.01 $\mu$M.

On the other hand, in Test Example 2, nonradioactive arachidonic acid was used as a substrate for 20-HETE producing reaction, and the amount of 20-HETE produced was measured using LC-MS/MS. In this case, the concentration of arachidonic acid, the substrate, is 20 $\mu$M.

In recent years, it is recommended that the substrate concentration for calculating the $IC_{50}$ value be set at the Km value (Assay Guidance Manual, Sittampalam et. al. (URL: http://www.ncbi.nlm.nih.gov/books/NBK53196/)). According to this, in the aforementioned Test Example 2, human kidney microsomes were used to calculate the Km value, and the calculated Km value of 20 $\mu$M was set as the concentration of the substrate, arachidonic acid.

From the above, in the light of the current science level, the conditions used for the test in the aforementioned Test Example 2 are believed to be more appropriate, compared to the conditions of the test disclosed in WO03/022821, and thus the $IC_{50}$ value calculated under the conditions of Test Example 2 is believed to be more reasonable than the

value disclosed in WO03/022821.

INDUSTRIAL APPLICABILITY

The compound of the present invention has an excellent effect of inhibiting 20-HETE producing enzymes, and thus the present invention makes it possible to provide a medical product effective in preventing or treating diseases from polycystic kidney or the like, and is expected to relieve a burden on the patient and contribute to the development of the pharmaceutical industry.

## Claims

**1.** A compound represented by formula [I'] shown below:

[Formula 1]

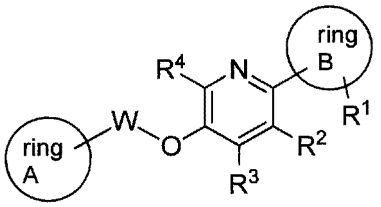

[I']

wherein
the structure represented by formula [III] shown below:

[Formula 2]

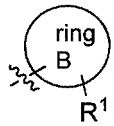

[III]

represents any of the structures represented by formula group [IV] shown below:

[Formula 3]

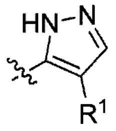 , 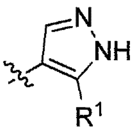 ,

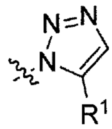 , 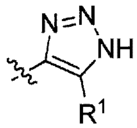 ,

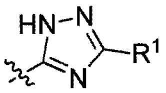 , 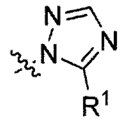 , 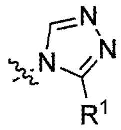 ,

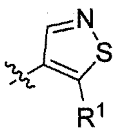 , 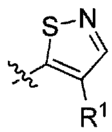 [IV]

wherein

$R^1$ represents a hydrogen atom, hydroxy, carbamoyl, cyano, a fluorine atom, a chlorine atom, a bromine atom, methyl, hydroxymethyl, methoxymethyl, difluoromethyl, trifluoromethyl, methoxy, or cyclopropylaminocarbonyl;
$R^2$, $R^3$, and $R^4$ each independently represent a hydrogen atom, a fluorine atom, or methyl;
W represents a single bond, $C_{1-3}$alkanediyl, or the formula $-O-CH_2CH_2-$;
ring A represents

(a) $C_{4-6}$cycloalkyl, wherein the $C_{4-6}$cycloalkyl is substituted with one substituent selected from substituent group A11,
(b) 4- to 6-membered saturated nitrogen-containing heterocyclyl, wherein the 4- to 6-membered saturated nitrogen-containing heterocyclyl is substituted with one substituent selected from substituent group A21 and may be further substituted with one substituent selected from substituent group A22,
(c) phenyl, wherein the phenyl is substituted with one substituent selected from substituent group A31 and may be further substituted with one substituent selected from substituent group A32,
(d) pyridyl, wherein the pyridyl is substituted with one substituent selected from substituent group A41,
(e) naphthyl,
(f) 2,3-dihydrobenzofuran, wherein the 2,3-dihydrobenzofuran may be substituted with one to three substituents selected from substituent group A51,
(g) 2H-chromenyl, wherein the 2H-chromenyl may be substituted with one oxo,
(h) quinolyl, wherein the quinolyl may be substituted with one $C_{1-6}$alkoxy,
(j) quinoxalyl,
(k) a group represented by formula [II-1] shown below, wherein the group represented by formula [II-1] is substituted with one $C_{1-6}$alkyl, wherein the $C_{1-6}$alkyl may be substituted with one substituent selected from substituent group B61,
(m) a group represented by formula [II-2] shown below, wherein the group represented by formula [II-2] is substituted with one $C_{1-6}$alkylcarbonyl,
(n) a group represented by formula [II-3] shown below, wherein the group represented by formula [II-3] is substituted with one $C_{1-6}$alkylcarbonyl,
(p) a group represented by formula [II-4] shown below, wherein the group represented by formula [II-4] is substituted with one $C_{1-6}$alkylcarbonyl,
(r) 4- to 6-membered saturated oxygen-containing heterocyclyl, or
(s) 4- to 6-membered saturated sulfur-containing heterocyclyl, wherein the 4- to 6-membered saturated

sulfur-containing heterocyclyl may be substituted with one or two oxo;

[Formula 4]

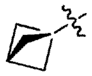 [II−1], 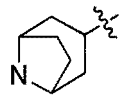 [II−2],

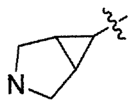 [II−3], 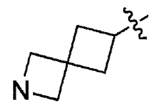 [II−4]

wherein substituent group A11 represents the group consisting of

(i) $C_{1-6}$alkylcarbonylamino and
(ii) $C_{1-6}$alkylearbonyl($C_{1-6}$alkyl)amino;

substituent group A21 represents the group consisting of

(i) $C_{1-6}$alkylcarbonyl, wherein the $C_{1-6}$alkylcarbonyl may be substituted with one to three substituents selected from substituent group B21,
(ii) $C_{3-8}$cycloalkylcarbonyl, wherein the $C_{3-8}$cycloalkylcarbonyl may be substituted with one or two substituents selected from substituent group B22,
(iii) arylcarbonyl, wherein the arylcarbonyl may be substituted with one substituent selected from substituent group B23,
(iv) saturated heterocyclylcarbonyl, wherein the saturated heterocyclylcarbonyl may be substituted with one or two substituents selected from substituent group B24,
(v) heteroarylcarbonyl, wherein the heteroarylcarbonyl may be substituted with one substituent selected from the group consisting of $C_{1-6}$alkyl, wherein the $C_{1-6}$alkyl may be substituted with one hydroxy,
(vi) $C_{1-6}$alkoxycarbonyl,
(vii) mono$C_{1-6}$alkylaminocarbonyl,
(viii) di$C_{1-6}$alkylaminocarbonyl,
(ix) $C_{3-8}$cycloalkylaminocarbonyl,
(x) $C_{3-8}$cycloalkyl($C_{1-6}$alkyl)aminocarbonyl,
(xi) $C_{1-6}$alkylsulfonyl, wherein the $C_{1-6}$alkylsulfonyl may be substituted with one $C_{1-6}$alkoxycarbonylamino,
(xii) $C_{3-8}$cycloalkylsulfonyl,
(xiii) saturated heterocyclylsulfonyl, wherein the saturated heterocyclylsulfonyl may be substituted with one substituent selected from substituent group B25, and
(xiv) di$C_{1-6}$alkylaminosulfonyl;

substituent group A22 represents the group consisting of

(i) a halogen atom and
(ii) $C_{1-6}$alkyl;

substituent group B21 represents the group consisting of

(i) hydroxy,
(ii) carbamoyl,
(iii) ureide,
(iv) a halogen atom,
(v) $C_{3-8}$cycloalkyl, wherein the $C_{3-8}$cycloalkyl may be substituted with one hydroxy,
(vi) saturated heterocyclyl, wherein the saturated heterocyclyl may be substituted with one or two substituents selected from the group consisting of hydroxy and oxo,
(vii) heteroaryl, wherein the heteroaryl may be substituted with one oxo,
(viii) $C_{1-6}$alkoxy,
(ix) aryloxy,

(x) saturated heterocyclylcarbonyl,
(xi) $C_{1-6}$alkylsulfonyl,
(xii) halo-$C_{1-6}$alkylsulfonyl,
(xiii) arylsulfonyl,
(xiv) $C_{1-6}$alkylcarbonylamino, wherein $C_{1-6}$alkyl in the $C_{1-6}$alkylcarbonylamino may be substituted with one substituent selected from the group consisting of hydroxy and saturated heterocyclyl,
(xv) $C_{1-6}$alkylcarbonyl($C_{1-6}$alkyl)amino,
(xvi) $C_{3-8}$cycloalkylcarbonylamino, wherein $C_{3-8}$cycloalkyl in the $C_{3-8}$cycloalkylcarbonylamino may be substituted with one or two halogen atoms,
(xvii) arylcarbonylamino,
(xviii) saturated heterocyclylcarbonylamino,
(xix) mono$C_{1-6}$alkylaminocarbonyl,
(xx) di$C_{1-6}$alkylaminocarbonyl,
(xxi) $C_{1-6}$alkoxycarbonylamino, wherein $C_{1-6}$alkoxy in the $C_{1-6}$alkoxycarbonylamino may be substituted with one substituent selected from the group consisting of $C_{1-6}$alkoxy and aryl,
(xxii) $C_{1-6}$alkoxycarbonyl($C_{1-6}$alkyl)amino,
(xxiii) $C_{3-8}$cycloalkoxycarbonylamino, wherein $C_{3-8}$cycloalkoxy in the $C_{3-8}$cycloalkoxycarbonylamino may be substituted with one $C_{1-6}$alkyl,
(xxiv) mono$C_{1-6}$alkylaminocarbonylamino, and
(xxv) di$C_{1-6}$alkylaminocarbonylamino;

substituent group B22 represents the group consisting of

(i) hydroxy,
(ii) carbamoyl,
(iii) a halogen atom,
(iv) $C_{1-6}$alkyl, and
(v) $C_{1-6}$alkoxycarbonylamino;

substituent group B23 represents

(i) $C_{1-6}$alkoxy, wherein the $C_{1-6}$alkoxy may be substituted with one carbamoyl;

substituent group B24 represents the group consisting of

(i) oxo,
(ii) a halogen atom,
(iii) $C_{1-6}$alkyl,
(iv) $C_{1-6}$alkylcarbonyl, and
(v) $C_{1-6}$alkoxycarbonyl;

substituent group B25 represents the group consisting of

(i) $C_{1-6}$alkylcarbonyl and
(ii) $C_{1-6}$alkoxycarbonyl, wherein the $C_{1-6}$alkoxycarbonyl may be substituted with one aryl;

substituent group A31 represents the group consisting of

(i) amino,
(ii) $C_{1-6}$alkyl,
(iii) halo-$C_{1-6}$alkyl,
(iv) $C_{2-6}$alkenyl, wherein the $C_{2-6}$alkenyl may be substituted with one substituent selected from substituent group B32,
(v) saturated heterocyclyl, wherein the saturated heterocyclyl may be substituted with one or two substituents selected from substituent group B34,
(vi) $C_{1-6}$alkoxy,
(vii) halo-$C_{1-6}$alkoxy,
(viii) $C_{1-6}$alkylsulfanyl,

(ix) halo-$C_{1-6}$alkylsulfanyl,
(x) saturated heterocyclylcarbonyl, wherein the saturated heterocyclylcarbonyl may be substituted with one or two $C_{1-6}$alkyl,
(xi) $C_{1-6}$alkylsulfonyl, wherein the $C_{1-6}$alkylsulfonyl may be substituted with one substituent selected from substituent group B35,
(xii) $C_{3-8}$cycloalkylsulfonyl,
(xiii) arylsulfonyl, wherein the arylsulfonyl may be substituted with one $C_{1-6}$alkyl,
(xiv) di$C_{1-6}$alkylaminosulfonyl,
(xv) $C_{1-6}$alkoxycarbonylamino, and
(xvi) S-methylsulfonimidoyl

substituent group A32 represents the group consisting of

(i) a halogen atom,
(ii) $C_{1-6}$alkyl,
(iii) halo-$C_{1-6}$alkyl, and
(iv) $C_{1-6}$alkoxy;

substituent group B32 represents

(i) aryl;

substituent group B34 represents the group consisting of

(i) $C_{1-6}$alkylcarbonyl,
(ii) $C_{1-6}$alkoxycarbonyl,
(iii) mono$C_{1-6}$alkylaminocarbonyl, and
(iv) di$C_{1-6}$alkylaminocarbonyl;

substituent group B35 represents the group consisting of

(i) $C_{3-8}$cycloalkyl,
(ii) saturated heterocyclyl, and
(iii) saturated heterocyclylcarbonyl;

substituent group A41 represents the group consisting of

(i) $C_{1-6}$alkyl,
(ii) halo-$C_{1-6}$alkyl,
(iii) triazolyl,
(iv) $C_{1-6}$alkylsulfonyl, wherein the $C_{1-6}$alkylsulfonyl may be substituted with one $C_{3-8}$cycloalkyl, and
(v) $C_{1-6}$alkylcarbonylamino;

substituent group A51 represents the group consisting of

(i) a halogen atom and
(ii) $C_{1-6}$alkyl; and

substituent group B61 represents the group consisting of

(i) $C_{1-6}$alkylcarbonylamino and
(ii) $C_{1-6}$alkylcarbonyl($C_{1-6}$alkyl)amino;

or a pharmaceutically acceptable salt thereof.

**2.** The compound or pharmaceutically acceptable salt thereof according to Claim 1, wherein the structure represented by formula [III] shown below:

[Formula 5]

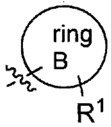

[III]

is any of the structures represented by formula group [V] shown below:

[Formula 6]

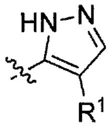

,

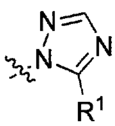

,

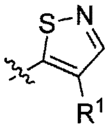

[V]

wherein

$R^1$ is a hydrogen atom, a fluorine atom, a chlorine atom, a bromine atom, or methyl;
$R^2$ is a hydrogen atom, a fluorine atom, or methyl;
$R^3$ is a hydrogen atom or methyl;
$R^4$ is a hydrogen atom;
W is $C_{1-2}$alkanediyl;
ring A is

(a) 4- to 6-membered saturated nitrogen-containing heterocyclyl, wherein the 4- to 6-membered saturated nitrogen-containing heterocyclyl is substituted with one substituent selected from substituent group A21",
(b) phenyl, wherein the phenyl is substituted with one substituent selected from substituent group A31" and may be further substituted with one halogen atom,
(c) pyridyl, wherein the pyridyl is substituted with one substituent selected from substituent group A41",
(d) 2,3-dihydrobenzofuran, wherein the 2,3-dihydrobenzofuran is substituted with one halogen atom and two $C_{1-6}$alkyl, or
(e) 4- to 6-membered saturated oxygen-containing heterocyclyl;

wherein
substituent group A21" represents the group consisting of

(i) $C_{1-6}$alkylcarbonyl, wherein the $C_{1-6}$alkylcarbonyl may be substituted with one to three substituents selected from substituent group B21",
(ii) $C_{3-8}$cycloalkylcarbonyl, wherein the $C_{3-8}$cycloalkylcarbonyl may be substituted with one $C_{1-6}$alkoxycarbonylamino,
(iii) $C_{1-6}$alkoxycarbonyl,
(iv) mono$C_{1-6}$alkylaminocarbonyl,
(v) di$C_{1-6}$alkylaminocarbonyl,
(vi) $C_{1-6}$alkylsulfonyl, wherein the $C_{1-6}$alkylsulfonyl may be substituted with one $C_{1-6}$alkoxycarbonylamino,
(vii) $C_{3-8}$cycloalkylsulfonyl,
(viii) saturated heterocyclylsulfonyl, wherein the saturated heterocyclylsulfonyl may be substituted with one substituent selected from substituent group B25, and
(ix) di$C_{1-6}$alkylaminosulfonyl;

substituent group B21" represents the group consisting of

(i) a halogen atom,
(ii) $C_{3-8}$cycloalkyl, wherein the $C_{3-8}$cycloalkyl may be substituted with one hydroxy,
(iii) aryloxy,
(iv) $C_{3-8}$cycloalkylcarbonylamino, wherein $C_{3-8}$cycloalkyl in the $C_{3-8}$cycloalkylcarbonylamino may be substituted with one or two halogen atoms,

(v) arylcarbonylamino,
(vi) $C_{1-6}$alkoxycarbonylamino, wherein $C_{1-6}$alkoxy in the $C_{1-6}$alkoxycarbonylamino may be substituted with one substituent selected from the group consisting of aryl, and
(vii) $C_{3-8}$cycloalkoxycarbonylamino, wherein $C_{3-8}$cycloalkoxy in the $C_{3-8}$cycloalkoxycarbonylamino may be substituted with one $C_{1-6}$alkyl;

substituent group B25 represents the group consisting of

(i) $C_{1-6}$alkylcarbonyl and
(ii) $C_{1-6}$alkoxycarbonyl, wherein the $C_{1-6}$alkoxycarbonyl may be substituted with one aryl;

substituent group A31" represents the group consisting of

(i) halo-$C_{1-6}$alkyl,
(ii) halo-$C_{1-6}$alkoxy,
(iii) halo-$C_{1-6}$alkylsulfanyl,
(iv) $C_{1-6}$alkylsulfonyl, wherein the $C_{1-6}$alkylsulfonyl may be substituted with one substituent selected from substituent group B35",
(v) $C_{3-8}$cycloalkylsulfonyl, and
(vi) di$C_{1-6}$alkylaminosulfonyl;

substituent group B35" represents the group consisting of

(i) $C_{3-8}$cycloalkyl and
(ii) saturated heterocyclylcarbonyl; and

substituent group A41" is the group consisting of

(i) halo-$C_{1-6}$alkyl and
(ii) $C_{1-6}$alkylsulfonyl, wherein the $C_{1-6}$alkylsulfonyl may be substituted with one $C_{3-8}$cycloalkyl.

3. The compound or pharmaceutically acceptable salt thereof according to Claim 1 or 2, wherein the structure represented by formula [III] shown below:

[Formula 7]

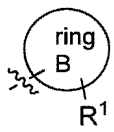

[III]

is the structure of formula [VI] shown below:

[Formula 8]

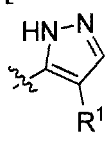

[VI].

4. The compound or pharmaceutically acceptable salt thereof according to Claim 3, wherein ring A is 4- to 6-membered saturated nitrogen-containing heterocyclyl, wherein the 4- to 6-membered saturated nitrogen-containing heterocyclyl is substituted with one substituent selected from substituent group A21".

5. The compound or pharmaceutically acceptable salt thereof according to Claim 3, wherein ring A is phenyl, wherein the phenyl is substituted with one substituent selected from substituent group A31" and may be further substituted with one halogen atom.

**6.** The compound or pharmaceutically acceptable salt thereof according to Claim 1, represented by formula [I] shown below:

[Formula 9]

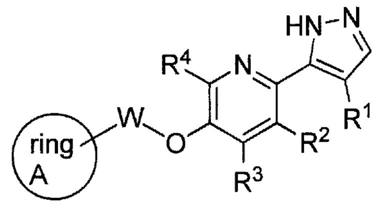

[I]

wherein

$R^1$ represents a hydrogen atom, a fluorine atom, or methyl;
$R^2$, $R^3$, and $R^4$ each independently represent a hydrogen atom, a fluorine atom, or methyl;
W represents a single bond, $C_{1-3}$alkanediyl, or the formula $-O-CH_2CH_2-$;
ring A represents

(a) C4-6cycloalkyl, wherein the $C_{4-6}$cycloalkyl is substituted with one substituent selected from substituent group A11,
(b) 4- to 6-membered saturated nitrogen-containing heterocyclyl, wherein the 4- to 6-membered saturated nitrogen-containing heterocyclyl is substituted with one substituent selected from substituent group A21 and may be further substituted with one substituent selected from substituent group A22,
(c) phenyl, wherein the phenyl is substituted with one substituent selected from substituent group A31 and may be further substituted with one substituent selected from substituent group A32,
(d) pyridyl, wherein the pyridyl is substituted with one substituent selected from substituent group A41,
(e) naphthyl,
(f) 2,3-dihydrobenzofuran, wherein the 2,3-dihydrobenzofuran may be substituted with one to three substituents selected from substituent group A51,
(g) 2H-chromenyl, wherein the 2H-chromenyl may be substituted with one oxo,
(h) quinolyl, wherein the quinolyl may be substituted with one $C_{1-6}$alkoxy,
(j) quinoxalyl,
(k) a group represented by formula [II-1] shown below, wherein the group represented by formula [II-1] is substituted with one $C_{1-6}$alkyl, wherein the $C_{1-6}$alkyl may be substituted with one substituent selected from substituent group B61,
(m) a group represented by formula [II-2] shown below, wherein the group represented by formula [II-2] is substituted with one $C_{1-6}$alkylcarbonyl,
(n) a group represented by formula [II-3] shown below, wherein the group represented by formula [II-3] is substituted with one $C_{1-6}$alkylcarbonyl, or
(p) a group represented by formula [II-4] shown below, wherein the group represented by formula [II-4] is substituted with one $C_{1-6}$alkylcarbonyl;

[Formula 10]

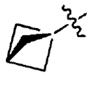 [II−1], 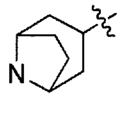 [II−2],

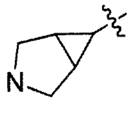 [II−3], 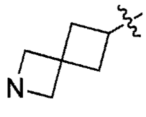 [II−4]

wherein substituent group A11 represents the group consisting of

(i) $C_{1-6}$alkylcarbonylamino and

(ii) $C_{1-6}$alkylcarbonyl($C_{1-6}$alkyl)amino;

substituent group A21 represents the group consisting of

(i) $C_{1-6}$alkylcarbonyl, wherein the $C_{1-6}$alkylcarbonyl may be substituted with one to three substituents selected from substituent group B21,
(ii) $C_{3-8}$cycloalkylcarbonyl, wherein the $C_{3-8}$cycloalkylcarbonyl may be substituted with one or two substituents selected from substituent group B22,
(iii) arylcarbonyl, wherein the arylcarbonyl may be substituted with one substituent selected from substituent group B23,
(iv) saturated heterocyclylcarbonyl, wherein the saturated heterocyclylcarbonyl may be substituted with one or two substituents selected from substituent group B24,
(v) heteroarylcarbonyl, wherein the heteroarylcarbonyl may be substituted with one substituent selected from the group consisting of $C_{1-6}$alkyl, wherein the $C_{1-6}$alkyl may be substituted with one hydroxy,
(vi) $C_{1-6}$alkoxycarbonyl,
(vii) mono$C_{1-6}$alkylaminocarbonyl,
(viii) di$C_{1-6}$alkylaminocarbonyl,
(ix) $C_{3-8}$cycloalkylaminocarbonyl,
(x) $C_{3-8}$cycloalkyl($C_{1-6}$alkyl)aminocarbonyl,
(xi) $C_{1-6}$alkylsulfonyl, wherein the $C_{1-6}$alkylsulfonyl may be substituted with one $C_{1-6}$alkoxycarbonylamino,
(xii) $C_{3-8}$cycloalkylsulfonyl,
(xiii) saturated heterocyclylsulfonyl, wherein the saturated heterocyclylsulfonyl may be substituted with one substituent selected from substituent group B25, and
(xiv) di$C_{1-6}$alkylaminosulfonyl;

substituent group A22 represents the group consisting of

(i) a halogen atom and
(ii) $C_{1-6}$alkyl;

substituent group B21 represents the group consisting of

(i) hydroxy,
(ii) carbamoyl,
(iii) ureide,
(iv) a halogen atom,
(v) $C_{3-8}$cycloalkyl, wherein the $C_{3-8}$cycloalkyl may be substituted with one hydroxy,
(vi) saturated heterocyclyl, wherein the saturated heterocyclyl may be substituted with one or two substituents selected from the group consisting of hydroxy and oxo,
(vii) heteroaryl, wherein the heteroaryl may be substituted with one oxo,
(viii) $C_{1-6}$alkoxy,
(ix) aryloxy,
(x) saturated heterocyclylcarbonyl,
(xi) $C_{1-6}$alkylsulfonyl,
(xii) halo-$C_{1-6}$alkylsulfonyl,
(xiii) arylsulfonyl,
(xiv) $C_{1-6}$alkylcarbonylamino, wherein $C_{1-6}$alkyl in the $C_{1-6}$alkylcarbonylamino may be substituted with one substituent selected from the group consisting of hydroxy and saturated heterocyclyl,
(xv) $C_{1-6}$alkylcarbonyl($C_{1-6}$alkyl)amino,
(xvi) $C_{3-8}$cycloalkylcarbonylamino, wherein $C_{3-8}$cycloalkyl in the $C_{3-8}$cycloalkylcarbonylamino may be substituted with one or two halogen atoms,
(xvii) arylcarbonylamino,
(xviii) saturated heterocyclylcarbonylamino,
(xix) mono$C_{1-6}$alkylaminocarbonyl,
(xx) di$C_{1-6}$alkylaminocarbonyl,
(xxi) $C_{1-6}$alkoxycarbonylamino, wherein $C_{1-6}$alkoxy in the $C_{1-6}$alkoxycarbonylamino may be substituted with one substituent selected from the group consisting of $C_{1-6}$alkoxy and aryl,
(xxii) $C_{1-6}$alkoxycarbonyl($C_{1-6}$alkyl)amino,

(xxiii) $C_{3-8}$cycloalkoxycarbonylamino, wherein $C_{3-8}$cycloalkoxy in the $C_{3-8}$cycloalkoxycarbonylamino may be substituted with one $C_{1-6}$alkyl,
(xxiv) mono$C_{1-6}$alkylaminocarbonylamino, and
(xxv) di$C_{1-6}$alkylaminocarbonyl;

substituent group B22 represents the group consisting of

(i) hydroxy,
(ii) carbamoyl,
(iii) a halogen atom,
(iv) $C_{1-6}$alkyl, and
(v) $C_{1-6}$alkoxycarbonylamino;

substituent group B23 represents

(i) $C_{1-6}$alkoxy, wherein the $C_{1-6}$alkoxy may be substituted with one carbamoyl;

substituent group B24 represents the group consisting of

(i) oxo,
(ii) a halogen atom,
(iii) $C_{1-6}$alkyl,
(iv) $C_{1-6}$alkylcarbonyl, and
(v) $C_{1-6}$alkoxycarbonyl;

substituent group B25 represents the group consisting of

(i) $C_{1-6}$alkylcarbonyl and
(ii) $C_{1-6}$alkoxycarbonyl, wherein the $C_{1-6}$alkoxycarbonyl may be substituted with one aryl;

substituent group A31 represents the group consisting of

(i) amino,
(ii) $C_{1-6}$alkyl,
(iii) halo-$C_{1-6}$alkyl,
(iv) $C_{2-6}$alkenyl, wherein the $C_{2-6}$alkenyl may be substituted with one substituent selected from substituent group B32,
(v) $C_{1-6}$alkoxy,
(vi) halo-$C_{1-6}$alkoxy,
(vii) $C_{1-6}$alkylsulfanyl,
(viii) halo-$C_{1-6}$alkylsulfanyl,
(ix) saturated heterocyclylcarbonyl, wherein the saturated heterocyclylcarbonyl may be substituted with one or two $C_{1-6}$alkyl,
(x) $C_{1-6}$alkylsulfonyl, wherein the $C_{1-6}$alkylsulfonyl may be substituted with one substituent selected from substituent group B35,
(xi) $C_{3-8}$cycloalkylsulfonyl,
(xii) arylsulfonyl, wherein the arylsulfonyl may be substituted with one $C_{1-6}$alkyl,
(xiii) di$C_{1-6}$alkylaminosulfonyl, and
(xiv) $C_{1-6}$alkoxycarbonylamino;

substituent group A32 represents the group consisting of

(i) a halogen atom,
(ii) $C_{1-6}$alkyl,
(iii) halo-$C_{1-6}$alkyl, and
(iv) $C_{1-6}$alkoxy;

substituent group B32 represents

(i) aryl;

substituent group B35 represents the group consisting of

(i) $C_{3-8}$cycloalkyl,
(ii) saturated heterocyclyl, and
(iii) saturated heterocyclylcarbonyl;

substituent group A41 represents the group consisting of

(i) $C_{1-6}$alkyl,
(ii) halo-$C_{1-6}$alkyl,
(iii) triazolyl,
(iv) $C_{1-6}$alkylsulfonyl, wherein the $C_{1-6}$alkylsulfonyl may be substituted with one $C_{3-8}$cycloalkyl, and
(v) $C_{1-6}$alkylcarbonylamino;

substituent group A51 represents the group consisting of

(i) a halogen atom and
(ii) $C_{1-6}$alkyl; and

substituent group B61 represents the group consisting of

(i) $C_{1-6}$alkylcarbonylamino and
(ii) $C_{1-6}$alkylcarbonyl($C_{1-6}$alkyl)amino.

**7.** The compound or pharmaceutically acceptable salt thereof according to Claim 1, 4, 5, or 6, which is shown below:

[Formula 11]

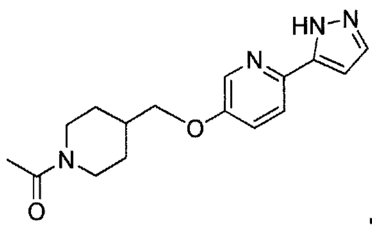
,
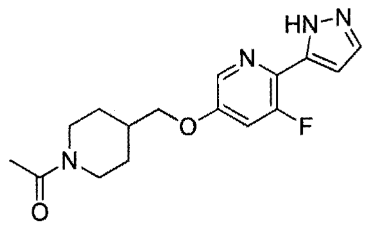
,
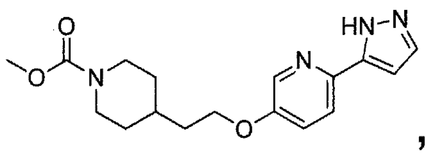
,
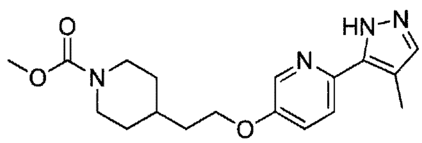
,
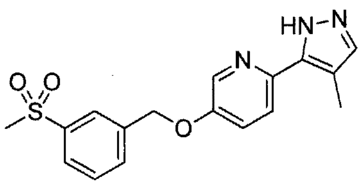
,
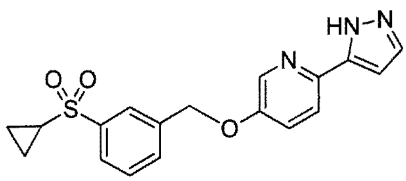
.

**8.** The compound or pharmaceutically acceptable salt thereof according to any one of Claims 1, 4, 5, 6, and 7, which is shown below:

[Formula 12]

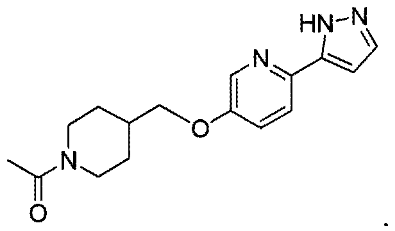

9. The compound or pharmaceutically acceptable salt thereof according to any one of Claims 1, 4, 5, 6, and 7, which is shown below:

[Formula 13]

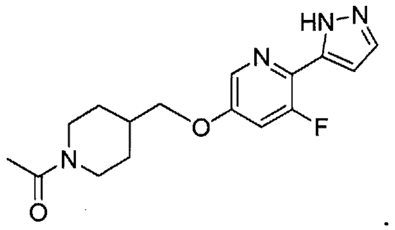

10. The compound or pharmaceutically acceptable salt thereof according to any one of Claims 1, 4, 5, 6, and 7, which is shown below:

[Formula 14]

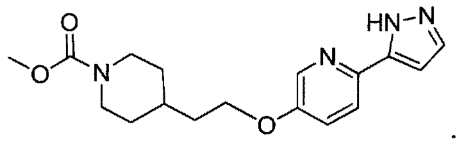

11. The compound or pharmaceutically acceptable salt thereof according to any one of Claims 1, 4, 5, 6, and 7, which is shown below:

[Formula 15]

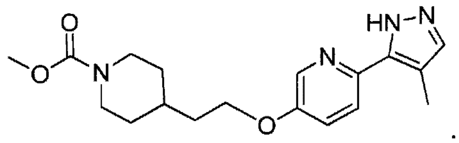

12. The compound or pharmaceutically acceptable salt thereof according to any one of Claims 1, 4, 5, 6, and 7, which is shown below:

[Formula 16]

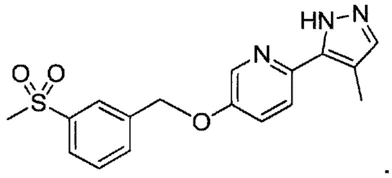

13. The compound or pharmaceutically acceptable salt thereof according to any one of Claims 1, 4, 5, 6, and 7, which is shown below:

[Formula 17]

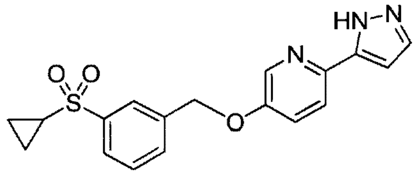

.

**14.** A pharmaceutical comprising the compound or pharmaceutically acceptable salt thereof according to any one of Claims 1 to 13 as an active ingredient.

**15.** An agent that inhibits 20-HETE producing enzyme, wherein the agent comprises the compound or pharmaceutically acceptable salt thereof according to any one of Claims 1 to 13 as an active ingredient.

**16.** An agent that prevents or ameliorates polycystic kidney disease, wherein the agent comprises the compound or pharmaceutically acceptable salt thereof according to any one of Claims 1 to 13 as an active ingredient.

## INTERNATIONAL SEARCH REPORT

International application No.
PCT/JP2017/005388

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D401/04, A61K31/4439, A61K31/444, A61K31/4545, A61K31/46, A61K31/4709, A61K31/498, A61K31/5355, A61K31/5377, A61P13/12, A61P43/00, C07D401/14, C07D405/14, C07D409/14, C07D413/14, C07D417/14, C07D451/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2004-262890 A (Taisho Pharmaceutical Co., Ltd.), 24 September 2004 (24.09.2004), entire text (Family: none) | 1-16 |
| A | WO 2003/022821 A1 (Taisho Pharmaceutical Co., Ltd.), 20 March 2003 (20.03.2003), entire text (Family: none) | 1-16 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 May 2017 (01.05.17) | 16 May 2017 (16.05.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2017/005388

C (Continuation). DOCUMENTS CONSIDERED TO BE RELEVANT

| Category* | Citation of document, with indication, where appropriate, of the relevant passages | Relevant to claim No. |
|---|---|---|
| A | WO 2001/096309 A1 (Taisho Pharmaceutical Co., Ltd.), 20 December 2001 (20.12.2001), entire text & JP 4904659 B2 & US 2003/0186979 A1 & KR 10-2003-0010690 A & CN 1436173 A | 1-16 |

Form PCT/ISA/210 (continuation of second sheet) (January 2015)

**INTERNATIONAL SEARCH REPORT**

International application No.
PCT/JP2017/005388

Continuation of A. CLASSIFICATION OF SUBJECT MATTER
(International Patent Classification (IPC))

*C07D401/04*(2006.01)i, *A61K31/4439*(2006.01)i, *A61K31/444*(2006.01)i, *A61K31/4545*(2006.01)i, *A61K31/46*(2006.01)i, *A61K31/4709*(2006.01)i, *A61K31/498*(2006.01)i, *A61K31/5355*(2006.01)i, *A61K31/5377*(2006.01)i, *A61P13/12*(2006.01)i, *A61P43/00*(2006.01)i, *C07D401/14*(2006.01)i, *C07D405/14*(2006.01)i, *C07D409/14*(2006.01)i, *C07D413/14*(2006.01)i, *C07D417/14*(2006.01)i, *C07D451/02*(2006.01)i

(According to International Patent Classification (IPC) or to both national classification and IPC)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 01032164 A
- WO 03022821 A
- WO 2004092163 A
- WO 2008051406 A2

### Non-patent literature cited in the description


- *Journal of Vascular Research,* 1995, vol. 32, 79
- *The American Journal of Physiology,* 1999, vol. 277, R607
- *Physiological Reviews,* 2002, vol. 82, 131
- *American Journal of Physiology Renal Physiology,* 2009, vol. 296, F575
- *Journal of Lipid Research,* 2013, vol. 55, 1139
- Protective Groups in Organic Synthesis. 2007
- *Tetrahedron Letters,* 1979, vol. 20, 3437
- *Chemical Reviews,* 1995, vol. 95, 2457
- Mitsunobu reaction. *Synthesis,* 1981, 1
- *Tetrahedron Letters,* 1995, vol. 36, 2531
- *Tetrahedron Letters,* 1996, vol. 37, 2463
- *Tetrahedron Letters,* 2002, vol. 43, 399
- *Tetrahedron,* 1984, vol. 40, 1433
- Handbook of Organopalladium Chemistry for Organic Synthesis. 493
- *Angewandte Chemie International Edition in English,* 1963, vol. 2, 565
- *Angewandte Chemie Intarnational Edition in English,* 1979, vol. 18, 72
- Protecting Groups in Organic Synthesis. 2007, 402-403